# EUROPEAN PATENT SPECIFICATION

(11) **EP 3 229 811 B1**
(45) Date of publication and mention of the grant of the patent: **24.02.2021**
(21) Application number: 15826215.4
(22) Date of filing: 04.12.2015
(51) Int. Cl.: A61K 31/685, A61P 29/00, A61P 1/16, A61P 1/06

(54) **PREVENTION AND TREATMENT OF INFLAMMATORY CONDITIONS**
VORBEUGUNG UND BEHANDLUNG VON INFLAMMATORISCHEN KRANKEITEN
PREVENTION ET TRAITEMENT DE MALADIES INFLAMMATOIRES

(30) Priority: 09.12.2014 US 201462089690 P
(43) Date of publication of application: 18.10.2017
(73) Proprietor: GRI Bio, Inc., La Jolla, CA 92037 (US)
(72) Inventor: CHATURVEDI, Vipin, Kumar, La Jolla, CA 92037 (US)
(74) Representative: Forresters IP LLP
(86) International application number: PCT/US2015/063930
(87) International publication number: WO 2016/094226

(56) References cited:
- WO-A1-2014/166941
- US-A- 4 837 023
- US-A1- 2003 171 251
- VERHAAR AUKE P ET AL: "Miltefosine suppresses inflammation in a mouse model of inflammatory bowel disease.", INFLAMMATORY BOWEL DISEASES AUG 2013, vol. 19, no. 9, August 2013 (2013-08), pages 1974-1982, XP009188851, ISSN: 1536-4844
- MARICIC IGOR ET AL: "Recognition of lysophosphatidylcholine by type II NKT cells and protection from an inflammatory liver disease.", JOURNAL OF IMMUNOLOGY (BALTIMORE, MD. : 1950) 1 NOV 2014, vol. 193, no. 9, 1 November 2014 (2014-11-01), pages 4580-4589, XP002755030, ISSN: 1550-6606
- WELLER KARSTEN ET AL: "Miltefosine Inhibits Human Mast Cell Activation and Mediator Release Both In Vitro and In Vivo", JOURNAL OF INVESTIGATIVE DERMATOLOGY, NATURE PUBLISHING GROUP, US, vol. 129, no. 2, 1 February 2009 (2009-02-01), pages 496-498, XP009170963, ISSN: 0022-202X
- HILGARD P ET AL: "INVESTIGATION INTO THE IMMUNOLOGICAL EFFECTS OF MILTEFOSINE A NEW ANTICANCER AGENT UNDER DEVELOPMENT", JOURNAL OF CANCER RESEARCH AND CLINICAL ONCOLOGY, vol. 117, no. 5, 1991, pages 403-408, XP9188834, ISSN: 0171-5216
- DÖLLE S ET AL: "Long-term reduction in local inflammation by a lipid raft molecule in atopic dermatitis.", ALLERGY SEP 2010, vol. 65, no. 9, September 2010 (2010-09), pages 1158-1165, XP002755031, ISSN: 1398-9995
- LISA M. FOX ET AL: "Recognition of Lyso-Phospholipids by Human Natural Killer T Lymphocytes", PLOS BIOLOGY, vol. 7, no. 10, 27 October 2009 (2009-10-27), page e1000228, XP055254725, DOI: 10.1371/journal.pbio.1000228
- ZHANG, G. ET AL: "Sulfatide-activated type II NKT cells prevent allergic airway inflammation by inhibiting type I NKT cell function in a mouse model of asthma", AMERICAN JOURNAL OF PHYSIOLOGY - LUNG CELLULAR AND MOLECULAR PHYSIOLOGY, vol. 301, no. 6, December 2011 (2011-12), pages L975-L984, XP002755032, DOI: 10.1152/AJPLUNG.00114.2011
- KUMAR V: "NKT-cell subsets: Promoters and protectors in inflammatory liver disease", JOURNAL OF HEPATOLOGY 2013 ELSEVIER NLD, vol. 59Activation of NKT-, no. 3, September 2013 (2013-09), pages 618-620, XP002755033, ISSN: 0168-8278

## Description

### BACKGROUND

### Field

The present invention relates to miltefosine for use in alleviating or preventing at least one inflammatory condition in a subject, the method comprising administering the composition in an amount sufficient to activate type II NKT cells of the subject.

### Description of the Related Art

Excessive alcohol use is a major cause of liver disease in the Western world. Evidence of liver injury is observed in individuals who consume four or more alcoholic drinks a day (four 12 ounces beers, four glasses of wine, or four ounces of hard liquor for men or half that quantity for women). Although how alcohol damages the liver is not fully understood, chronic alcohol consumption results in the secretion of pro-inflammatory cytokines (TNF-alpha, IL6 and IL8), oxidative stress, lipid peroxidation, and acetaldehyde toxicity, resulting in inflammation, apoptosis and eventually fibrosis of liver cells.

Alcoholic liver disease (ALD) has three main phases: alcoholic fatty liver disease, alcoholic hepatitis, and cirrhosis. Alcoholic fatty liver disease, which is characterized by an accumulation of fatty acids in the liver, is usually asymptomatic and reversible if the individual abstains from alcohol for a couple of weeks. In severe cases, weakness, nausea, abdominal pain, loss of appetite, and malaise may be experienced. Although most heavy drinkers exhibit some level of fatty liver disease, in some cases, heavy drinking need only have occurred daily over a period of than less than a week, only one in five heavy drinkers develops alcoholic hepatitis, and one in four develops cirrhosis. Alcoholic hepatitis is characterized by the inflammation of hepatocytes and is generally reversible by abstinence. Cirrhosis, which is characterized by inflammation, fibrosis (cellular hardening) and damaged membranes preventing detoxification of chemicals in the body, ending in scarring and necrosis, is generally irreversible.

The cellular and molecular mechanisms underlying different phases of liver tissue damage in alcoholic liver disease are poorly understood. Although progress has been made in several areas, an effective therapeutic approach to halt this disease is still lacking. This is in part owing to the fact that the liver is a unique organ immunologically as well as anatomically. For example, while hepatic parenchymal cells have metabolic functions, non-parenchymal cells perform immunological functions. In addition to parenchymal hepatocytes, the liver contains several non-parenchymal cells such as LSEC, Kupffer cells, dendritic cells, NK cells and NKT cells that all can participate in immunity. How the immune response is orchestrated to give either tolerance or immunity to alcohol-induced damage is not known. WO14/1466941 discloses a disease model where effector T cells are administered in the absence of other types of T and B lymphocytes.

### SUMMARY

The invention is defined in the claims. In accordance with some aspects of the disclosure herein, a method for alleviating or preventing at least one inflammatory condition in a subject is provided. The method can comprise administering an amount of an NKT-2 activator sufficient to activate type II NKT cells to the subject.

In accordance with some aspects of the disclosure herein, a method for alleviating or preventing at least one inflammatory condition in a subject is provided. The method can comprise administering an amount of NKT-2 activator and an amount of sulfatide to the subject, in which the amount of NKT-2 activator and the amount of sulfatide together are sufficient to activate type II NKT cells in the subject.

In accordance with some aspects of the disclosure herein, a method for inhibiting type I NKT cell-mediated tissue damage in a subject is provided. The method can comprise administering an effective amount of a NKT-2 activator to the subject, in which activation of type I NKT cells is reduced.

In accordance with some aspects of the disclosure herein, a composition for use in alleviating or preventing at least one inflammatory condition in a subject is provided. The composition can comprise an amount of a NKT-2 activator sufficient to activate type II NKT cells in the subject.

In accordance with some aspects of the disclosure herein, a composition for use in alleviating or preventing at least one inflammatory condition in a subject is provided. The composition can comprise an amount of a NKT-2 activator and an amount of a sulfatide, in which the amount of NKT-2 activator and the amount of sulfatide together are sufficient to activate type II NKT cells in the subject.

A number of Alternatives are also provided herein:
Alternative 1, forming part of the present disclosure, includes a method for alleviating or preventing at least one inflammatory condition in a subject is provided. The method can comprise administering an amount of a NKT-2 activator sufficient to activate type II NKT cells to the subject.
Alternative 2, forming part of the present disclosure, includes the method of Alternative 1, in which the inflammatory condition is selected from the group consisting of: fatty liver disease, alcohol induced hepatitis, non-alcoholic steatosis hepatitis, cirrhosis, non-alcoholic fatty liver disease, fibrosis, primary sclerosing cholangitis, primary biliary sclerosis, fulminating cirrhosis, idiopathic hepatitis, viral-induced hepatitis (A, B, C and other), inflammatory hepatitis associated with hepato-biliary carcinoma, multiple sclerosis, type 1 diabetes, ischemic reperfusion injury, solid organ transplantation, systemic lupus erythematosus, rheumatoid arthritis, amyotrophic lateral sclerosis, and inflammatory bowel disease (Crohn's and colitis).
Alternative 3, forming part of the present disclosure, includes the method of any one of Altrnatives 1-2, in which the method further comprises administering to the subject an amount of a RAR agonist sufficient to inhibit activation of type I NKT cells to the subject.
Alternative 4, forming part of the present disclosure, includes the method of Alternative 3, in which the NKT-2 activator and the RAR agonist are administered simultaneously.
Alternative 5, forming part of the present disclosure, includes the method of Alternative 3, in which the NKT-2 activator and the RAR agonist are administered separately.
Alternative 6, forming part of the present disclosure, includes the method of Alternative 3, in which the NKT-2 activator and the RAR agonist are administered sequentially.
Alternative 7, forming part of the present disclosure, includes the method of any one of Alternatives 1-6, in which the NKT-2 activator is administered orally.
Alternative 8 includes a composition for use the method of any one of Alternatives 1-7, in which the NKT-2 activator comprises at least one of miltefosine, a miltefosine analog, or a phospholipid, the latter two forming aspects of the disclosure.
Alternative 9, forming part of the present disclosure, includes the method of Alternative 8 in which the phospholipid comprises a lysophophatidylcholine (LPC), an analog of LPC, lyso platelet-activating factor (LPAF), a lysosphingomyelin (LSM), or an analog of LSM.
Alternative 10, forming part of the present disclosure, includes the method of Alternative 9, in which the LPC comprises one of LPC (C18:0), LPC (C16:0), LPC (C18:1(9Z)), LPC(C18:1 (1oleyl)), or LignA(LPC)(C24:0).
Alternative 11 includes the method of any one of Alternatives 1-7, in which the NKT-2 activator comprises at least one of miltefosine or a miltefosine analog, the latter forming an aspect of the disclosure.
Alternative 12, forming part of the present disclosure, includes the method of any one of Alternatives 8-11, in which the miltefosine analog comprises a compound from Table 2.1 or Table 2.2.
Alternative 13, forming part of the present disclosure, includes the method of any one of Alternatives 8-11, in which the miltefosine analog comprises a compound from Table 2.1.
Alternative 14 includes the method of any one of Alternatives 1-7, in which the NKT-2 activator comprises miltefosine.
Alternative 15 includes the method of any one of Alternatives 3-14, in which the RAR agonist comprises ATRA or isotretinoin.
Alternative 16 includes the method of any one of Alternatives 3-14, in which the RAR agonist comprises tazarotene.
Alternative 17 includes the method of any one of Alternatives 3-14, in which the RAR agonist comprises retinol or an ester of retinol.
Alternative 18 includes the method of any one of Alternative 1-17, in which activation of type II NKT cells comprises at least one of: production of IL-2 by the type II NKT cells, proliferation of type II NKT cells, or expression of CD69 on the surface of type II NKT cells.
Alternative 19 includes the method of any one of Alternatives 3-18, wherein inhibition of activation of type I NKT cells comprises inhibition of accumulation of alpha-GalCer/CD1d-tetramer⁺TCR-beta⁺ cells.
Alternative 20, forming part of the present disclosure, includesa a method for alleviating or preventing at least one inflammatory condition in a subject comprising administering an amount of NKT-2 activator and an amount of sulfatide to the subject, in which the amount of NKT-2 activator and the amount of sulfatide together are sufficient to activate type II NKT cells in the subject.
Alternative 21, forming part of the present disclosure, includes the method of Alternative 20 in which the inflammatory condition is selected from the group consisting of: fatty liver disease, alcohol induced hepatitis, non-alcoholic steatosis hepatitis, cirrhosis, non-alcoholic fatty liver disease, fibrosis, primary sclerosing cholangitis, primary biliary sclerosis, fulminating cirrhosis, idiopathic hepatitis, viral-induced hepatitis (A, B, C and other), inflammatory hepatitis associated with hepato-biliary carcinoma, multiple sclerosis, type 1 diabetes, ischemic reperfusion injury, solid organ transplantation, systemic lupus erythematosus, rheumatoid arthritis, amyotrophic lateral sclerosis, and inflammatory bowel disease (Crohn's and colitis).
Alternative 22, forming part of the present disclosure, includes the method of Alternative 20 or Alternative 21, in which the sulfatide and NKT-2 activator are administered simultaneously.
Alternative 23, forming part of the present disclosure, includes the method of Alternative 20 or Alternative 21, in which the sulfatide and NKT-2 activator are administered separately.
Alternative 24, forming part of the present disclosure, includes the method of any one of Alternatives 20-23, in which the NKT-2 activator is administered orally.
Alternative 25, forming part of the present disclosure, includes the method of any one of Alternatives 20-24, in which the amount of NKT-2 activator is sufficient to activate type II NKT cells in the subject, and the amount of sulfatide is sufficient to activate type II NKT cells in the subject.
Alternative 26, forming part of the present disclosure, includes the method of any one of Alternatives 20-25, in which the method furhter comprises administering to the subject an amount of a RAR agonist sufficient to inhibit activation of type I NKT cells to the subject.
Alternative 27, forming part of the present disclosure, includes the method of Alternative 26, in which the NKT-2 activator and the RAR agonist are administered simultaneously.
Alternative 28, forming part of the present disclosure, includes the method of Alternative 26, in which the sulfatide and NKT-2 activator are administered separately.
Alternative 29, forming part of the present disclosure, includes the method of Alternative 26, in which the NKT-2 activator and the RAR agonist are administered sequentially.
Alternative 30, forming part of the present disclosure, includes the method of any one of Alternatives 20-29, in which the NKT-2 activator comprises at least one of miltefosine, a miltefosine analog, or a phospholipid.
Alternative 31, forming part of the present disclosure, includes the method of Alternative 30, in which the phospholipid comprises a lysophophatidylcholine (LPC), an analog of LPC, lyso platelet-activating factor (LPAF), a lysosphingomyelin (LSM), or an analog of LSM.
Alternative 32, forming part of the present disclosure, includes the method of Alternative 31, in which the LPC comprises one of LPC (C18:0), LPC (C16:0), LPC (C18:1(9Z)), LPC(C18:1 (1oleyl)), or LignA(LPC)(C24:0).
Alternative 33, forming part of the present disclosure, includes the method of any one of Alternatives 20-29, in which the NKT-2 activator comprises at least one of miltefosine or a miltefosine analog.
Alternative 34, forming part of the present disclosure, includes the method of any one of Alternatives 30-32, in which the miltefosine analog comprises a compound from Table 2.1 or Table 2.2.
Alternative 35, forming part of the present disclosure, includes the method of any one of Alternative 30-32, in which the miltefosine analog comprises a compound from Table 2.1.
Alternative 36, forming part of the present disclosure, includes the method of any one of Alternatives 20-29, in which the NKT-2 activator comprises miltefosine.
Alternative 37, forming part of the present disclosure, includes the method of any one of Alternatives 20-36, in which the sulfatide has the following chemical structure: 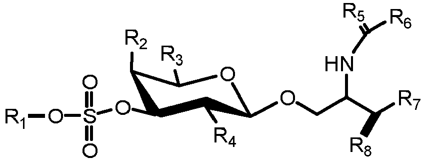 in which R1 is selected from the group consisting of a bond, a hydrogen, a C₁ to C₃₀ alkyl, C₁ to C₃₀ substituted alkyl, a C₁ to C₃₀ alkenyl, a C₁ to C₃₀ substituted alkenyl and a C₅ to C₁₂ sugar; R₂ is selected from the group consisting of a hydrogen, a hydroxy group, a methoxy group, and an alkoxy group; R₃ is selected from the group consisting of a hydrogen, a hydroxy group, a methoxy group, an ethoxy group, and an alkoxy group; R₄ is selected from the group consisting of a hydrogen, a hydroxy group and an alkoxy group; R₅ is selected from the group consisting of a hydrogen, a hydroxyl, a carbonyl, an alkoxy and a bond; R₆ is selected from the group consisting of a C₁ to C₄₀ alkyl, a C₁ to C₄₀ substituted alkyl, a C₁ to C₄₀ alkenyl, a C₁ to C₄₀ substituted alkenyl and a C₁ to C₄₀ alkynl; R₇ is selected from the group consisting of a C₁ to C₄₀ alkyl, a C₁ to C₄₀ substituted alkyl, a C₁ to C₄₀ alkenyl, a C₁ to C₄₀ substituted alkenyl and a C₁ to C₄₀ alkynl; and R₈ is selected from the group consisting of a hydrogen, a hydroxyl group, a carbonyl, an alkoxy group and a bond.
Alternative 38, forming part of the present disclosure, includes the method of any one of Alternatives 20-36, in which the sulfatide has the following chemical structure: 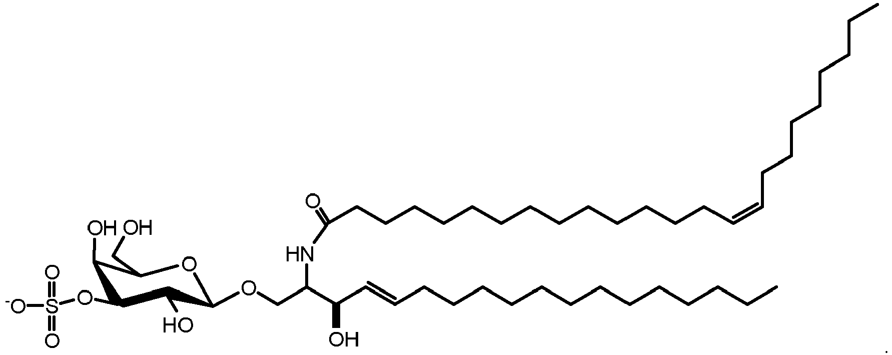
Alternative 39, forming part of the present disclosure, includes the method of any one of Alternatives 20-35, in which the RAR agonist comprises ATRA or isotretinoin.
Alternative 40, forming part of the present disclosure, includes the method of any one of Alternatives 20-38, in which the RAR agonist comprises tazarotene.
Alternative 41, forming part of the present disclosure, includes the method of any one of Alternatives 20-38, in which the RAR agonist comprises retinol or an ester of retinol.
Alternative 42, forming part of the present disclosure, includes a method for inhibiting type I NKT cell-mediated tissue damage in a subject, the method comprising administering an effective amount of a phospholipid to the subject, wherein activation of type I NKT cells is reduced.
Alternative 43, forming part of the present disclosure, includes the method of Alternative 42 in which the inflammatory condition is selected from the group consisting of: fatty liver disease, alcohol induced hepatitis, non-alcoholic steatosis hepatitis, cirrhosis, non-alcoholic fatty liver disease, fibrosis, primary sclerosing cholangitis, primary biliary sclerosis, fulminating cirrhosis, idiopathic hepatitis, viral-induced hepatitis (A, B, C and other), inflammatory hepatitis associated with hepato-biliary carcinoma, multiple sclerosis, type 1 diabetes, ischemic reperfusion injury, solid organ transplantation, systemic lupus erythematosus, rheumatoid arthritis, amyotrophic lateral sclerosis, and inflammatory bowel disease (Crohn's and colitis).
Alternative 44, forming part of the present disclosure, includes the method of any one of Alternatives 42-43, in which the phospholipid comprises at least one of miltefosine, a miltefosine analog, or a phospholipid.
Alternative 45, forming part of the present disclosure, includes the method of Alterantive 44, in which the phospholipid comprises a lysophophatidylcholine (LPC), an analog of LPC, lyso platelet-activating factor (LPAF), a lysosphingomyelin (LSM), or an analog of LSM.
Alternative 46, forming part of the present disclosure, includes the method of Alternative 45, in which the LPC comprises one of LPC (C18:0), LPC (C16:0), LPC (C18:1(9Z)), LPC(C18:1 (1oleyl)), or LignA(LPC)(C24:0).
Alternative 47, forming part of the present disclosure, includes the method of any one of Alternative 42-43, in which the phospholipid comprises at least one of miltefosine or a miltefosine analog.
Alternative 48, forming part of the present disclosure, includes the method of any one of Alternative 44-47, in which the miltefosine analog comprises a compound from Table 2.1 or Table 2.2.
Alternative 49, forming part of the present disclosure, includes the method of any one of Alternative 44-47, in which the miltefosine analog comprises a compound from Table 2.1.
Alternative 50, forming part of the present disclosure, includes the method of any one of Alternative 42-43, in which the NKT-2 activator comprises miltefosine.
Alternative 51, forming part of the present disclosure, includes a composition for use in alleviating or preventing at least one inflammatory condition in a subject, the composition comprising an amount of a NKT-2 activator sufficient to activate type II NKT cells in the subject.
Alternative 52, forming part of the present disclosure, includes the composition of Alternative 51 further comprising an amount of a RAR agonist sufficient to inhibit activation of type I NKT cells to the subject.
Alternative 53, forming part of the present disclosure, includes the composition of any one of Alternative 48-49, in which the inflammatory condition is selected from the group consisting of: fatty liver disease, alcohol induced hepatitis, non-alcoholic steatosis hepatitis, non-alcoholic fatty liver disease, fibrosis, primary sclerosing cholangitis, primary biliary sclerosis, cirrhosis, fulminating cirrhosis, idiopathic hepatitis, viral-induced hepatitis (A, B, C and other), inflammatory hepatitis associated with hepato-biliary carcinoma, autoimmune hepatitis, multiple sclerosis, type 1 diabetes, ischemic reperfusion injury, solid organ transplantation, systemic lupus erythematosus, rheumatoid arthritis, amyotrophic lateral sclerosis, and inflammatory bowel disease (Crohn's and colitis).
Alternative 54, forming part of the present disclosure, includes the composition of any one of Alternatives 51-53, in which the NKT-2 activator comprises at least one of miltefosine, a miltefosine analog, or a phospholipid.
Alternative 55, forming part of the present disclosure, includes the composition of Alternative 54 in which the phospholipid comprises a lysophophatidylcholine (LPC), an analog of LPC, lyso platelet-activating factor (LPAF), a lysosphingomyelin (LSM), or an analog of LSM.
Alternative 56, forming part of the present disclosure, includes the composition of Alternative 55, in which the LPC comprises one of LPC (C18:0), LPC (C16:0), LPC (C18:1(9Z)), LPC(C18:1(1oleyl)), or LignA(LPC)(C24:0).
Alternative 57, forming part of the present disclosure, includes the composition of any one of Alternatives 51-53, in which the phospholipid comprises at least one of miltefosine or a miltefosine analog.
Alternative 58, forming part of the present disclosure, includes the composition of any one of Alternatives 54-57, in which the miltefosine analog comprises a compound from Table 2.1 or Table 2.2.
Alternative 59, forming part of the present disclosure, includes the composition of any one of Alternatives 51-57, in which the miltefosine analog comprises a compound from Table 2.1.
Alternative 60, forming part of the present disclosure, includes the composition of any one of Alternatives 51-53, in which the NKT-2 activator comprises miltefosine.
Alternative 61, forming part of the present disclosure, includes the composition of any one of Alternatives 52-60, in which the RAR agonist comprises ATRA or isotretinoin.
Alternative 62, forming part of the present disclosure, includes the composition of any one of Alternatives 52-60, in which the RAR agonist comprises tazarotene.
Alternative 63, forming part of the present disclosure, includes the composition of any one of Alternatives 52-60, in which the RAR agonist comprises retinol or an ester of retinol.
Alternative 64, forming part of the present disclosure, includes the composition of any one of Alternatives 51-63, in which the composition is formulated for oral administration.
Alternative 65, forming part of the present disclosure, includes a composition for use in alleviating or preventing at least one inflammatory condition in a subject, the composition comprising an amount of a NKT-2 activator and an amount of a sulfatide, in which the amount of NKT-2 activator and the amount of sulfatide together are sufficient to activate type II NKT cells in the subject.
Alternative 66, forming part of the present disclosure, includes the composition of Alternative 61, in which the inflammatory condition is selected from the group consisting of: fatty liver disease, alcohol induced hepatitis, non-alcoholic steatosis hepatitis, cirrhosis, non-alcoholic fatty liver disease, fibrosis, primary sclerosing cholangitis, primary biliary sclerosis, fulminating cirrhosis, idiopathic hepatitis, viral-induced hepatitis (A, B, C and other), inflammatory hepatitis associated with hepato-biliary carcinoma, autoimmune hepatitis, multiple sclerosis, type 1 diabetes, ischemic reperfusion injury, solid organ transplantation, systemic lupus erythematosus, rheumatoid arthritis, amyotrophic lateral sclerosis, and inflammatory bowel disease (Crohn's and colitis).
Alternative 67, forming part of the present disclosure, includes the composition of any one of Alternatives 65-66, in which the amount of NKT-2 activator is sufficient to activate type II NKT cells in the subject, and the amount of sulfatide is sufficient to activate type II NKT cells in the subject.
Alternative 68, forming part of the present disclosure, includes the composition of any one of Alternatives 653-67, in which the NKT-2 activator comprises at least one of miltefosine, a miltefosine analog, or a phospholipid.
Alternative 69, forming part of the present disclosure, includes the composition of Alternative 68 in which the phospholipid comprises a lysophophatidylcholine (LPC), an analog of LPC, lyso platelet-activating factor (LPAF), a lysosphingomyelin (LSM), or an analog of LSM.
Alternative 70, forming part of the present disclosure, includes the composition of Alternative 69, in which the LPC comprises one of LPC (C18:0), LPC (C16:0), LPC (C18:1(9Z)), LPC(C18:1(1oleyl)), or LignA(LPC)(C24:0).
Alternative 71, forming part of the present disclosure, includes the composition of any one of Alternatives 65-67, in which the NKT-2 activator comprises at least one of miltefosine or a miltefosine analog.
Alternative 72, forming part of the present disclosure, includes the composition of any one of Alternatives 68-71, in which the miltefosine analog comprises a compound from Table 2.1 or Table 2.2.
Alternative 73, forming part of the present disclosure, includes the composition of any one of Alternatives 68-71, in which the miltefosine analog comprises a compound from Table 2.1.
Alternative 74, forming part of the present disclosure, includes the composition of any one of Alternatives 65-67, in which the NKT-2 activator comprises miltefosine.
Alternative 75, forming part of the present disclosure, includes the composition of any one of Alternatives 65-74, in which the sulfatide has the following chemical structure: 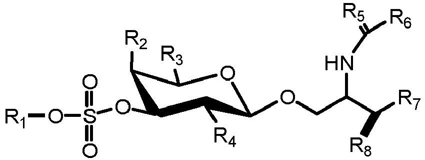 in which R1 is selected from the group consisting of a bond, a hydrogen, a C₁ to C₃₀ alkyl, C₁ to C₃₀ substituted alkyl, a C₁ to C₃₀ alkenyl, a C₁ to C₃₀ substituted alkenyl and a C₅ to C₁₂ sugar; R₂ is selected from the group consisting of a hydrogen, a hydroxy group, a methoxy group, and an alkoxy group; R₃ is selected from the group consisting of a hydrogen, a hydroxy group, a methoxy group, an ethoxy group, and an alkoxy group; R₄ is selected from the group consisting of a hydrogen, a hydroxy group and an alkoxy group; R₅ is selected from the group consisting of a hydrogen, a hydroxyl, a carbonyl, an alkoxy and a bond; R₆ is selected from the group consisting of a C₁ to C₄₀ alkyl, a C₁ to C₄₀ substituted alkyl, a C₁ to C₄₀ alkenyl, a C₁ to C₄₀ substituted alkenyl and a C₁ to C₄₀ alkynl; R₇ is selected from the group consisting of a C₁ to C₄₀ alkyl, a C₁ to C₄₀ substituted alkyl, a C₁ to C₄₀ alkenyl, a C₁ to C₄₀ substituted alkenyl and a C₁ to C₄₀ alkynl; and R₈ is selected from the group consisting of a hydrogen, a hydroxyl group, a carbonyl, an alkoxy group and a bond.
Alternative 76, forming part of the present disclosure, includes the composition of any one of Alternatives 65-74, in which the sulfatide has the following chemical structure: 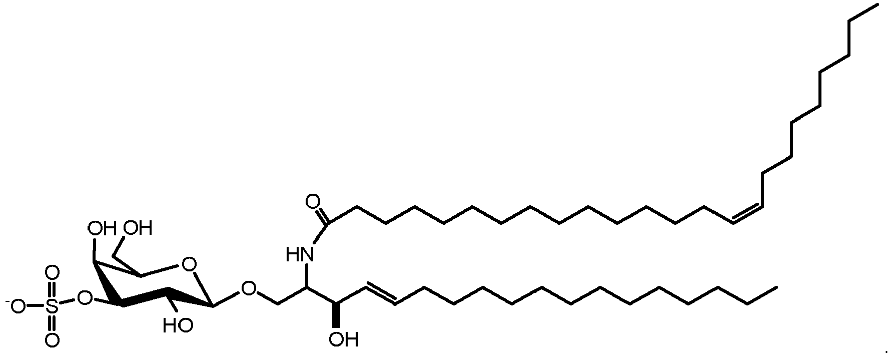
Alternative 77, forming part of the present disclosure, includes the composition of any one of Alternatives 65-76, in which the composition further comprises an amount of a RAR agonist sufficient to inhibit activation of type I NKT cells in the subject.
Alternative 78, forming part of the present disclosure, includes the composition of Alternative 77, in which the RAR agonist comprises ATRA or isotretinoin.
Alternative 79, forming part of the present disclosure, includes the composition of Alternative 77, in which the RAR agonist comprises tazarotene.
Alternative 80, forming part of the present disclosure, includes the composition of Alternative 77, in which the RAR agonist comprises retinol or an ester of retinol.
Alternative 81 includes the method of any one of Alternatives 1-50, in which the inflammatory condition is selected from the group consisting of: fatty liver disease, alcohol induced hepatitis, non-alcoholic steatosis hepatitis, cirrhosis, non-alcoholic fatty liver disease, fibrosis, primary sclerosing cholangitis, primary biliary sclerosis, fulminating cirrhosis, idiopathic hepatitis, viral-induced hepatitis (A, B, C and other), inflammatory hepatitis associated with hepato-biliary carcinoma, multiple sclerosis, type 1 diabetes, ischemic reperfusion injury, solid organ transplantation, systemic lupus erythematosus, rheumatoid arthritis, amyotrophic lateral sclerosis, and inflammatory bowel disease (Crohn's and colitis).
Alternative 82 includes a composition for use in the method of any one of Alternatives 1-50 or 81 in which the amount of NKT-2 activator is sufficient to activate CD8αα⁺ T cells in the liver of the subject.
Alternative 83 includes a composition for use in the method of any one of Alternatives 1-50 or 81-82, further comprising administering isolated CD8αα⁺, TCRαβ⁺ T cells to the subject.
Alternative 84 includes a composition for use in the method of one of Alternatives 82-83, in which the isolated CD8αα⁺, TCRαβ⁺ T cells are administered to the subject prior to the NKT-2 activator.
Alternative 85 includes a composition for use in the method of one of Alternatives 82-83, in which the isolated CD8αα⁺, TCRαβ⁺ T cells are administered to the subject concurrent with the NKT-2 activator.
Alternative 86 includes the method of one of Alternatives 82-83, in which the isolated CD8αα⁺, TCRαβ⁺ T cells are administered to the subject subsequent to the NKT-2 activator.
Alternative 87 includes a composition for use in the method of any one of Alternatives 82-87, and further comprises obtaining a cell sample from a mammal, isolating CD8αα⁺, TCRαβ⁺ T cells from the cell sample, and expanding the isolated T cells.
Alternative 88 includes a composition for use in the method of Alternative 87, in which the mammal is the subject.
Alternative 89 includes a composition for use in the method of Alternative 87, in which the mammal is an organism other than the subject.
Alternative 90 includes a composition for use in the method of any one of Alternatives 82-89, in which the isolated T cells are CD8αα⁺, TCRαβ⁺, CD200⁺.
Alternative 91 includes a composition for use in the method of any one of Alternatives 82-89, in which the isolated T cells are CD8αα⁺, TCRαβ⁺, CD122⁺.
Alternative 92 includes a composition for use in the method of any one of Alternatives 82-89, in which isolated T cells are CD8αα⁺, TCRαβ⁺, CD200⁺, CD122⁺.
Alternative 93 includes a composition for use in the method of any one of Alternatives 82-92, in which the isolated T cells are a mixture of two or more of CD8αα⁺ TCRαβ⁺ T cells, CD8αα⁺ TCRαβ CD200⁺ T cells, CD8αα⁺ TCRαβCD122⁺ T cells, and CD8αα⁺, TCRαβ⁺, CD200⁺ CD122⁺ T cells.
Alternative 94, forming part of the present disclosure, includes a method for alleviating or preventing at least one inflammatory condition in a subject, the method comprising administering isolated CD8αα+, TCRαβ+ T cells to the subject, and administering an amount of an NKT-2 activator sufficient to activate type II NKT cells to the subject.
Alternative 95, forming part of the present disclosure, includes the method of Alternative 94, in which the inflammatory condition is selected from the group consisting of: fatty liver disease, alcohol induced hepatitis, non-alcoholic steatosis hepatitis, autoimmune hepatitis, cirrhosis, non-alcoholic fatty liver disease, fibrosis, primary sclerosing cholangitis, primary biliary sclerosis, fulminating cirrhosis, idiopathic hepatitis, viral-induced hepatitis (A, B, C and other), inflammatory hepatitis associated with hepato-biliary carcinoma, multiple sclerosis, type 1 diabetes, ischemic reperfusion injury, solid organ transplantation, systemic lupus erythematosus, rheumatoid arthritis, amyotrophic lateral sclerosis, and inflammatory bowel disease (Crohn's and colitis).
Alternative 96, forming part of the present disclosure, includes the method of any one of Alternatives 94-95, in which the NKT-2 activator comprises at least one of miltefosine or a miltefosine analog.
Alternative 97, forming part of the present disclosure, includes the method of any one of Alternatives 94-95, in which the NKT-2 activator comprises miltefosine.
Alternative 98, forming part of the present disclosure, includes the method of any one of Alternatives 94-97, in which the amount of NKT-2 activator is sufficient to activate CD8αα⁺ T cells in the liver of the subject.
Alternative 99, forming part of the present disclosure, includes the method of any one of Alternatives 94-98, in which the isolated CD8αα⁺, TCRαβ⁺ T cells are administered to the subject prior to the NKT-2 activator.
Alternative 100, forming part of the present disclosure, includes the method of any one of Alternatives 94-98, in which the isolated CD8αα⁺, TCRαβ⁺ T cells are administered to the subject concurrent with the NKT-2 activator.
Alternative 101, forming part of the present disclosure, includes the method of any one of Alternatives 94-98, in which the isolated CD8αα⁺, TCRαβ⁺ T cells are administered to the subject subsequent to the NKT-2 activator.
Alternative 102, forming part of the present disclosure, includes the method of any one of Alternatives 94-101, and further comprises obtaining a cell sample from a mammal, isolating CD8αα⁺, TCRαβ⁺ T cells from the cell sample, and expanding the isolated T cells.
Alternative 103, forming part of the present disclosure, includes the method of Alternative 102, in which the mammal is the subject.
Alternative 104, forming part of the present disclosure, includes the method of Alternative 102, in which the mammal is an organism other than the subject.
Alternative 105, forming part of the present disclosure, includes the method of any one of Alternatives 94-104, in which the isolated T cells are CD8αα⁺, TCRαβ⁺, CD200⁺.
Alternative 106, forming part of the present disclosure, includes the method of any one of Alternatives 94-104, in which the isolated T cells are CD8αα⁺, TCRαβ⁺, CD122⁺.
Alternative 107, forming part of the present disclosure, includes the method of any one of Alternatives 94-104, in which the isolated T cells are CD8αα⁺, TCRαβ⁺, CD200⁺, CD122⁺.
Alternative 108, forming part of the present disclosure, includes the method of any one of Alternatives 94-104, in which the isolated T cells comprise a mixture of two or more of CD8αα⁺ TCRαβ⁺ T cells, CD8αα⁺ TCRαβ⁺ CD200⁺ T cells, CD8αα⁺ TCRαβ⁺ CD122⁺ T cells, and CD8αα⁺, TCRαβ⁺, CD200⁺ CD122⁺ T cells.
Alternative 109, forming part of the present disclosure, includes the composition of any one of Alternatives 51-80, in which the inflammatory condition is selected from the group consisting of: fatty liver disease, alcohol induced hepatitis, non-alcoholic steatosis hepatitis, cirrhosis, non-alcoholic fatty liver disease, fibrosis, primary sclerosing cholangitis, primary biliary sclerosis, fulminating cirrhosis, idiopathic hepatitis, viral-induced hepatitis (A, B, C and other), inflammatory hepatitis associated with hepato-biliary carcinoma, multiple sclerosis, type 1 diabetes, ischemic reperfusion injury, solid organ transplantation, systemic lupus erythematosus, rheumatoid arthritis, amyotrophic lateral sclerosis, and inflammatory bowel disease (Crohn's and colitis).
Alternative 110, forming part of the present disclosure, includes the composition of any one of Alternatives 51-80, or 109, in which the amount of NKT-2 activator is sufficient to activate CD8αα⁺ T cells in the liver of the subject.
Alternative 111, forming part of the present disclosure, includes the composition of any one of Alternatives 51-80, or 109 or 110, further comprising isolated CD8αα+, TCRαβ+ T cells for use in administration to the subject.
Alternative 112, forming part of the present disclosure, includes the composition of Alternative 111, in which the isolated T cells are CD8αα+, TCRαβ⁺, CD200+.
Alternative 113, forming part of the present disclosure, includes the composition of Alternative 111, in which the isolated T cells are CD8αα+, TCRαβ⁺, CD122+. Alternative 114, forming part of the present disclosure, includes the composition of Alternative 111, in which the isolated T cells are CD8αα+, TCRαβ⁺, CD200+, CD122+.
Alternative 115, forming part of the present disclosure, includes the composition of Alternative 111, in which the isolated T cells comprise a mixture of two or more of CD8αα⁺ TCRαβ⁺ T cells, CD8αα⁺ TCRαβ⁺ CD200⁺ T cells, CD8αα⁺ TCRαβ⁺ CD122⁺ T cells, and CD8αα⁺, TCRαβ⁺, CD200⁺ CD122⁺ T cells.

### BRIEF DESCRIPTION OF THE DRAWINGS

**Figure 1** is a graph depicting stimulation of a type II NKT cell by miltefosine in accordance with some embodiments herein.
**Figure 2A** is a graph depicting inhibition of type I NKT cell expansion following injection of miltefosine in accordance with some embodiments herein.
**Figure 2B** is a series of graphs depicting flow cytometry data showing inhibition of type I NKT cell expansion following injection of miltefosine in accordance with some embodiments herein.
**Figure 3A** is a graph depicting inhibition of ConA-induced hepatitis following treatment with miltefosine in accordance with some embodiments herein.
**Figure 3B** is a series of microscope images depicting inhibition of ConA-induced hepatitis following treatment with miltefosine in accordance with some embodiments herein. **Figure 3C** is a black-and-white reproduction of **Figure 3B****.**
**Figure 4A** is a graph depicting inhibition of the accumulation of type I NKT cells during alcoholic liver diseases (ALD) in mice treated with miltefosine in accordance with some embodiments herein.
**Figure 4B** is series of graphs depicting flow cytometry data showing inhibition of the accumulation of type I NKT cells during alcoholic liver diseases (ALD) in mice treated with miltefosine in accordance with some embodiments herein.
**Figure 4C** is a graph depicting inhibition of the accumulation of activated (CD69⁺) type I NKT cells during alcoholic liver diseases (ALD) in mice treated with miltefosine in accordance with some embodiments herein.
**Figure 4D** is a graph depicting inhibition of the accumulation of neutrophils during alcoholic liver diseases (ALD) in mice treated with miltefosine in accordance with some embodiments herein.
**Figure 5** is a graph depicting treatment of chronic and relapsing experimental autoimmune encephalomyelitis (EAE), a prototype for multiple sclerosis with miltefosine in accordance with some embodiments herein.
**Figure 6** is a graph depicting treatment of chronic experimental autoimmune encephalomyelitis (EAE), a prototype for multiple sclerosis with sulfatide.
**Figure 7** is a graph depicting that *in vitro* expansion of type I NKT cells in human PBMC is inhibited after Miltefosine treatment in accordance with some embodiments herein.
**Figures 8A and 8B** are a pair of graphs depicting mean disease scores of mice receiving different amounts of expanded T_{reg} cell populations in terms of the number of days after inducement of EAE with an injection of MBPAcl-9 (myelin basic protein) in accordance with some embodiments herein. **Figure 8A** depicts results for mice injected with 2D11 cells. **Figure 8B** depicts results for mice injected with a p42-50-reactive T cell line.
**Figures 9A-9C** are a series of graphs showing that treatment of mice with agonistic anti-Vβ6 mAb in vivo results in activation of CD8αα⁺ VP6⁺ T cells and prevention of EAE in accordance with some embodiments herein. **Figure 9A** depicts characterization of the cells by flow cytometry. **Figure 9B** depicts relative quantities of Vβ6 and Vβ8.2 based on real-time PCR data. **Figure 9C** depicts percentage of TL-tetramer/CD8+ T cells.

### DETAILED DESCRIPTION

The invention is defined in the claims. In accordance with some embodiments herein, compositions comprising an amount of NKT-2 activator sufficient to activate Type II NKT cells can be useful in alleviating or preventing any of a number of inflammatory conditions. Without being limited by any theory, it is contemplated herein that upon activation, Type II NKT cells can inhibit activation Type I NKT cells, and thus can alleviate or prevent Type I NKT cell-mediated damage associated with inflammation. Accordingly, in some embodiments, compositions for use in methods of alleviating or preventing inflammatory conditions are provided wherein the method comprises administering a composition comprising an amount of NKT-2 activator, for example miltefosine or an analog of miltefosine sufficient to activate Type II NKT cells, the latter example forming part of the present disclosure. Optionally the composition can further comprise an amount of sulfatide sufficient to activate Type II NKT cells. Optionally the composition can comprise an amount of RAR agonist sufficient to inhibit activation of Type I NKT cells.

### NKT cells

The liver harbors a number of specialized cells of the innate immune system, including Kupffer cells, Natural Killer (NK) cells, Natural Killer T (NKT) cells and dendritic cells. NKT cells are unique in that they share the cell surface receptors of NK cells (e.g., NK1.1) and in addition express T cell receptors (TCR), enabling them to recognize lipid antigens in the context of CD1d molecules and bridge the innate immune responses to adaptive immunity. NKT cells have the ability to regulate the activity of other cells that contribute to inflammation of tissue and the associated cellular damage. Upon activation, NKT cells rapidly secrete large quantities of IFN-gamma, IL-4, granulocyte-macrophage colony-stimulating factor, as well as multiple other cytokines and chemokines. Since NKT cells are capable of secreting both Th1 and Th2 cytokines, it can be difficult to predict the consequences of NKT cell activation in vivo. Depending upon context, NKT cell activation triggers cascades of events that promote or suppress different immune responses. In some contexts, activation of NKT cells leads to the activation of NK cells, dendritic cells (DCs) and B cells. Aspects of NKT cells are discussed in Kumar, "NKT-cell subsets: Promoters and protectors in inflammatory liver disease" Journal of Hepatology, 2013, 59: 618-620.

NKT cells recognize lipid antigens presented in the context of the monomorphic MHC class I-like molecule, CD1d. CD1d-restricted NKT cells are categorized into type I (also referred to as "type INKT cells" or "NKT-1" cells) and type II (also referred to as "type II NKT cells" or "NKT-2" cells), which recognize different lipid antigens presented by CDld molecules. While both NKT cell subsets are predominantly NK1.1+ (mouse) or CD161+/CD56+ (human), their relative numbers are different in mice and humans: thus, while type I NKT cells predominate in mice, the type II NKT cell subset predominates in humans.

Type I, also known as invariant NKT cells, express a semi-invariant T cell receptor (TCR) characterized in mice by Vα14-Jα18 and Vβ8.2, Vβ7, or Vβ2 or in humans by Vα24-JαQ and Vβ11, are strongly reactive with the marine sponge-derived glycolipid alpha-galactosyl ceramide ("alpha-GalCer" or "αGalCer"), and are identified by alpha-GalCer/CDld-tetramers in flow cytometry. Type I NKT cells also recognize lipid-based antigens, such as, bacterial-derived lipids and a self-glycolipid, isoglobotrihexosyl ceramide (iGb3). Type I NKT cells display memory markers and are unique in storing preformed mRNA for cytokines. Mice lacking the Jα18 gene (Jα18 mice) are deficient only in type I NKT cells.

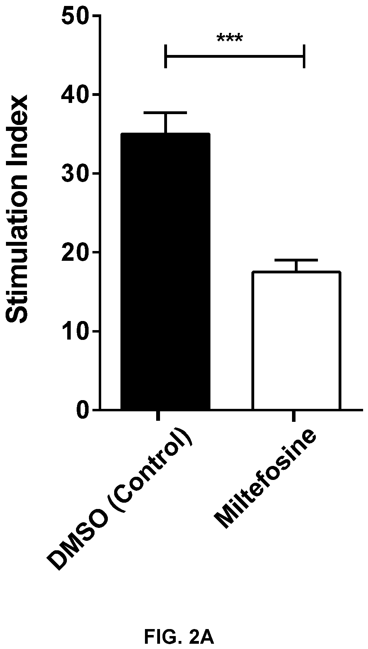

Type II NKT cells, which are distinct from type I NKT cells, are regulatory cells that can modulate the activity of several other cell subsets, including type I NKT cells. Type II NKT cells recognize the self-glycolipid, sulfatide (3'-sulfogalactosyl ceramide) in both mice and in humans. A major subset of type II NKT cells, which can be identified using sulfatide/CDld-tetramers in flow cytometry, predominantly utilize the Vβ8.1/Vβ3.1-Jβ2.7 and Vα1/Vα3-Jα7 gene segments and are reactive to sulfatides. Activation of type II NKT cells can be evaluated by assessing the *in vitro* proliferative response of type II NKT cells to a candidate agent, as well as by assessing CD69 expression and cytokine secretion profile by intracellular cytokine staining or real-time PCR for IFN-gamma, IL-4 or IL-13. In addition, the ability of activated type II NKT cells to anergize type I NKT cells can be evaluated by assessing the proliferative response of type I NKT cells to alpha-GalCer (a potent activator of type I NKT cells) using CF8E dilution analysis and intracellular cytokine staining of alpha-GalCer/CO 1 d tetramer cells. Activation of NKT cells (type I or type II) in accordance with some embodiments herein can be quantified as a stimulation index, based on thymidine incorporation (*see, e.g.* **Figure 2A**).

As used herein "NKT-2 activators", including variations of this root term refer to compounds that, upon contact with type II NKT cells, induce at least one of secretion of IL-2 by type II NKT cells, proliferation of type II NKT cells, or elevated CD69 expression on the surface of type II NKT cells. Example NKT-2 activators suitable in accordance with methods, kits, and composition herein include, but are not limited to, miltefosine, miltefosine analogs (for example the compounds listed in **Table 2.1** and **Table 2.2**), phospholipids such as a lysophophatidylcholine (LPC)(e.g., LPC (C18:0), LPC (C16:0), LPC (C18:1(9Z)), LPC(C18:1(1oleyl)), and/or LignA(LPC)(C24:0)), an analog of LPC, lyso platelet-activating factor (LPAF), a lysosphingomyelin (LSM), and/or an analog of LSM. Without being limited by any theory, it is contemplated that NKT-2 activators in accordance with some embodiments herein can thus activate type II NKT cells, which in turn can inhibit activation of type I NKT cells.

In some embodiments, activation of type II NKT cells comprises secretion of IL-2 by type II NKT cells, proliferation of type II NKT cells, elevated CD69 expression on the surface of type II NKT cells, any two of these, any three of these, or all four of these.

In some embodiments, inhibition of activation of type I NKT cells comprises a inhibition of proliferation of type I NKT cells, reduced accumulation of type I NKT cells, decreased CD69 expression on the surface of type I NKT cells, any two of these, or all three of these.

### NKT-2 activators and Type I and Type II NKT Cells following Ischemic Reperfusion Injury

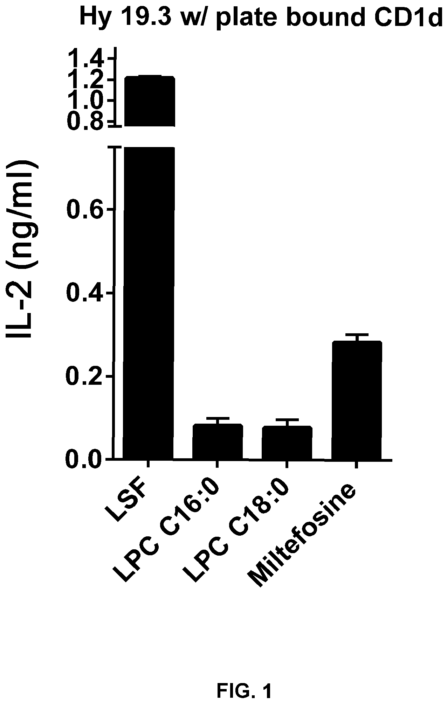

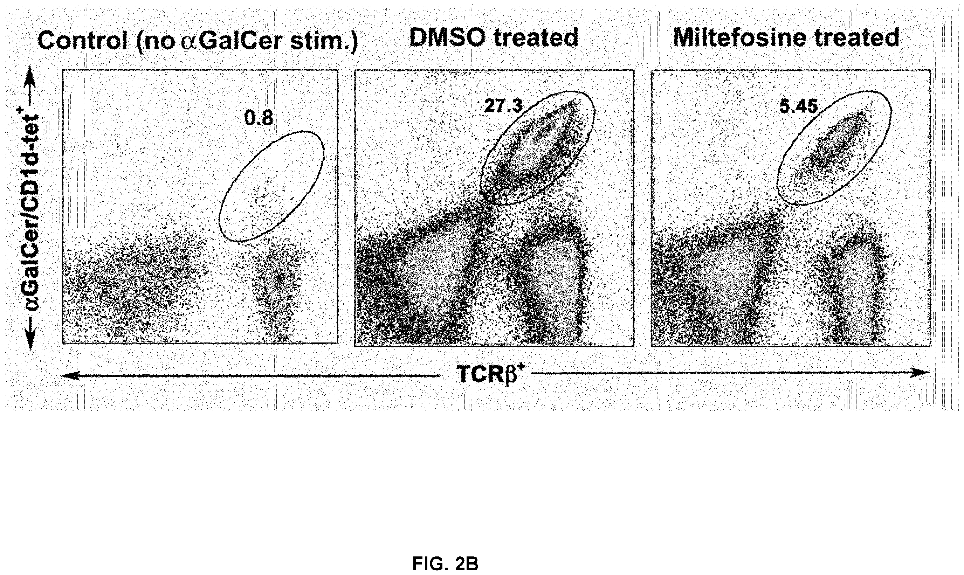

Reperfusion injury occurs when blood supply is restored to an organ or tissue after a period of ischemia. Hepatic reperfusion injury generally occurs in connection with surgery or trauma and plays a major role in the quality and function of graft tissue after liver transplant. Development of ischemic reperfusion injury (IRI) occurs in at least two phases: an initial period (following from about 1 to about 6 hours of reperfusion), which is dominated by Kupffer cell activation, release of reactive oxygen species, CD4⁺ cell recruitment and secretion of proinflammatory cytokines, and a later period (following the initial phase from about 6 to about 48 hours of reperfusion), which is characterized by neutrophil accumulation and induction of necrosis. Typically, Type I NKT cells also become activated and secrete IFN-gamma following ischemic reperfusion. As it has been shown that administration of NKT-2 activators such as miltefosine can stimulate type II NKT cells (*see* **Figure 1**) and inhibit type I NKT cell expansion (*see* **Figure 2A** and **Figure 2B**), it is contemplated that NKT-2 activators such as miltefosine and analogs of miltefosine and phospholipids in accordance with some embodiments herein can be useful for inhibiting Type I NKT activation associated with ischemic reperfusion.

Accordingly, in some embodiments, a composition comprising an amount of NKT-2 activator, for example miltefosine or a miltefosine analog, or a phospholipid sufficient to activate type II NKT cells is provided for use in treating, ameliorating, preventing, or reducing the risk of developing ischemic reperfusion injury, the latter two examples forming part of the present disclosure. Optionally, the composition further comprises an amount of RAR agonist, for example tazarotene sufficient to inhibit type I NKT cells. Optionally, the composition further comprises a sulfatide in an amount sufficient to activate type II NKT cells. In some embodiments, the composition is administered to a subject suffering from, or at risk for developing ischemic reperfusion injury.

In some aspects of the disclosure, a composition comprising an amount of NKT-2 activator (for example miltefosine or a miltefosine analog or a phospholipid) and an amount of sulfatide that together are sufficient to activate type II NKT cells is provided for use in treating, amerliorating, preventing, or reducing the risk of developing ischemic reperfusion injury. Optionally, the composition further comprises an amount of RAR agonist (for example, tazarotene) sufficient to inhibit type I NKT cells. In some embodiments, the composition is administered to a subject suffering from, or at risk for developing ischemic reperfusion injury. Optionally, the composition is for oral administration.

In some aspects of the disclosure, an amount of NKT-2 activator (for example miltefosine or a miltefosine analog or a phospholipid) sufficient to activate type II NKT cells is administered to a subject suffering from, or at risk for IRI. Optionally, an amount of RAR agonist (for example, tazarotene) sufficient to inhibit activation of type I NKT cells is administered to the subject. Optionally, the administration is oral. Optionally, an amount of sulfatide sufficient to activate type II NKT cells is further administered to the subject. In some aspects of the disclosure, the NKT-2 activator and RAR agonist are administered simultaneously. In some aspects of the disclosure, the NKT-2 activator and RAR agonist are administered separately. In some aspects of the disclosure, the NKT-2 activator is administered first and the RAR agonist is administered subsequently. In some aspects of the disclosure, the RAR agonist is administered first and the NKT-2 activator is administered subsequently. In some aspects of the disclosure, alternating administrations of RAR agonist and NKT-2 activator are performed.

### NKT-2 activators and activated Type I and Type II NKT Cells in Liver Disease

As described in U.S. Patent No. 8,044,029 and U.S. Pub. No. 2011/0118197, and US Pub. No. 2014/0187504, administration of a self-glycolipid ligand, sulfatide, as well as synthetic sulfatide analogs can modulate the activity of type I and type II NKT cells. Without being limited by any theory, CDld-dependent recognition of sulfatide activates type II NKT cells and predominantly plasmacytoid dendritic cells (pDC), but not conventional dendritic cell (cDC) populations (which are normally activated by type 1 NKT cells), leading to a rapid recruitment of type I NKT cells into liver in an IL-12 and MIP2-dependent fashion. However, the recruited type I NKT cells are not activated, do not secrete cytokines and become anergic. Administration of sulfatide *in vivo* has been shown to a) suppress in vitro proliferation of type I NKT cells in response to stimulation by alpha-GalCer, and b) increase the percentage of sulfatide/CDld-tetramer+ cells with intracytoplasmic IFN-gamma staining.

It has been observed that secretion of IFN-gamma by hepatic type I NKT cells during the early phase of ischemia and reperfusion injury is significantly decreased in mice receiving sulfatide compared to untreated animals. Further, as with the absence of type I NKT cells in Jα18^{-/-} mice, liver injury following ischemia/reperfusion is significantly reduced in mice treated with sulfatide. This observation indicates a pathogenic role for type I NKT cells in mediating hepatic ischemia and reperfusion injury. Without being limited by any theory, the role of IFN-gamma secretion by type I NKT cells may be a common feature of ischemic organ injury, as kidney ischemia and reperfusion injury is also attenuated in Jα18^{-/-} mice, which lack type 1 NKT

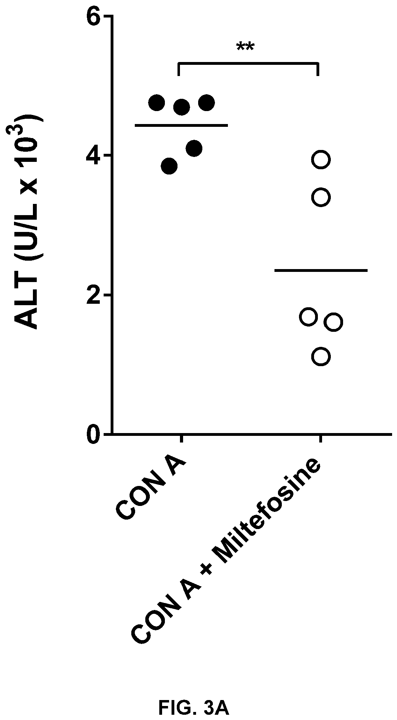

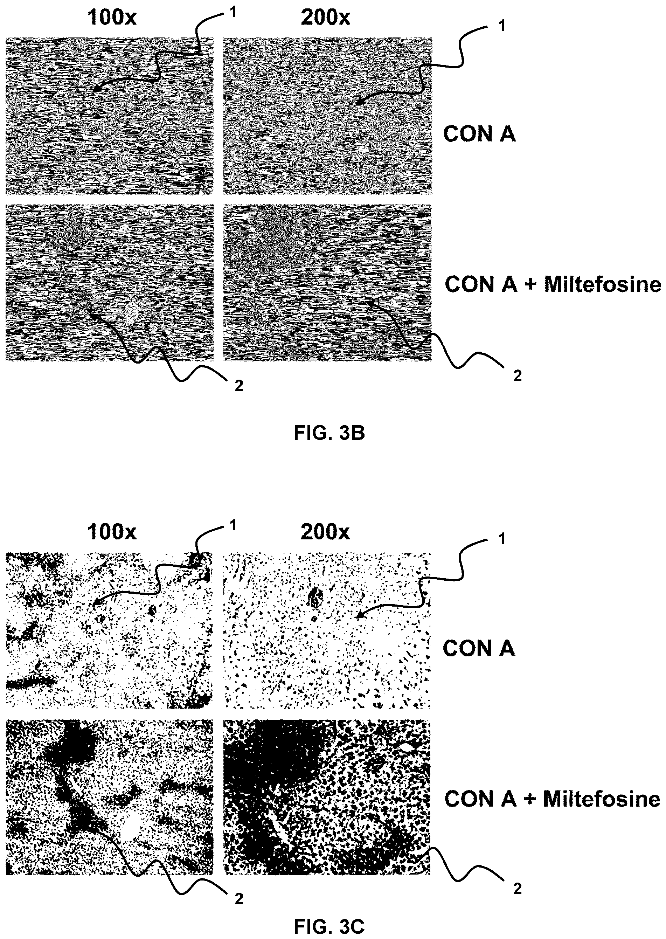

Similarly to IRI, administration of sulfatide or NKT-2 activator (for example miltefosine or a miltefosine analog or a phospholipid) can protect against Concanavalin A (ConA)-induced hepatitis as indicated by reduced hepatocellular necrosis, and reduced serum levels of alanine aminotransferase (ALT) and aspartate amino transferase (AST), markers of hepatocellular damage. Moreover, administration of miltefosine 12 hours before ConA reduced serum levels of ALT 24 hours later (**Figure 3A**), and reduced necrosis of cells as determined by H&E staining of liver sections (**Figure 3B**). Without being limited by any theory, these observations indicate a pathogenic role for type I NKT cells in ConA-induced hepatitis, and a protective role of sulfatide and/or phosopholipid (for example miltefosine or a miltefosine analog).

Without being limited by any theory, data from both models of liver damage, IRI and ConA-induced hepatitis, are consistent with an immunoregulatory pathway in which type I NKT cells play a detrimental role and sulfatide/NKT-2 activator -reactive type II NKT cells play a protective role in hepatic inflammation arising from diverse causes. Such protection is associated with the inhibition of cytokine secretion by type I NKT cells (inhibited phenotype) and a significant reduction in hepatic recruitment of immature myeloid cells (CD11b+Gr-1+) CD11b+Gr-1- and NK cells. Inhibition in type I NKT cells is also associated with the tolerization or modification of conventional dendritic cells (cDCs) and they together with inhibited type I NKT cells inhibit activation/expansion of adaptive Thl/Thl7 CD4+/CD8+ T cells. This leads to a model where administration of sulfatide and/or NKT-2 activator (for example miltefosine or an analog of miltefosine or a phospholipid) stimulates the activity of type II NKT cells, which in turn inhibit type I NKT cell activity and the liver damage caused by type INKT cell-mediated inflammation.

Accordingly, in some embodiments, a composition comprising an amount of NKT-2 activator (for example miltefosine or a miltefosine analog or a phospholipid, the latter two forming part of the present disclosure) sufficient to activate type II NKT cells is provided for use in treatment of liver injury or liver disease. Optionally, the composition further comprises an amount of RAR agonist (for example, tazarotene) sufficient to inhibit activation of type I NKT cells. Optionally, the composition further comprises an amount of sulfatide sufficient to activate type II NKT cells. In some embodiments, the composition is administered to a subject suffering from liver injury or at risk of developing a liver injury or liver disease, for example fatty liver disease, alcohol induced hepatitis, non-alcoholic steatosis hepatitis, non-alcoholic fatty liver disease, fibrosis, primary sclerosing cholangitis, primary biliary sclerosis, cirrhosis, fulminating cirrhosis, idiopathic hepatitis, viral-induced hepatitis (A, B, C and other), inflammatory hepatitis associated with hepato-biliary carcinoma.

In some aspects of the disclosure, a composition comprising an amount of NKT-2 activator (for example miltefosine or a miltefosine analog or a phospholipid) and an amount of sulfatide that together are sufficient to activate type II NKT cells is provided for use in treating, ameliorating, preventing, or reducing the risk of developing liver injury or liver disease. Optionally, the composition further comprises an amount of RAR agonist (for example, tazarotene) sufficient to inhibit activation of type I NKT cells. In some aspect of the disclosure, the composition is administered to a subject suffering from, or at risk for developing a liver injury or liver disease, for example fatty liver disease, alcohol induced hepatitis, non-alcoholic steatosis hepatitis, non-alcoholic fatty liver disease, fibrosis, primary sclerosing cholangitis, primary biliary sclerosis, cirrhosis, fulminating cirrhosis, idiopathic hepatitis, viral-induced hepatitis (A, B, C and other), inflammatory hepatitis associated with hepato-biliary carcinoma.

In some aspects of the disclsoure, an amount of NKT-2 activator (for example miltefosine or a miltefosine analog or a phospholipid) sufficient to activate type II NKT cells is administered to a subject suffering from, or at risk for liver injury or liver disease. Optionally, an amount of RAR agonist (for example, tazarotene) sufficient to inhibit activation of type I NKT cells is administered to the subject. Optionally, the administration is oral. Optionally, an amount of sulfatide sufficient to activate type II NKT cells is further administered to the subject. In some aspects of the disclosure, the NKT-2 activator and RAR agonist are administered simultaneously. In some aspects of the disclosure, the NKT-2 activator and RAR agonist are administered separately. In some aspects of the disclosure, the NKT-2 activator is administered first and the RAR agonist is administered subsequently. In some aspects of the disclosure, the RAR agonist is administered first and the NKT-2 activator is administered subsequently. In some aspects of the disclosure, alternating administrations of RAR agonist and NKT-2 activator are performed. In some aspects of the disclosure, the subject suffers from, or is at risk for developing a liver injury or liver disease, for example fatty liver disease, alcohol induced hepatitis, non-alcoholic steatosis hepatitis, non-alcoholic fatty liver disease, fibrosis, primary sclerosing cholangitis, primary biliary sclerosis, cirrhosis, fulminating cirrhosis, idiopathic hepatitis, viral-induced hepatitis (A, B, C and other), inflammatory hepatitis associated with hepato-biliary carcinoma.

### NKT cells in Alcoholic Liver Disease

Alcoholic Liver Disease (ALD) develops as a result of damage to liver cells caused by excess alcohol consumption. Multiple insults may be required before clinical manifestations of ALD are observed. However, it has been observed that significantly increased numbers of type I NKT cells (alpha-GalCer/CDld-tetramer+ cells), CD4+ cells, and CD11b+Gr-1+ myeloid cells, but not type II NKT cells or CD11b+Gr-1- cells, are observed in the liver following chronic alcohol consumption. Enhanced expression of CD69 marker further indicates that type I NKT cells are at least partially activated. Thus, even in the absence of any clinical signs of disease (preclinical phase) cells mediating an inflammatory response accumulate in the liver following excess alcohol consumption. In the absence of type I NKT cells (Jα18-/- mice), accumulation of CD11b+Gr-1+ myeloid cells in the liver following chronic alcohol consumption is significantly reduced. Without being limited by any theory, these data suggest that type I NKT cells are involved in mediating the preclinical phase of ALD.

Consistent with the data suggesting a role for type I NKT cells in the preclinical phase of ALD, clinical manifestations of liver damage following excessive alcohol consumption are significantly reduced, as measured, for example, by histology and liver enzyme analysis, in type I NKT cell deficient (Jα18-/-) mice. Without being limited by any theory, these data suggest that type I NKT cells play an important role in alcoholic damage to the liver.

NKT cells express T cell receptors, which enable them to recognize lipid antigens in the context of CDld molecules. Not wishing to be bound by a particular theory, type I NKT cells are activated in the liver following ethanol consumption by local presentation of oxidized self-lipids by CDld-expressing antigen presenting cells (APCs). Activated type I NKT cells then initiate a multistep process resulting in Kupffer cell activation, recruitment/activation of granulocytes followed by inflammatory damage to hepatocytes.

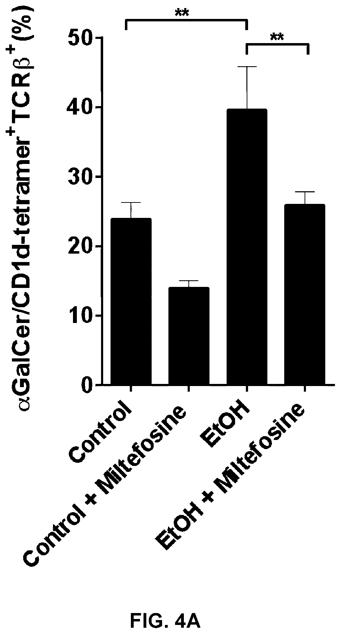

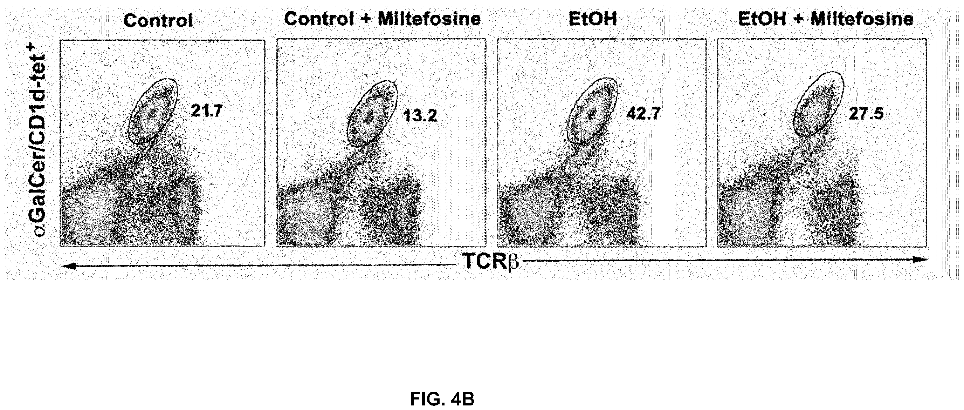

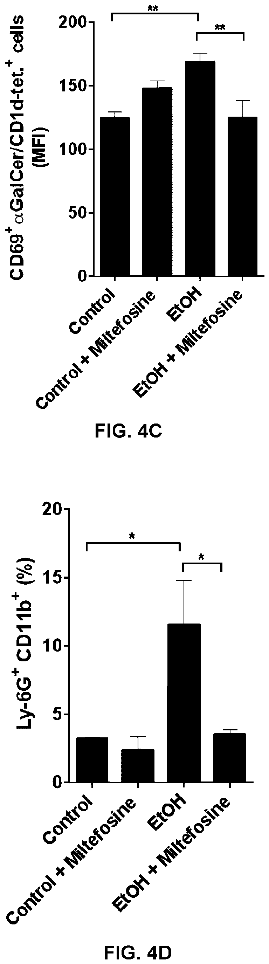

As shown in **Figure 4A** and **Figure 4B** administration of miltefosine in conjunction with ethanol in an murine model of alcoholic liver disease inhibited accumulation of type I NKT cells, as compared administration of just ethanol. Moreover, as shown in **Figure 4C****,** administration of miltefosine in conjunction with ethanol also reduced levels of activated (CD69⁺) NKT cells as compared with administration of ethanol alone. Moreover, as shown in **Figure 4D****,** administration of miltefosine in conjunction with ethanol also reduced levels of neutrophils as compared with administration of ethanol alone.

The interplay among NKT cell subtypes following alcohol consumption sets the stage for liver damage and in severe cases, alcoholic liver disease (ALD). Several embodiments described herein relate to methods and compositions for manipulating the opposing roles of type I NKT cells and type II NKT cells and their interactions with other liver cells in order to treat, alleviate or prevent injury to the liver following alcohol consumption.

Sulfatide treatment results in almost complete protection from liver damage following excess alcohol consumption. Not wishing to be bound by a particular theory, the protective effect of sulfatide can be mediated through the direct activation of Type II NKT cells, which exert an inhibitory effect on type I NKT cells and cDCs.

Accordingly, some embodiments herein relate to modulating the activity of NKT cell subtypes in order to treat, ameliorate, and/or prevent alcohol-induced injury to the liver. As described herein, administration of NKT-2 activators (for example miltefosine), sulfatides or retinoic acid receptor (RAR) agonists inhibits alcohol-induced liver damage. For example, oral administration of miltefosine reduces type I NKT cell accumulation and activation, and neutrophil accumulation induced by ethanol (*see* **Figures 4A****,** **4B****, C, and 4D).**

Several aspects of the disclosure relate to a protective role in alcohol induced liver injury for a major subset of type II NKT cells, which are reactive to sulfatide. Activation of this NKT cell subset by sulfatide inhibits type I NKT cell-mediated injury following excess alcohol consumption. Sulfatide-mediated protection is associated with activation of type II NKT cells and inhibition of IFN-gamma secretion by hepatic type I NKT cells and suppression of type I NKT cell-mediated recruitment of myeloid cells, for example, the CD11b⁺Gr-1^{int} and Gr-1⁻ subsets, and NK cells into the liver.

Accordingly, in some embodiments, a composition comprising an amount of NKT-2 activators (for example miltefosine or a miltefosine analog, the latter forming part of the present disclosure) sufficient to activate type II NKT cells is provided for use in treating, ameliorating, or preventing alcoholic liver disease. Optionally, the compound further comprises an amount of RAR agonist (for example, tazarotene) sufficient to inhibit activation of type I NKT cells. Optionally, the composition is for oral administration.

In some aspects of the disclosure, a composition comprising an amount of NKT-2 activators (for example miltefosine or a miltefosine analog) and an amount of sulfatide that together are sufficient to activate type II NKT cells is provided for use in treating, ameliorating, preventing, or reducing the risk of alchohlic liver disease. Optionally, the composition further comprises an amount of RAR agonist (for example, tazarotene) sufficient to inhibit activation of type I NKT cells. Optionally, the composition is for oral administration.

In some aspects of the disclosure, an amount of NKT-2 activator (for example miltefosine or a miltefosine analog) sufficient to activate type II NKT cells is administered to a subject suffering from, or at risk for developing alcoholic liver disease. Optionally, an amount of RAR agonist (for example, tazarotene) sufficient to inhibit activation of type I NKT cells is administered to the subject. Optionally, the administration is oral. Optionally, an amount of sulfatide sufficient to activate type II NKT cells is further administered to the subject. In some aspects of the disclosure, the NKT-2 activator and RAR agonist are administered simultaneously. In some aspects of the disclosure, the NKT-2 activator and RAR agonist are administered separately. In some aspects of the disclosure, the NKT-2 activator is administered first and the RAR agonist is administered subsequently. In some aspects of the disclosure, the RAR agonist is administered first and the NKT-2 activator is administered subsequently. In some aspects of the disclosure, alternating administrations of RAR agonist and NKT-2 activator are performed.

### Sulfatides

In some embodiments, the sulfatide comprises, for example, bovine brain-derived sulfatide, which is a mixture of about 20 different species obtained from Sigma Inc. (Chicago, IL, USA). In other embodiments, the sulfatide is semisynthetic and comprises a single species of sulfatide, for example, cis-tetracosenoyl sulfatide or lysosulfatide obtained from Maitreya Inc, (Pleasant Gap, PA, USA). In still other embodiments, the sulfatide can be a totally synthetic sulfatide.

Sulfatide derived from bovine brain myelin is comprised of a 2:1 mixture of saturated/unsaturated major acyl chains (C₂₄), with unsaturation occurring at C₁₉. Several embodiments relate to the use of synthetic sulfatide analogs, such as analogs with saturated acyl chain (C₂₄) and unsaturated chains (C₂₄: 1) as well as analogs comprised of different lengths of acyl chains in the fatty acid or sphingosine moiety (shorter as well as longer, for example, C₁₈, C₃₂) and positional isomers with 3' vs. 4'-sulfated group on the galactose moiety (3'-803 vs. 4'-803).

In some embodiments the sulfatide has the following chemical formula I: 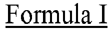 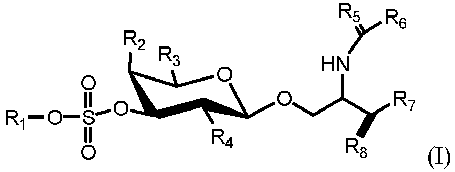 wherein R₁ can be a bond, a hydrogen, a C₁ to C₃₀ alkyl, a C₁ to C₃₀ substituted alkyl, a C₁ to C₃₀ alkenyl, a C₁ to C₃₀ substituted alkenyl or a C₅ to C₁₂ sugar; R₂ can be a hydrogen, a hydroxy group, a methoxy group, or an alkoxy group; R₃ can be a hydrogen, a hydroxy group, a methoxy group, an ethoxy group, or an alkoxy group; R₄ can be a hydrogen, a hydroxy group or an alkoxy group; R₅ can be a hydrogen, a hydroxy group, a carbonyl, an alkoxy group or a bond; R₆ can be a C₁ to C₄₀ alkyl, a C₁ to C₄₀ substituted alkyl, a C₁ to C₄₀ alkenyl, a C₁ to C₄₀ substituted alkenyl, or a C₁ to C₄₀ alkynl; R7 can be a C₁ to C₄₀ alkyl, a C₁ to C₄₀ substituted alkyl, a C₁ to C₄₀ alkenyl, a C₁ to C₄₀ substituted alkenyl, or a C₁ to C₄₀ alkynl; and R₈ can be a hydrogen, a hydroxy group, a carbonyl, an alkoxy group or a bond.

In other embodiments, the sulfatide has the following chemical formula II: 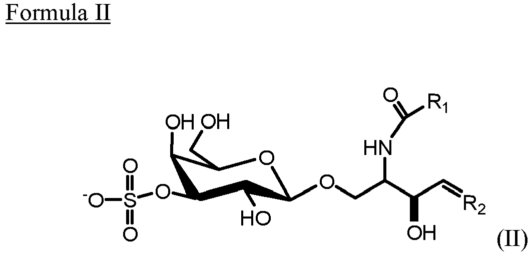 wherein R₁ is selected from the group consisting of a C₁ to C₄₀ alkyl, a C₁ to C₄₀ substituted alkyl, a C₁ to C₄₀ alkenyl, a C₁ to C₄₀ substituted alkenyl and a C₁ to C₄₀ alkynl; and R₂ is selected from the group consisting of a hydrogen, a hydroxyl group, a carbonyl, an alkoxy group and a bond.

In another embodiment, the sulfatide has the following chemical structure:

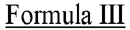

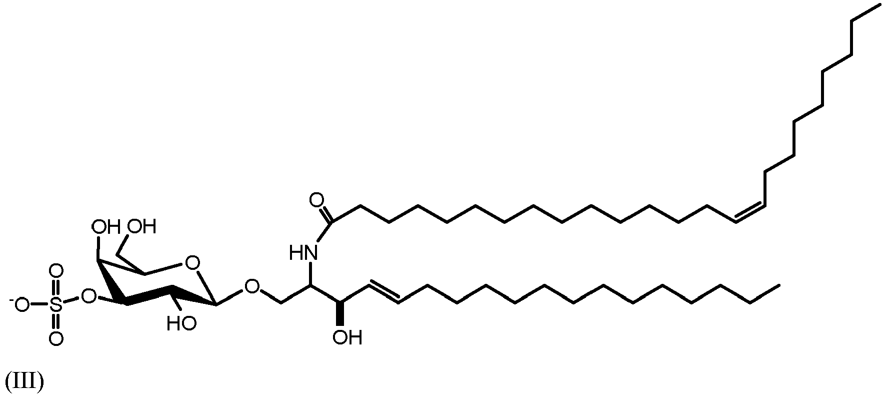

As used herein, the term "alkyl" means any unbranched or branched, saturated hydrocarbon. The term "substituted alkyl" means any unbranched or branched, substituted saturated hydrocarbon. Cyclic compounds, both cyclic hydrocarbons and cyclic compounds having heteroatoms, are within the meaning of "alkyl."

As used herein, the term "substituted" means any substitution of a hydrogen atom with a functional group.

As used herein, the term "functional group" has its common definition, and refers to chemical moieties preferably selected from the group consisting of a halogen atom, C₁-C₂₀ alkyl, substituted C₁-C₂₀ alkyl, perhalogenated alkyl, cyloalkyl, substituted cycloalkyl, aryl, substituted aryl, benzyl, heteroaryl, substituted heteroaryl, cyano, and nitro.

As used herein, the terms "halogen" and "halogen atom" refer to any one of the radio-stable atoms of column 17 of the Periodic Table of the Elements, preferably fluorine, chlorine, bromine, or iodine, with fluorine and chlorine being particularly preferred.

As used herein, the term "alkenyl" means any unbranched or branched, substituted or unsubstituted, unsaturated hydrocarbon. The term "substituted alkenyl" means any unbranched or branched, substituted unsaturated hydrocarbon, substituted with one or more functional groups, with unbranched C₂-C₆ alkenyl secondary amines, substituted C₂-C₆ secondary alkenyl amines, and unbranched C₂-C₆ alkenyl tertiary amines being within the definition of "substituted alkyl." Cyclic compounds, both unsaturated cyclic hydrocarbons and cyclic compounds having heteroatoms, are within the meaning of "alkenyl."

As used herein, the term "alkoxy" refers to any unbranched, or branched, substituted or unsubstituted, saturated or unsaturated ether.

As used herein, the term "sulfatide" retains its general accustomed meaning and refers to a cerebroside sulfuric ester containing one or more sulfate groups in the sugar portion of the molecule.

As used herein, the term "cerebroside" refers to any lipid compound containing a sugar, and generally of the type normally found in the brain and nerve tissue.

The compounds of formula (I), (II) and (III) may be in the form of pharmaceutically acceptable nontoxic salts thereof. Salts of formula (I), (II) and (III) include acid added salts, such as salts with inorganic acids (e.g., hydrochloric acid, sulfuric acid, nitric acid and phosphoric acid) or with organic acids (e.g., acetic acid, propionic acid, maleic acid, oleic acid, palmitic acid, citric acid, succinic acid, tartaric acid, fumaric acid, glutamic acid, pantothenic acid, laurylsulfonic acid, methanesulfonic acid and phthalic acid).

The compounds of formula (I), (II) and (III) may be in the form of solvates thereof (e.g., hydrates).

The compounds of formula (I), (II) and (III) can be produced by any purposive method to synthesize sulfatides.

The compounds of formulas (I), (II) and (III) can also be isolated from natural products (e.g., biological organisms) and purified by column chromatography or the like.

In one embodiment, the sulfatide has the chemical formula: (2S, 3R, 4E)-N-nervonic-1-[-D-(3-sulfate)-galactopyranosyl]-2-amino-octadecene-3-ol. This chemical formula is also referred to as cis-tetracosenoyl sulfatide.

In some embodiments, the specific amount of sulfatide administered to a patient will vary depending upon the disease or condition being treated, as well as the age, weight and sex of the patient being treated. Generally, to achieve such a final concentration in, e.g., the intestines or blood, the amount of sulfatide molecule in a single dosage composition of the present embodiments will generally be about 0.1 milligrams to about 100 milligrams, preferably about 2.0 milligrams to about 60 milligrams, more preferably about 20 milligrams to about 50 milligrams. Likewise, the amount of a secondary therapeutic compound in a single oral dosage composition of the present embodiments will generally be in the range of about 0.01 milligrams to about 1000 milligrams, more preferably about 0.1 milligrams to about 100 milligrams. Obviously, the exact dosage will vary with the disease or disorder being treated, the preferred ranges being readily determinable. In some embodiments, the patient is a human.

In some embodiments, 0.1-10 mg/kg body weight of sulfatide are administered to the subject, for example about 0.1 mg/kg body weight, 0.2 mg/kg body weight, 0.3 mg/kg body weight, 0.5 mg/kg body weight, 1 mg/kg body weight, 2 mg/kg body weight, 3 mg/kg body weight, 5 mg/kg body weight, 9 mg/kg body weight, or ranges such as

0.2 - 0.5 mg/kg body weight, 0.2 - 1 mg/kg body weight, 0.2 - 5 mg/kg body weight, 0.2 - 10 mg/kg body weight, 0.5 - 1 mg/kg body weight, 0.5 - 5 mg/kg body weight, 0.5 - 10 mg/kg body weight, 1 - 5 mg/kg body weight, 1 - 10 mg/kg body weight, 2 -5 mg/kg body weight, 2 - 10 mg/kg body weight, or 5 - 10 mg/kg body weight. Preferably, this dosage is repeated as needed. Optionally, the dosage can be repeated Optionally, the dosage can be repeated five times daily, four times daily, three times daily, twice daily, once daily, or less frequently, for example once every 2, 3, 4, 5, 6, 7, 8, 9, 10, 11, 12, 13, 14, 15, 16, 17, 18, 19, or 20 days, including ranges between any two of the listed values, for example every 1-2 days, every 1-3 days, every 1-5 days, every 1-10 days, every 1-20 days, every 2-3 days, every 3-5 days, every 3-10 days, every 3-20 days, every 5-10 days, or every 5-20 days.

### Retinoic Acid Receptor (RAR) Agonists

As described herein, administration of the Retinoic Acid Receptor (RAR) agonist, all trans retinoic acid (ATRA), significantly inhibits liver damage caused by alcohol consumption. Ethanol ingestion depletes liver retinyl esters and alters physiological levels of all trans retinoic acid (ATRA), a biologically active from of vitamin A that supports many biological functions and can enhance generation, stability and function of naive CD4+ T cell differentiation into FoxP3+ Tregs even in the presence of IL-6. Several embodiments described herein relate to the finding that ATRA inhibits type I NKT cell effector function, including suppressing cytokine secretion by these cells. Further, ATRA has a direct inhibitory effect on type I NKT cell activity, exerting its inhibitory effect in the absence of antigen-presenting cells. In addition, ATRA does not directly inhibit the activity of conventional MHC-restricted CD4+ T cells that recognize protein antigens, such as myelic basic protein or MBPAcl-9. Not wishing to be bound by a particular theory, these data indicate that ATRA protects against liver damage caused by excessive alcohol consumption by inhibiting type I NKT cells.

As described herein, RAR agonists induce inhibition in type I NKT cells. Further, liver damage caused by type I NKT cell mediated inflammation resulting from excess alcohol consumption can be prevented, reduced or mitigated by administration of an RAR agonist. The liver can become inflamed for a variety of different reasons. For example, liver inflammation can be caused by bacterial or viral infection, injury, or attack from one's own immune system. While inflammation is normally a protective response and a required step of the healing process, prolonged or chronic inflammation can cause injury. Several embodiments described herein relate to the RAR agonist mediated modulation of the innate immune mechanisms leading to liver injury following, related to or caused by inflammation. Some embodiments relate to methods and compositions for RAR agonist mediated inhibition of type I NKT cell activity which modulate interactions among the components of the innate immune system to provide tolerance to gut-derived or metabolite-derived antigens without affecting or minimally affecting the innate immune response to non-self identified pathogens.

As RAR agonists can directly anergize Type I NKT cells, RAR agonists may be used to treat any indication in which Type I NKT cells play a pathogenic role. Some examples of diseases which can be treated by the embodiments of the present disclosure include, alcohol induced hepatitis, non-alcoholic steatosis hepatitis, cirrhosis, non-alcoholic fatty liver disease, fibrosis, primary sclerosing cholangitis, primary biliary sclerosis, fulminating cirrhosis, idiopathic hepatitis, viral-induced hepatitis (A, B, C and other), inflammatory hepatitis associated with hepato-biliary carcinoma, multiple sclerosis, type 1 diabetes, ischemic reperfusion injury, solid organ transplantation, systemic lupus erythematosus, rheumatoid arthritis, amyotrophic lateral sclerosis, and inflammatory bowel disease (Crohn's and colitis).

In some aspects of the disclosure, are various indications of autoimmune or immune related diseases or disorders are treated, prevented or mitigated by RAR agonist mediated inhibition of type I NKT cell activity. In particular, one aspect of the present disclosure is related to a method of treating a patient suffering from symptoms of an autoimmune or immune related disease or disorder, such as, for example, multiple sclerosis, systemic lupus erythematosus, AIDS, Alzheimer's disease, rheumatoid arthritis, insulin dependent diabetes mellitus, autoimmune hepatitis, asthma, celiac disease, primary sclerosing cholangitis, primary biliary sclerosis, with an effective amount of an RAR agonist. In some aspects of the disclosure, RAR agonist mediated inhibition of type I NKT cell activity treats, prevents or mitigates asthma symptoms. In some aspects of the disclosure, the patient is a human.

Some aspects of the disclosure relate a method of inhibiting or preventing type I NKT cell mediated inflammation following ischemic reperfusion by administering an RAR agonist. Type I NKT cells play a pathogenic role in conditions such as ischemia and reperfusion injury. Reperfusion injury can occur in a variety of tissues when blood supply is restored after a period of ischemia. Examples include skeletal muscle tissue following a crush injury, cardiac muscle in connection with a myocardial infarction or cardiac surgery, or ischemic heart disease, neural tissue in connection with a stroke or brain trauma, and hepatic and renal tissue in connection with surgery or trauma. Ischemic reperfusion injury also plays a major role in the quality and function of graft tissue in organ transplant. Ischemia and reperfusion injury is a major cause for increased length of hospitalization and decreased long-term graft survival. In some embodiments, RAR agonist mediated inhibition of type I NKT cell activity inhibits or prevents hepatic ischemic reperfusion injury in connection with surgery or trauma.

Aspects of the disclosure described herein relate to the inhibition of type I NKT cell activity by one or more retinoic acid receptor (RAR) agonists. Retinoic acid receptors comprise three major subtypes: RARα, RARβ, and RARγ. Some aspects of the disclosure relate to the inhibition of type I NKT cell activity by one or more pan-active RAR agonists, precursors of such pan-active RAR agonists and mixtures thereof. As used herein, the term "pan-active RAR agonist" refers to a RAR agonist which affects, for example, activates, RARα, RARβ, and RARγ substantially equally or non-selectively. Some aspects of the disclosure relate to the inhibition of type I NKT cell activity by one or more active RAR agonists effective to selectively, or even specifically, affect, for example, activate, at least one, and preferably both, of RARβ and RARγ relative to RARα, precursors of such active RAR agonists and mixtures thereof. As used in this context, the term "selectively" means that the RAR agonist precursors of the RAR agonist and mixtures thereof are more effective, preferably at least about 10 or about 100 times to about 1000 times or more as effective, to affect at least one, and preferably both, of RAR β and RARγ relative to RARα. Some aspects of the disclosure relate to the inhibition of type I NKT cell activity by one or more subtype-selective RAR agonists, precursors of such subtype-selective RAR agonists and mixtures thereof. As used herein, the term "subtype-selective RAR agonist" refers to a RAR agonist which selectively affects, for example, activates one RAR subtype. Retinoid compounds having RARα, RAR B, and RARy-selectivity are known in the art and disclosed, for example, in U.S. Pat. Nos. 6,534,544 and 6,025,388.

Several aspects of the disclosure relate to a method of inhibiting pro-inflammatory type I NKT cell activity by administering one or more retinoic acid receptor (RAR) agonists. Examples of RAR agonists include, but are not limited to, the RAR agonists listed in Table 1.

**Table 1: RAR agonists**

| Compound Name | Specificity | Structure |
|---|---|---|
| Tretinoin | Pan-RAR agonist | 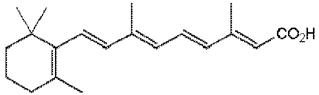 |
| 9-cis RA | Pan-RAR and RXR agonist | 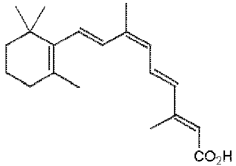 |
| 13-cis-RA | Pan-RAR agonist | 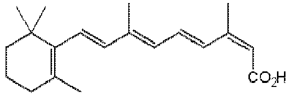 |
| Fenretinide | RAR agonist RAR - independent effects | 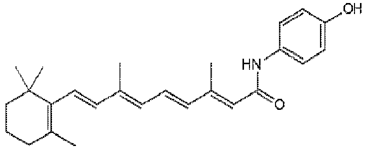 |
| EC 23 | Pan-RAR agonist | 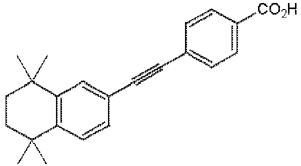 |
| TTNPB | Pan-RAR agonist | 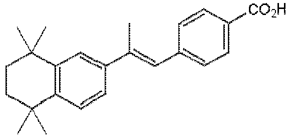 |
| Ch 55 | Pan-RAR agonist | 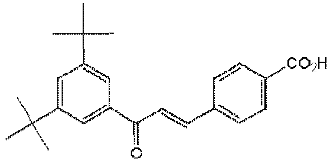 |
| Tazarotene | RARβ/γ agonist | 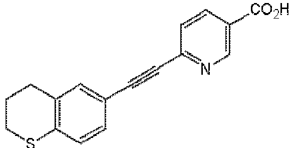 |
| BMS 753 | RARα agonist | 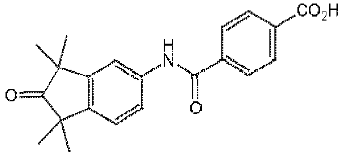 |
| AM80 | RARα agonist | 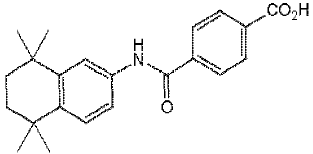 |
| AM580 | RARα agonist | 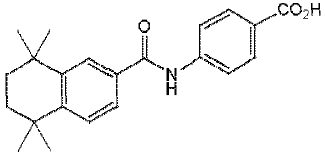 |
| AC55649 | RARβ2 agonist | 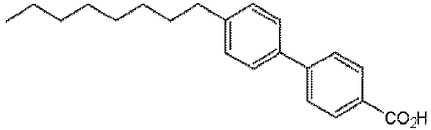 |
| AC261066 | RARβ2 agonist | 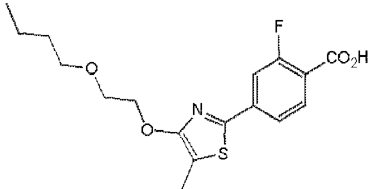 |
| Adapalene | RARβ and γ agonist | 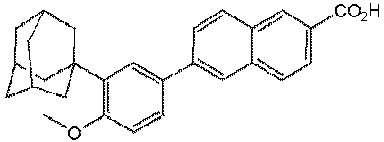 |
| CD437 | RARγ agonist | 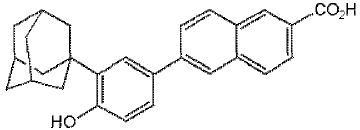 |
| CD1530 | RARγ agonist | 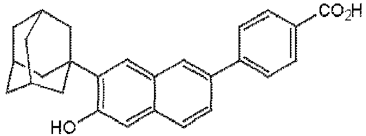 |
| CD2665 | RARγ agonist | 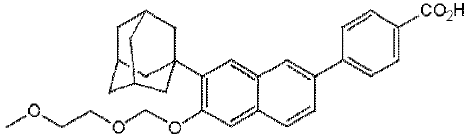 |
| MM11253 | RAR agonist | 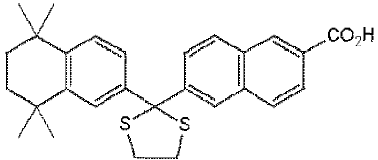 |
| LE135 | RARβ agonist | 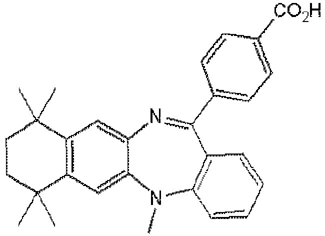 |
| BMS493 | Pan-RAR inverse agonist | 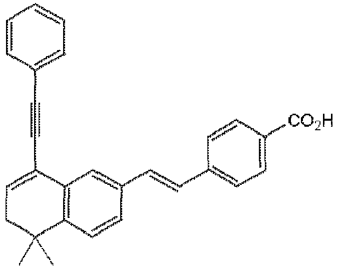 |
| BMS453 | RARβ agonist RARα or RARγ Antagonist | 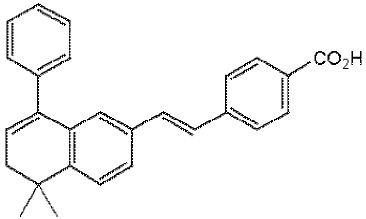 |

In some aspects of the disclosure, pro-inflammatory type I NKT cell activity is inhibited by one or more RAR agonists selected from the group consisting of ATRA, retinol, 9-cis-RA or 13-cis-RA, tretinoin, AM580, AC55649, CD1530 or Tazarotene. In some embodiments, pro-inflammatory type I NKT cell activity is inhibited by one or more polyolefinic retinoids, such as isoretinoin and acitretin. In some embodiments, pro-inflammatory type I NKT cell activity is inhibited by one or more RAR agonists selected from the group consisting of etretinate, acitretin and isotretinoin.

Several aspects of the disclosure relate to the inhibition of pro-inflammatory type I NKT cell activity by tazarotene, tazarotenic acid or a mixture thereof. Tazarotene is an ethyl ester prodrug that is metabolized to the corresponding free acid, tazarotenic acid. Tazarotene has a rigid ring-locked structure that offers limited conformational flexibility compared to all-trans-retinoic acid, the natural ligand for the retinoic acid receptors (RARs). This structural change confers tazarotenic acid with specificity for the RARs and selectivity for RARβ and RARγ.

Examples of RAR agonists further include esters of cis- and trans- retinoic acids, for example, alkyl esters, such as primary, secondary or tertiary alcohols, including but not limited to: methyl, ethyl, propyl, iso-propyl, butyl, iso-butyl, hexyl, heptyl, ethylhexyl, octyl, nonyl, lauryl, oleyl, stearyl, hydroxyethyl, hydroxypropyl, benzyl, alpha-methylbenzyl, alpha-propylphenyl, amyl, iso-amyl, anisyl, cetyl, menthyl, cinnamyl, pinacol, furyl, or myristyl.

Pharmaceutically acceptable salts of RAR agonists can also be used to inhibit type I NKT cell activity. Compounds disclosed herein which possess a sufficiently acidic, a sufficiently basic, or both functional groups, and accordingly can react with any of a number of organic or inorganic bases, and inorganic and organic acids, to form a salt.

Examples of acids that may be used to form acid addition salts from RAR agonists with basic groups include inorganic acids such as hydrochloric acid, hydrobromic acid, hydroiodic acid, sulfuric acid, phosphoric acid, and the like, and organic acids such as p-toluenesulfonic acid, methanesulfonic acid, oxalic acid, p-bromophenyl-sulfonic acid, carbonic acid, succinic acid, citric acid, benzoic acid, acetic acid, and the like. Examples of such salts include the sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, gamma-hydroxybutyrate, glycolate, tartrate, methanesulfonate, propanesulfonate, naphthalene-1-sulfonate, naphthalene-2-sulfonate, mandelate, and the like.

Examples of bases that may be used to form base addition salts from RAR agonists with acidic groups include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines; dicyclohexylamine; tributyl amine; pyridine; N-methyl, N-ethylamine; diethylamine; triethylamine; mono-, bis-, or tris-(2-hydroxy-lower alkyl amines), such as mono-, bis-, or tris-(2-hydroxyethyl)amine, 2-hydroxy-tert-butylamine, or tris-(hydroxymethyl)methylamine, N,N-di-lower alkyl-N-(hydroxy lower alkyl)-amines, such as N,N-dimethyl-N-(2-hydroxyethyl)amine, or tri-(2-hydroxyethyl)amine; N-methyl-D-glucamine; and amino acids such as arginine, lysine, and the like.

As used herein, the term "patient" or "subject" refers to the recipient of a therapeutic treatment and includes all organisms within the kingdom animalia. In preferred embodiments, the animal is within the family of mammals, such as humans, bovine, ovine, porcine, feline, buffalo, canine, goat, equine, donkey, deer and primates. In some embodiments, the animal comprises a non-human mammal. The most preferred animal is human. Accordingly, in some embodiments, the patient or subject is a human.

As used herein, the terms "treat" "treating" and "treatment" include "prevent" "preventing" and "prevention" respectively.

As used herein the term "an effective amount" of an agent is the amount sufficient to treat, inhibit, or prevent liver damage resulting from pro-inflammatory type 1 NKT cell activity.

Some aspects of the disclosure relate to pretreatment of patients with a sulfatide and/or an RAR agonist prior to clinical manifestation of ALD. In several aspects of the disclosure, patients are treated with an effective amount of sulfatide and/or an RAR agonist prior to binge alcohol consumption. In some other aspects of the disclosure, patients are treated with an effective amount of sulfatide and/or an RAR agonist from about 0.5 hour to about 18 hours prior to binge alcohol consumption. In some other aspects of the disclosure, patients are treated with an effective amount of sulfatide and/or an RAR agonist during a period of chronic alcohol consumption. In some aspects of the disclosure, the patient is a human.

In some aspects of the disclosure, the specific amount of RAR agonist administered to a patient will vary depending upon the disease or condition being treated, as well as the age, weight and sex of the patient being treated. Generally, to achieve such a final concentration in, e.g., the intestines or blood, the amount of RAR agonist in a single dosage composition of the present disclosure will generally be about 0.1 milligrams to about 100 milligrams, preferably about 2.0 milligrams to about 60 milligrams, more preferably about 20 milligrams to about 50 milligrams. Examples of suitable doses include: between about 0.1 to about 10 mg/day; between about 0.5 to about 2 mg/day; between about 0.01-100 mg/day; between about 1 to about 50 mg/day; between about 0.1 mg/day to about 1.0 mg/day; between about 1.0 mg/day and about 5.0 mg/day; between about 5.0 mg/day and about 10.0 mg/day; between about 10.0 mg/day and about 15 mg/day; between about 15.0 mg/day and about 20.0 mg/day; between about 20.0 mg/day and about 25.0 mg/day; between about 30.0 mg/day and about 35.0 mg/day; between about 35.0 mg/day and about 40.0 mg/day; between about 40.0 mg/day and about 45.0 mg/day; between about 45.0 mg/day and about 50.0 mg/day; between about 50.0 mg/day and about 55.0 mg/day; between about 55.0 and about 60.0 mg/day; between about 60.0 mg/day and about 65.0 mg/day; between about 65.0 mg/day and about 70.0 mg/day; between about 70.0 mg/day and about 75.0 mg/day; between about 75.0 mg/day and about 80.0 mg/day; between about 80.0 mg/day and about 85.0 mg/day; between about 85.0 and about 90.0 mg/day; between about 85.0 mg/day and about 90.0 mg/day; between about 90.0 mg/day and about 95.0 mg/day; and between about 95.0 mg/day and about 100.0 mg/day. Obviously, the exact dosage will vary with the disease or disorder being treated, the preferred ranges being readily determinable. In some embodiments, the patient is a human.

When tazarotene is orally administered to effect a reduction in type I NKT cell activity, the daily dosage of tazarotene preferably is in a range of about 0.3 mg/day to about 7 mg/day or about 8 mg/day, more preferably in a range of about 0.6 mg/day to about 6.5 mg/day or about 7 mg/day. In some aspects of the disclosure, orally administered tazarotene is administered in daily dosages of tazarotene including 0.4 mg/day, 0.75 mg/day, 1.5 mg/day, 2.8 mg/day, 3 mg/day, 4.5 mg/day, 6 mg/day and 6.3 mg/day.

In accordance with the aspects of the disclosure, RAR agonist can be administered to alleviate a patient's symptoms, or can be administered to counteract a mechanism of the disorder itself. In certain embodiments, RAR agonist may be administered as a prophalactic measure. In some embodiments multiple doses of RAR agonist is administered. It will be appreciated by those of skill in the art that these treatment purposes are often related and that treatments can be tailored for particular patients based on various factors. These factors can include the age, gender, or health of the patient, and the progression of autoimmune or immune related disease or disorder. The treatment methodology for a patient can be tailored accordingly for dosage, timing of administration, route of administration, and by concurrent or sequential administration of other therapies. In some embodiments, the patient is a human.

In some aspects of the disclosure, one or more RAR agonist compounds can be administered alone or in combination with another therapeutic compound. For example, one or more RAR agonist compounds can be administered in combination with a sulfatide. Any currently known therapeutic compound used in treatment of the alcoholic liver disease, inflammatory disease, autoimmune disease or reperfusion injury can be used. In some aspects of the disclosure, RAR agonist can be administered in combination with hydrogen sulfide (H2S). In some aspects of the disclosure RAR agonist can be administered in combination with antioxidants. In some aspects of the disclosure RAR agonist can be administered in combination with, for example, corticosteroids, biologics (e.g. anti-TNF-alpha and anti-IL-6), immunomodulators (e.g. RU-486), disease modifying anti-rheumatic drugs (DMARDS, such as leflunomide), COX-2 inhibitors (celecoxib), non-steroidal antiinflammatory drugs (NSAIDS, such as naproxen), oral anti-diabetic (OAD, such as metformin or sitaglipten), GLP-1 agonists, insulin, PPAR agonists/antagonists, EGF mediators (anti-cancer agents), other agents effective to treat hepatic cancers, cell-based therapies for liver cancers; interferons (IFN) for Hepatitis C, multiple sclerosis or lupus erythematosus; and LFA-1 antagonists.

The RAR agonist compounds and sulfatide compounds described herein may be used as an active ingredient incorporated into a pharmaceutical composition. In some aspects of the disclosure the pharmaceutical composition may comprise a single active ingredient. In some aspects of the disclosure the pharmaceutical composition may comprise two, three, four, five or more active ingredients. All modes of administration are contemplated, for example, orally, rectally, parenterally, topically, or by intravenous, intramuscular, intrasternal or subcutaneous injection or in a form suitable by inhalation. The formulations may, where appropriate, be conveniently presented in discrete dosage units and may be prepared by any of the methods well known in the art of pharmacy. The active ingredients will ordinarily be formulated with one or more pharmaceutically acceptable excipients in accordance with known and established practice. Thus, the pharmaceutical composition can be formulated as a liquid, powder, elixir, injectable solution, suspension, suppository, etc.

In some aspects of the disclosure, pro-inflammatory type I NKT cell activity is inhibited by one or more RAR agonists selected from the group consisting of ATRA, retinol, 9-cis-RA or 13-cis-RA, tretinoin, AM580, AC55649, CD1530 or Tazarotene. In some aspects of the disclosure, pro-inflammatory type I NKT cell activity is inhibited by one or more polyolefinic retinoids, such as isoretinoin and acitretin. In some aspects of the disclosure, pro-inflammatory type I NKT cell activity is inhibited by one or more RAR agonists selected from the group consisting of etretinate, acitretin and isotretinoin.

Several embodiments relate to the inhibition of pro-inflammatory type I NKT cell activity by tazarotene, tazarotenic acid or a mixture thereof. Tazarotene is an ethyl ester prodrug that is metabolized to the corresponding free acid, tazarotenic acid. Tazarotene has a rigid ring-locked structure that offers limited conformational flexibility compared to all-trans-retinoic acid, the natural ligand for the retinoic acid receptors (RARs). This structural change confers tazarotenic acid with specificity for the RARs and selectivity for RARβ and RARγ.

Examples of RAR agonists further include esters of cis- and trans- retinoic acids, for example, alkyl esters, such as primary, secondary or tertiary alcohols, including but not limited to: methyl, ethyl, propyl, iso-propyl, butyl, iso-butyl, hexyl, heptyl, ethylhexyl, octyl, nonyl, lauryl, oleyl, stearyl, hydroxyethyl, hydroxypropyl, benzyl, alpha-methylbenzyl, alpha-propylphenyl, amyl, iso-amyl, anisyl, cetyl, menthyl, cinnamyl, pinacol, furyl, or myristyl.

Pharmaceutically acceptable salts of RAR agonists can also be used to inhibit type I NKT cell activity. Compounds disclosed herein which possess a sufficiently acidic, a sufficiently basic, or both functional groups, and accordingly can react with any of a number of organic or inorganic bases, and inorganic and organic acids, to form a salt.

Examples of acids that may be used to form acid addition salts from RAR agonists with basic groups include inorganic acids such as hydrochloric acid, hydrobromic acid, hydroiodic acid, sulfuric acid, phosphoric acid, and the like, and organic acids such as p-toluenesulfonic acid, methanesulfonic acid, oxalic acid, p-bromophenyl-sulfonic acid, carbonic acid, succinic acid, citric acid, benzoic acid, acetic acid, and the like. Examples of such salts include the sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, gamma-hydroxybutyrate, glycolate, tartrate, methanesulfonate, propanesulfonate, naphthalene-1-sulfonate, naphthalene-2-sulfonate, mandelate, and the like.

Examples of bases that may be used to form base addition salts from RAR agonists with acidic groups include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines; dicyclohexylamine; tributyl amine; pyridine; N-methyl, N-ethylamine; diethylamine; triethylamine; mono-, bis-, or tris-(2-hydroxy-lower alkyl amines), such as mono-, bis-, or tris-(2-hydroxyethyl)amine, 2-hydroxy-tert-butylamine, or tris-(hydroxymethyl)methylamine, N,N-di-lower alkyl-N-(hydroxy lower alkyl)-amines, such as N,N-dimethyl-N-(2-hydroxyethyl)amine, or tri-(2-hydroxyethyl)amine; N-methyl-D-glucamine; and amino acids such as arginine, lysine, and the like.

In some aspects of the disclosure, the specific amount of RAR agonist administered to a patient will vary depending upon the disease or condition being treated, as well as the age, weight and sex of the patient being treated. Generally, to achieve such a final concentration in, e.g., the intestines or blood, the amount of RAR agonist in a single dosage composition of the present embodiments will generally be about 0.1 milligrams to about 100 milligrams, preferably about 2.0 milligrams to about 60 milligrams, more preferably about 20 milligrams to about 50 milligrams. Examples of suitable doses include: between about 0.1 to about 10 mg/day; between about 0.5 to about 2 mg/day; between about 0.01-100 mg/day; between about 1 to about 50 mg/day; between about 0.1 mg/day to about 1.0 mg/day; between about 1.0 mg/day and about 5.0 mg/day; between about 5.0 mg/day and about 10.0 mg/day; between about 10.0 mg/day and about 15 mg/day; between about 15.0 mg/day and about 20.0 mg/day; between about 20.0 mg/day and about 25.0 mg/day; between about 30.0 mg/day and about 35.0 mg/day; between about 35.0 mg/day and about 40.0 mg/day; between about 40.0 mg/day and about 45.0 mg/day; between about 45.0 mg/day and about 50.0 mg/day; between about 50.0 mg/day and about 55.0 mg/day; between about 55.0 and about 60.0 mg/day; between about 60.0 mg/day and about 65.0 mg/day; between about 65.0 mg/day and about 70.0 mg/day; between about 70.0 mg/day and about 75.0 mg/day; between about 75.0 mg/day and about 80.0 mg/day; between about 80.0 mg/day and about 85.0 mg/day; between about 85.0 and about 90.0 mg/day; between about 85.0 mg/day and about 90.0 mg/day; between about 90.0 mg/day and about 95.0 mg/day; and between about 95.0 mg/day and about 100.0 mg/day. Obviously, the exact dosage will vary with the disease or disorder being treated, the preferred ranges being readily determinable. In some aspects of the disclosure, the patient is a human.

When tazarotene is orally administered to effect a reduction in type I NKT cell activity, the daily dosage of tazarotene preferably is in a range of about 0.3 mg/day to about 7 mg/day or about 8 mg/day, more preferably in a range of about 0.6 mg/day to about 6.5 mg/day or about 7 mg/day. In some aspects of the disclosure, orally administered tazarotene is administered in daily dosages of tazarotene including 0.4 mg/day, 0.75 mg/day, 1.5 mg/day, 2.8 mg/day, 3 mg/day, 4.5 mg/day, 6 mg/day and 6.3 mg/day.

In accordance with the aspects of the disclosure, RAR agonist can be administered to alleviate a patient's symptoms, or can be administered to counteract a mechanism of the disorder itself. In certain aspects of the disclosure, RAR agonist may be administered as a prophalactic measure. In some aspects of the disclosure multiple doses of RAR agonist is administered. It will be appreciated by those of skill in the art that these treatment purposes are often related and that treatments can be tailored for particular patients based on various factors. These factors can include the age, gender, or health of the patient, and the progression of autoimmune or immune related disease or disorder. The treatment methodology for a patient can be tailored accordingly for dosage, timing of administration, route of administration, and by concurrent or sequential administration of other therapies. In some embodiments, the patient is a human.

In some aspects of the disclosure, one or more RAR agonist compounds can be administered alone or in combination with another therapeutic compound. For example, one or more RAR agonist compounds can be administered in combination with a NKT-2 activator (for example miltefosine or a miltefosine analog or a phospholipid), a sulfatide, or a NKT-2 activator (for example miltefosine or a miltefosine analog or a phospholipid) and a sulfatide.

### NKT-2 activators

A number of NKT-2 activators can be used in accordance with some embodiments herein. In some aspects of the disclosure, the NKT-2 activator comprises a phospholipid, for example a lysophospholipid, for example lysophosphatidylcholine (LPC), miltefosine, or a miltefosine analog. Effects of some LPC compounds on Type II NKT cells have been described by Maricic et al., "Recognition of Lysophosphatidylcholine by Type II NKT Cells and Protection from an Inflammatory Liver Disease" J. Immunology, published online September 26 2014, 1400699.

In some aspects of the disclosure, the NKT-2 activator comprises a LPC having the formula of formula IV: 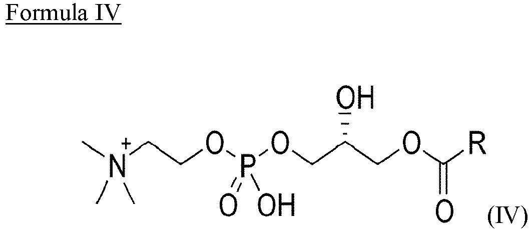 wherein R is selected from the group consisting of a C₁ to C₄₀ alkyl, a C₁ to C₄₀ substituted alkyl, a C₁ to C₄₀ alkenyl, a C₁ to C₄₀ substituted alkenyl and a C₁ to C₄₀ alkynl. Example LPCs include LPC (C18:0), the structure of which is depicted below, 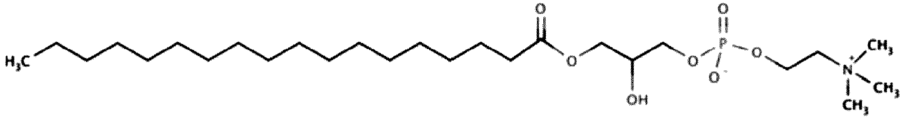 LPC (C16:0), the structure of which is depicted below, 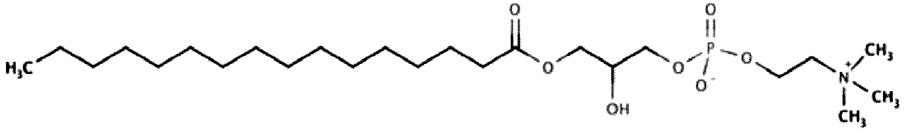 LPC (C18:1(9Z)), the structure of which is depicted below, 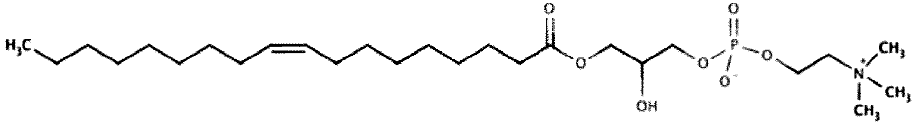 LPC (C18:1(1oleyl)), the structure of which is depicted below, 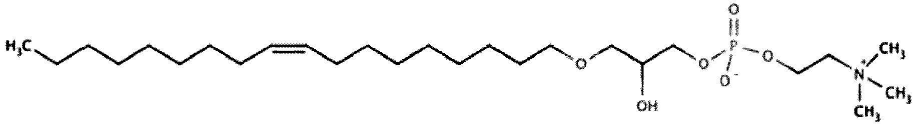 and Lign A (LPC (C24:0)), the structure of which is depicted below, 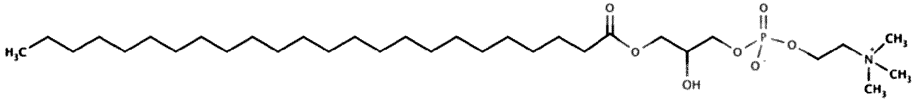 Additional example phospholipids contemplated as suitable NKT-2 activators include, but are not limited to, lyso platelet-activating factor(LPAF), such as LPAF (C18:0), the structure of which is depicted below, 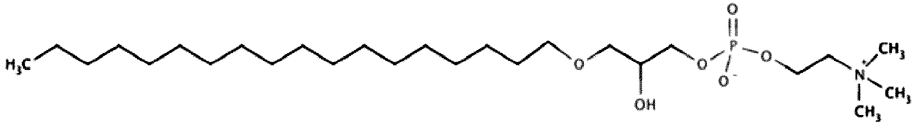 and lysosphingomyelin (LSM), the structure of which is depicted below,

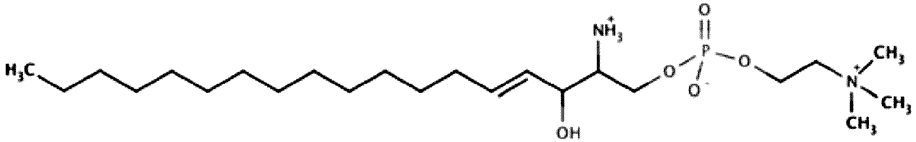

In some embodiments, the NKT-2 activator comprises miltefosine. The structure of miltefosine is shown below:

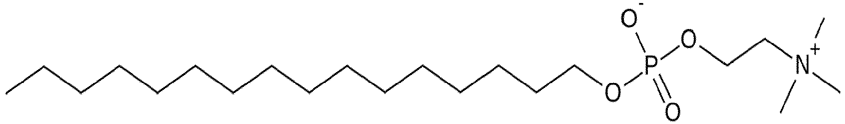

In some aspects of the disclosure, the NKT-2 activator comprises a miltefosine analog. As used herein, "miltefosine analog" refers broadly to structural analogs of miltefosine and functional analogs of miltefosine. Some miltefosine analogs in accordance with some embodiments herein comprise compounds of formula V: 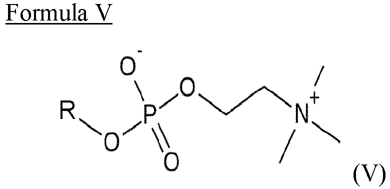 wherein R is selected from the group consisting of a C₁ to C₃₀ alkyl, a C₁ to C₃₀ substituted alkyl, a C₁ to C₃₀ alkenyl, a C₁ to C₃₀ substituted alkenyl and a C₁ to C₃₀ alkynl.

Example miltefosine analogs of the disclosure include, but are not limited to compounds listed in Table 2.1 and 2.2, below.

**Table 2.1: Miltefosine analogs**

| Name | Compound Name | Structure Name |
|---|---|---|
| J101Y | 2-Dodecyl-N-(1,2,2,6,6-pentamethyl-4-piperidinyl)-succinimide | 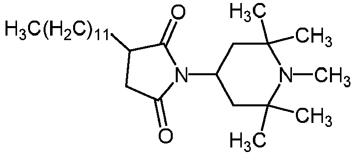 |
| J102Y | Dodecanoic acid 3-(dimethylamino)-propylamide | 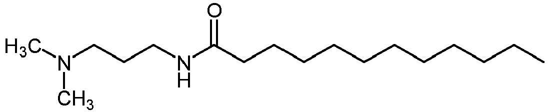 |
| J115C | N-Farnesoyl-D-erythrosphingosine | 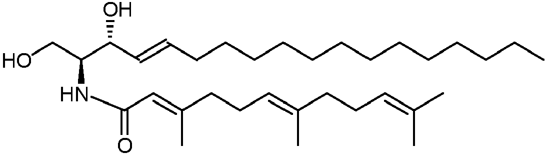 |
| J117Y | 18b-Glycyrrhetinic acid | 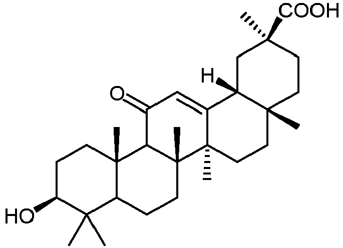 |
| J118Y | Ursodesoxycholic acid | 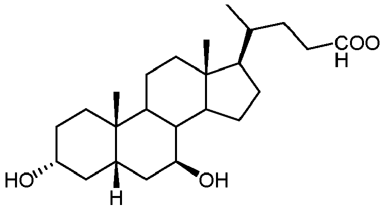 |
| J119Y | Mannidmonooleate | 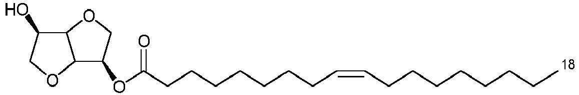 |
| J120Y | C₂₁H₂₇NO₃ | 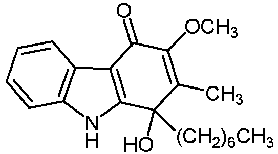 |
| J121Y | C₃₂H₄₉NO₂ | 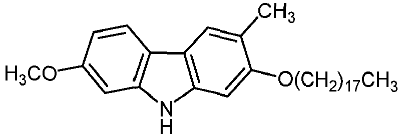 |
| J122Y | C₂₂H₂₉NO₂ | 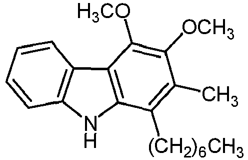 |
| J123Y | C₂₆H₃₁NO₃ | 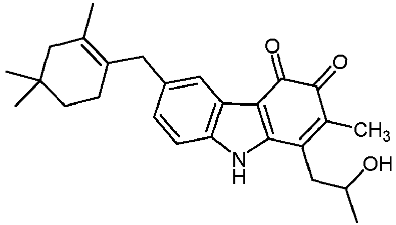 |
| J124Y | Cyclododecylamin | 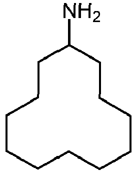 |
| J125Y | Capsaicin | 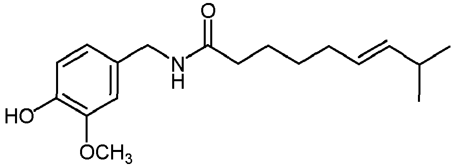 |
| J127C | (N-Hexadecansulfonyl-D-erythrosphingosinoyl)-succinic acid | 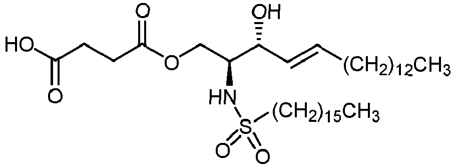 |
| J129Y | (2S,3S,5S,6S)-5,6-Dimethoxy-5,6-dimethyl-[1,4]dioxane-2,3-dicarboxylic dimethyl ester | 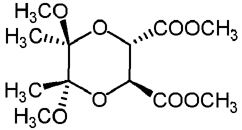 |
| J130Y | Ethyl-2-oxo-piperidine-carboxylate | 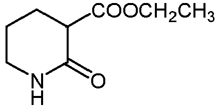 |
| J131Y | Methyl-1H-1,2,4-Triazole-3-carboxylate | 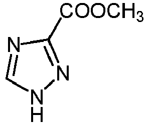 |
| J132Y | 4-(2-Ca rboxyethyl)-4-nitropimelic acid | 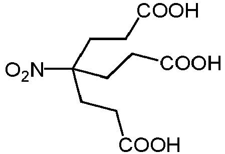 |
| J133C | 30-Methylsphingosine | 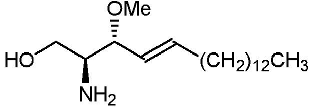 |
| J134C | Sphingosine | 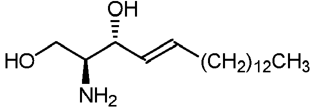 |
| J135Y | C₄₂H₇₇NO₄ | 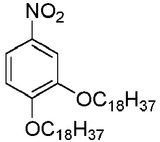 |
| J136Y | C₄₂H₇₉NO₂ | 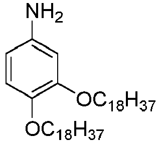 |
| J137Y | C₂₈H₅₀O₂ | 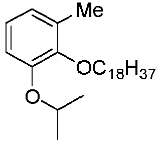 |
| J138Y | C₁₉H₁₇NO₂ | 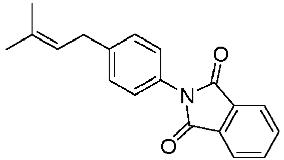 |
| J139Y | C₁₄H₁₃NO₂ | 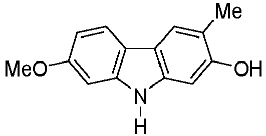 |
| J13C | N-(3,6,9,12,15-Pentoxapalmitoyl)-sphingosine | 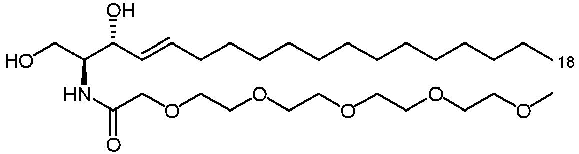 |
| J140Y | C₁₃H₁₁NO | 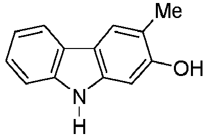 |
| J141Y | C₁₅H₁₅NO₂ | 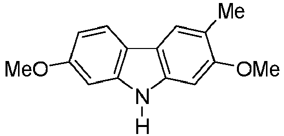 |
| J142Y | C₁₅H₁₃NO₃ | 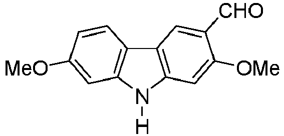 |
| J143Y | C₂₀H₂₅NO₄ | 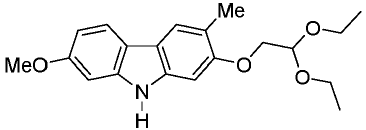 |
| J144Y | Furoclausine A | 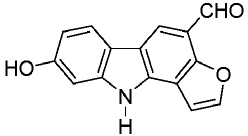 |
| J145Y | C₁₅H₁₃NO₃ | 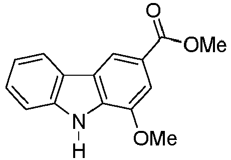 |
| J146Y | C₁₅H₁₅NO₂ | 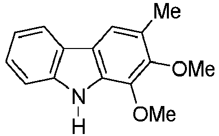 |
| J147Y | C₁₇H₁₃NO₃ | 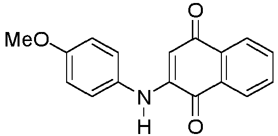 |
| J148Y | C₁₇H₁₁NO₃ | 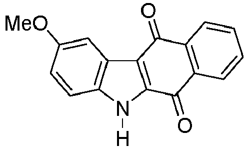 |
| J149Y | C₁₇H₁₁NO₃ | 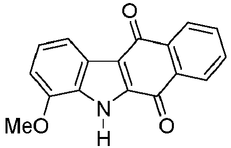 |
| J14Y | Perinaphthenon | 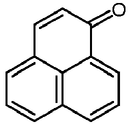 |
| J150Y | C₁₈H₁₃NO₃ | 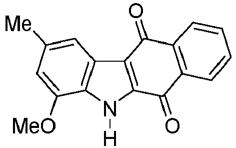 |
| J151Y | C₁₅H₁₃NO₄ | 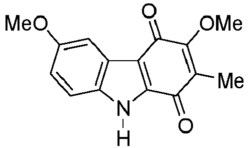 |
| J152Y | C₁₄H₁₁NO₃ | 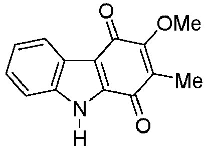 |
| J153Y | C₁₆H₁₇NO₄ | 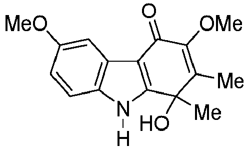 |
| J154Y | C₁₄H₁₁NO₂ | 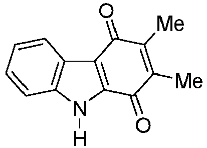 |
| J155Y | C₁₅H₁₃NO₃ | 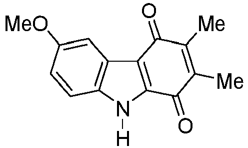 |
| J156Y | C₁₅H₁₃NO₄ | 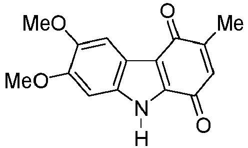 |
| J157Y | C₁₃H₁₁NO₂ | 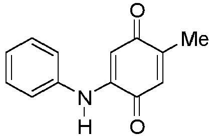 |
| J158Y | C₁₄H₁₃NO₃ | 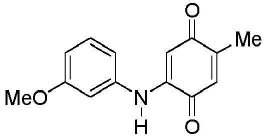 |
| J159Y | C₁₇H₁₃BrN₂O₂ | 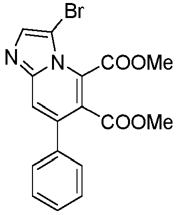 |
| J160Y | C₁₉H₁₈N₂O₅ | 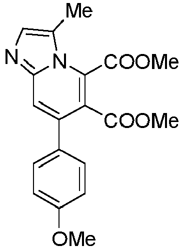 |
| J161Y | C₂₃H₂₀N₂O₂ | 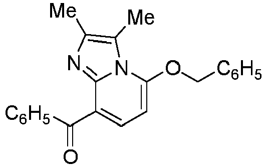 |
| J162Y | C₂₅H₂₁N₃O₃ | 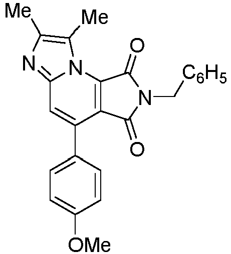 |
| J163Y | C₁₅H₁₀N₄O₂ | 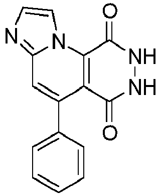 |
| J164Y | C₁₆H₁₂N₄O₃ | 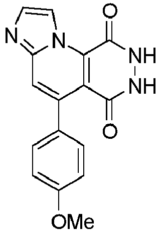 |
| J165Y | C₂₆H₁₇N₅O₉ | 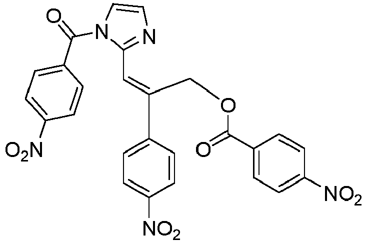 |
| J166Y | C₁₇H₂₀N₂O₈ | 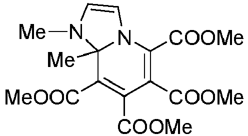 |
| J167Y | C₁₇H₁₄N₂O₄ | 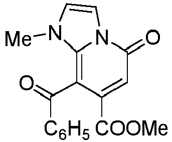 |
| J168Y | C₁₇H₁₄N₂O₄ | 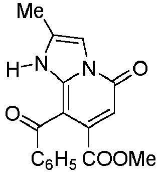 |
| J169Y | C₁₈H₁₆N₂O₄ | 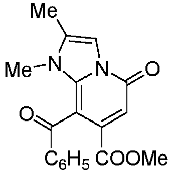 |
| J170Y | C₁₆H₁₆N₂O₂ | 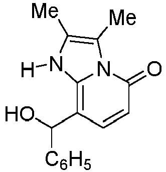 |
| J171Y | C₁₇H₁₄N₂O₄ | 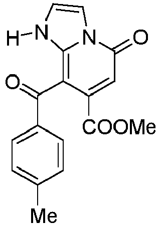 |
| J172Y | C₁₆H₁₁N₃O₆ | 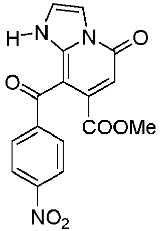 |
| J173Y | C₁₇H₁₄N₂O₄ | 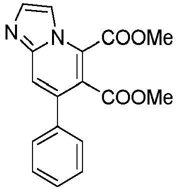 |
| J174Y | C₂₁H₁₆N₂O₄ | 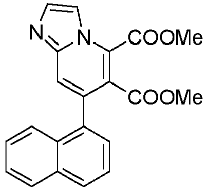 |
| J175Y | C₁₉H₁₉N₃O₄ | 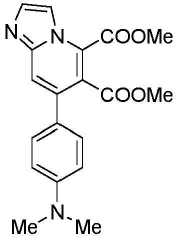 |
| J176Y | C₁₈H₁₆N₂O₅ | 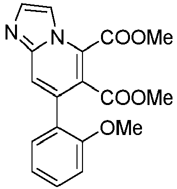 |
| J177Y | C₁₉H₁₈N₂O₆ | 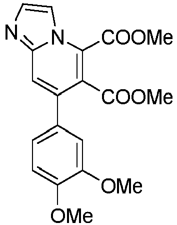 |
| J178Y | C₃₀H₂₆N₄O₃ | 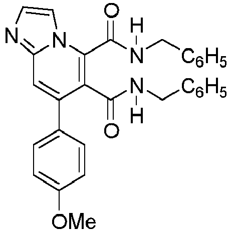 |
| J179Y | C₁₆H₁₆N₂O₃ | 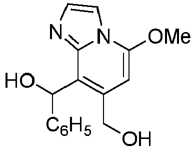 |
| J184Y | C₂₀H₁₉N₅O₃ | 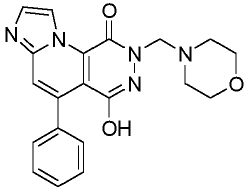 |
| J185Y | C₂₀H₁₄N₂O₄ | 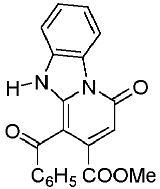 |
| J186Y | C₁₁H₉N₃O₃ | 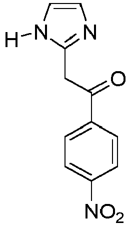 |
| J187Y | C₃₂H₂₈N₂O₄ | 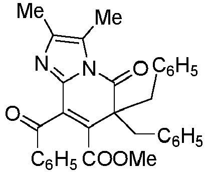 |
| J188Y | C₂₃H₁₈N₂O₄ | 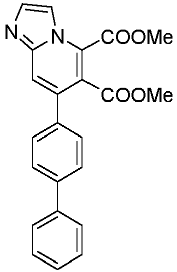 |
| J189Y | 6-Heptyl-2-methyl-4-oxocyclohex-2-ene carboxylic acid ethyl ester | 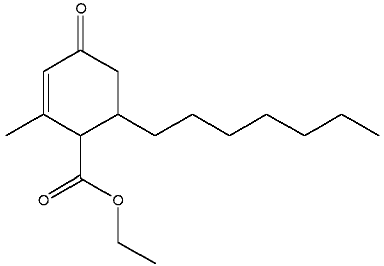 |
| J190Y | 3-Desoxyandrosterone | 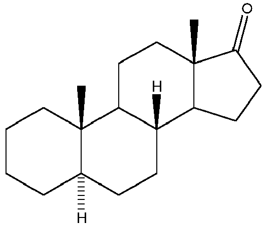 |
| J191Y | 6-(Heptadecyloxy)-1-hydroxynaphthalene-2-carboxylic acid | 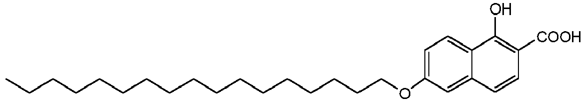 |
| J192Y | 2-(10-Hydroxydecyl)-5,6-dimethoxy-3-methyl-[1,4]benzoquinone | 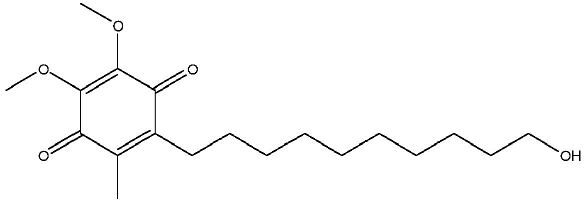 |
| J193Y | 1-Pyridin-2-yl-heptadecan-1-one | 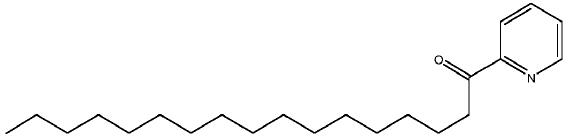 |
| J194Y | N-(2-Hydroxyethyl)stearamide | 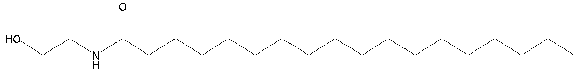 |
| J195Y | N-[2-(2-Hydroxyethoxy)ethyl]stearamide | 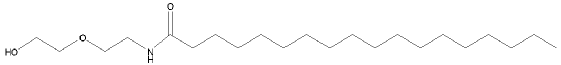 |
| J196C | 3O-Methyl-N-a rach idoyl-D-erythro-sphingosine | 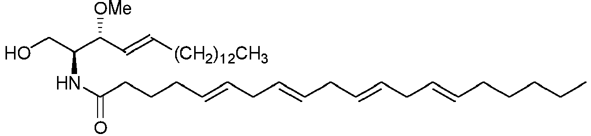 |
| J196C | 3O-Methyl-N-a rach idoyl-D-erythro-sphingosine | 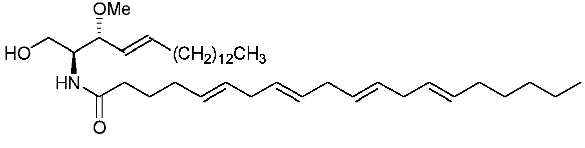 |
| J197C | N-Arachidoyl-D-erythro-sphingosine | 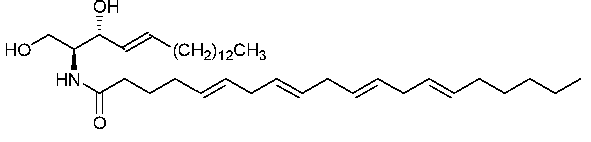 |
| J197C | N-Arachidoyl-D-erythro-sphingosine | 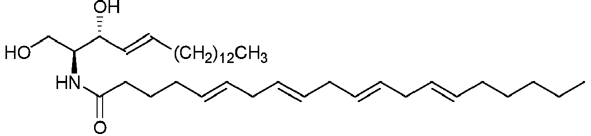 |
| J198Y | N-(1,3-Dihydroxybutan-2-yl)stearamide | 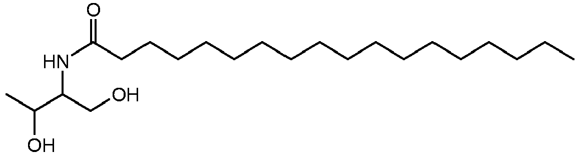 |
| J199Y | N-[2-(2-Aminoethoxy)ethyl]ste aramide | 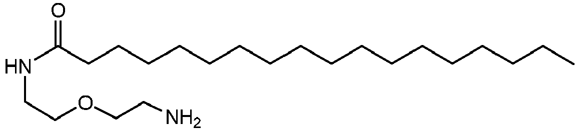 |
| J19Y | a-Naphthoflavon | 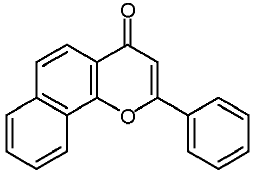 |
| J200Y | N-(3-Amino-2-hydroxypropyl)stearamide | 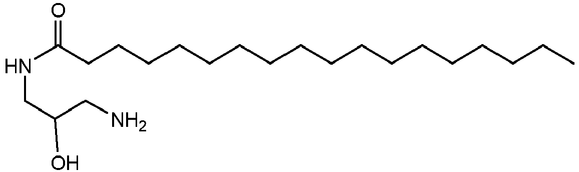 |
| J201C | 1O-(all-Benzyl-galactosyl)-N-palmitoyl-D-erythro-sphingosine | 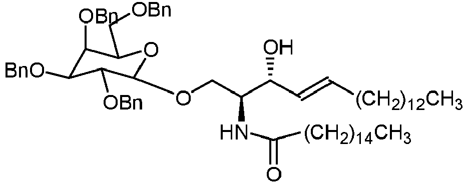 |
| J202Y | 1-(Piperazin-1-yl)octadecan-1-one | 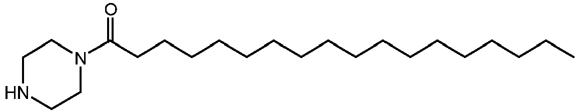 |
| J203Y | N-(2-Aminoethyl)stearamide | 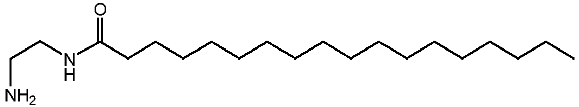 |
| J204Y | N-(2-Aminocyclohexyl)stearamide | 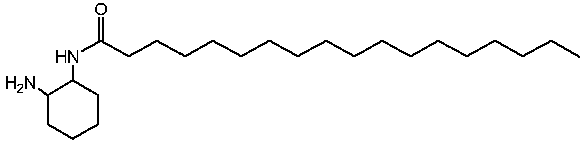 |
| J205Y | N-[2-(1H-Imidazol-4-yl)ethyl]stearamide | 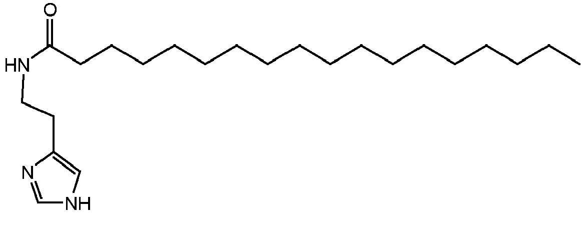 |
| J206C | 3O-Methyl-N-farnesoyl-D-erythro-sphingosine | 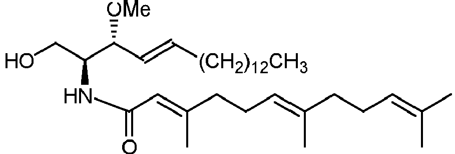 |
| J209C | Ceramide-1-phosphate | 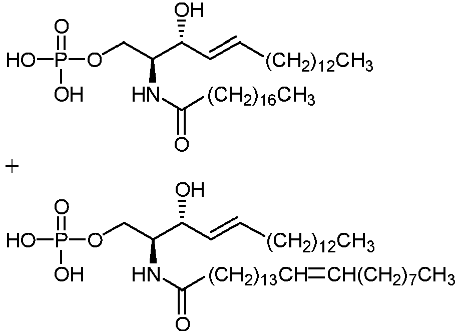 |
| J210C | N-AclGNIIE-D-erythro-sphingosine | 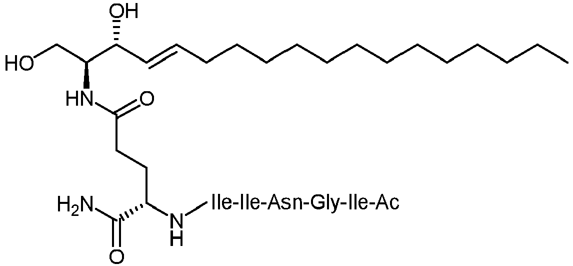 |
| J217Y | 3-Hydroxyadamantane-1-carboxylic acid | 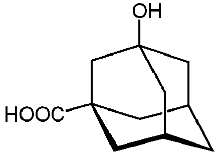 |
| J218Y | Triethylene glycol monododecyl ether | 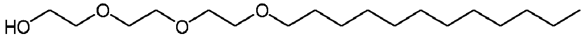 |
| J219Y | (1S)-Stearoylpyrrolidine-2-carboxylic acid | 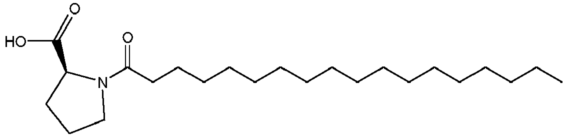 |
| J21C | N-(Cholesteryloxyacetyl)-sphingosine | 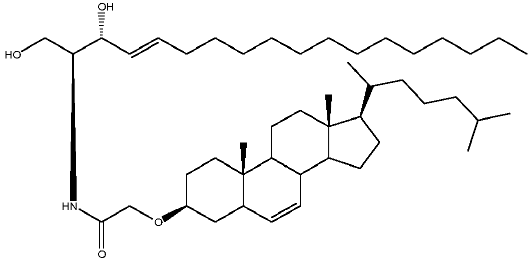 |
| J220C | N-AclLQIGNIIE-D-erythro-sphingosine | 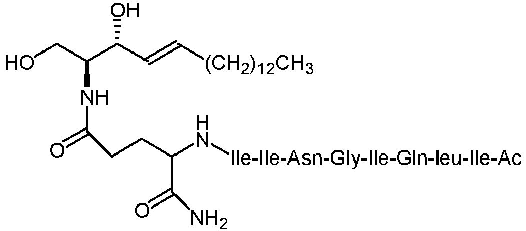 |
| J221C | (3S)-Cbz-Amino-octan-(1,4R)-diol | 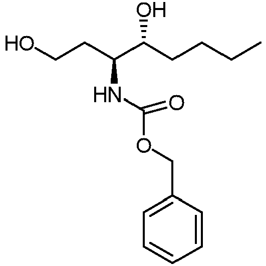 |
| J223Y | 3-Amino-N-octadecylpropanamide | 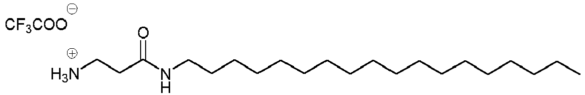 |
| J224Y | O-Palmitoyl-L-carnitine chloride | 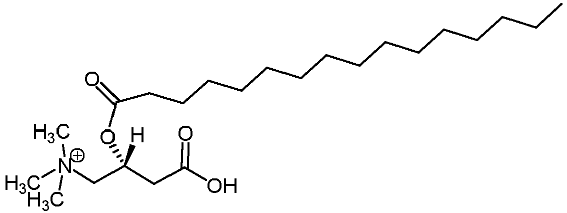 |
| J225Y | tert-Butyl 2-(octadecylcarbamoyl)et hylcarbamate | 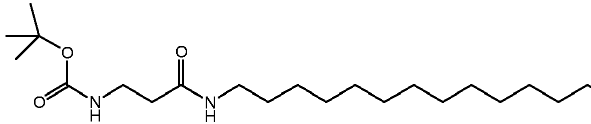 |
| J226Y | N-Palmitoyl-L-serine phosphoric acid (L-NASPA) | 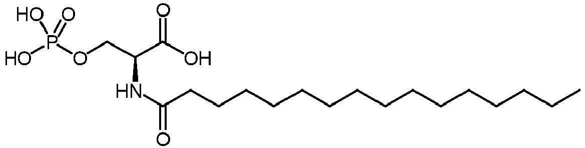 |
| J227Y | 2-Amino-N-octadecylacetamide | 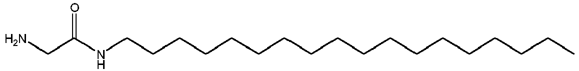 |
| J228Y | 1-Dodecanoylpyrrolidine-2-carboxylic acid | 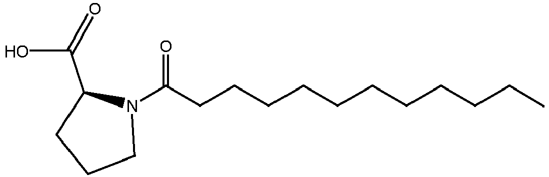 |
| J229Y | 3-(tert-Butoxycarbonyl)-2-dodecanamidopropanoic acid | 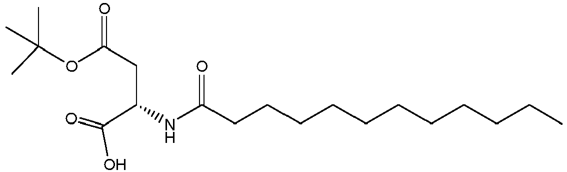 |
| J22C | N-(3,6-Dioxaheptanoyl)-sphingosine | 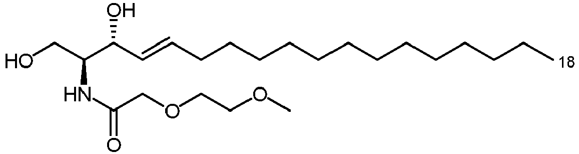 |
| J230Y | 2-Dodecanamidosuccinic acid | 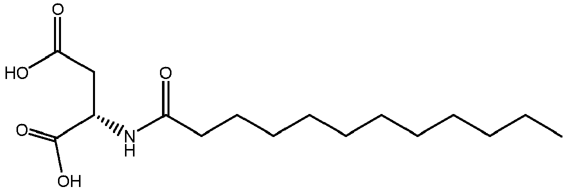 |
| J231Y | 3-(tert-Butoxycarbonyl)-2-stearamidopropanoic acid | 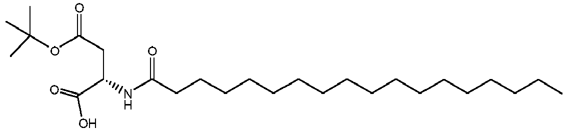 |
| J232C | 3O-Methyl-N-(2-propenoyl)-D-erythro-sphingosine | 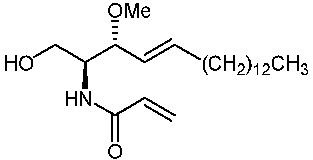 |
| J234Y | (2S,3R,4S,5S)-N-Dodecyl-2,3,4,5,6-pentahydroxyhexanamide | 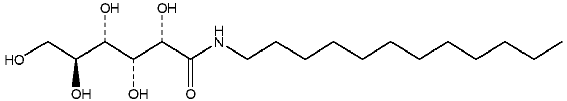 |
| J235C | 3-O-Methyl-N-(w-amino-4,7,10,13-tetroxapentadecanoyl)-sphingosine | 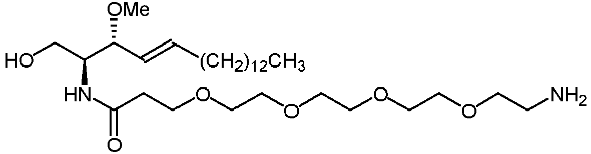 |
| J236C | N-(2-Propenoyl)-D-erythro-sphingosine | 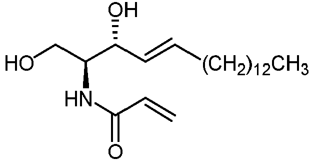 |
| J239Y | (2S,3R,4S,5S)-N-Dodecyl-2,3,4,5,6-pentaacetoxyhexanamide | 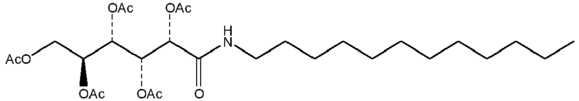 |
| J240C | 3-O-Methyl-N-(w-amino-3,6-bisoxanonanoyl)-sphingosine | 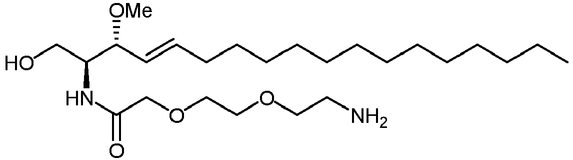 |
| J241C | 3O-Methyl-1-acetoxy-2-acetamido-D-erythro-ro-sphingosine | 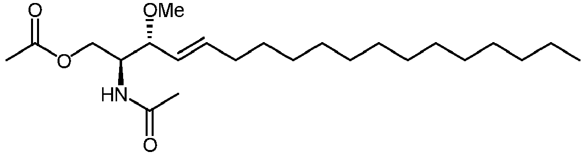 |
| J242C | 3O-Methyl-2-acetamido-D-erythro-sphingosine | 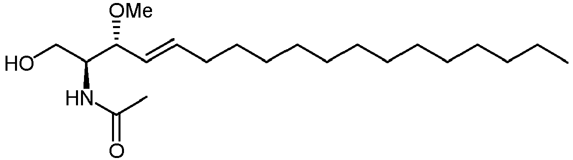 |
| J245C | (S)-4,5-Dihydro-4-((R,E)-1-methoxyhexadec-2-enyl)-2-phenyloxazole | 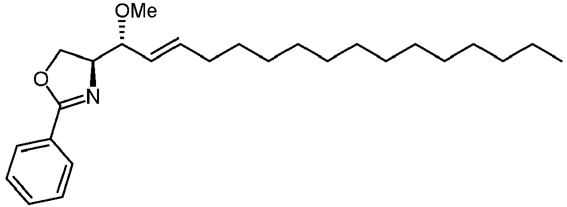 |
| J246C | 2N-Ethyl-3O-methyl-D-erythro-sphingosine | 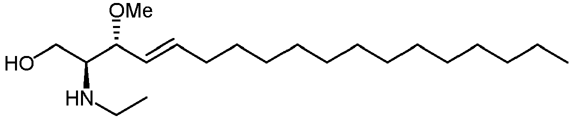 |
| J247C | 2N-Acetyl-(10,2N,3O)-trimethyl-D-erythro-sphingosine | 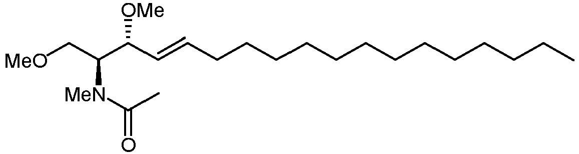 |
| J248C | 2N-Acetyl-3O-methyl-D-erythro-sphingosine | 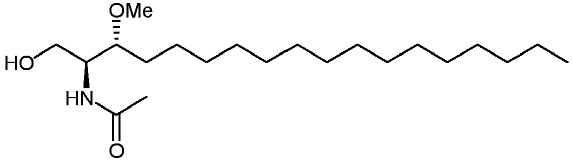 |
| J249Y | Dodecylamine | 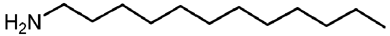 |
| J250Y | N-Dodecyl-2,3,4,5,6-penta(N-Boc-glycinoyl)hexanamide | 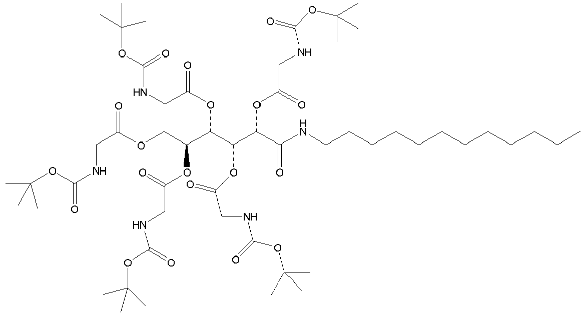 |
| J251Y | (S)-4-(Methylthio)-2-(stearamido)butanoic acid | 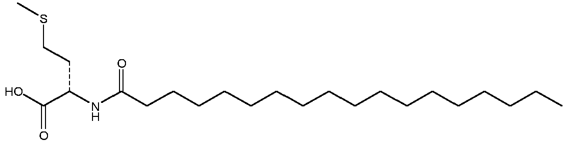 |
| J252Y | (S)-2-(Dodecanamido)-4-(methylsulfinyl)butanoic acid | 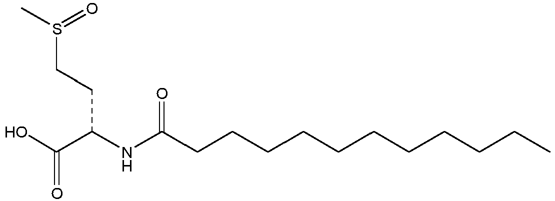 |
| J258Y | 2-Amino-N-dodecylacetamide hyd rochloride | 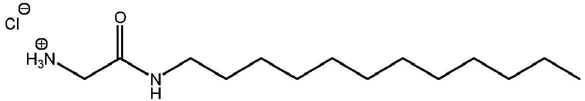 |
| J259Y | 3-Amino-N-octadecylpropanamide hydrochloride | 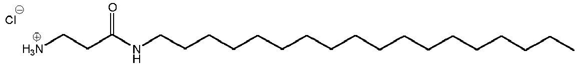 |
| J260Y | 2-Amino-N-octadecylacetamide hydrochloride | 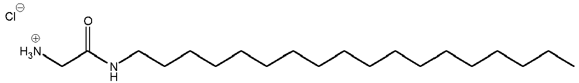 |
| J265Y | (S)-2-Amino-4-(methylsulfinyl)-N-octadecylbutanamide TFA salt | 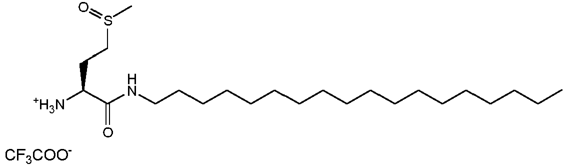 |
| J281Y | 3-Methyl-2-octadecyloxyphenol | 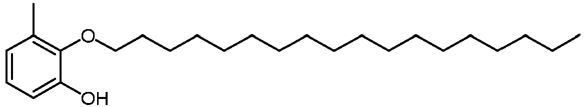 |
| J282Y | 3-Methyl-4-nitro-2-octadecyloxyphenol | 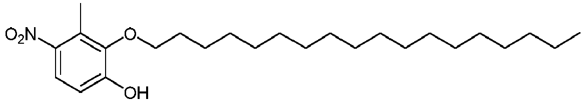 |
| J283Y | 3-Methyl-2-octadecyloxy-5-nitrophenol | 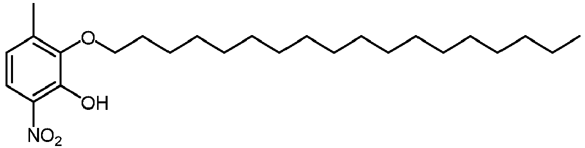 |
| J284Y | 3-Methyl-4,6-dinitro-2-octadecyloxyphenol | 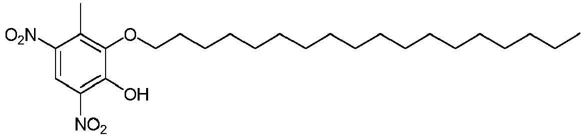 |
| J288Y | (S)-2-Amino-3-hydroxy-N-octadecylpropanamide | 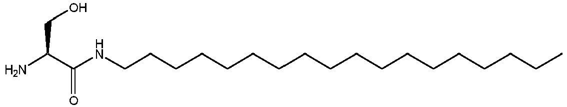 |
| J28C | N-Methoxyacetyl-sphingosine | 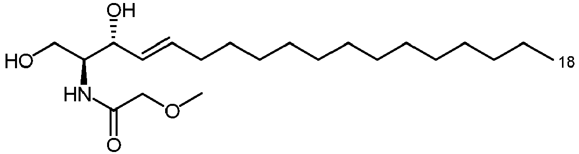 |
| J29C | N-Linolenoylsphingosine | 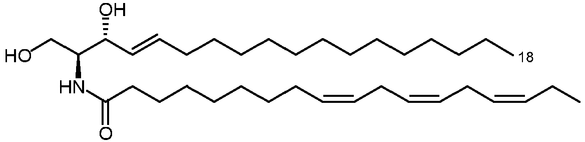 |
| J306C | 2N-Acetyl-1-amino-3O-methyl-1-desoxy-erythro-sphingosine | 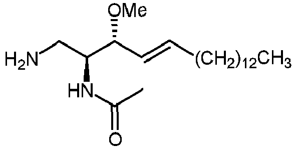 |
| J30C | N-(16-Acetoxyhexadecanoyl)-sphingosine | 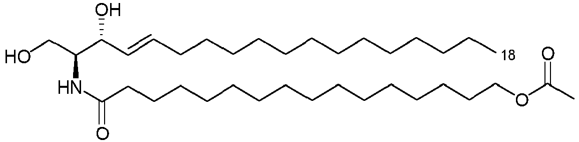 |
| J310C | 2N-Ethyl-D-erythro-sphingosine | 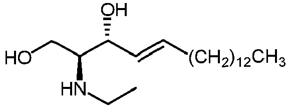 |
| J31S | 5a-Pregnane-3b-ol | 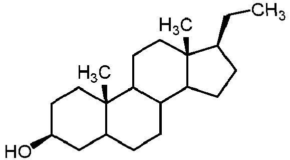 |
| J347C | 1N,2N-Diacetyl-1-desoxy-3O-methyl-erythro-sphingosine | 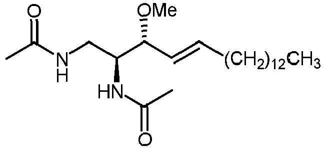 |
| J350C | 2N-(15-Methylpalmitoyl)-3O-methyl-eryth ro-sphingosine | 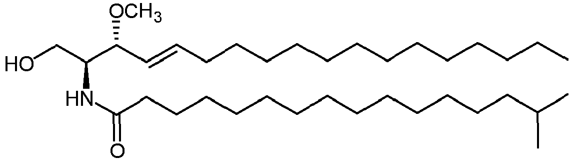 |
| J35C | 3-O-Methyl-N-(3,6,9,12,15-pentoxapalmitoyl)-sphingosine | 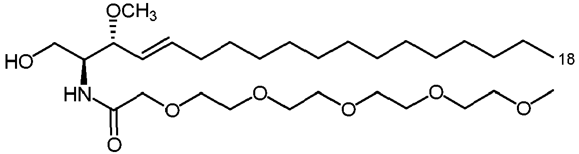 |
| J363C | 2N-(15-Methylpalmitoyl)-1-amino-3O-methyl-1-desoxy-erythro-sphing. | 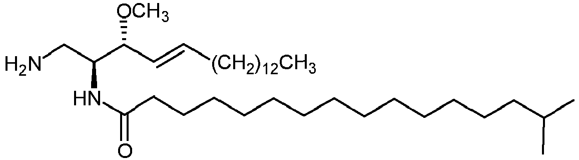 |
| J365C | 2N-Acetyl-1-dimethylamino-3O-methyl-1-desoxy-erythro-sphing. TFA | 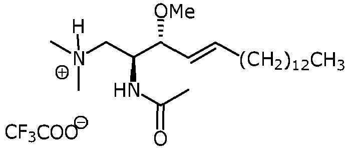 |
| J366C | 2N-Geranoyl-1-amino-3-O-methyl-1-desoxy-erythro-sphing. TFA | 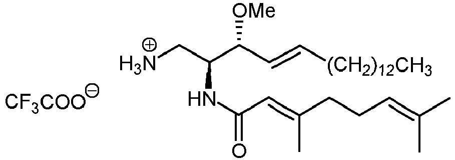 |
| J36C | 3O-Methyl-N-hexanoyl-sphingosine | 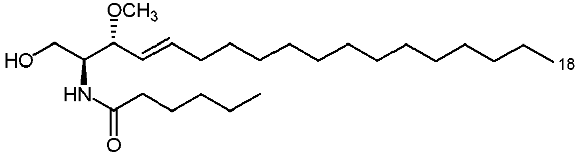 |
| J37C | 3O-Methyl-N-palmitoyl-sph ingosine | 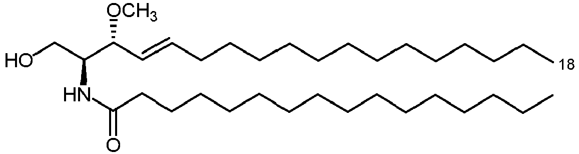 |
| J390E | 1-Elaidylphosphocholine (C18:1,trans) | 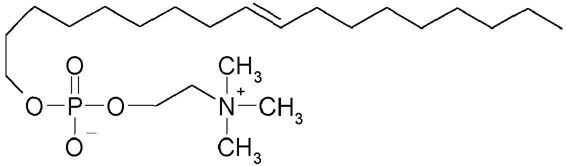 |
| J397E | 13-(Z)-Docosenylphosphocholi ne | 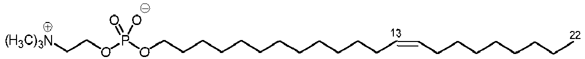 |
| J401E | peracetylated Glucosamin-1-O-hexadecyl-2-O-methyl-glycerol | 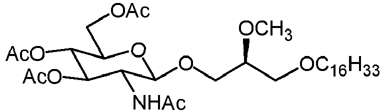 |
| J403E | 1-O-Phosphocholine-2-O-benzyl-octadecane | 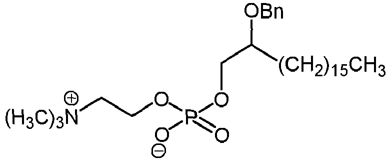 |
| J404E | N-acetylated Glucosamin-1-O-hexadecyl-2-O-methyl-glycerol | 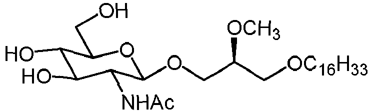 |
| J406E | lyso-PAF-16 (LPAF) | 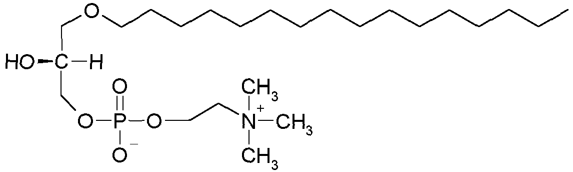 |
| J407E | PAF-16 Antagonist (PAFA) | 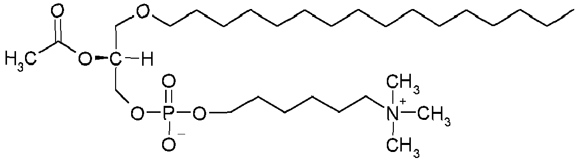 |
| J408E | 2-O-Methyl PAF C-16 (TF003, 2OMePAF) | 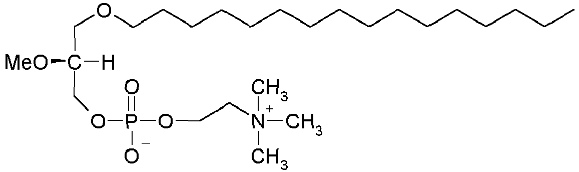 |
| J409E | Methylcarbamyl PAF C-16 (mcPAF) | 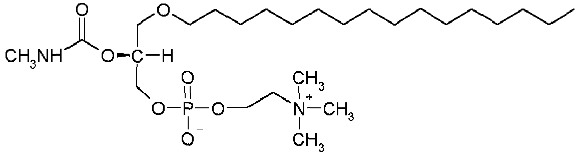 |
| J410E | Edelfosine (TF001, ET-18) | 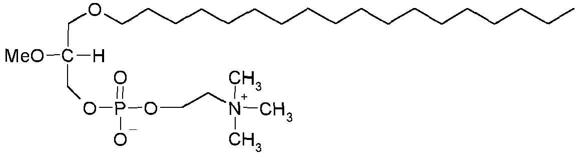 |
| J411E | Pyrrolidino PAF C-16 (PyPAF) | 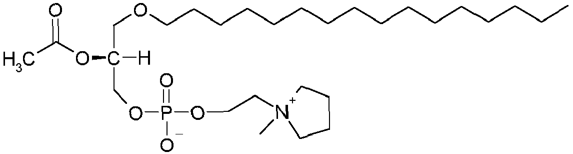 |
| J412E | Hexadecyl methyl glycerol (HMG) | 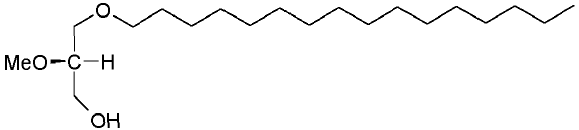 |
| J413E | Butanoyl PAF (BaPAF) | 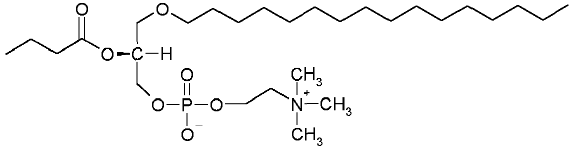 |
| J414E | Butenoyl PAF (BePAF) | 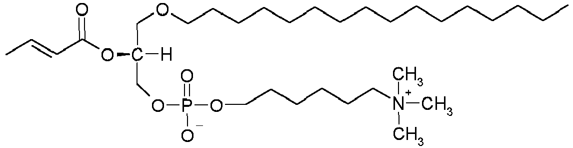 |
| J415E | 2-O-Ethyl PAF C-16 (TF004, 2OEtPAF) | 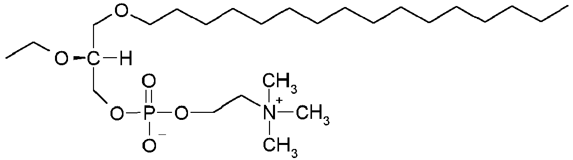 |
| J416E | 2-Thio PAF (2ThPAF) | 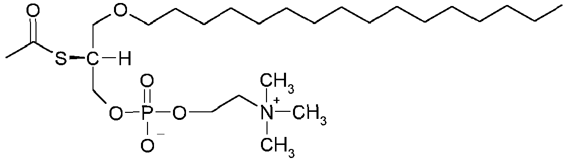 |
| J417E | Ilmofosine (ILM) | 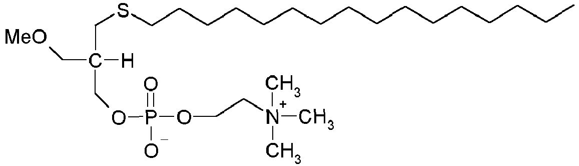 |
| J418E | Lysolecithin, palmitoyl (LPC16) | 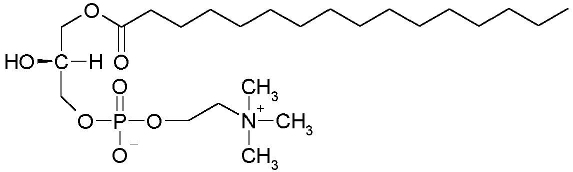 |
| J419E | PAF-16 (PAF) | 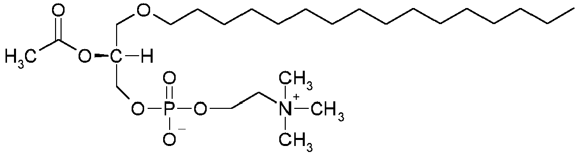 |
| J420E | Miltefosine (HePC) | 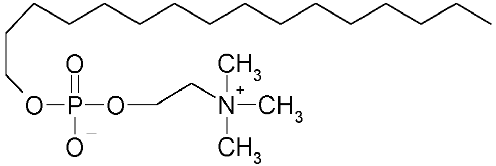 |
| J421E | Propionyl PAF C-16 (PrPAF) | 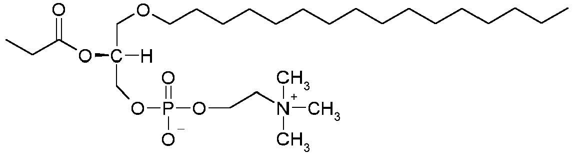 |
| J422E | Enantio PAF C-16 (enPAF) | 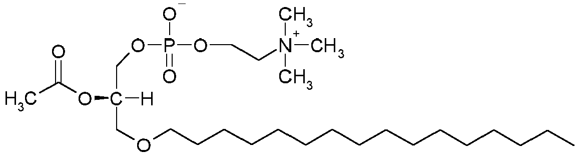 |
| J423E | 1-O-Octadecyl-(2,2-dimethyl)-propanediol-3-phosphocholine (OPPC) | 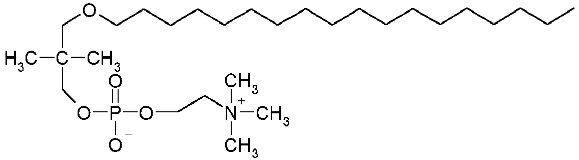 |
| J424E | Dodecylphosphocholine (DoPC) | 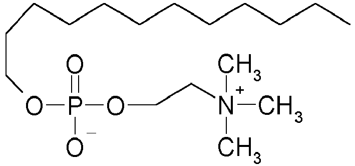 |
| J425E | 1-O-Hexadecyl-propanediol-3-phosphocholine (HPPC) | 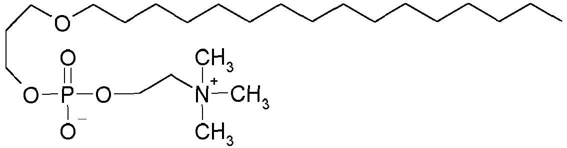 |
| J426E | Octadecyl-phosphocholine (OcPC) | 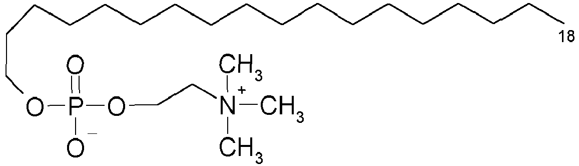 |
| J427E | Perifosine (PFS) | 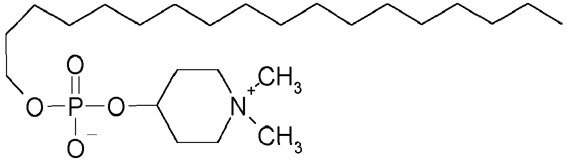 |
| J428E | rac-2-Benzyloxy-1-octadecanol | 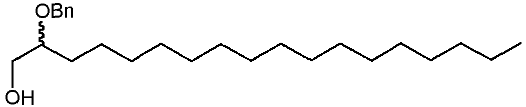 |
| J429E | 1-Phosphocholine-2-hydroxy-octadecane | 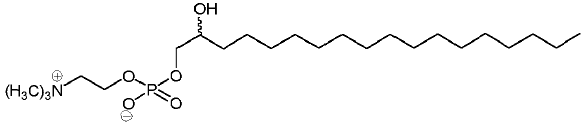 |
| J42C | N-(w-Hydroxypalmitoyl)-sphingosine | 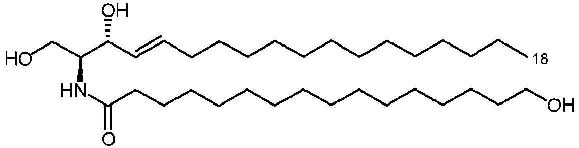 |
| J431Y | Boc-L-4-(Naphtalen-2-yl)-phenylalanine | 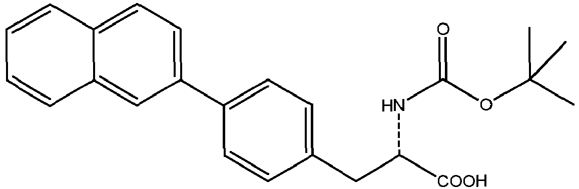 |
| J432E | (Z)-Hexadec-9-en-1-yl-phosphoryl choline | 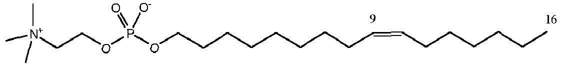 |
| J434E | Glucosamin-1-O-hexadecyl-2-O-methyl-glycerol | 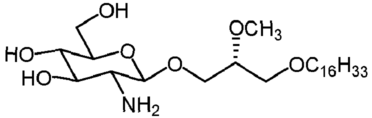 |
| J435E | 12-(Z)-Heneicosen-1-phosphate | 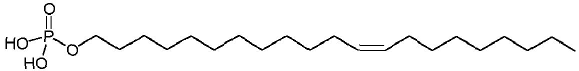 |
| J436E | 12-(Z)-Heneicosen-pyrophosphate | 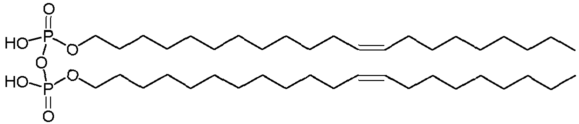 |
| J437Y | (2S)-Amino-3-(4-(naphthalen-6-yl)phenyl)propanoic acid hydrochloride | 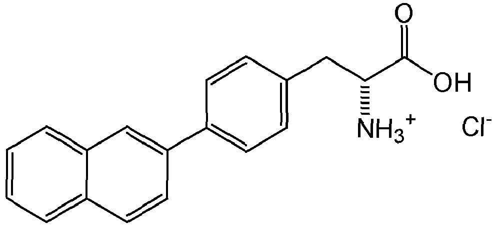 |
| J438Y | (2S)-Amino-3-(4-(naphthalen-6-yl)phenyl)-3-propanol hydrochloride | 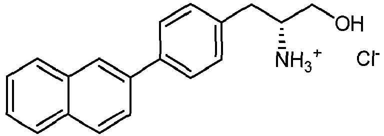 |
| J440E | (2-Amino-2-methylprop-1-yl)phosphoric acid mono-hexadecyl ester | 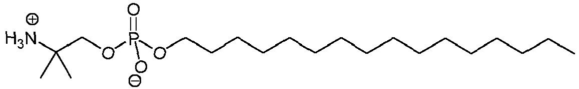 |
| J441E | (2-Amino-1-(R)-methyleth-1-yl)phosphoric acid mono-hexadecyl ester | 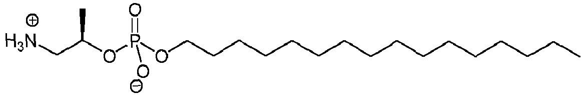 |
| J442E | (2-(R)-Aminoprop-1-yl)phosphoric acid mono-hexadecyl ester | 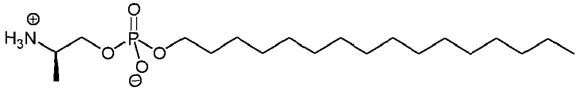 |
| J443E | Hexadecylphosphoglycerol | 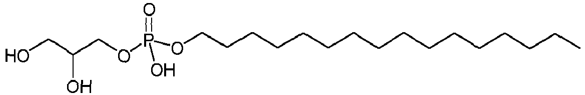 |
| J444C | 1-O-Glucosyl-2-acetamido-3-O-methyl-L-threo-sphingosine | 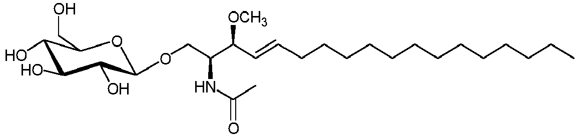 |
| J446E | 1-Hexadecylphosphonylcholine TFA adduct | 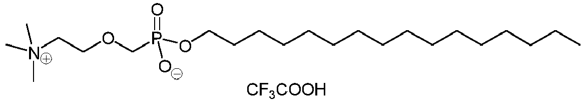 |
| J447C | 1-O-Glucosyl-2-N-hexadecanoyl-3-O-methyl-L-threo-sphingosine | 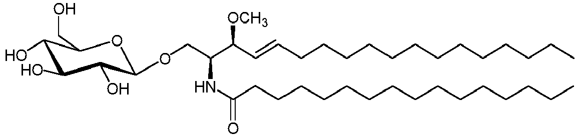 |
| J448E | 1-O-Octadecyl-2-O-methyl-sn -glycerol-3-O-pent-1-yl-trimethylamm. | 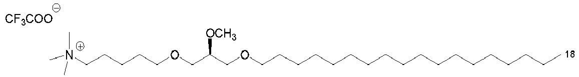 |
| J449E | 1-Stearoyl-2-hydroxy-sn-glycero-3-phosphocholine (18:0 Lyso PC) | 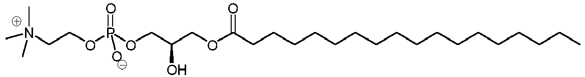 |
| J451E | 1-Oleoyl-2-hydroxy-sn-glycero-3-phosphocholine (18:1 Lyso PC) | 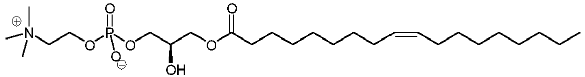 |
| J452C | 1-O-Glucosyl-3-O-methyl-2-N-octanoyl-L-threo-sphingosine | 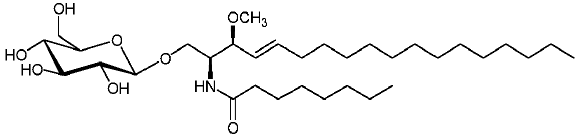 |
| J453E | Hexadecylpyridiniumphosphonate TFA adduct | 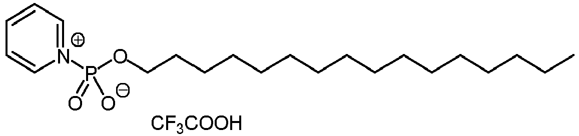 |
| J454E | Ethylene glycol monohexadecyl ether | 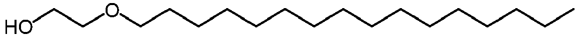 |
| J455Y | Emodin; 3-methyl-1,6,8-trihydroxyanthraquinone | 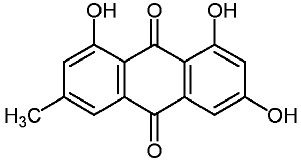 |
| J457Y | Theophylline-7-acetic acid-N-octadecylamide | 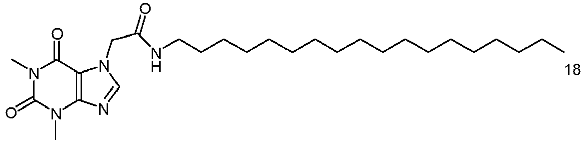 |
| J458Y | 1-Mesyl-3-hexadecylcarbamoylimidazolidin-2-one | 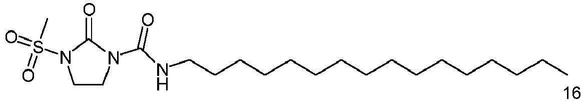 |
| J459Y | 3-Hexadecylcarbamoylimidazolidin-2-one | 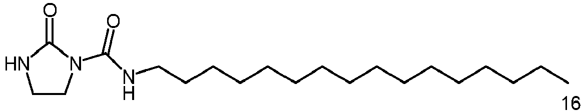 |
| J460Y | 2-[(Hexadecylcarbamoyl)-methoxy]acetic acid | 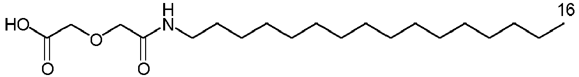 |
| J461Y | 2-[(Octadecylcarbamoyl)-methoxy]acetic acid | 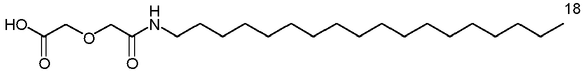 |
| J462E | Hexadecyl-(3-aza-4-oxo-5-oxa-heptane-1,8-diol) hydrogenphosphate | 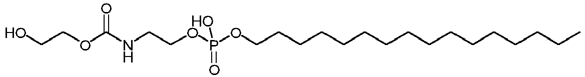 |
| J463E | 2-(2-Ethoxyethoxy)-ethyl hexadecyl hydrogenphosphate | 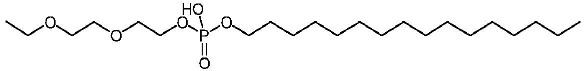 |
| J464Y | N-(1,3-Dihydroxy-2-(hydroxymethyl)propan-2-yl)palmitamide | 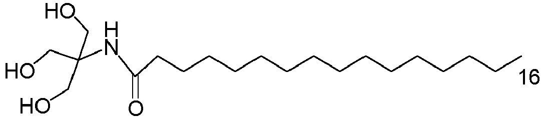 |
| J464Y | N-(l,3-Dihydroxy-2-(hydroxymethyl)propan-2-yl)palmitamide | 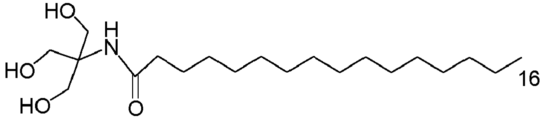 |
| J465E | 2-(2-Hydroxyethoxy)ethyl 2-hexadecylphosphorylethylcarbamate | 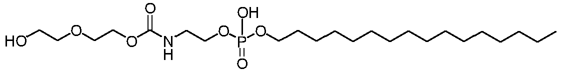 |
| J466E | Hexadecyl 2-ureidoethyl hydrogenphosphate | 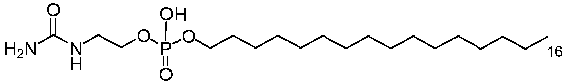 |
| J467E | 2-(Dicyclopropylamino)ethyl hexadecyl hydrogenphosphate TFA salt | 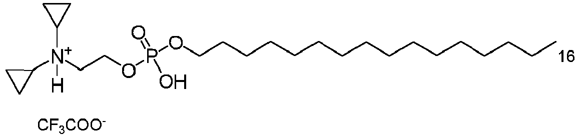 |
| J468Y | 2-(2-(Hexadecylamino)ethoxy)ethanol hyd rochloride | 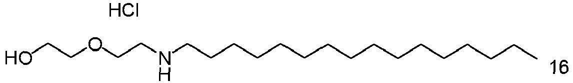 |
| J469Y | 2-(2-(Octadecylamino)ethoxy)ethanol hyd rochloride | 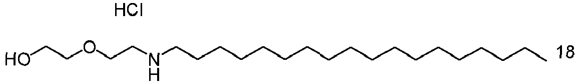 |
| J471Y | 4-Methylpyrimidin-2-[4-(palmitamido)benzenesulfonylamide] | 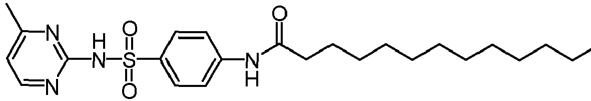 |
| J472Y | 2-Amino-N-hexadecylbenzamide | 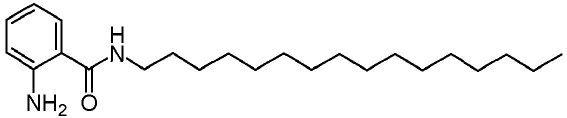 |
| J473C | 1-O-Glucosyl-2-acetamido-3-O-methyl-D-erythro-sphingosine | 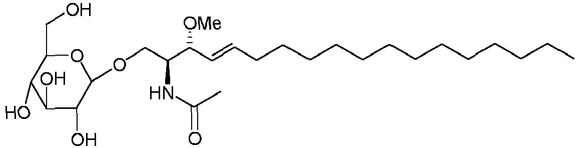 |
| J474C | 1-O-Lactosyl-2-N-hexadecanoyl-3-O-methyl-L-threo-sphingosine | 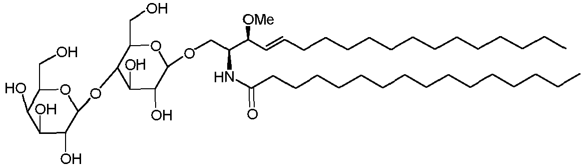 |
| J475E | (E)-Hexadec-9-en-1-yl-phosphorylcholine TFA salt | 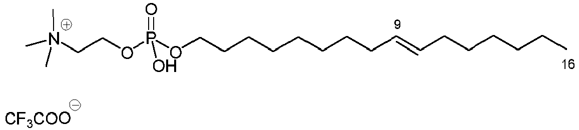 |
| J477C | 1-O-Glucosyl-3-O-methyl-2-N-butanoyl-L-threo-sphingosine | 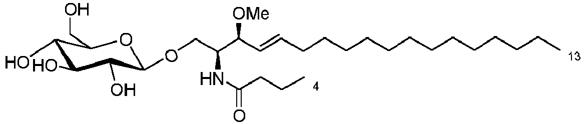 |
| J478C | 1-O-Glucosyl-3-O-methyl-2-N-hexanoyl-L-threo-sphingosine | 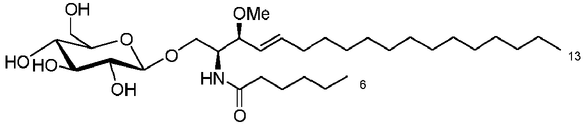 |
| J479Y | 1,1,3,3-Tetramethyl-2-palmitoylguanidine | 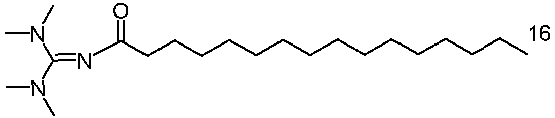 |
| J480Y | N-Hexadecyl-3-(piperidin-1-yl)propanamide | 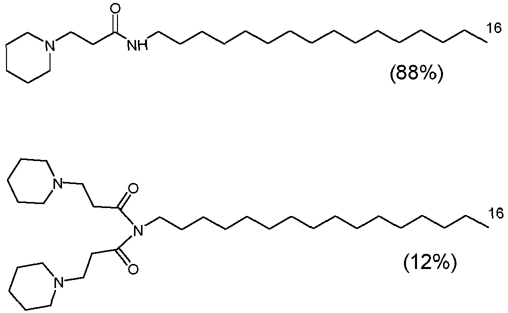 |
| J481E | protected glucofuranosyl hexadecylphosphate | 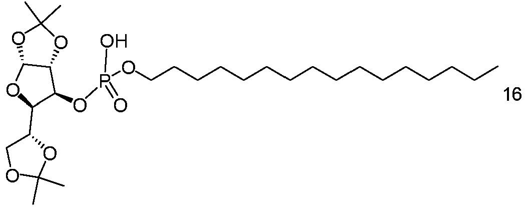 |
| J482C | 2-N-Acetyl-1-O-lactosyl-3-O-methyl-L-threo-sphingosine | 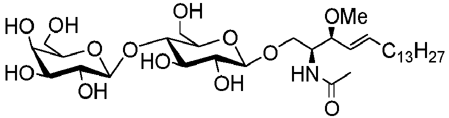 |
| J483C | 1-O-Lactosyl-3-O-methyl-2-N-octanoyl-L-threo-sphingosine | 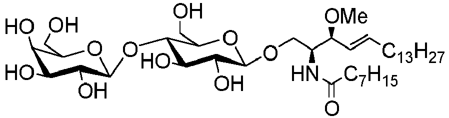 |
| J484C | 2-N-Acetyl-3-O-methyl-L-threo-sphingosine | 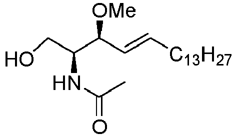 |
| J485E | (3-O-Glucosyl) hexadecylphosphate | 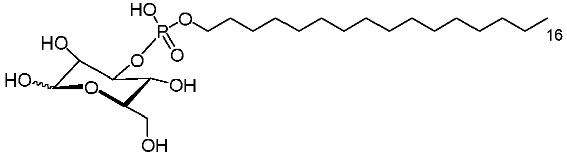 |
| J486Y | N-Hexadecyl shikimic carboxamide | 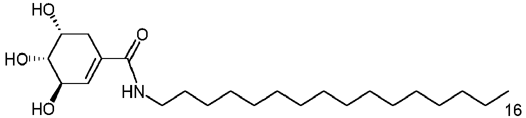 |
| J487Y | N,N-Dimethyl-N-hexadecyl-(2-hydroxyethyl)ammonium chloride | 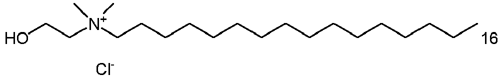 |
| J488Y | TRIS-hexadecylamine | 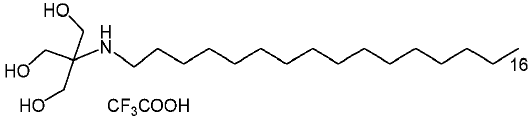 |
| J489Y | 2-[(Hexadecylamino)methyl]-aniline bis-TFA salt | 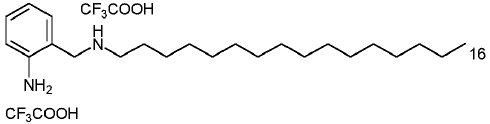 |
| J490Y | Hexadecylamine | 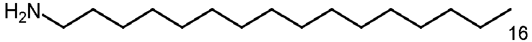 |
| J491C | 1-O-Glucosyl-3-O-methyl-L-th reo-sphingosine | 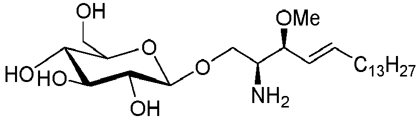 |
| J492C | 2-N-[(2-Glucosyloxy)acetyl]-3-O-methyl-L-threo-sphingosine | 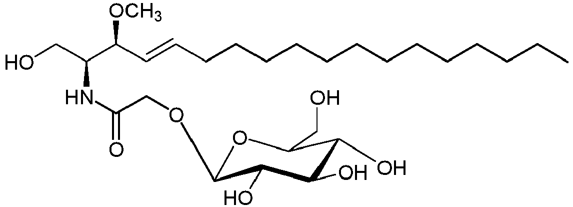 |
| J493Y | O-(Hexadecyl)ethanolamine TFA salt | 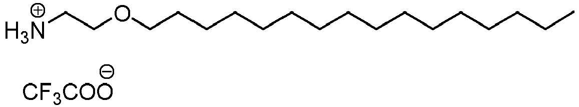 |
| J494E | Hexadecyl monoacetone glucose hydrogenphosphate | 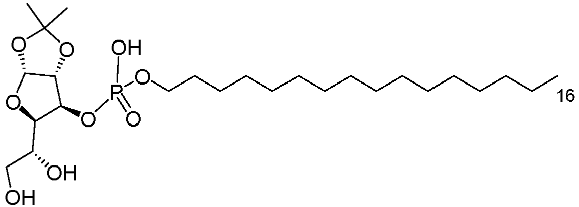 |
| J495Y | O,O'-Dihexadecyl-(+)-ethambutol bis-TFA salt | 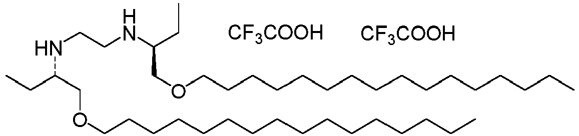 |
| J496Y | O-Hexadecyl-(+)-ethambutol bis-TFA salt | 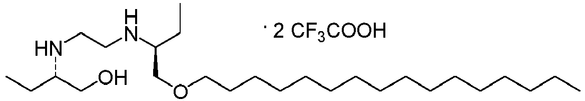 |
| J497Y | N-(Imidazolidin-2-ylidene)-N'-palmitoylhydrazide TFA salt | 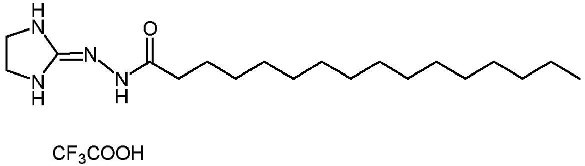 |
| J498Y | N-(1-Palmitoylimidazolidin-2-ylidene)-N'-palmitoylhydrazide TFA salt | 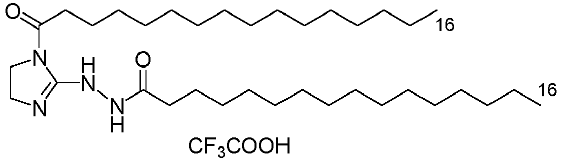 |
| J499Y | O-Hexadecansulfonyl choline TFA salt | 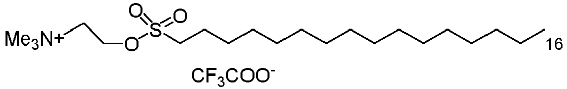 |
| J500E | Hexadecyl methyl phosphocholine TFA salt | 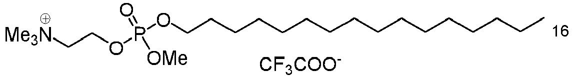 |
| J501E | Butylmethylphosphoch oline TFA salt | 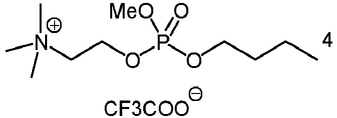 |
| J502E | Butylphosphocholine TFA salt | 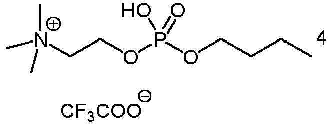 |
| J503E | (Methylpentaglycol)me thylphosphocholine TFA salt | 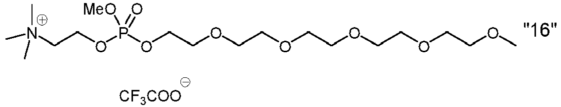 |
| J504E | (Methylpentaglycol)ph osphocholine TFA salt | 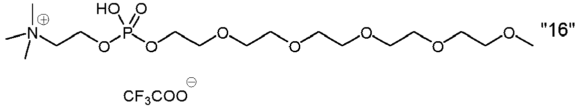 |
| J505Y | Dodecylamine dihydrogenphosphate | 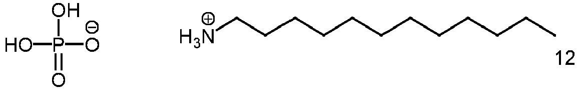 |
| J506Y | Hexadecylamine dihydrogenphosphate | 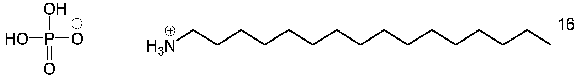 |
| J507Y | Dodecylamine trifluoroacetate | 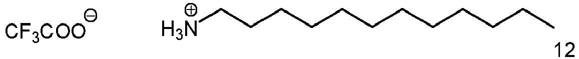 |
| J508Y | Hexadecylamine trifluoroacetate | 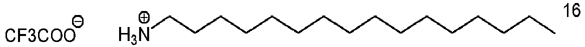 |
| J509Y | Dodecylamine hydrochloride | 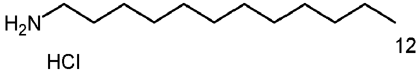 |
| J510Y | Hexadecylamine hydrochloride | 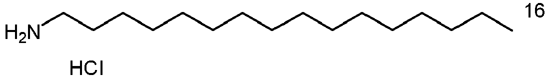 |
| J511Y | Dodecylamine dihydrogencitrate | 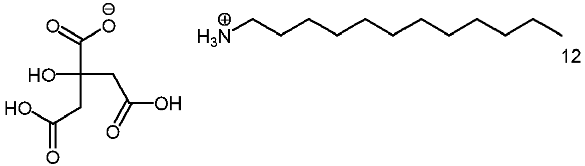 |
| J512Y | Hexadecylamine dihydrogencitrate | 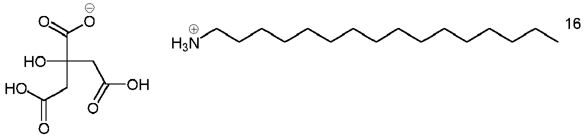 |
| J513E | (N,N-Diisopropyl)-geranylgeranyl-methyl phosphoramidite | 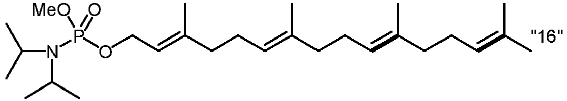 |
| J514E | 15-Methylmiltefosine TFA salt | 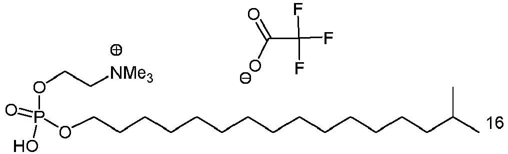 |
| J515Y | 3-N-(Hexadecylamino)-diacetoneglucofuranose TFA salt | 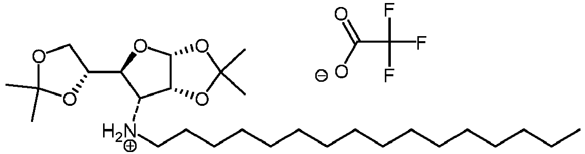 |
| J516E | 3,7,11,15-Tetramethylmiltefosine TFA salt | 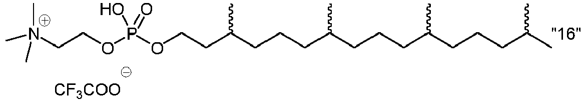 |
| J517Y | 3-(N,N-Dihexadecylamino)-diacetoneglucofuranose TFA salt | 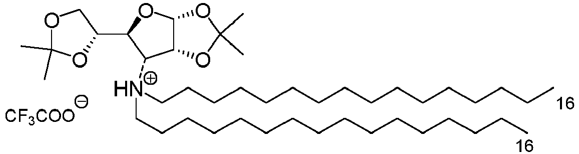 |
| J518Y | 4-(N-Hexadecansulfonylamino)-2,2,6,6-tetramethylpiperidine TFA salt | 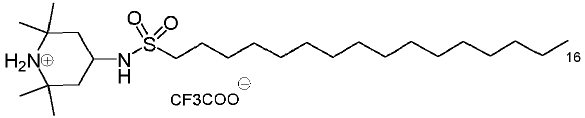 |
| J519Y | 3-(Hexadecylamino)-monoacetoneglucose TFA salt | 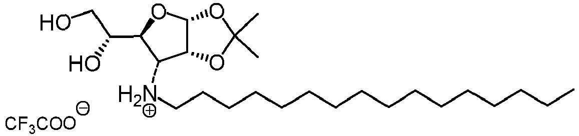 |
| J51C | N-(w-Amino-4,7,10,13-tetroxapentadecanoyl)-sphingosine | 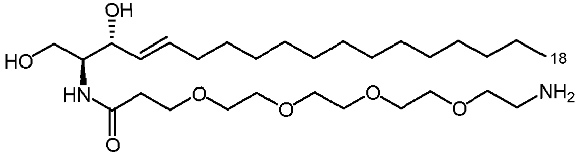 |
| J51C2 | N-(w-Amino-4,7,10,13-tetroxapentadecanoyl)-sphingosine | 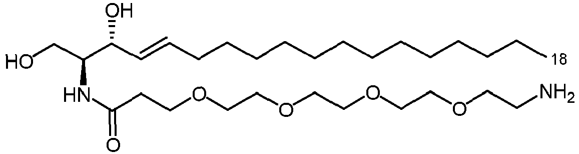 |
| J520Y | 3-(N,N-Dimethyloctadecylammonio)propanesulfonate | 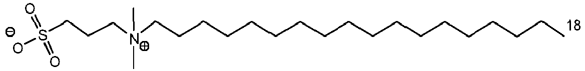 |
| J521Y | Hexadecyltrimethylammonium bromide | 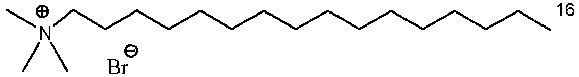 |
| J522E | Methyl cycloheptadecyl phosphocholine TFA salt | 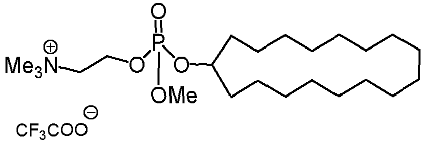 |
| J523E | Cycloheptadecyl phosphocholine TFA salt | 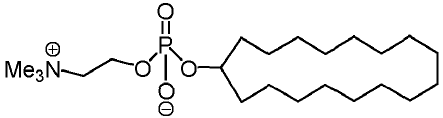 |
| J524Y | Thonozonium bromide | 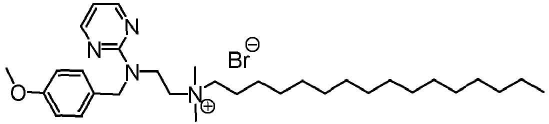 |
| J524Y | Thonozonium bromide | 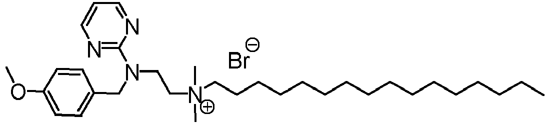 |
| J525E | Hexadecyl methyl 3-(piperidin-1-yl)propyl phosphate TFA salt | 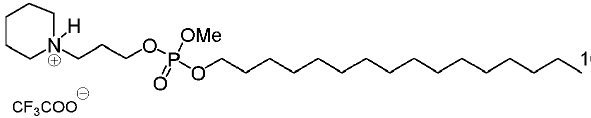 |
| J526E | Hexadecyl 3-(piperidin-1-yl)propyl phosphate TFA salt | 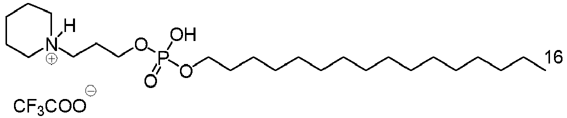 |
| J527E | N-(C10-16 alkyl)-N,N-dimethylglycine betaine | 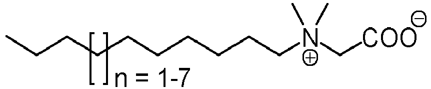 |
| | | >80% n = 5 (C₁₄) and n = 7 (C₁₆) |
| J528E | Hexadecylmannityl phosphocholine methyl ester TFA salt | 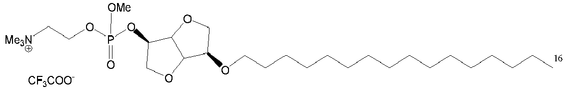 |
| J529E | Hexadecyl mannityl phosphocholine TFA salt | 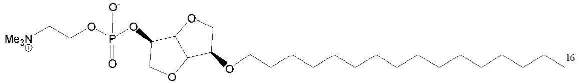 |
| J530Y | 3-(N,N-Dimethylpalmitylammonio) propansulfonate betaine | 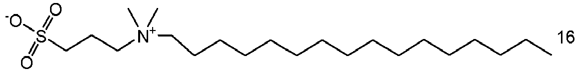 |
| J531Y | 5-(2-Aminoethyl)benzene-1,2,3-triol hexadecanesulfonamide | 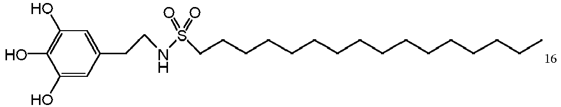 |
| J532Y | N-Hexadecylpyridinium chloride | 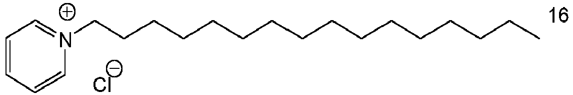 |
| J533Y | Dodecyl trimethylammonium bromide | 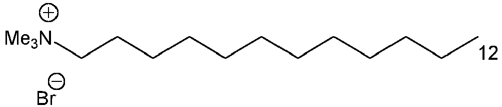 |
| J534E | 1-O-Tetradecyl-2-O-methyl-rac-glycero-3-phosphocholine | 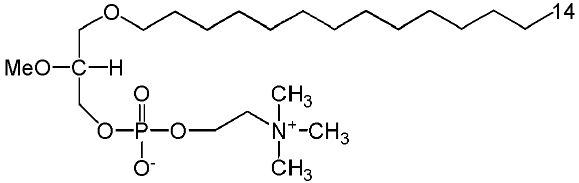 |
| J535E | Tetradecyl-phosphocholine | 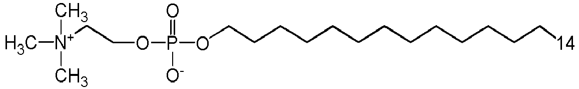 |
| J536Y | 2-(3,4,5-Trimethoxyphenyl)-N-methyl-N-hexadecansulfonyl ethylamine | 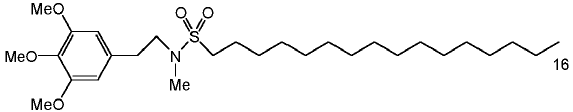 |
| J537E | Hentriacontan-16-yl-methyl phosphocholine TFA salt | 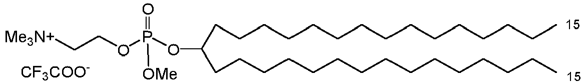 |
| J538E | O-Hecadecyl-O'-methyl-O"-tropinyl phosphate TFA salt | 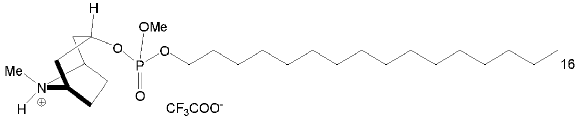 |
| J539C | 1-O-Glucosyl-3-O-methyl-2-N-trimethyl-L-threo-sphingosine TFA salt | 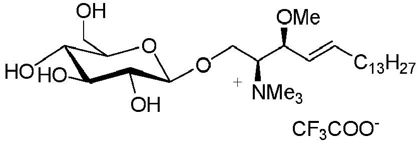 |
| J540C | 2-N-Acetyl-(2N,3O)-dimethyl-1-O-tetramethylglucosyl-sphingosine | 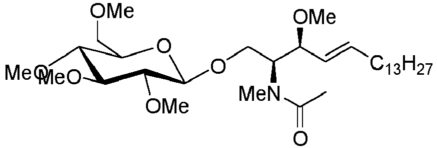 |
| J541E | Hexadecyl dicholinyl phosphate bis-TFA salt | 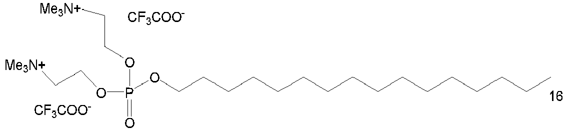 |
| J542E | 16-Hentriacontanyl phosphocholine | 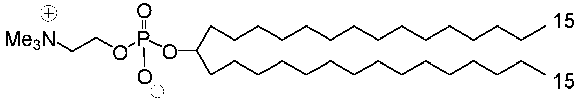 |
| J543Y | N,N-Bis-(N',N'-dimethylaminopropyl)-hexadecylsulfonamide bis-TFA | 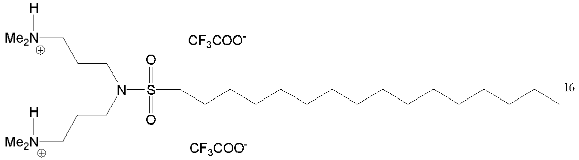 |
| J543Y | N,N-Bis-(N',N'-dimethylaminopropyl)-hexadecylsulfonamide bis-TFA | 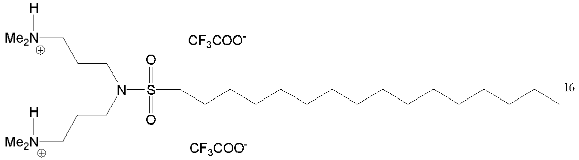 |
| J544E | O-Hexadecyl-O'-tropinylphosphate TFA salt | 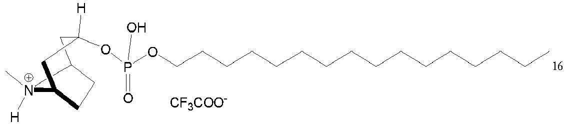 |
| J545E | Hexadecyl methyl (N-methyltropanium)phosphate TFA salt | 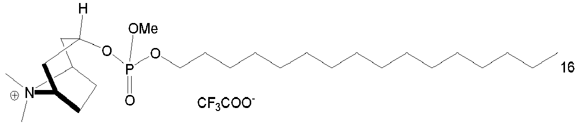 |
| J546Y | N',N'-[3-(N,N,N-trimethylammonium)-propyl]-hexadecylsulfonamide | 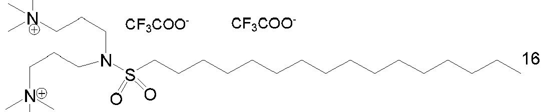 |
| J547E | Hexadecyl N-methyltropanium phosphate | 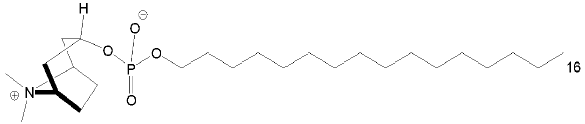 |
| J548Y | 1-Hexadecansulfonyl-4-[3-(morpholin-1-yl)propyl]-piperazine bis-TFA | 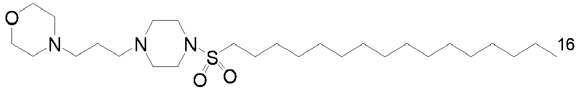 |
| J549E | Hexadecyl 2-(N,N,N-triethylammonium)ethyl methyl phosphate TFA | 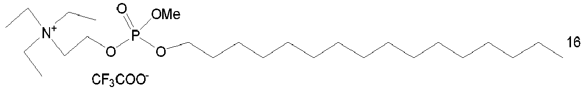 |
| J550E | Hexadecyl 2-(N,N,N-triethylammonium)ethyl phosphate | 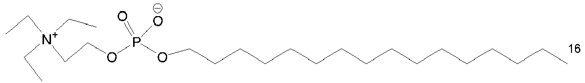 |
| J551E | Chloromethylmiltefosine | 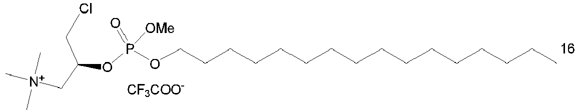 |
| J552E | Chloromiltefosine | 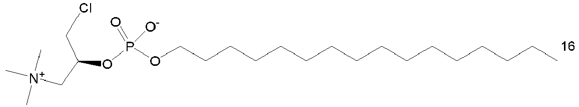 |
| J553E | (14-Cyclopentyl)-tetradecyl methyl phosphocholine | 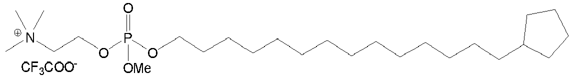 |
| J554Y | 3-(N,N-Dimethyl-N-dodecylamino)-propanesulfonate | 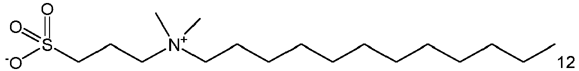 |
| J555Y | 3-(N,N-Dimethyl-N-tetradecylamino)-propanesulfonate | 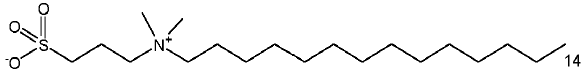 |
| J556Y | O-Hexadecyl-O'-(3-trimethylammoniumpropyl)-mannit TFA salt | 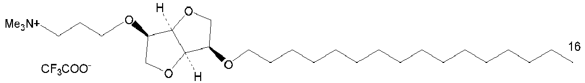 |
| J557E | Hexadecyl [2-(3-methylimidazolium)ethyl] methyl phosphate TFA salt | 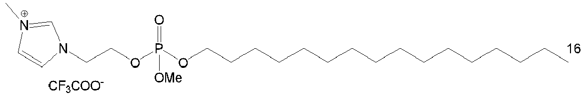 |
| J558E | (14-Cyclopentyl)-tetradecyl phosphocholine | 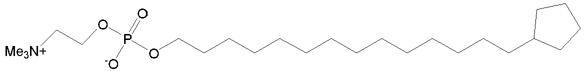 |
| J561Y | Dodecyl b-D-maltoside | 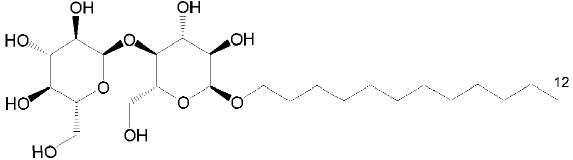 |
| J562Y | N,N-Dimethyldodecylamine N-oxide | 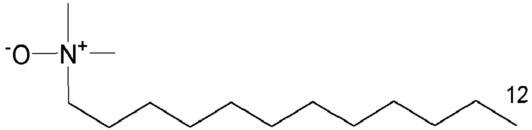 |
| J563Y | 2-(1H-Imidazol-1-yl)ethyl hexadecane-1-sulfonate TFA salt | 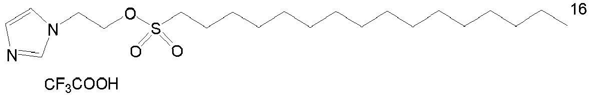 |
| J564Y | Octyl b-D-glucopyranoside | 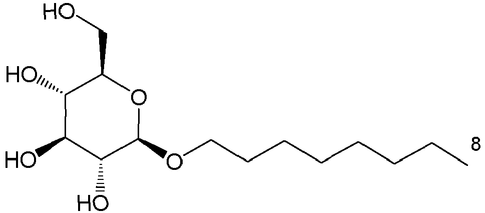 |
| J565Y | Sodium lauryl sulfate | 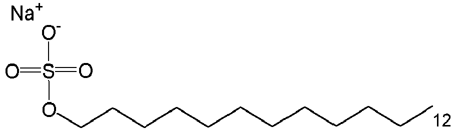 |
| J566E | 1,13-Bis(hexadecylcholinylp hosphoryl)-tetraethylene glycol bis-TFA | 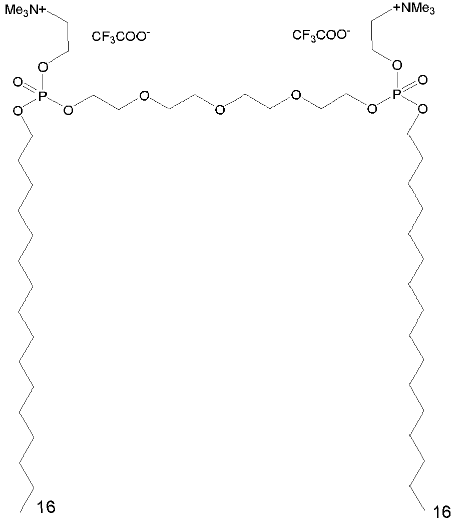 |
| J567E | Hexadecyl 2-(3-methylimidazolium) phosphate | 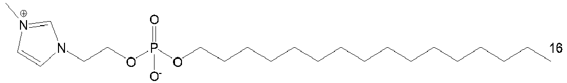 |
| J568E | 2-(Imidazol-1-yl)ethyl hexadecyl methyl phosphate | 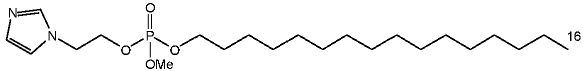 |
| J569E | Tris-amine-headed O-methyl miltefosine tris-TFA salt | 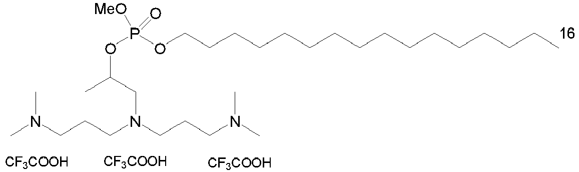 |
| J570E | 1,2-Dihexanoyl-sn-glycero-3-phosphocholine | 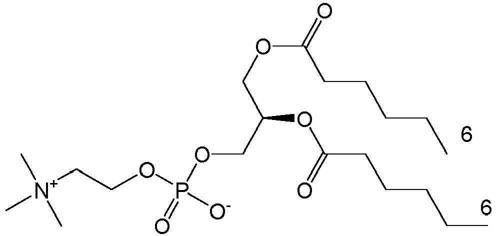 |
| J571E | Octylphosphocholine | 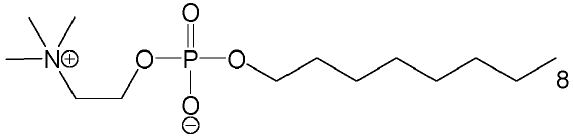 |
| J572E | Decylphosphocholine | 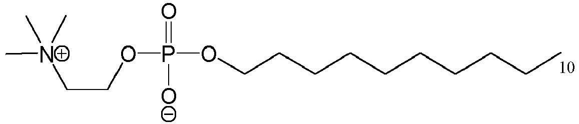 |
| J573Y | N,N-Dimethyl-N-tetradecylglycine | 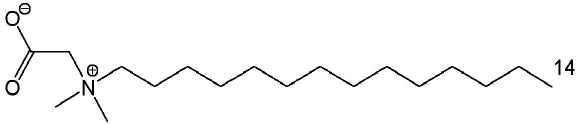 |
| J573Y | N,N-Dimethyl-N-tetradecylglycine | 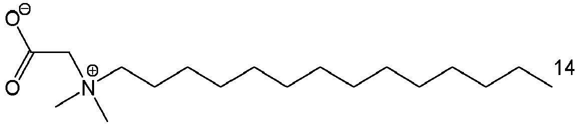 |
| J574E | 2-(1H-Imidazol-1-yl)ethyl hexadecyl phosphate | 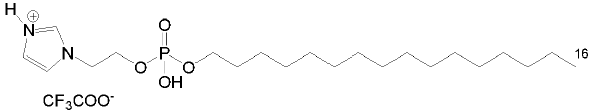 |
| J575Y | 1,1-N,N-Dimethyl-4-N'-hexadecansulfonyl piperazinium TFA salt | 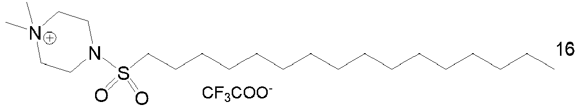 |
| J575Y | 1,1-N,N-Dimethyl-4-N'-hexadecansulfonyl piperazinium TFA salt | 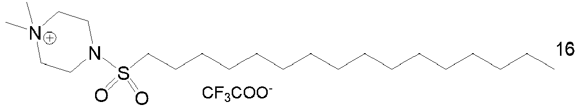 |
| J576E | Tris-amine-headed miltefosine tris-TFA salt | 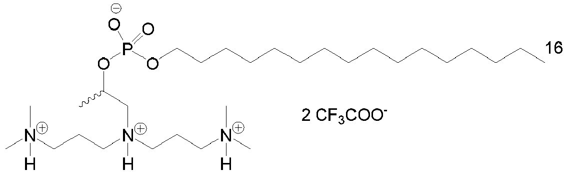 |
| J577E | O-Methyl edelfosine TFA salt | 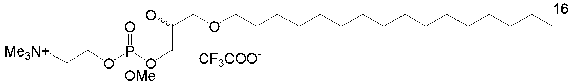 |
| J578Y | 4-(3-(4-(Hexadecylsulfonyl)-1-methylpiperazin-1-ium-1-yl)propyl)-4-methylmorpholin-4-ium, bis-TFA salt | 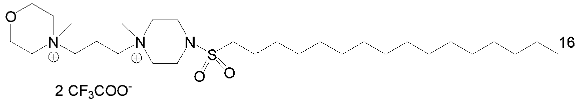 |
| J578Y | 4-(3-(4-(Hexadecylsulfonyl)-1-methylpiperazin-1-ium-1-yl)propyl)-4-methylmorpholin-4-ium, bis-TFA salt | 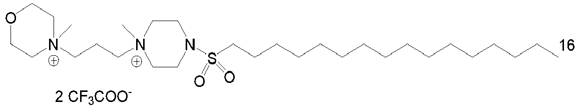 |
| J579Y | 1-Methyl-4-hexadecansulfonyl piperazine TFA salt | 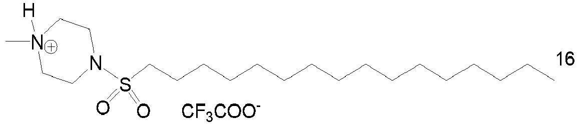 |
| J580Y | 2-(Dimethylamino)-N-(2,6-dimethylphenyl)-acetamide, Lidocain | 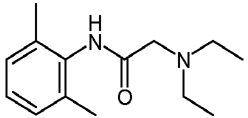 |
| J581C | 2-N-Acetyl-1-O-glucosyl-4,5-dihydro-L-threo-sphingosine | 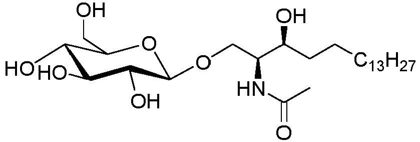 |
| J582C | (S)-4,5-Dihydro-4-((S,E)-1-methoxyhexadec-2-en-1-yl)-2-methyloxazole | 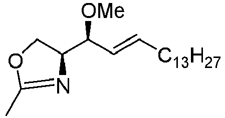 |
| J583E | Tris-ammonium-headed O-methyl miltefosine tris-TFA salt | 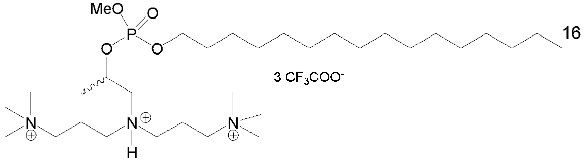 |
| J584E | O-Methyl O'-(4-nonylphenyl) phosphocholine TFA salt | 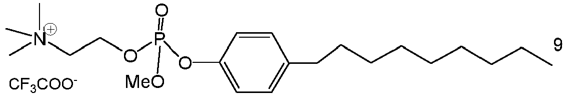 |
| J585Y | N-Hexadecyl-N,N-dimethyl-[(1R,2R,3R,5S)isopinoca mpheyl]amm. | 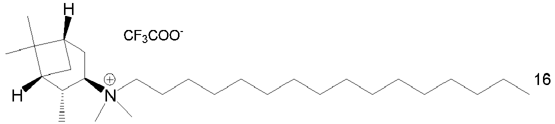 |
| J586E | 4-Nonylphenyl phosphocholine | 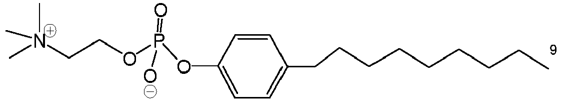 |
| J587E | Tris-amine-headed miltefosine bis-TFA salt | 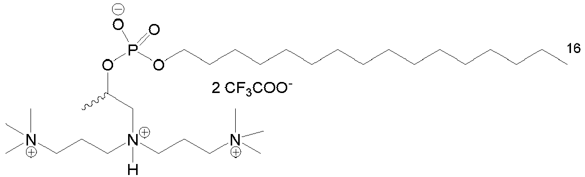 |
| J588Y | N-Hexadecyl-N,N-d imethyl-exo-norbornylammonium TFA salt | 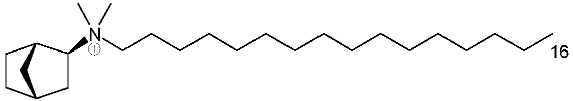 |
| J589E | O-[2-(hexadecyldimethylam monio)cyclopent-1-yl] O'-methyl PC bis-TFA | 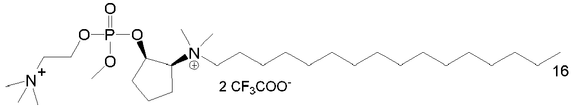 |
| J590Y | 2-(Hexadecyldimethylam monio)cyclopentanol TFA salt | 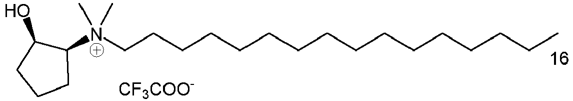 |
| J591E | 1,16-Bis(O-Methylphosphocholinyl )hexadecane bis-TFA salt | 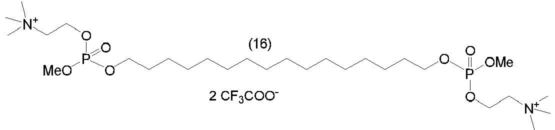 |
| J592C | 3-O-Methyl-(1-O-2-N-oxazolidin-2-one)-L-threo-sphingosine | 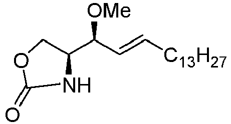 |
| J593C | 2-N-Acetyl-3-O-methyl-(1-O-2-N-oxazolidin-2-one)-L-threo-sphingosin | 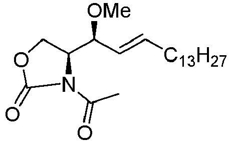 |
| J594E | O-[2-(hexadecyldimethylam monio)cyclopent-1-yl] PC bis-TFA | 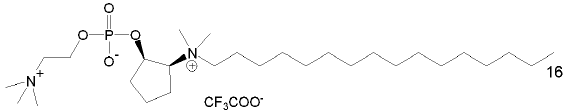 |
| J595E | O-[2-(N-hexadecanoylamino)cy clopent-1-yl]O'-methyl phosphocholine TFA | 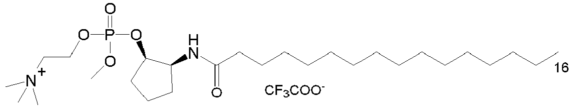 |
| J596C | 2-N-Acetyl-3-O-methyl-1-O-(tetra-pivaloyl-glucosyl)-L-th reo-sphingosin | 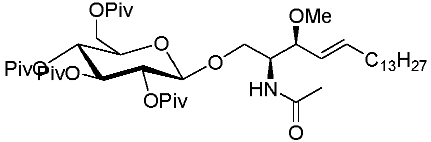 |
| J596C | 2-N-Acetyl-3-O-methyl-1-O-(tetra-pivaloyl-glucosyl)-L-th reo-sphingosin | 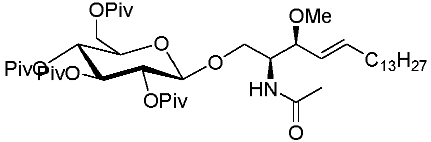 |
| J597E | O-Methyl-O'-[trans-4-nonylcyclohexan-1-yl] phosphocholine TFA | 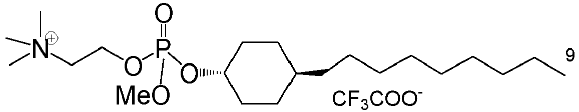 |
| J598E | O-[2-(N-hexadecanoylamino)cy clopent-1-yl] phosphocholine TFA | 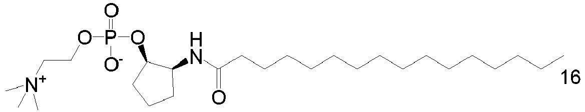 |
| J599E | O-Methyl-O'-[cis-4-nonylcyclohexan-1-yl] phosphocholine TFA | 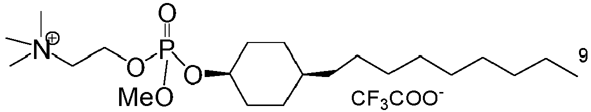 |
| J600E | 2-Hexadecyloxy-6,6-dimethyl-1,3,6,2-tetrahydrodioxaaza-phosphocinium TFA | 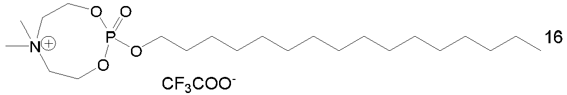 |
| J601Y | 2-Hexadecansulfonyl-5,5-dimethyl-2,5-diazabicycloheptanium TFA salt | 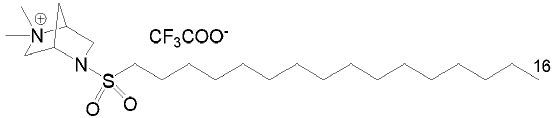 |
| J601Y | 2-Hexadecansulfonyl-5,5-dimethyl-2,5-diazabicycloheptanium TFA salt | 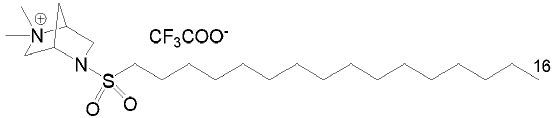 |
| J602E | O-[trans-4-nonylcyclohexan-1-yl] phosphocholine TFA | 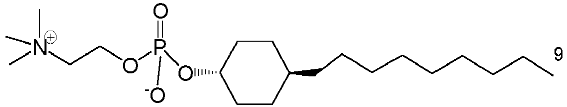 |
| J603E | O-[cis-4-nonylcyclohexan-1-yl] phosphocholine TFA | 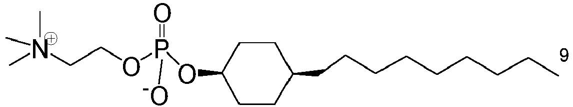 |
| J604E | O-[2-(hexadecyldimethylammonio)cyclopent-1-yl] PC Chloride | 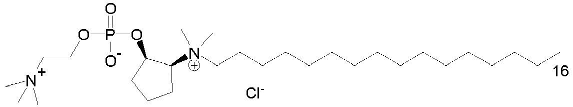 |
| J605Y | N,N-Dimethyloctadecylamine N-oxide | 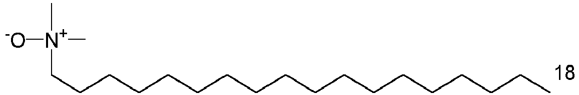 |
| J605Y | N,N-Dimethyloctadecylamine N-oxide | 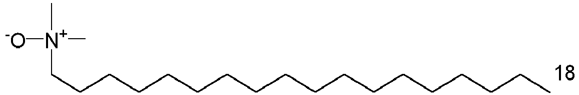 |
| J606E | O-Methyl-O'-hexadecyl-O"-(cis-1-trimethylammonio-cyclopent-2-yl) phosphate TFA | 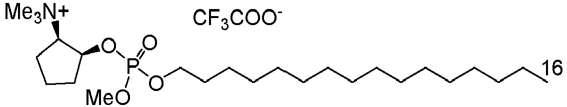 |
| J607Y | N-Tetradecyl-N,N-dimethylglycine-N'-methyl-D-glucosamide TFA | 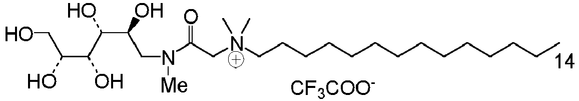 |
| J607Y(C I-BB-576) | N-Tetradecyl-N,N-dimethylglycine-N'-methyl-D-glucosamide chloride | 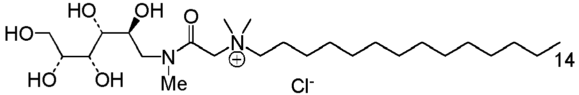 |
| J608E | O-Methyl-O'-[trans-4-nonylcyclohexan-1-yl] phosphocholine Chloride | 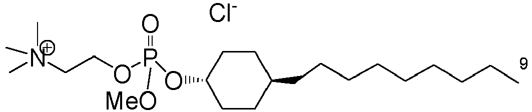 |
| J609E | O-Methyl-O'-(4-(trimethylammonio)benzyl)-O"-hexadecylphosphate TFA | 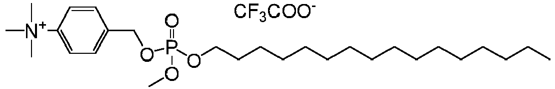 |
| J610Y | N-Hexadecyl-3-(trimethylammonio)propanoic amide TFA | 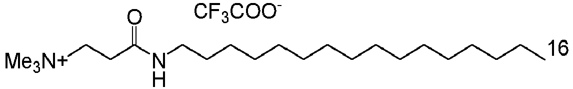 |
| J611Y | 1-O-Glucosyl-octadecanol | 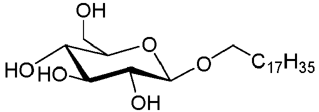 |
| J612C | 2-N-Acetyl-4,5-dihydro-1-O-glucosyl-3-O-methyl-L-threo-sphingosin | 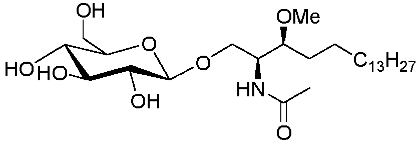 |
| J613C | 2-N-Methyl-3-O-methyl-(1-O-2-N-oxazolidin-2-one)-L-threo-sphingosin | 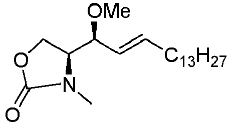 |
| J614Y | Hexadecyl dimethyl(tris(hydroxymethyl)methyl)ammonium TFA | 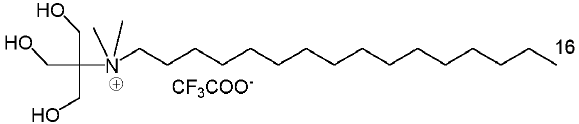 |
| J615E | O-Hexadecyl-O'-(cis-1-trimethylammonio-cyclopent-2-yl) phosphate | 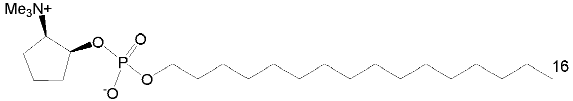 |
| J616E | O-Hexadecyl-O'-methyl-O"-(trans-1-trimethylammonio-cyclopent-2-yl) phosphate TFA | 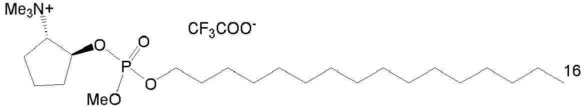 |
| J617E | 1,16-Bis(phosphocholinyl)hexadecane | 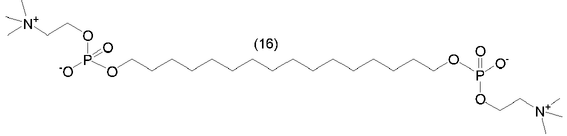 |
| J618E | O-[cis-4-nonylcyclohexan-1-yl] phosphocholine chloride | 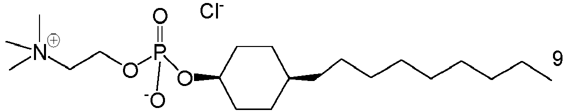 |
| J619Y | Trimethyl(4-tetradecylphenyl)ammonium TFA | 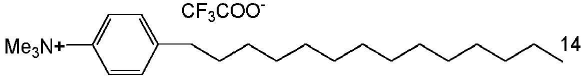 |
| J620Y | 1-Methyl-1-(2-dodecanoyl-ethyl)-piperidinium chloride | 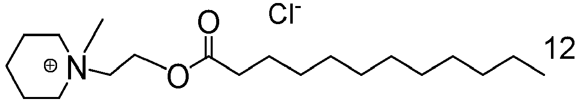 |
| J621E | O-Hexadecyl-O'-[trans-1-trimethylammonio-cyclopent-2-yl] phosphate | 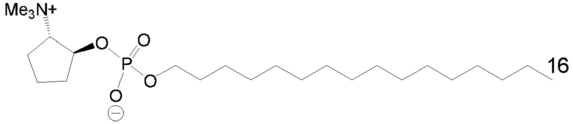 |
| J621E | O-Hexadecyl-O'-[trans-1-trimethylammonio-cyclopent-2-yl] phosphate | 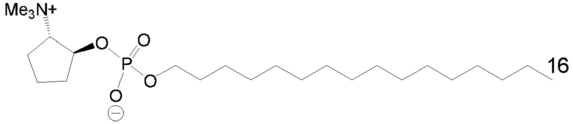 |
| J622Y | 1-Hexadecanoyloxy-2-tetramethylguanidinylc yclopentan TFA | 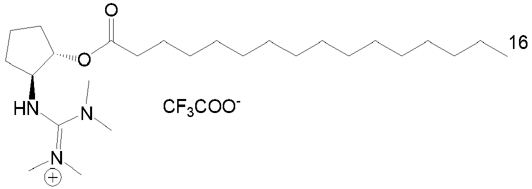 |
| J623E | O-Cholinyl-O'-methyl-O"-[3-(4-nonylphenyl)propan-1-yl]phosphate TFA | 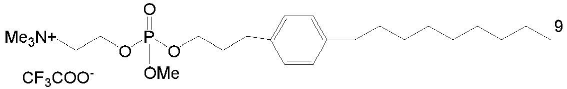 |
| J624E | O-Cholinyl-O'-methyl-O"-[6-(4-pentylphenyl)hexan-1-yl]phosphate TFA | 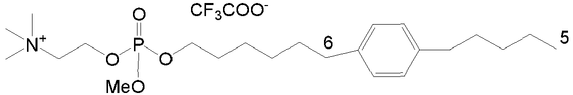 |
| J625E | O-Hexadecyl-O'-methyl-O"-[(R)-2-(trimethylammonio)-2-phenylethan-1-yl]phosphate TFA | 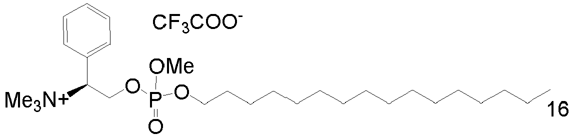 |
| J626Y | 1-(2-Dodecanoyloxy-3-(2-oxo-1-pyrrolidinyl)propyl)-1-methylpiperidinium TFA | 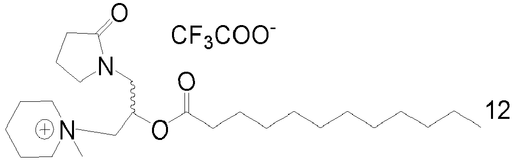 |
| J627E | O-Hexadecyl-O'-methyl-O"-(4-trimethylammoniobut-1-yl)phosphate TFA | 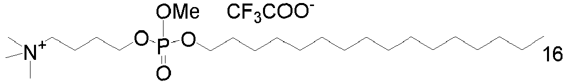 |
| J628E | O-Hexadecyl-O'-(4-(trimethylammonio)be nzyl)phosphate | 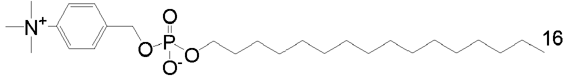 |
| J629E | O-Cholinyl-O'-[3-(4-nonylphenyl)propan-1-yl]phosphate | 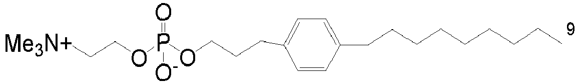 |
| J630E | O-Hexadecyl-O'-[(R)-2-(trimethylammonio)-2-phenylethan-1-yl]phosphate | 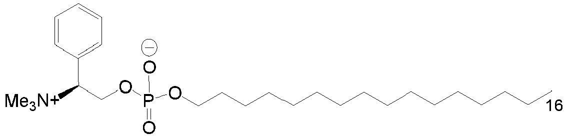 |
| J631Y | 1-N-Acetyl-Octadecylamin | 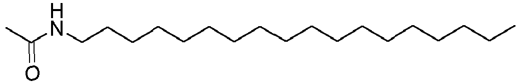 |
| J632E | 1,20-Bis(O-methyl phosphocholinyl)eicosane bis-TFA | 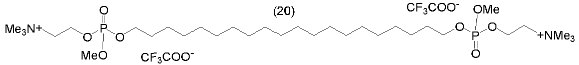 |
| J633E | O-Hexadecyl-O'-(4-trimethylammoniobut-1-yl)phosphate | 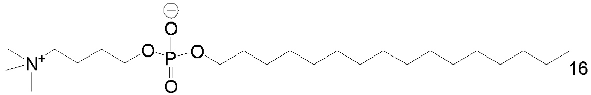 |
| J634E | O-Cholinyl-O'-methyl-O"-[3-(4-nonylcyclohexyl)propa n-1-yl]phosphate TFA | 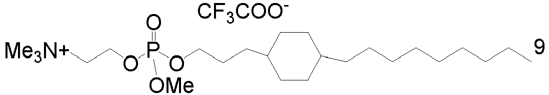 |
| J635E | O-Cholinyl-O'-methyl-O"-[6-(4-pentylcyclohexyl)hexan-1-yl]phosphate TFA | 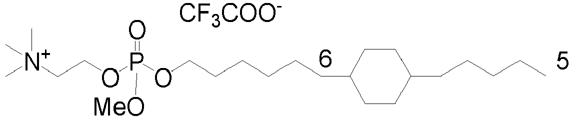 |
| J636Y | 2-(R)-N-Actamid-1-O-glucosyl-hyd roxyoctadecan-2-yl | 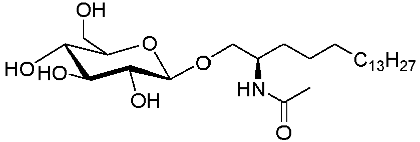 |
| J637Y | trans-2-Hexadecyldimethylammoniocyclopentanol, TFA | 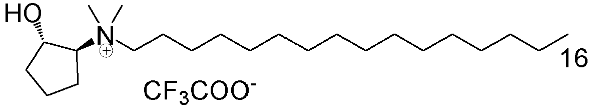 |
| J638E | O-Hexadecyl-O'-methyl-O"-[(S)-2-(trimethylammonio)-2-phenylethan-1-yl]phosphate, TFA | 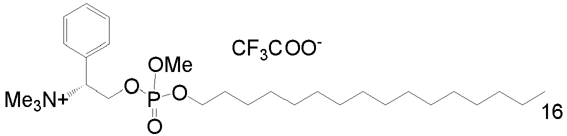 |
| J639E | 1,20-Bis(phosphocholinyl)eicosane | 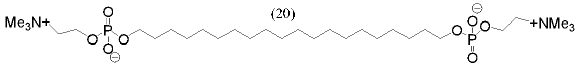 |
| J640E | O-Cholinyl-O'-[6-(4-pentylphenyl)hexan-1-yl]phosphate | 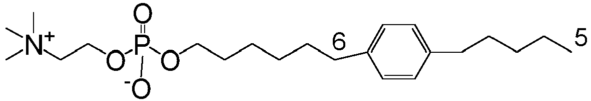 |
| J641E | O-Cholinyl-O'-[3-(4-nonylcyclohexyl)propan-1-yl]phosphate | 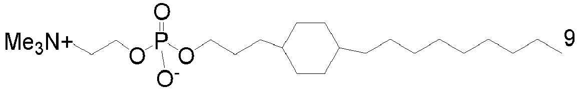 |
| J642E | O-Cholinyl-O'-methyl-O"-[3-(4-nonylcyclohexyl)propan-1-yl]phosphate Chloride | 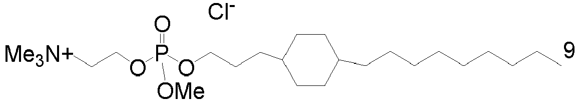 |
| J643Y | 2-(R)-N-Acetamid-1-hydroxyocatdecan-2-yl | 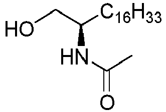 |
| J644Y | N-Hexadecanoyl-N-methyl-(L)-arginine,TFA | 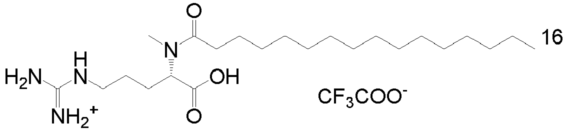 |
| J645E | O-Hexadecyl-O'-methyl-O"-[(6-trimethylammonio)hex-1-yl]phosphate TFA | 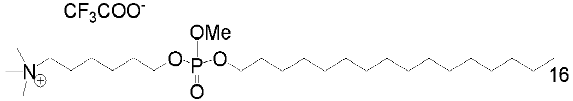 |
| J646E | O-Hexadecyl-O'-[(S)-2-(trimethylammonio)-2-phenylethan-1-yl]phosphate | 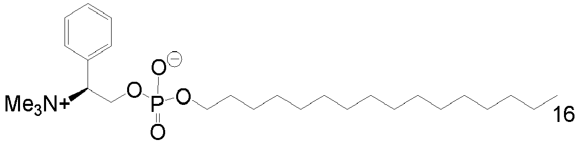 |
| J647E | O-[trans-2-(hexadecyldimethylammonio)cyclopent-1-yl]-O'-methyl phosphocholine bisTFA | 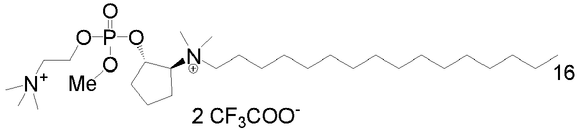 |
| J648E | O-Cholinyl-O'-[6-(4-pentylcyclohexyl)hexan-1-yl]phosphate | 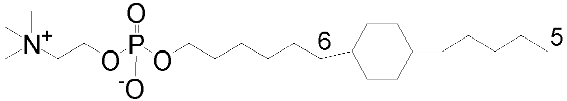 |
| J649Y | 1-(2-Hexadecanoyloxy-3-(2-oxo-1-pyrrolidinyl)propyl)-1-methylpiperidinium TFA | 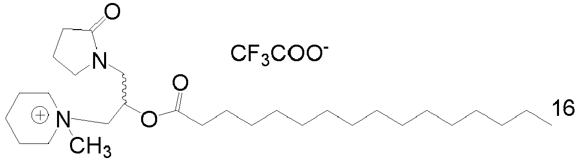 |
| J650Y | 1-[2-Hexadecanoyloxy-3-(2-oxo-1-pyrrolidinyl)propyl]piperidinium TFA | 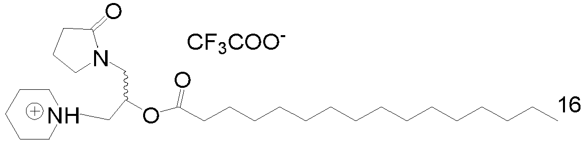 |
| J651E | O-Cholinyl-O'-[3-(4-nonylphenyl)propan-1-yl]phosphate (TFA-free) | 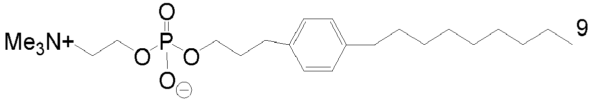 |
| J651E | O-Cholinyl-O'-[3-(4-nonylphenyl)propan-1-yl]phosphate (TFA-free) | 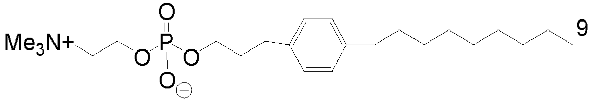 |
| J652E | O-[trans-4-nonylcyclohexan-1-yl]phosphocholine (TFA-free) | 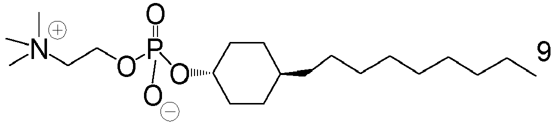 |
| J652E | O-[trans-4-nonylcyclohexan-1-yl] phosphocholine (TFA-free) | 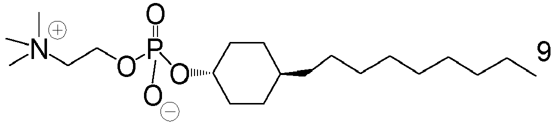 |
| J653Y | N-Hexadecyl-N-Methyl-3-(trimethylammonio)propanoic amide TFA | 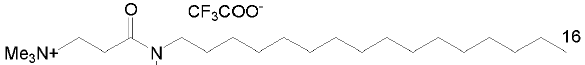 |
| J654Y | 1-[2-Decanoyloxy-3-(2-oxo-1-pyrrolidinyl)propyl]-1-methylpiperidinium TFA | 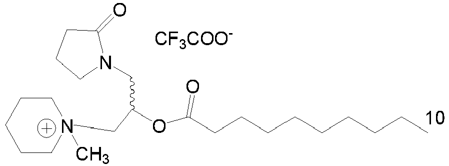 |
| J655Y | 1-[2-Tetradeca noyloxy-3-(2-oxo-1-pyrrolidinyl)propyl]-1-methylpiperidinium TFA | 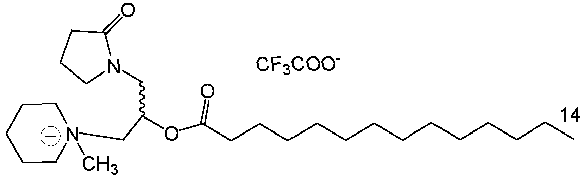 |
| J656Y | 1-(2-Dodecanoyloxy-3-(2-oxo-1-pyrrolidinyl)propyl)-1-methylpiperidinium chloride | 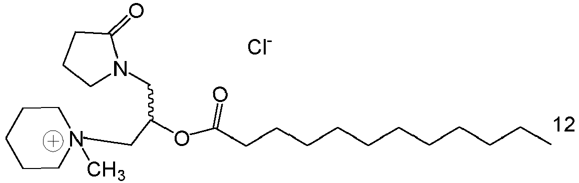 |
| J657E | O-Hexadecyl-O'-[(6-trimethylammonio)hex-1-yl]phosphate | 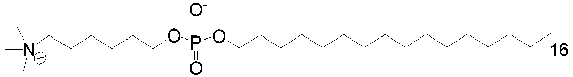 |
| J658E | O-[(1S,2S)-2-(hexadecyldimethylammonio)cyclopent-1-yl]-phosphocholine TFA | 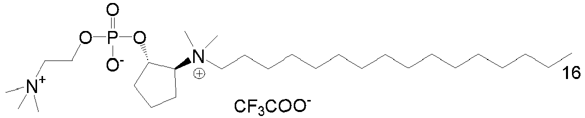 |
| J659Y | H-Leu-Gly-Glu-Pro-Gln-Leu-Palm | 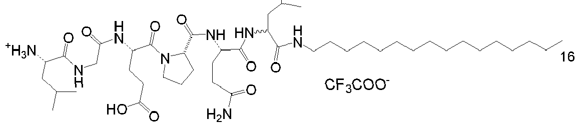 |
| J660Y | 1-((2S)-2-Dodecanoyloxy-3-(2-oxo-1-pyrrolidinyl)propyl)-1-methylpiperidinium TFA | 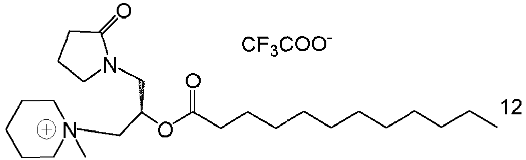 |
| J661Y | 1-((2R)-2-Dodecanoyloxy-3-(2-oxo-1-pyrrolidinyl)propyl)-1-methylpiperidinium TFA | 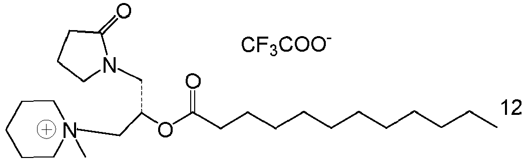 |
| J662C | (4S,5S)-4-(hydroxymethyl)-5-pentadecyloxazoldin-2-on | 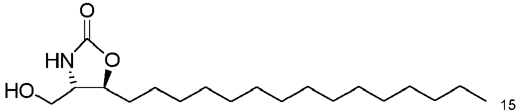 |
| J662C | (4S,5S)-4-(hydroxymethyl)-5-pentadecyloxazoldin-2-on | 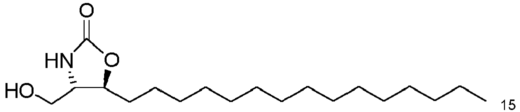 |
| J663Y | 1-(2-Dodecanoyloxy-3-(2-oxo-1-pyrrolidinyl)propyl)-1-methyl-((S)-2-(methoxymethyl)pyrrolidinium) TFA | 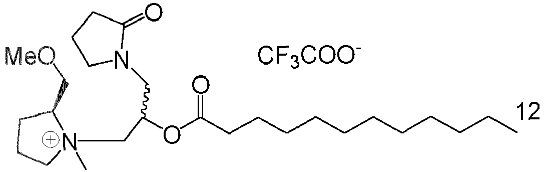 |
| J664Y | 1-(3-(3',3'-Dimethyl-3'-azonia-heptadecanoylamino)propyl)pyrrolidin-2-one TFA | 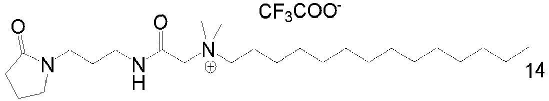 |
| J665Y | 1-(2-Dodecanoyloxy-3-(2-oxo-1-pyrrolidinyl)propyl)-1-methylmorpholinium TFA | 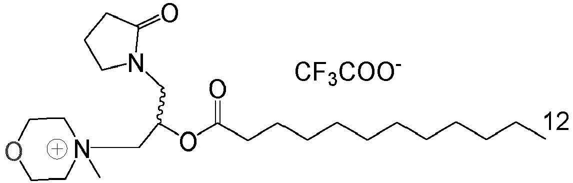 |
| J666Y | 1-[2-Dodecanoyloxy-3-(2-oxo-1-pyrrolidinyl)propyl]-1,3,5-trimethylpiperidinium TFA | 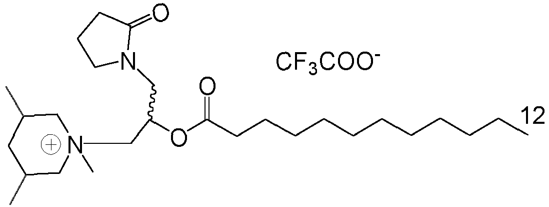 |
| J667Y | 1-(2-Dodecyloxy-3-(2-oxo-1-pyrrolidinyl)propyl)-piperidinium TFA | 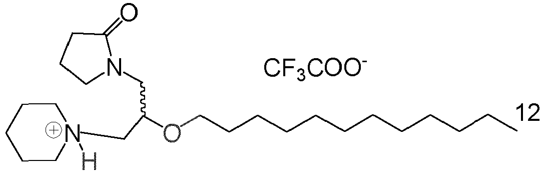 |
| J668Y | 1-(2-Dodecanoyloxy-3-(N-acetyl-N-methylamino)propyl)-1-methylpiperidinium TFA | 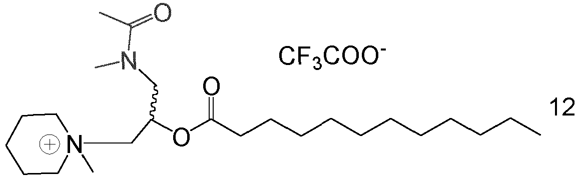 |
| J669Y | 1-[2-Dodecanoyloxy-3-(2-oxo-1-pyrrolidinyl)propyl]-1-methylazocanium TFA | 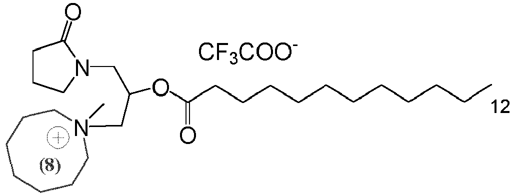 |
| J670Y | 1-[2-Tredecanoyloxy-3-(2-oxo-1-pyrrolidinyl)propyl]-1-methylpiperidinium TFA | 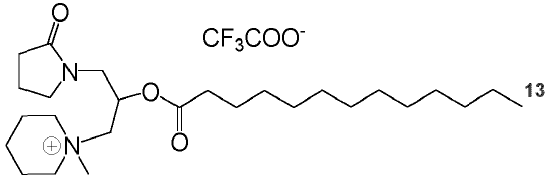 |
| J671Y | 1-(2-Dodecyloxy-3-(2-oxo-1-pyrrolidinyl)propyl)-1-methylpiperidinium TFA | 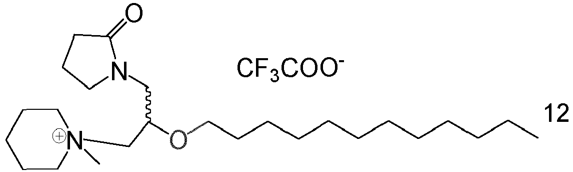 |
| J672Y | 1-[2-Dodecanoyloxy-3-(2-oxo-1-pyrrolidinyl)propyl]azepanium TFA | 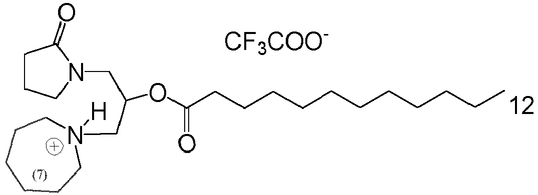 |
| J673Y | 1-(2-Dodeca noyloxy-3-(2-oxo-1-pyrrolidinyl)propyl)-1,2,6-trimethylpiperidinium TFA | 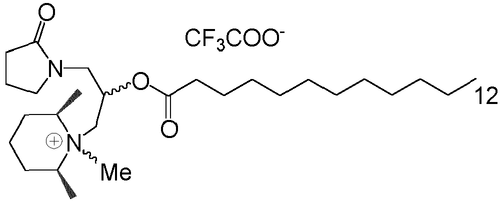 |
| J674Y | 1-Trimethylammonio-2-dodecanoyloxy-3-(2-oxo-1-pyrrolidinyl)propane TFA | 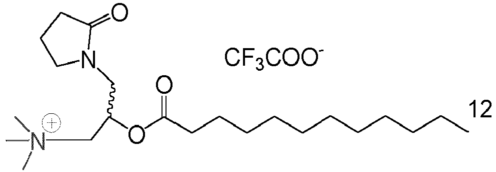 |
| J675Y | 1-(N,N-Dimethyldodecylammonio)-3-(2-oxo-1-pyrrolidinyl)propane TFA | 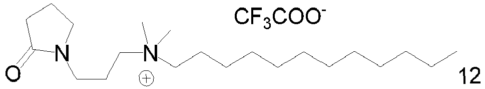 |
| J676Y | 1-[2-Dodecyloxy-3-(2-oxo-1-pyrrolidinyl)propyl]-1-methylazepanium TFA | 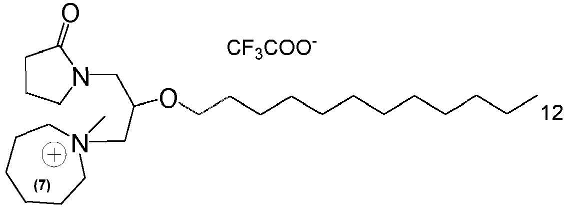 |
| J677Y | 1-(2-Dodeca noyloxy-3-(2-oxo-1-pyrrolidinyl)propyl)-1-methyl-(2R)-2-methoxymethylpyrrolidinium TFA | 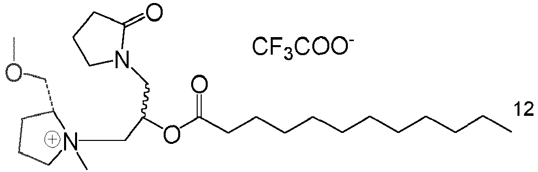 |
| J678Y | 2-Hydroxy-3-(N,N-Dimethylmyristylammo nio)propanesulfonate | 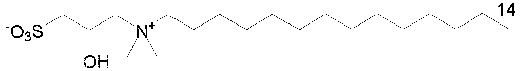 |
| J679Y | 1-[2-Dodecanoyloxy-3-(2-oxo-1-pyrrolidinyl)propyl]-1-methyl-4-carboxamido-piperidinium TFA | 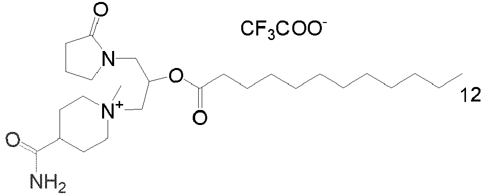 |
| J680Y | 1-[2-Dodeca noyloxy-3-(2-oxo-1-piperidinyl)propyl]-1-methylpiperidinium TFA | 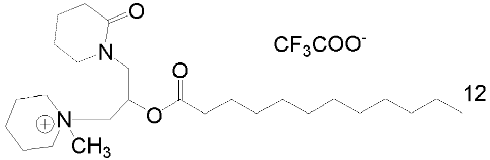 |
| J681Y | 1-(2-Undecanoyloxy-3-(2-oxo-1-pyrrolidinyl)propyl)-1-methylazepanium TFA | 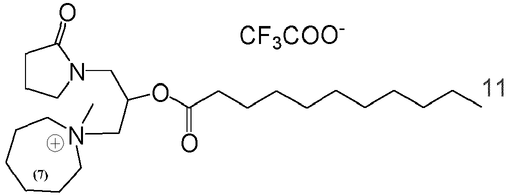 |
| J682Y | 1-[2-Tredeca noyloxy-3-(2-oxo-1-pyrrolidinyl)propyl]-1-methylazepanium TFA | 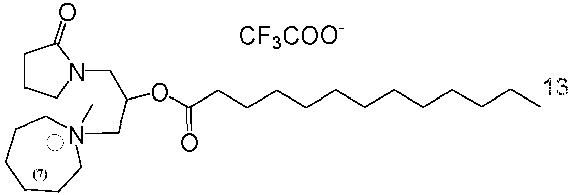 |
| J683Y | 1-(2-Dodeca noyloxy-3-(2-oxo-1-pyrrolidinyl)propyl)-1-methylazepanium TFA | 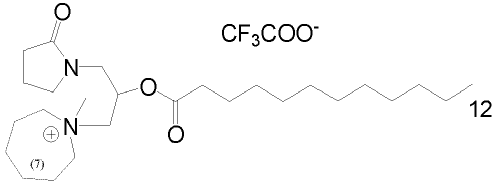 |
| J684Y | 1-(N,N-Dimethyldodecylammo nio )-2-hydroxy-3-(2-oxo-1-pyrrolidinyl)propane TFA | 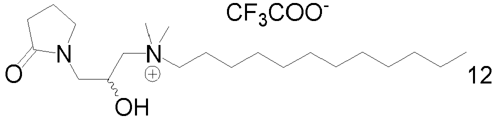 |
| J685Y | 1-(1-Methylazepanium-1-yl)pentadecane-3-sulfonate | 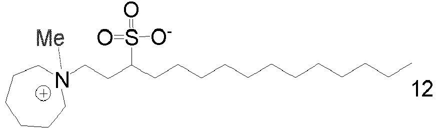 |
| J686Y | 1-(Azepanium-1-yl)pentadecane-3-sulfonate | 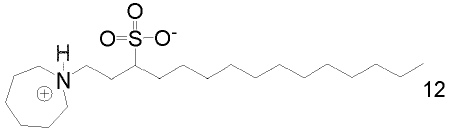 |
| J687Y | 1-[2-Dodeca noyloxy-3-(2-aza-bicyclo[2. 2.1]heptan-3-one-2-yl)propyl]-1-methylazepanium TFA | 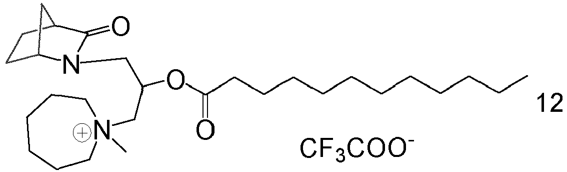 |
| J688Y | 1-[2-Dodeca noyloxy-3-(2-oxo-5-methyl-1-pyrrolidinyl)propyl]-1-methylazepanium TFA | 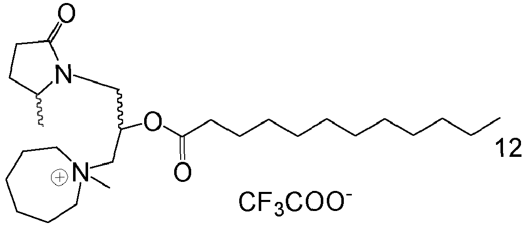 |
| J689Y | 1-(2-Tridecyl-3-sulfonatopropyl)azepan ium | 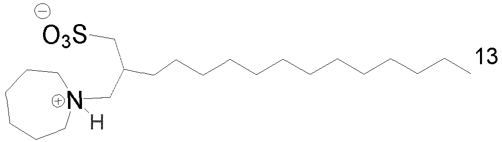 |
| J690C | 1-O-Glucosyl-3-O-methyl-2-N-methylcarbamat-L-threo-sphingosin | 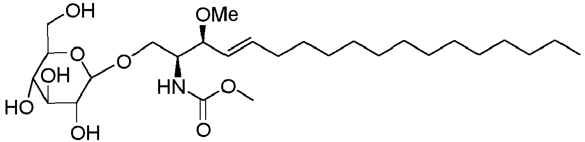 |
| J691Y | 1-(1-Azepanium-1-yl)heptadecane-3-sulfonate | 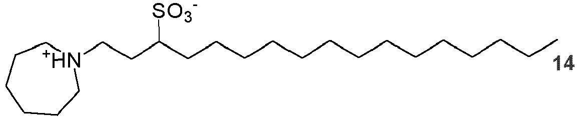 |
| J692Y | 2-Hydroxy-3-(N,N-Dimethyldodecylammonio)propanesulfonate | 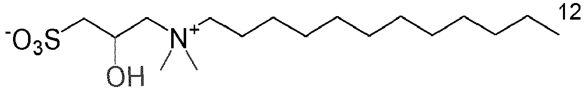 |
| J693Y | 2-Acetoxy-3-(N,N-Dimethyltetradecylammonio)propanesulfonate | 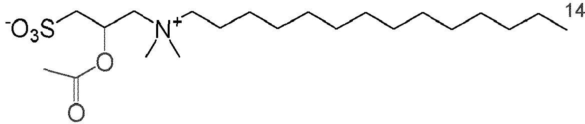 |
| J694Y | 4-(N,N-Dimethylmyristylammonio)butanesulfonate | 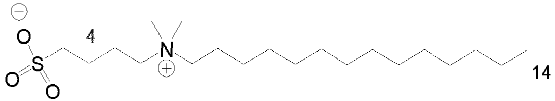 |
| J695Y | 1-(2-Tetradecanoyloxy-3-sulfonatopropyl)-1-methylazepanium | 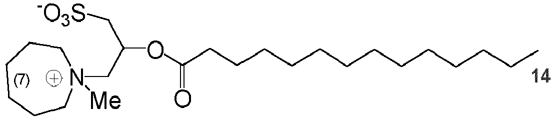 |
| J696Y | 1-(2-Dodeca noyloxy-3-sulfonatopropyl)-1-methylazepanium | 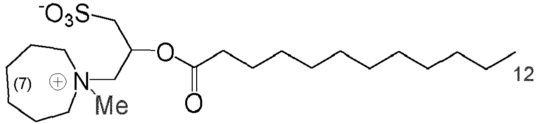 |
| J697Y | 1-(N,N-Dimethyldodecylammonio)-2-methoxy-3-(2-oxo-1-pyrrolidinyl)propane TFA | 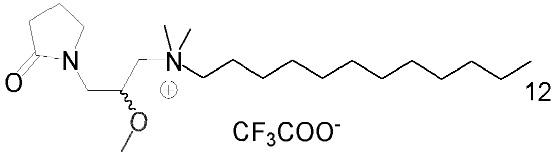 |
| J698Y | 1-(N,N-Dimethyldodecylammonio)-2-acetoxy-3-(2-oxo-1-pyrrolidinyl)propane TFA | 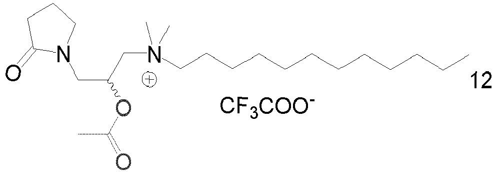 |
| J699Y | 2-Hydroxy-3-(N-Methyldodecylammonio)propanesulfonate | 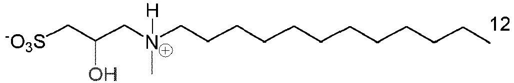 |
| J6C | N-cis-Octadecenoylsphingosine | 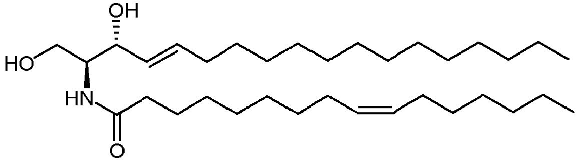 |
| J700E | O-Hexadecyl-O'-[1-(2-oxo-pyrrolidin-1-yl)-3-(1-methylpiperidinium-1-yl)propan-2-yl]phosphat | 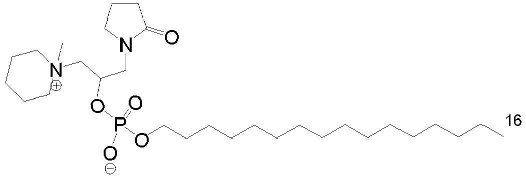 |
| J701Y | 1-Methyl-1-(2-Tridecyl-3-sulfonatopropyl)azepanium | 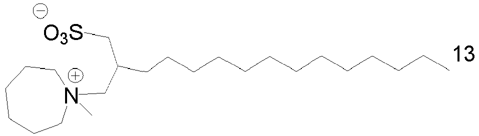 |
| J702Y | Tetradecyl trimethylammonium chloride | 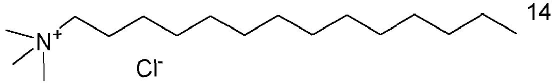 |
| J703Y | 1-Methyl-(1-Piperidinium-1-yl)heptadecane-3-sulfonate | 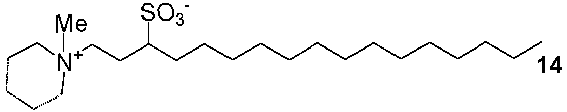 |
| J704Y | 1-(N-Methylpiperidinium-1-yl)pentadecane-3-sulfonate | 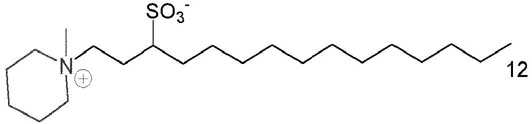 |
| J705Y | 2-Methoxy-3-(N,N-dimethyl-N-dodecylammonio)propane-1-sulfonate | 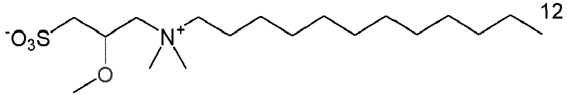 |
| J706Y | 2-Acetoxy-3-(N,N-dimethyl-N-dodecylammonio)propane-1-sulfonate | 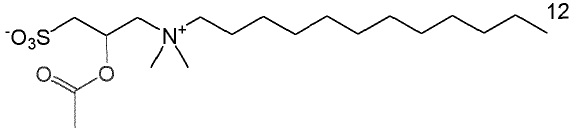 |
| J707Y | 2-Myristoyloxy-3-(N,N,N-trimethylammonio)propane-1-sulfonate | 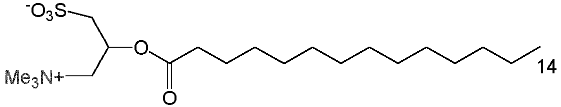 |
| J708Y | 2-Dodecanoyloxy-3-(N,N,N-trimethylammonio)propane-1-sulfonate | 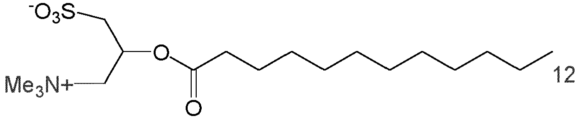 |
| J709Y | 1-(N-Methylazepanium-1-yl)heptadecane-3-sulfonate | 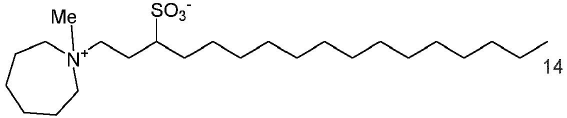 |
| J70C | N-Palmityl-D-erythro-sphingosine | 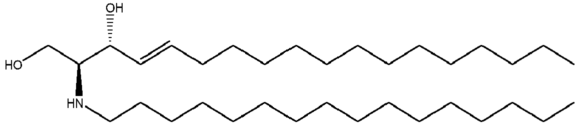 |
| J710Y | 1-O-Glucosyl-3-O-methyl-octadecan | 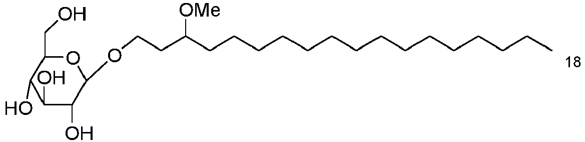 |
| J711Y | 1-O-Glucosyl-2-O-methyl-3-O-tetradecyl-rac-glycerin | 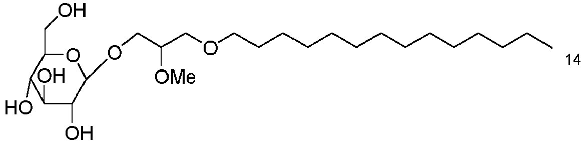 |
| J712Y | 1-Methyl-1-(3-sulfonato heptadecyl) azocanium | 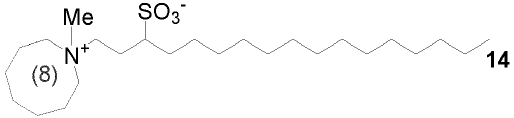 |
| J713Y | 1-Methyl-1-(3-sulfonato pentadecyl)-4-hydroxy-piperidinium | 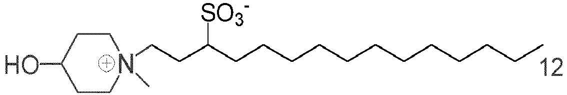 |
| J714Y | 2-Methoxy-3-(N,N-dimethyl-N-tetradecylammonio)propane-1-sulfonate | 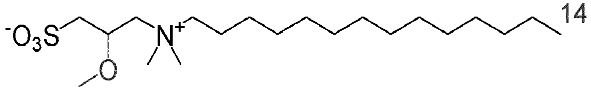 |
| J715Y | 1-(3-Sulfonato pentadecyl)-4-hydroxy piperidinium | 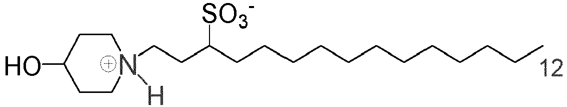 |
| J716Y | 1-Trimethylammonio-3-sulfonato pentadecane | 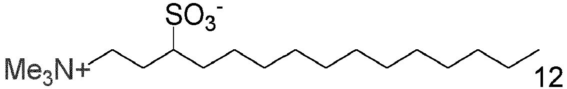 |
| J717Y | 1-Trimethylammonio-3-sulfonato heptadecane | 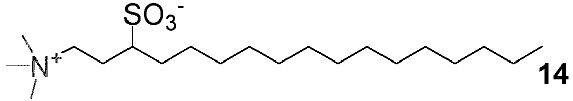 |
| J718Y | 1-Methyl-1-(3-sulfonato heptadecyl)-(2S)-2-(methoxymethyl)pyrrolidinium | 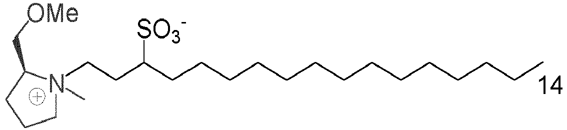 |
| J719Y | 1-Methyl-1-(3-sulfonato pentadecyl)-(2S)-2-(methoxymethyl)pyrrolidinium | 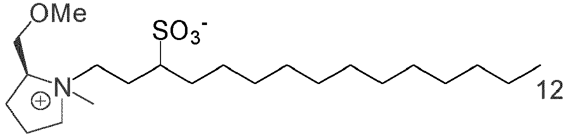 |
| J720Y | 1-Methyl-1-(3-sulfonato heptadecyl)-4-hydroxy-piperidinium | 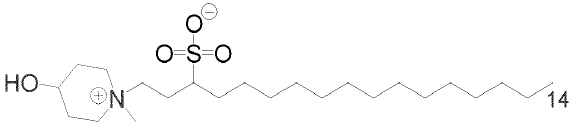 |
| J721C | 1-O-Glucosyl-3-O-methyl-2-N-(3-methylurea)-L-threo-sphingosin | 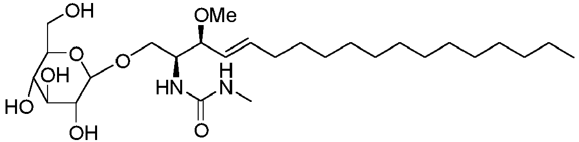 |
| J721C | 1-O-Glucosyl-3-O-methyl-2-N-(3-methylurea)-L-threo-sphingosin | 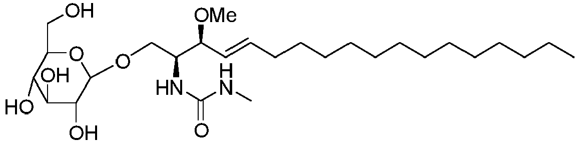 |
| J722Y | N-Methyl dodecylamin | 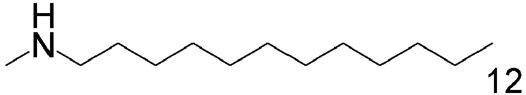 |
| J723Y | Tetradecylamine | 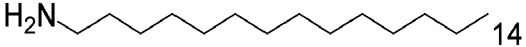 |
| J724Y | 4-(N-Methyldodecylammonio)butanesulfonate | 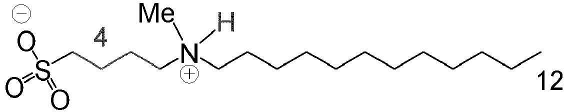 |
| J725Y | N,N-Dimethyl tetradecylamine | 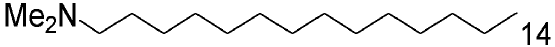 |
| J726Y | 4-(2-Dodecanoyloxy-3-sulfonatopropyl)-morpholinium | 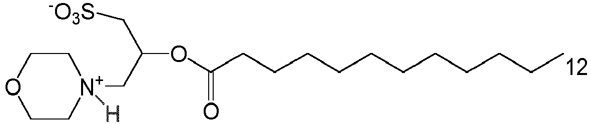 |
| J727Y | 4-(N,N-Dimethyl-N-dodecylammonio)butane-1-sulfonate | 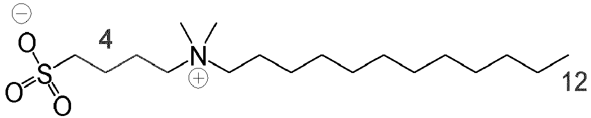 |
| J728Y | 2-(Piperidinium-1-yl)-pentadecan-1-sulfonat | 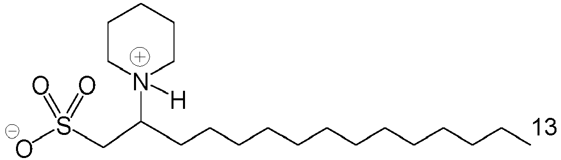 |
| J729Y | 3-(N-Methyl-N-dodecylammonio)propane-1-sulfonate | 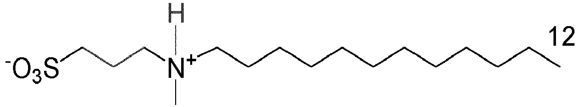 |
| J730Y | N,N-Dimethyldodecylamine | 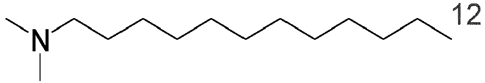 |
| J731Y | 3-(N,N-dimethyl-N-octylammonio)propane-1-sulfonate | 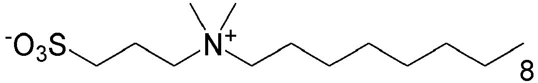 |
| J732Y | 3-(N,N-Dimethyl-N-decylammonio)propane-1-sulfonate | 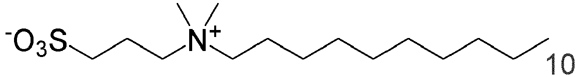 |
| J733Y | 1-Dimethylammonio-2-dodecanoyloxy-3-sulfonatopropane | 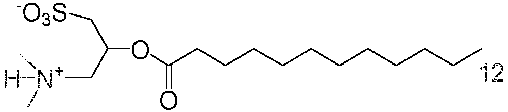 |
| J734Y | 2-(N,N-Dimethyldodecylammonio)ethanesulfonate | 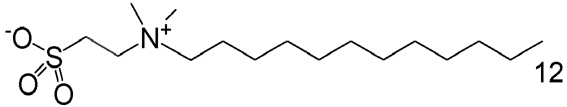 |
| J735Y | 2-(N-Methyldodecylammonio)ethanesulfonate | 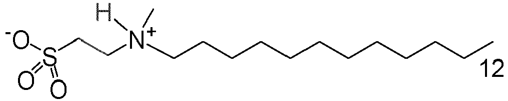 |
| J736Y | 1-(2-Dodeca noyloxy-3-sulfonatopropyl)-piperidinium | 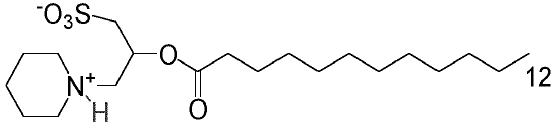 |
| J737Y | 4-(2-Tetradecanoyloxy-3-sulfonatopropyl)-morpholinium | 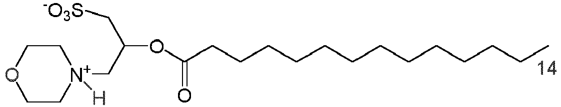 |
| J738Y | 4-Methyl-4-(2-dodecanoyloxy-3-sulfonatopropyl)-morpholinium | 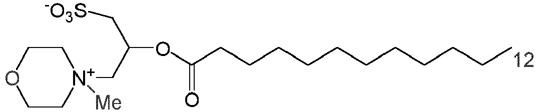 |
| J739Y | 4-Methyl-4-(2-tetradecanoyloxy-3-sulfonatopropyl)morpholinium | 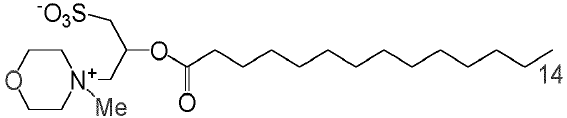 |
| J740Y | 2-Hydroxy-3-(N-methyltetradecylammonio)propanesulfonate | 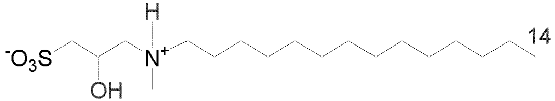 |
| J741Y | 2-(N-Methyltetradecylammonio)ethanesulfonate | 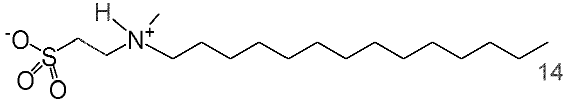 |
| J742Y | N-Methyltetradecylamine | 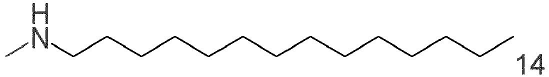 |
| J743Y | 3-(N-Methyltetradecylammonio)propanesulfonate | 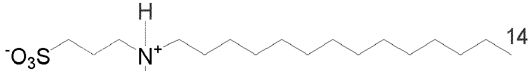 |
| J744Y | 1-(2-Tetradecanoyloxy-3-sulfonatopropyl)azepanium | 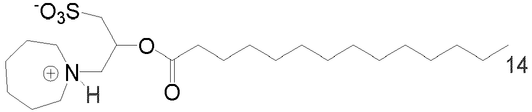 |
| J745Y | 1-(2-Dodecanoyloxy-3-sulfonatopropyl)azepanium | 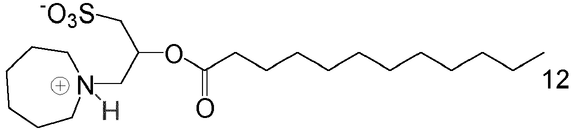 |
| J746Y | 2-(N,N-Dimethyltetradecylammonio)ethanesulfonate | 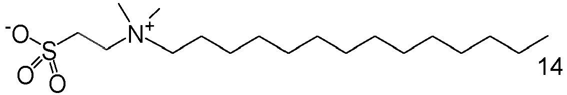 |
| J747Y | 1-Dimethylammonio-2-dodecanoyloxy-3-(2-oxo-1-pyrrolidinyl)propane TFA | 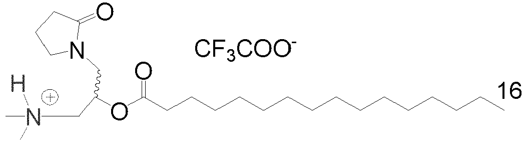 |
| J748Y | 2-Trimethylammoniopentadecane-1-sulfonate | 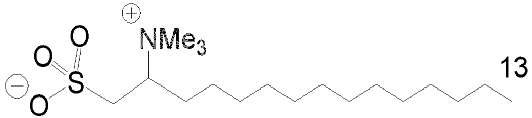 |
| J749Y | 1-Dimethylammonio-3-sulfonato pentadecane | 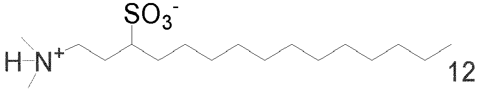 |
| J750Y | 2-Dimethylammoniumpentadecane-1-sulfonate | 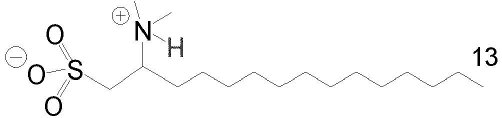 |
| J751Y | 2-(N-Methylpiperidinium-1-yl)-pentadecane-1-sulfonate | 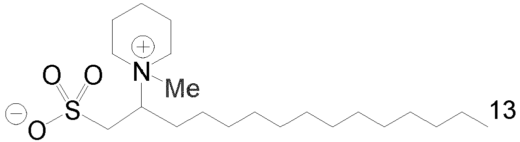 |
| J752Y | 4-(N-Methyltetradecylammonio)butane-1-sulfonate | 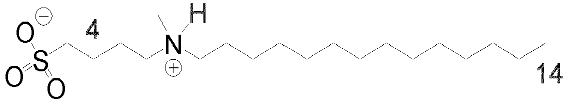 |
| J753Y | Tetradecyl(2-sulfonatoethyl)ammonium | 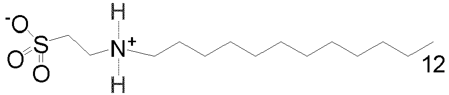 |
| J754Y | Bis(2-sulfonatoethyl)tetradecylammonium, betaine | 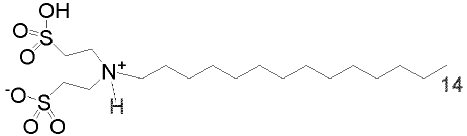 |
| J755Y | Bis(2-sulfonatoethyl)dodecyl ammonium, betaine | 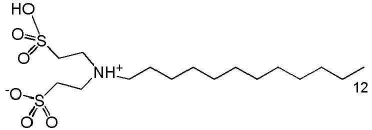 |
| J756Y | 1-Dodecyl-4-dimethylaminopiperidiniumiodide | 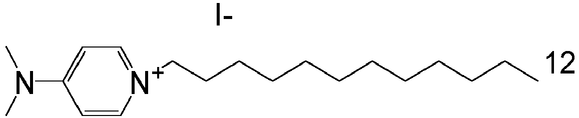 |
| J757Y | 3-N,N-Dimethylmyristylammoniopropyl phosphonic acid diethylester, TFA | 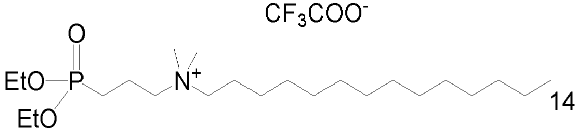 |
| J758Y | 1-Dimethylammonio-3-sulfonato heptadecane | 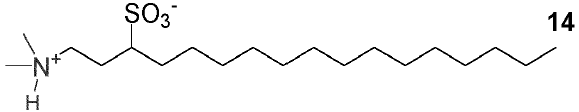 |
| J759Y | 1-Tetradecyl-4-dimethylaminopiperidinium iodide | 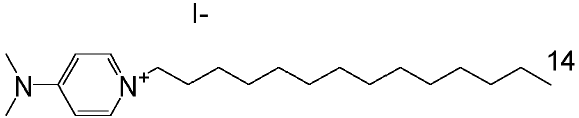 |
| J760C | 2-N-Acetamid-1-O-glucosyl-3-O-methyl-non-4en | 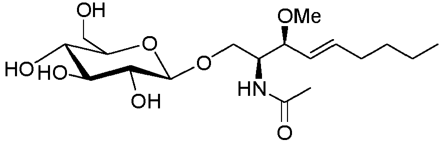 |
| J760C | 2-N-Acetamid-1-O-glucosyl-3-O-methyl-non-4en | 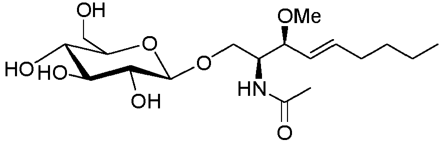 |
| J761C | 2-N-Acetamid-1-O-glucosyl-3-O-methyl-pentadec-4en | 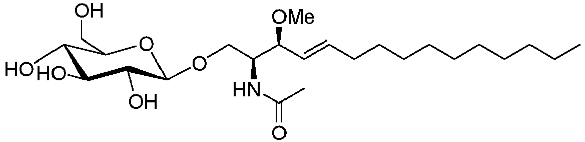 |
| J762Y | 1-[Trimethylammonio-2-hexadecanoyloxy-3-(2-oxo-1-pyrrolidinyl)propane TFA | 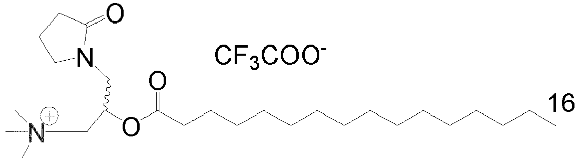 |
| J763Y | 1-[2-Hexadecyloxy-3-(2-oxo-1-pyrrolidinyl)propyl]-1-methylpiperidinium TFA | 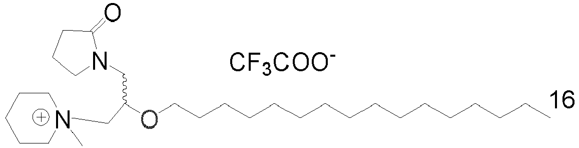 |
| J764Y | N-Methylhexadecylamine | 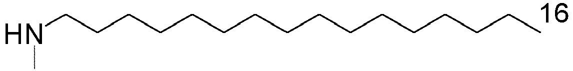 |
| J765Y | Specs: AG-690/40096484 | 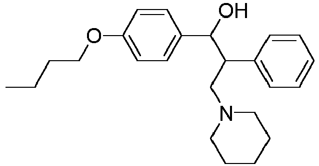 |
| J766Y | 1-(3-Sulfonatoheptadecyl)-4-hydroxy-piperidinium | 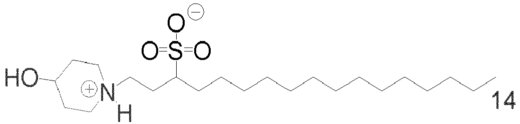 |
| J767Y | N-(2-Carboxyethyl)-N,N-dimethyltetradecylammonium, betaine | 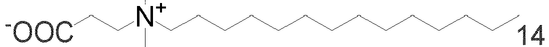 |
| J768Y | N-(2-Carboxyethyl)-N,N-dimethyldodecylammonium, betaine | 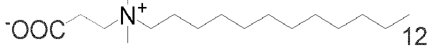 |
| J769Y | N,N-Bis(2-sulfonatoethyl)-N - methyl-tetradecylammonium, betaine | 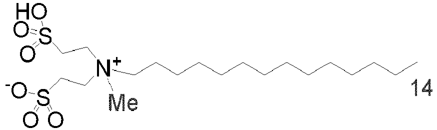 |
| J76C | 1-Acetyl-N-(4,7,10,13-tetroxa-16-acetylazapalmitoyl)-sphingosine | 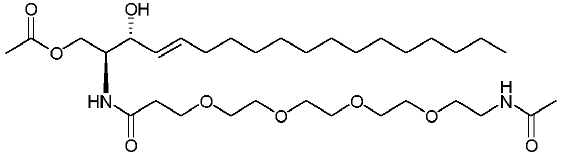 |
| J770Y | 2-(N-Tetradecylammonio)ethanesulfonate | 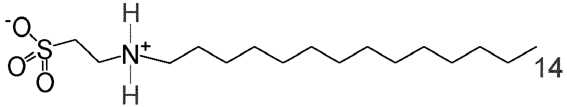 |
| J771Y | 1-[2-Hexadecyloxy-3-(2-oxo-1-pyrrolidinyl)propyl]-piperidinium TFA | 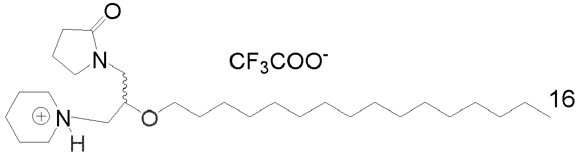 |
| J772Y | N,N-Bis(2-sulfonatoethyl)-N-methyl-dodecylammonium, betaine | 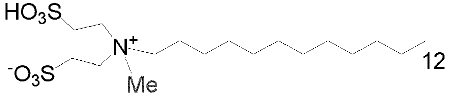 |
| J773Y | 3-N,N-Dimethylmyristylammoniopropylphosphonic acid | 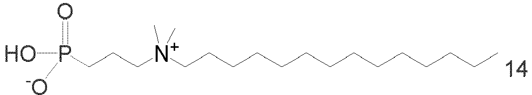 |
| J774Y | N-Hexadecyl-N-(3-sulfonatopropyl)piperidinium | 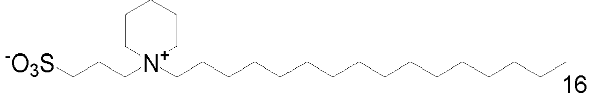 |
| J775Y | 2-Hydroxy-3-(N-Methylhexadecylammonio)propanesulfonate | 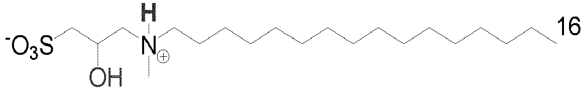 |
| J776Y | (E)-1-(2-Methoxybenzylidene)-2-hexadecansulfonylhydrazine | 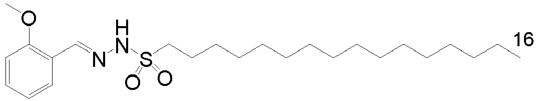 |
| J777Y | 3-N,N-Dimethylhexadecylammoniopropylphosphonic acid diethylester, Br | 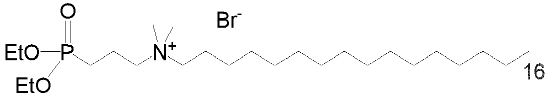 |
| J778C | 2 -N -Pal mitoyl-1-O-(?-D-glucosyl)-3-methoxyhex-4-en-1-ol | 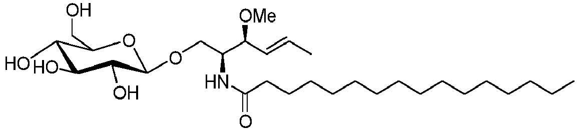 |
| J779Y | 3-N,N-Dimethyldodecylammoniopropyl phosphonic acid diethylester, Br | 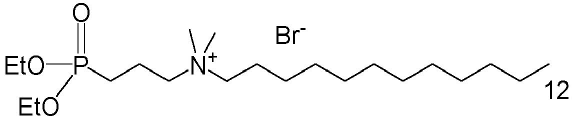 |
| J780Y | 3-N,N-Dimethylpalmitylammoniopropylphosphonic acid | 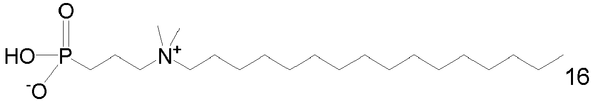 |
| J781Y | 2-Hydroxy-3-(N,N-Dimethylhexadecylam monio)propanesulfonate | 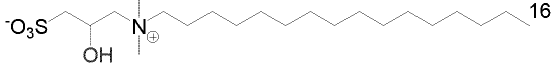 |
| J782Y | 4-(N-Methylhexadecylammonio)propanesulfonate | 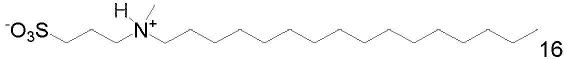 |
| J783Y | N,N-Dimethyl-N-(3-sulfonatopropyl)-N-(3-hexadecanoyloxypropyl)ammonium | 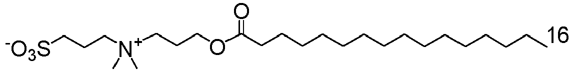 |
| J784Y | 2-Ethyl-3-(N-Methylmyristylamino)propane-1-ol | 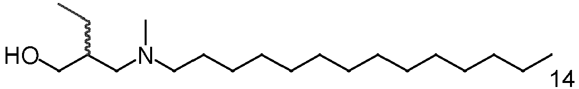 |
| J785Y | 3-(Dimethylamino)propyl tetradecanoate, TFA salt | 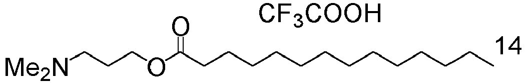 |
| J786Y | 3-(Trimethylammonino)propyl tetradecanoate, iodide | 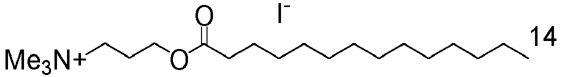 |
| J787Y | N,N-Dimethyl-N-(3-sulfonatopropyl)-N-(3'-tetradecanoyloxypropyl)ammonium | 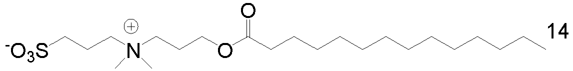 |
| J788Y | N-(3-(Dimethylamino)propyl)palmitamide | 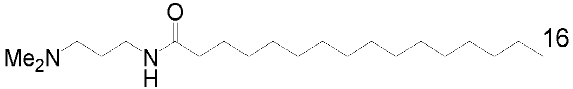 |
| J789Y | N-(3-(Trimethylammonio)propyl)palmitamide, TFA salt | 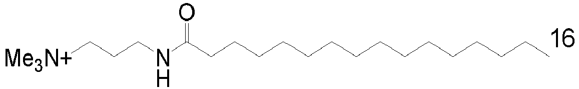 |
| J790Y | N,N-Dimethyl-N-(3-sulfonatopropyl)-N-(3'-dodecanoyloxypropyl)ammonium | 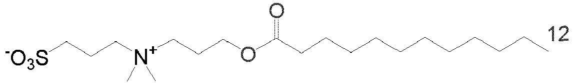 |
| J791Y | 2-Ethyl-3-(N-methyl-N-dodecylammonio)propane-1-sulfonate | 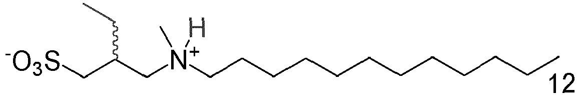 |
| J792Y | 2-Ethyl-3-(N,N-dimethyl-N-tetradecylammonio)propan-1-ol trifluoroacetate | 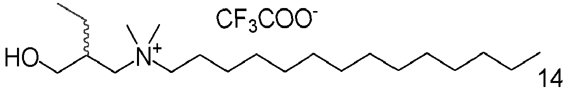 |
| J793Y | trans-1-Hexadecyl-1-(3-sulfonatopropyl)-4-trifluormethylpiperidinium | 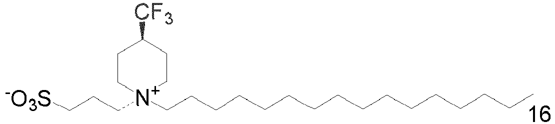 |
| J794Y | cis-1-Hexadecyl-1-(3-sulfonatopropyl)-4-trifluormethylpiperidinium | 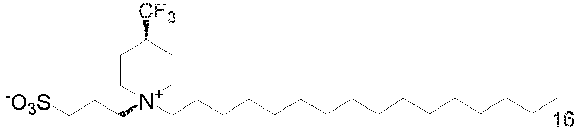 |
| J795Y | N,N-Dimethyl-N-(3-sulfonatopropyl)-N-(3-palmitoylaminopropyl)ammonium | 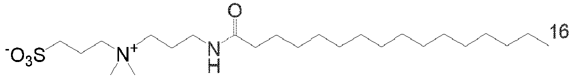 |
| J796Y | N-Hexadecyl-N-(3-sulfonatopropyl)-3-diethylaminocarbonyl-piperidinium | 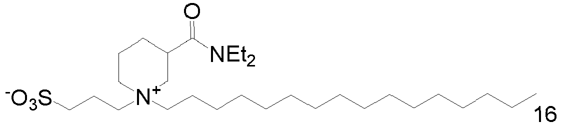 |
| J797Y | N-Hexadecyl-N-(3-sulfonatopropyl)-3-trifluormethyl-piperidinium | 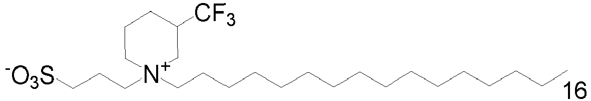 |
| J798Y | N-Hexadecyl-N-(3-sulfonatopropyl)-3-ethoxycarbonyl-piperidinium | 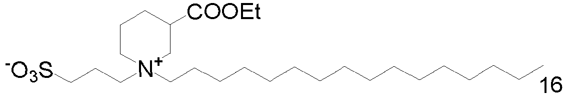 |
| J799Y | 3-N,N-Dimethyldodecylammoniopropylphosphonic acid | 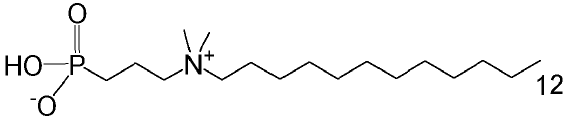 |
| J7C | N-(1-Adamantoyl)-sphingosine | 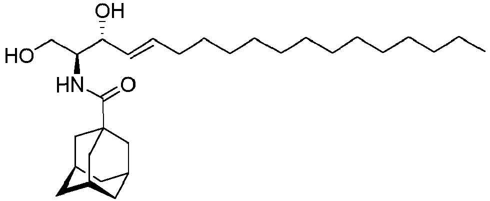 |
| J800Y | N-Tetradecyl-N-(3-sulfonatopropyl)-3-ethoxycarbonyl-piperidinium | 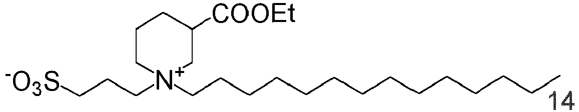 |
| J801Y | N-Dodecyl-N-(3-sulfonatopropyl)-3-ethoxycarbonyl-piperidinium | 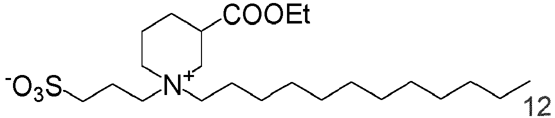 |
| J802Y | 1-(2-Tetradecyloxy-3-sulfonatopropyl)piperidinium | 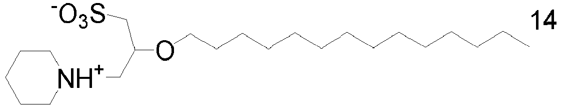 |
| J803Y | cis-N-Hexadecyl-N-(3-sulfonatopropyl)-3-diethylaminocarbonyl-piperidinium | 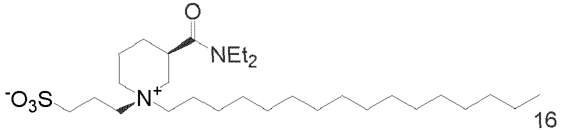 |
| J804Y | N-Methyl-N-(3-sulfonatopropyl)-N-(4-(2,3,4,6-tetra-Opivaloylglucopyranose-O1-yl)butyl)hexadecylammonium | 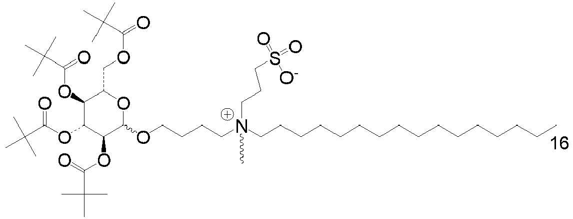 |
| J805Y | 2-Ethyl-3-(N-methyltetradecylammo nio)propanesulfonate | 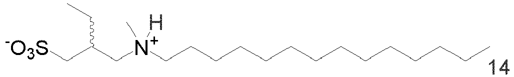 |
| J806Y | N,N-Dimethyl-N-(3-sulfonatopropyl)-N-(3'-myristoylaminopropyl)ammonium | 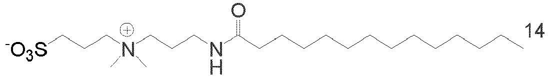 |
| J807Y | N-(3-(Dimethylamino)propyl)dodecanamide | 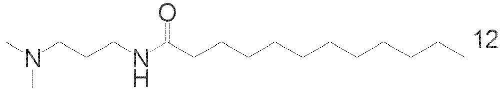 |
| J808Y | 1-Hexadecyl-1-(3-sulfonatopropyl)-4-hydroxypiperidinium | 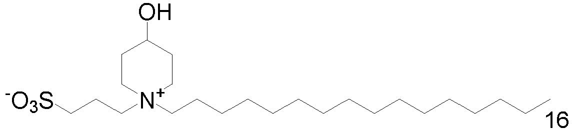 |
| J809C | 2-N-Acetyl-1-O-glucuronsäure-3-O-methyl-L-threo-sphingosin | 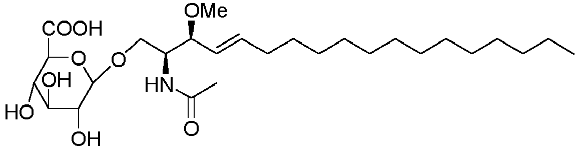 |
| J810Y | 2-Ethyl-3-(N,N-dimethyltetradecylammonio)propanesulfonate | 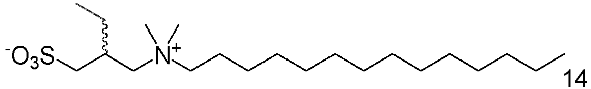 |
| J811Y | N,N-Dimethyl-N-(3-sulfonatopropyl)-N-(3'-dodecanoylaminopropyl)ammonium | 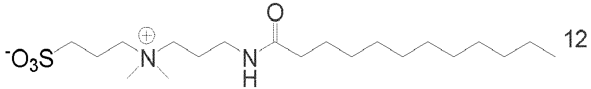 |
| J812Y | N-Methyl-N-(3-(dimethylamino)propyl)tetradecanamide | 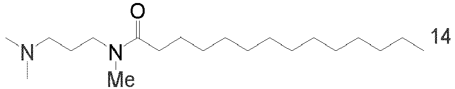 |
| J813Y | N-Methyl-N-(3-(trimethylammonio)propyl)tetradecanamide iodide | 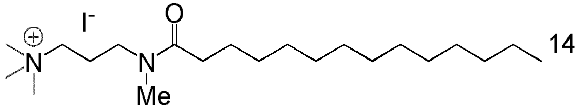 |
| J814Y | 2-Ethyl-3-(N,N-dimethyldodecylammonio)propanesulfonate | 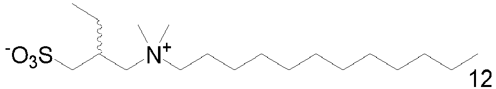 |
| J815Y | N-Methyl-N-(3-sulfonatopropyl)-N-(4-(glucopyranose-O1-yl)butyl)-hexadecylammonium | 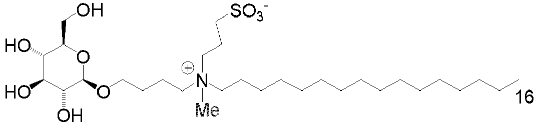 |
| J816Y | trans-N-Tetradecyl-N-(3-sulfonatopropyl)-3-diethylaminocarbonyl-piperidinium | 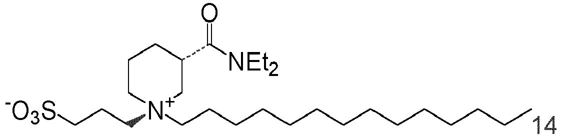 |
| J817Y | cis-N-Dodecyl-N-(3-sulfonatopropyl)-3-diethylaminocarbonyl-piperidinium | 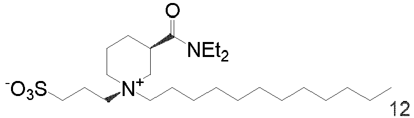 |
| J818Y | N-Methyl-N-(4-(glucopyranose-O1-yl)butyl)hexadecylammonium TFA | 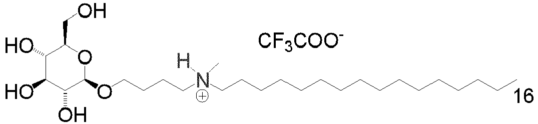 |
| J819Y | N-(3-(Trimethylammonio)propyl)dodecanoylamide iodide | 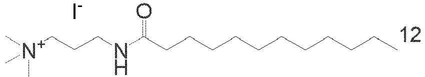 |
| J820Y | cis-N-Tetradecyl-N-(3-sulfonatopropyl)-3-diethylaminocarbonyl-piperidinium | 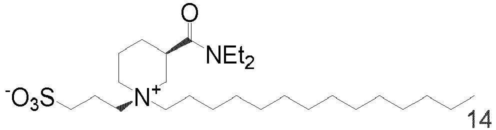 |
| J821Y | N-Methyl-N-(4-(2,3,4,6-tetra-Opivaloylglucopyranose-O1-yl)butyl)hexadecylamine | 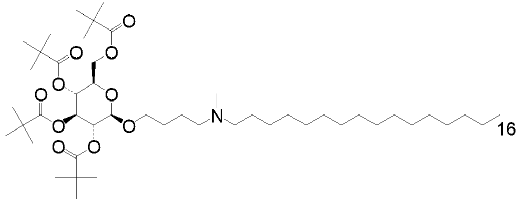 |
| J822Y | N-Hexadecylthiazolium TFA | 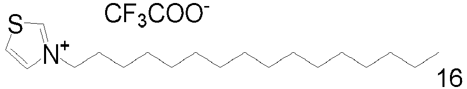 |
| J823Y | N,N-Dimethyl-N-(4-(b-D-glucopyranose-O1-yl)butyl)hexadecylammoniumTFA | 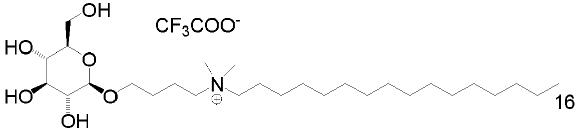 |
| J824Y | N-Methyl-N-(3-sulfonatopropyl)-N-(4-hydroxybutyl)hexadecylammonium | 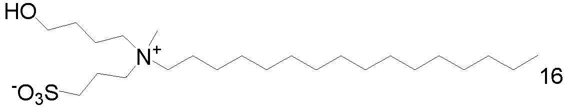 |
| J825Y | trans-N-Dodecyl-N-(3-sulfonatopropyl)-3-diethylaminocarbonyl-piperidinium | 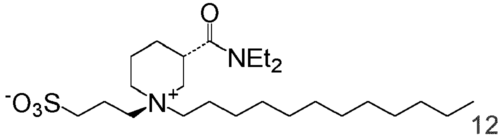 |
| J826Y | N-Methyl-N-(4-hyd roxybutyl)tetradecylamin | 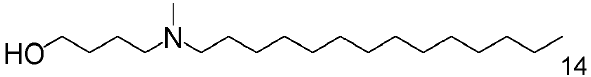 |
| J827Y | N,N-Dimethyl-N-(4-(b-D-glucopyranose-O1-yl)butyl)tetradecylammonium TFA | 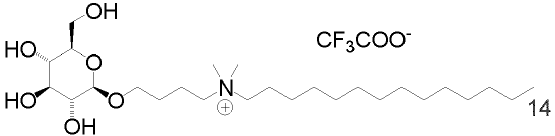 |
| J828Y | N-Methyl-N -(4-(b-D-glucopyranose-O1-yl)butyl)tetradecylammonium TFA | 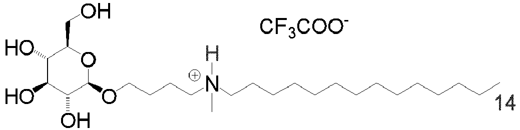 |
| J829Y | N-Methyl-N-(3-sulfonatopropyl)-N-(4-(b-D-glucopyranose-O1-yl)butyl)-tetradecylammonium | 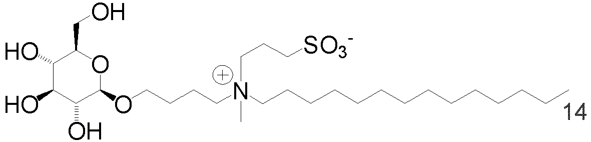 |
| J830Y | N-Methyl-N -(3-sulfonatopropyl)-N-(4-hyd roxybutyl)tetradecylammonium | 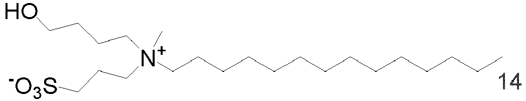 |
| J831Y | N-Methyl-N-(3-sulfonatopropyl)-N-(4-(S)-tyrosinoyloxybutyl)tetradecylammonium hyd rotrifluoroacetate | 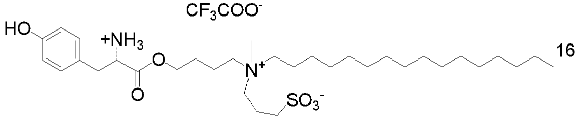 |
| J832Y | 3-(N-Methyl-N-hexadecyl-N-(4-(glucopyranose-O1-yl)butyl)ammonio)-propylphosphonic acid diethylester trifluoroacetate | 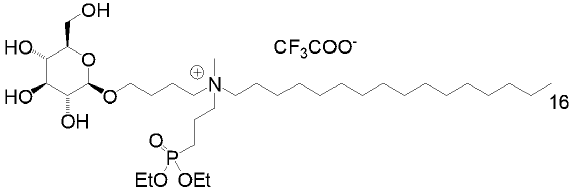 |
| J833Y | 3-(N-Methyl-N-tetradecyl-N-(4-(glucopyranose-O1-yl)butyl)ammonio)-propylphosphonic acid diethylester trifluoroacetate | 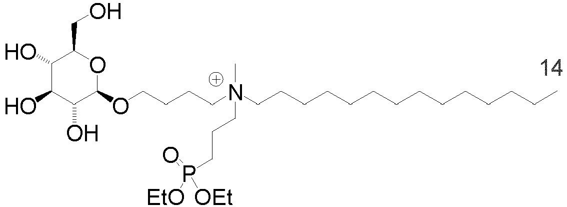 |
| J834Y | N-Methyl-N-(3-sulfonatopropyl)-N-(4-(1-metahnesulfonyl-2-imidazolidinone-3-yl)carbonyloxybutyl)tetradecylammonium | 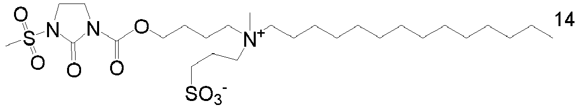 |
| J835Y | N,N-Dimethyl-N-(3sulfonatopropyl)-N-(3'-(N-methyl-N-myristoylamino)propyl)ammonium) | 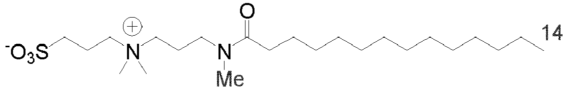 |
| J836Y | 4-Tridecylpyridinium trifluoroacetate | 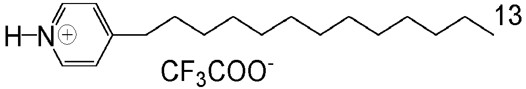 |
| J837Y | Thiazolium-3-yl-acetic acid hexadecylester,TFA salt | 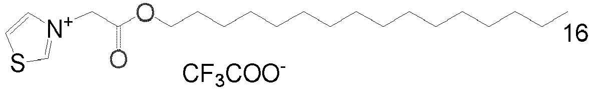 |
| J838Y | N,N-Dimethyl-N-(4-hydroxybutyl)tetradecylammonium trifluoroacetate | 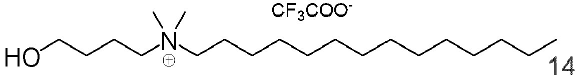 |
| J839Y | 3-N-Methyltetradecylammoniopropylphosphonic acid diethylester bromide | 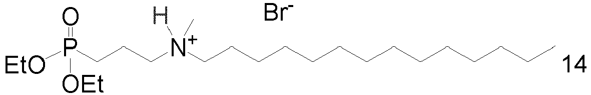 |
| J83Y | 6-Hydroxy-2,5,7,8-tetra methylchroma n-2-carboxylic acid | 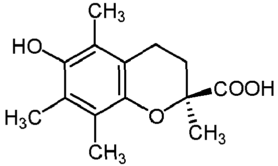 |
| J840Y | N-Methyl-4-tridecylpyridinium trifluoroacetate | 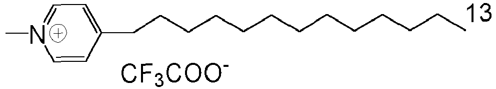 |
| J841Y | 3-N-Methyhexadecylammoniopropylphosphonic acid diethylester bromide | 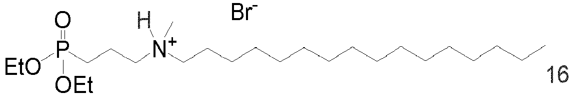 |
| J842Y | 3-N-Methylmyristylammoniopropylphosphonic acid | 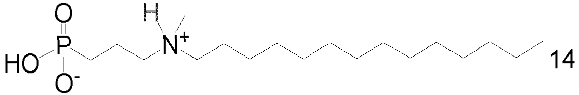 |
| J843Y | N-Methyl-N-tetradecyl-(D)-glucosammonium trifluoroacetate | 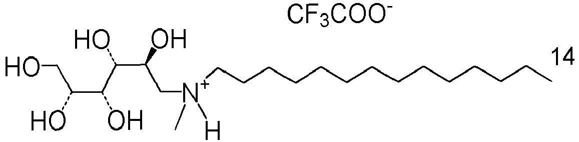 |
| J844Y | 3-N-Methylpalmitylammoniopropylphoshonic acid | 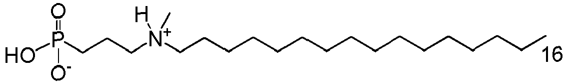 |
| J845Y | 3-(4-Tridecylpyridinium-1-yl)propane-1-sulfonate | 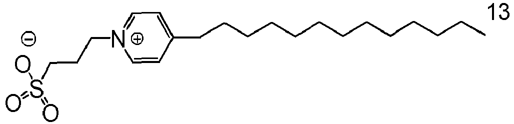 |
| J846Y | 3-(1-Tetradecyl-1H-benzo(d)imidazolium-3-yl)propane-1-sulfonate | 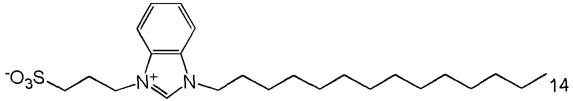 |
| J847Y | N-(4-(Beta-D-glucopyranose-O1-yl)butyl)tetradecylammonium trifluoroacetate | 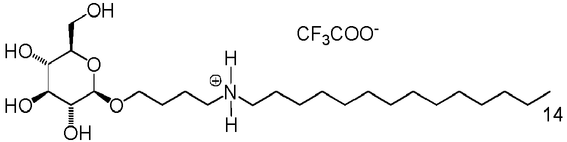 |
| J848Y | N-Tetradecyl-N,N-dimethylglycine-(N'-methyl-N'-(2,3-dihydroxypropyl)amide)trifluoroacetate | 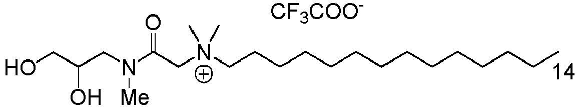 |
| J849Y | N,N-bis(4-(beta-(D)-glucopyranose-O1-yl)butyl)tetradecylammonium trifluoroacetate | 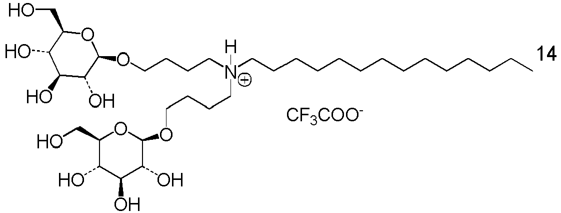 |
| J84Y | Methyl 6-Hydroxy-2,5,7,8-tetramethylchroman-2-carboxylate | 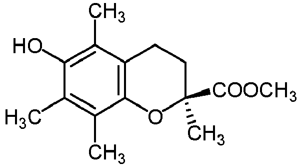 |
| J850Y | N-(3-Sulfonatopropyl)-N-(4-(b-D-glucopyranose-O1-yl)butyl)-tetradecylammonium | 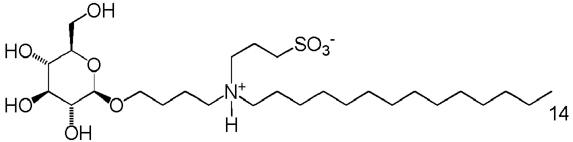 |
| J851Y | 3-(N-tetradecyl-N-(4-(β-D-glucopyranose-O1-yl)butyl)ammonio)-propylphosphonic acid diethylester trifluoroacetate | 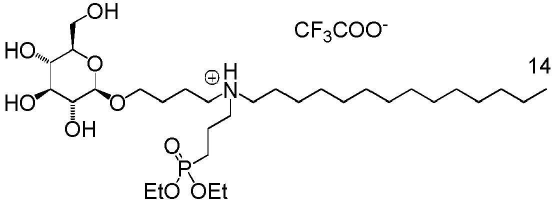 |
| J852Y | 3-(Hexadecyl(4-((3-hydroxypropyl)(methyl)ammonio)butyl)(methyl)ammonio)propane-1-sulfonateTFA salt | 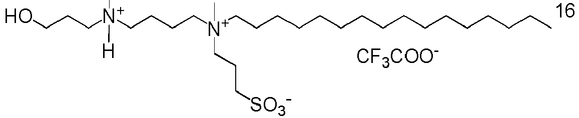 |
| J853Y | 1-Tetradecyl-3-(3-sulfonatopropyl)imidazolium | 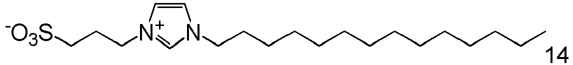 |
| J854Y | 4-(Methyl(tetradecyl)ammonio)butanoate | 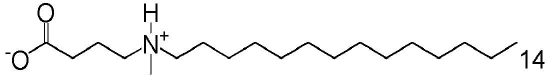 |
| J855Y | N-Methyl-N-(4-hyd roxybutyl)hexadecylamine | 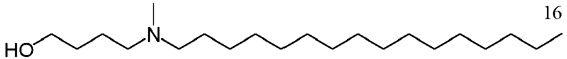 |
| J856Y | 3-(4-Tridecylpyridinium-1-yl)propylphosphonic acid | 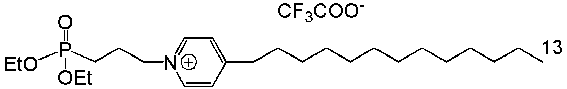 |
| J857Y | 4-(Dimethyl(tetradecyl)ammonio)butanoate | 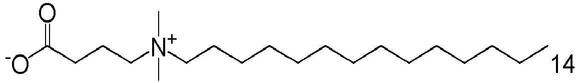 |
| J858Y | Hexadecyl-D-glucoside | 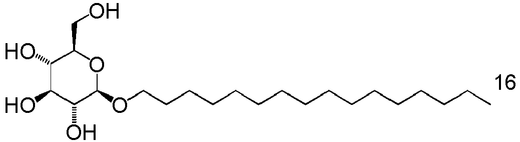 |
| J859Y | N-Methyldodecylsulfoxide | 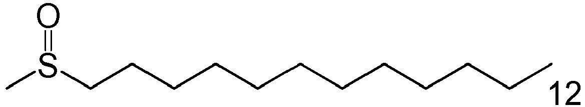 |
| J860Y | N,N-Dimethyl-N-(4-hyd roxybutyl)hexadecylammonium trifluoroacetate | 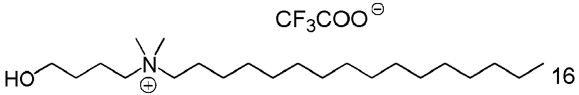 |
| J861Y | N-(4-Hydroxybutyl)tetradecylamine | 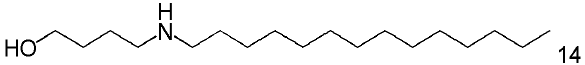 |
| J862Y | Tetradecylsulfinylbenzene | 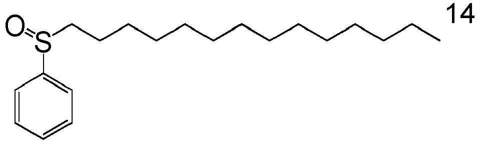 |
| J863Y | Tetradecylsulfonylbenzene | 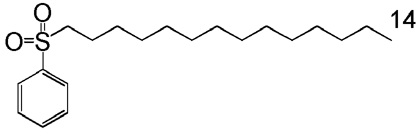 |
| J864Y | 2-(Tetradecylsulfinyl)ethanol | 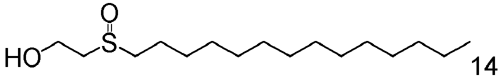 |
| J865Y | 2-(Tetradecylsulfinyl)ethyla cetate | 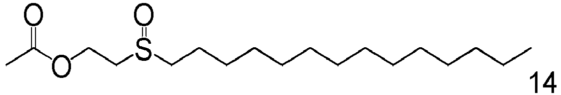 |
| J866Y | trans-2-((R)-Dodecylsulfinyl)cyclohexanol | 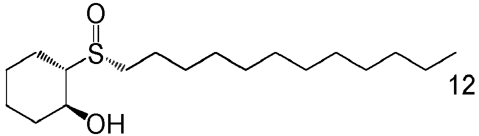 |
| J867Y | trans-2-((S)-Dodecylsulfinyl)cyclohexanol | 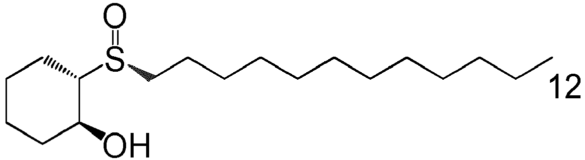 |
| J868Y | trans-2-((R)-Tertadecylsulfinyl)cyclohexanol | 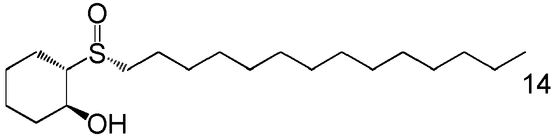 |
| J869Y | trans-2-((S)-Tertadecylsulfinyl)cyclohexanol | 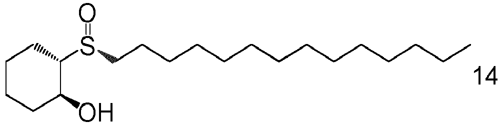 |
| J86C | N-Palmityl-D-threo-sphingosine | 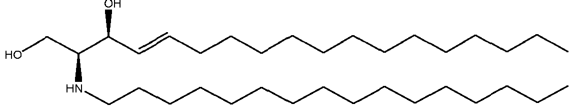 |
| J870Y | 3-(4-Tridecylpyridinium-1-yl)propylphosphonate | 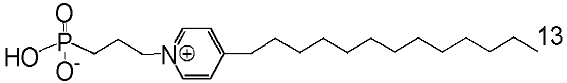 |
| J871Y | trans-1-Acetoxy-2-((R)-Dodecylsulfinyl)cyclohexane | 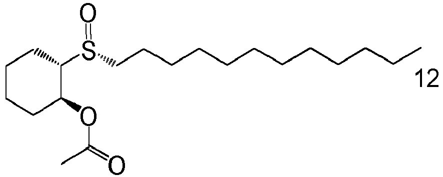 |
| J872Y | 3-(N-tetradecyl-N-(4-(β-D-glucopyranose-O1-yl)butyl)ammonio)-propylphosphonic acid | 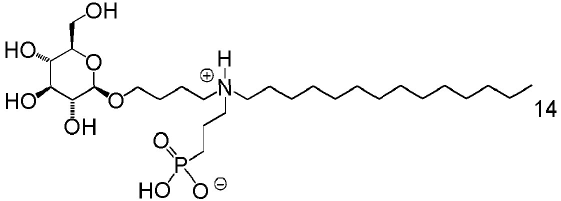 |
| J873Y | 3-(N-Tetradecyl-N-(4-hyd roxybutyl)amino)propylphosphonic acid diethylester | 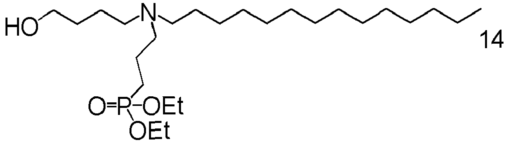 |
| J874Y | 3-(N-Methyl-N-tetradecyl-N-(4-(beta - D-glucopyranose-O1-yl)butyl)ammonio)-propylphosphonic acid | 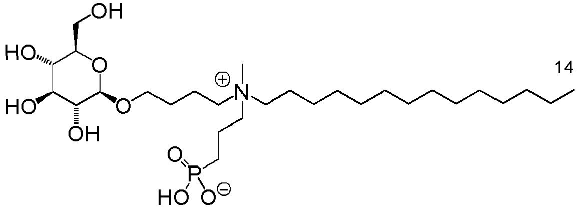 |
| J875Y | Tetradecylsulfinylcyclohexane | 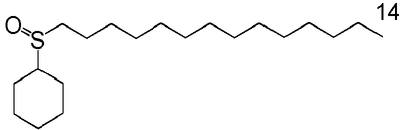 |
| J876Y | trans-2-((R)-Tertadecylsulfinyl)cyclohexyl acetate | 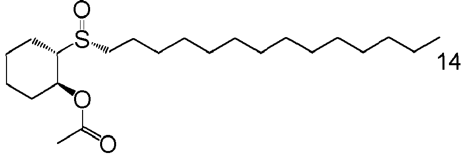 |
| J877Y | trans-2-((R)-Hexadecylsulfinyl)cyclohexanol | 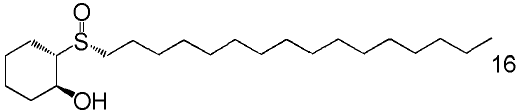 |
| J878Y | trans-2-((S)-Hexadecylsulfinyl)cyclohexanol | 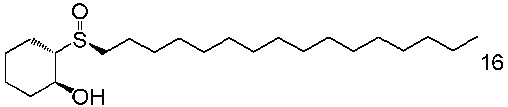 |
| J879Y | (trans)-2-((R)-hexadecylsulfinyl)cyclohexyl acetate | 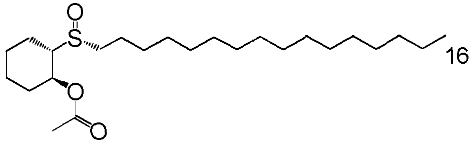 |
| J880Y | 2-(Tetradecylsulfinyl)ethyl methanesulfonate | 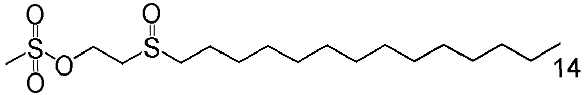 |
| J881Y | 2-(Dodecylsulfinyl)ethanol | 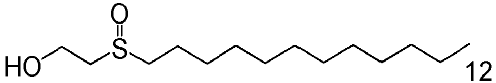 |
| J882Y | trans-2-(Dodecylthio)cyclohexanol | 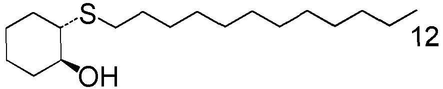 |
| J883Y | trans-2-(Tetradecylthio)cyclohexanol | 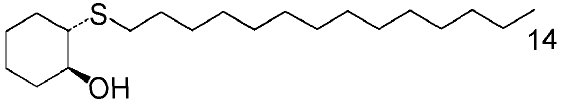 |
| J884Y | 2-(Dodecylsulfinyl)ethyl acetate | 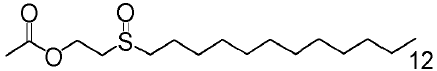 |
| J885Y | 3-Tetradecylthiazolium trifluoroacetate | 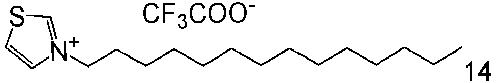 |
| J886Y | trans-2-((S)-Dodecylsulfinyl)cyclohexyl acetate | 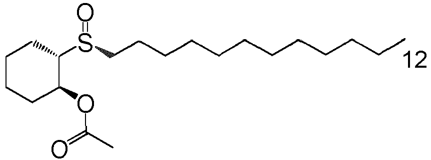 |
| J887Y | 1-(Methylsulfinyl)tetradecane | 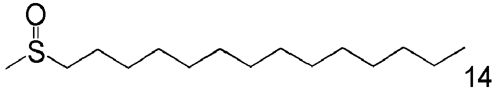 |
| J888Y | trans-2-((S)-Tetradecylsulfinyl)cyclohexyl acetate | 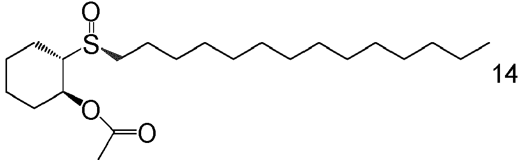 |
| J889Y | trans-2-((S)-Hexadecylsulfinyl)cyclohexyl acetate | 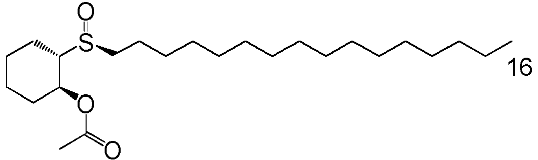 |
| J88C | N-Palmitoyl-D-erythro-sphingosine | 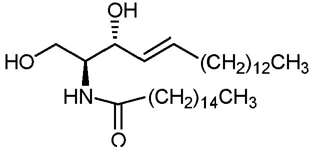 |
| J890Y | N,N-Dimethyl-2-(tetradecylsulfinyl)ethanaminium trifluoroacetate | 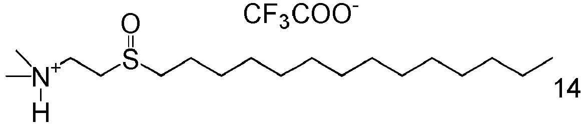 |
| J891Y | N,N-Dimethyldecylamine-N-oxide | 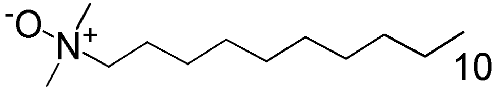 |
| J892Y | N,N-Dimethyldodecylamine-N-oxide | 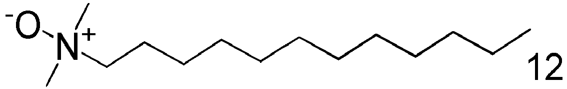 |
| J893Y | N,N-Dimethyltetradecylamine-N-oxide | 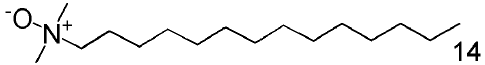 |
| J894Y | N-Dodecyl-N,N-d imethylglycine | 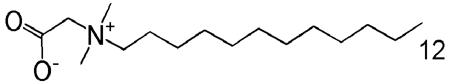 |
| J895Y | 1-(2-(Ethylsulfinyl)ethylsulfinyl)tetradecane | 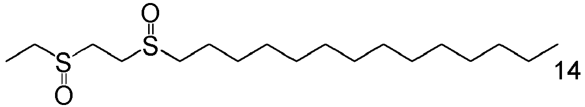 |
| J896Y | N-Dodecyl-N,N-dimethylglycine-N'-methyl-D-glucosamide trifluoroacetate | 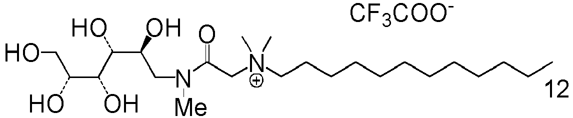 |
| J897Y | 2-(Dodecylsulfinyl)phenol | 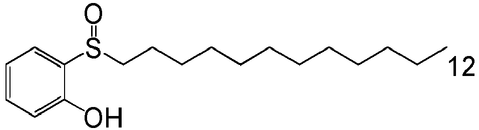 |
| J898Y | trans-2-((R)-Decylsulfinyl)cyclohexanol | 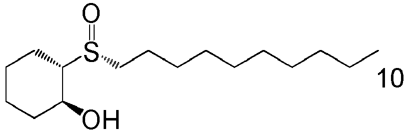 |
| J899Y | trans-2-((S)-Decylsulfinyl)cyclohexanol | 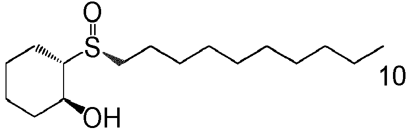 |
| J8C | N-(trans-(4-tert-Butylcyclohexylcarbonyl)-sphingosine | 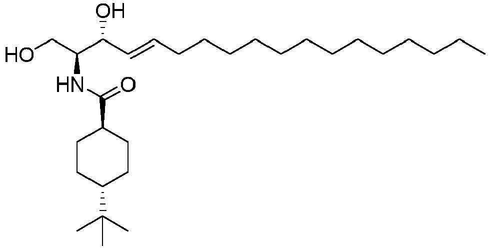 |
| J900Y | 2-(Dodecylsulfinyl)ethyl methanesulfonate | 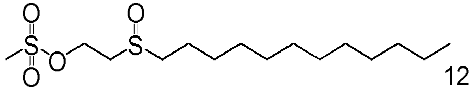 |
| J901Y | 2-(2-(Tetradecylsulfinyl)ethylsulfinyl)ethanol | 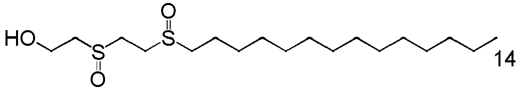 |
| J902Y | N,N-Dimethyl-2-(dodecylsulfinyl)ethanamine | 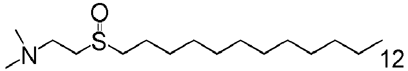 |
| J903Y | 1-(2-(Ethylsulfinyl)ethylsulfinyl)dodecane | 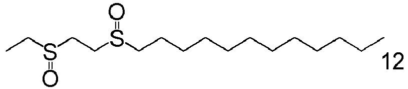 |
| J904Y | 2-(Dodecylsulfinyl)-N-methylacetamide | 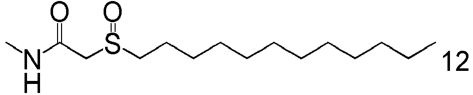 |
| J905Y | 3-(Dodecylsulfinyl)propane-1,2-diol | 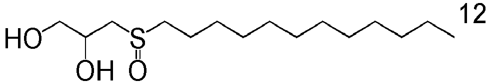 |
| J906Y | 2-(2-(Dodecylsulfinyl)ethylsulfinyl)ethanol | 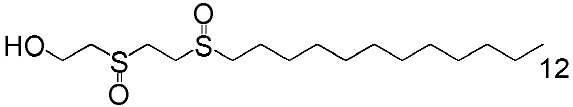 |
| J907Y | 2-(Dodecylsulfinyl)-N,N,N-trimethylethanaminium trifluoroacetate | 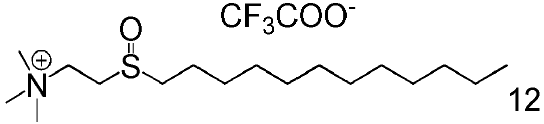 |
| J908Y | 1-(2-(2-Methoxyethoxy)ethylsulfinyl)dodecane | 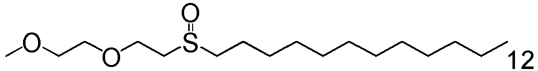 |
| J909Y | N-Methyl-2-(tetradecylsulfinyl)acetamide | 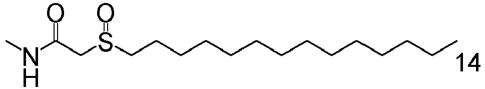 |
| J910Y | 1-Deoxy-1-(N-methyl-(N-dodecyl-N-methylglycinoyl)amino)-D-glucitol hydrotrifluoroacetate | 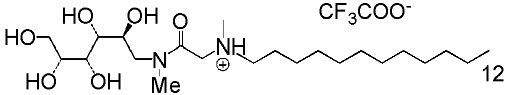 |
| J911Y | 1-Deoxy-1-(N-methyl-(N-tetradecyl-N-methylglycinoyl)amino)-D-glucitol hydrotrifluoroacetate | 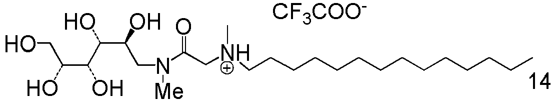 |
| J912Y | 4-(Benzyloxycarbonyl)-1-methyl-1-tetradecylpiperazin-1-ium iodide | 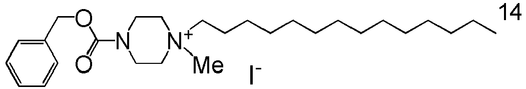 |
| J913Y | N,N,N-trimethyl-2-(tetradecylsulfinyl)ethanaminium trifluoroacetate | 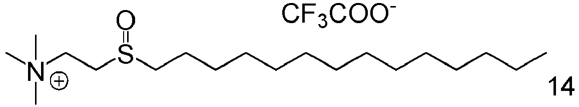 |
| J914Y | 3-(Tetradecylsulfinyl)propane-1,2-diol | 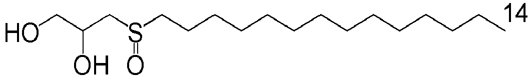 |
| J915Y | 1-(2-(2-Methoxyethoxy)ethylsulfinyl)tetradecane | 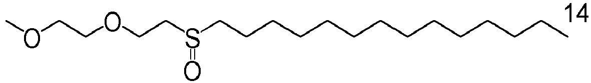 |
| J916Y | 2-Dodecyl-1,3-dioxo-1,3-dithian | 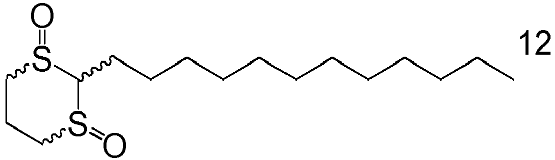 |
| J917Y | N,N-Dimethylhexadecylamine-N-oxide | 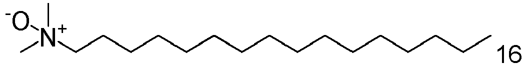 |
| J918Y | 1-(Dodecylsulfinyl)-2-(methylsulfinyl)benzene | 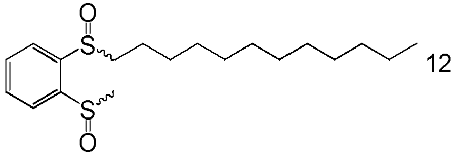 |
| J96Y | 3-(Octadecanoylamino)-1-(4-phenoxy-3-sulfophenyl)-2-pyrazolin-5-one | 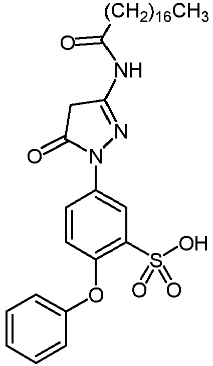 |
| J97Y | 5-Octadecyloxyisophthali c acid | 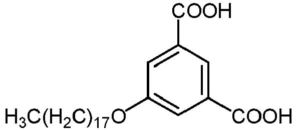 |
| J98Y | 4-(Octadecanoylamino)-benzoylacetic acid [5-carboxy-2-(3,5-dicarboxyphenoxy)-anilid | 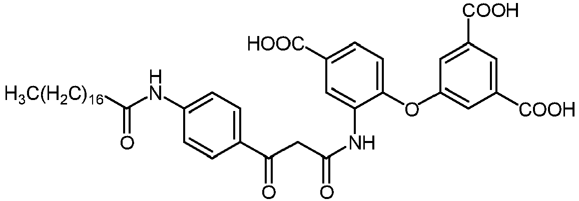 |

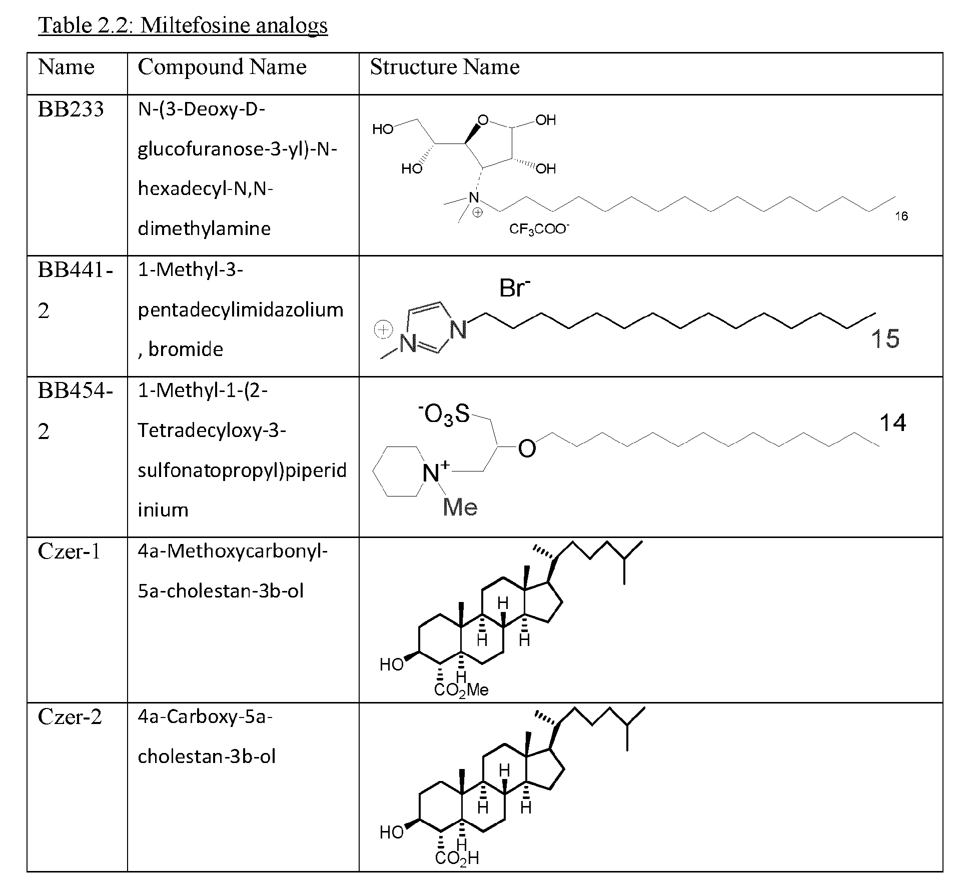

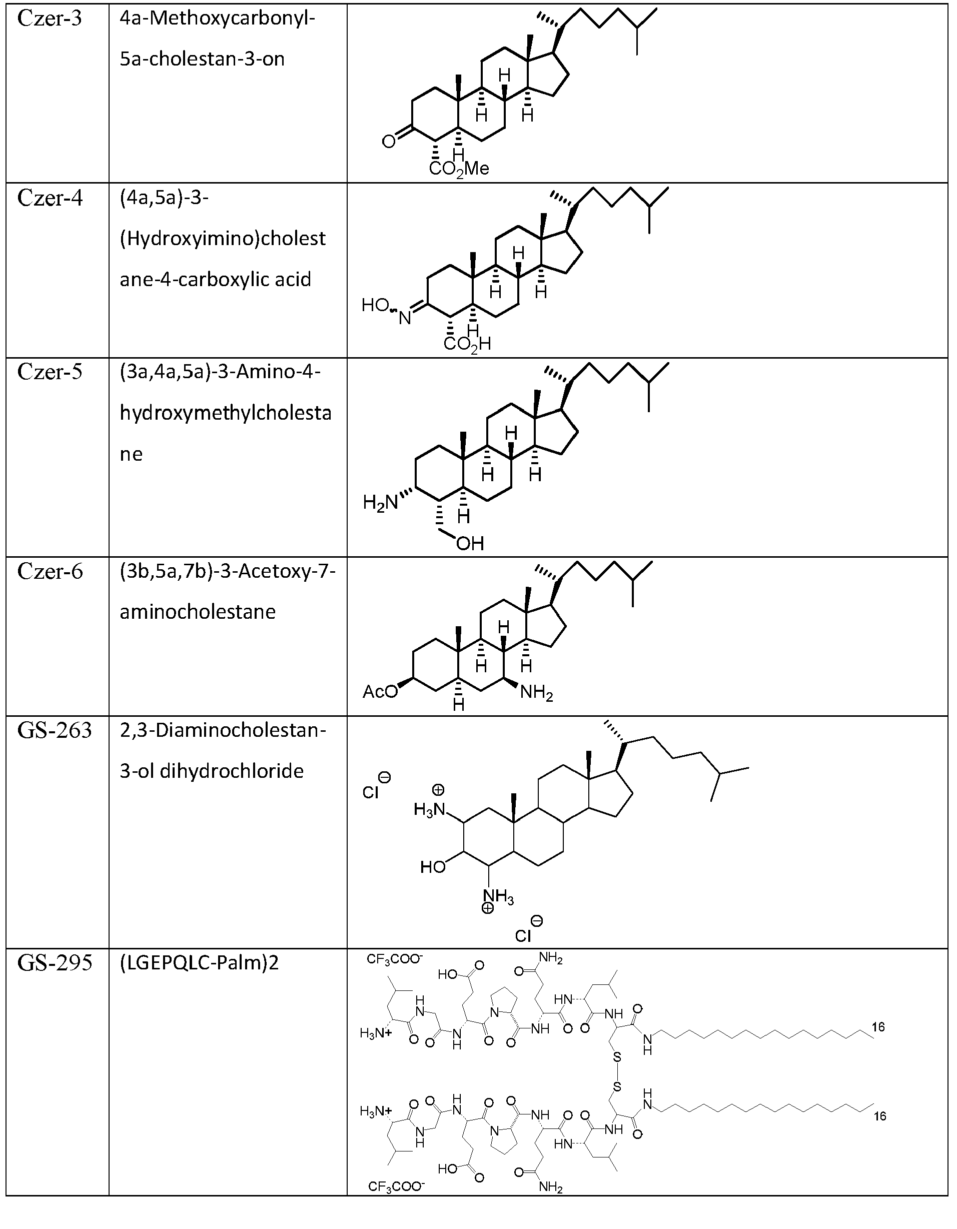

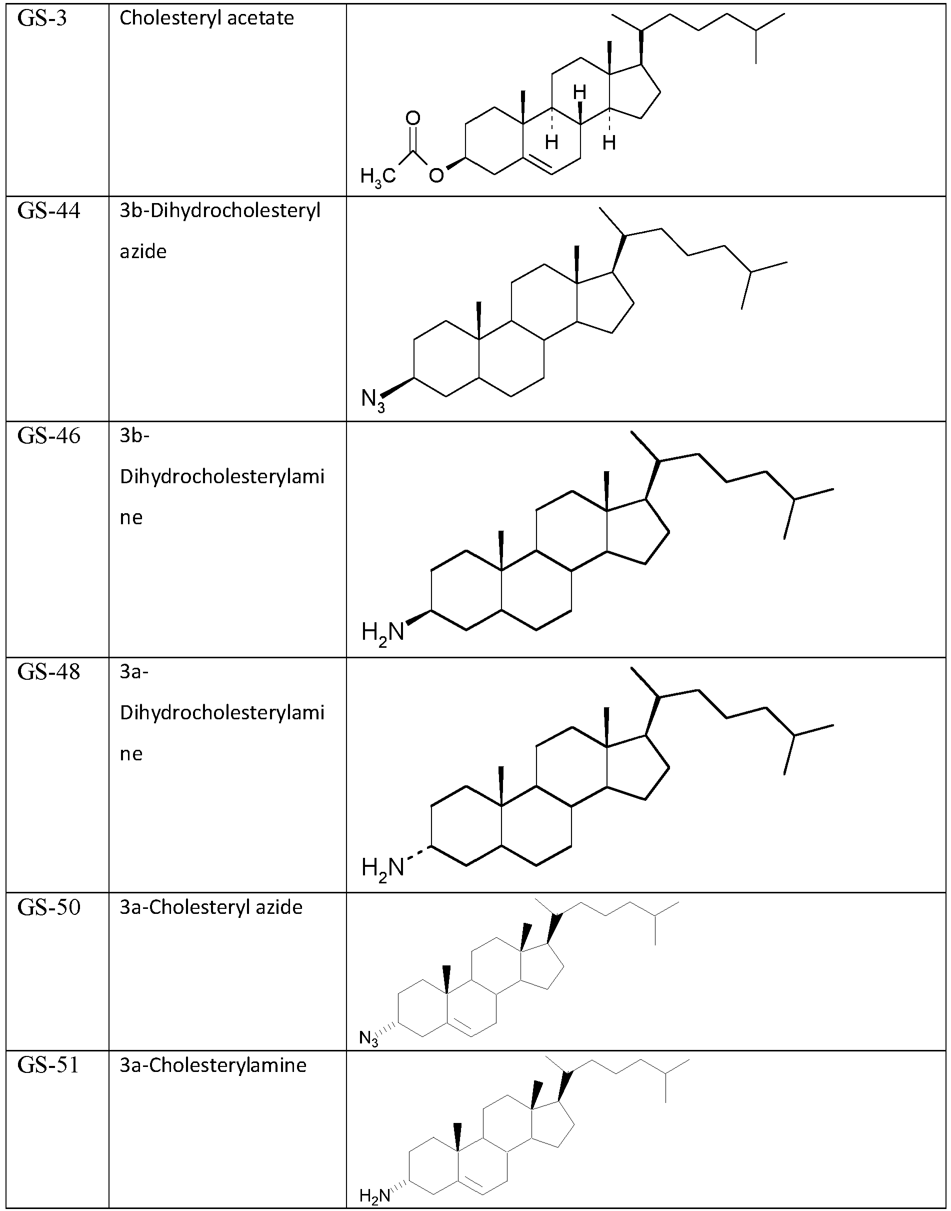

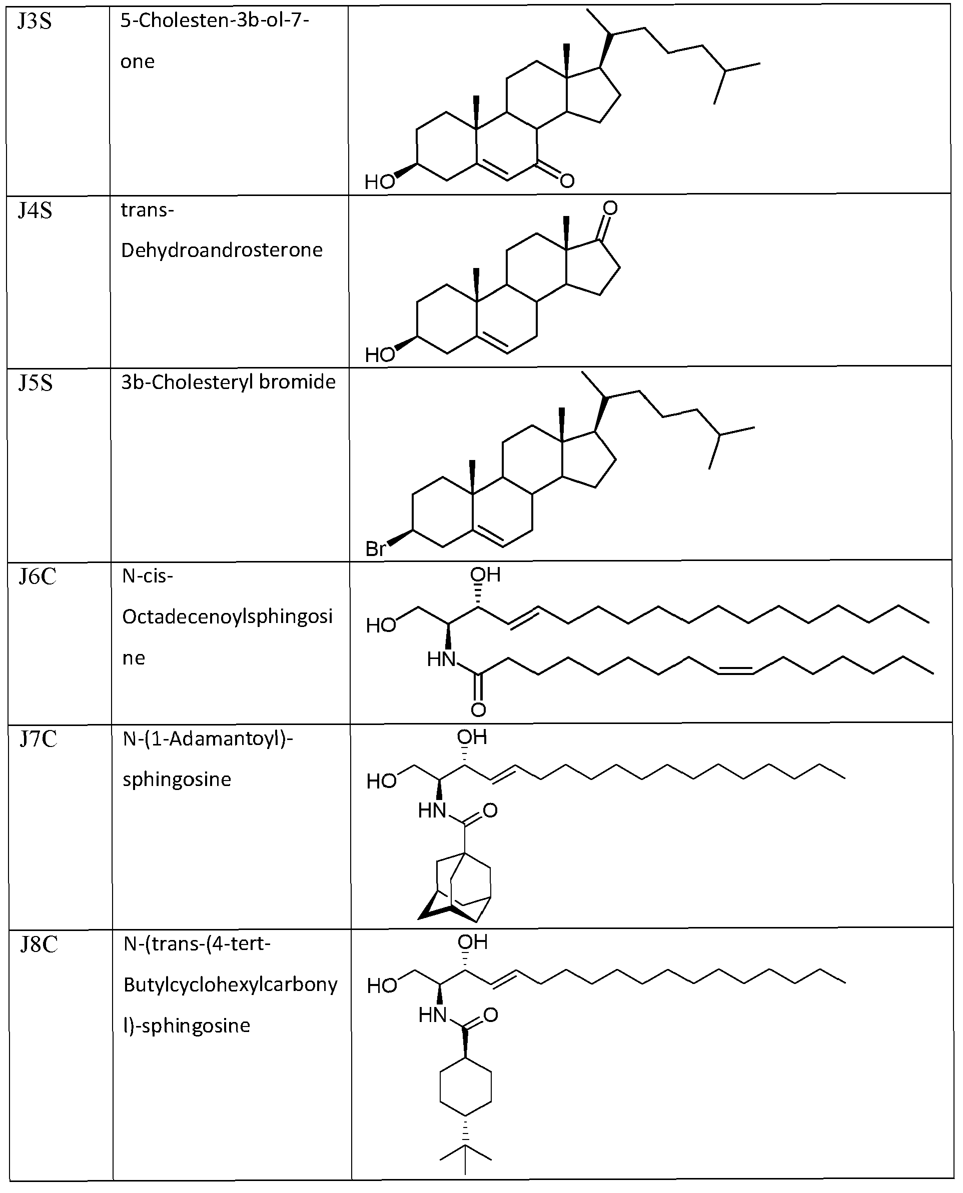

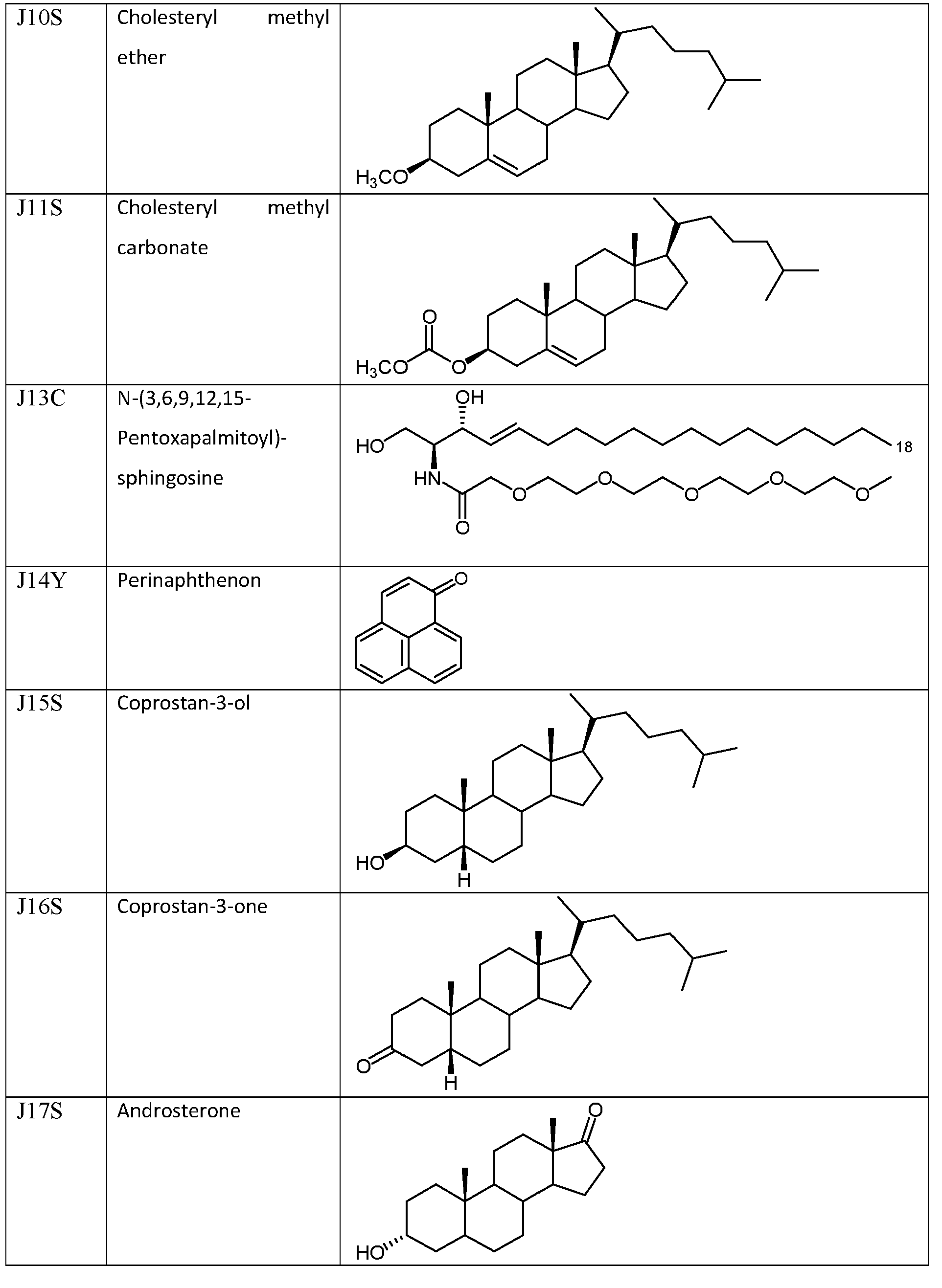

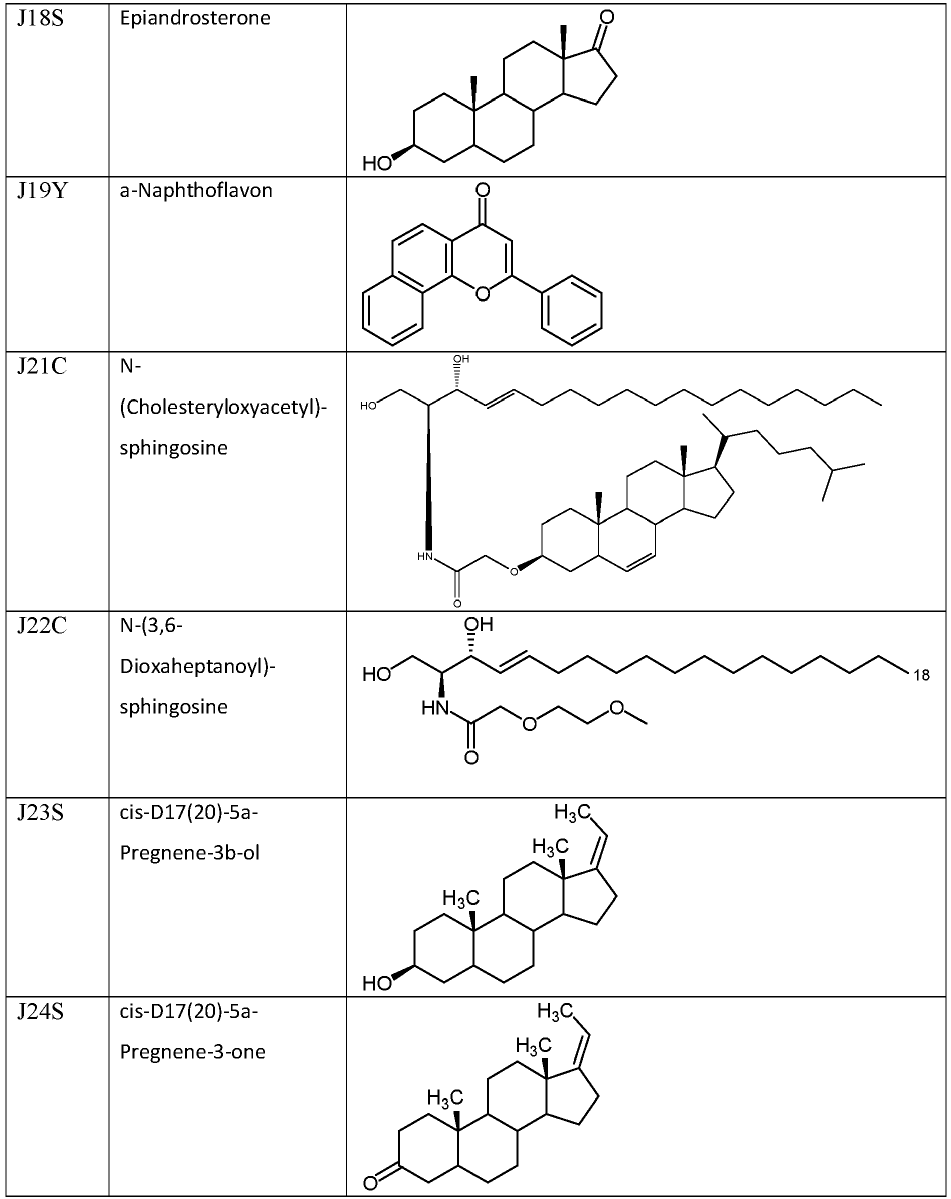

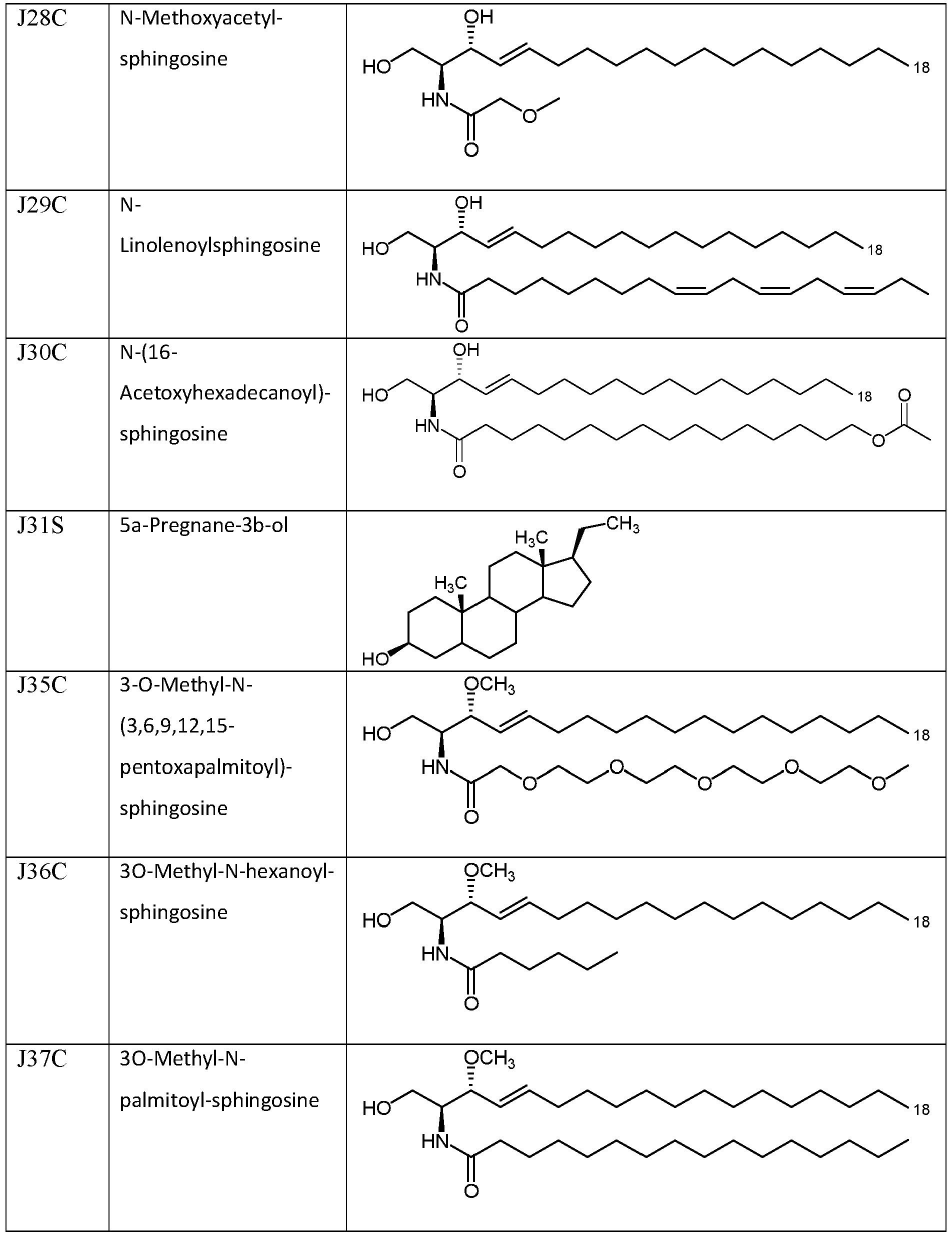

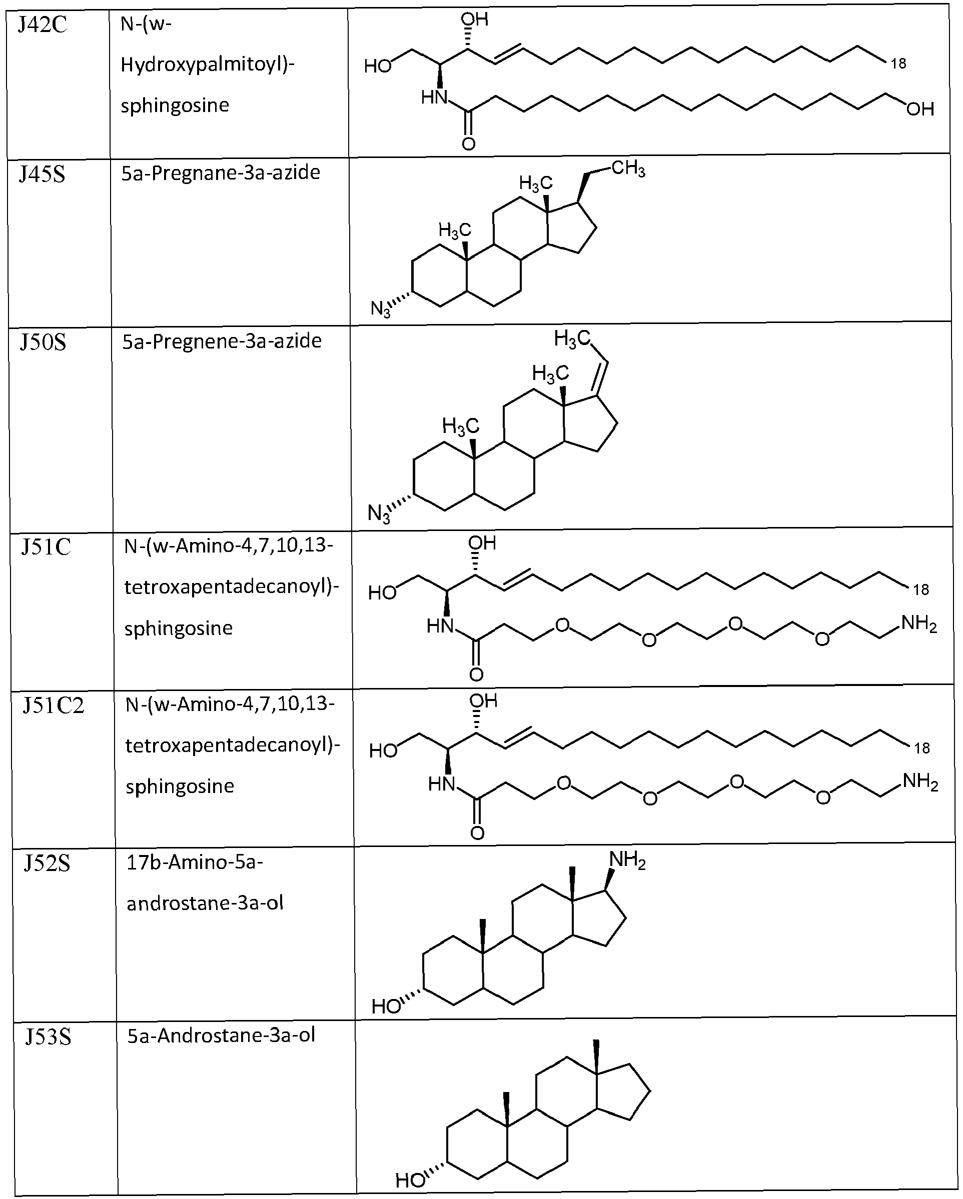

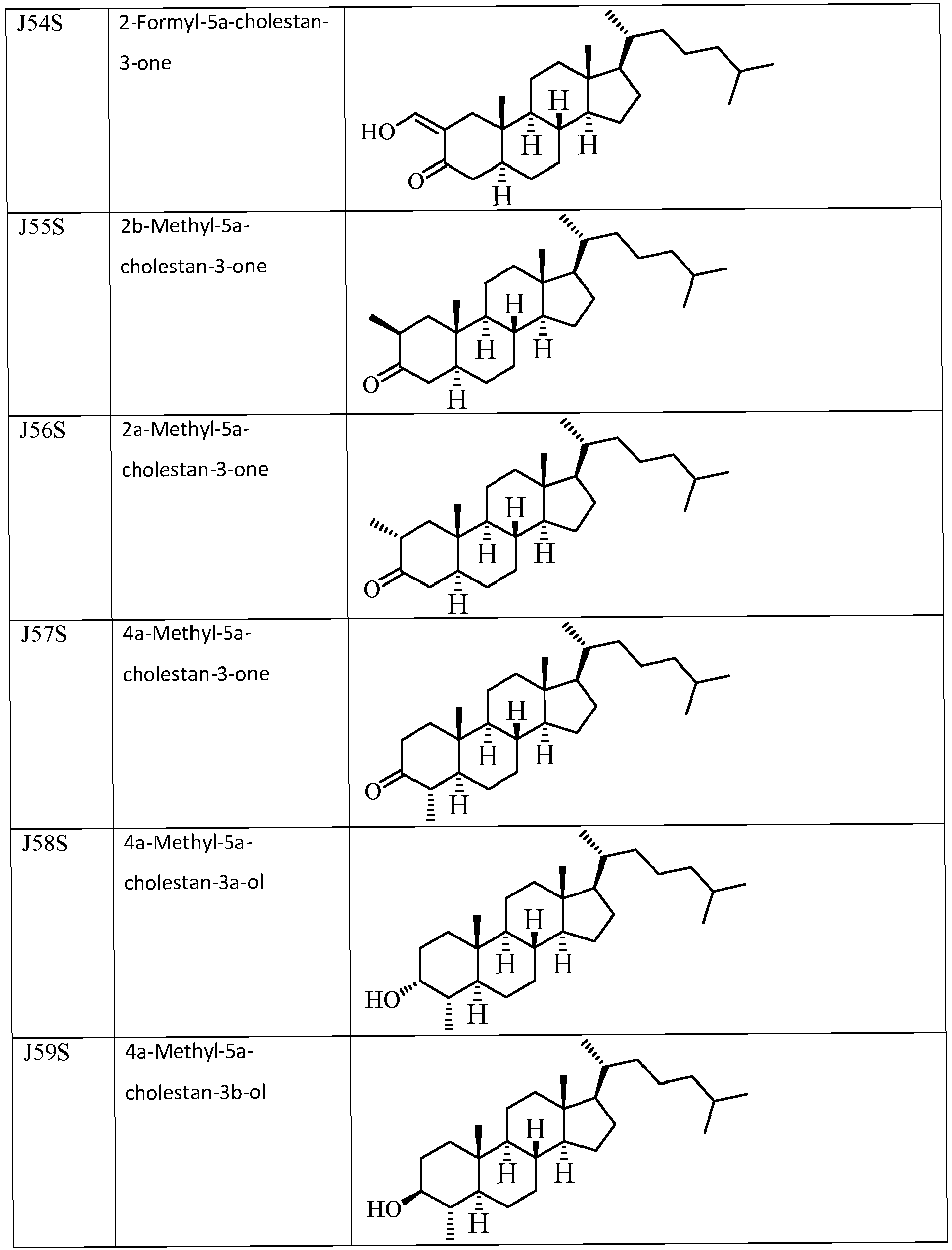

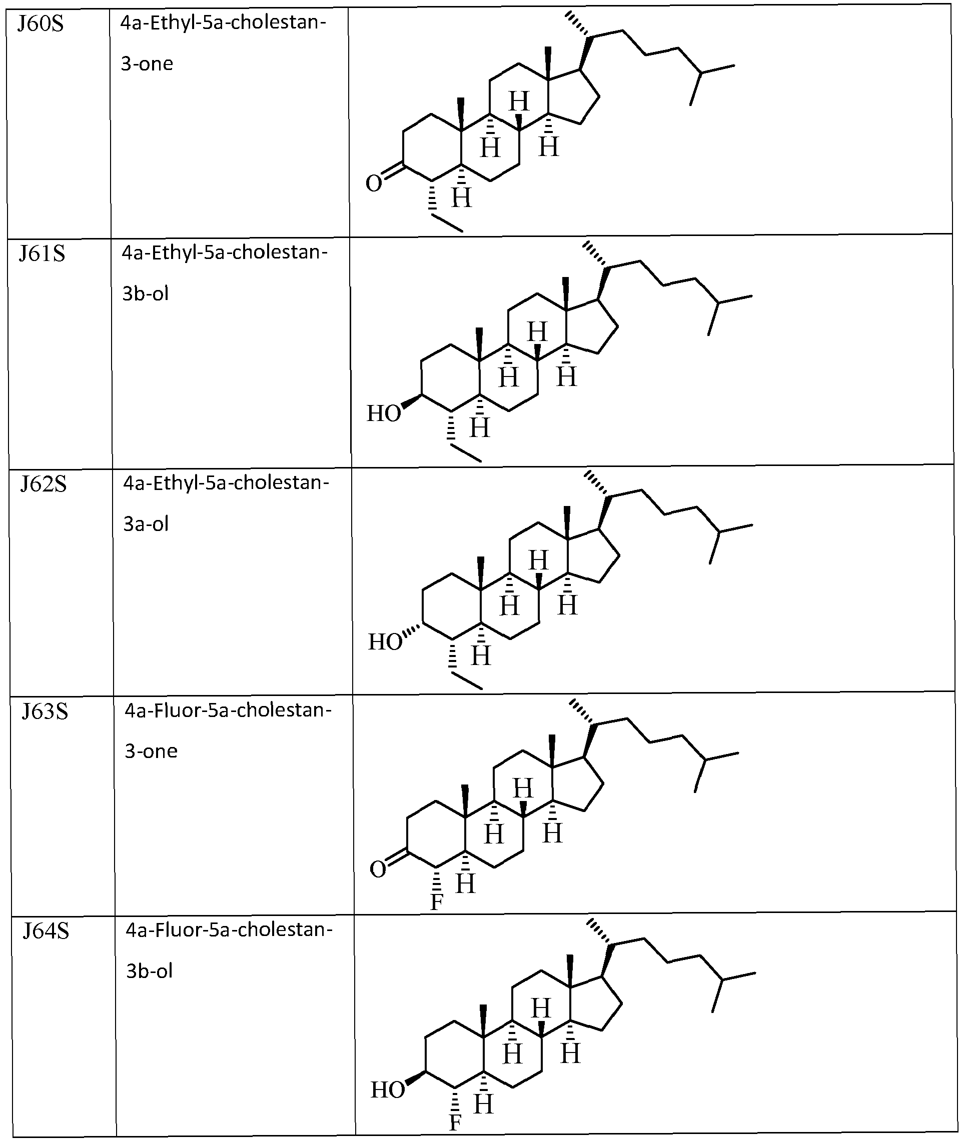

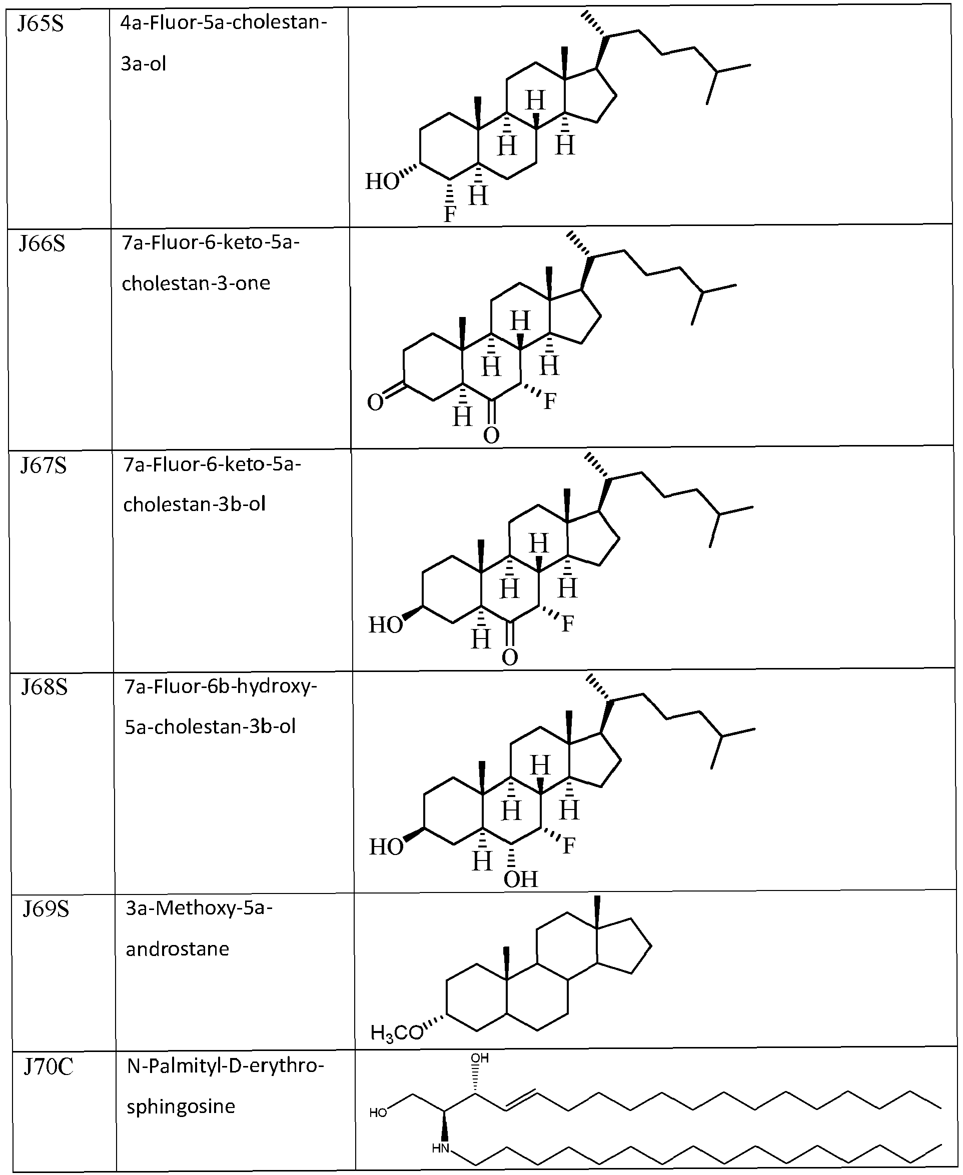

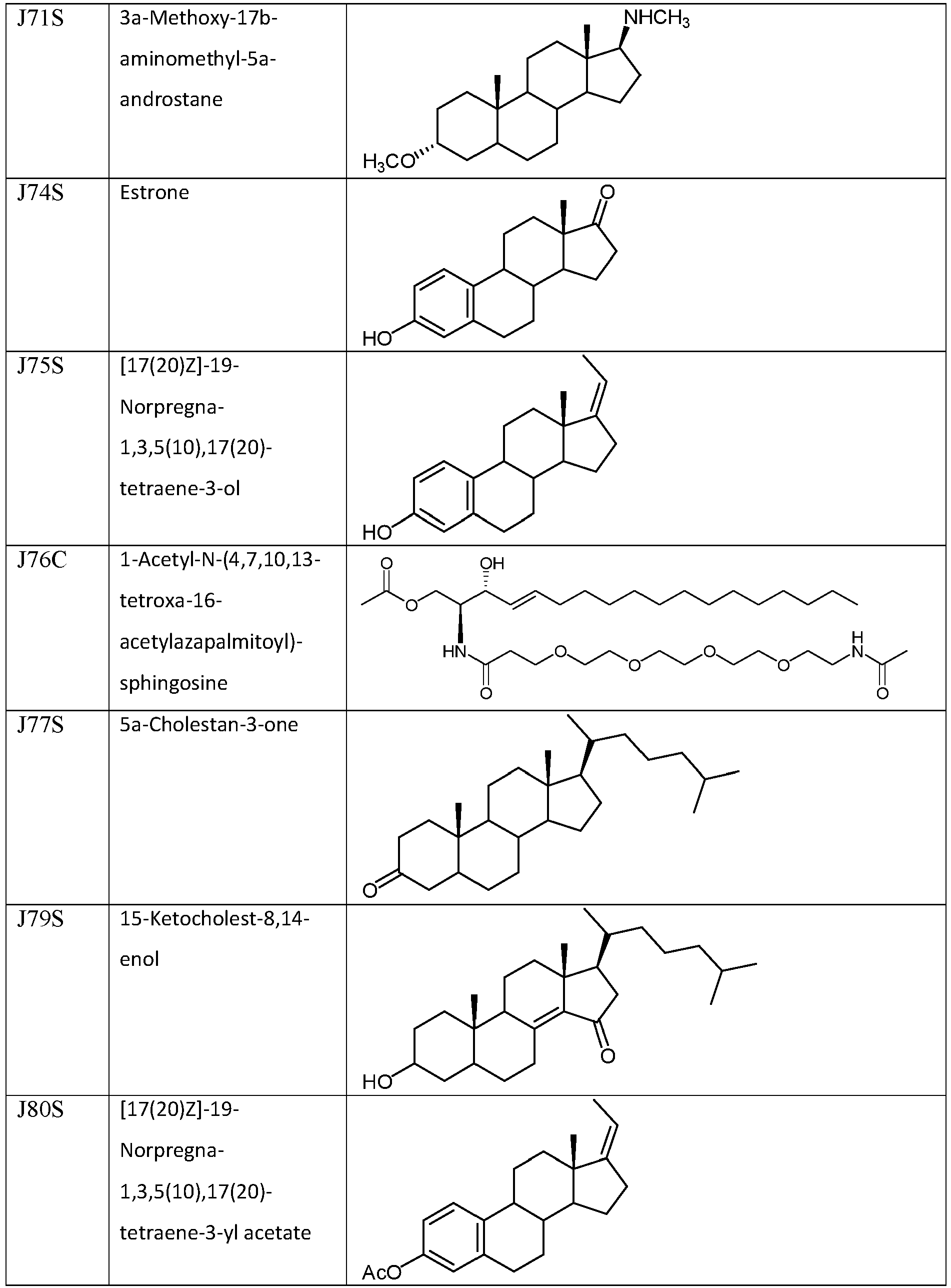

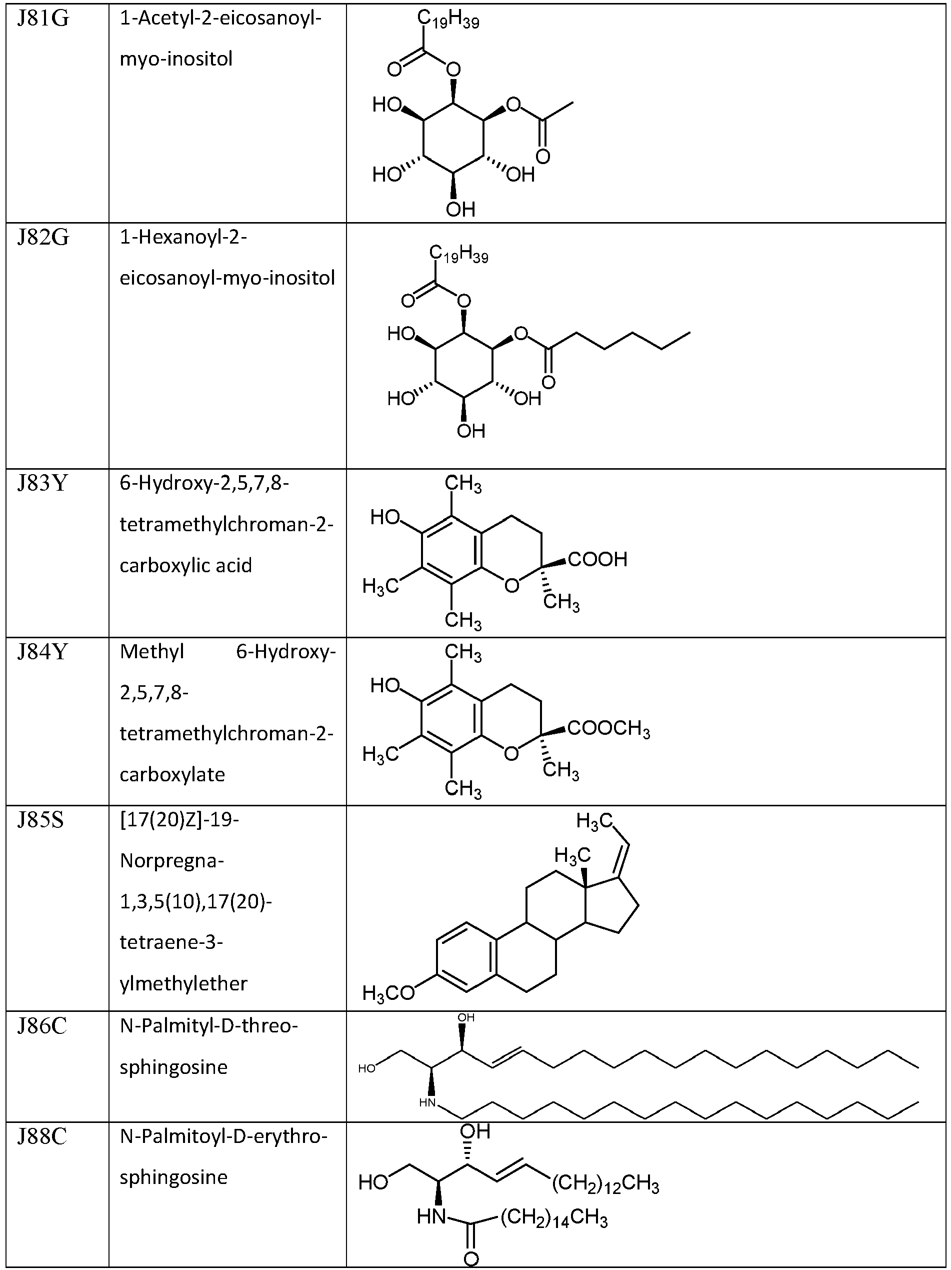

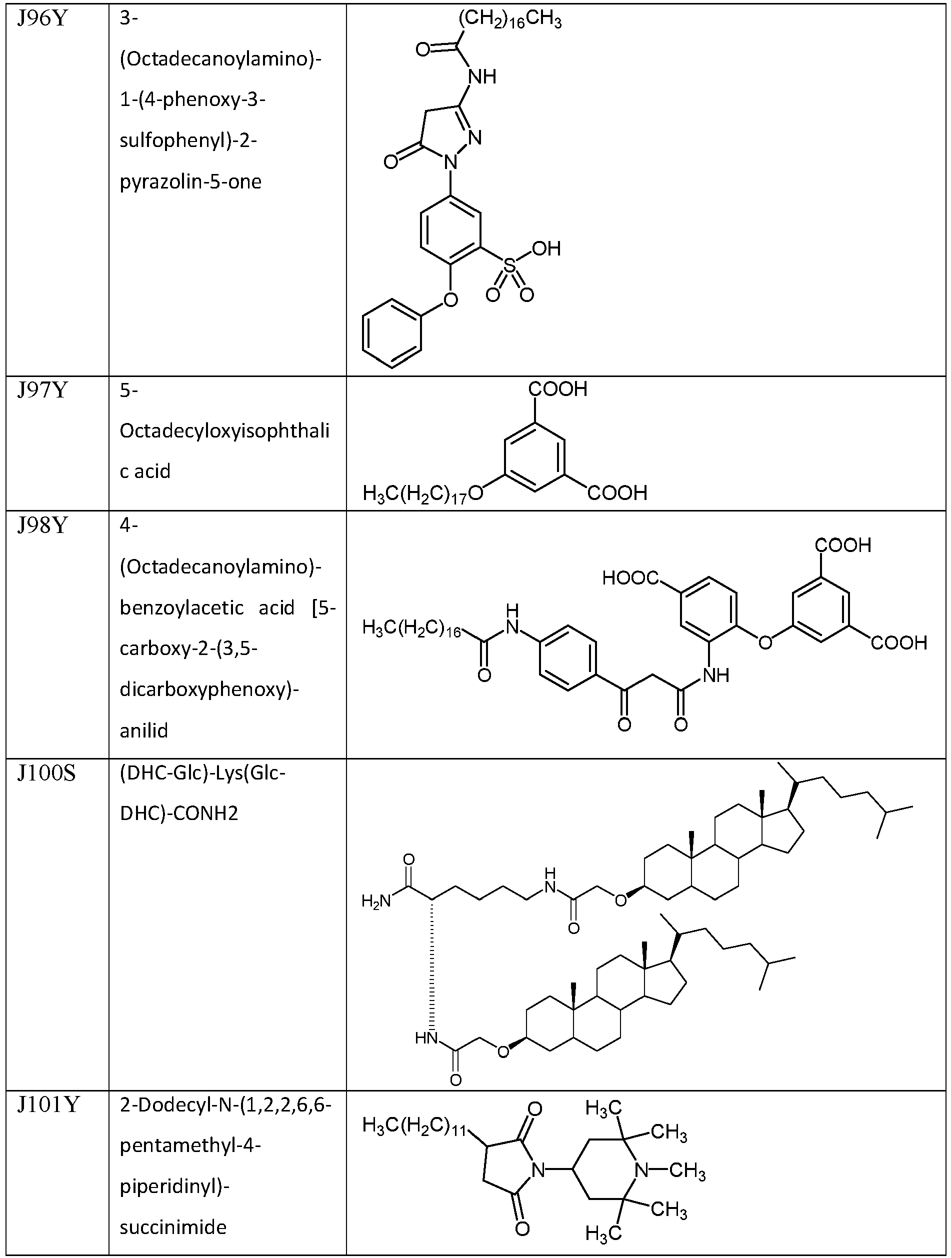

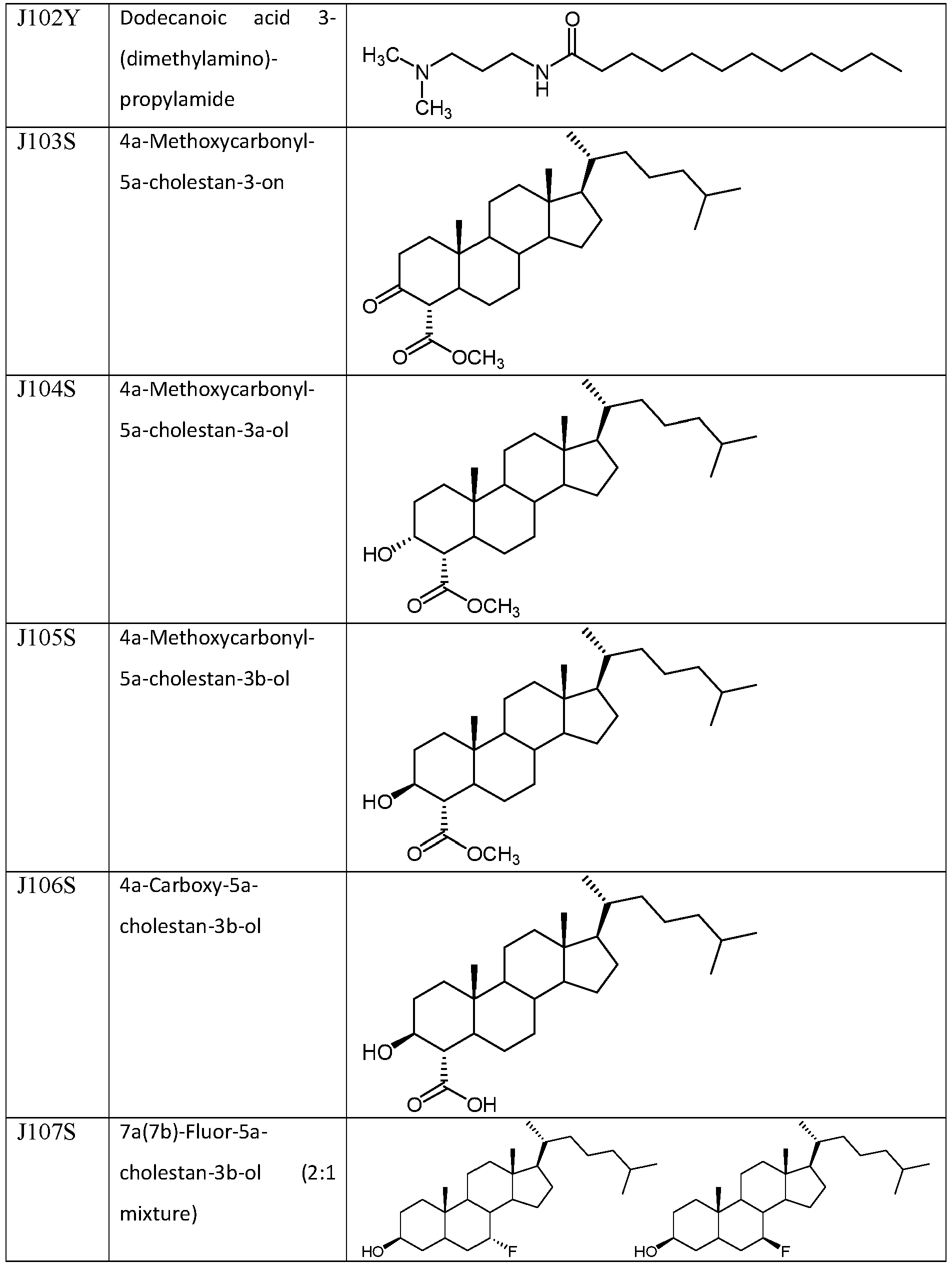

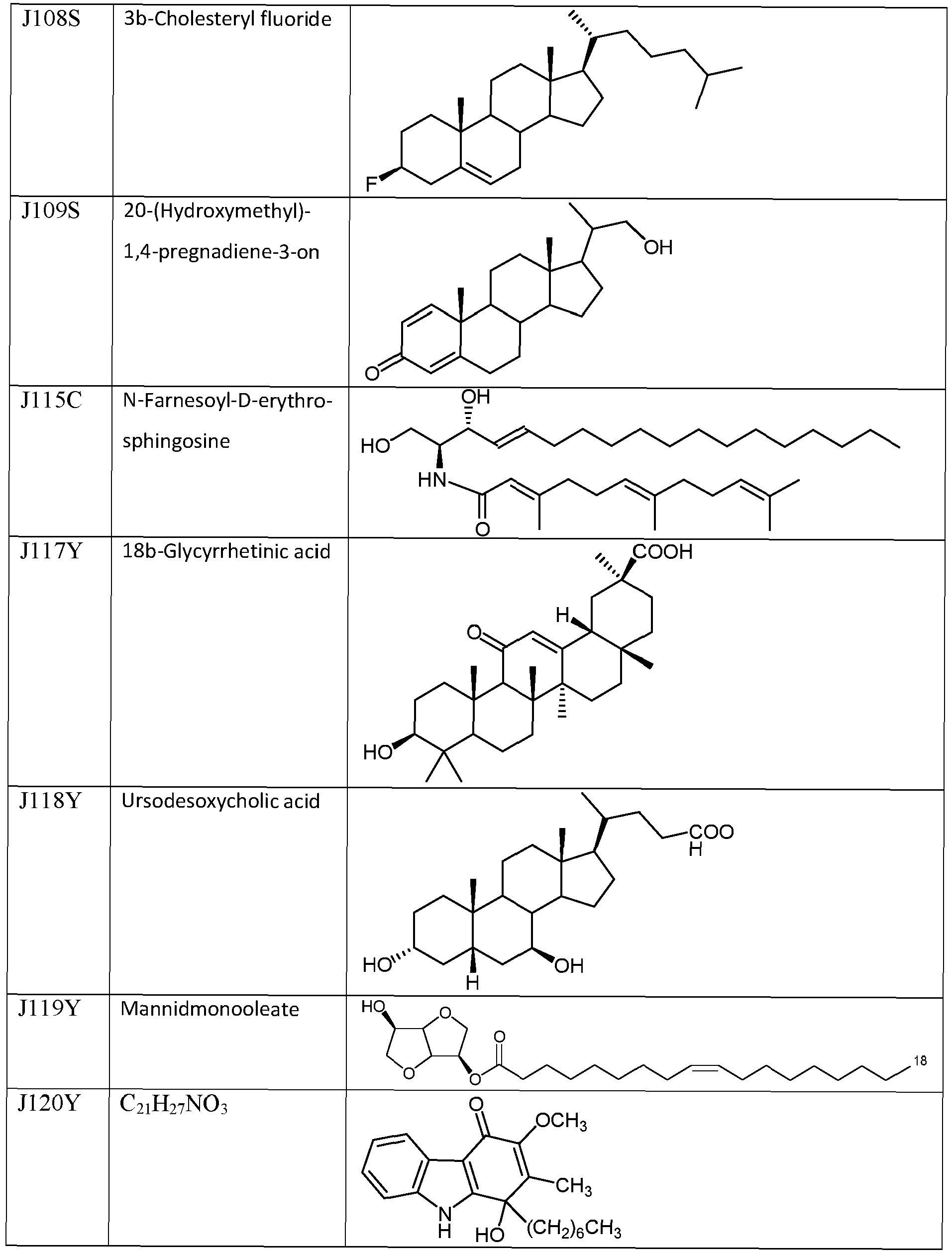

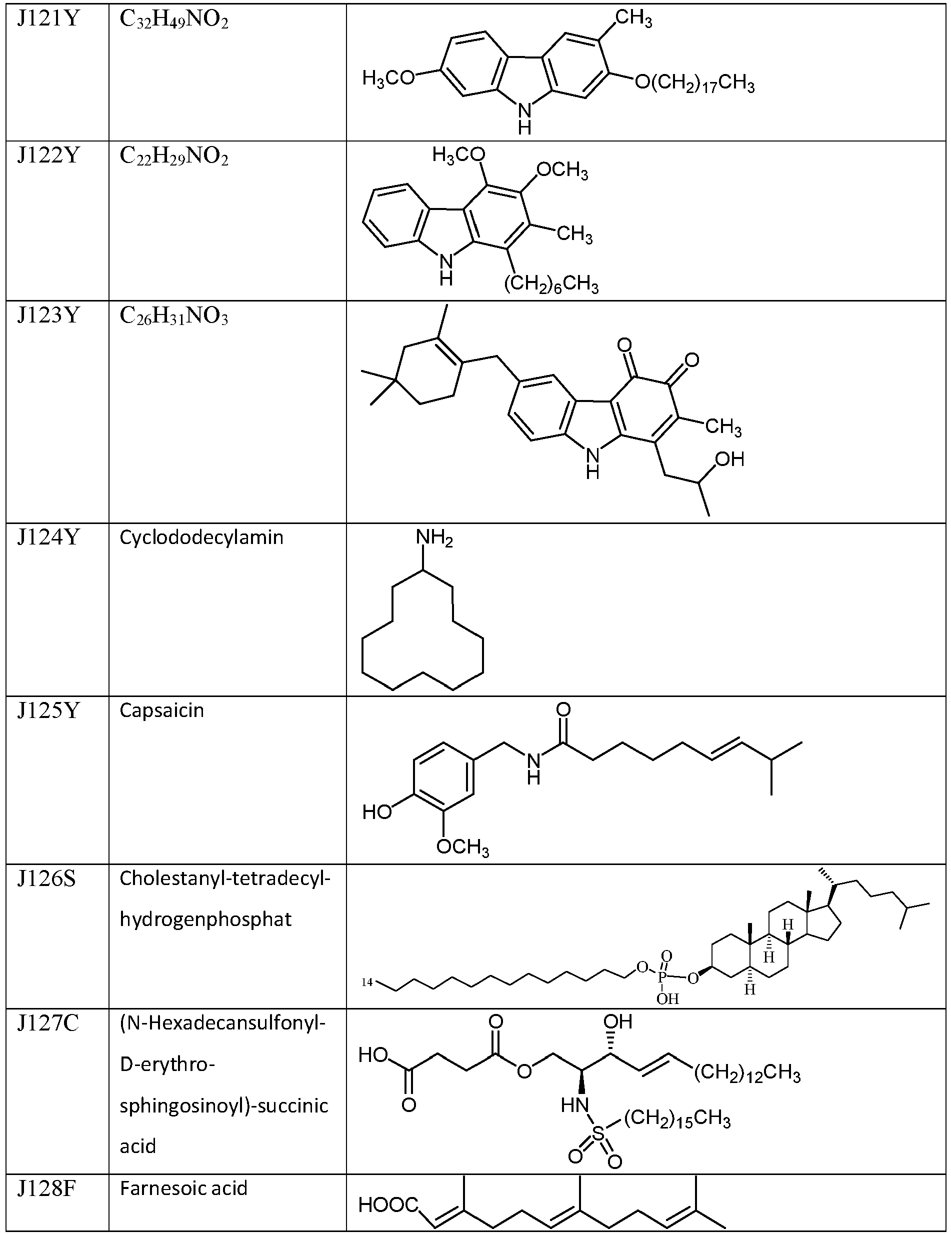

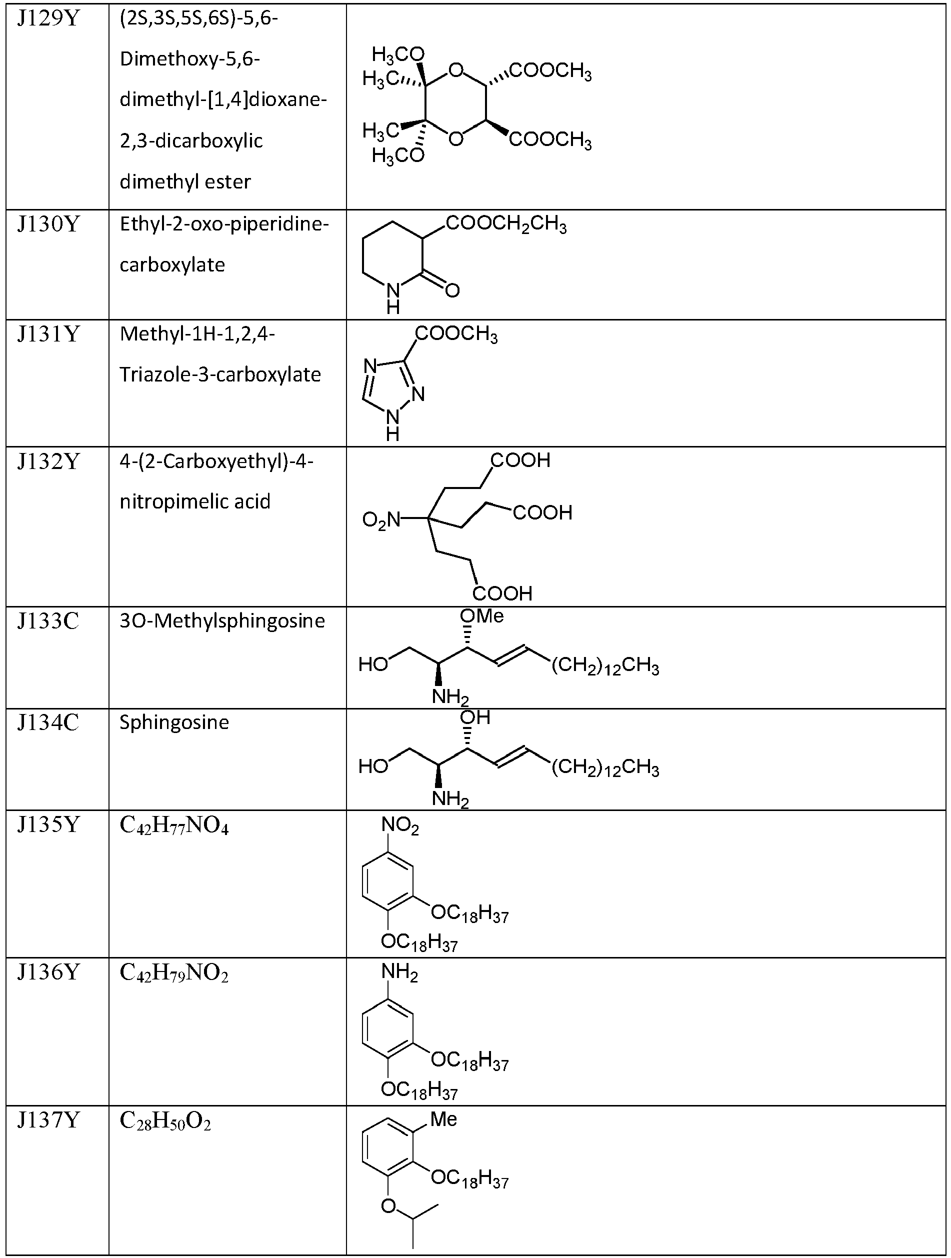

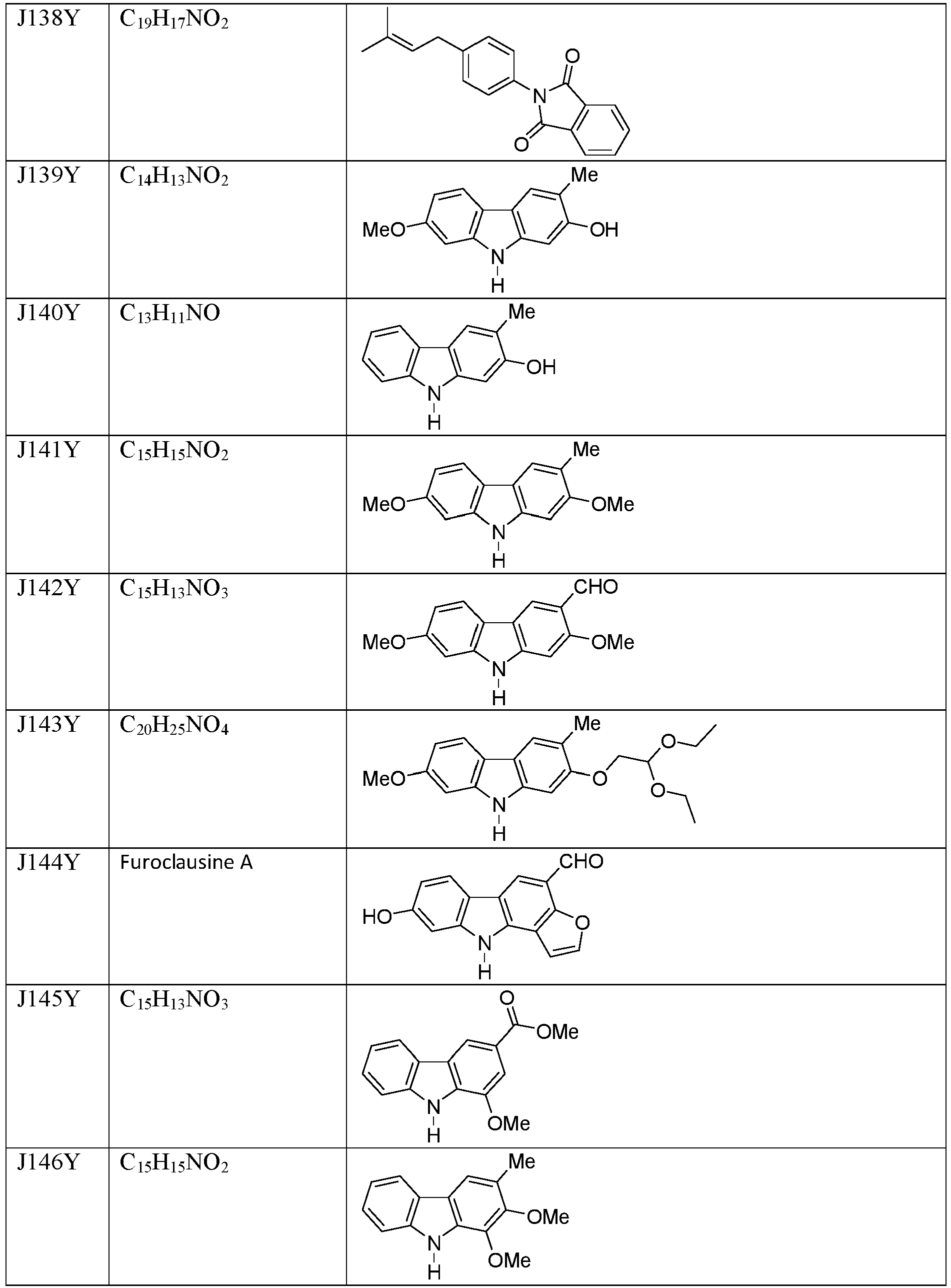

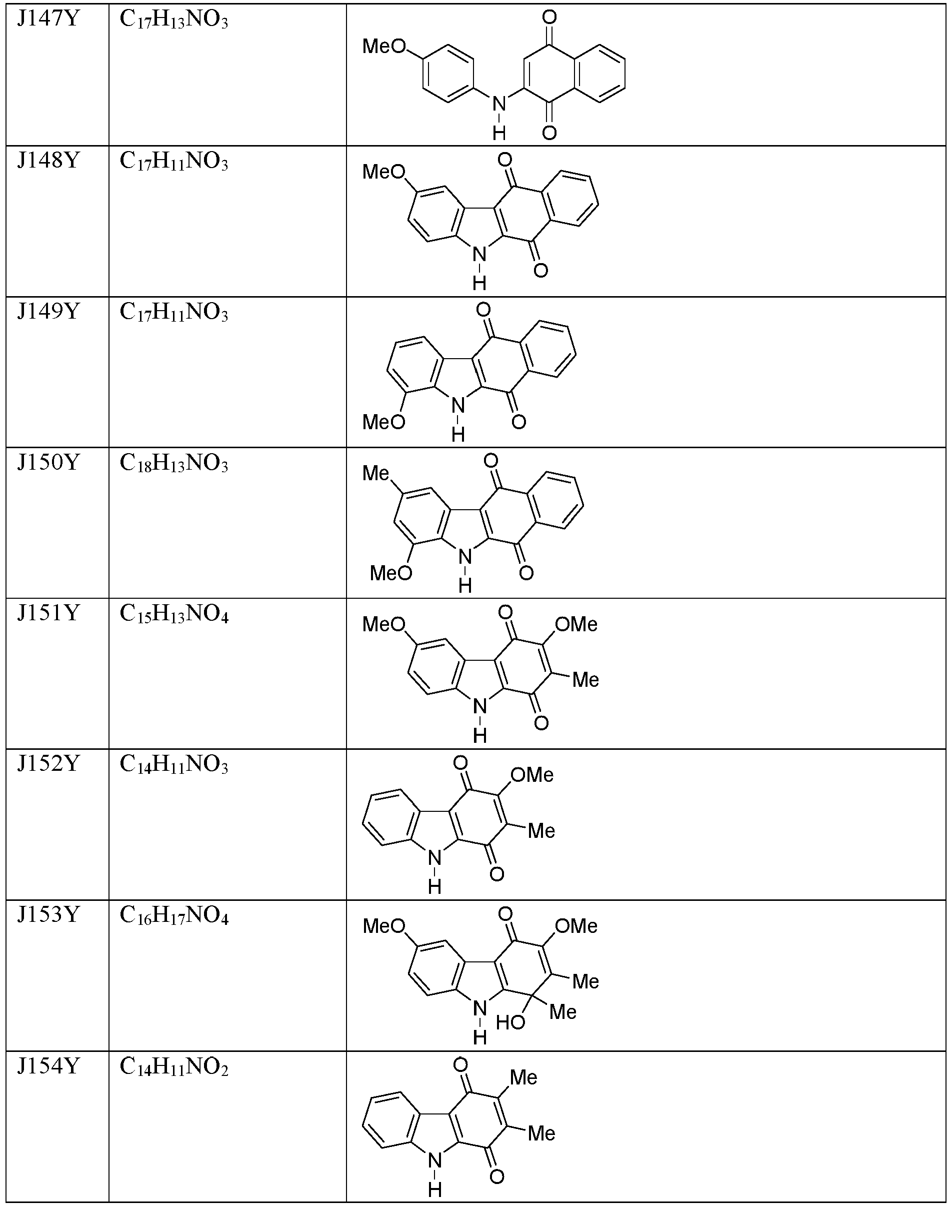

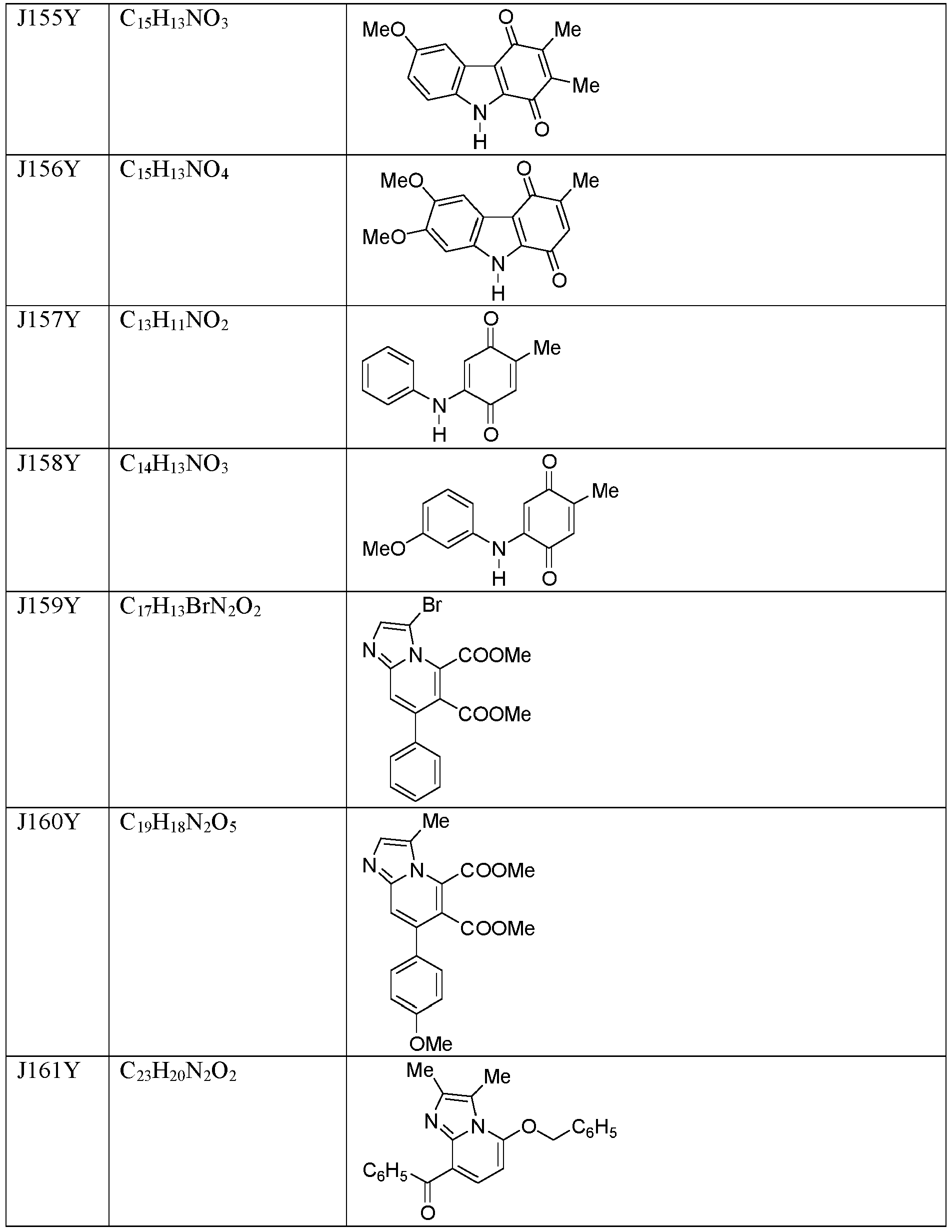

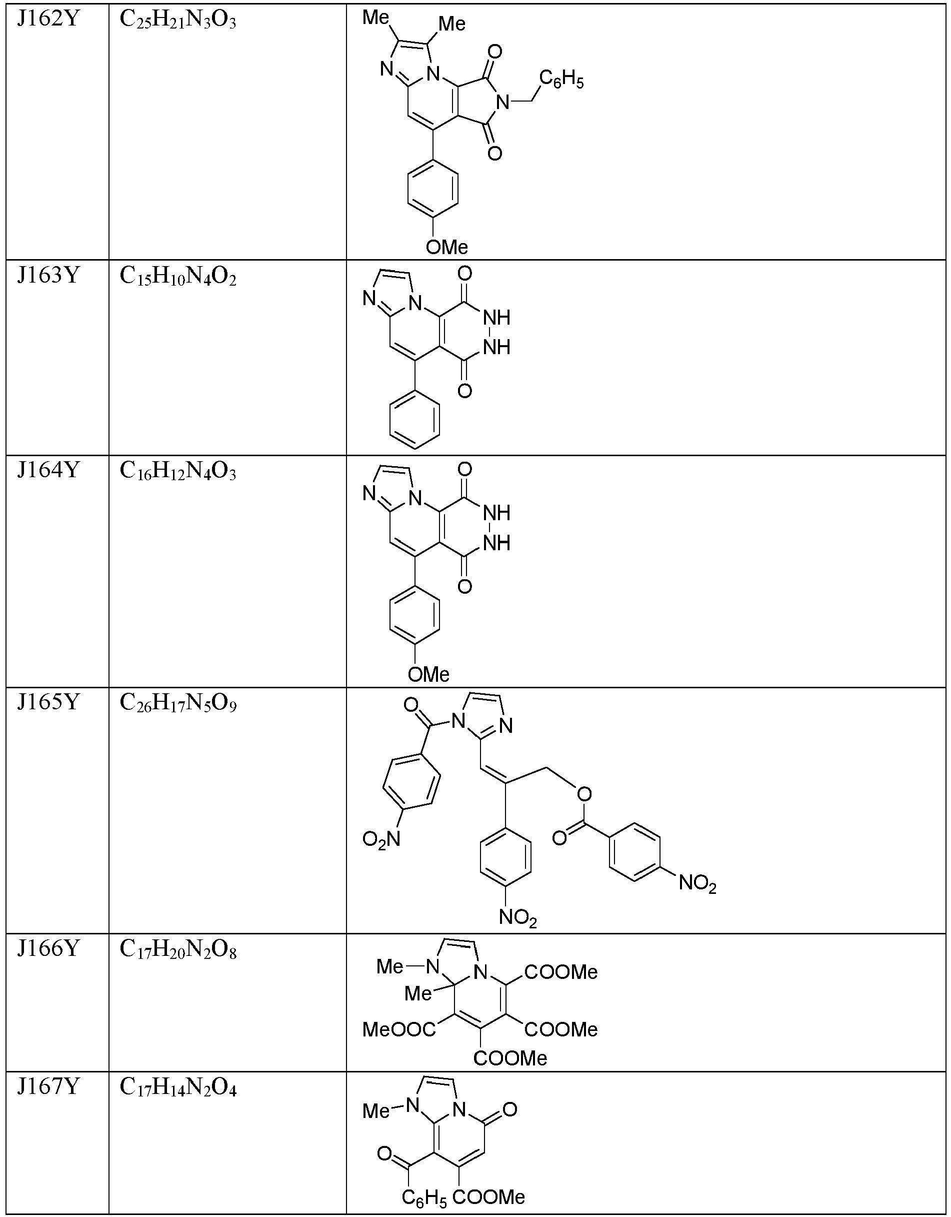

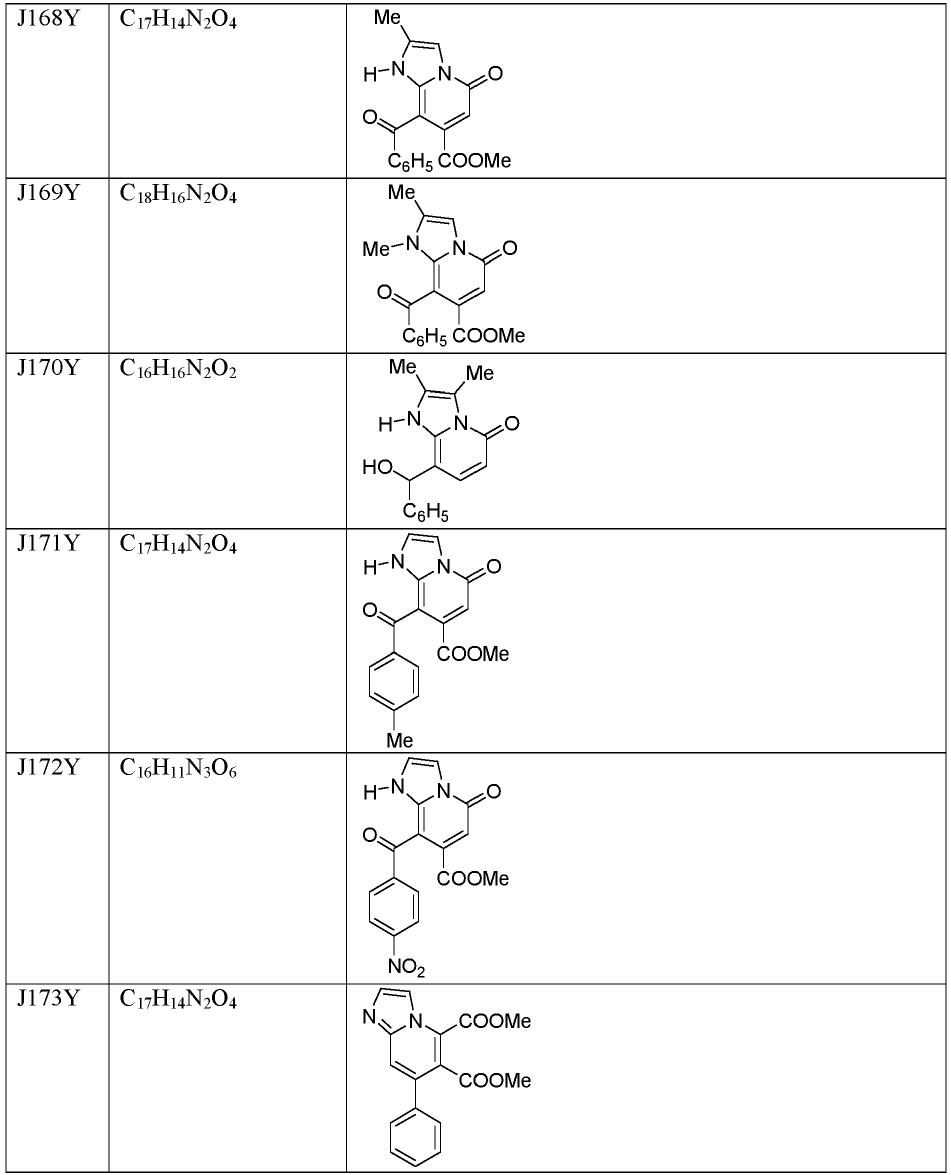

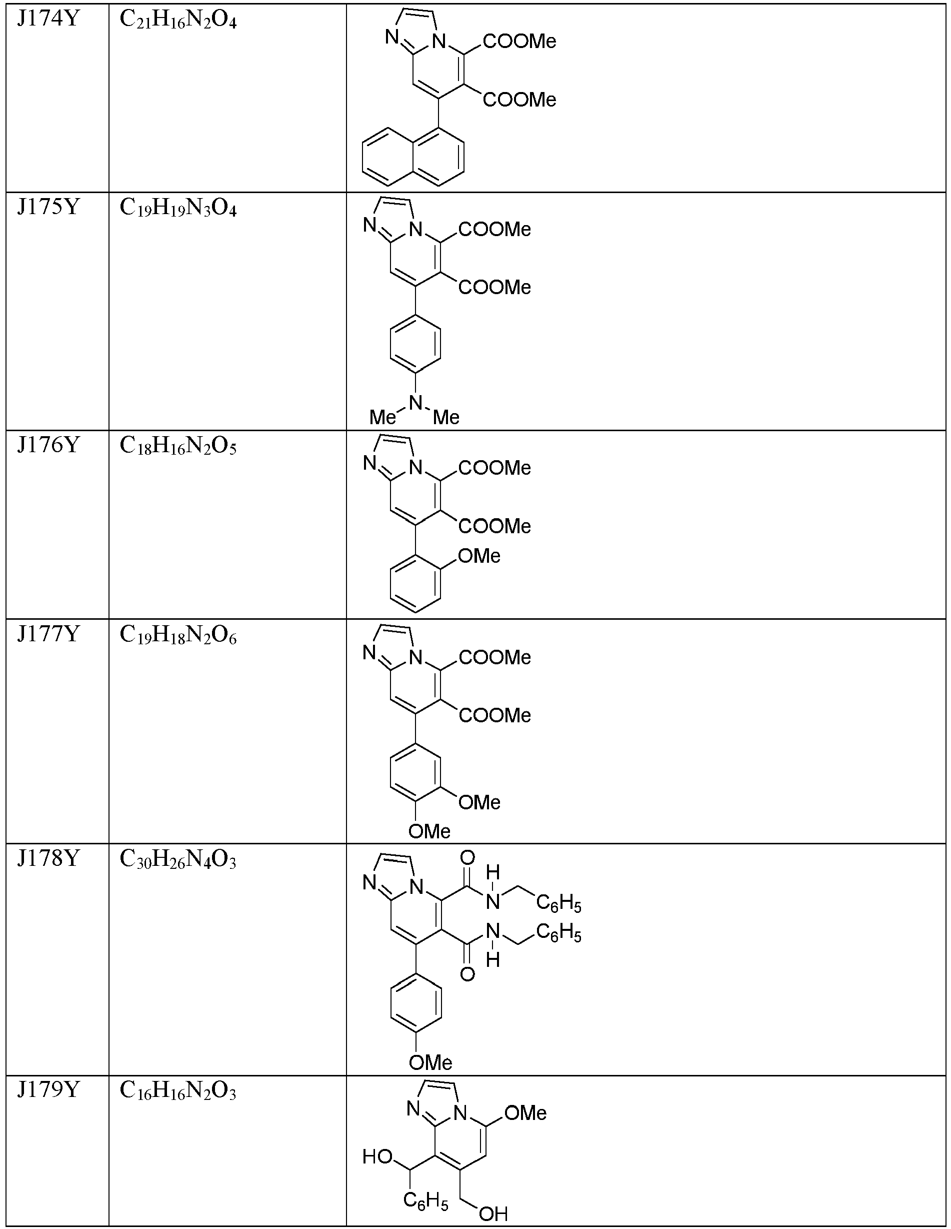

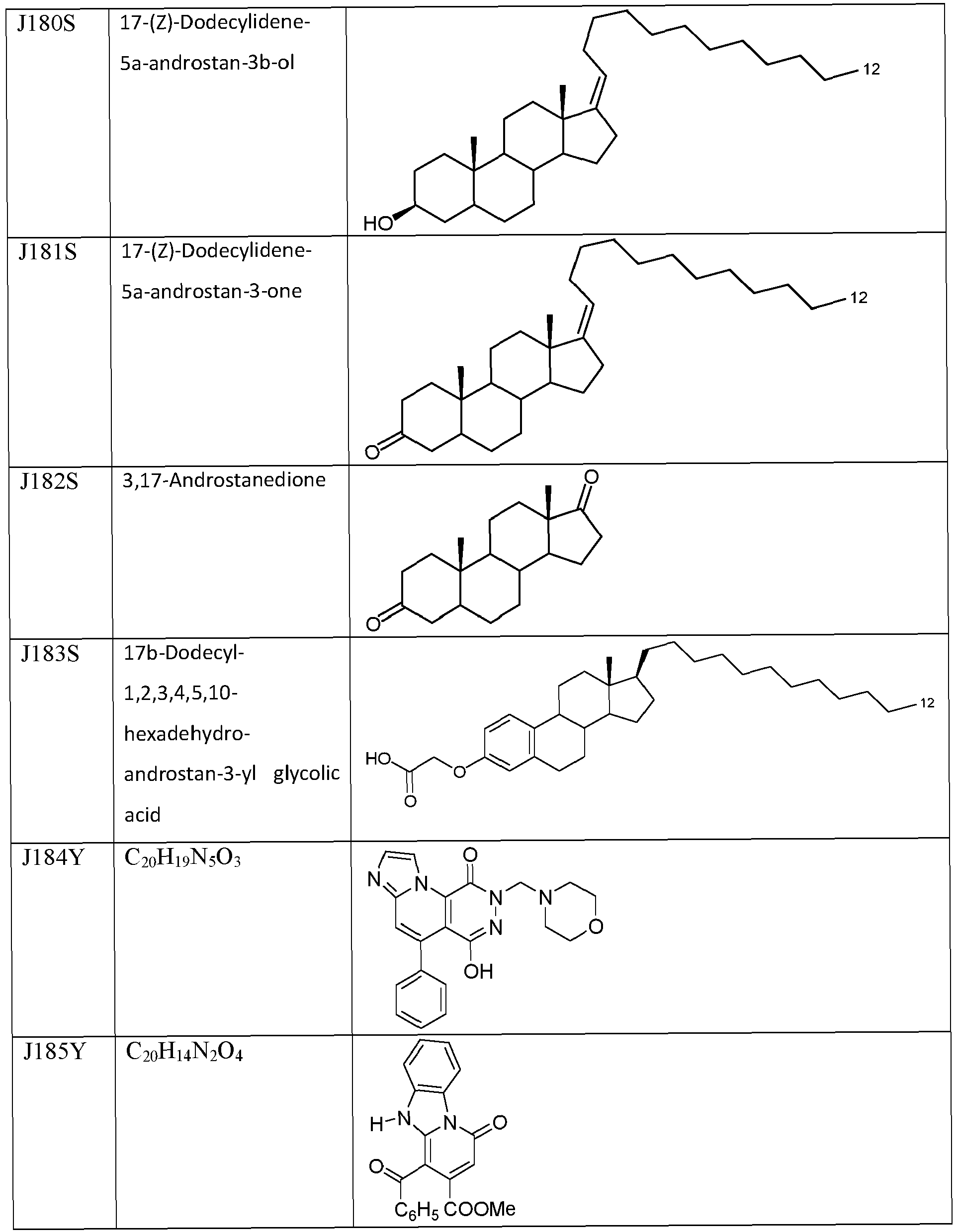

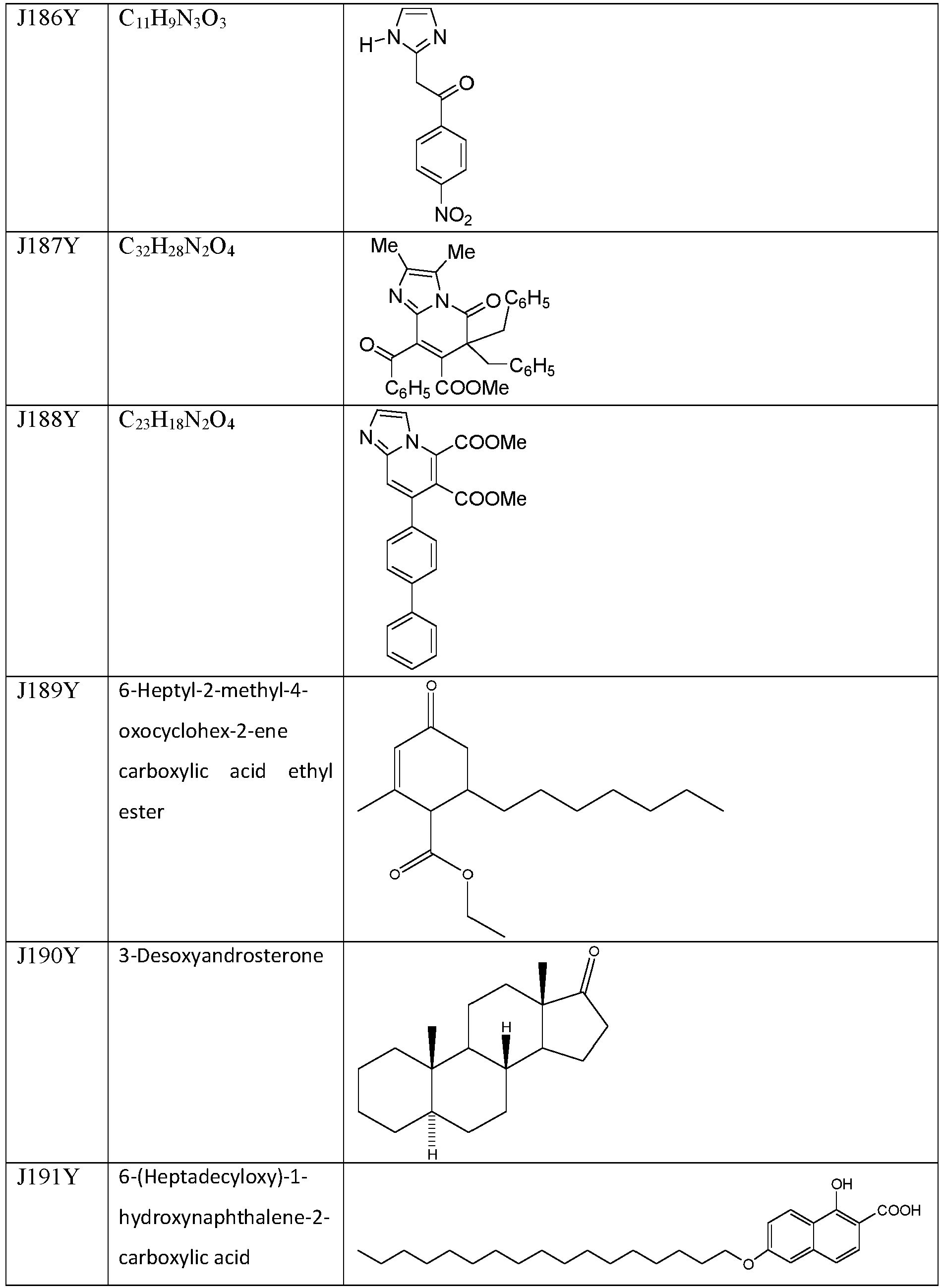

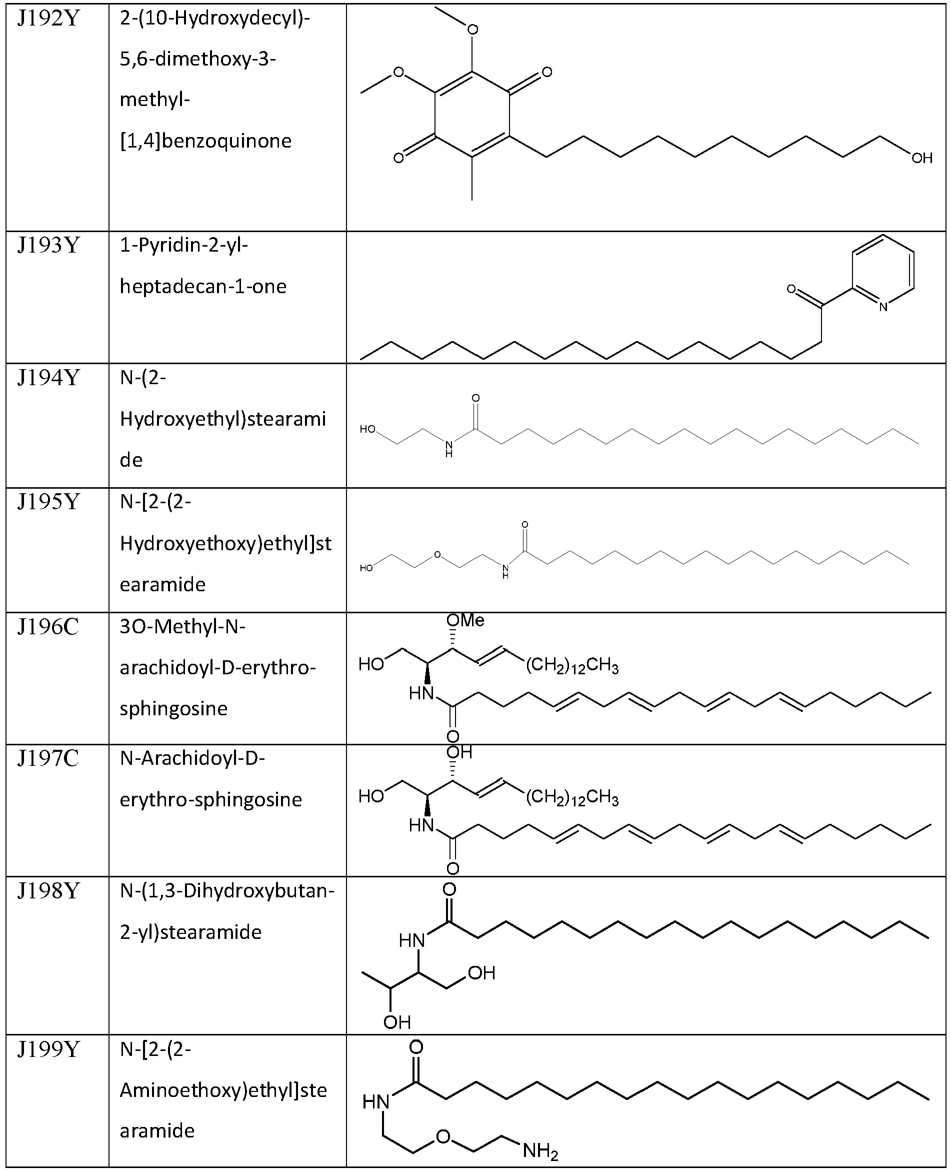

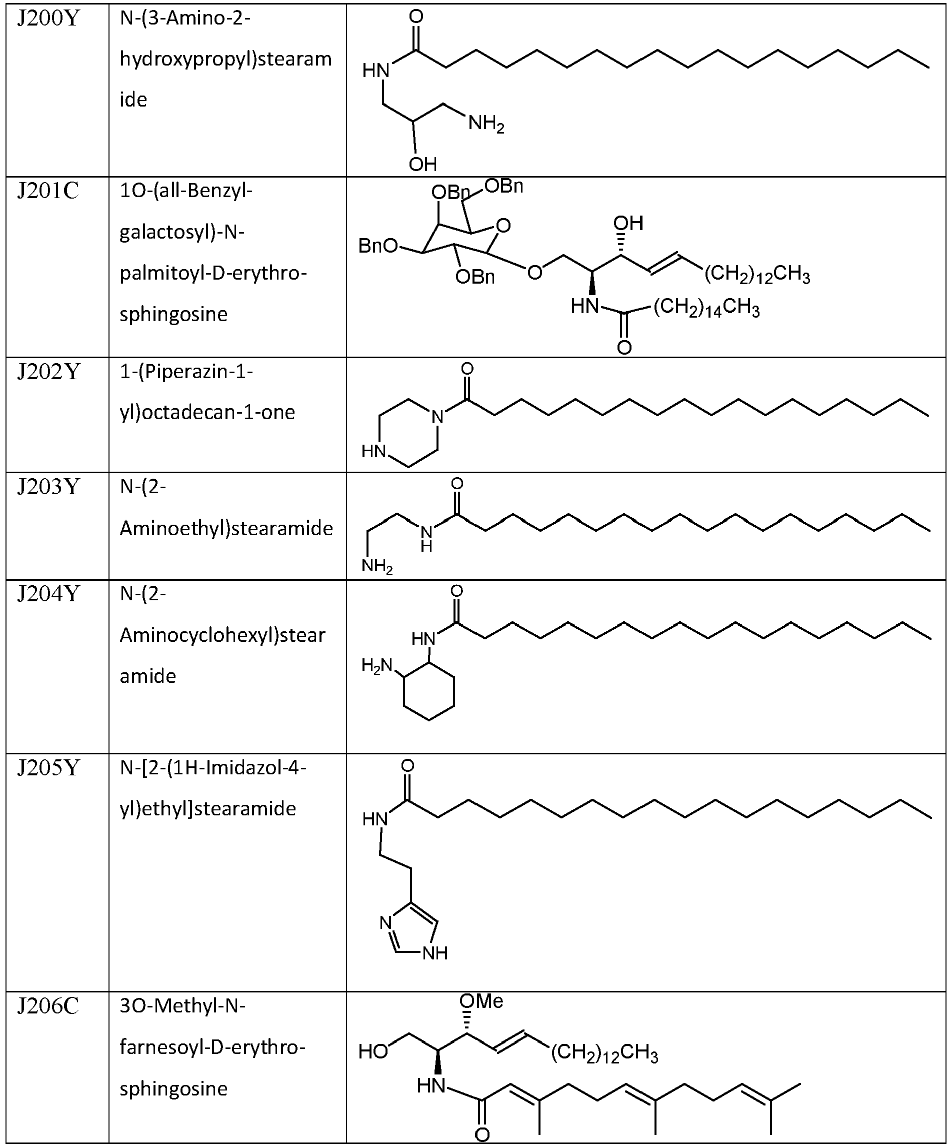

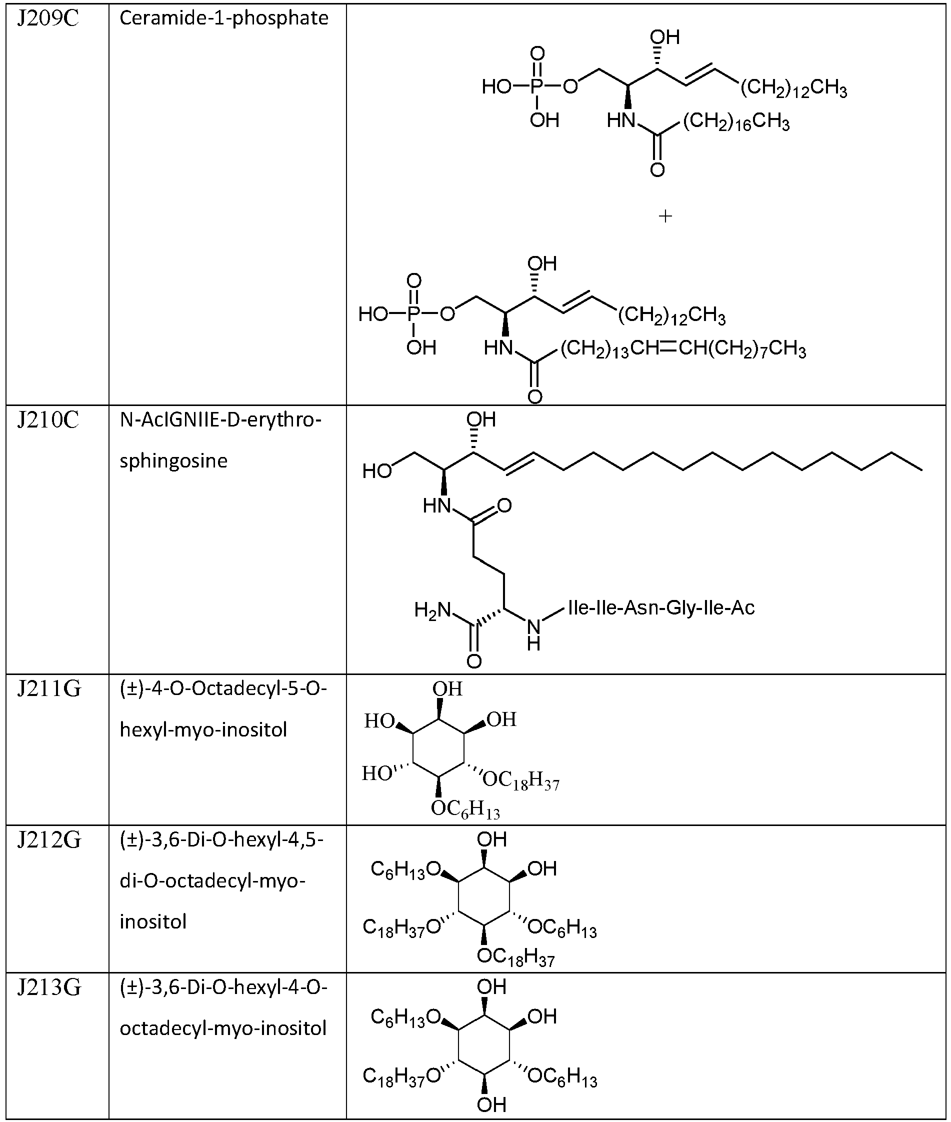

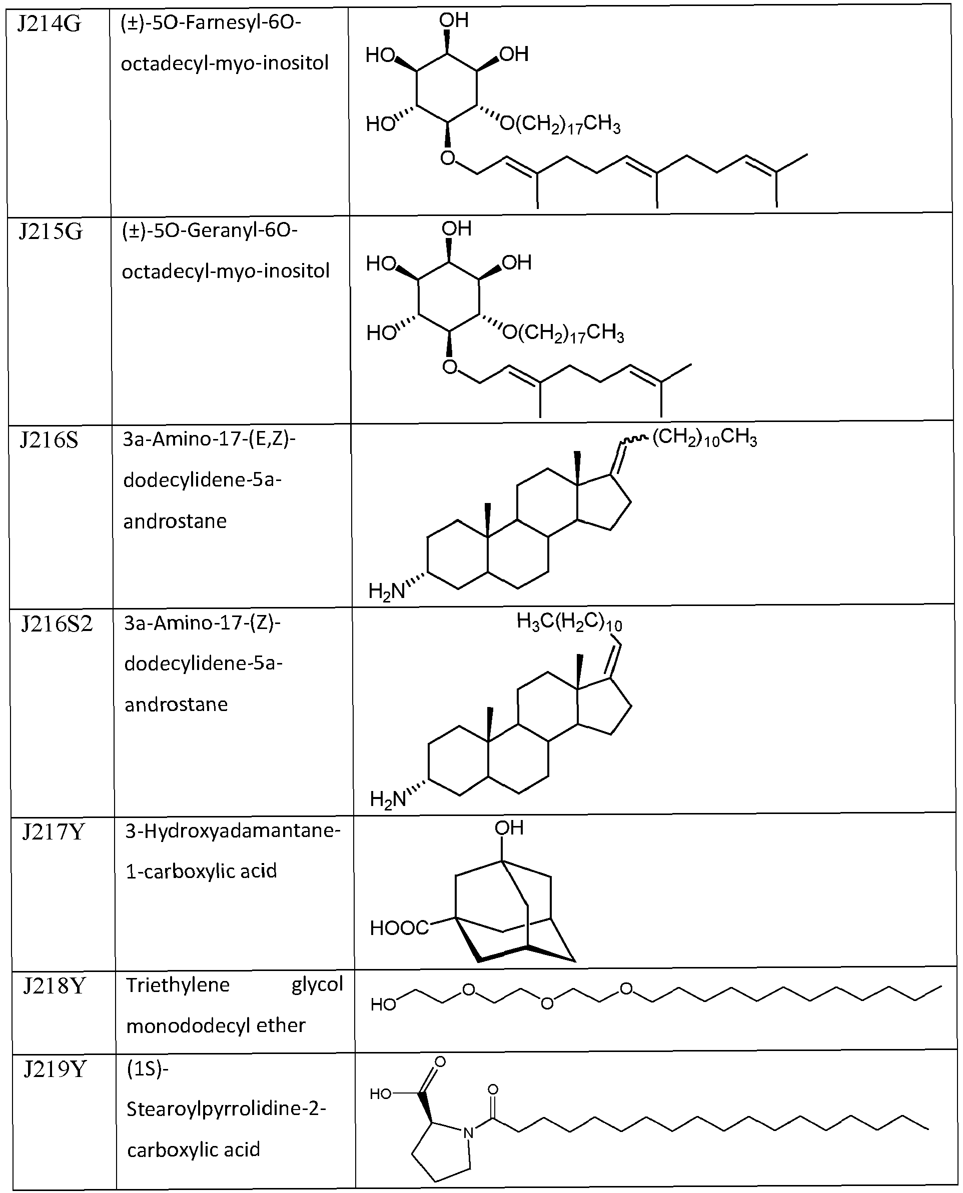

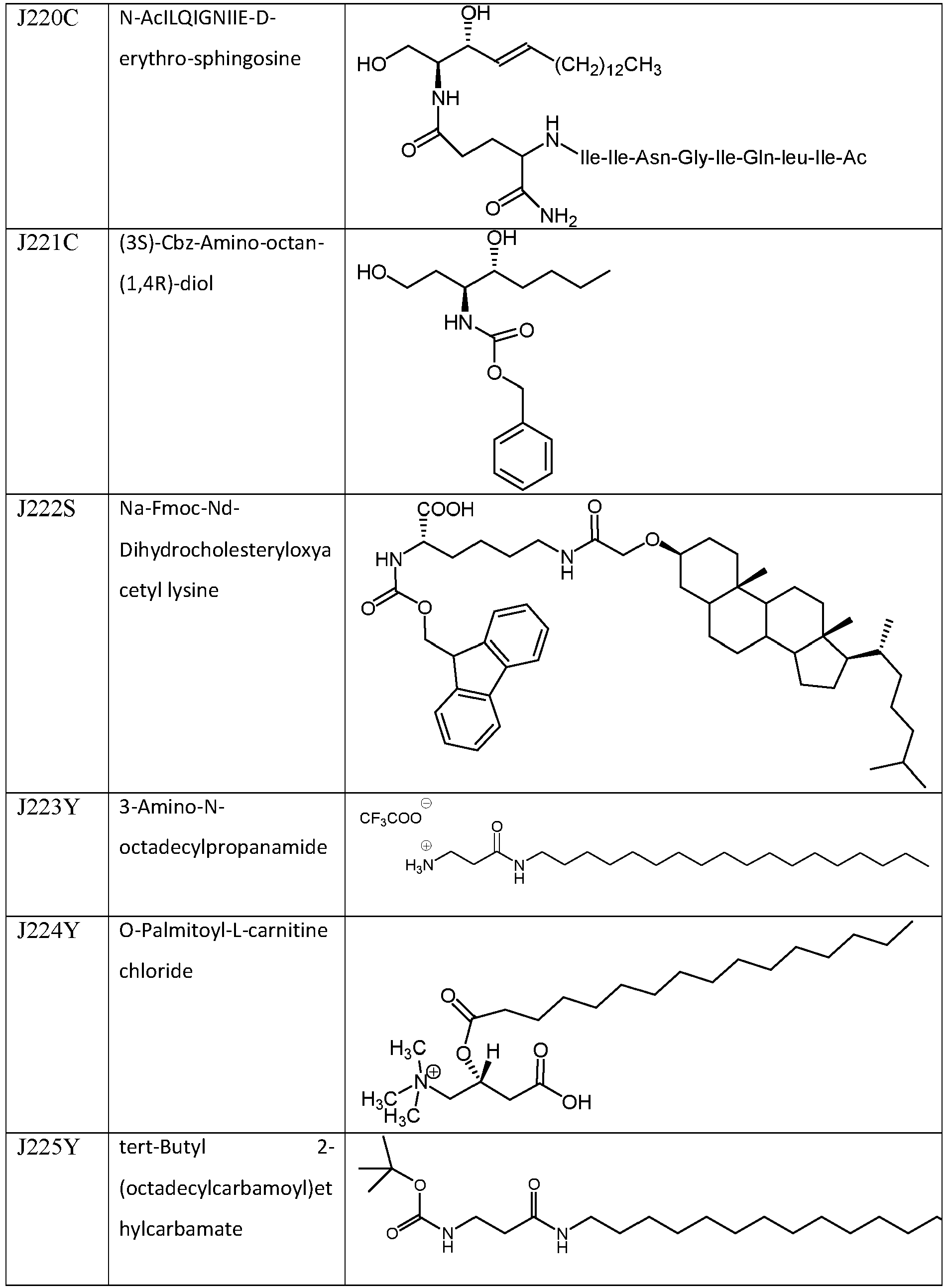

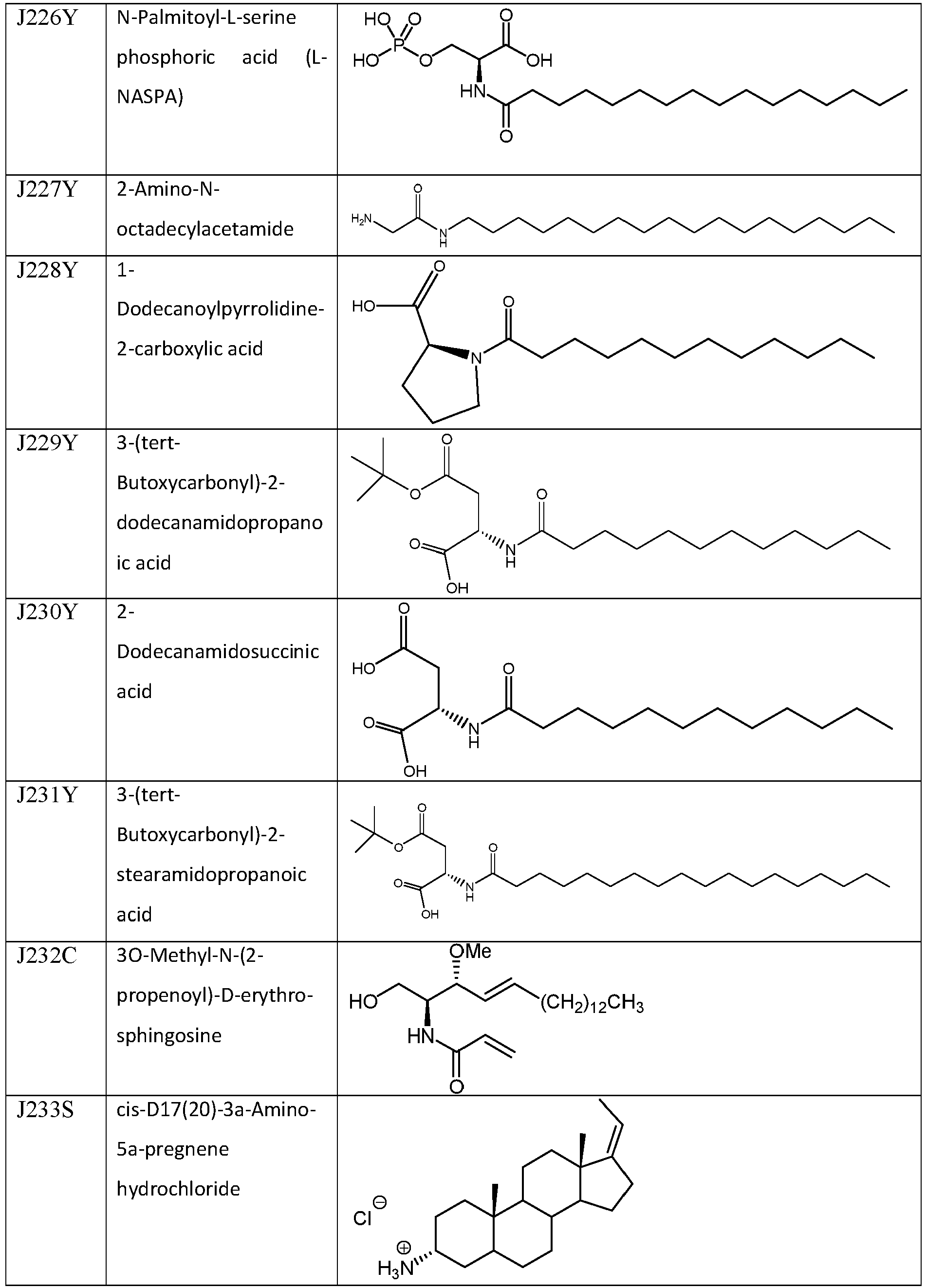

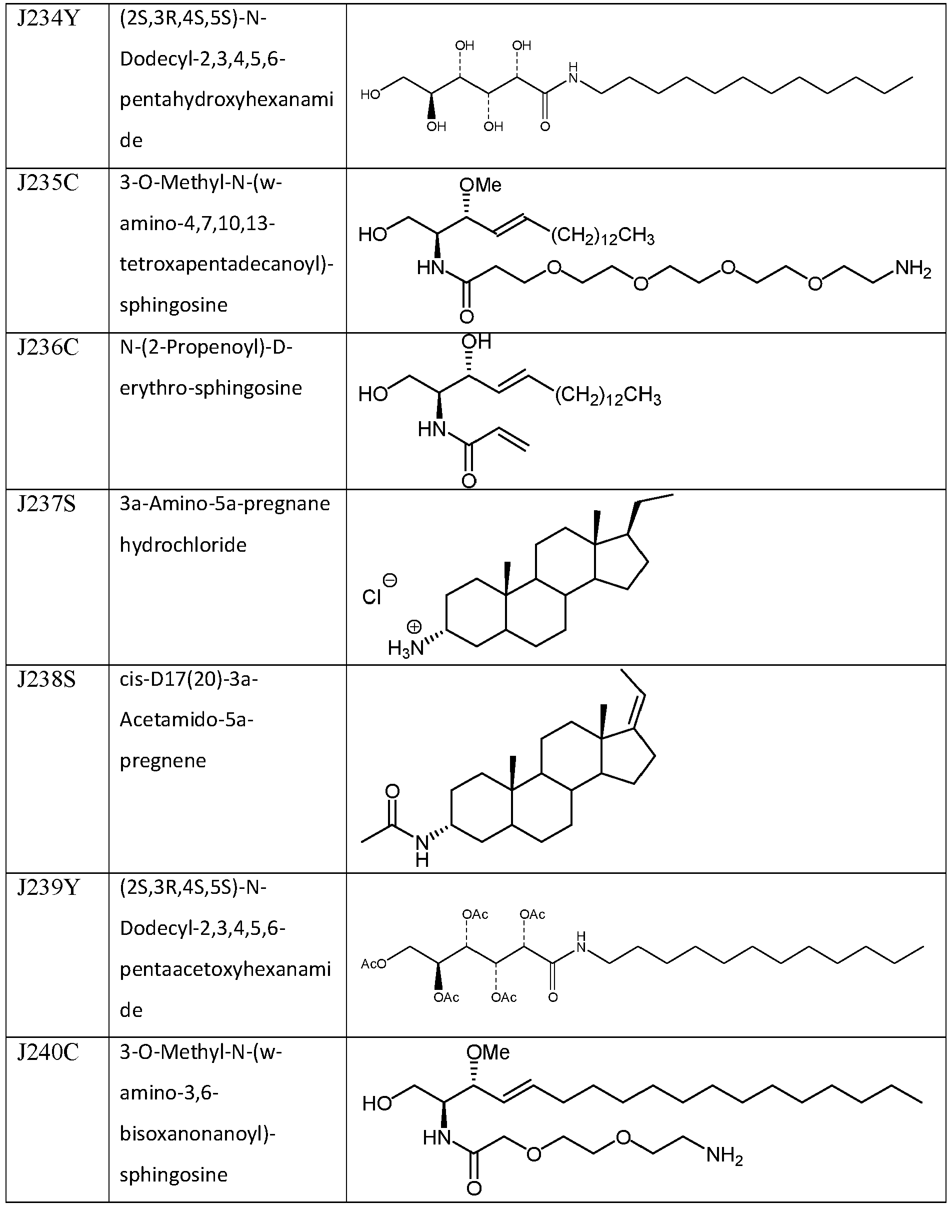

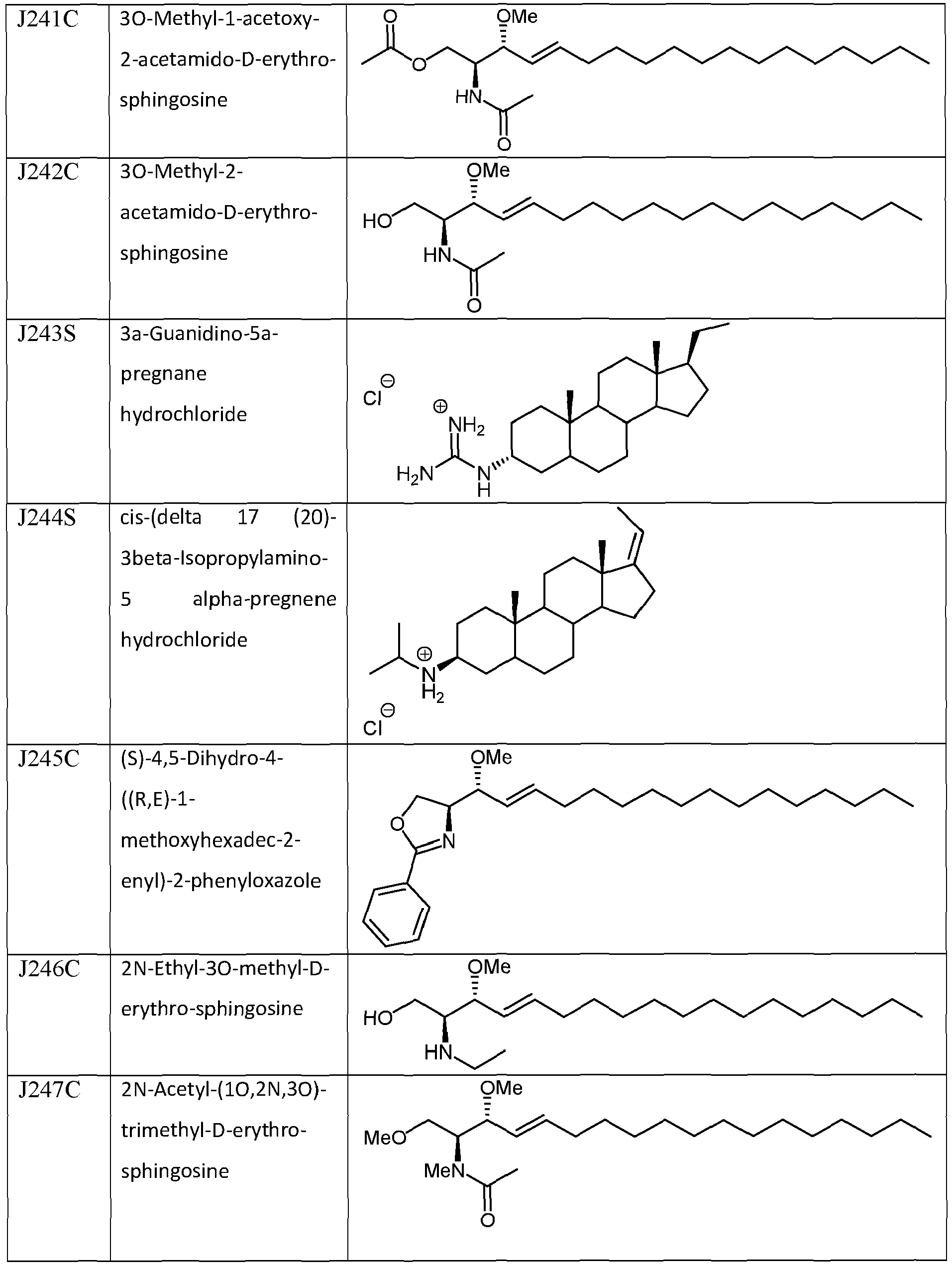

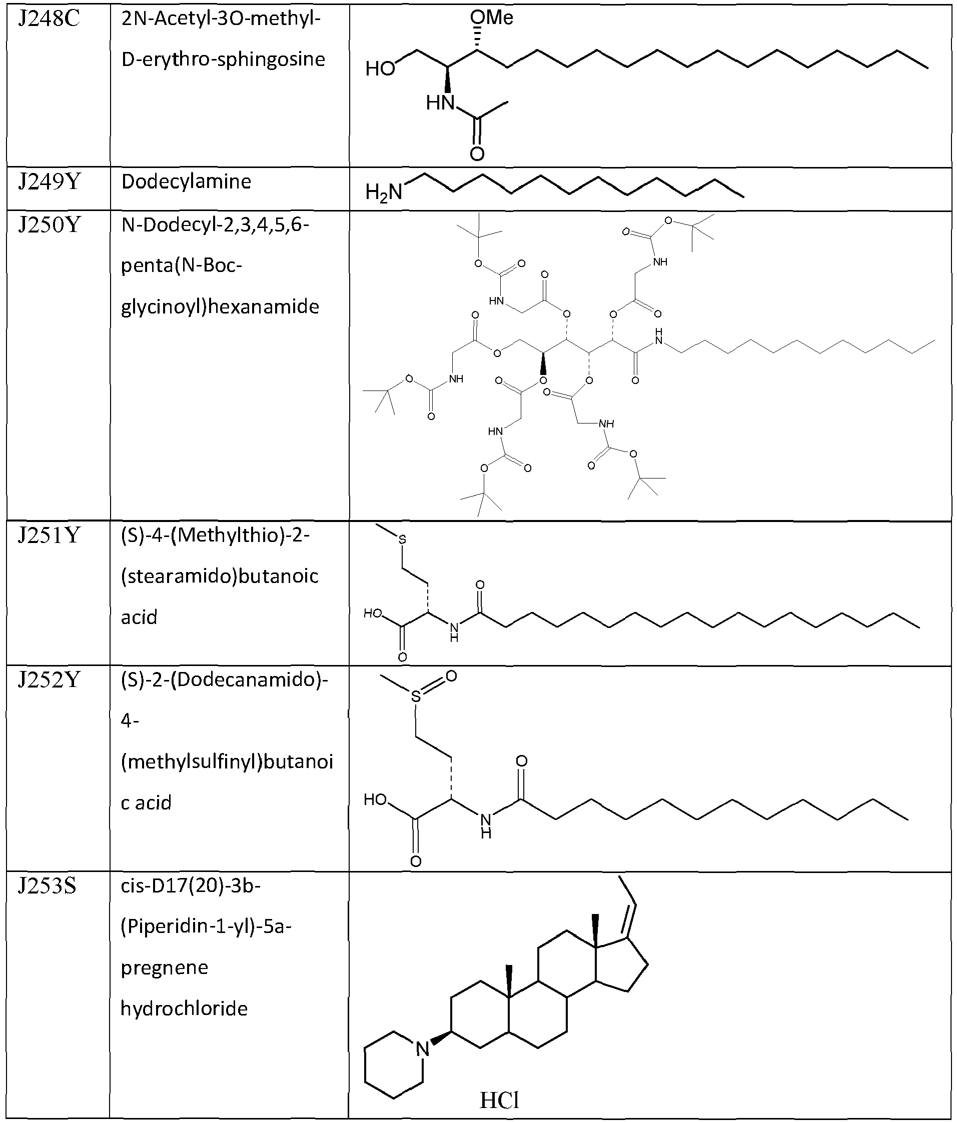

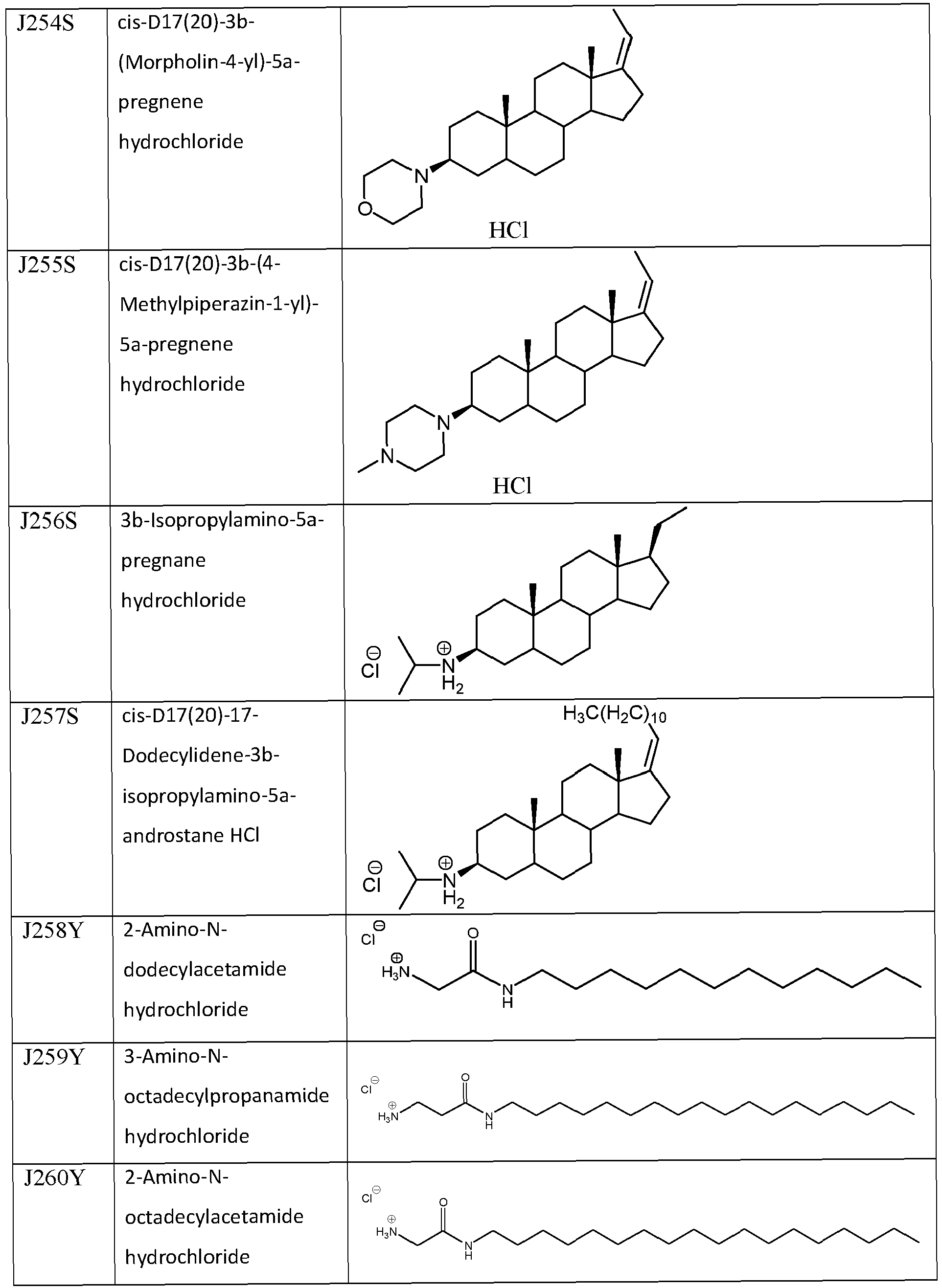

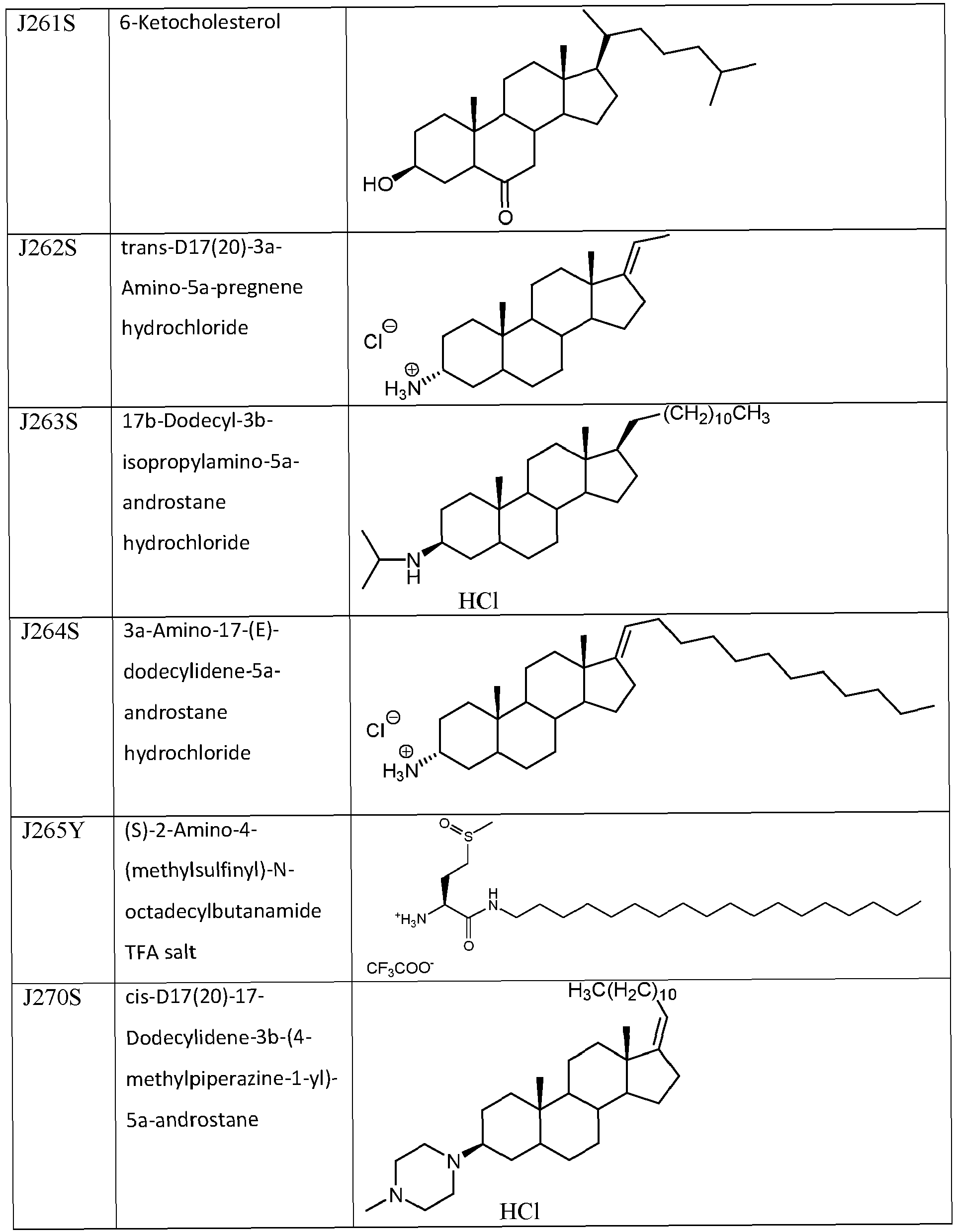

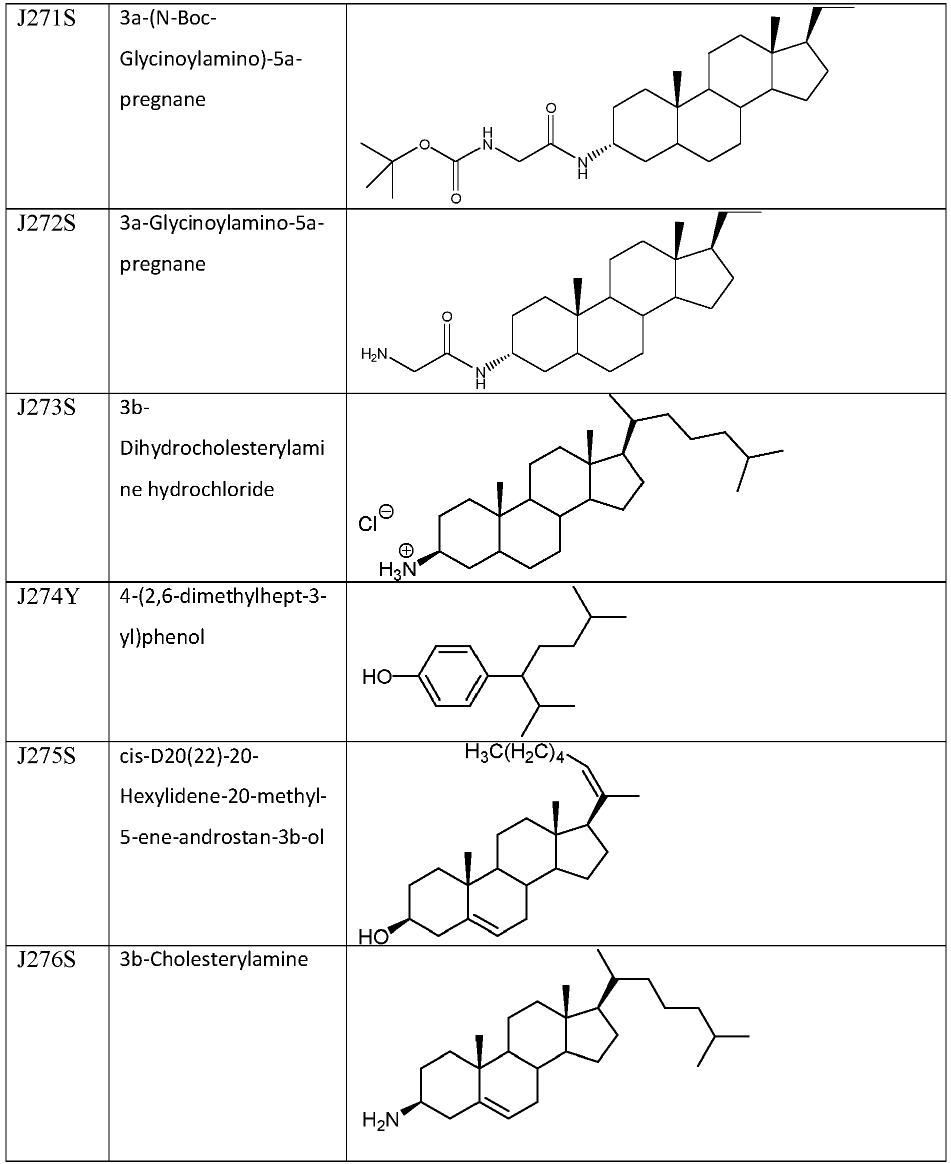

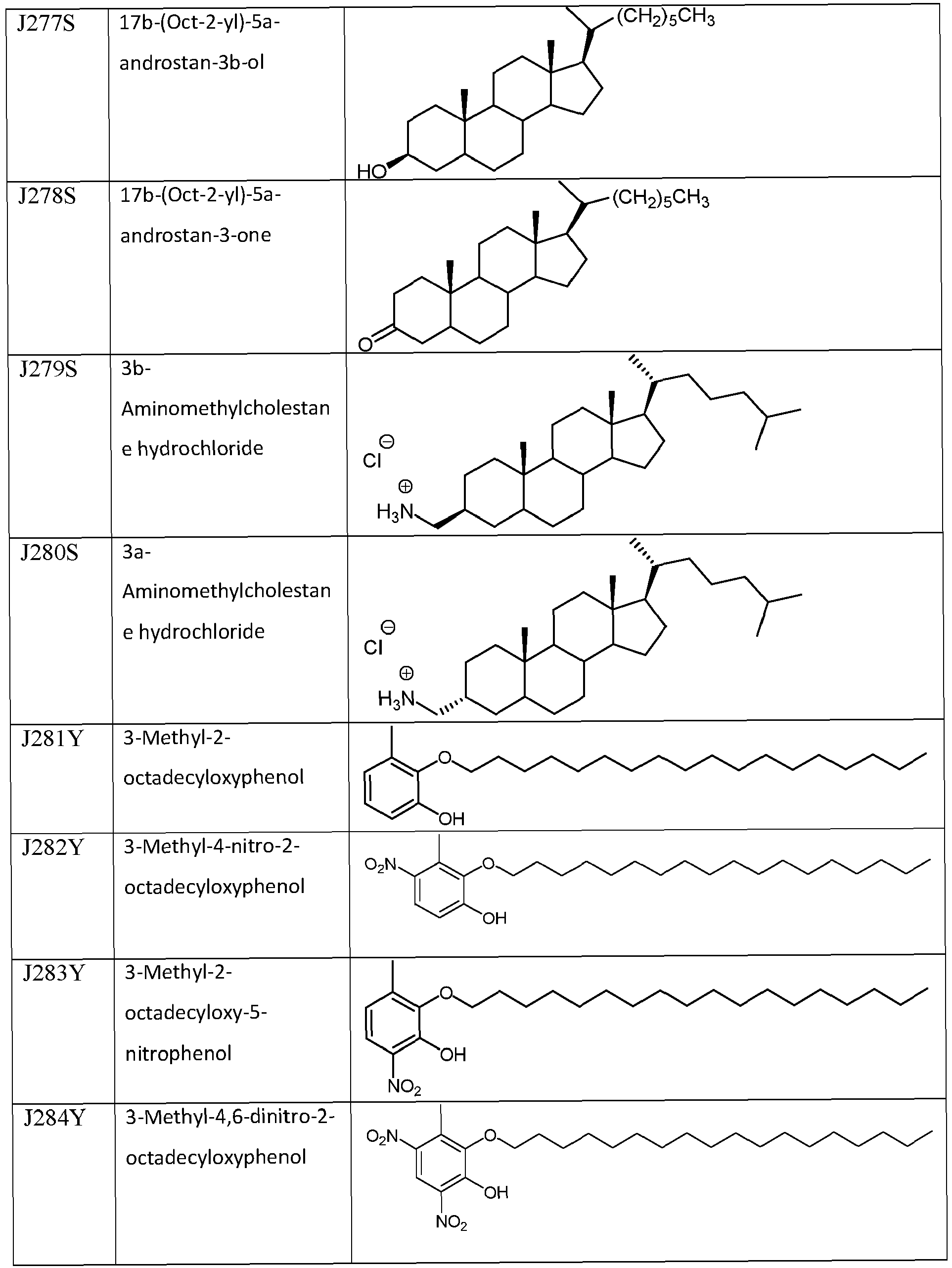

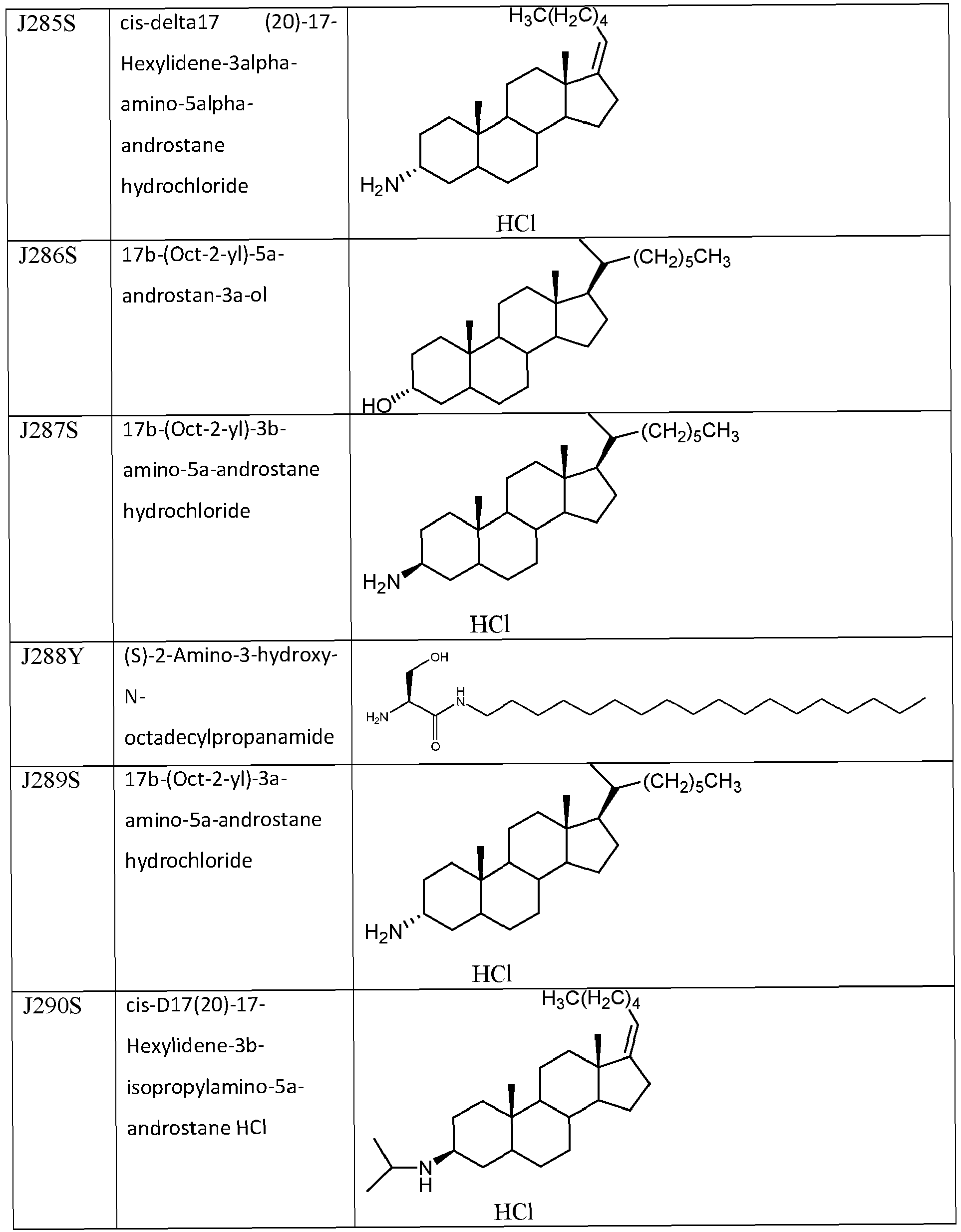

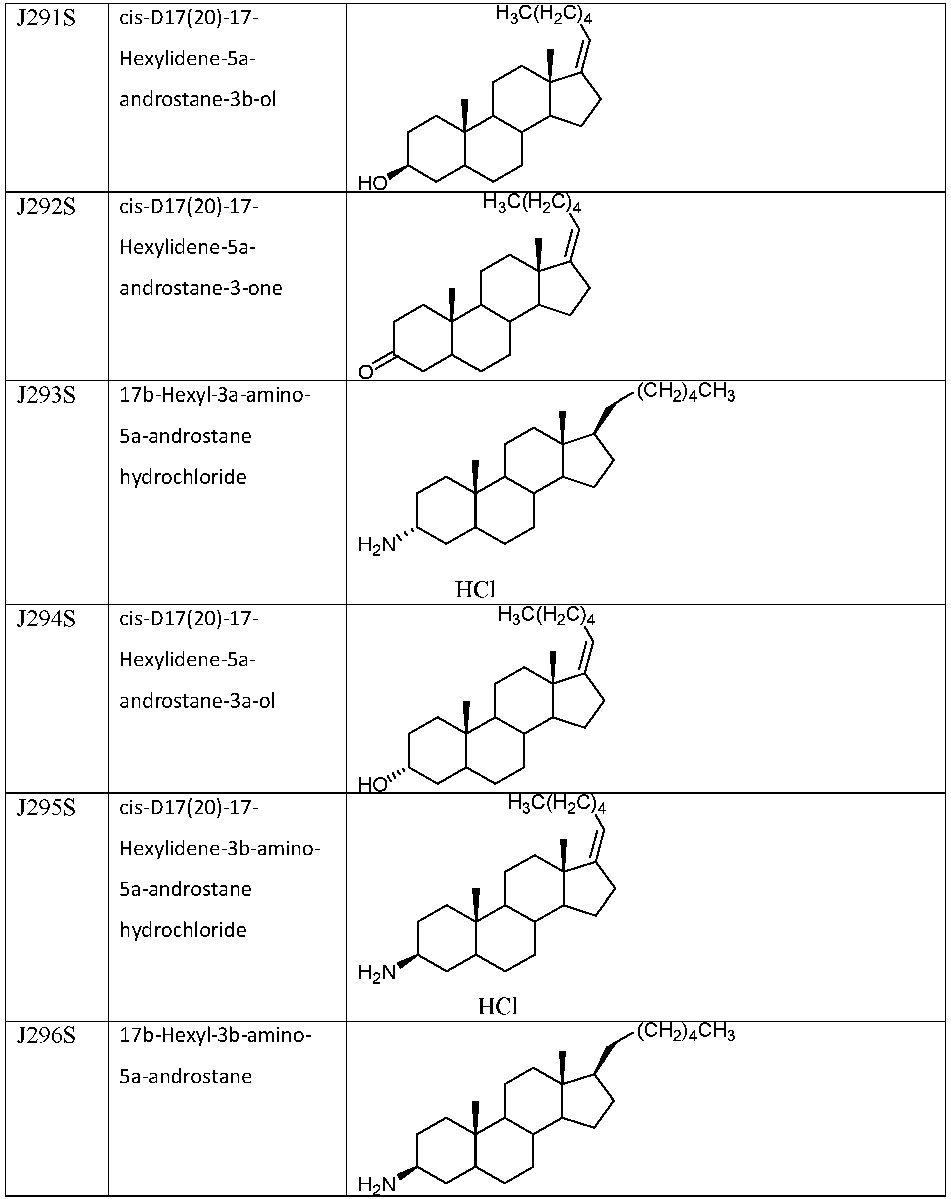

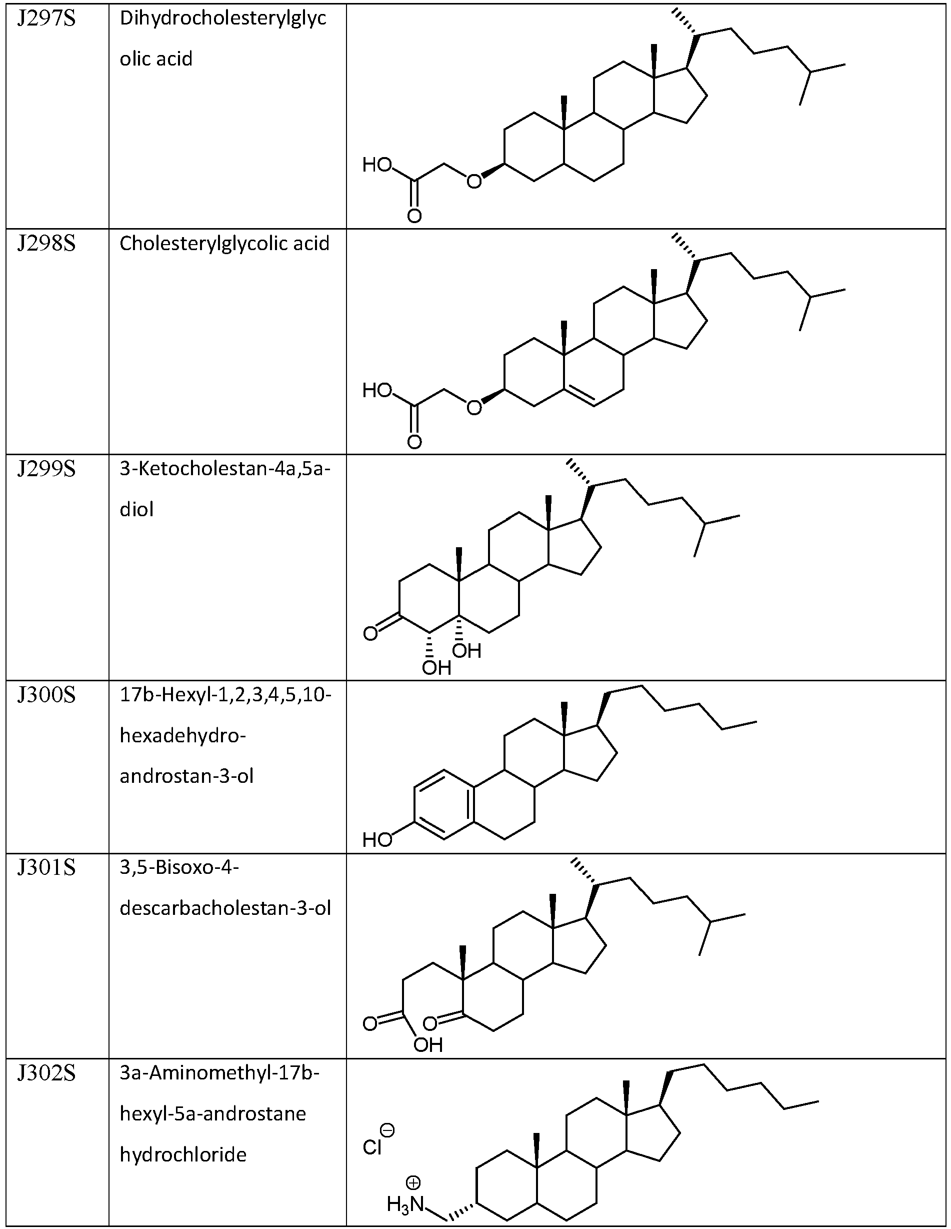

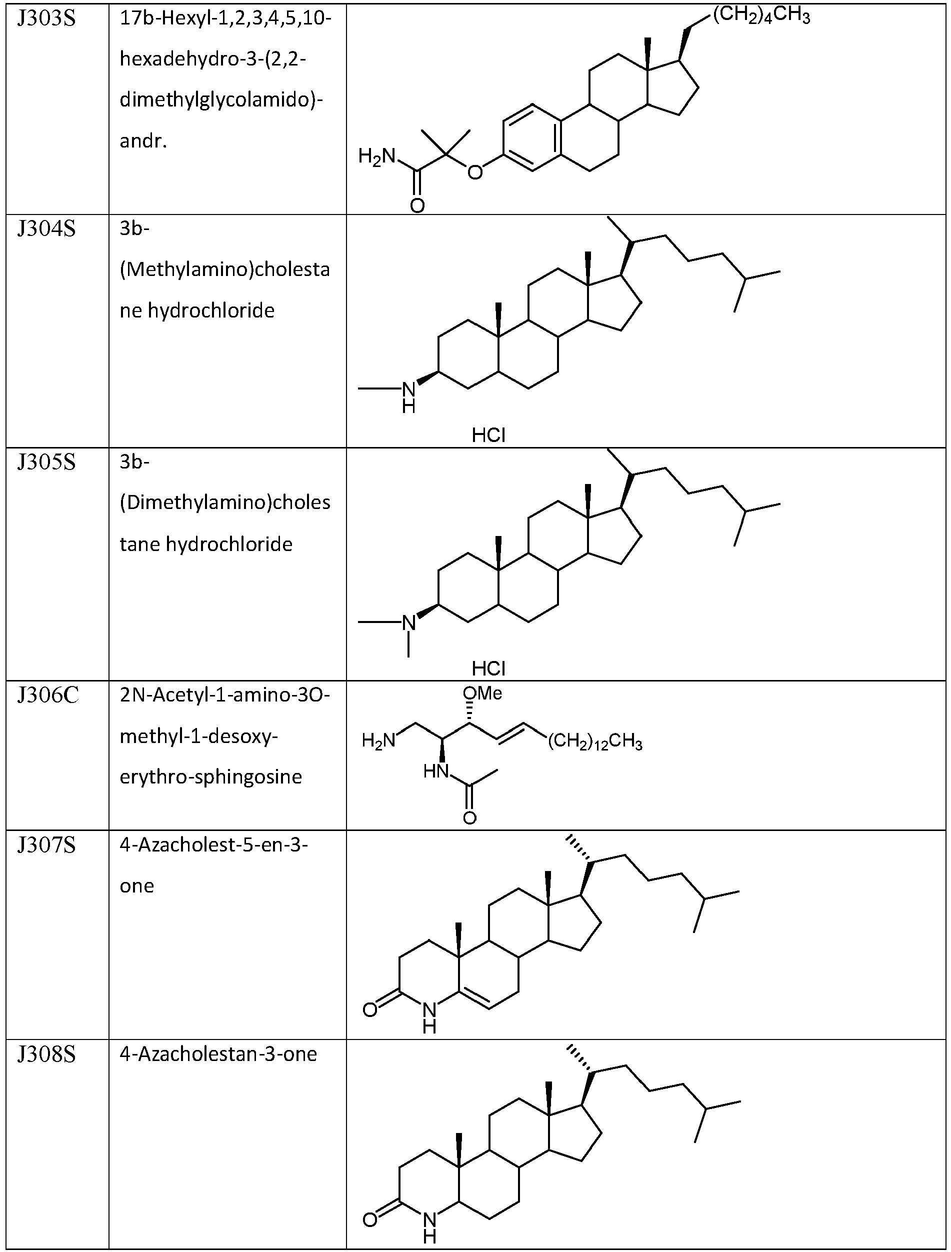

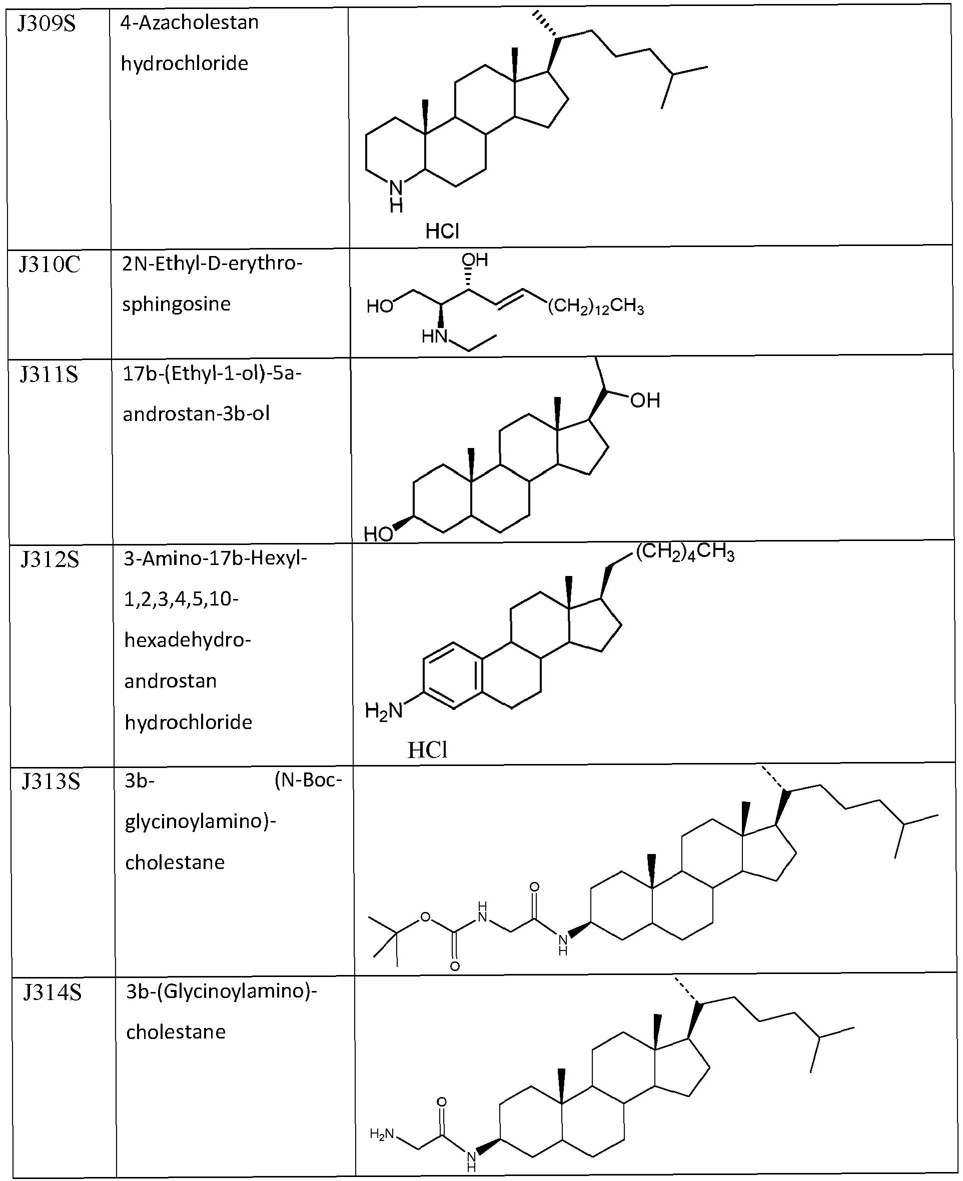

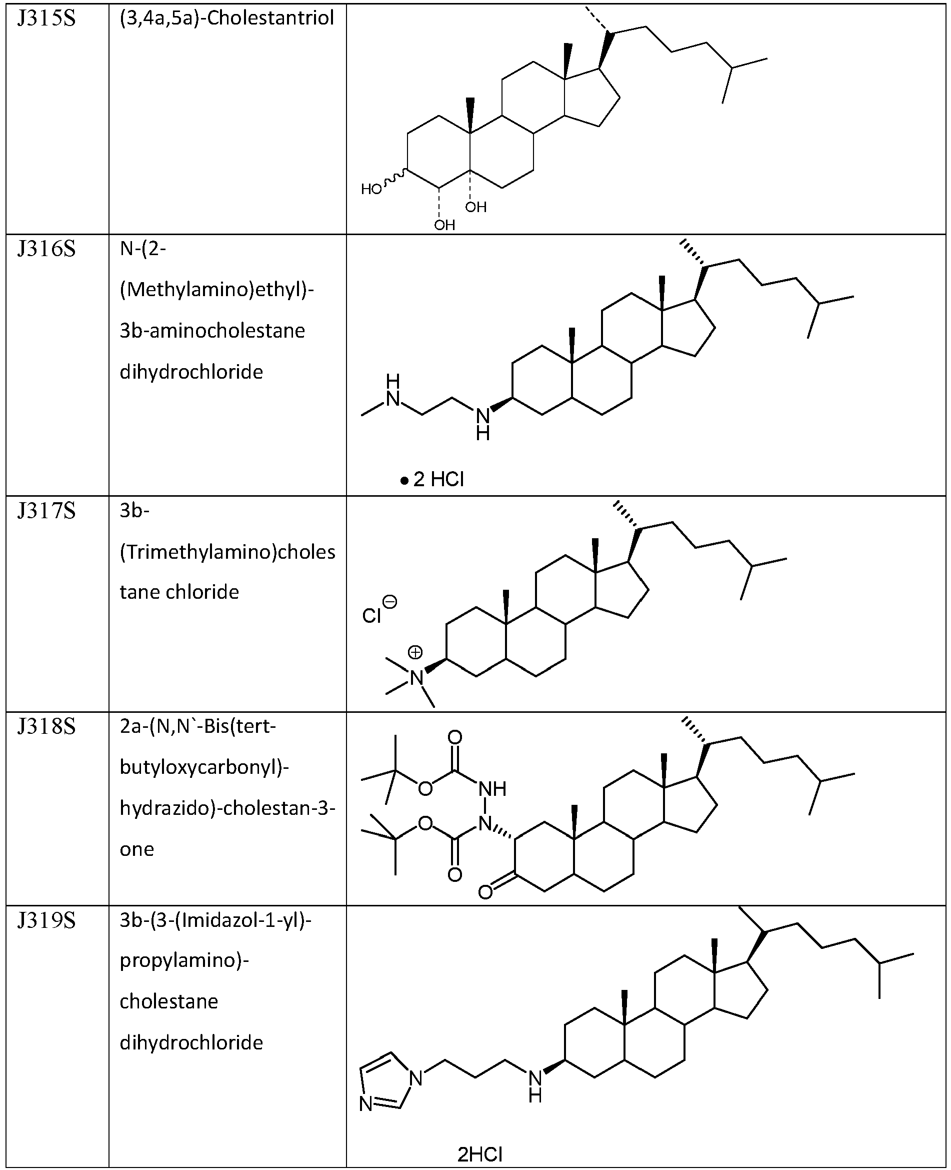

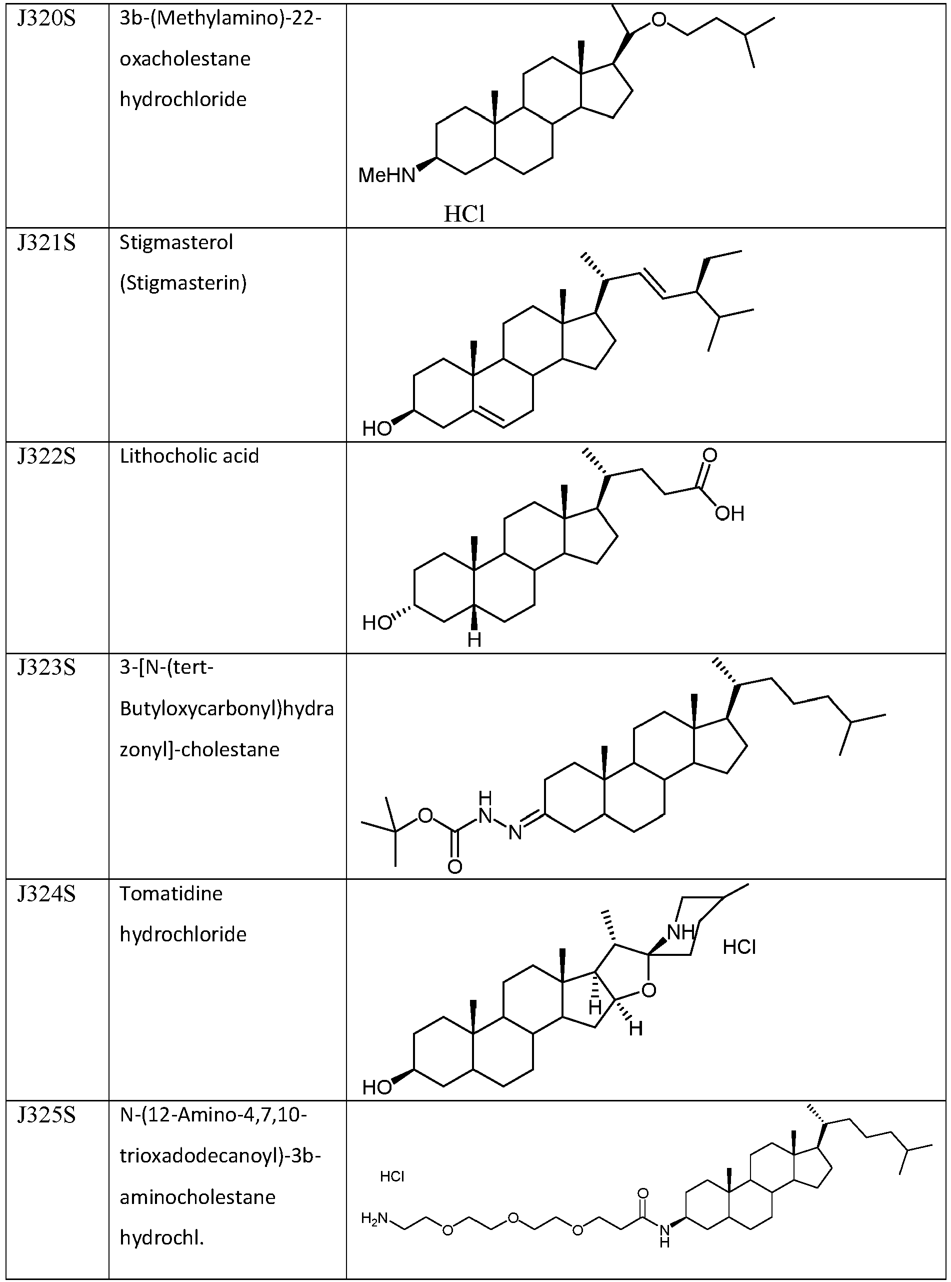

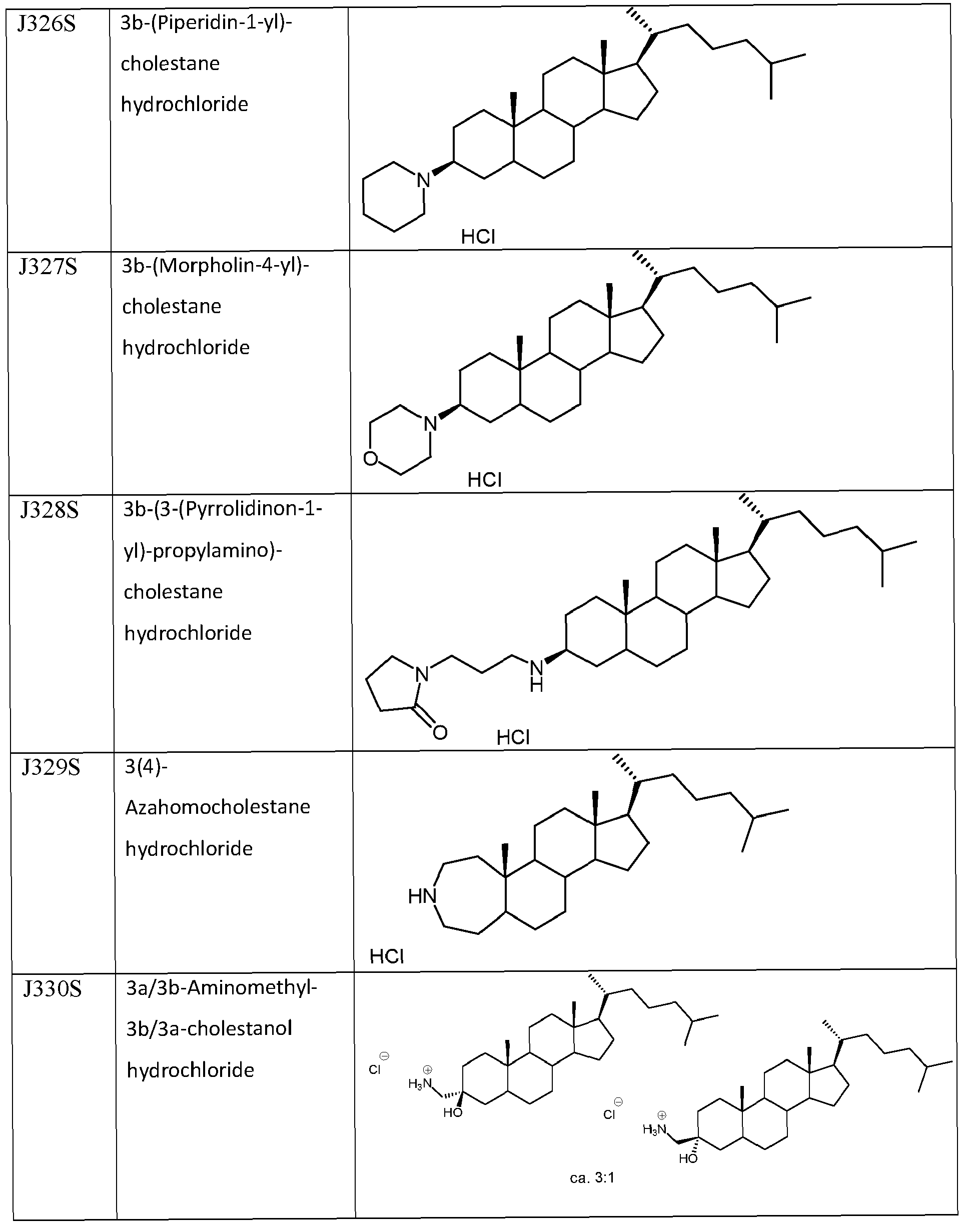

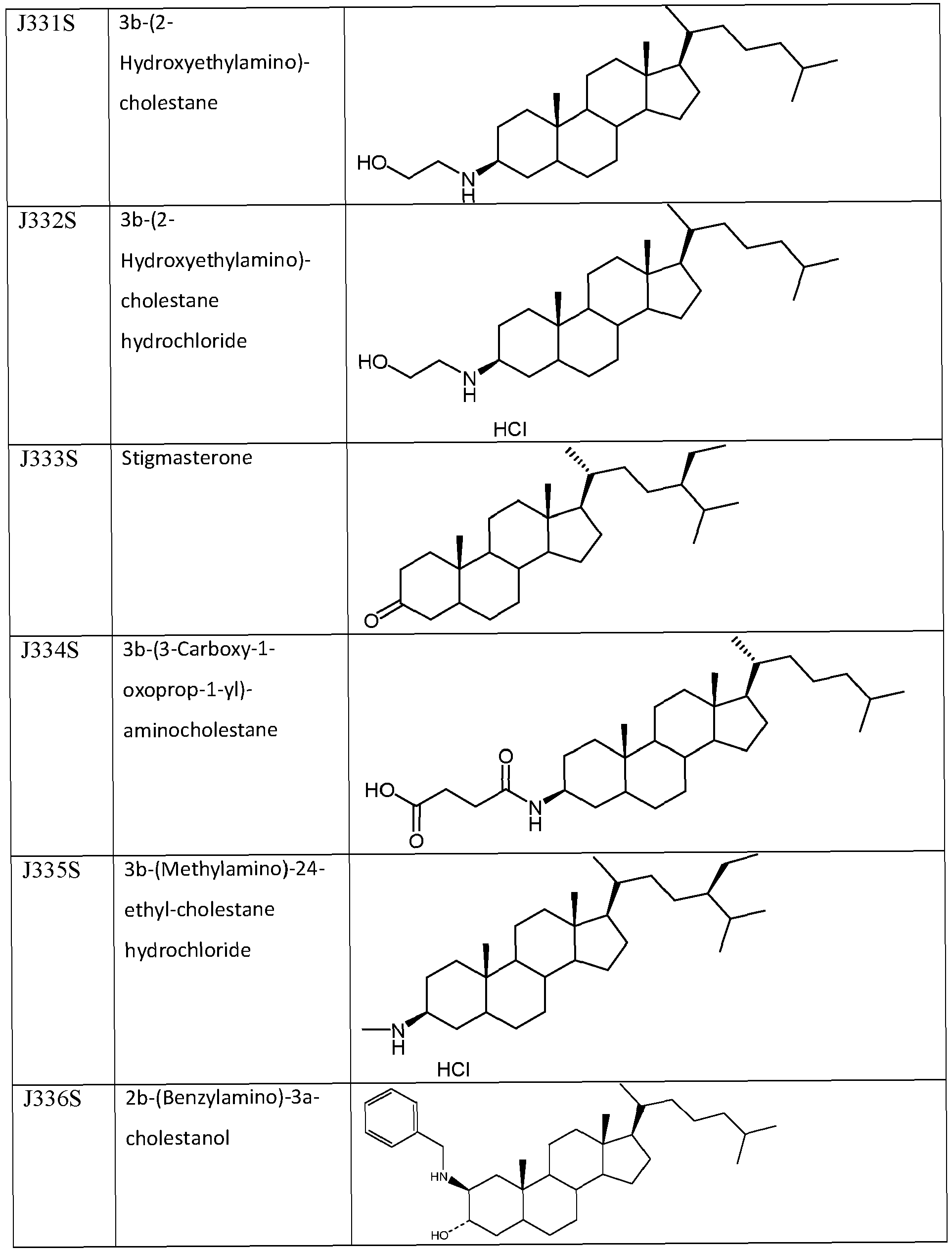

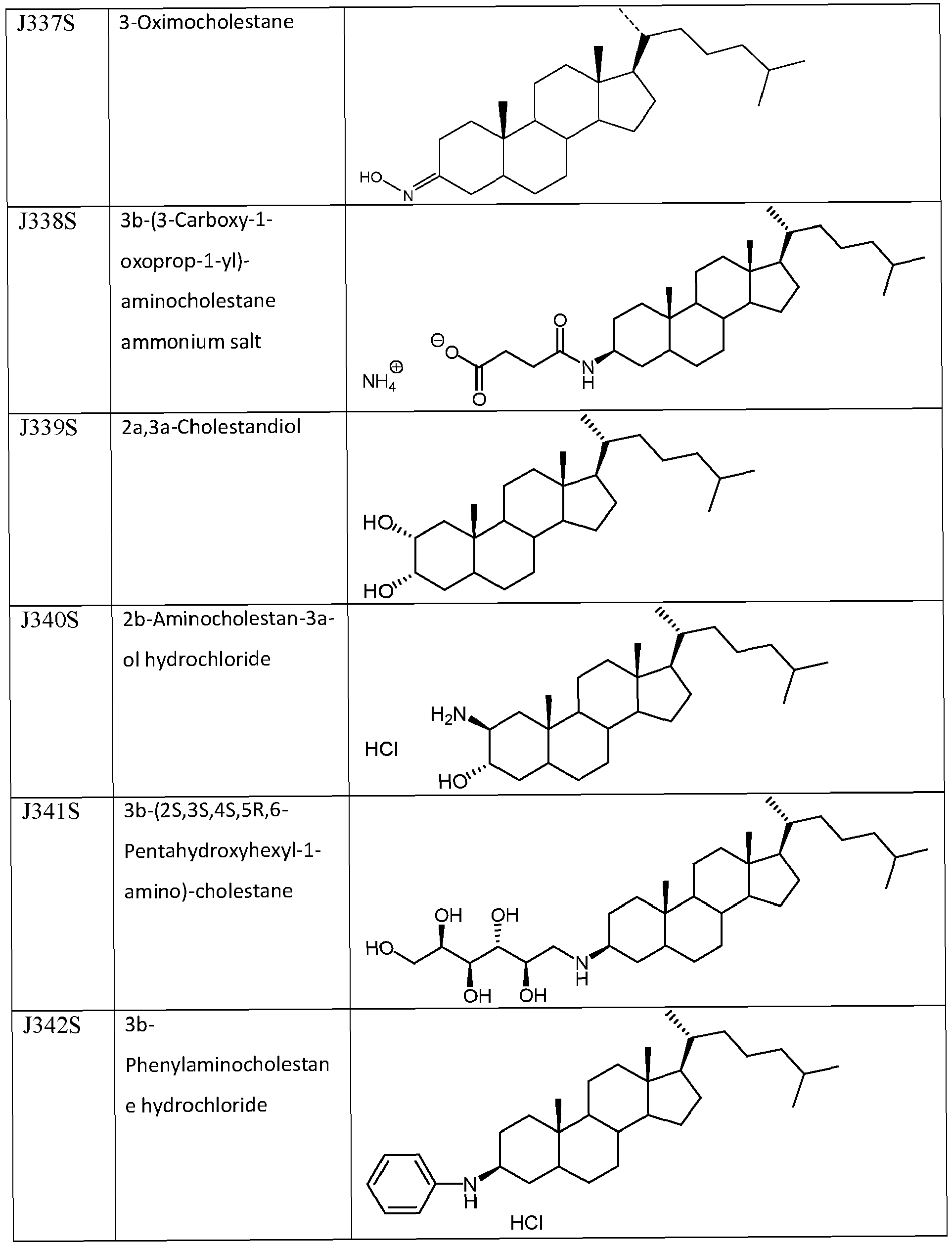

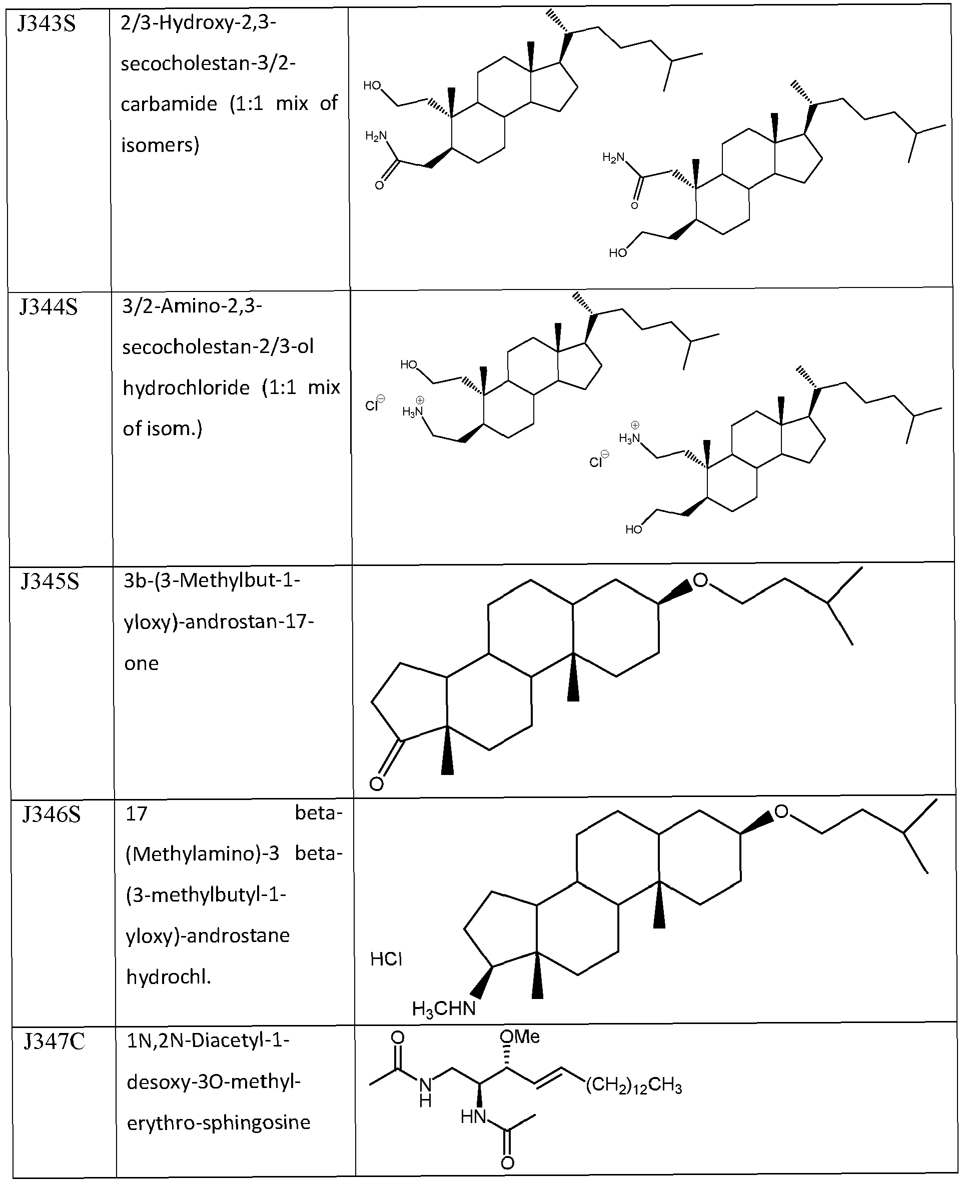

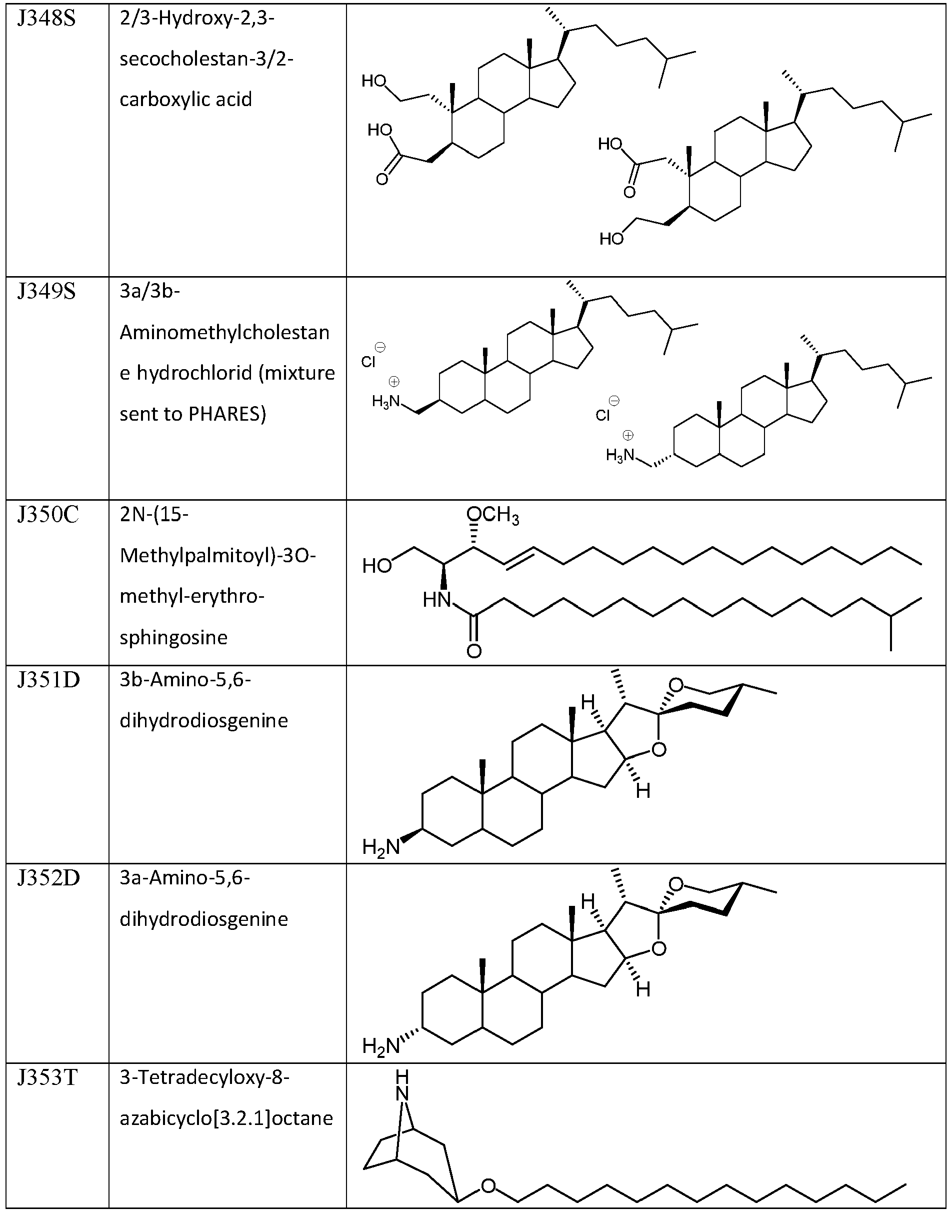

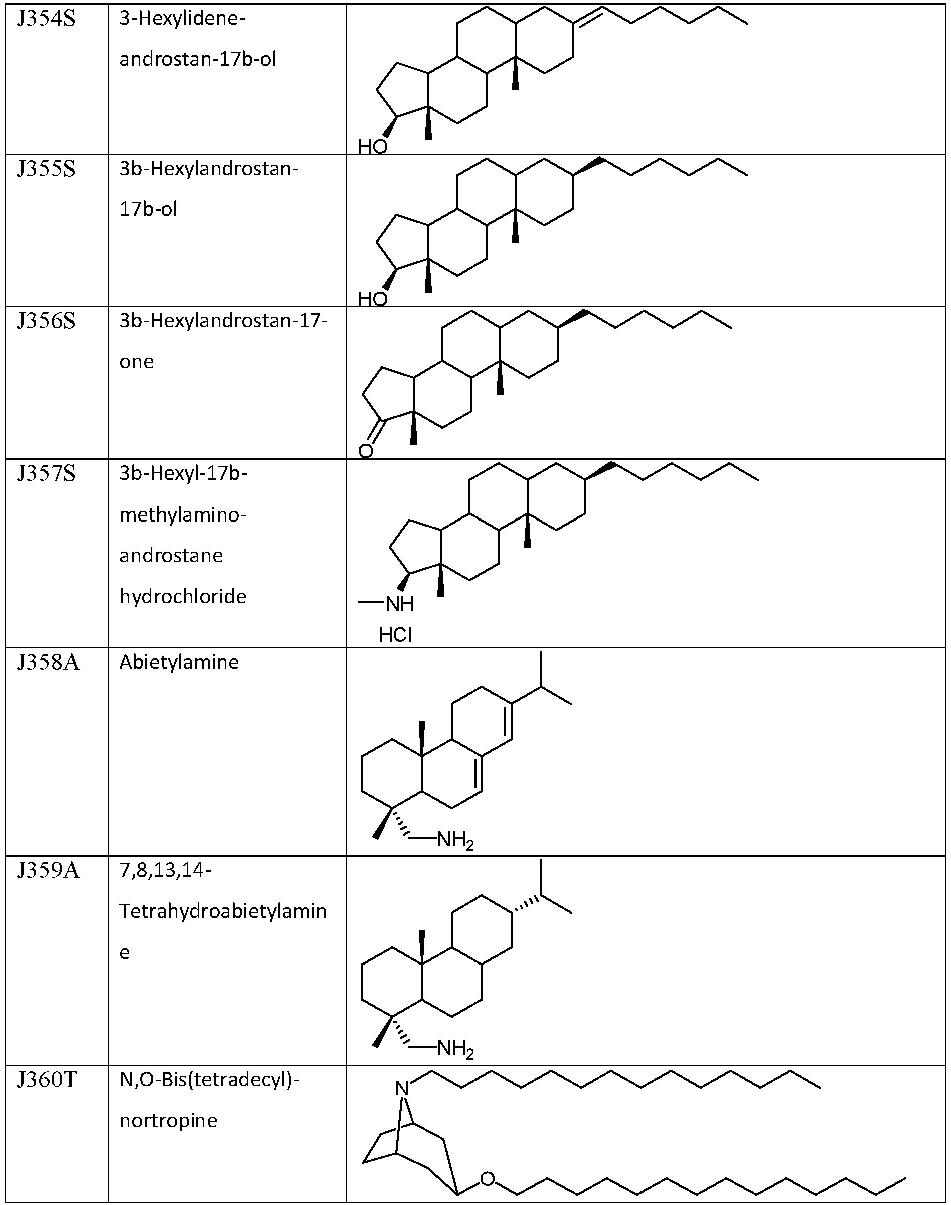

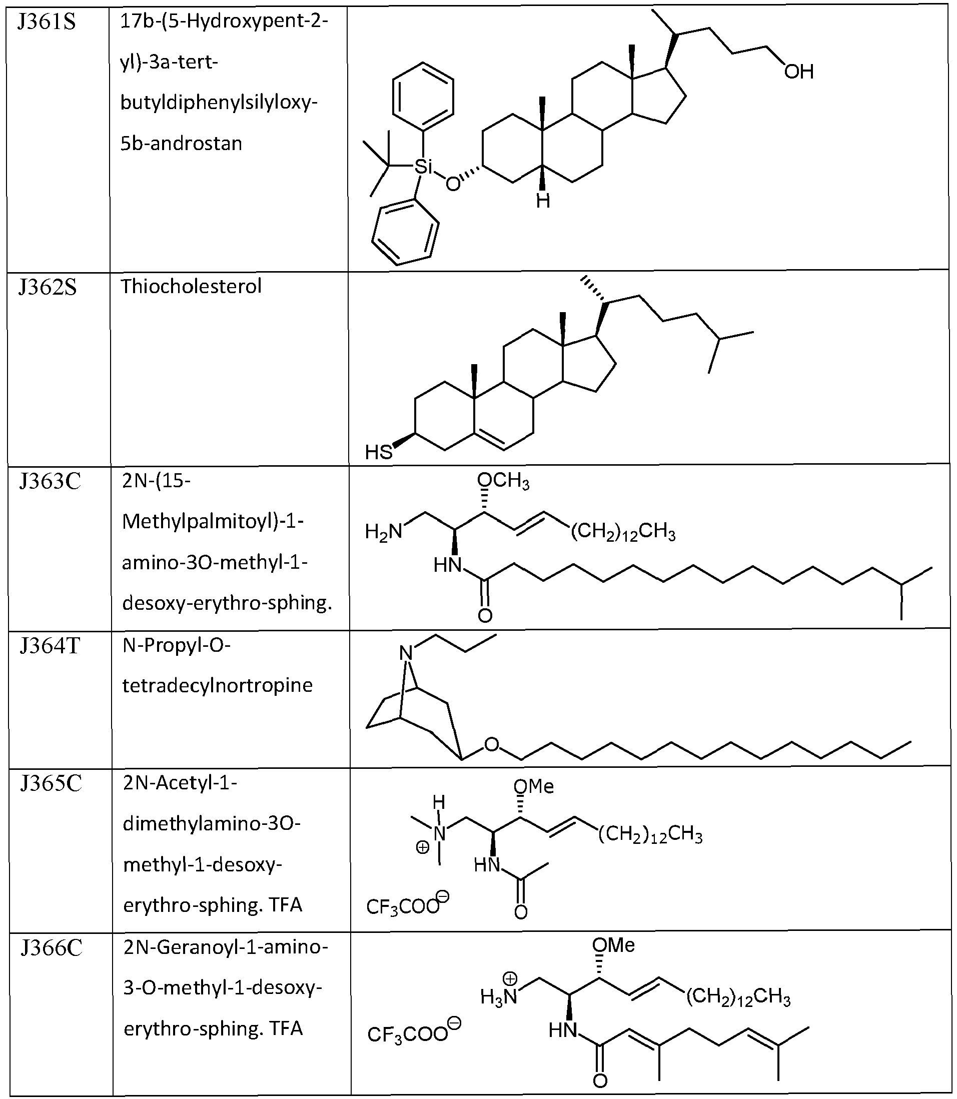

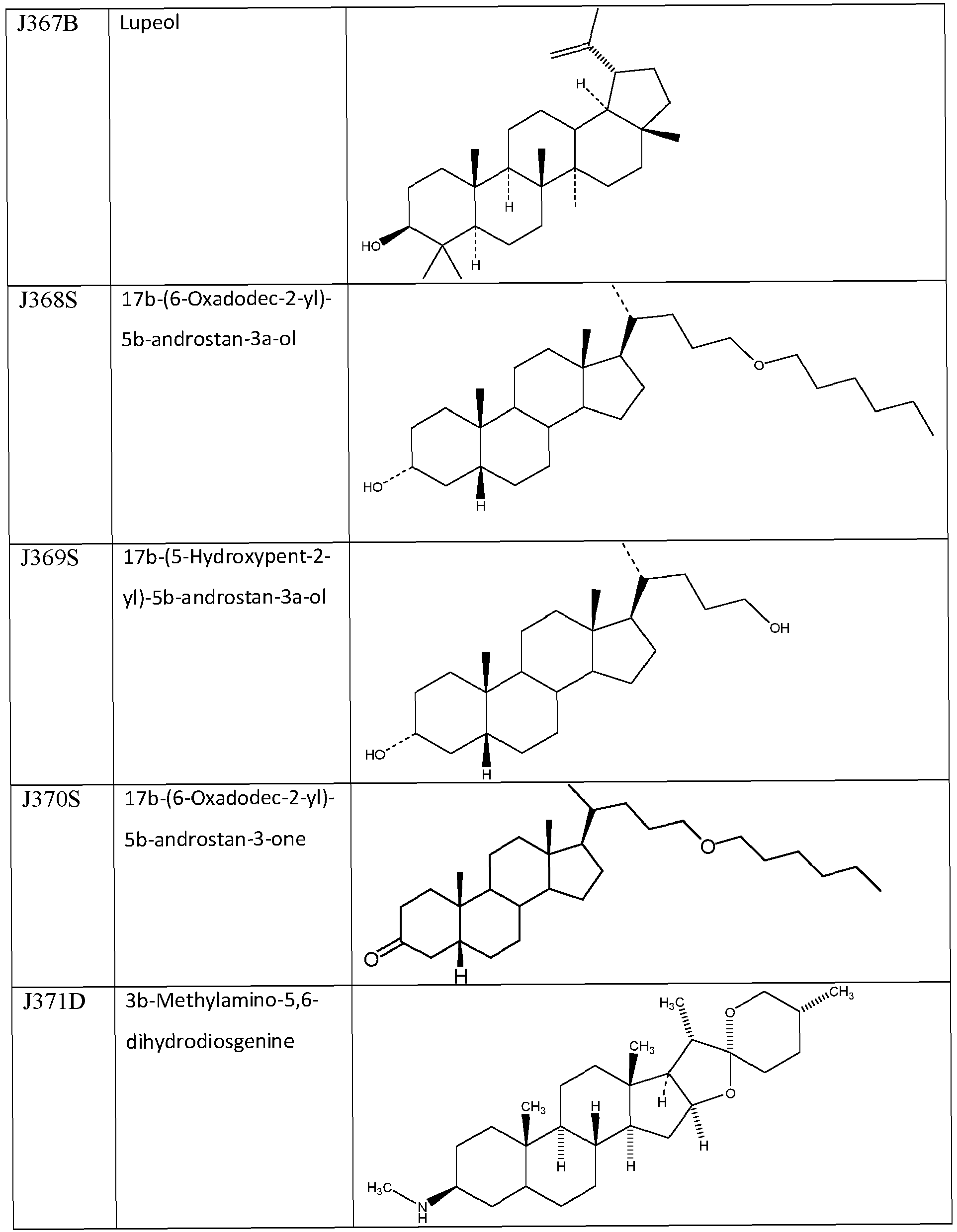

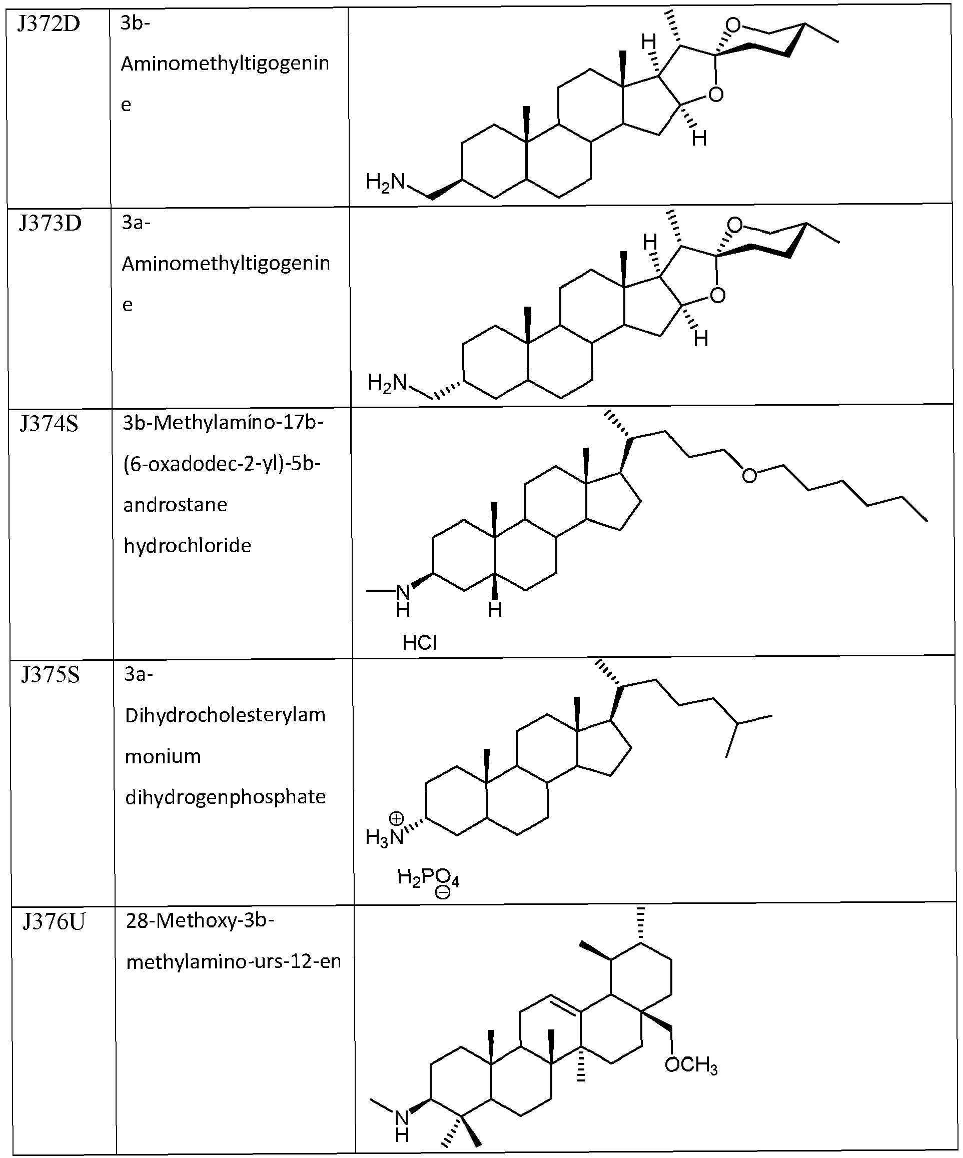

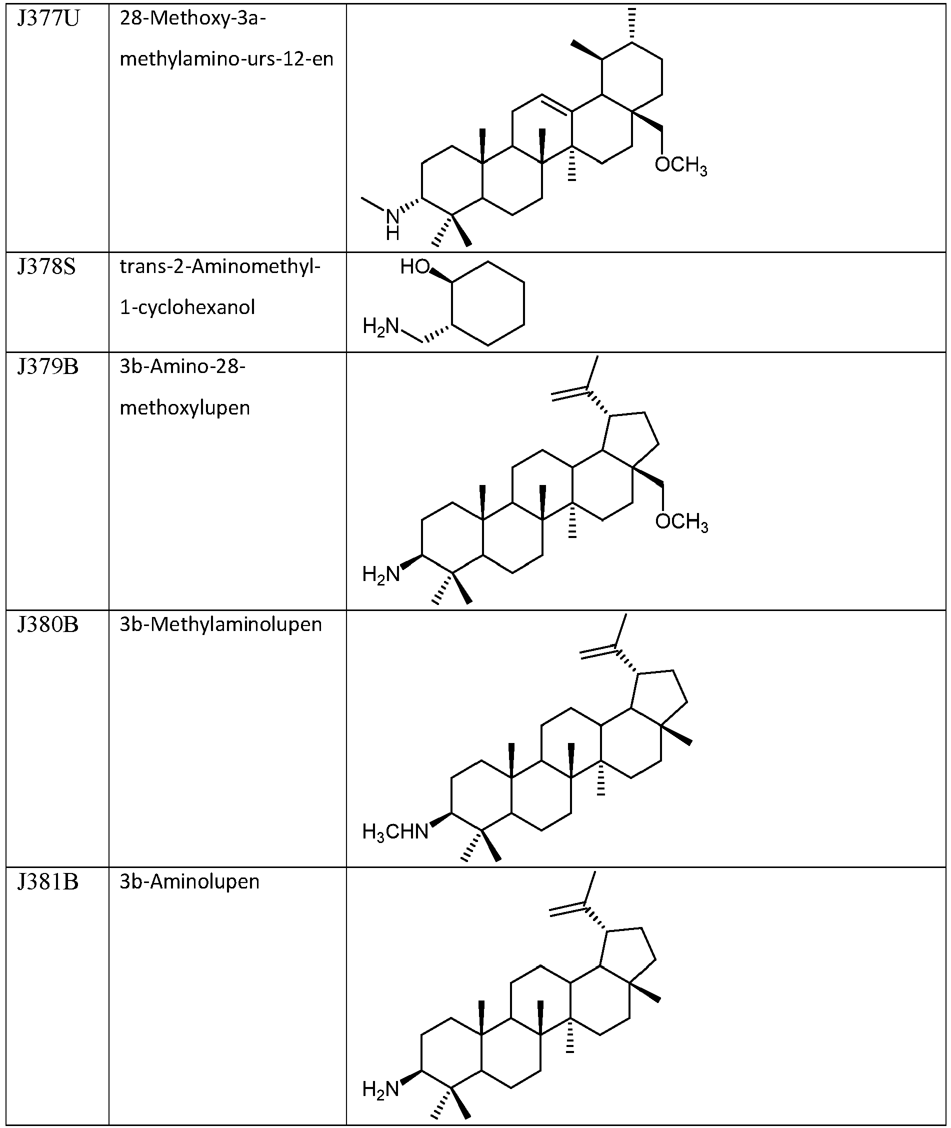

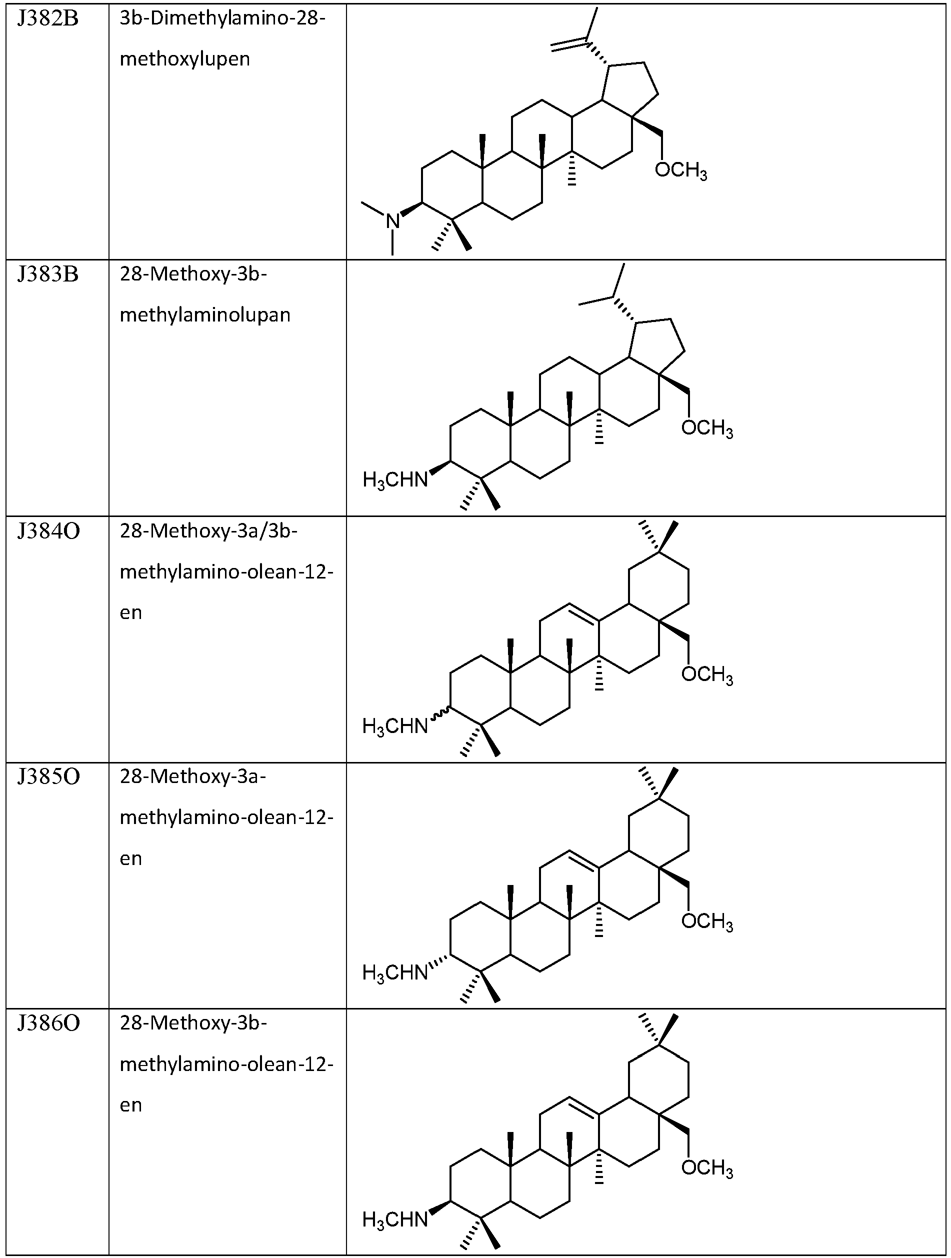

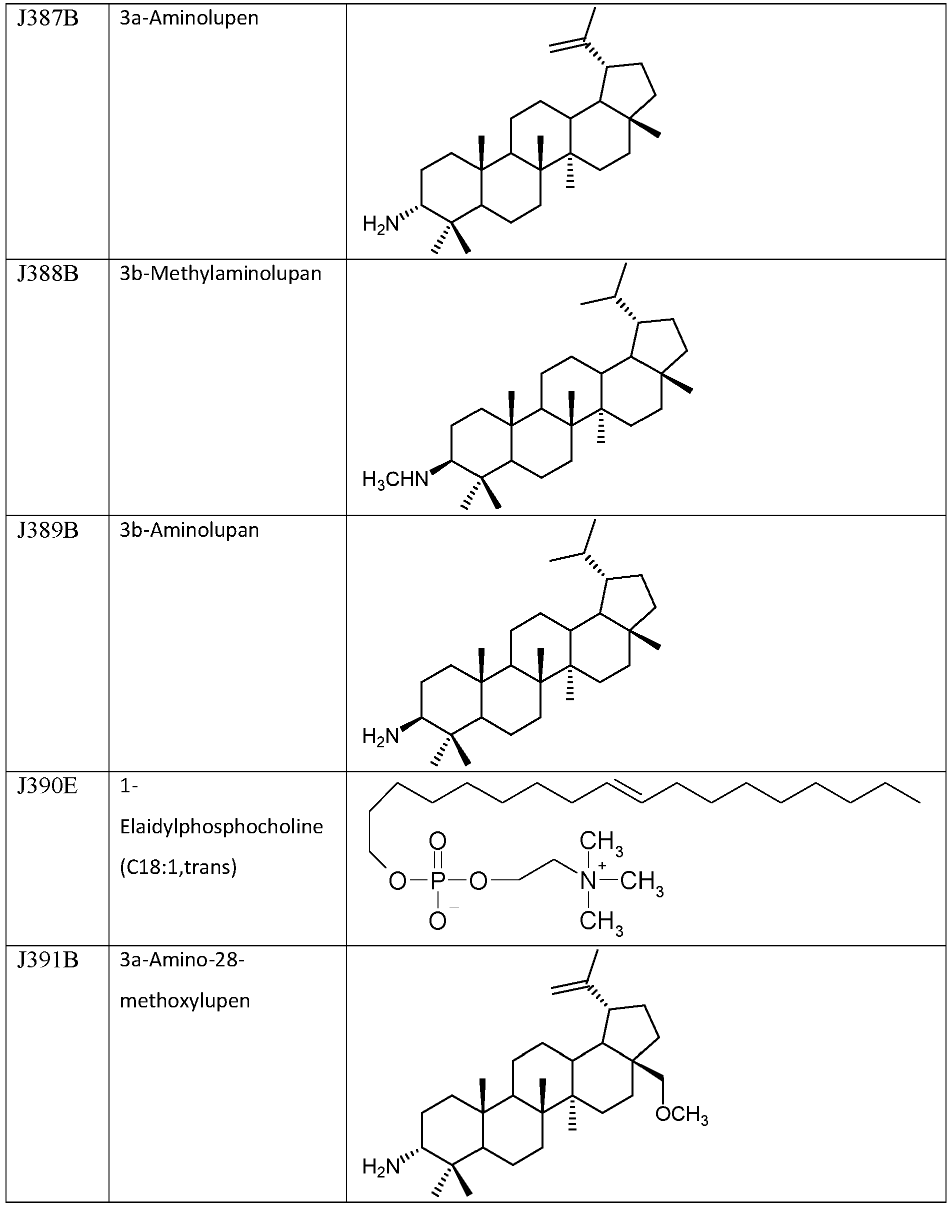

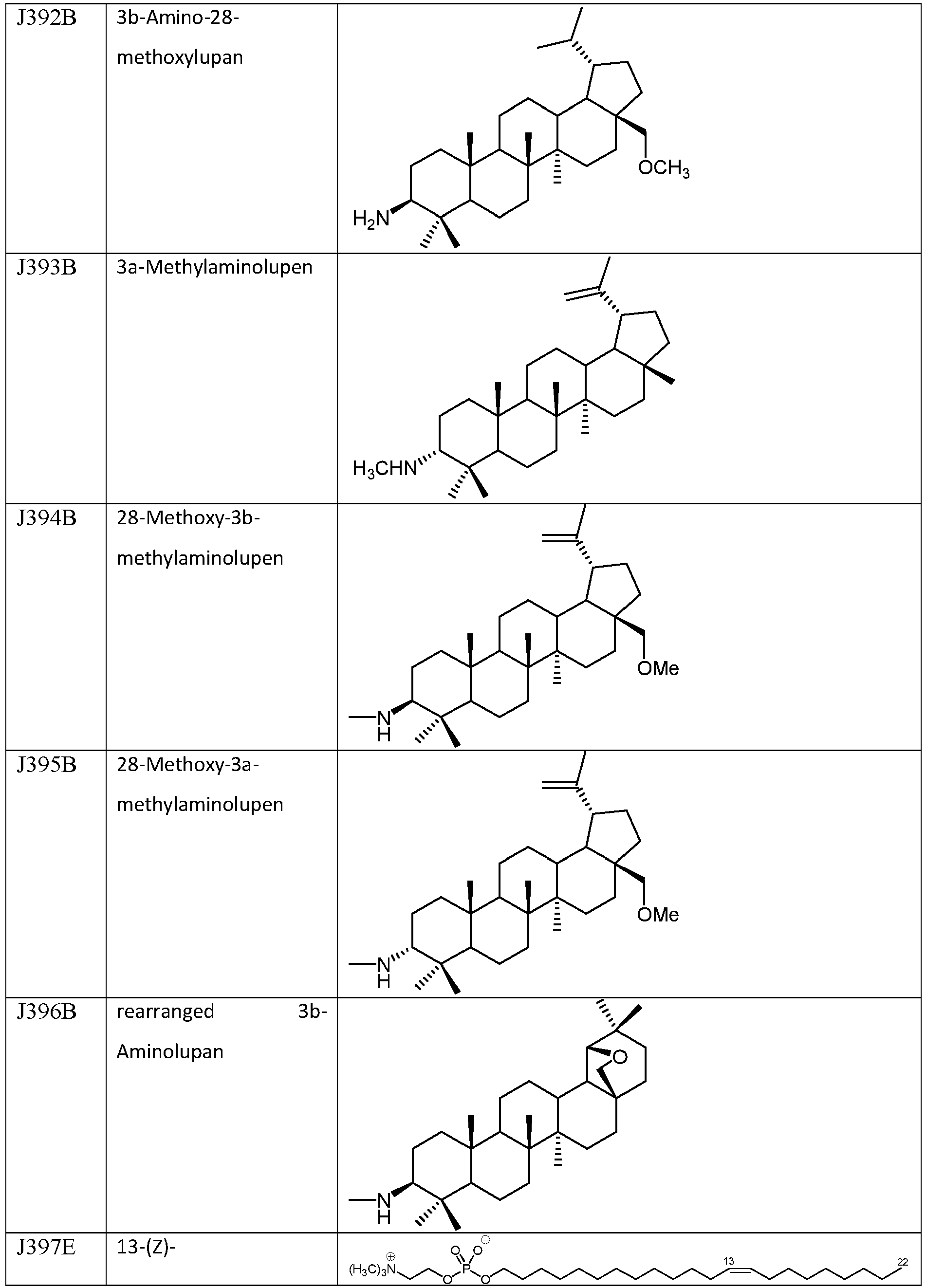

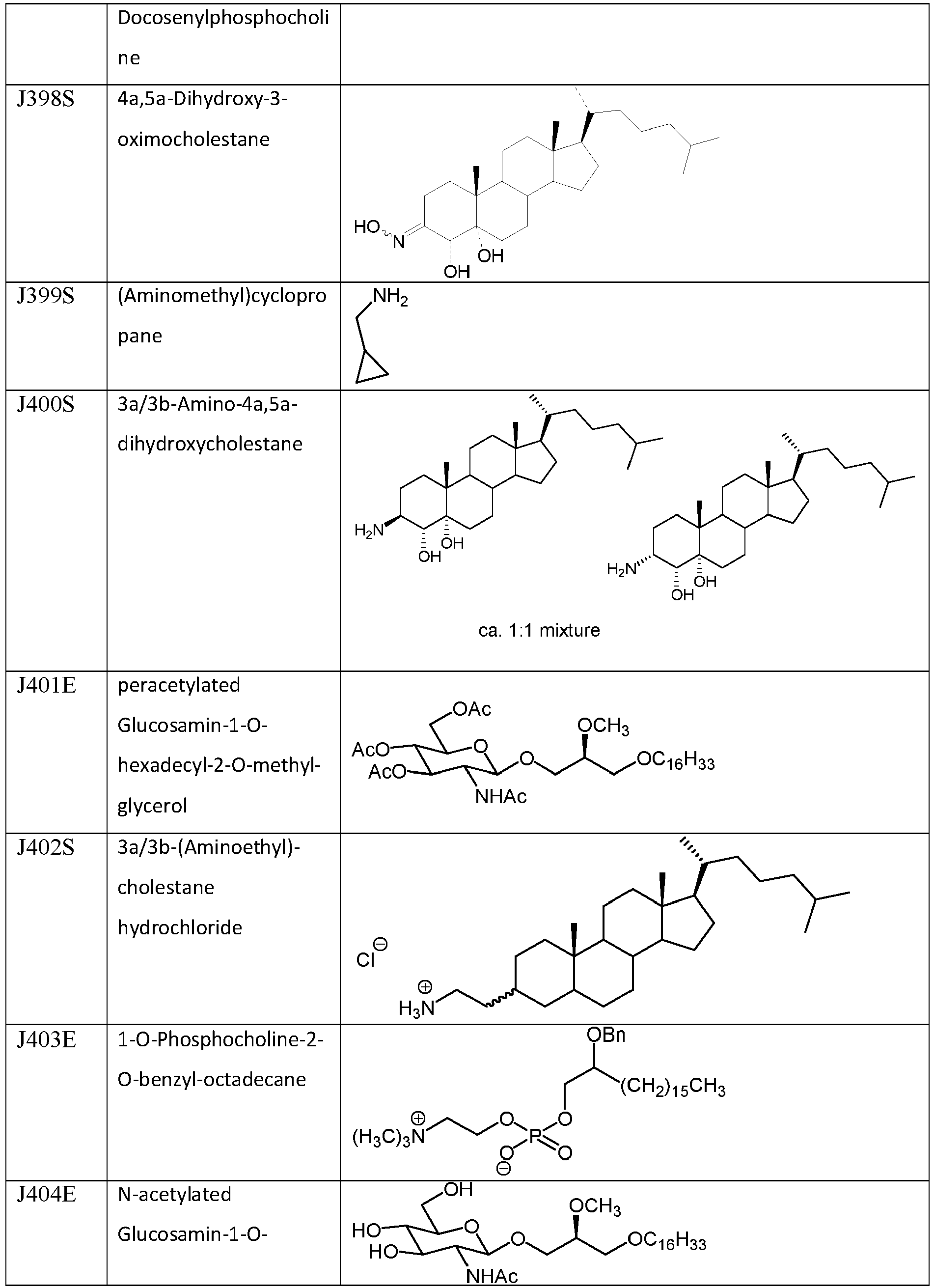

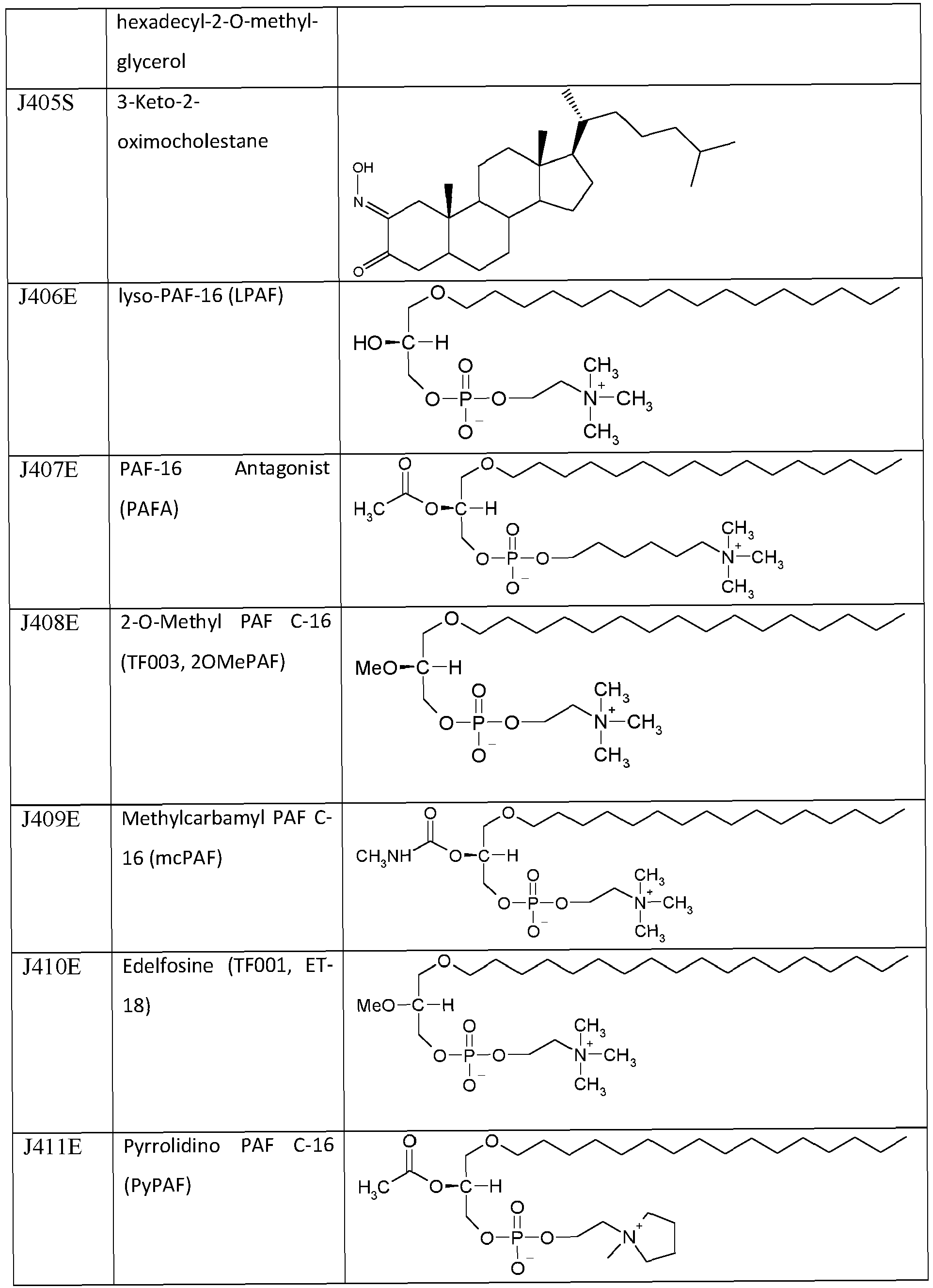

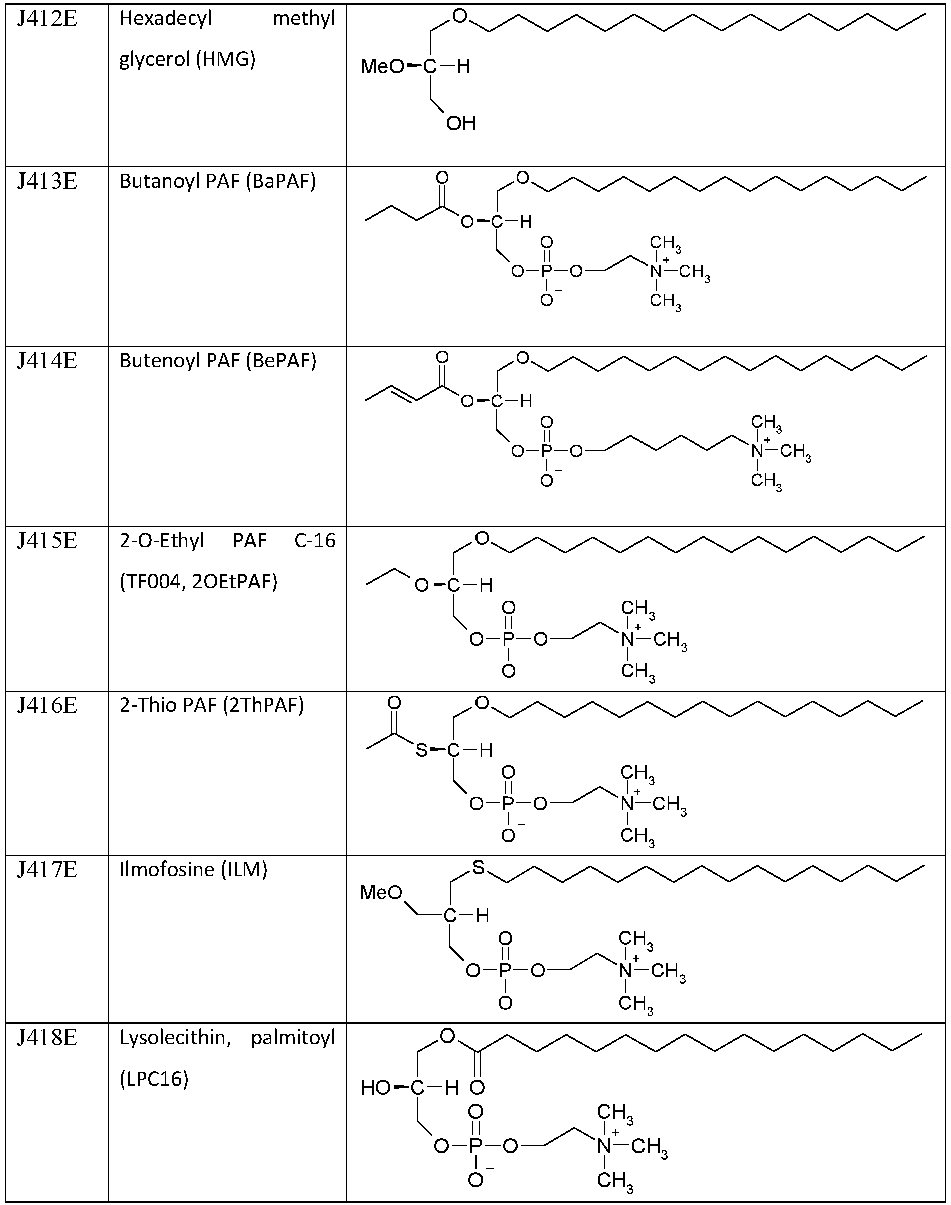

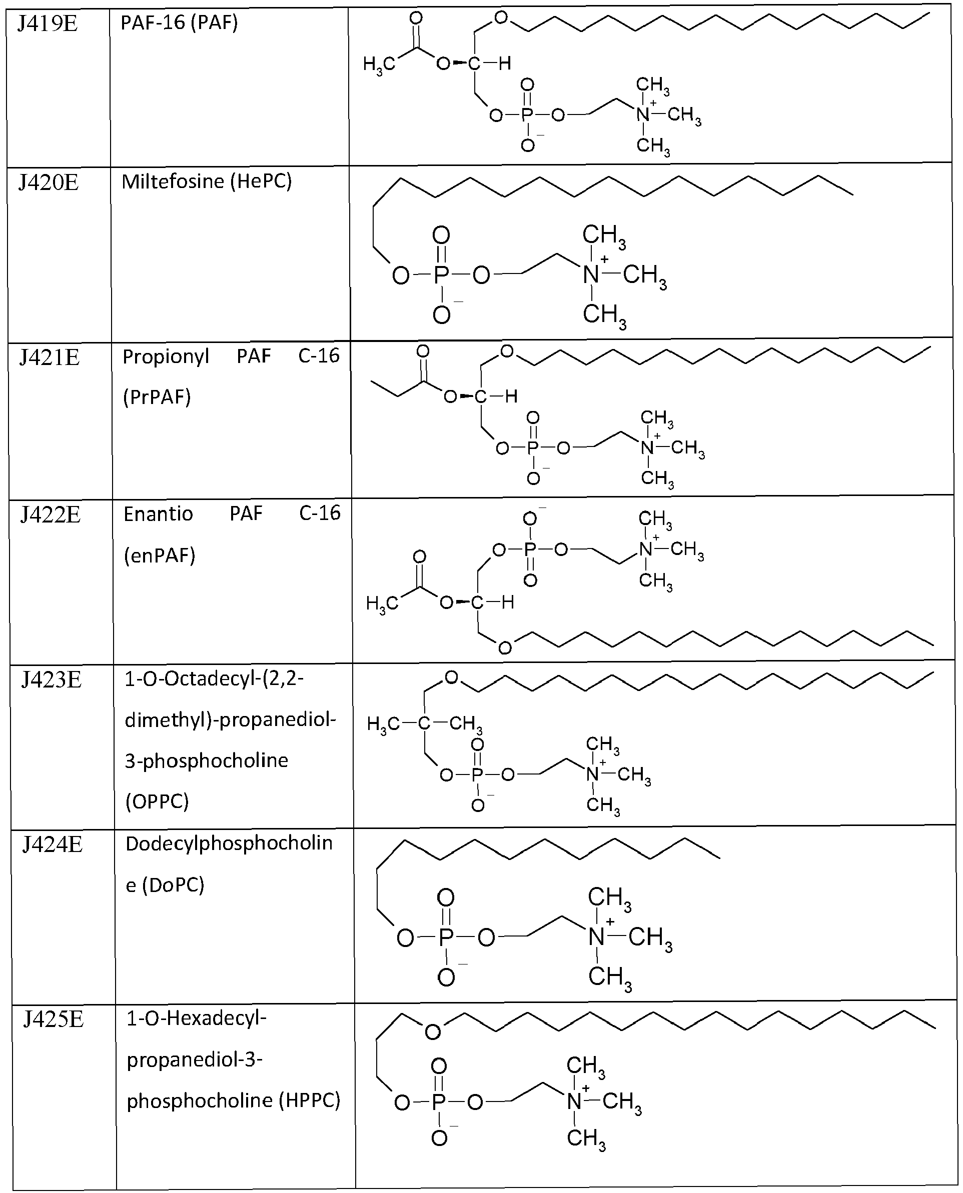

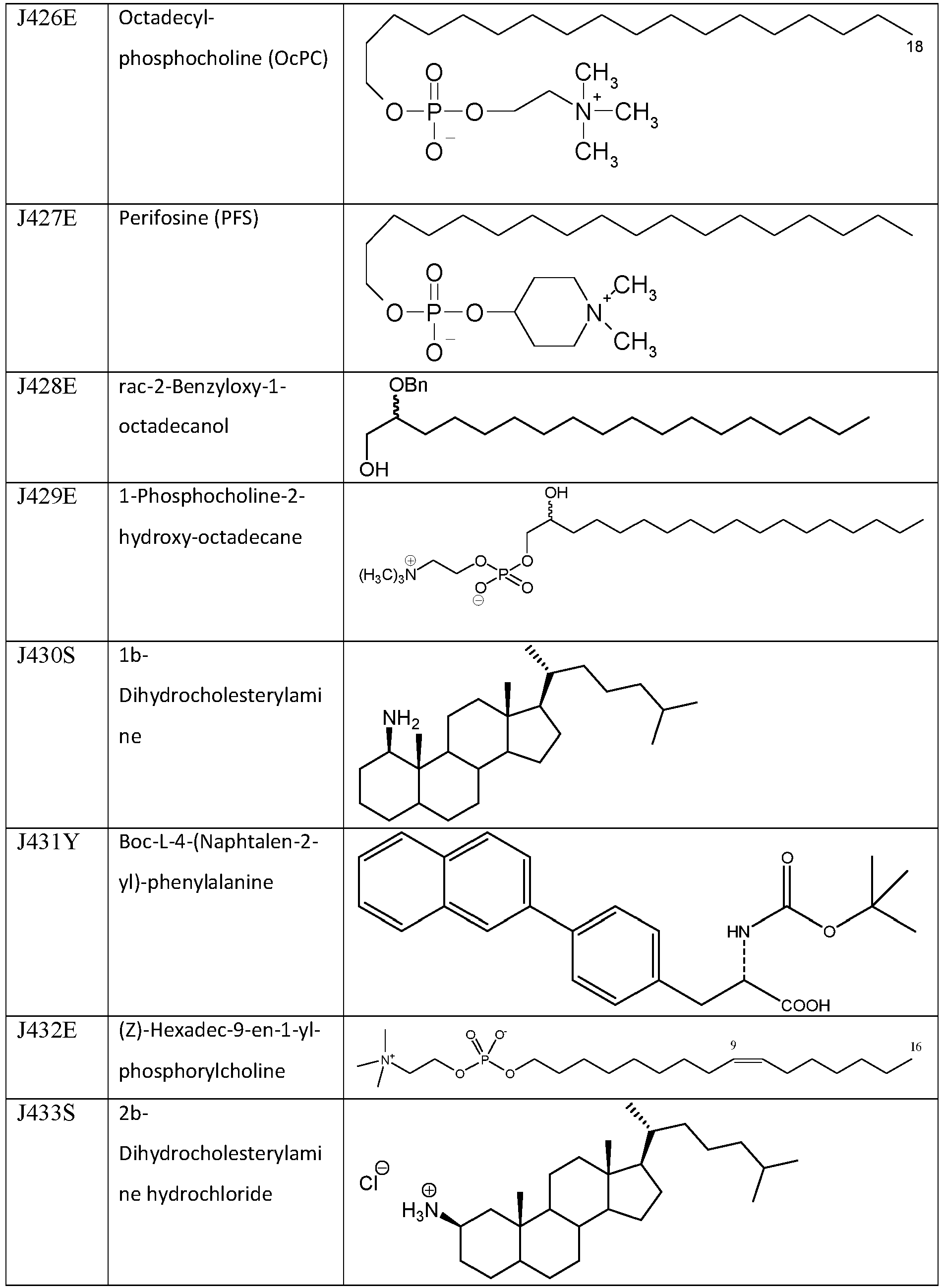

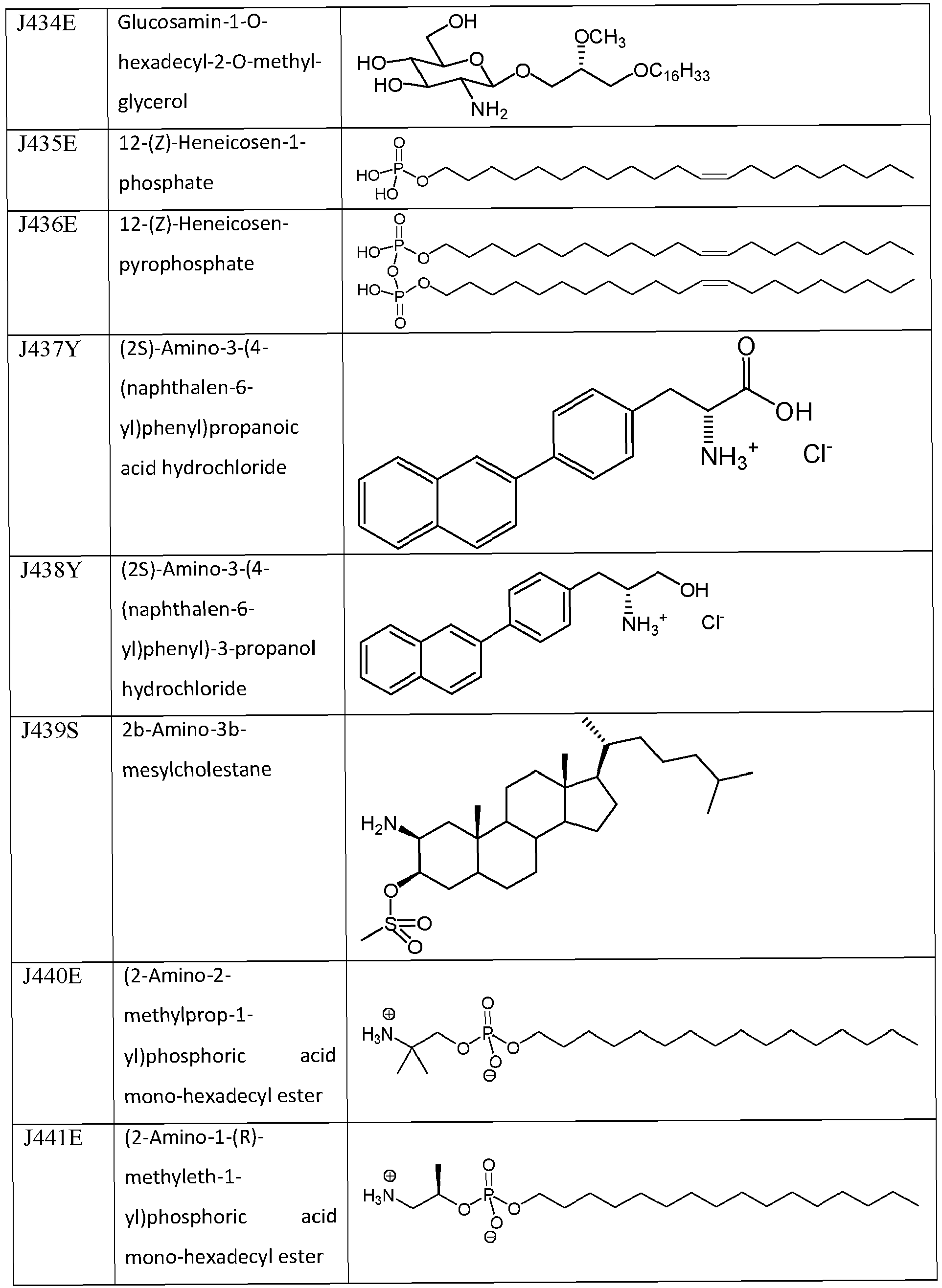

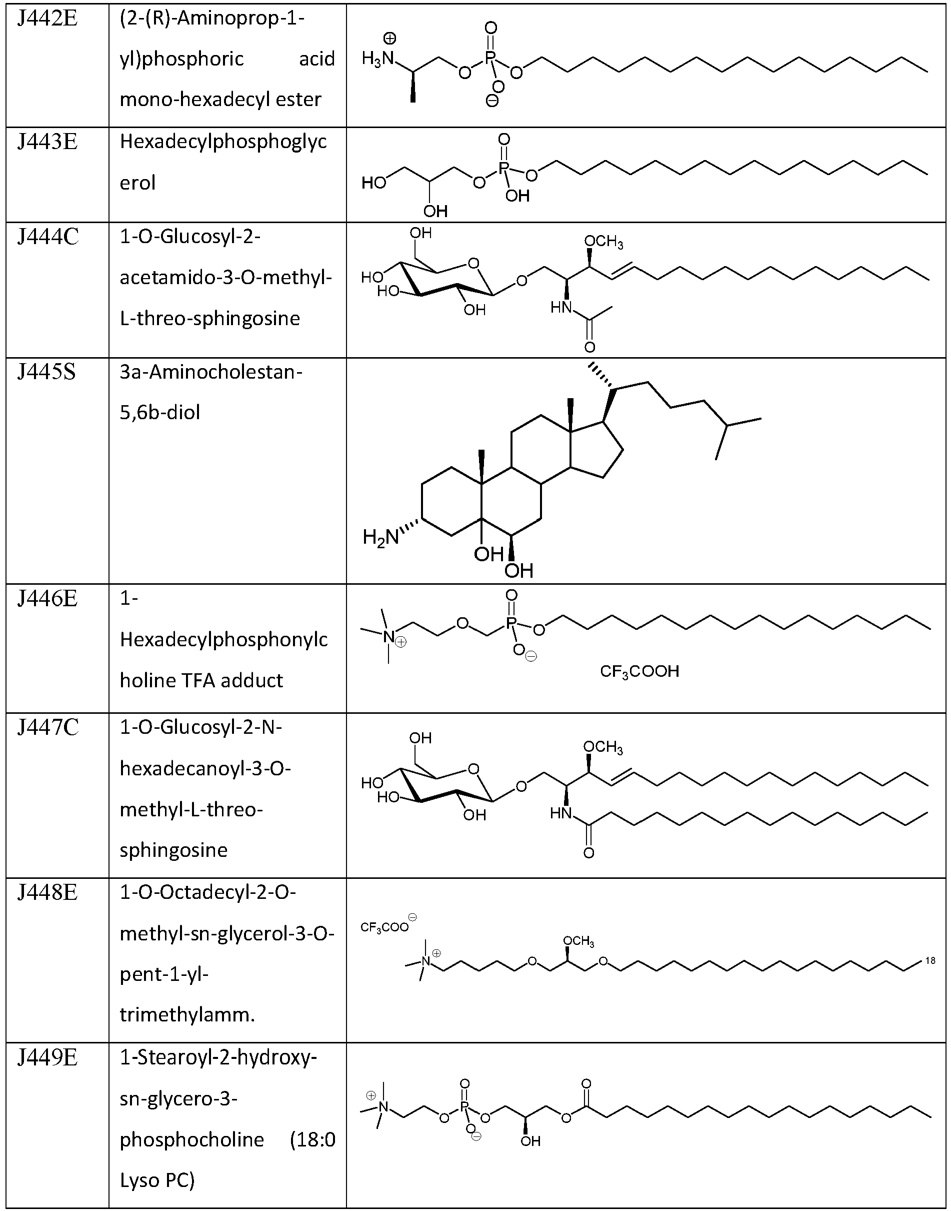

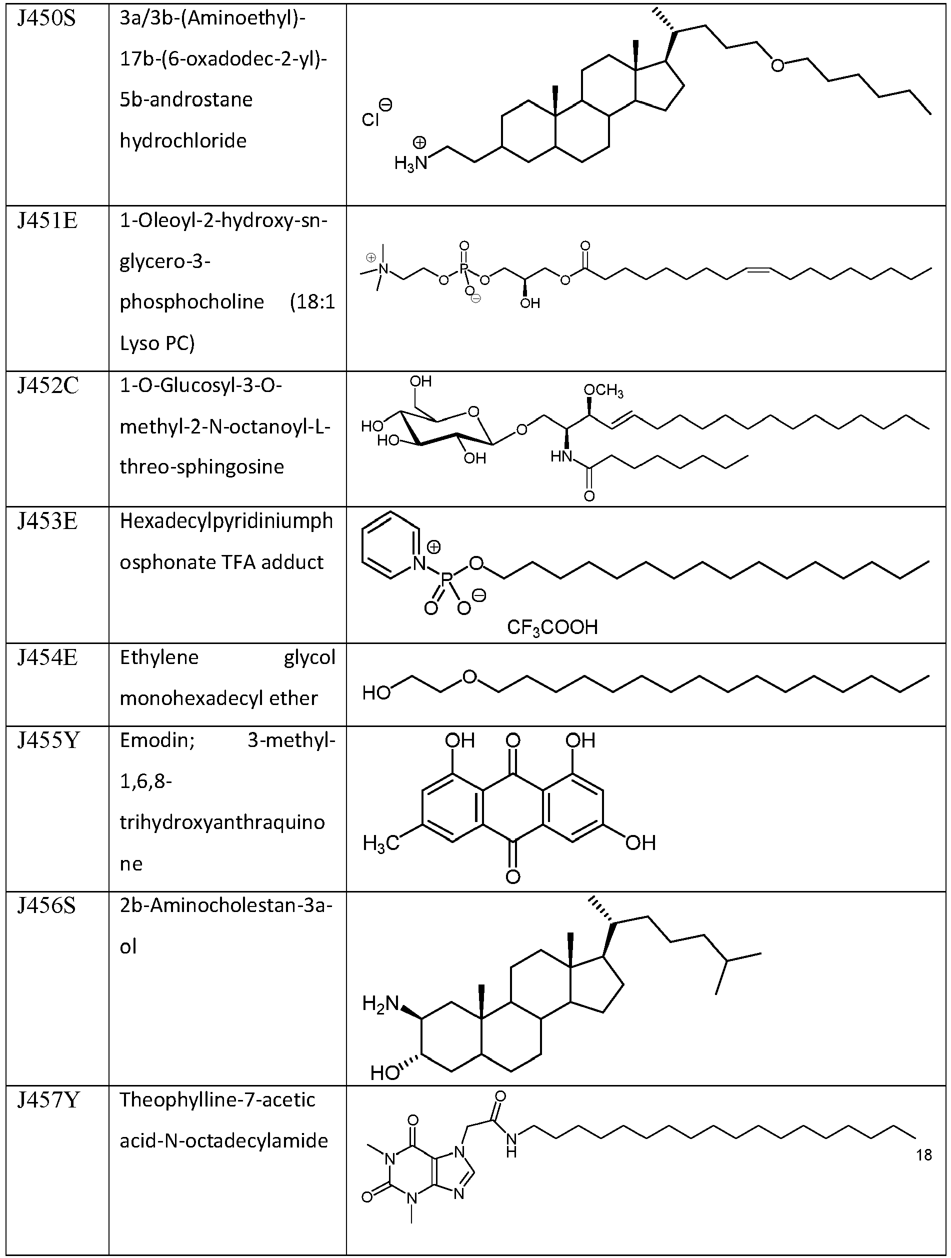

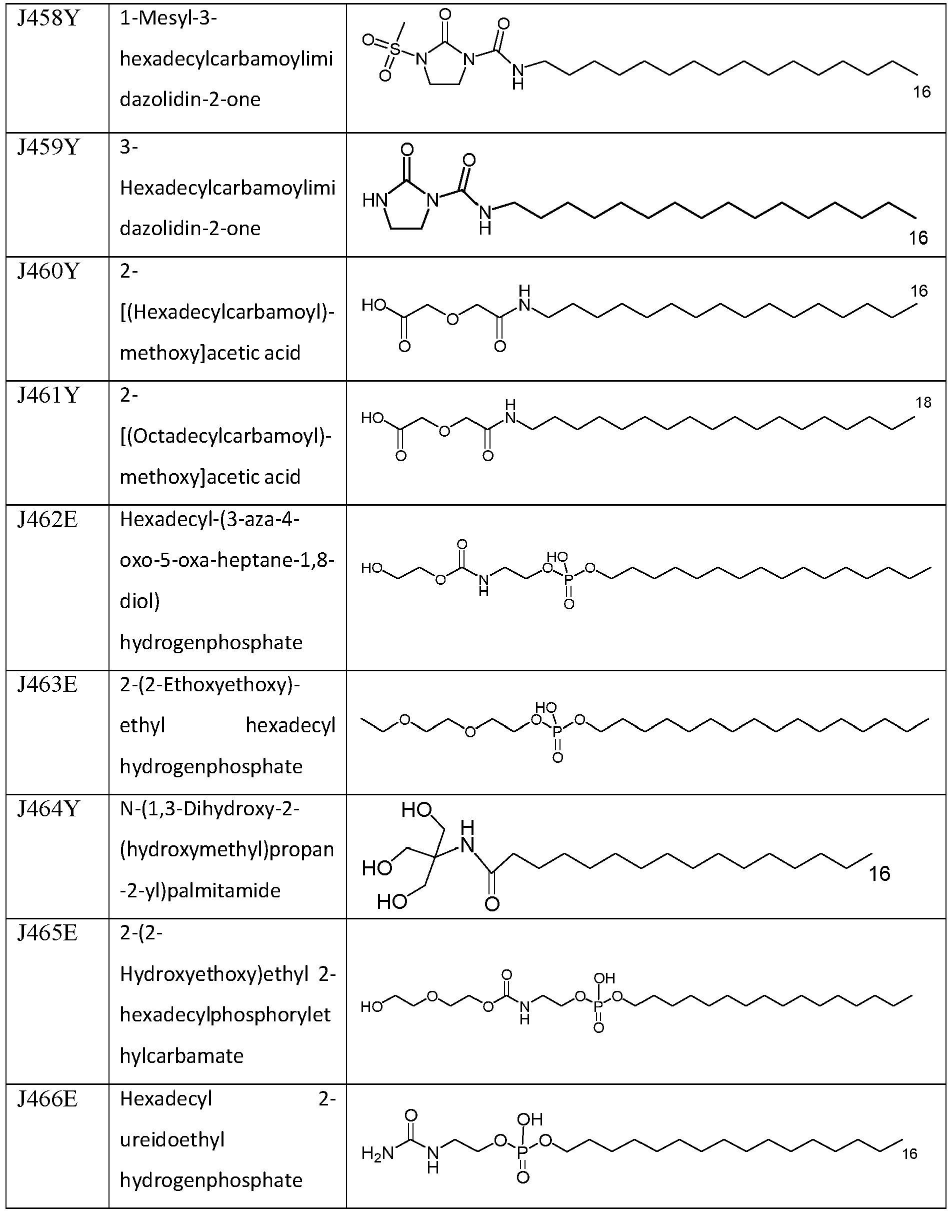

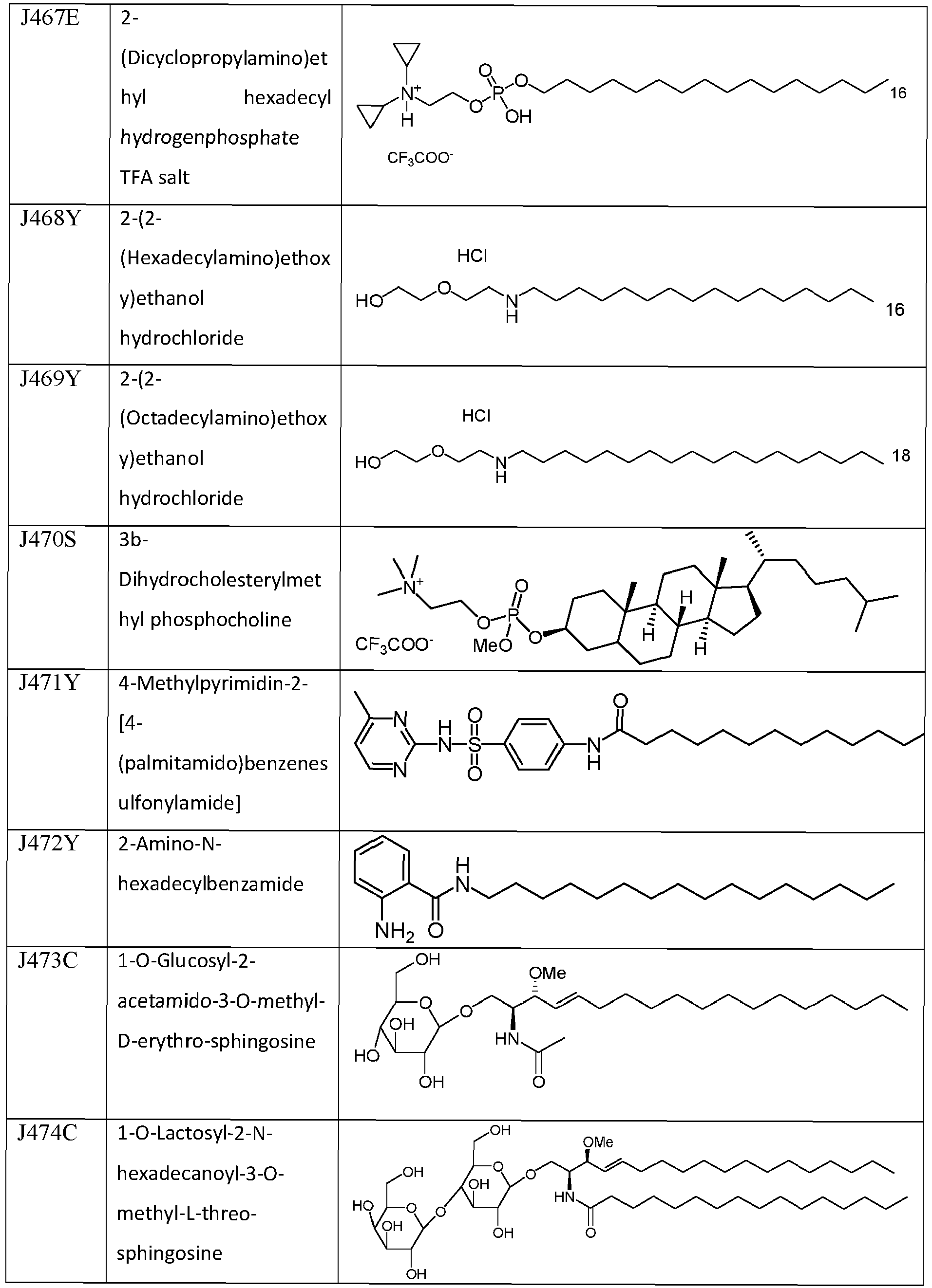

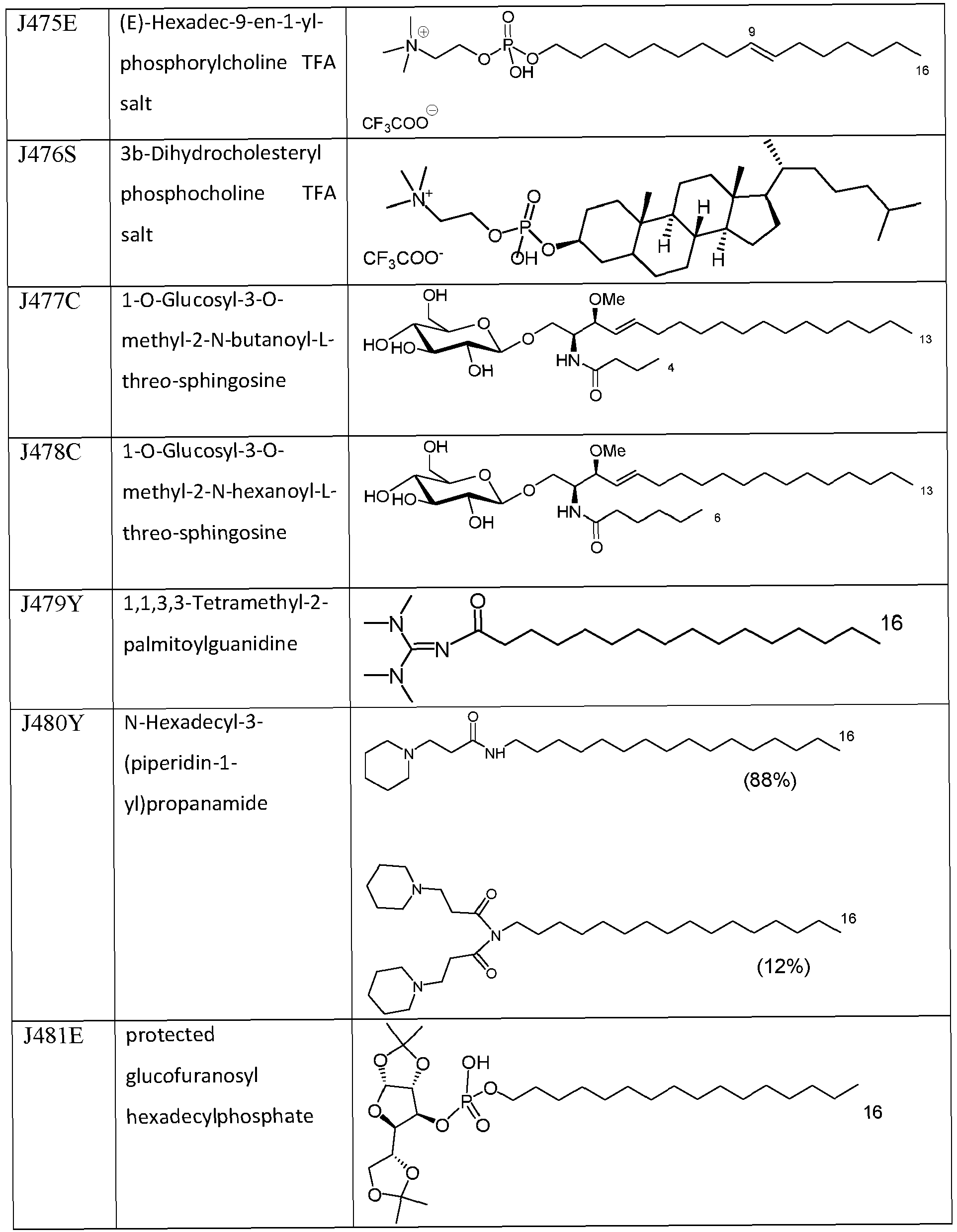

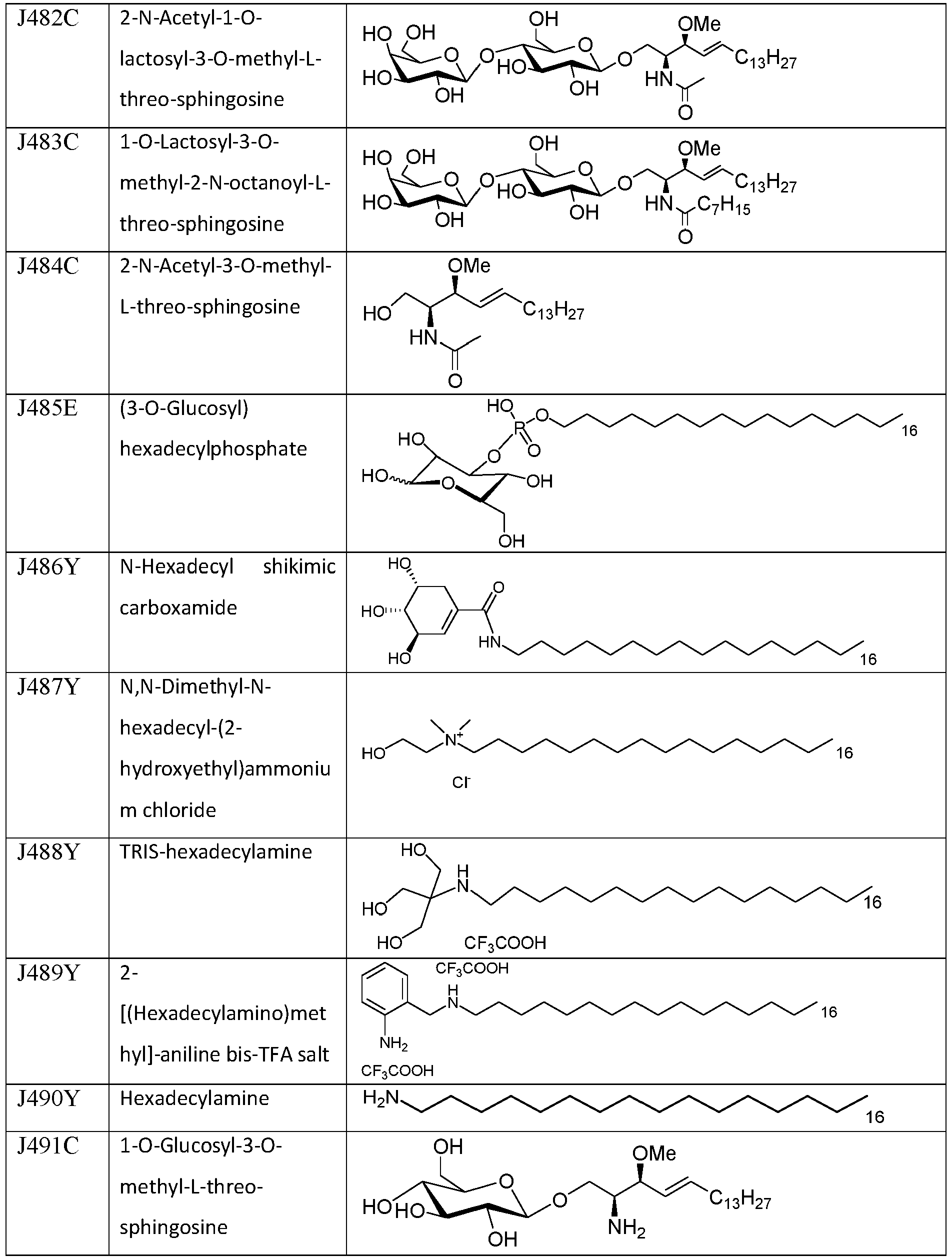

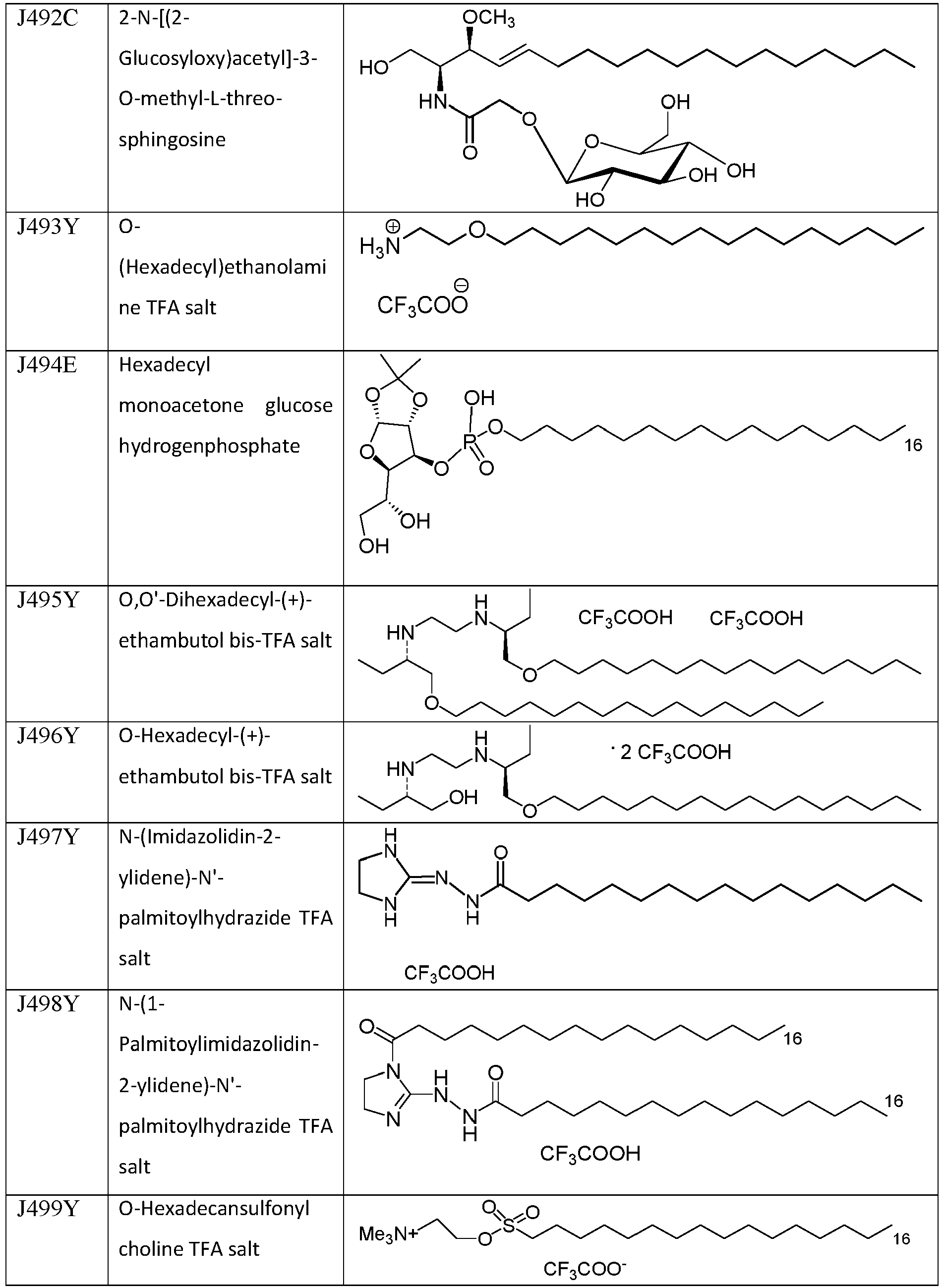

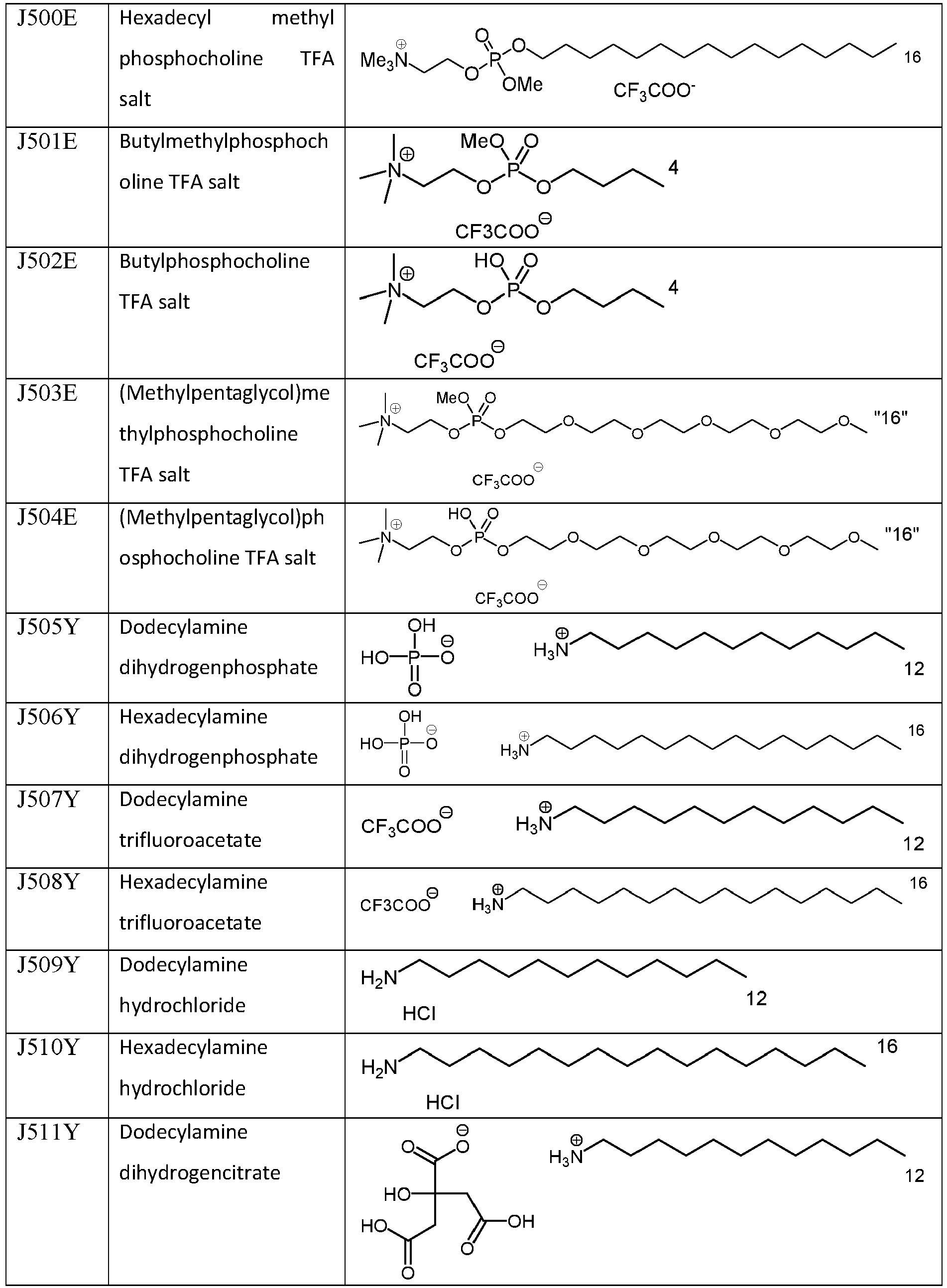

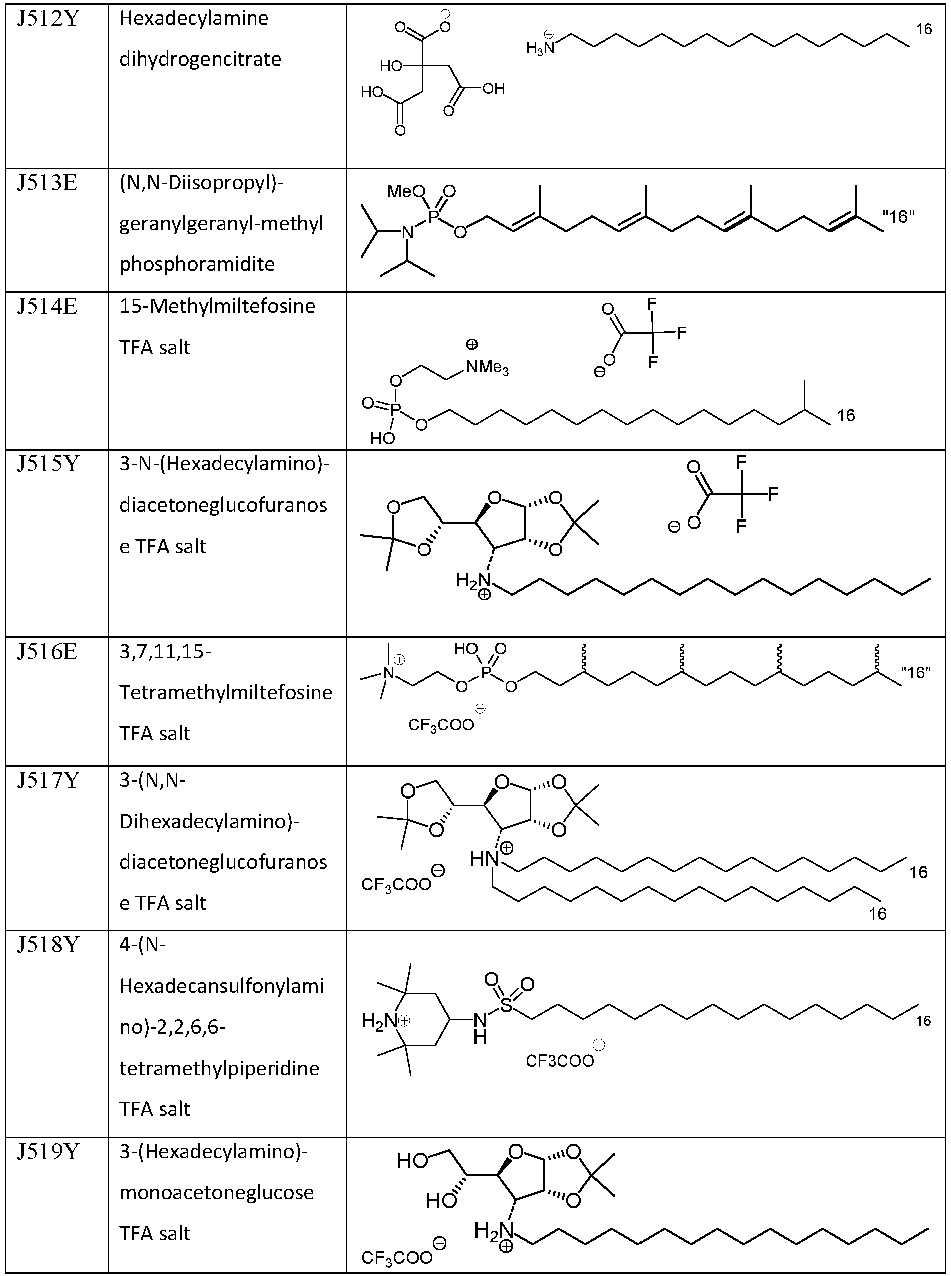

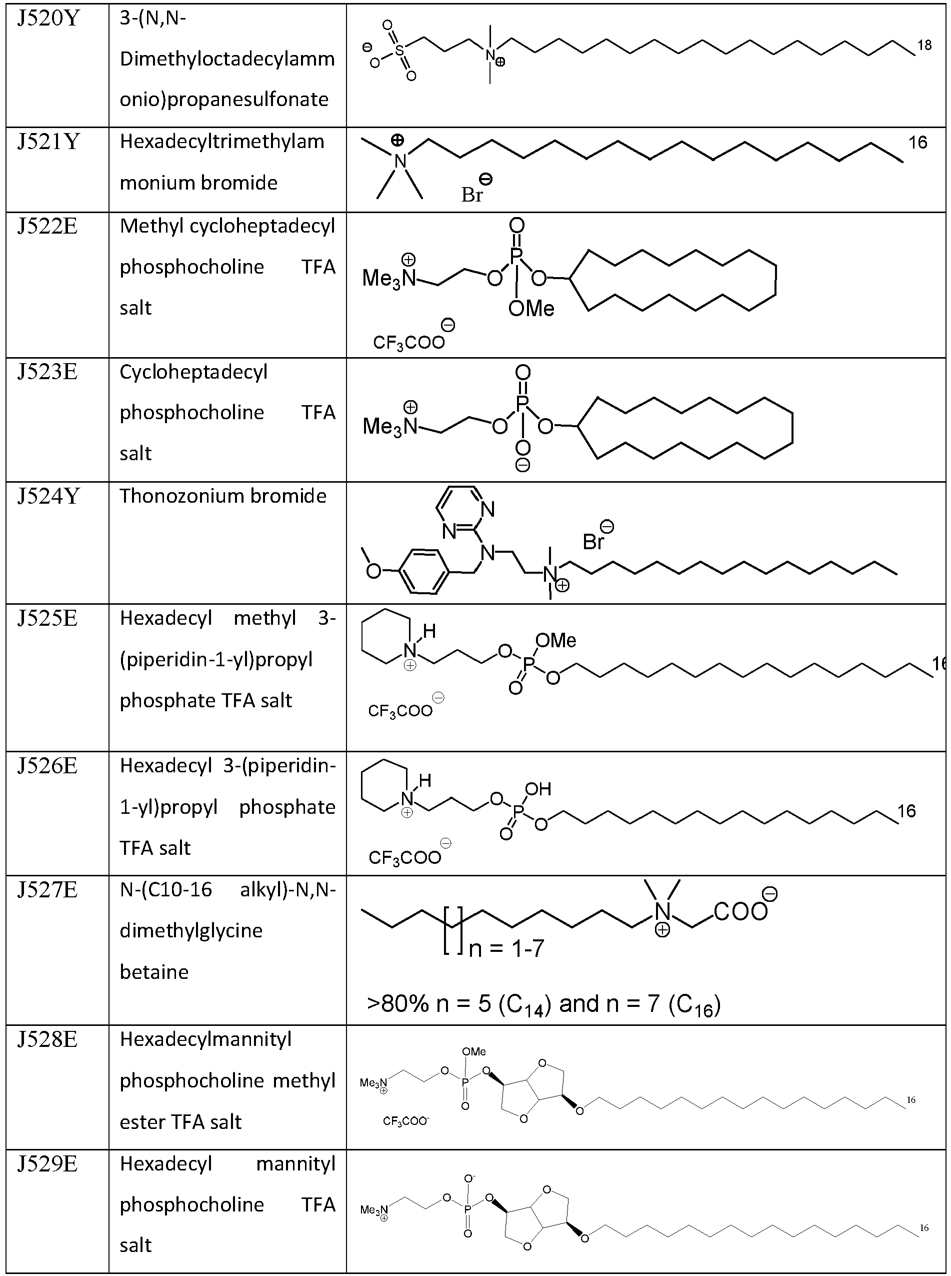

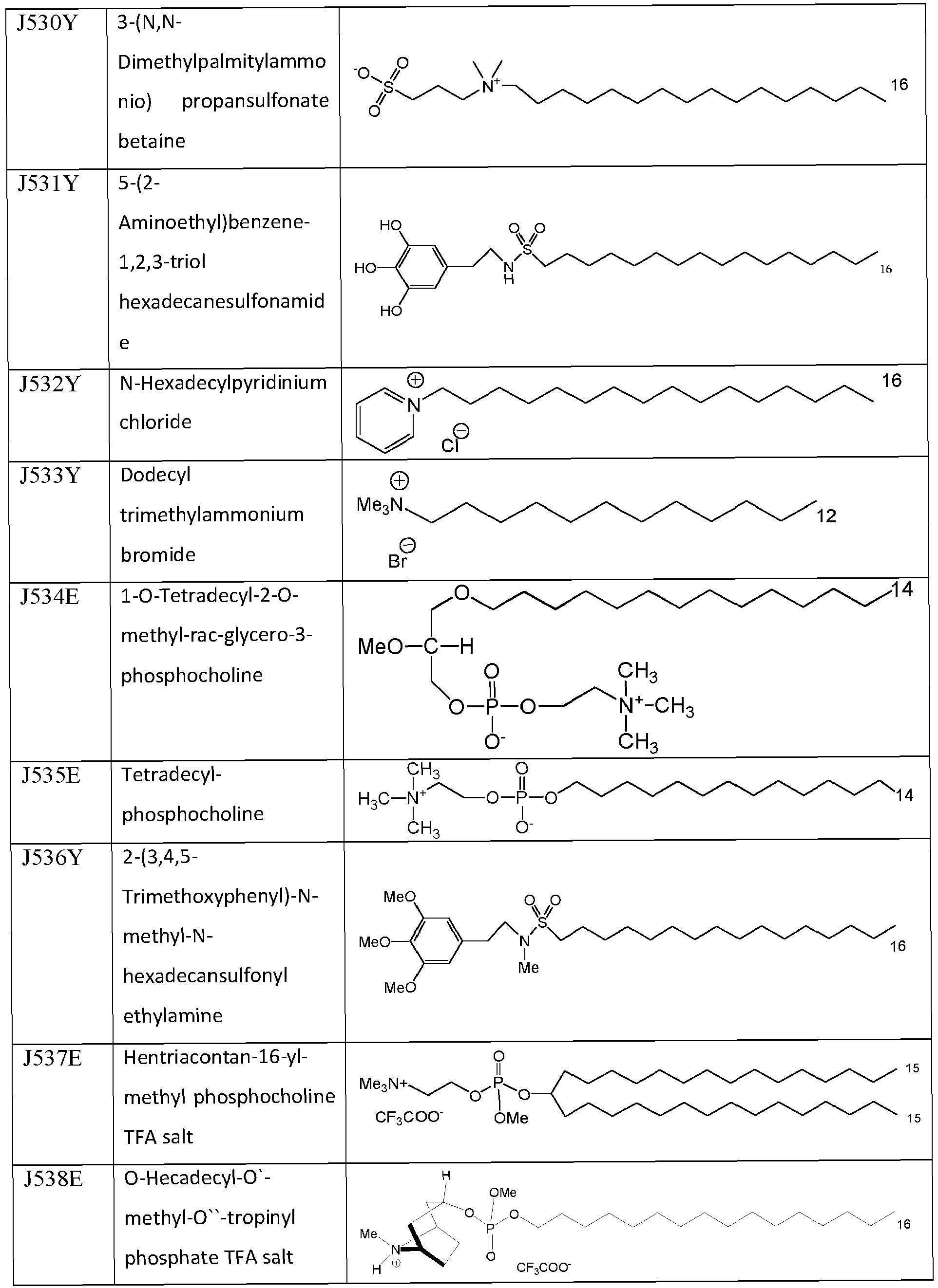

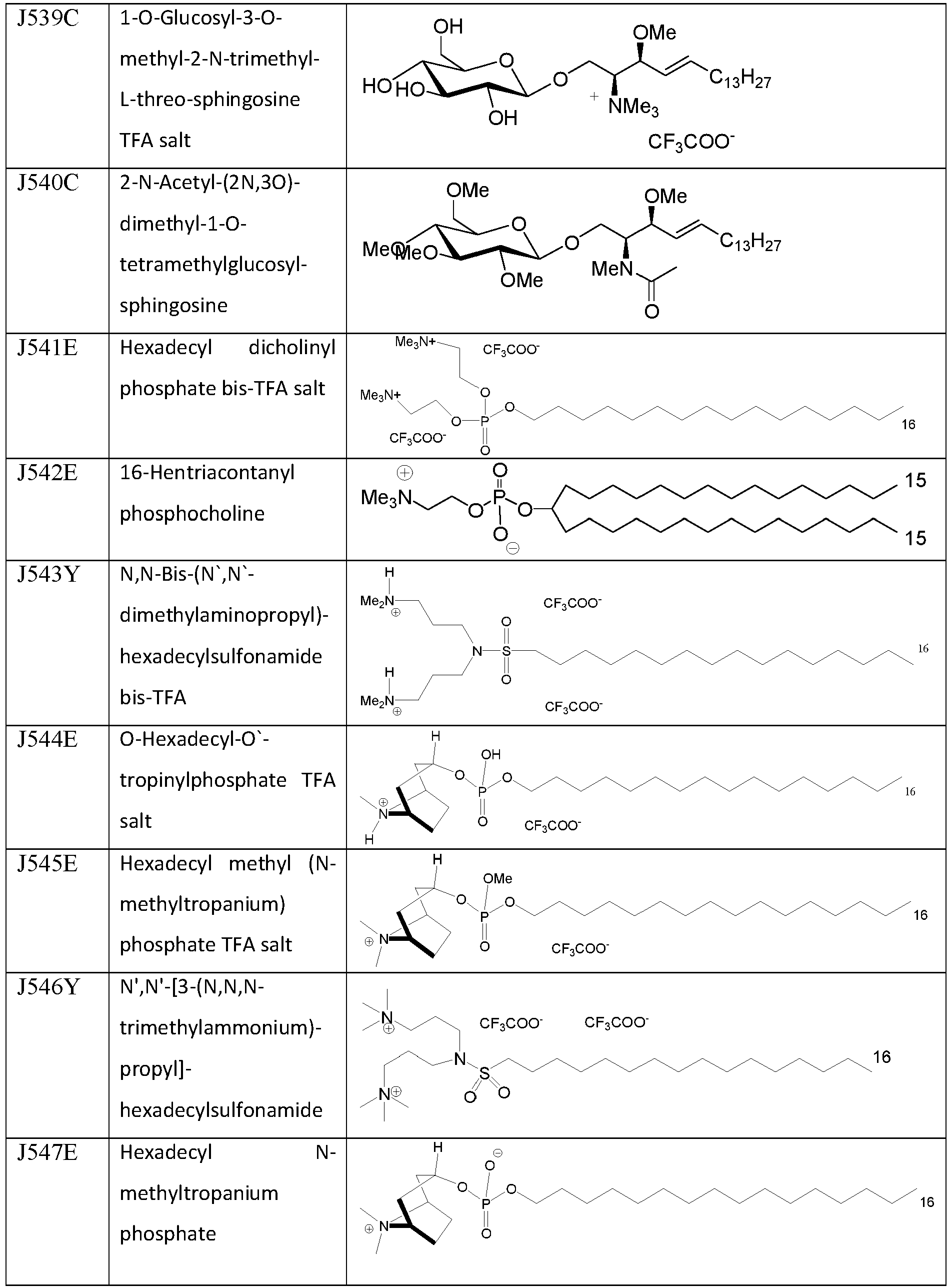

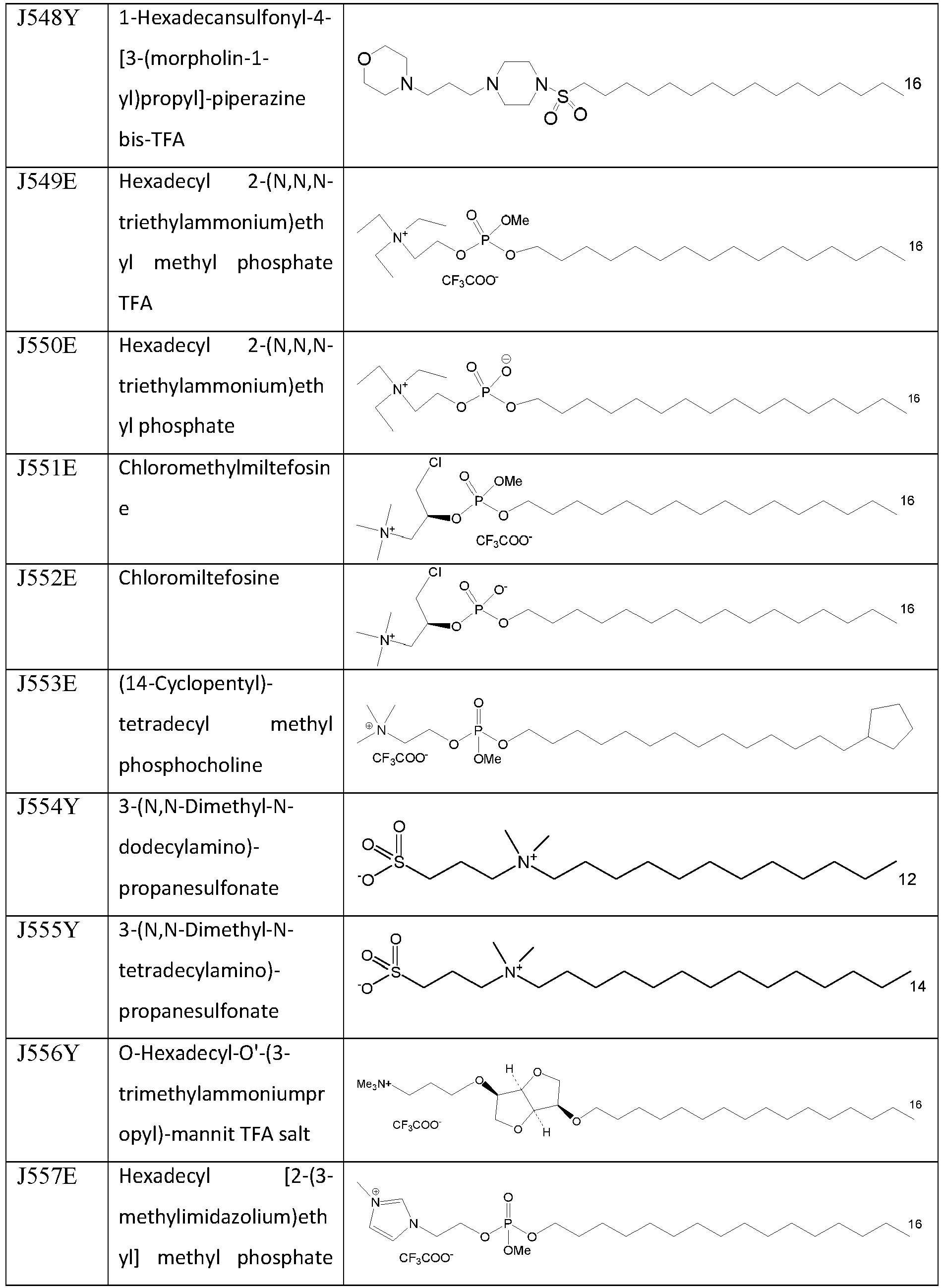

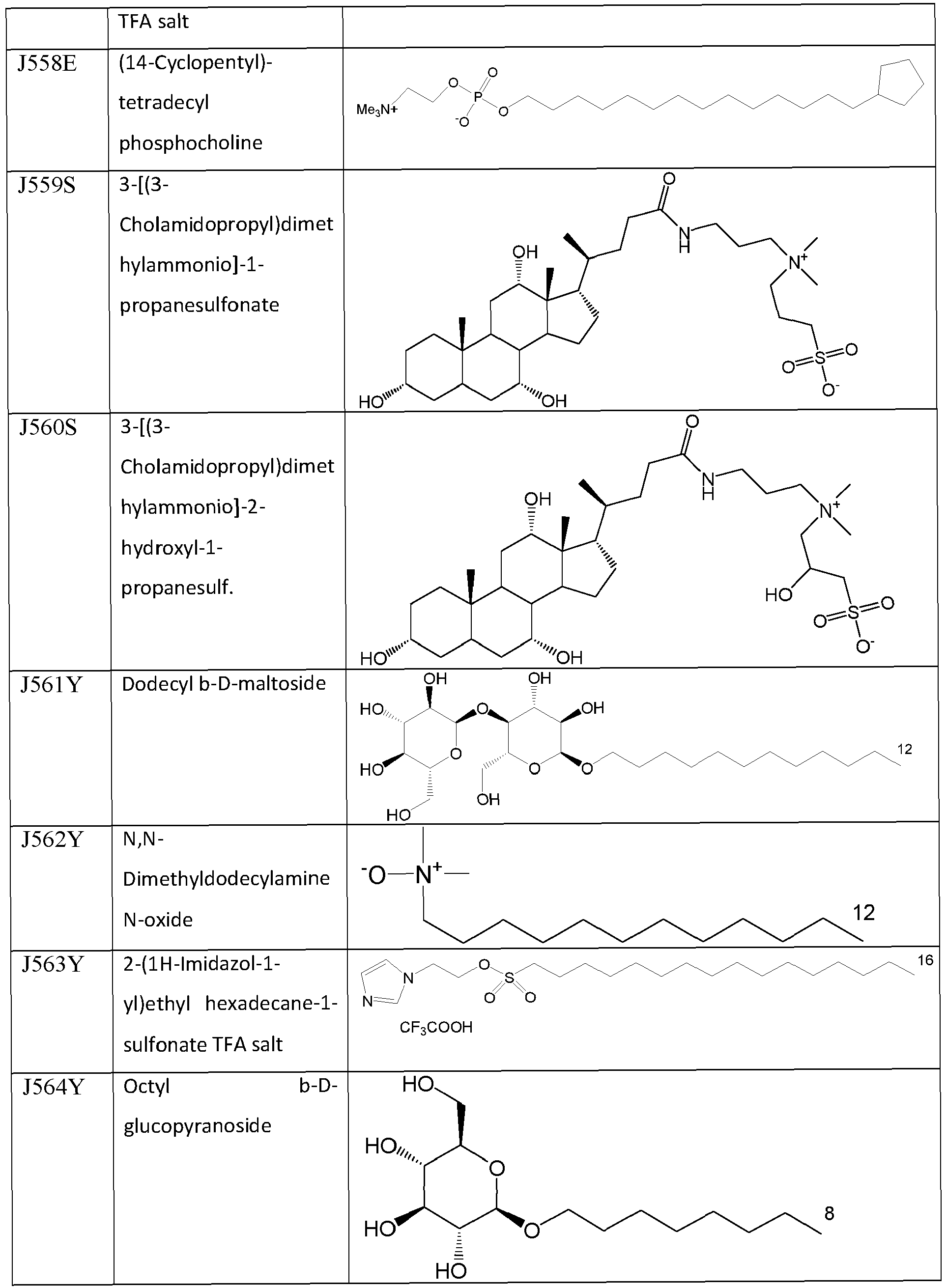

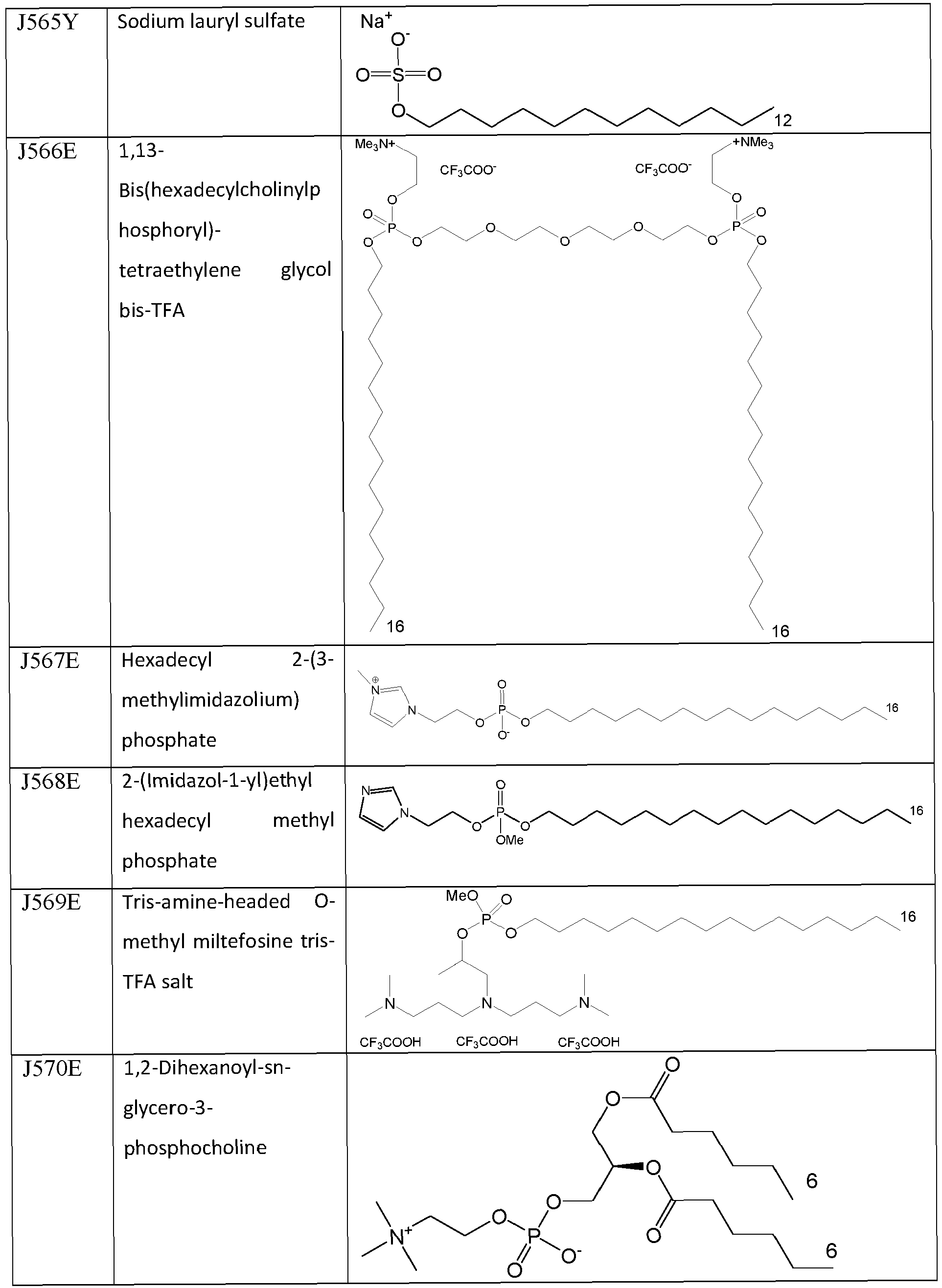

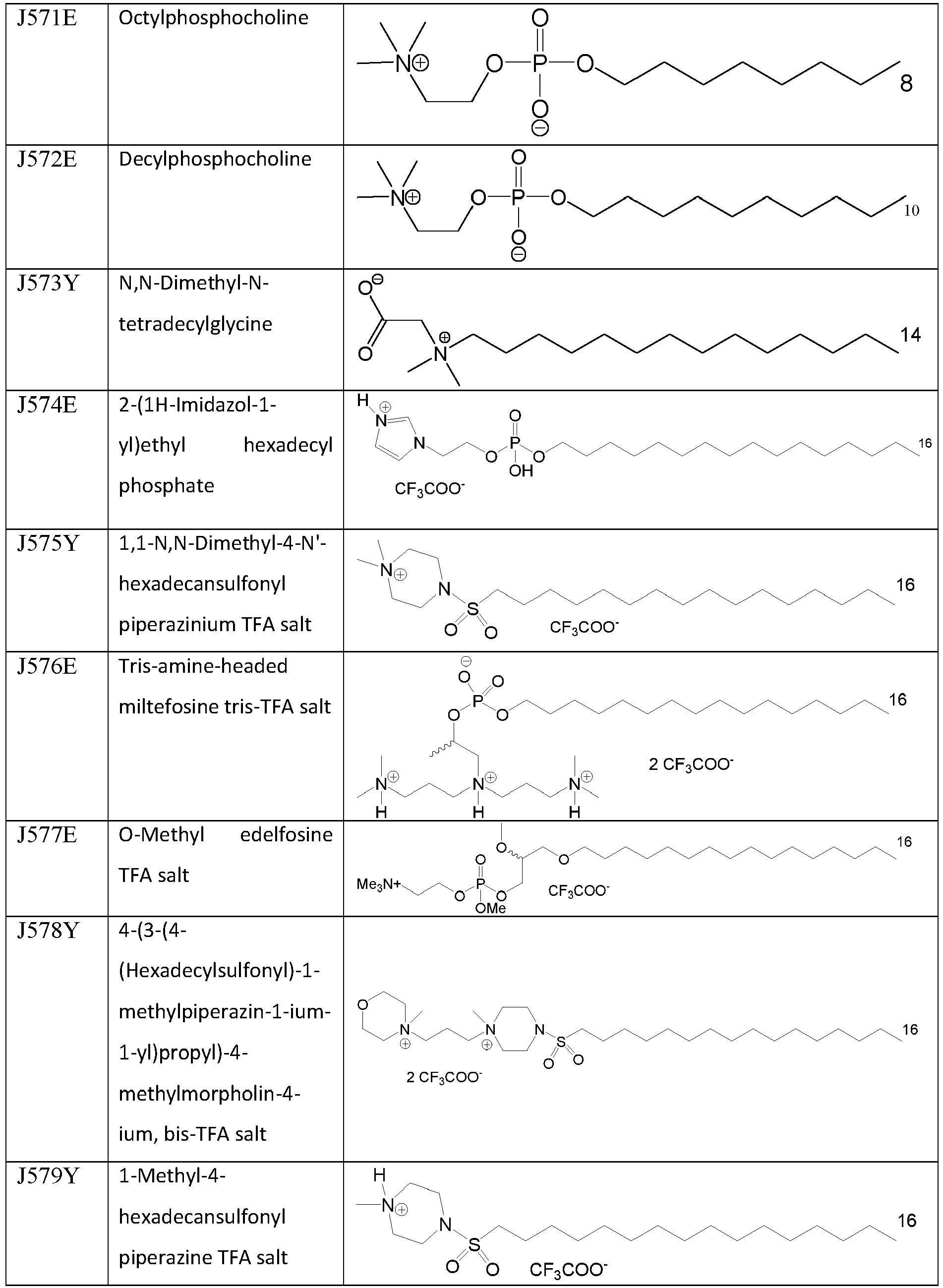

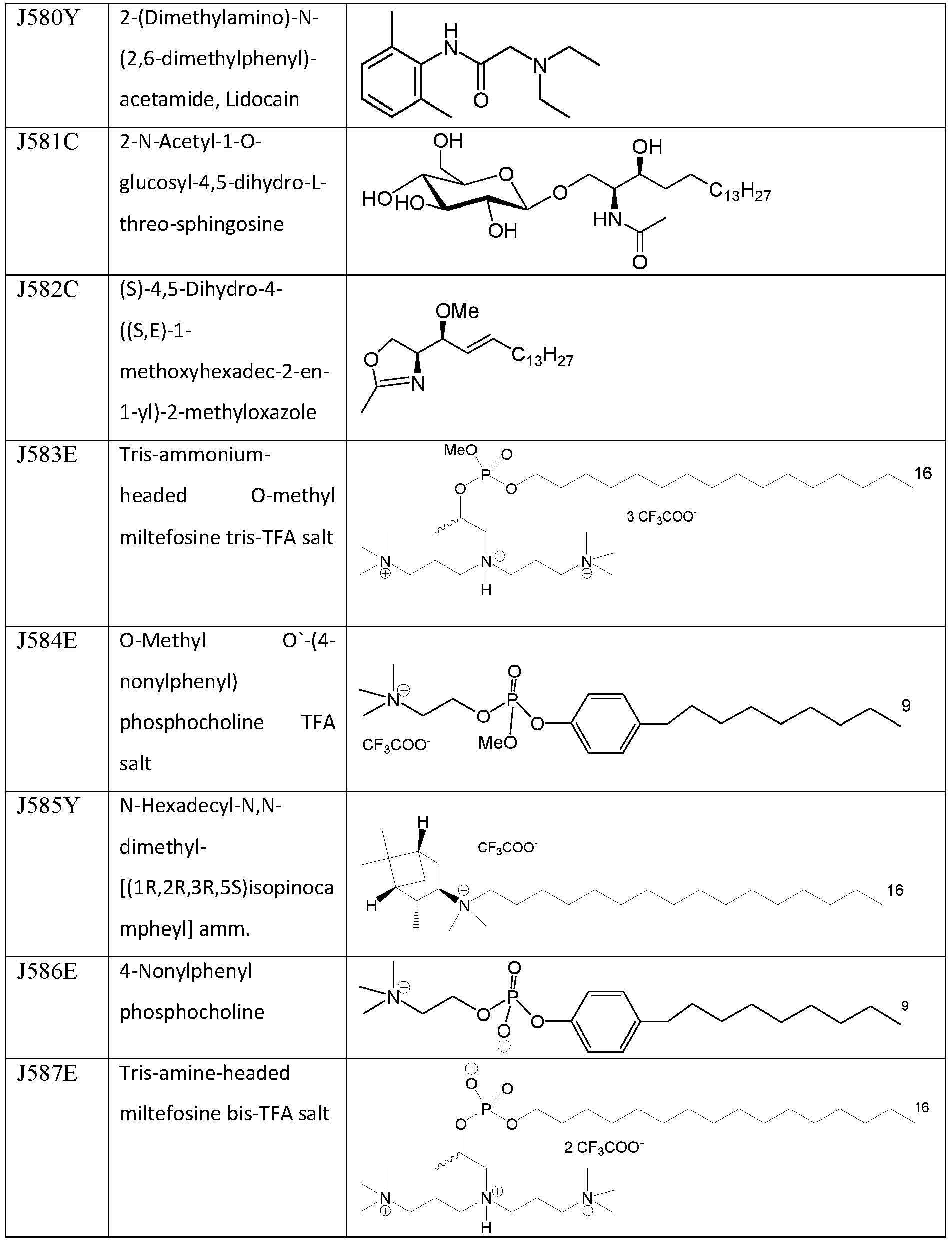

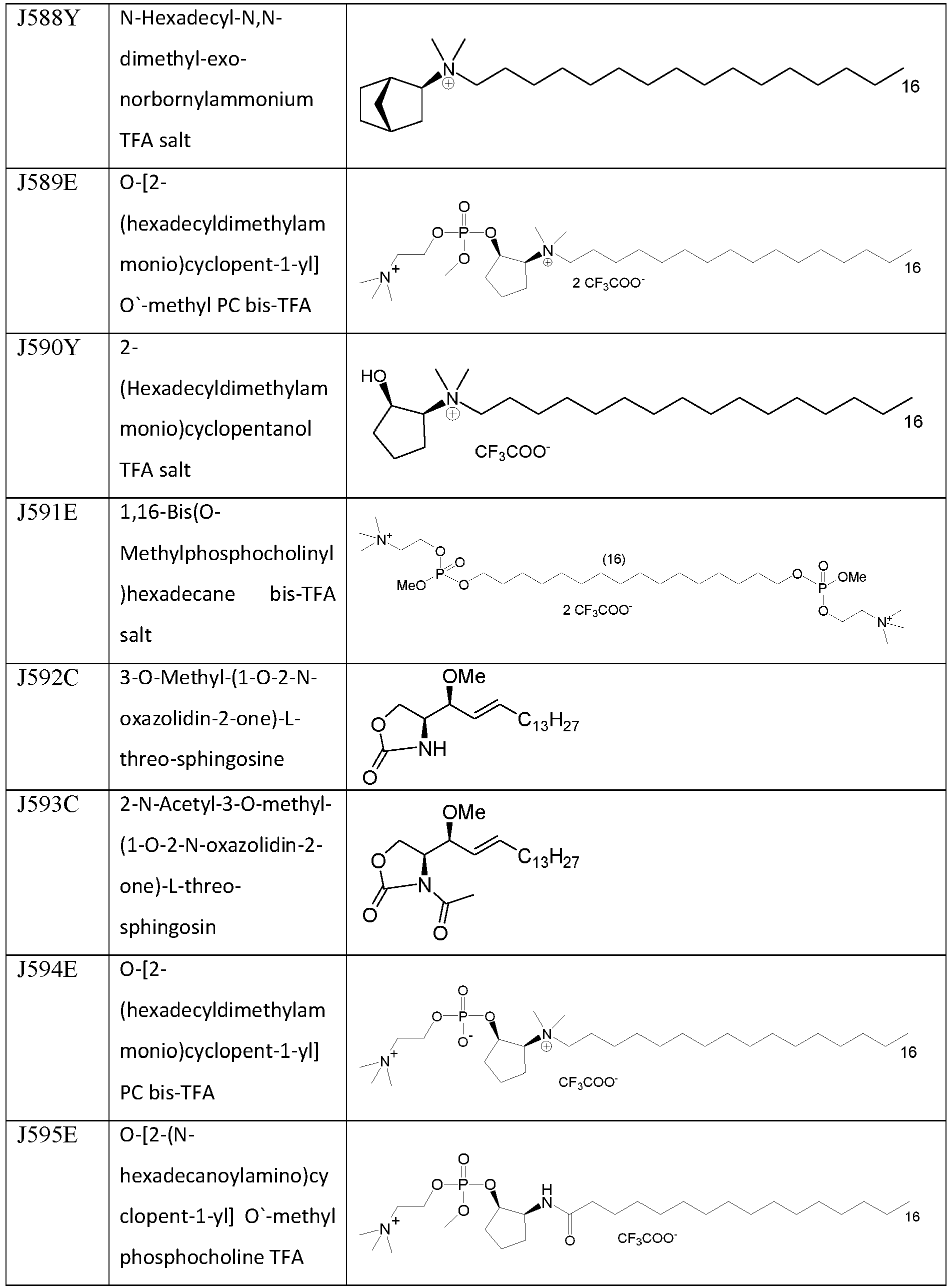

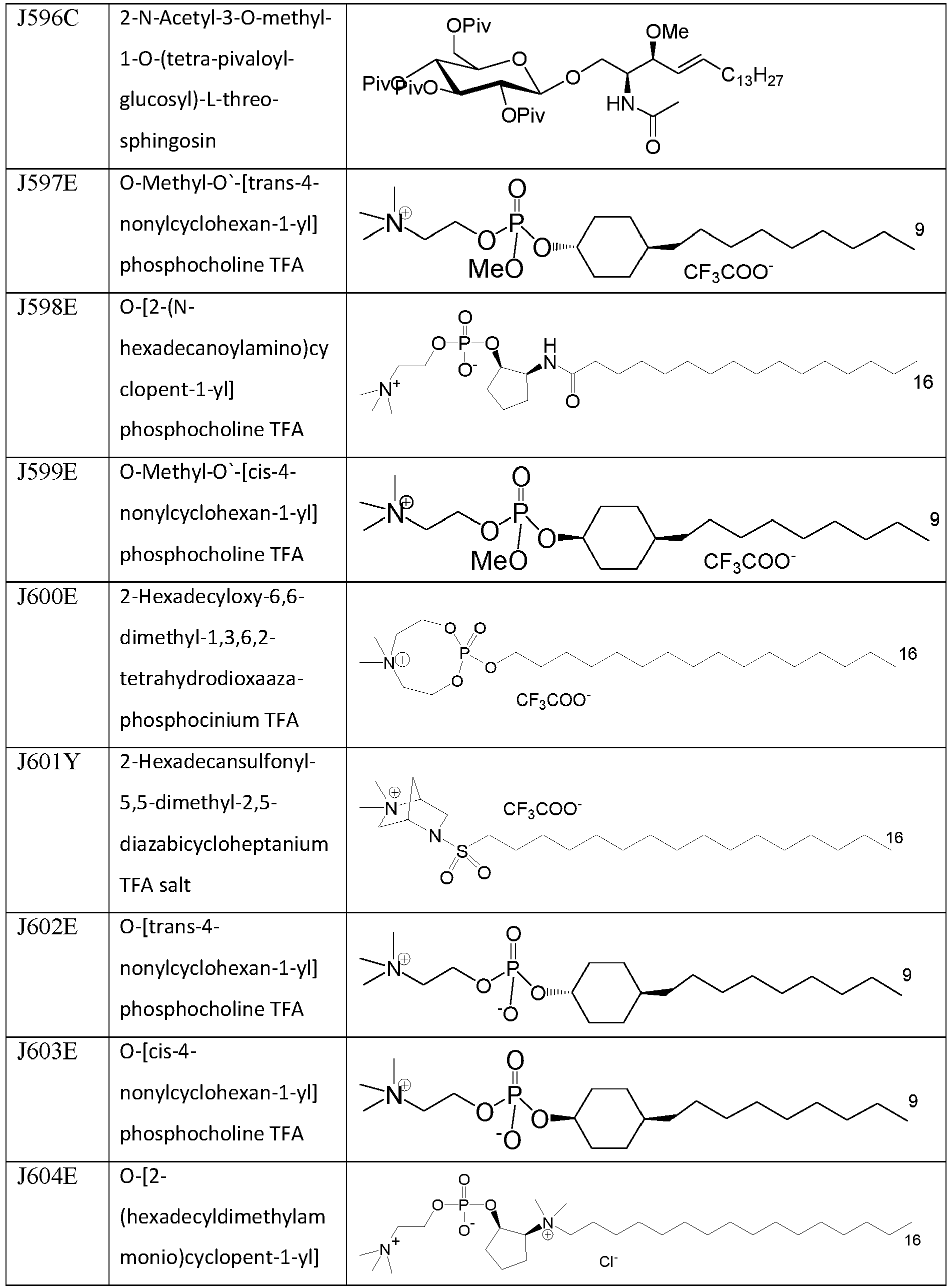

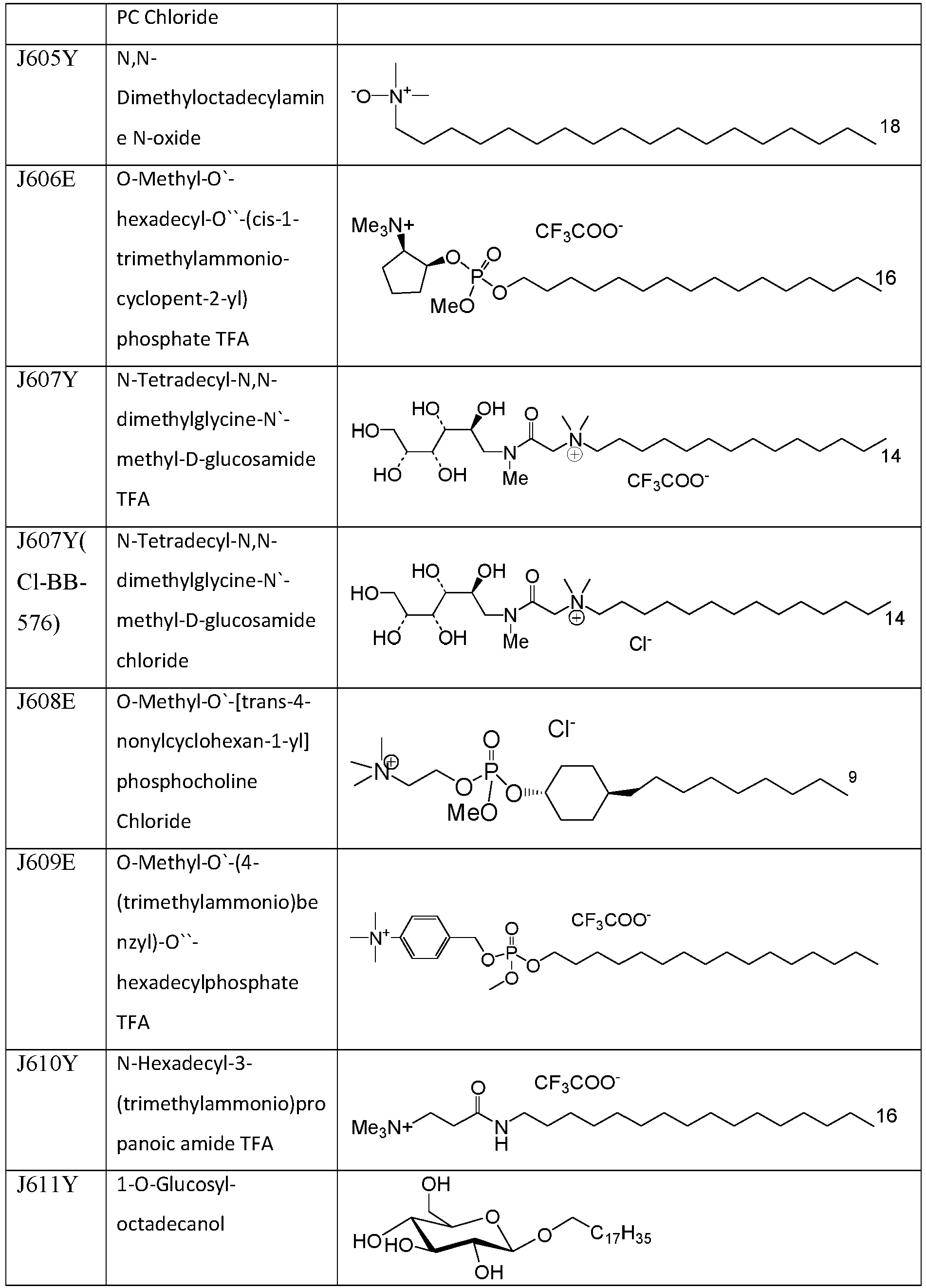

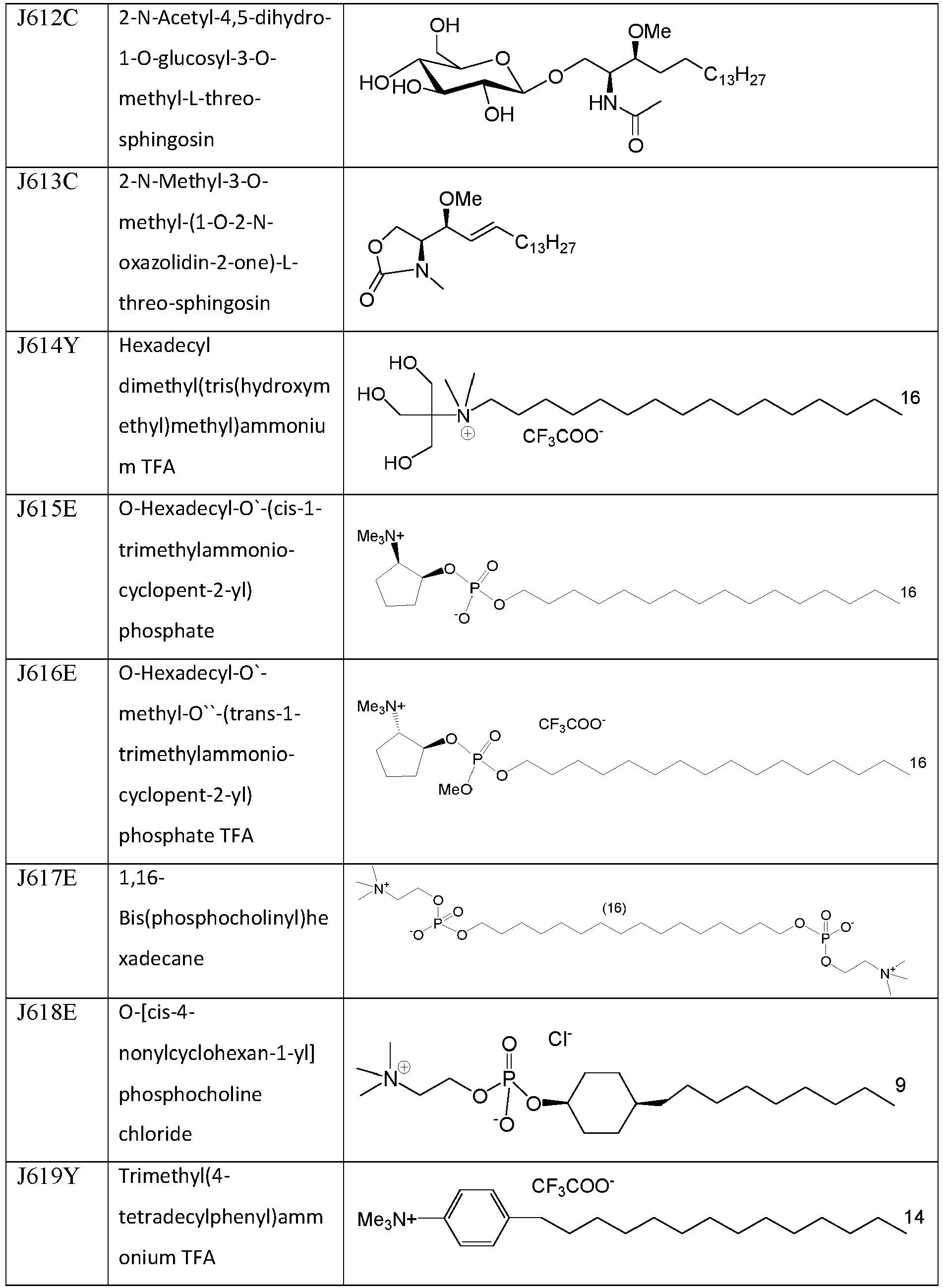

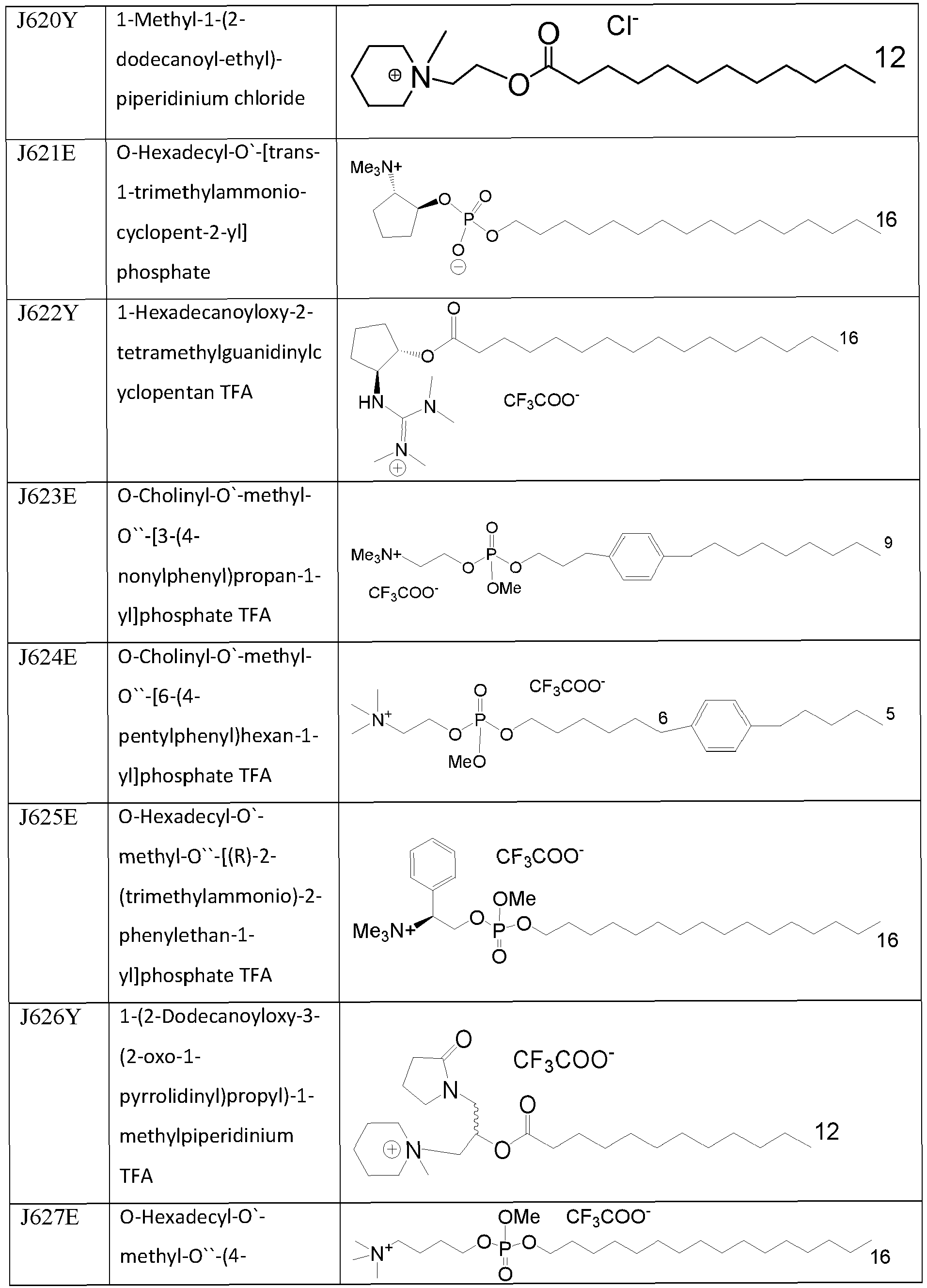

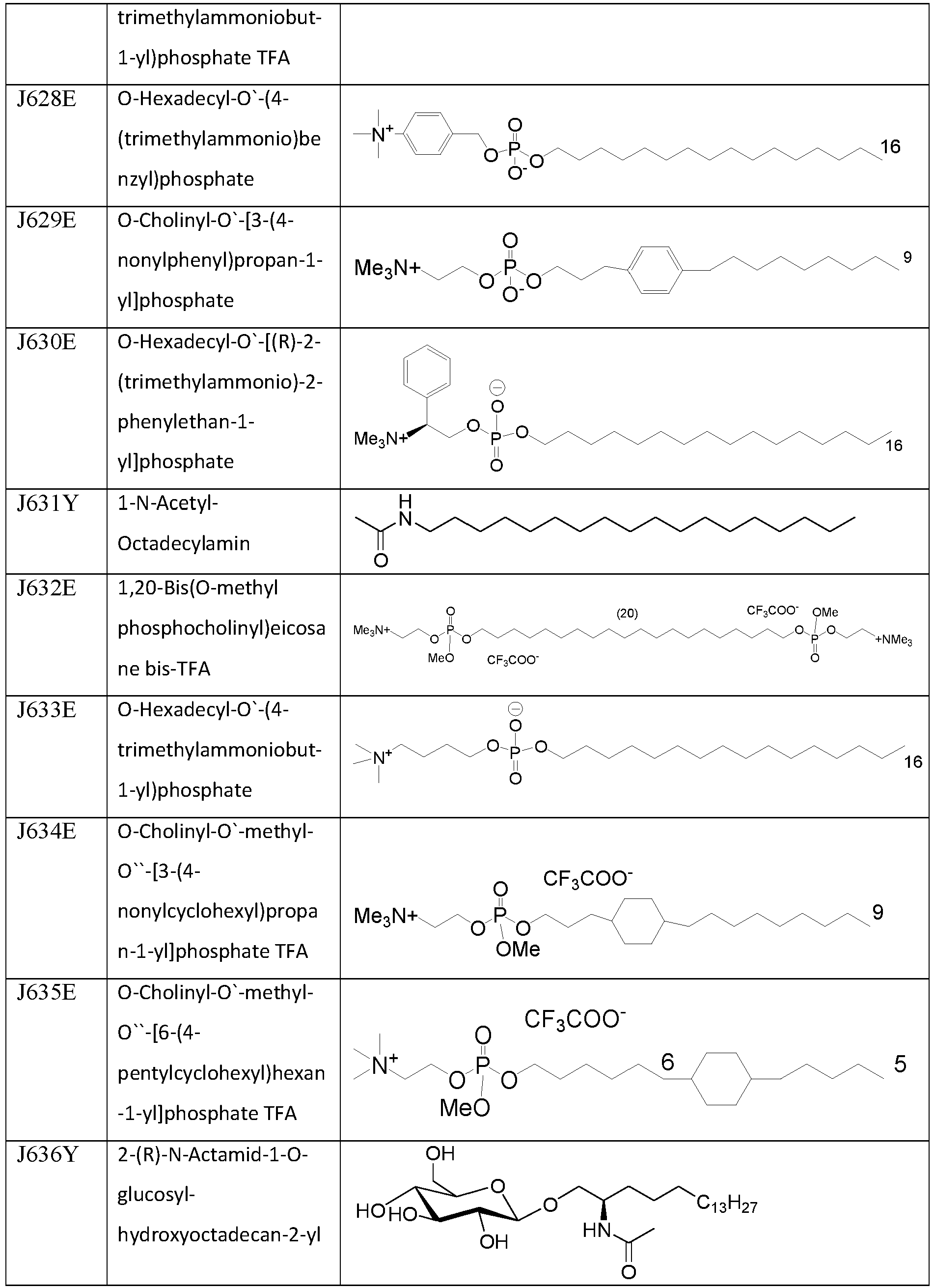

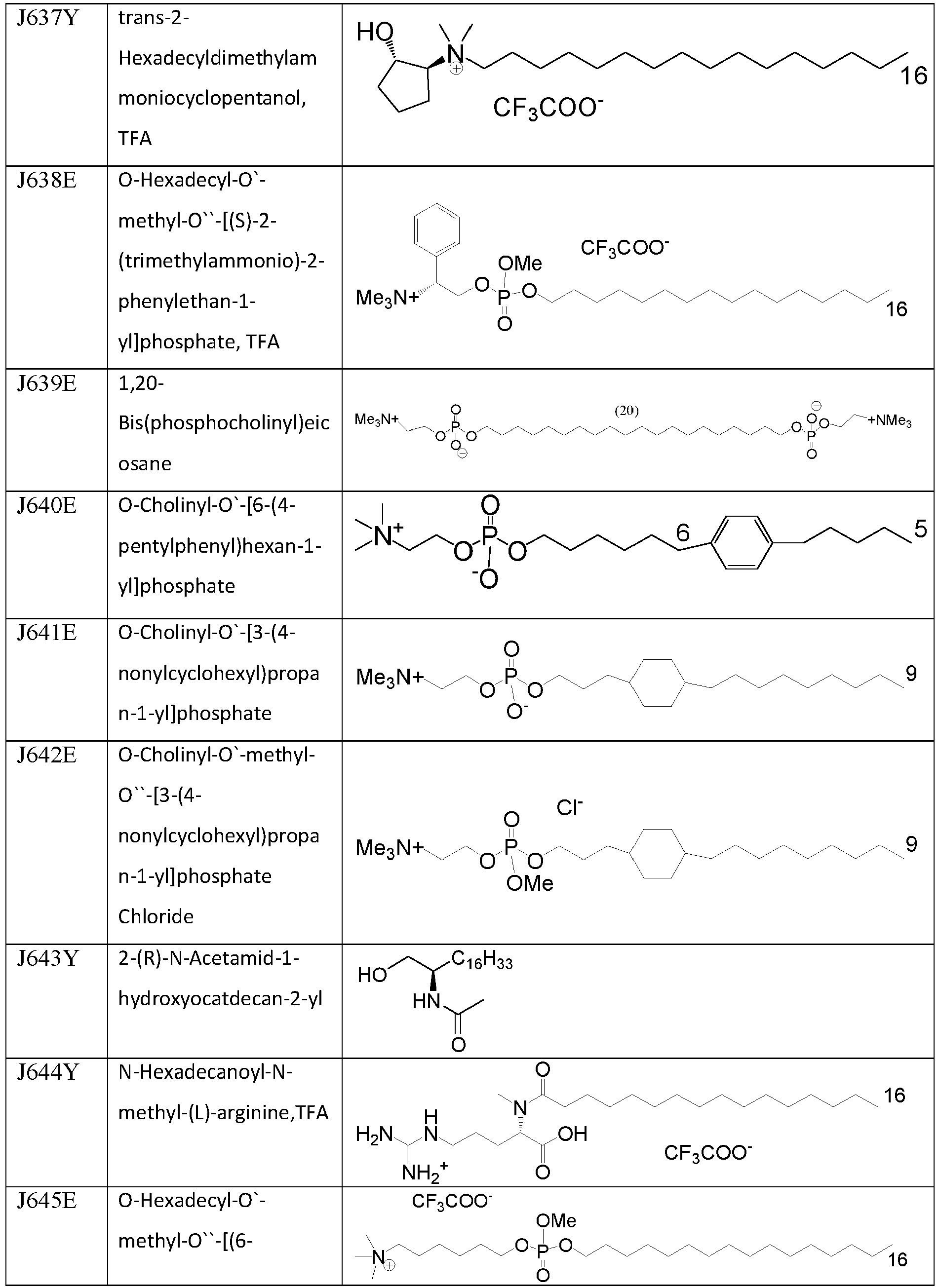

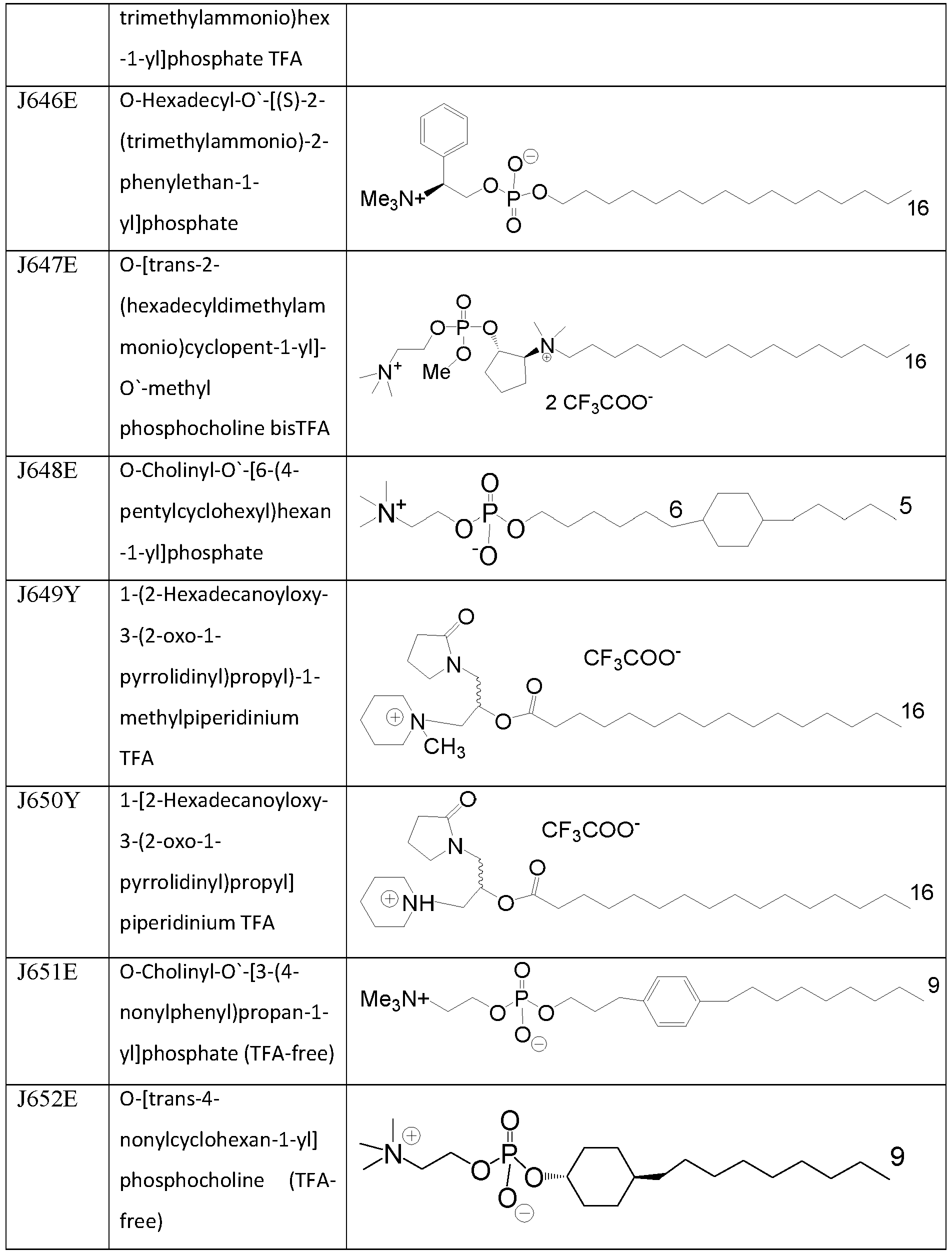

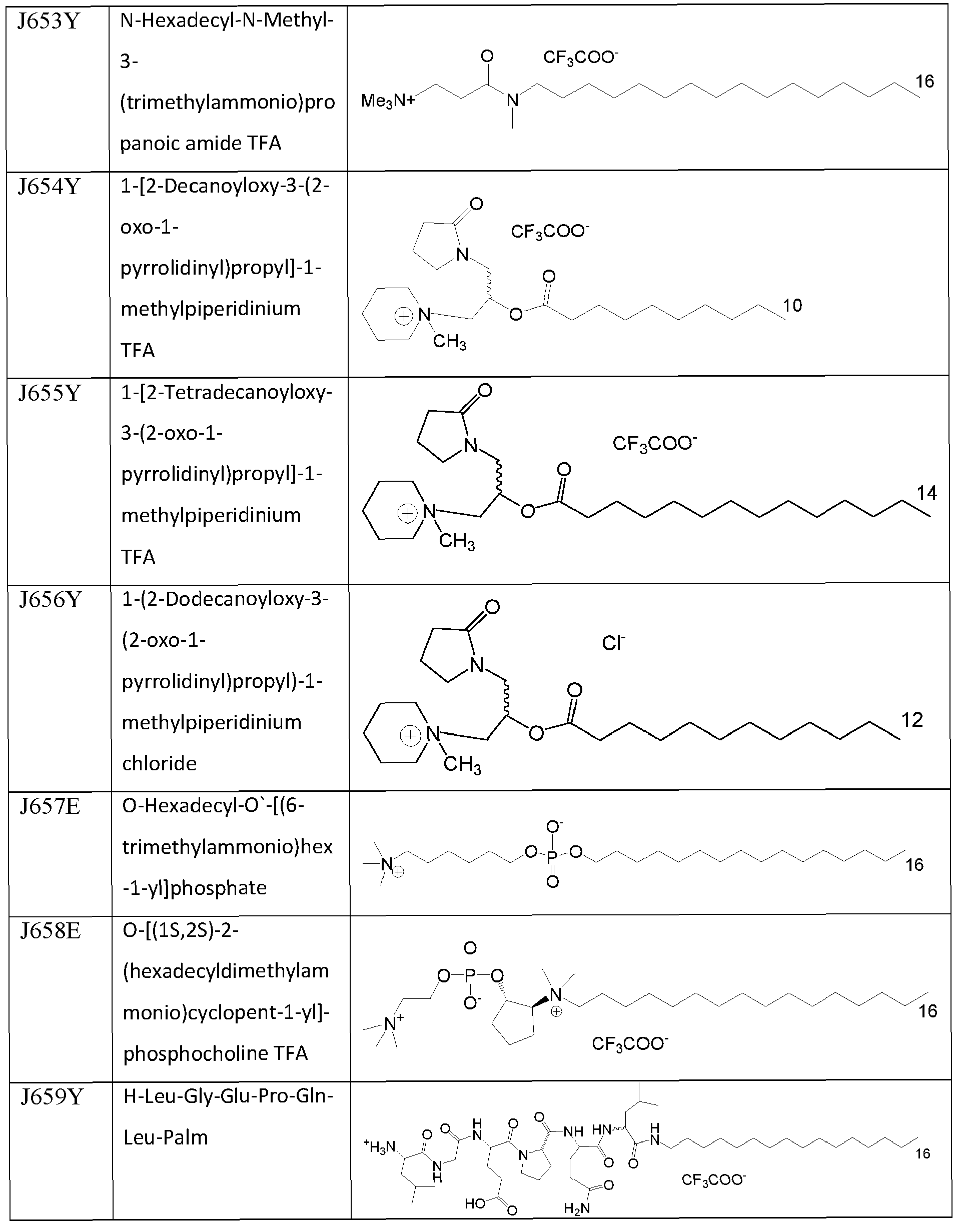

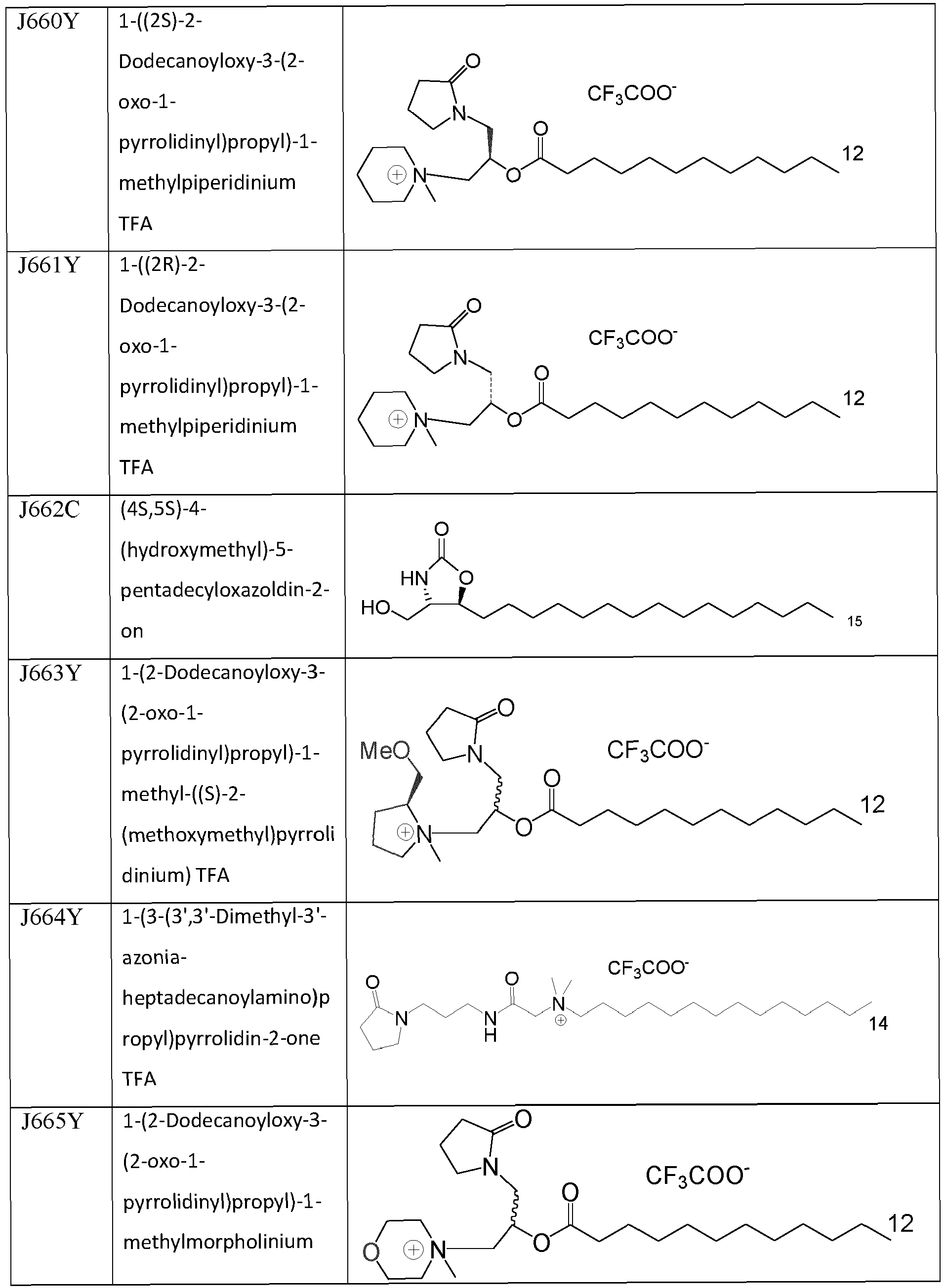

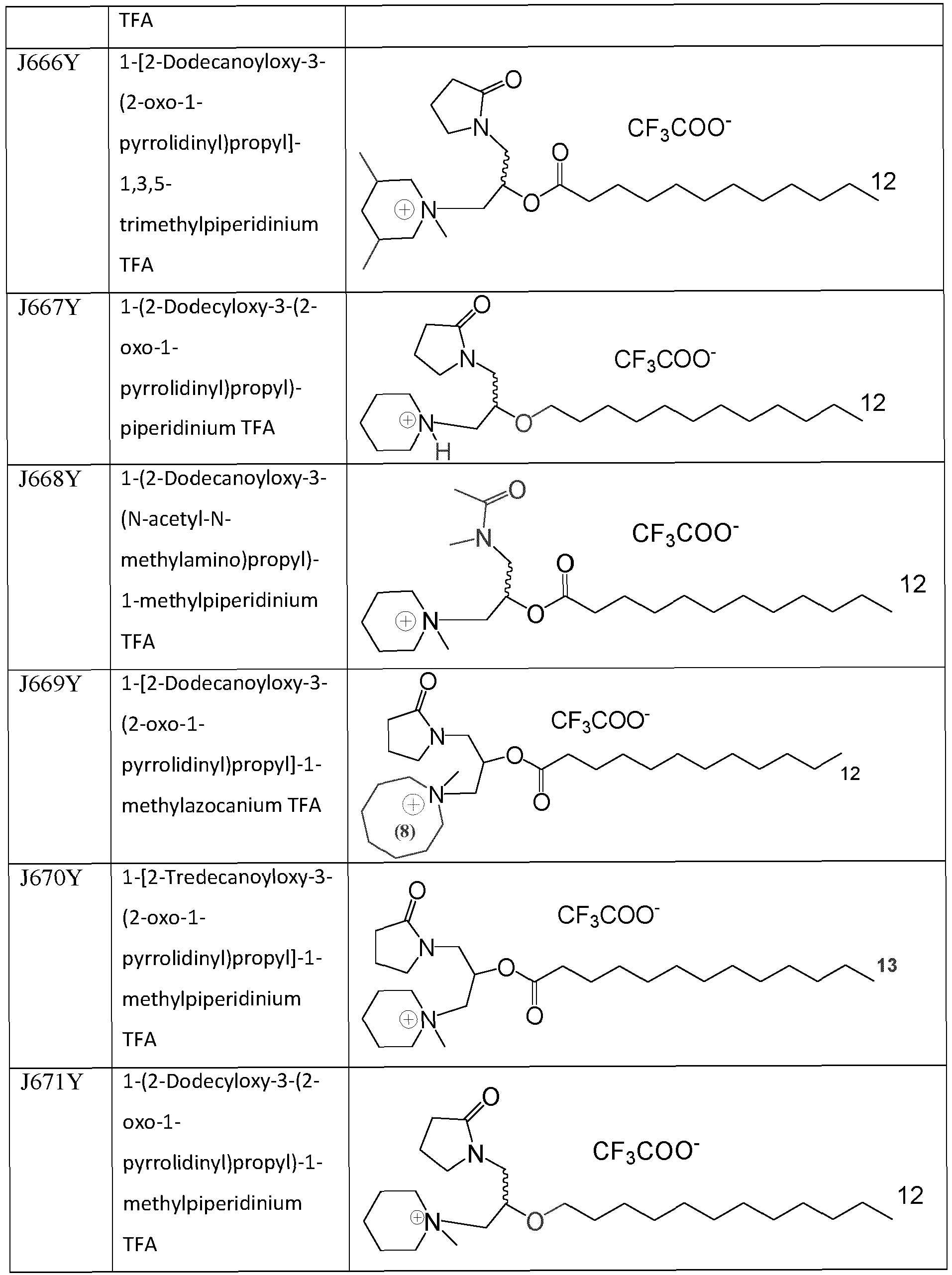

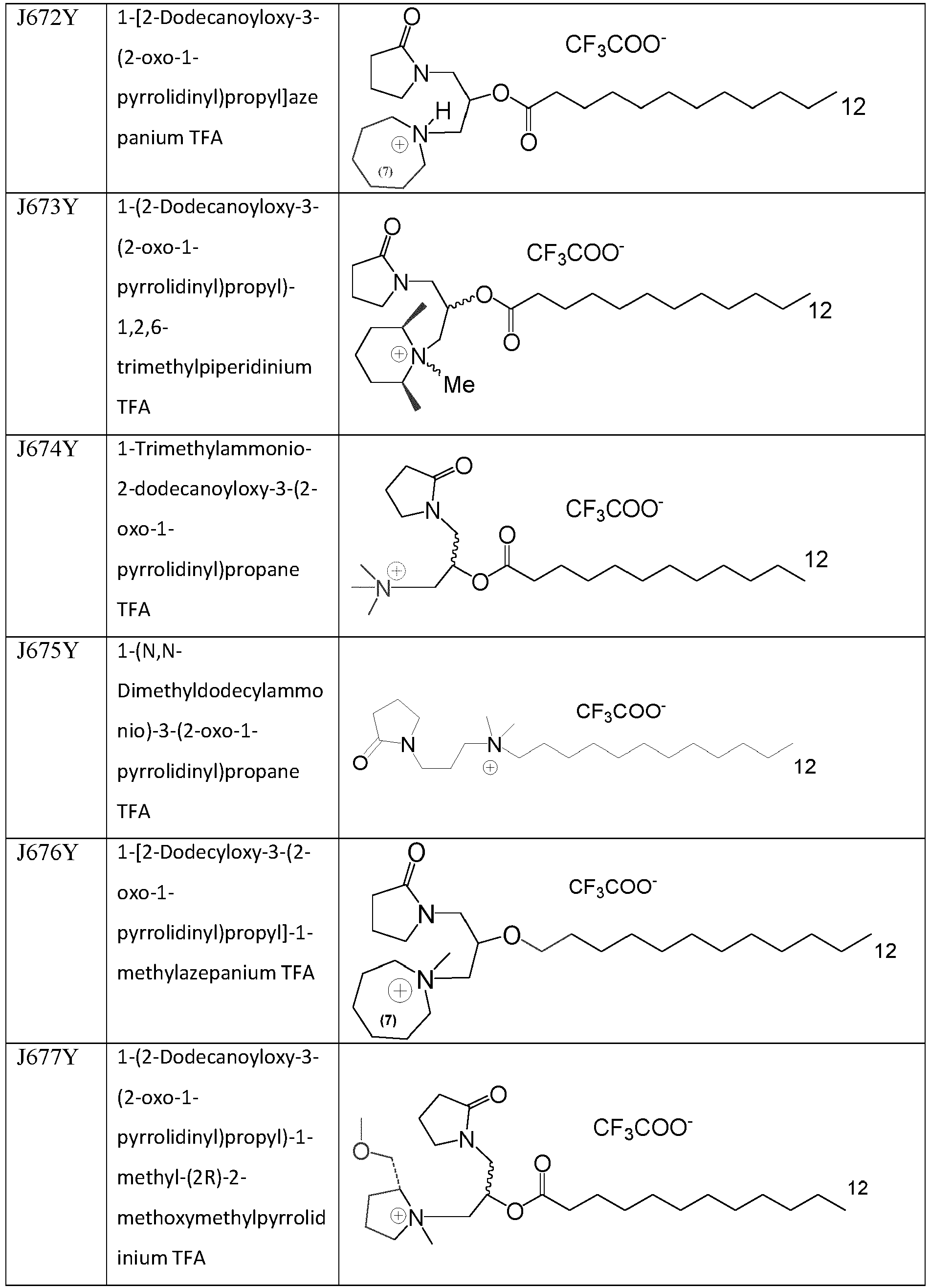

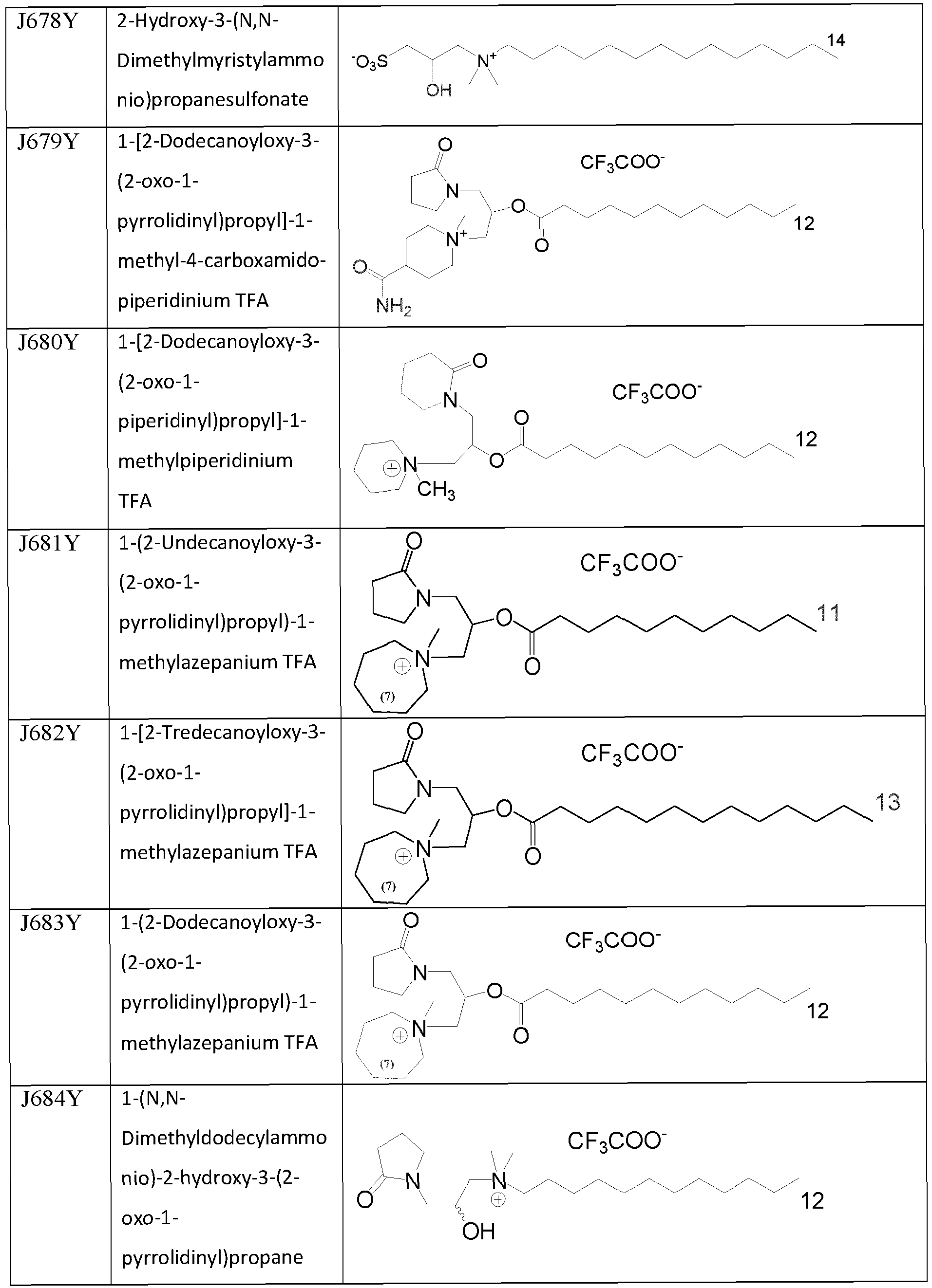

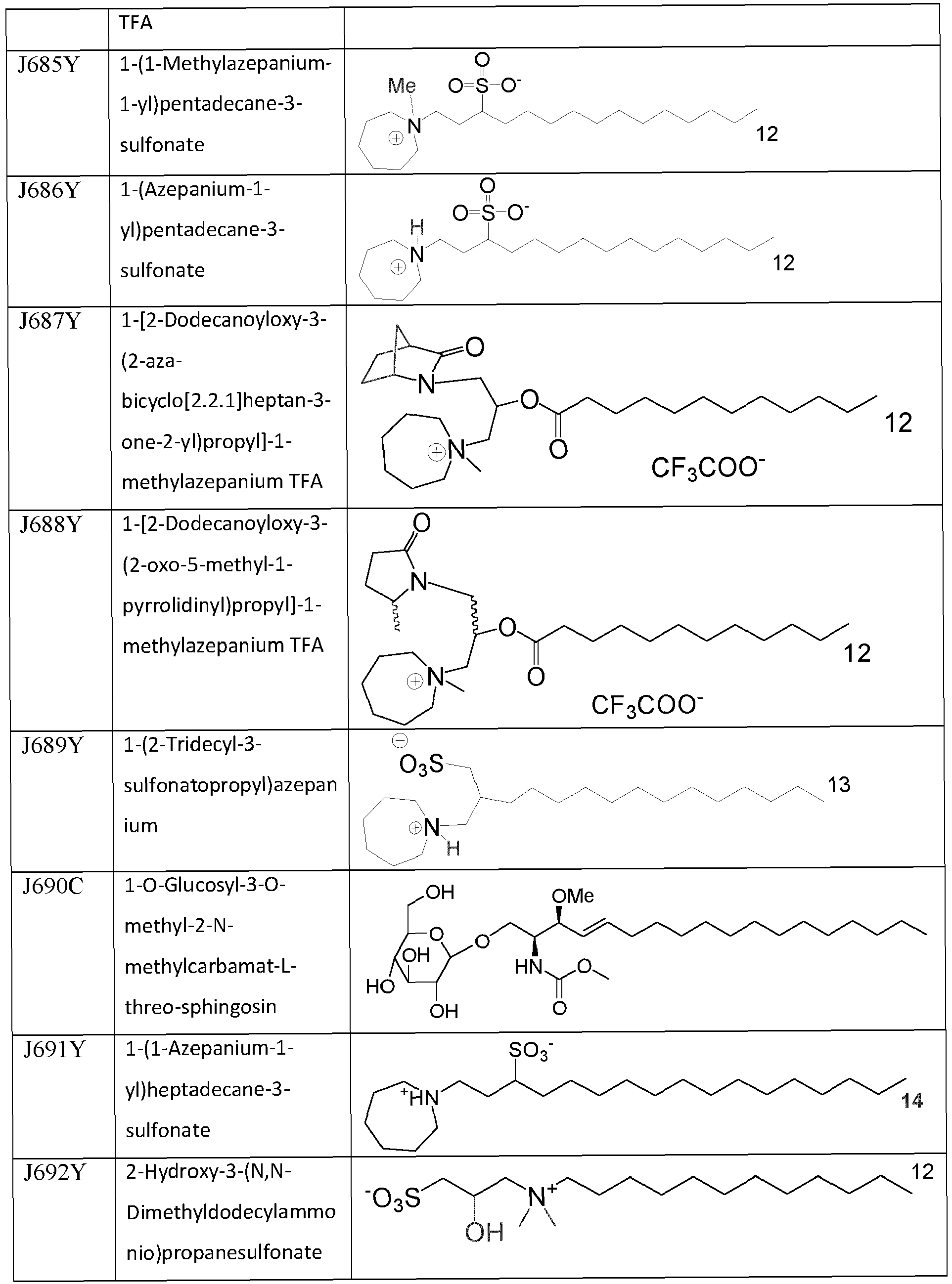

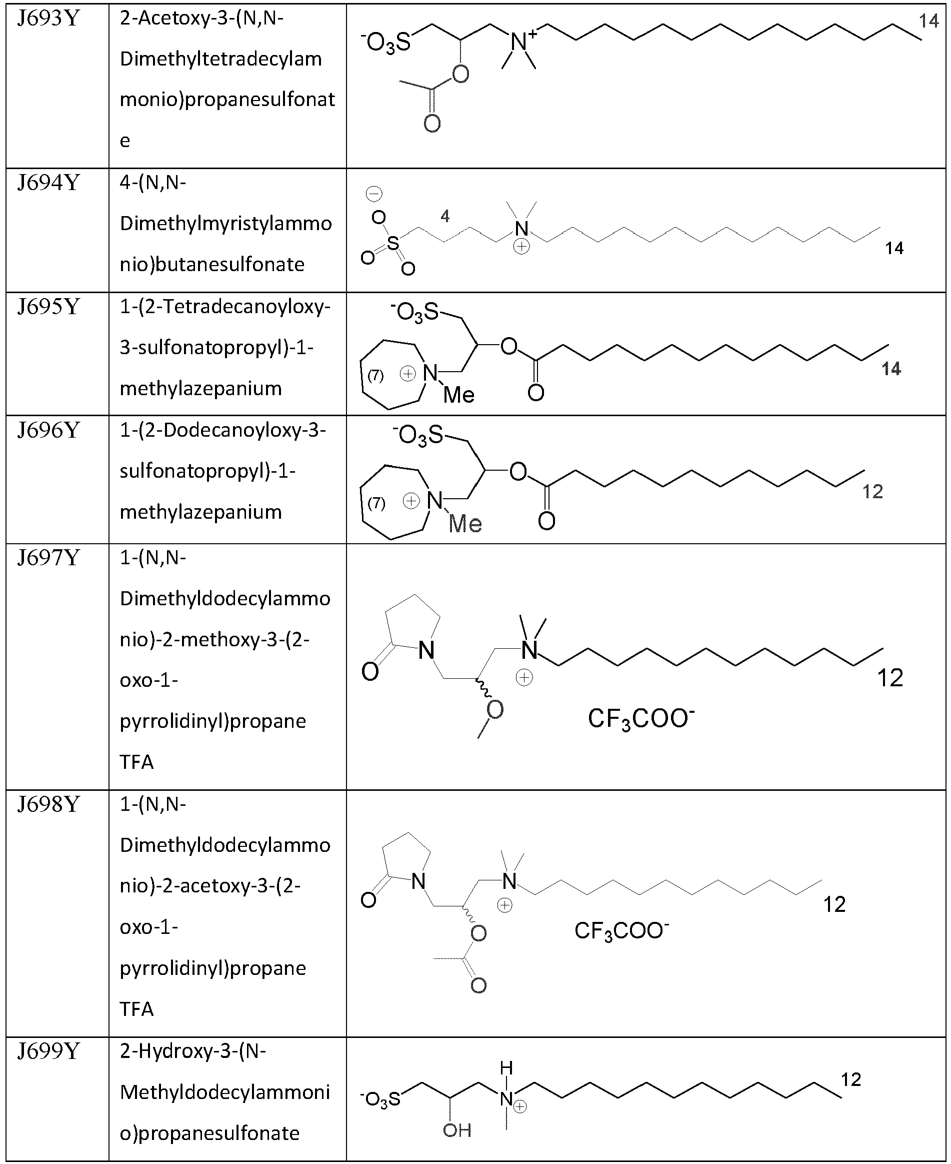

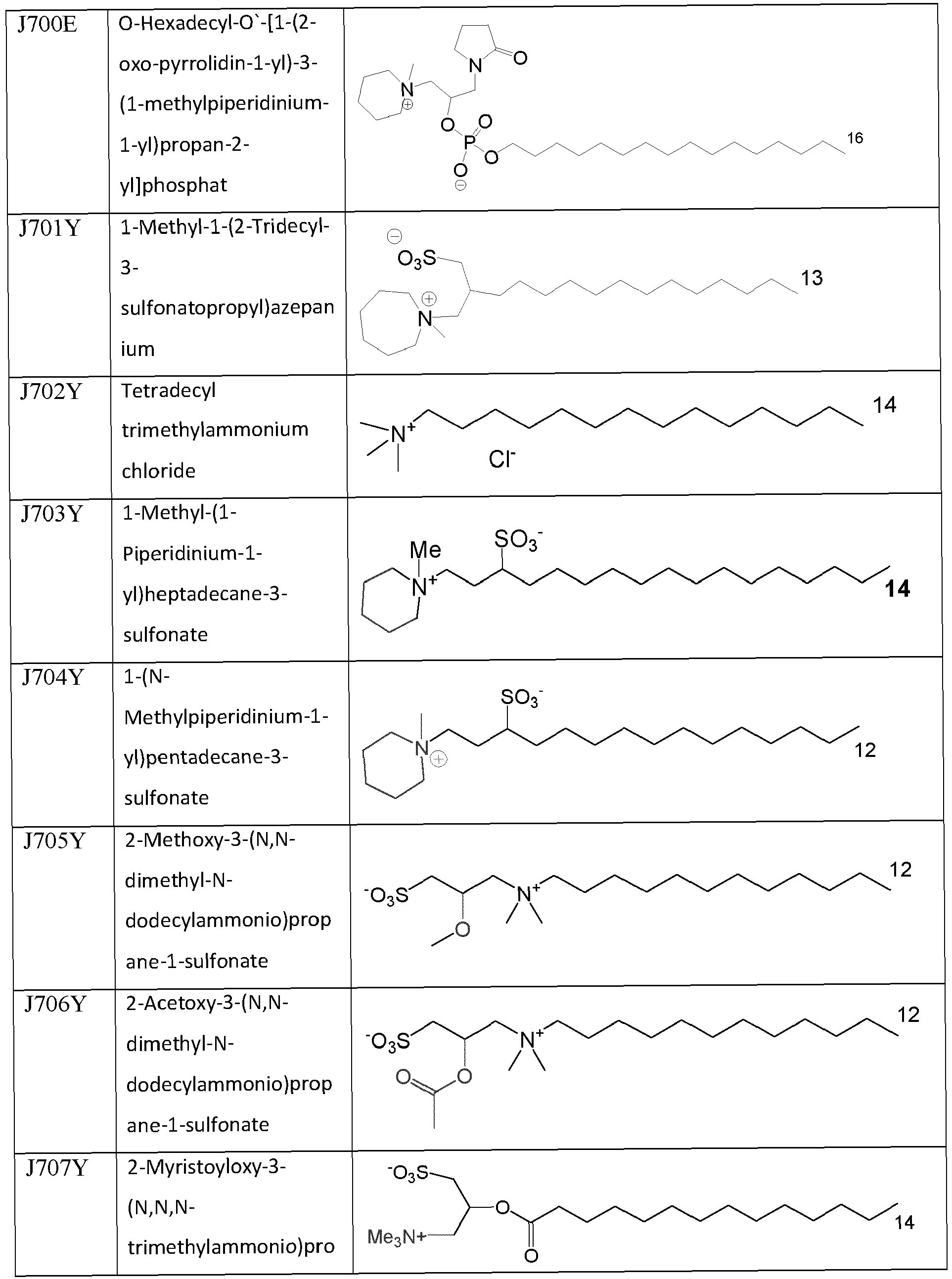

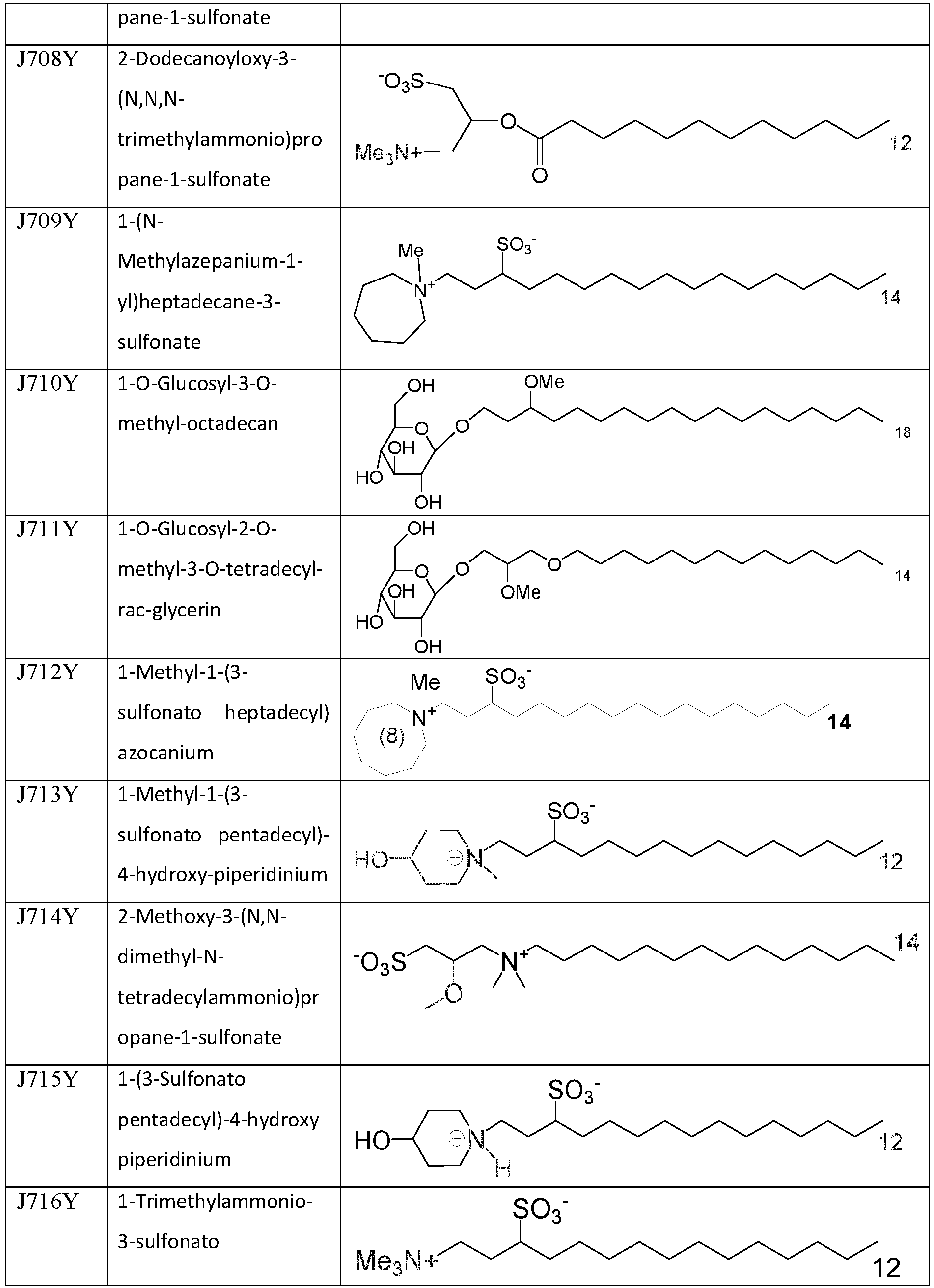

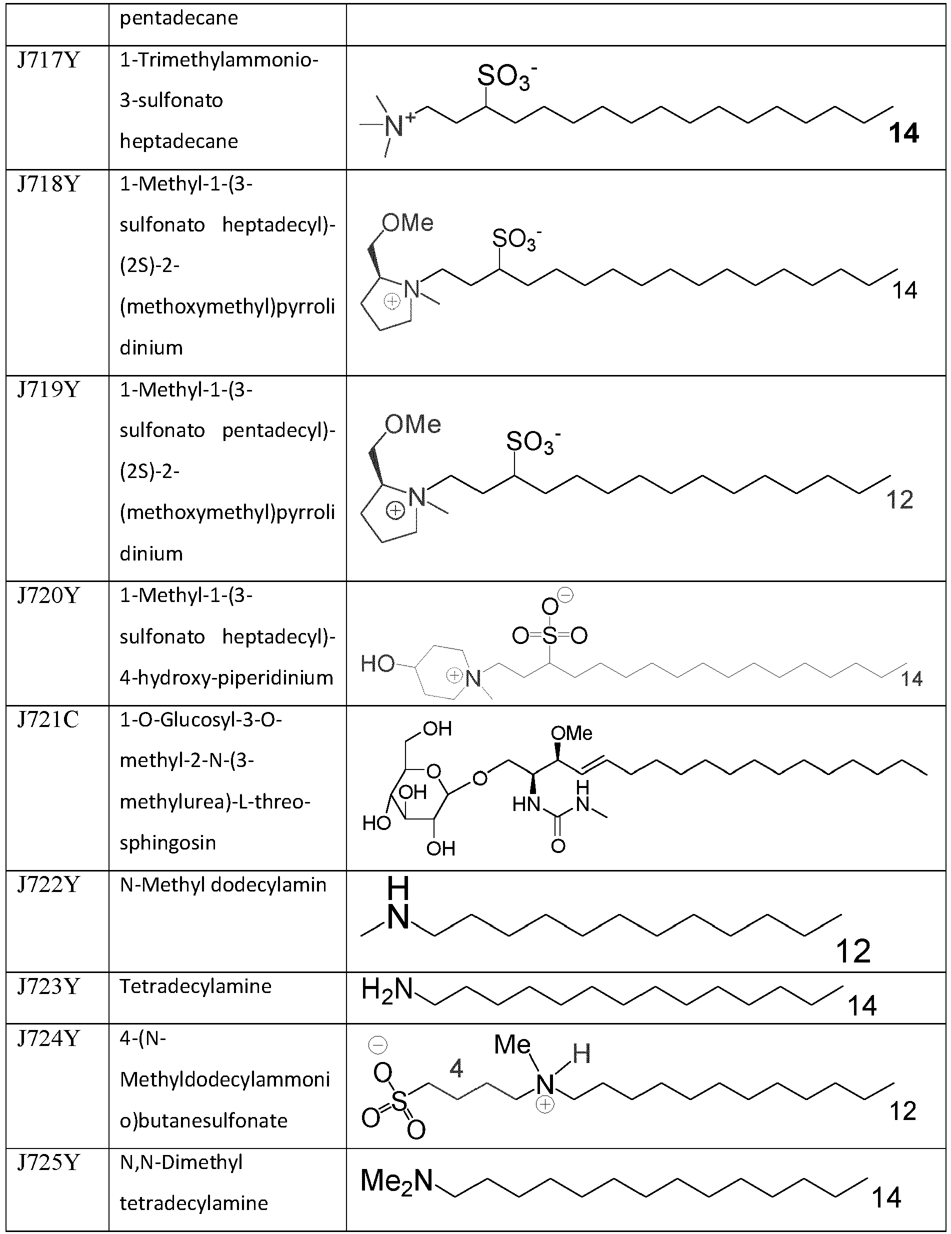

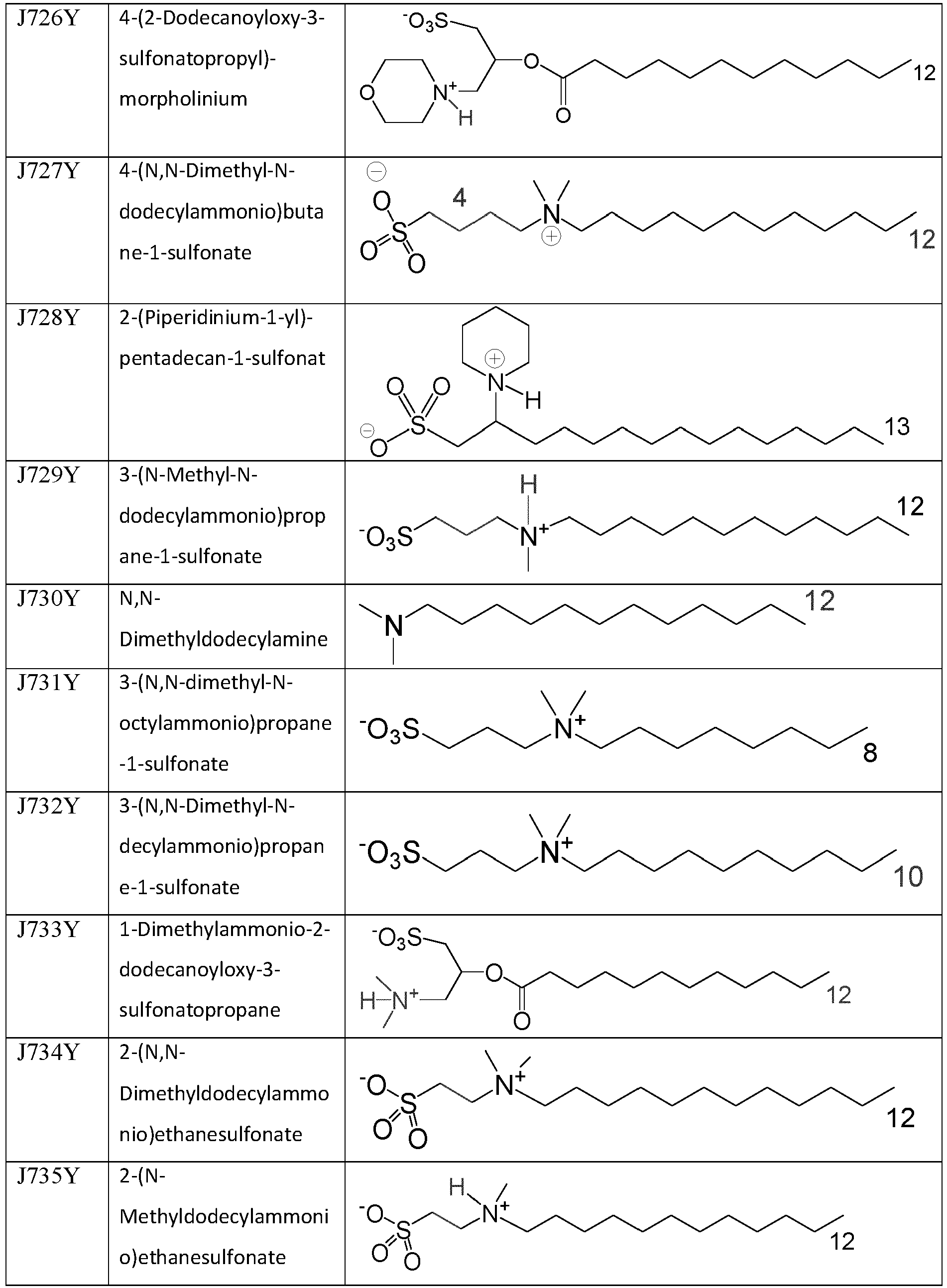

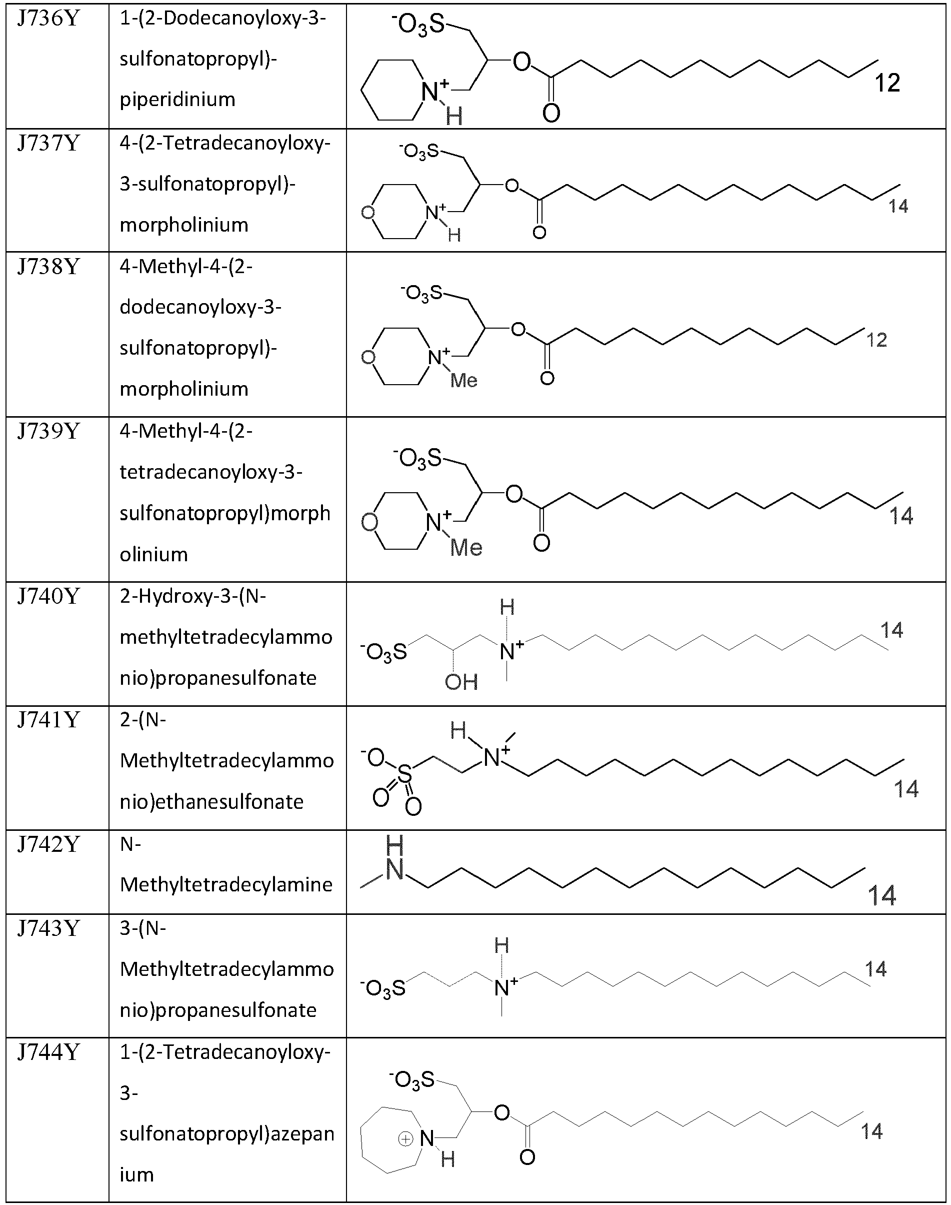

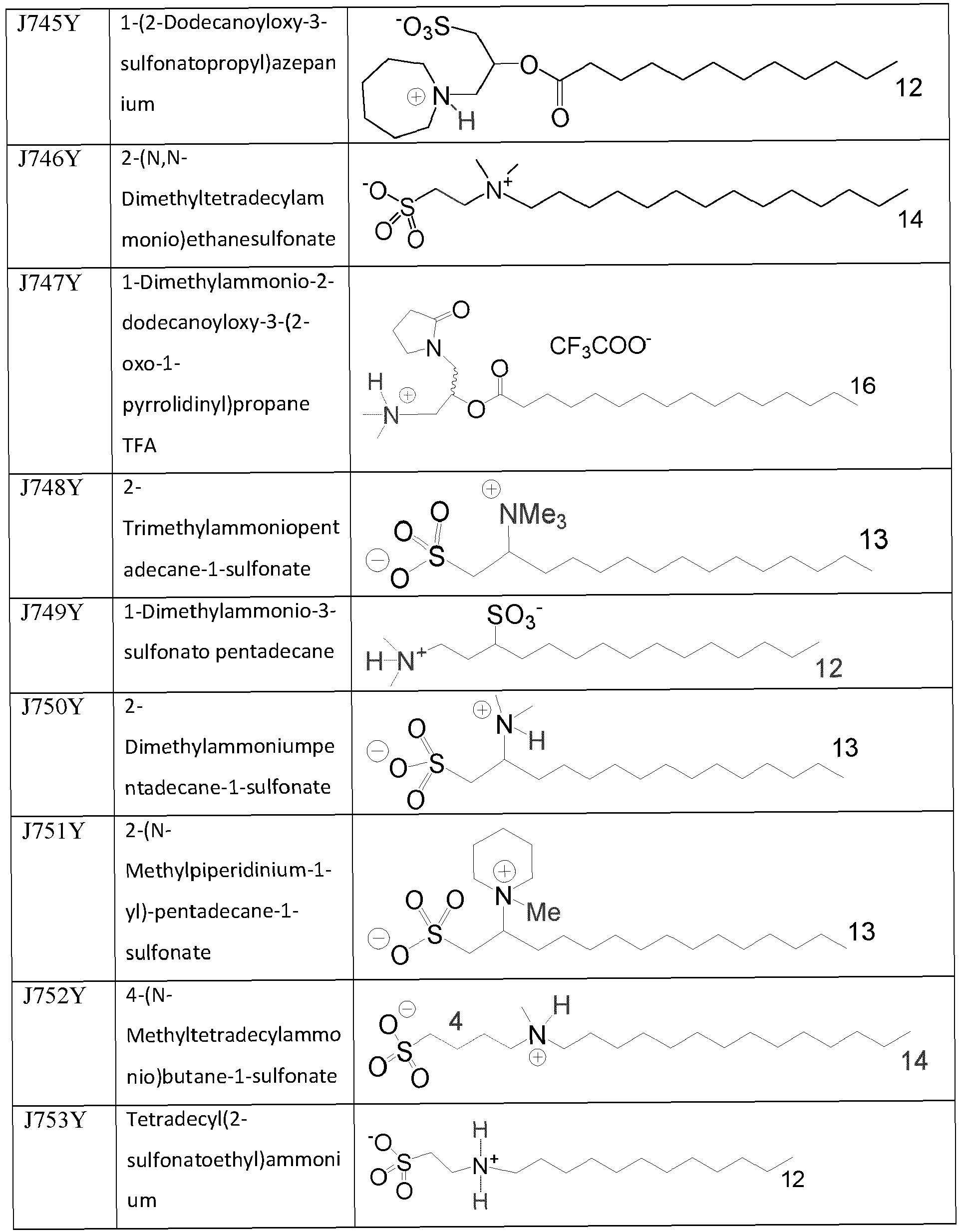

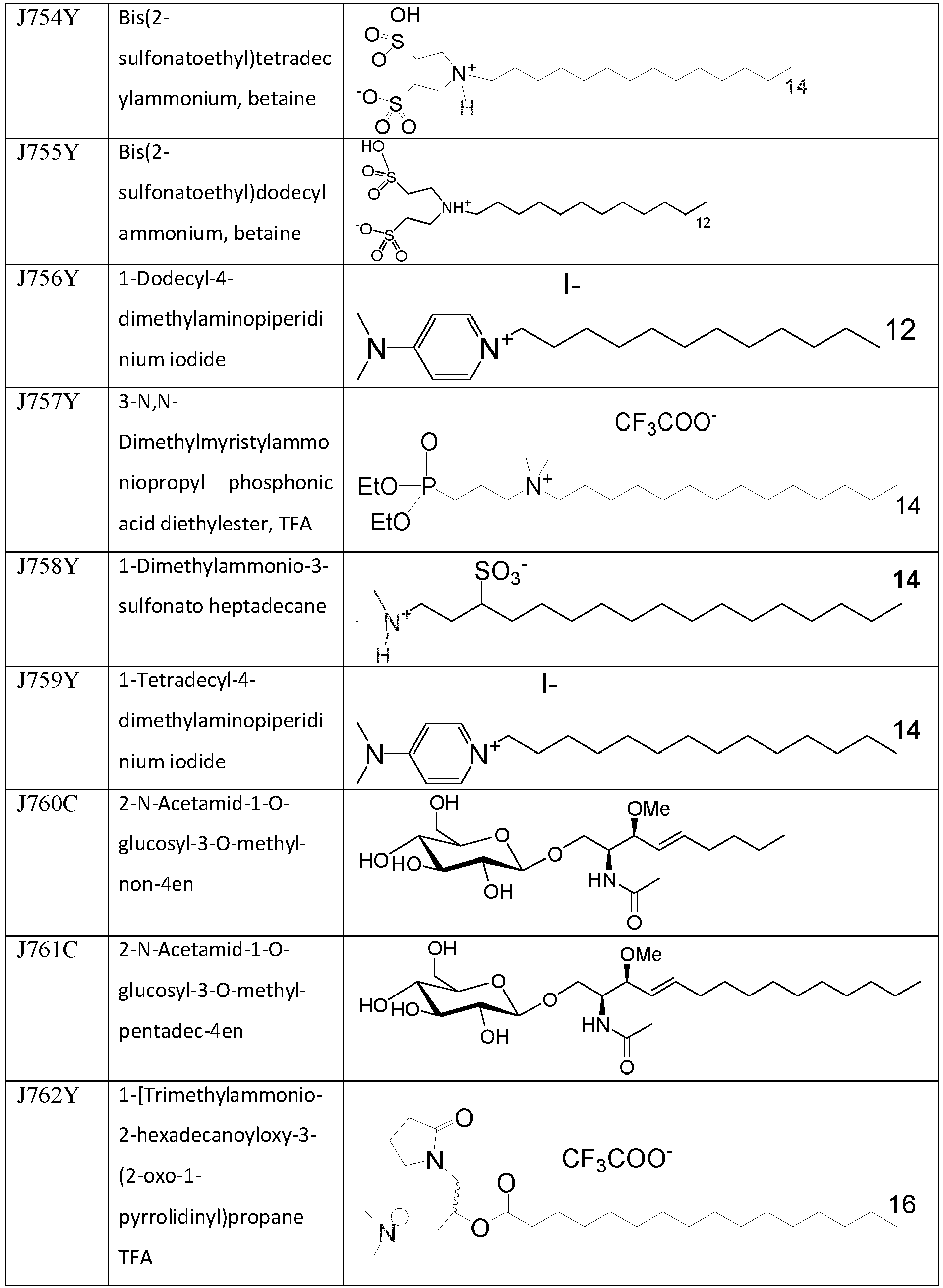

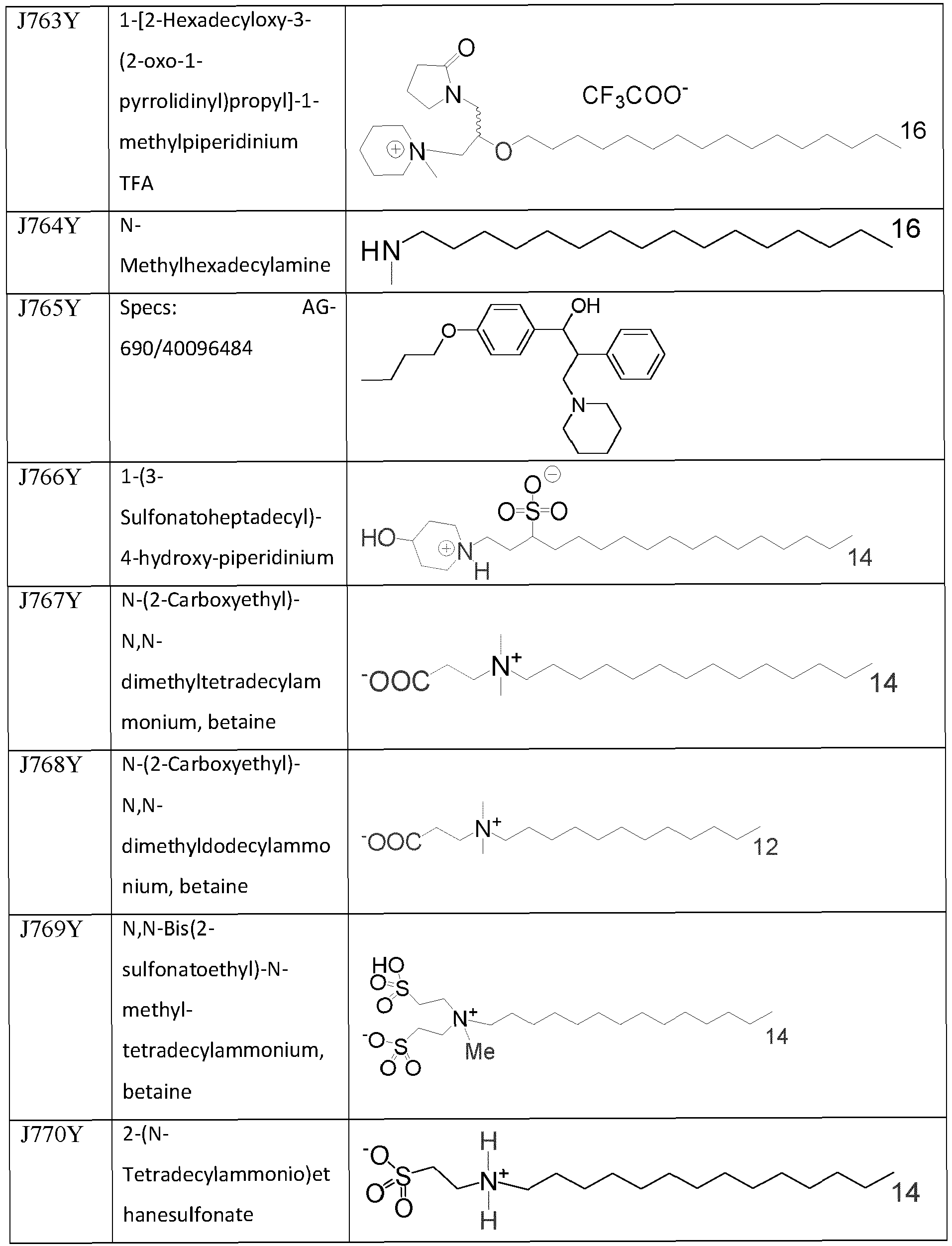

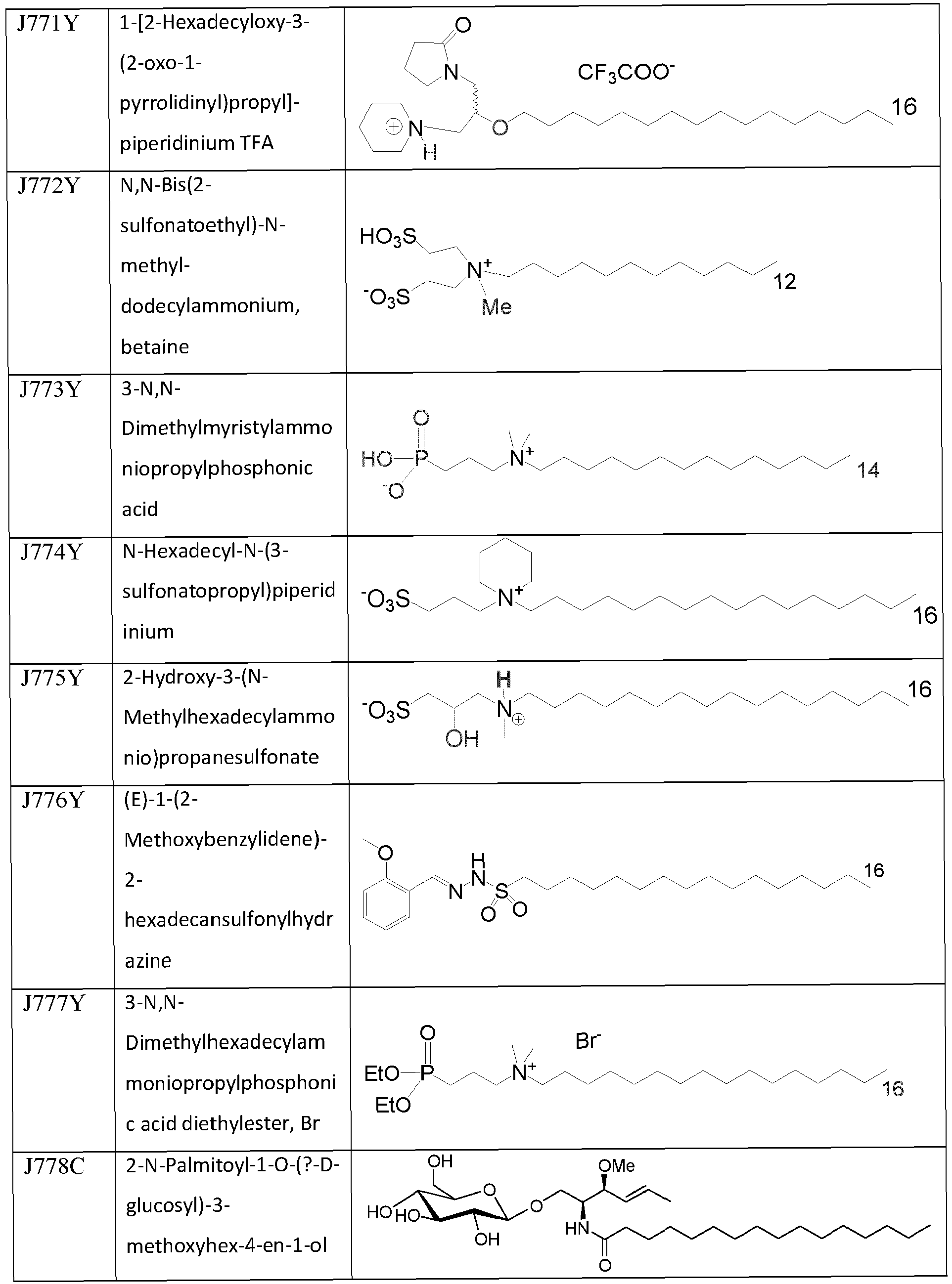

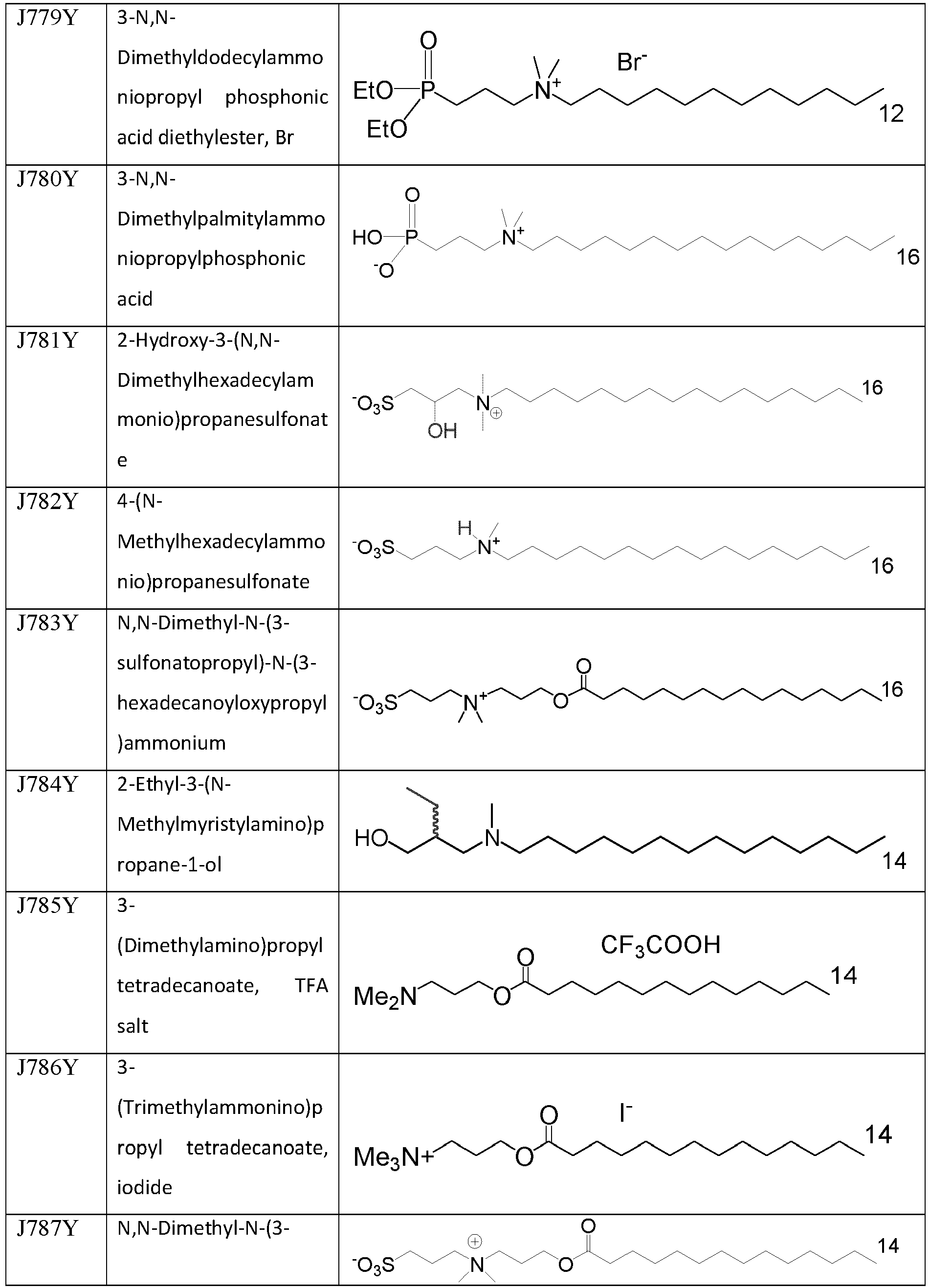

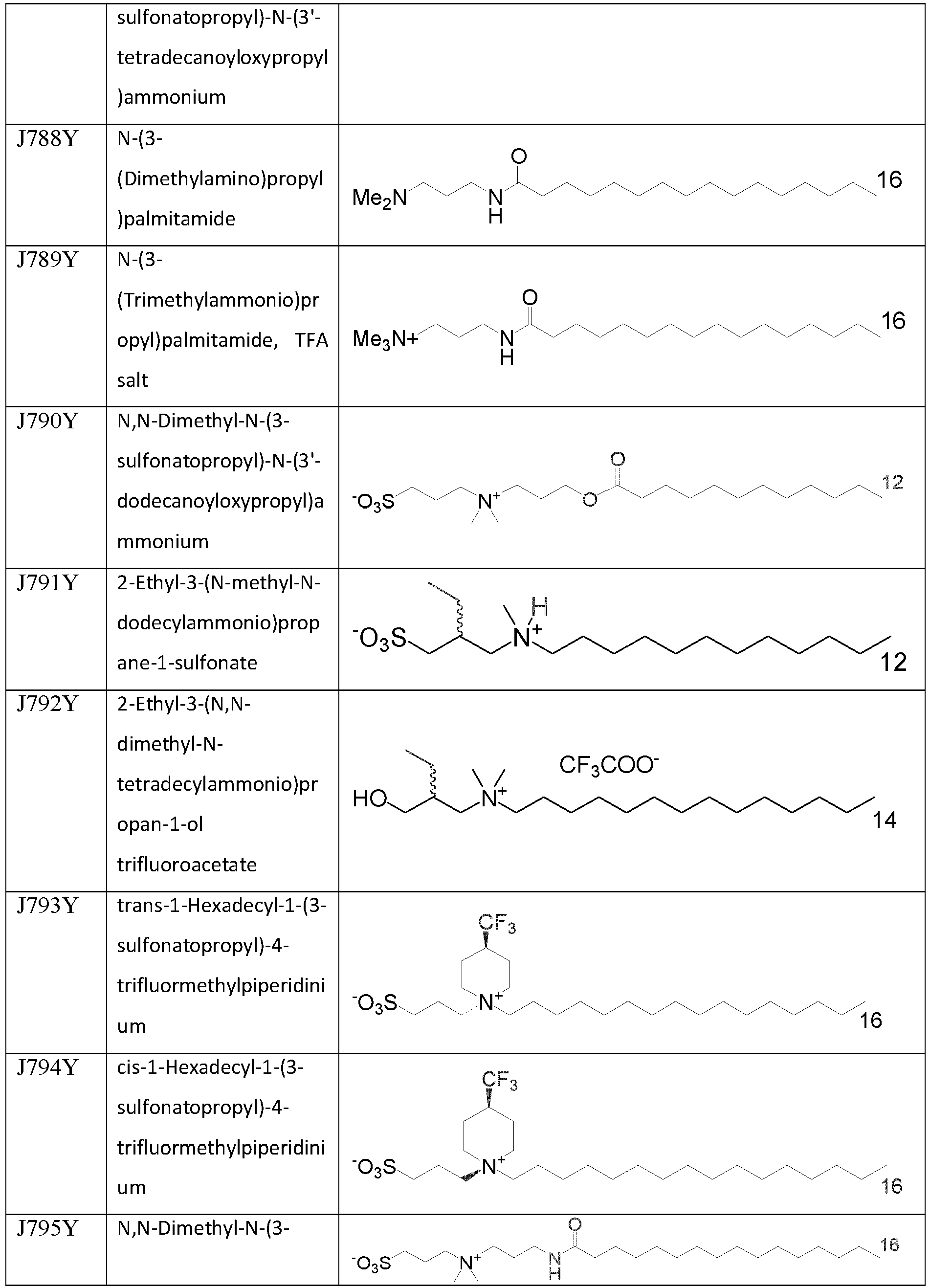

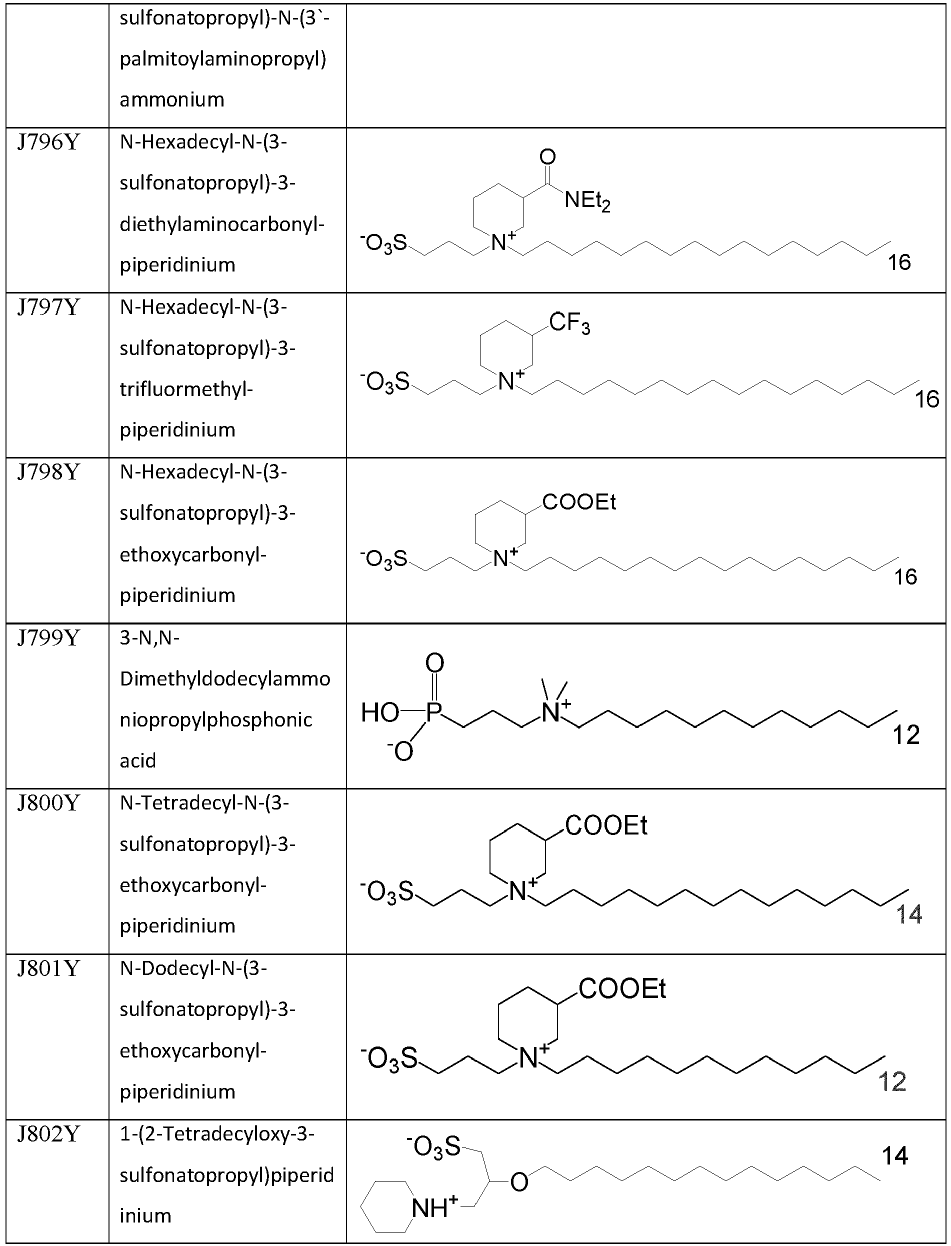

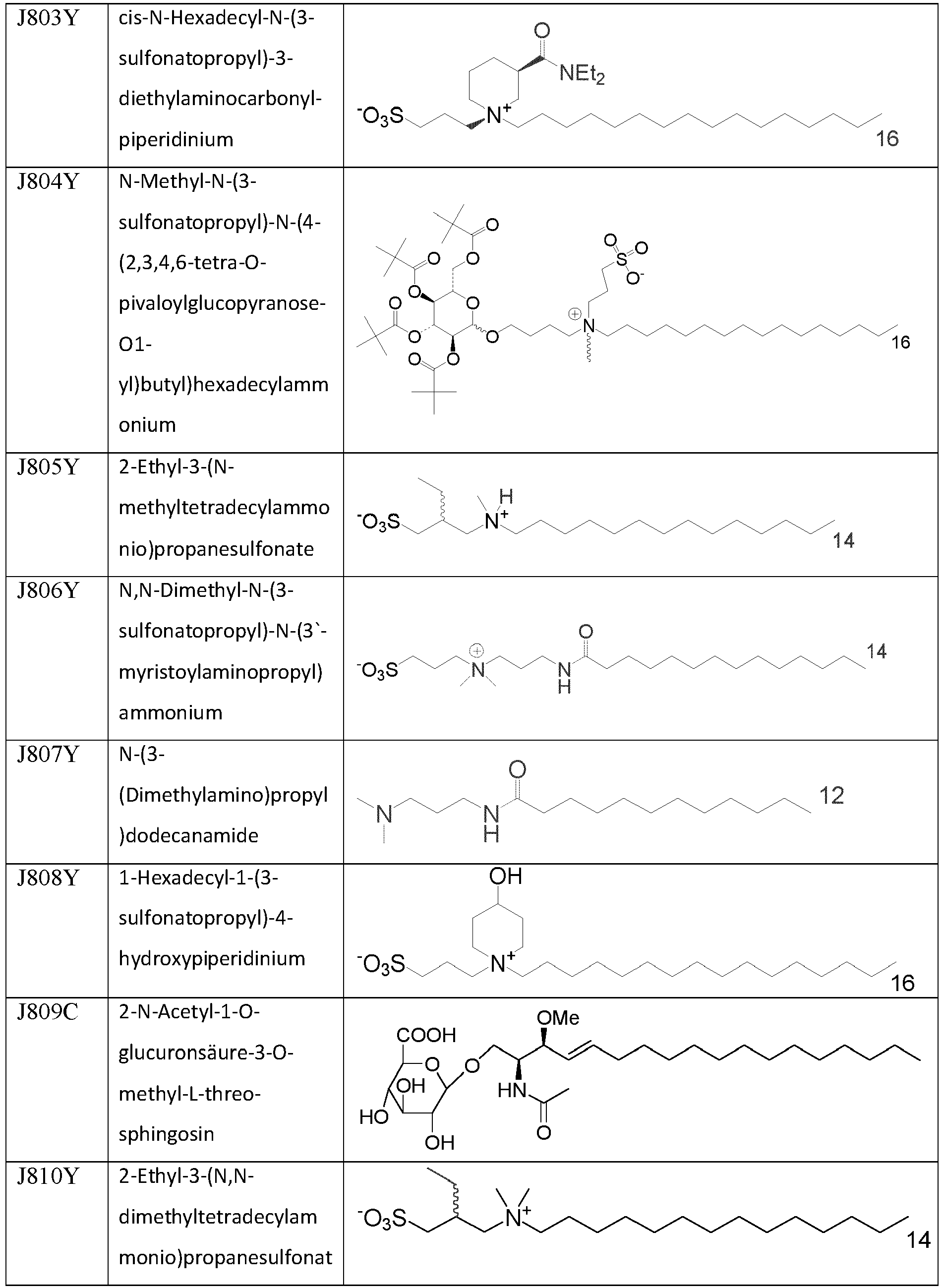

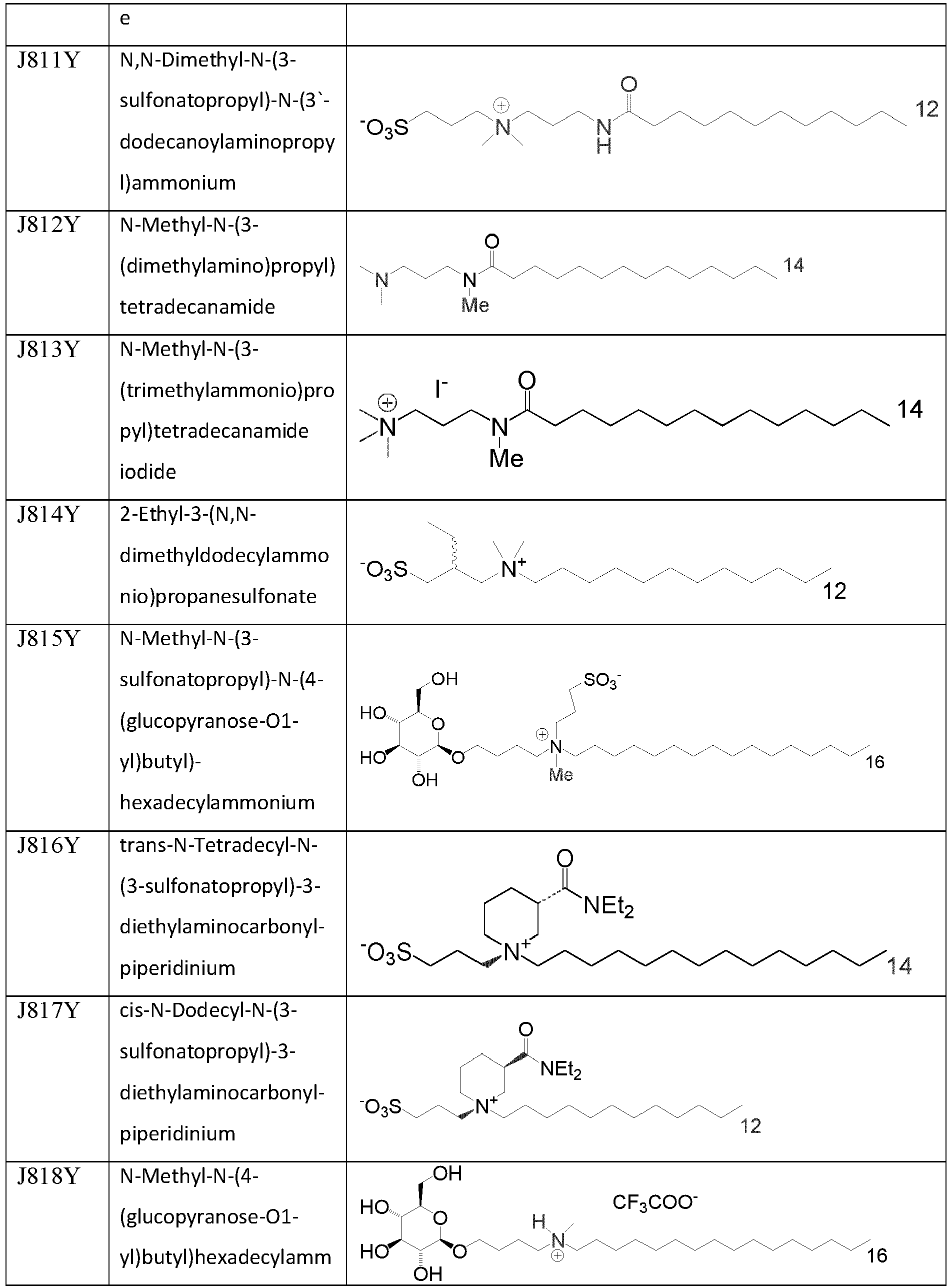

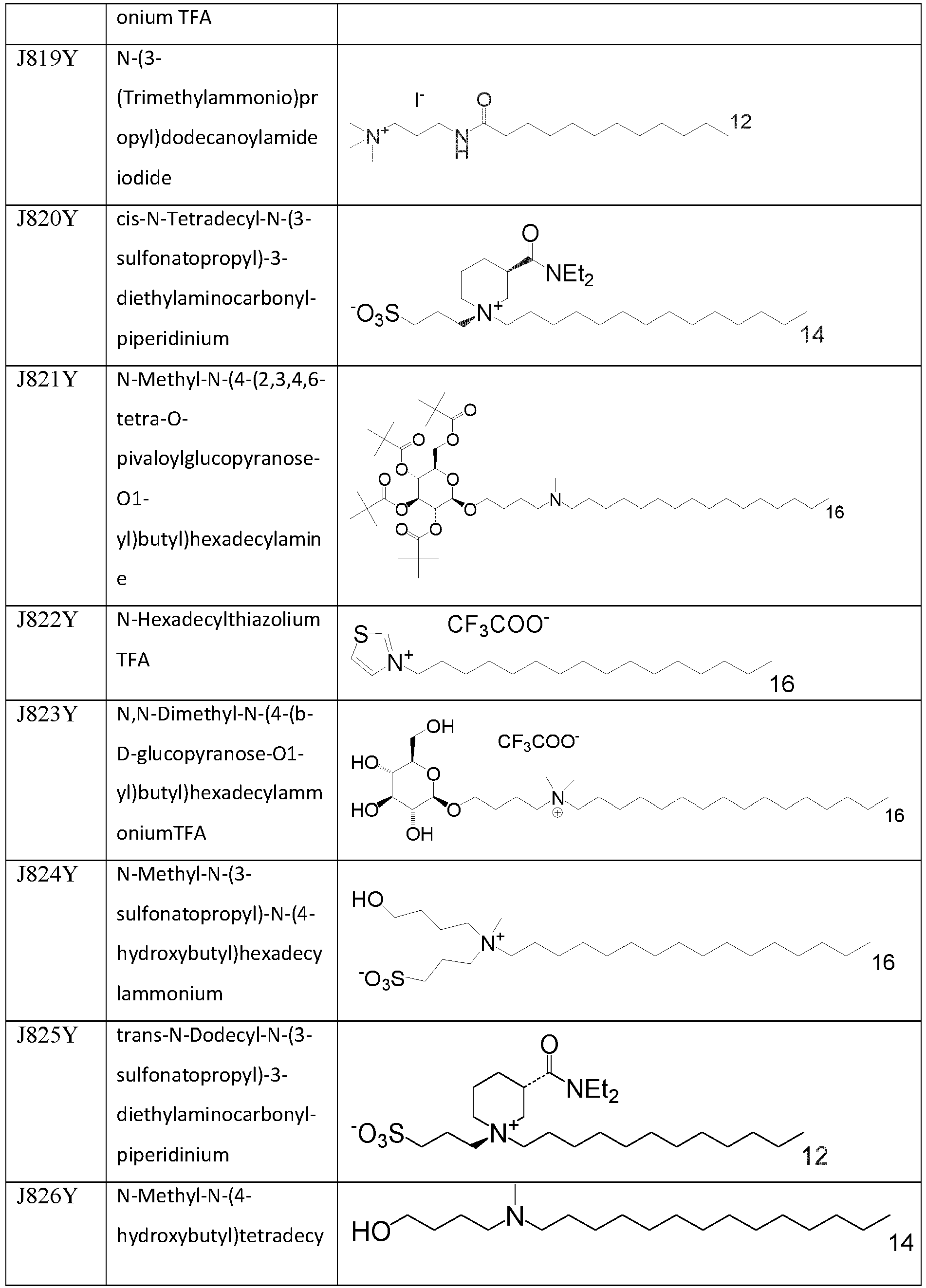

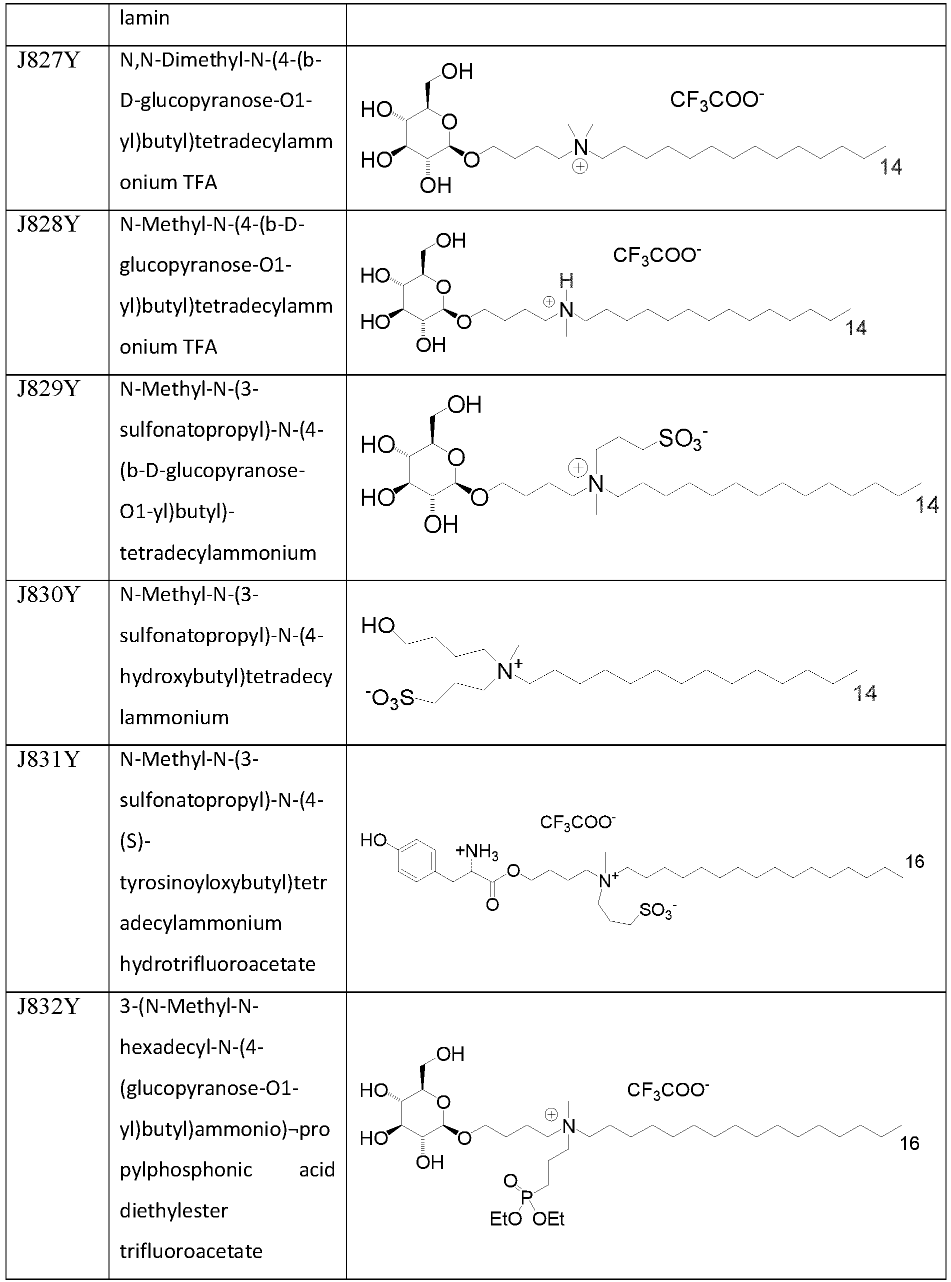

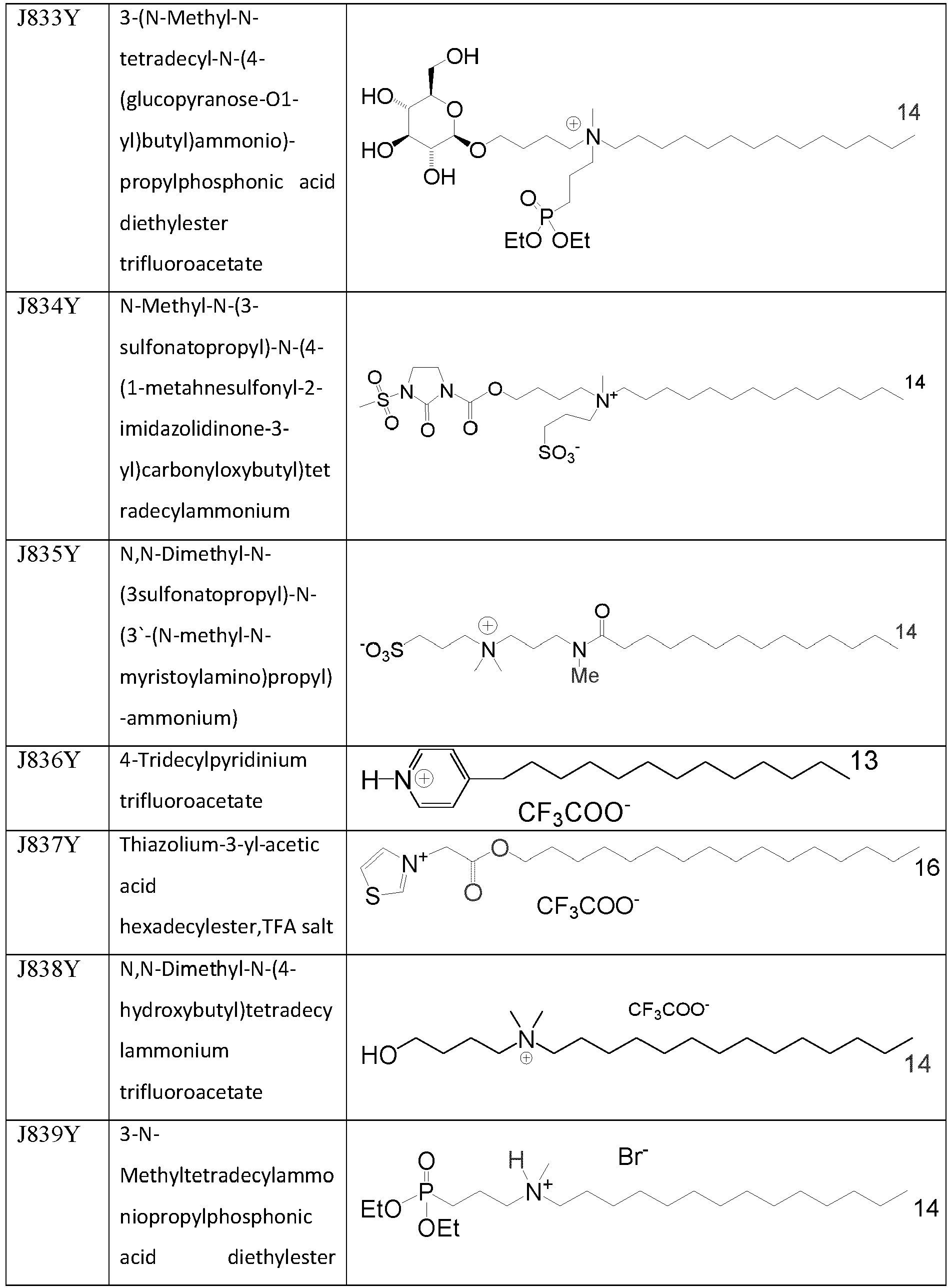

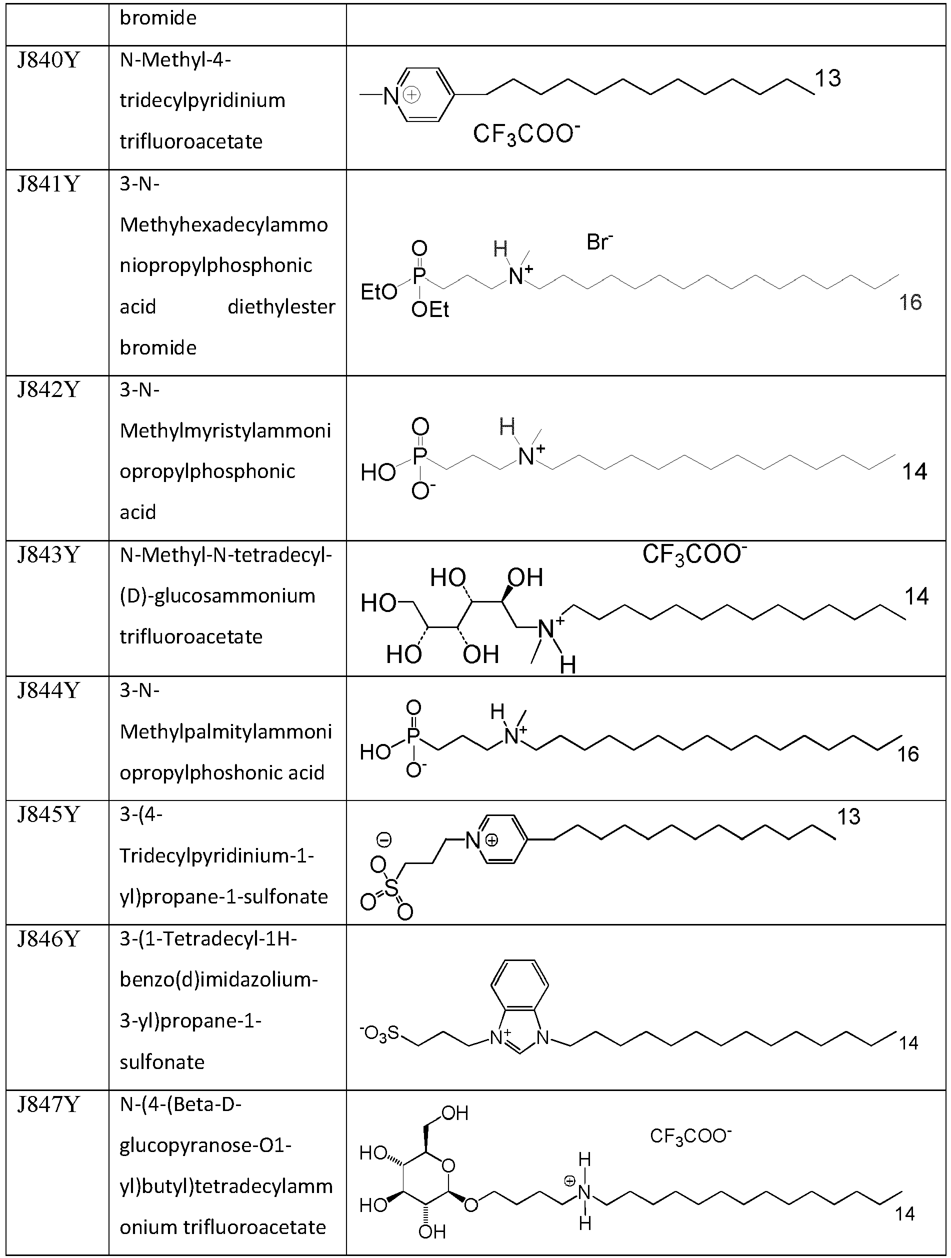

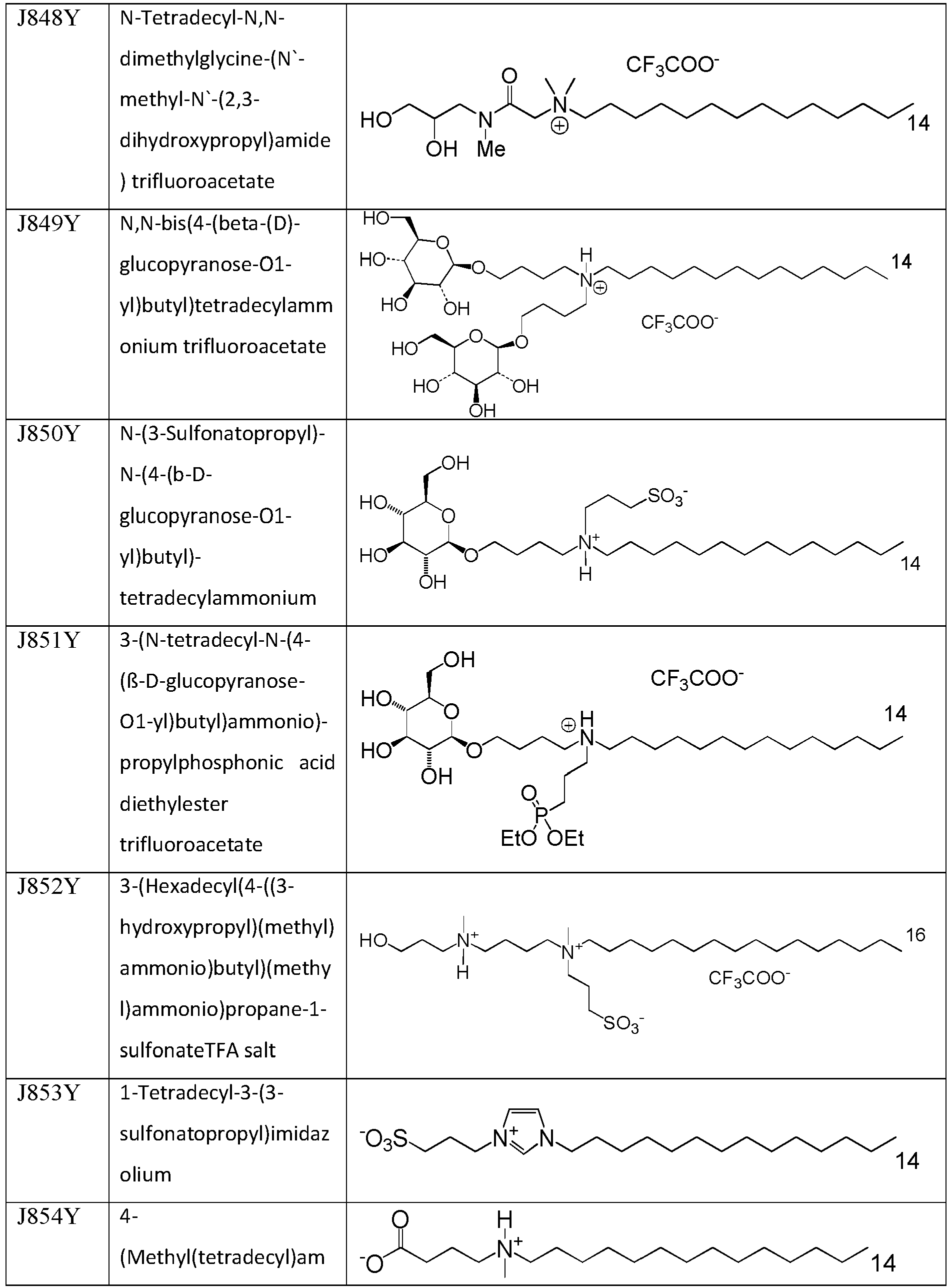

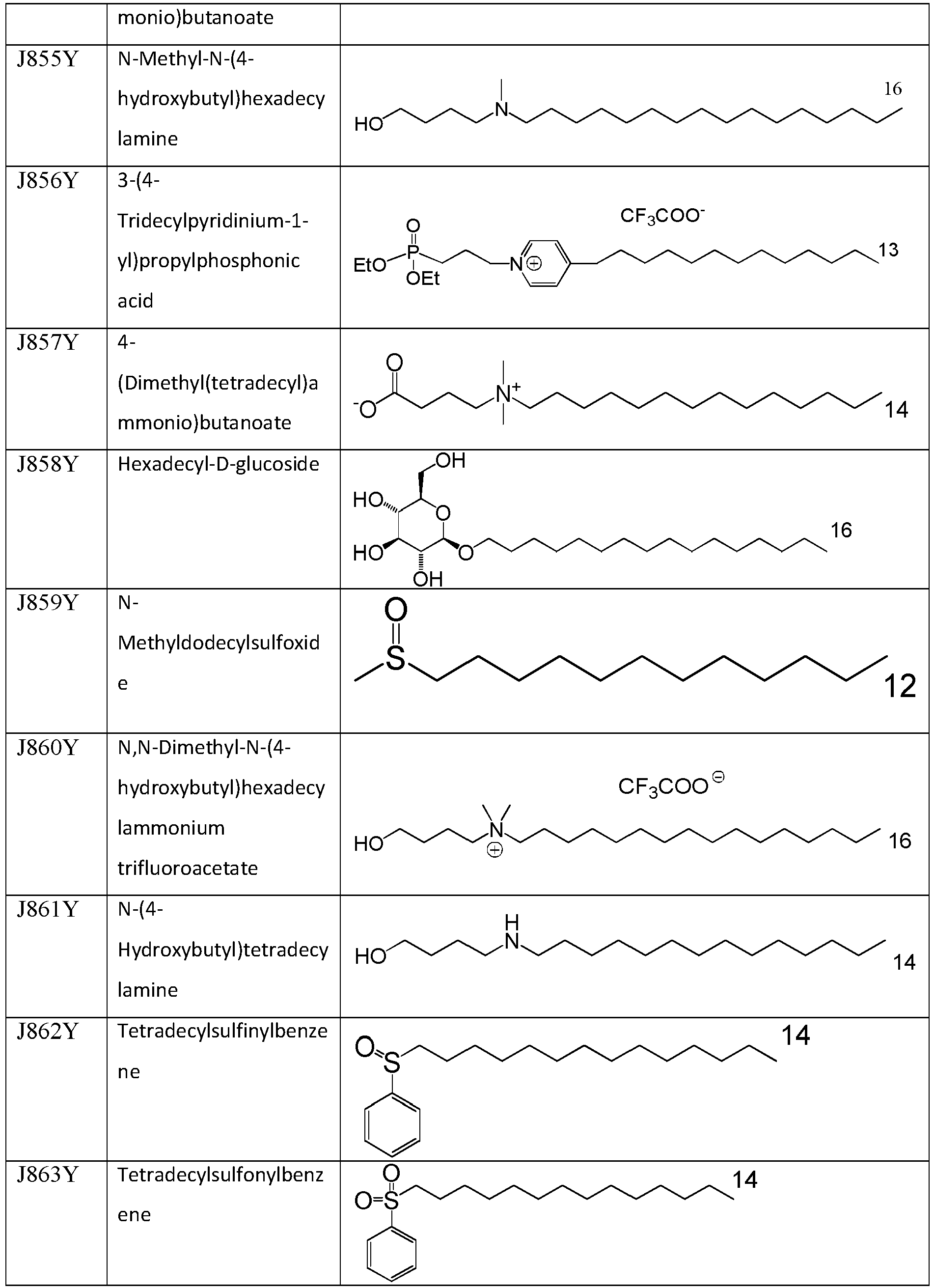

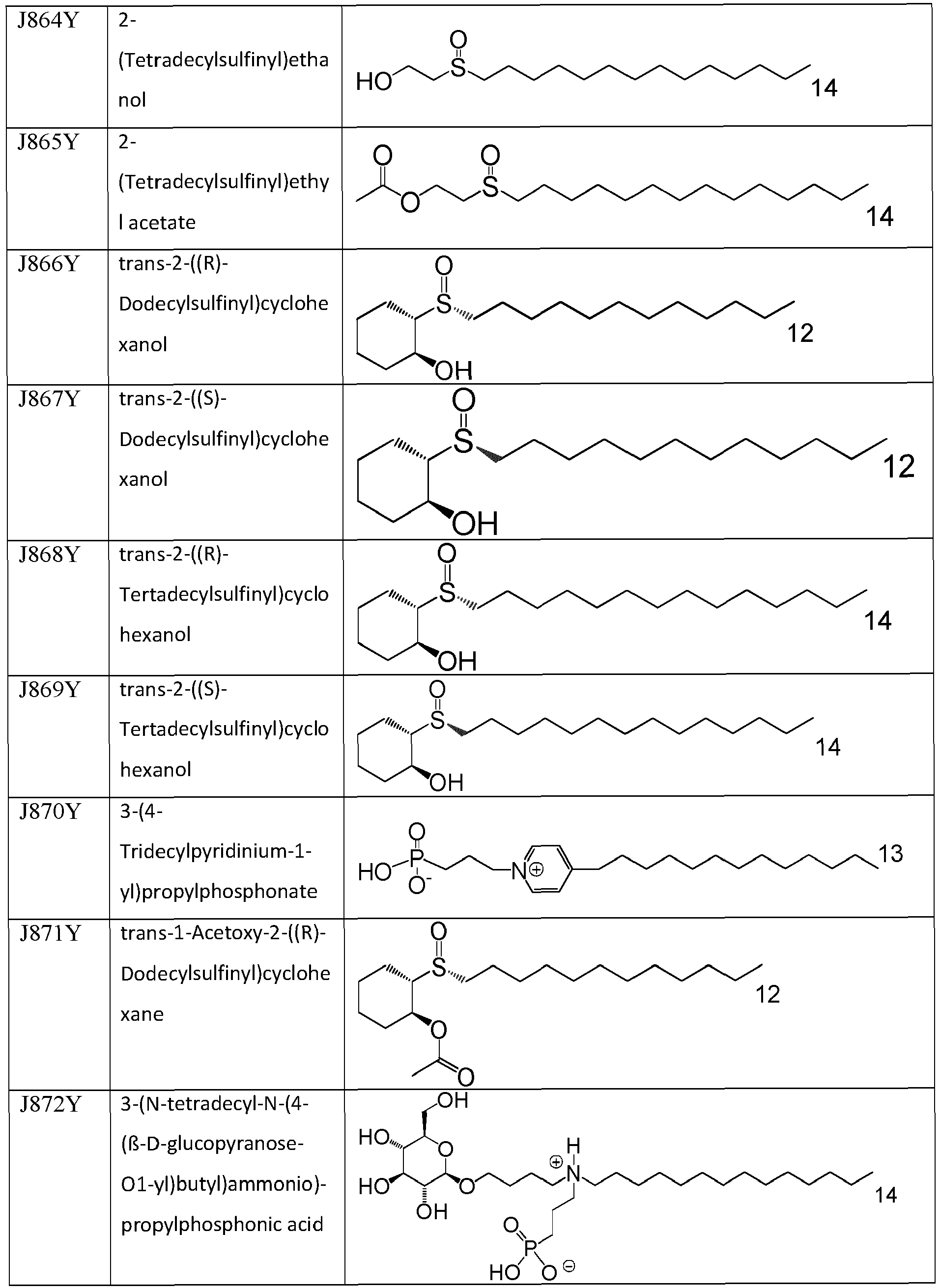

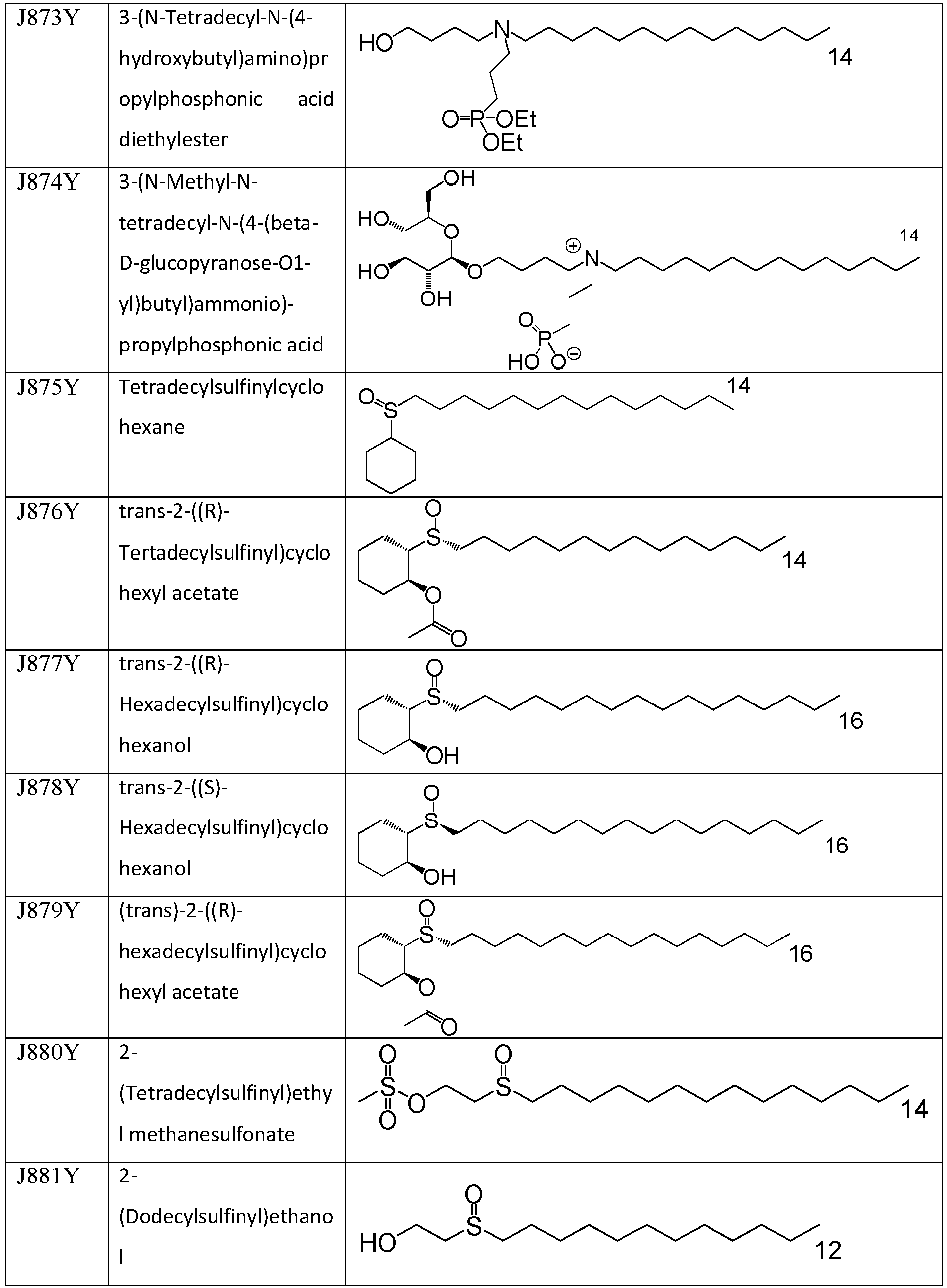

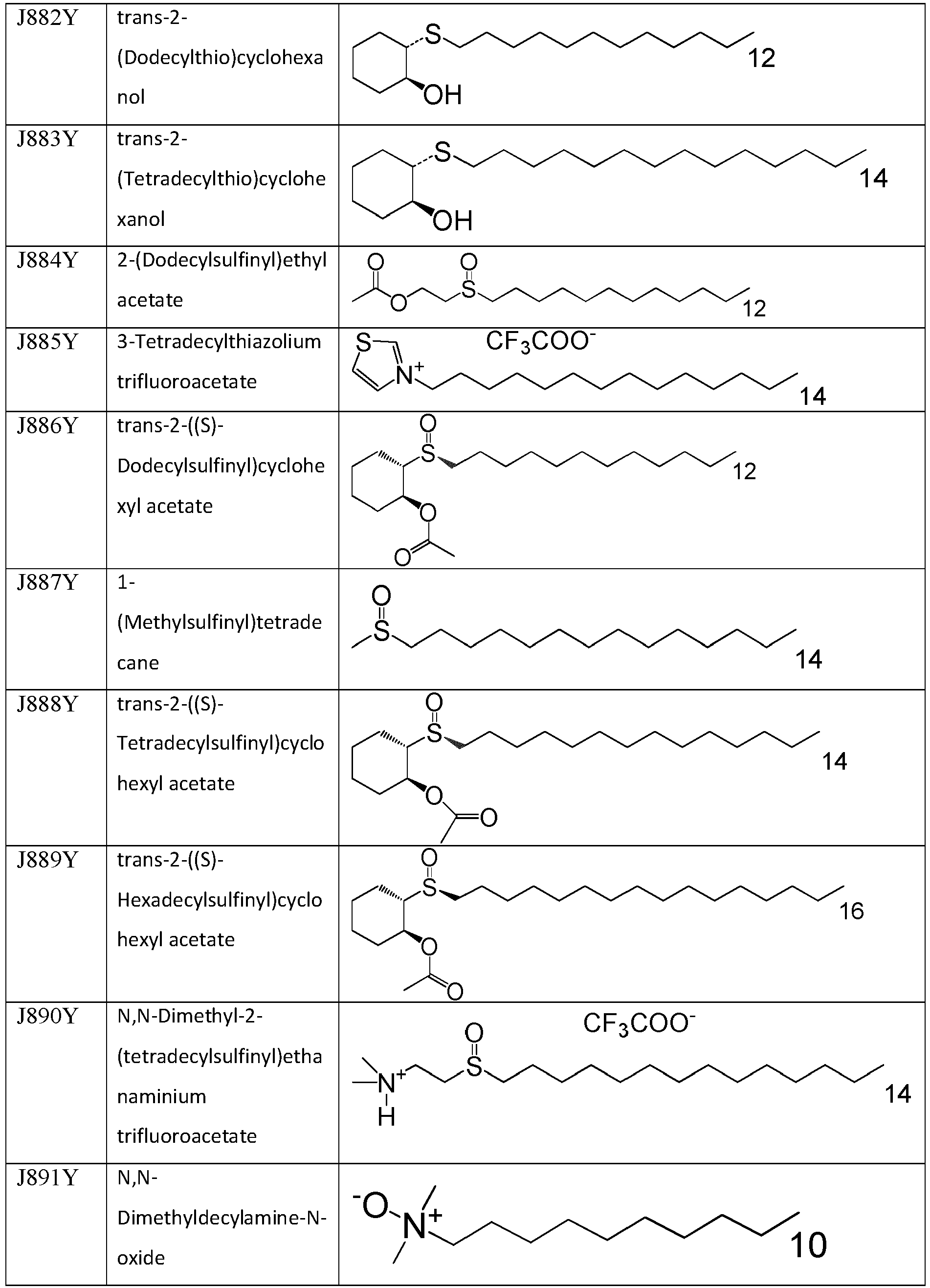

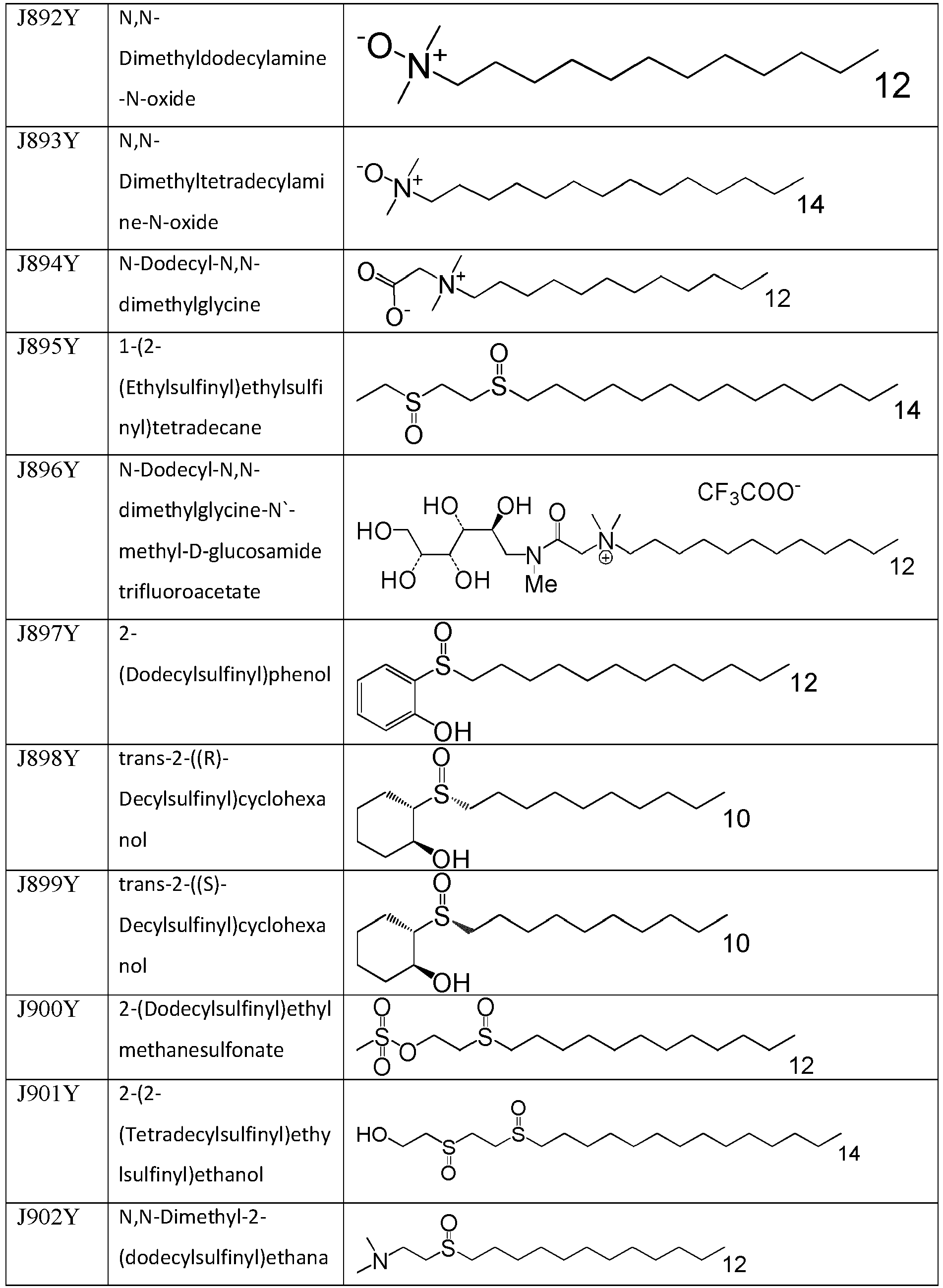

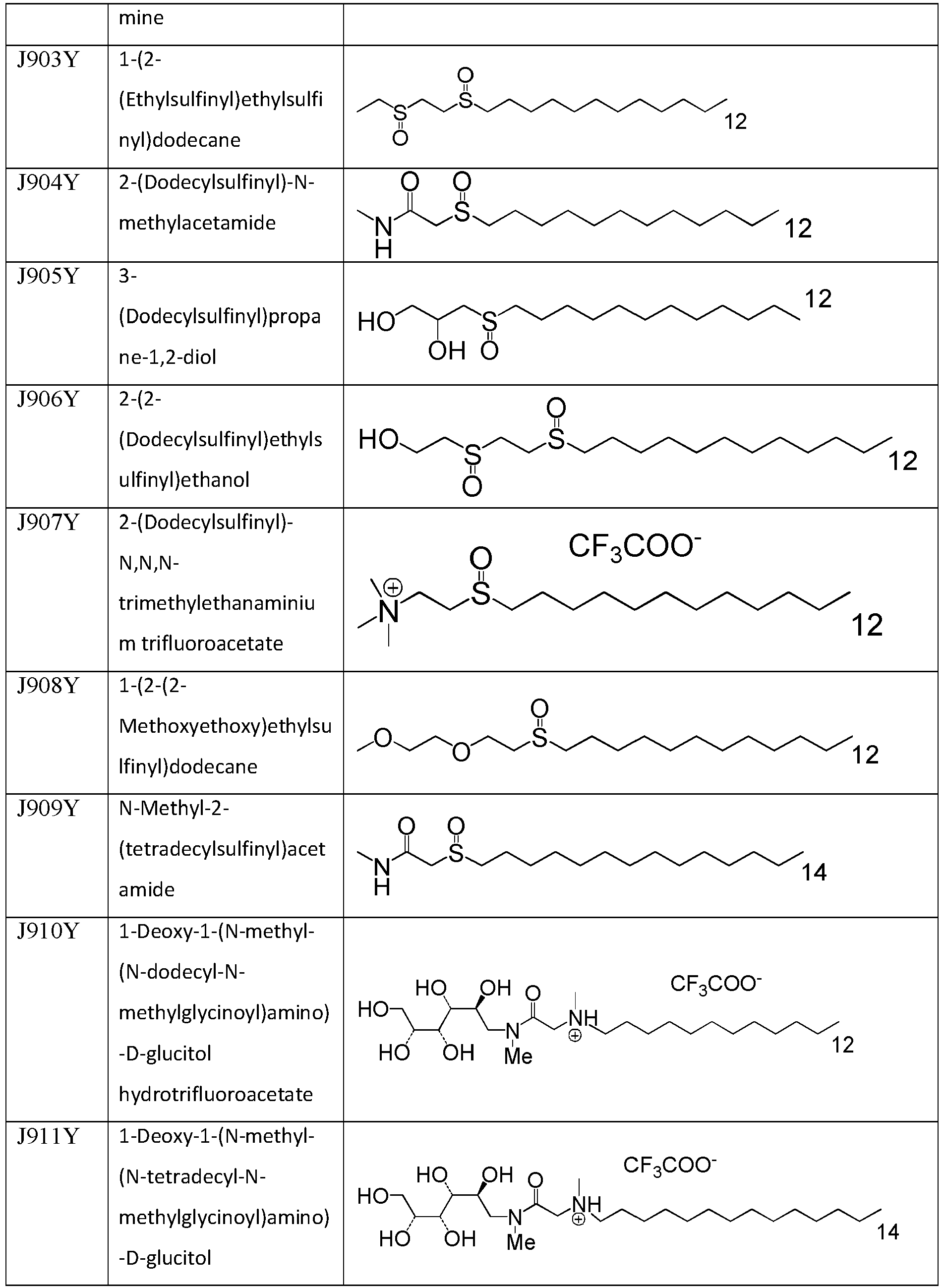

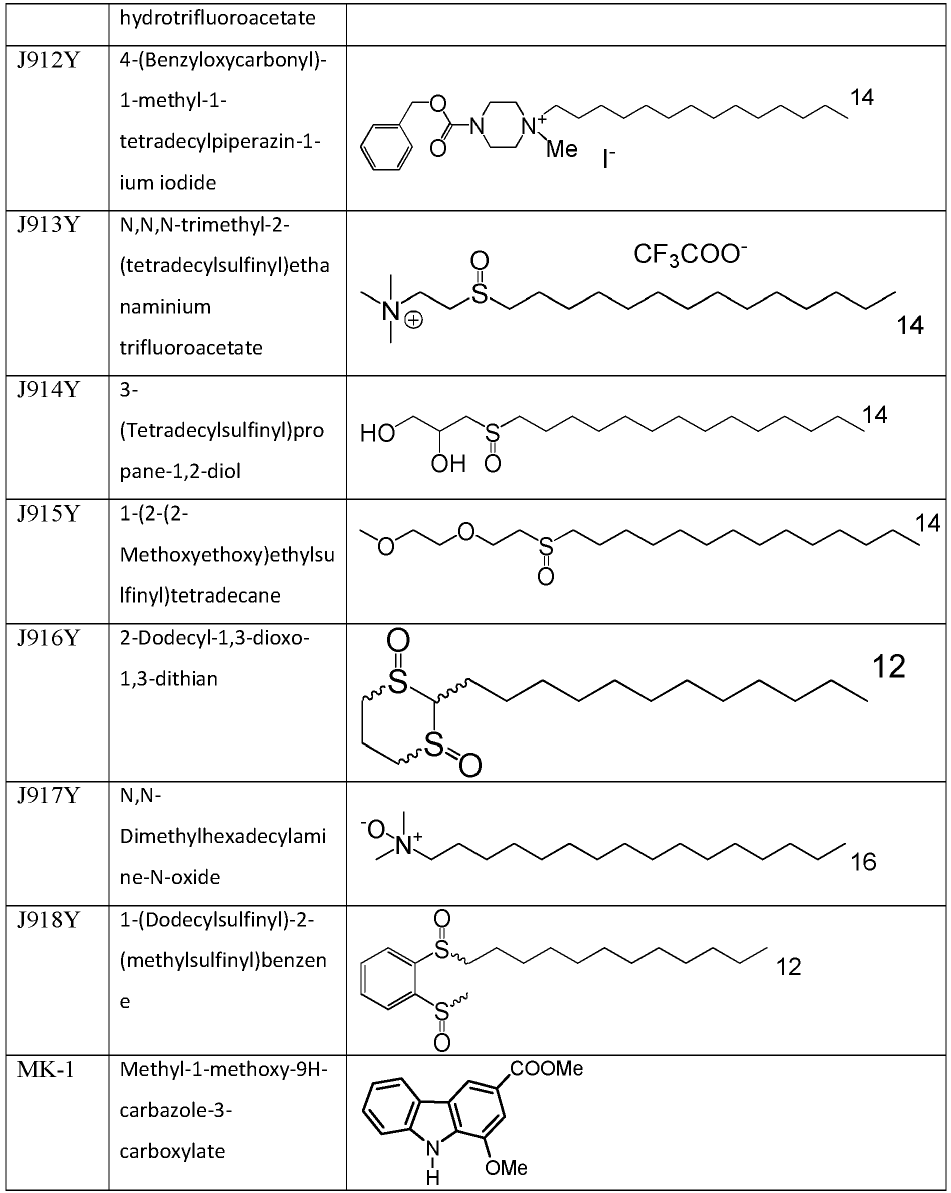

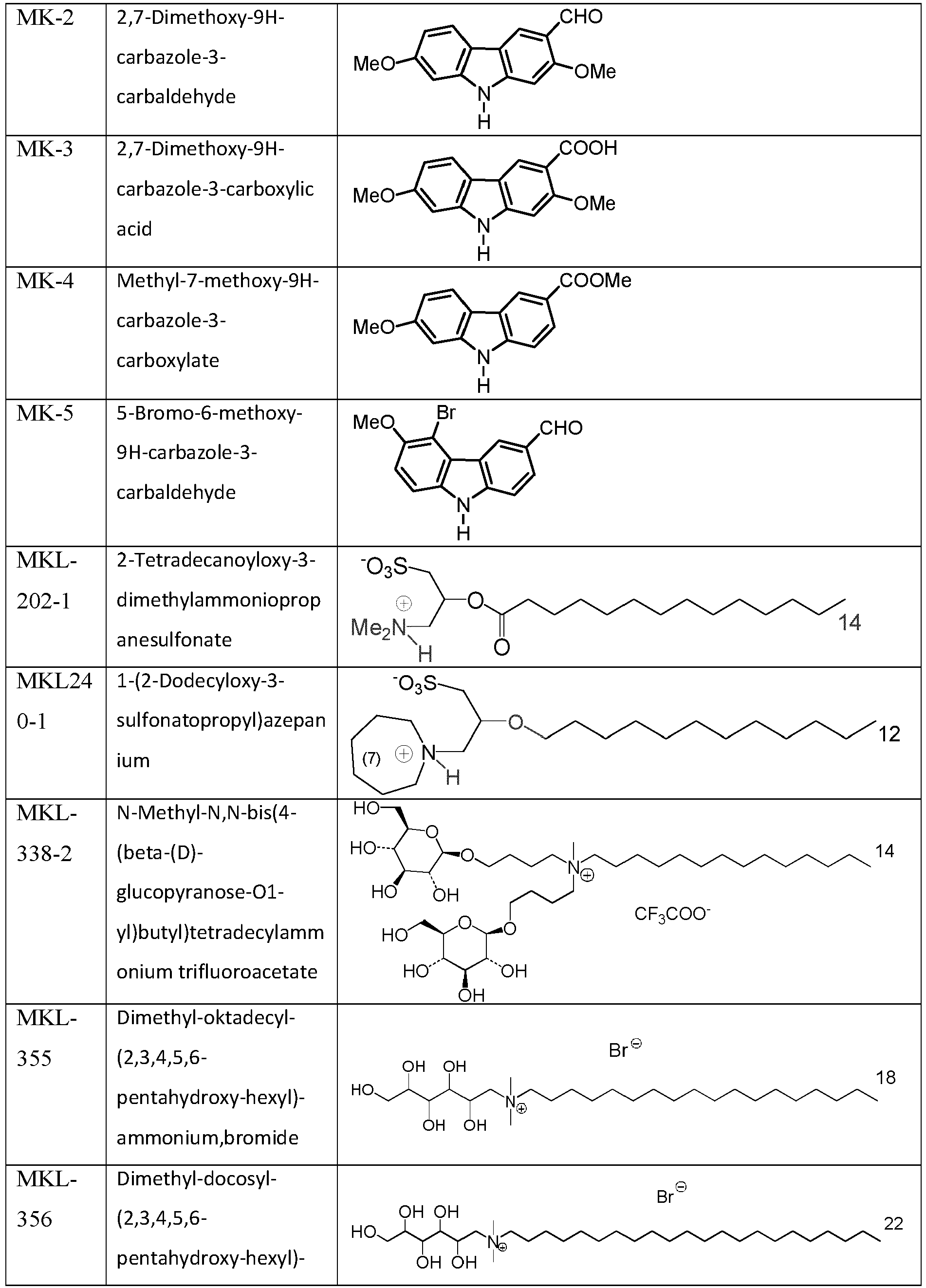

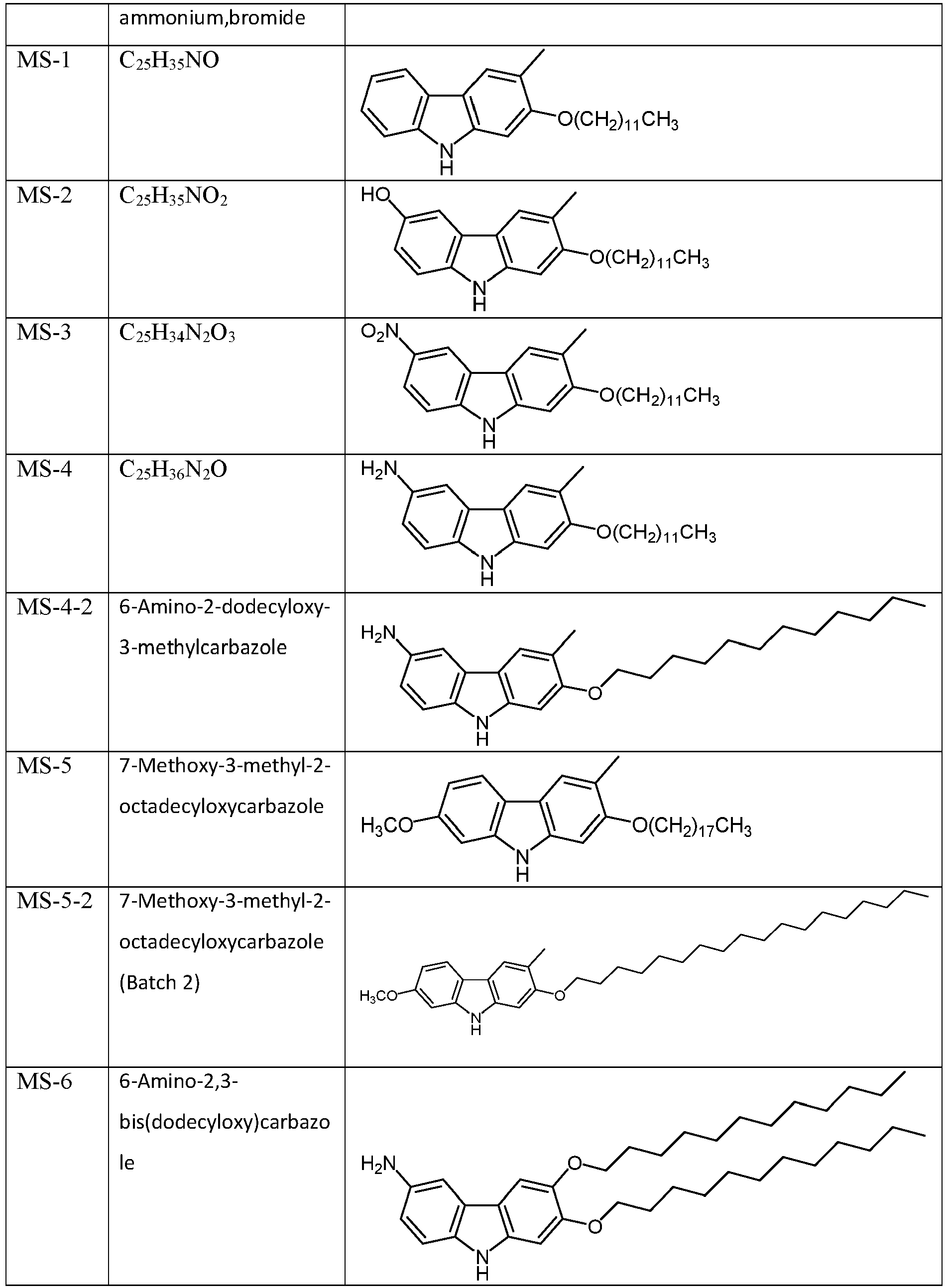

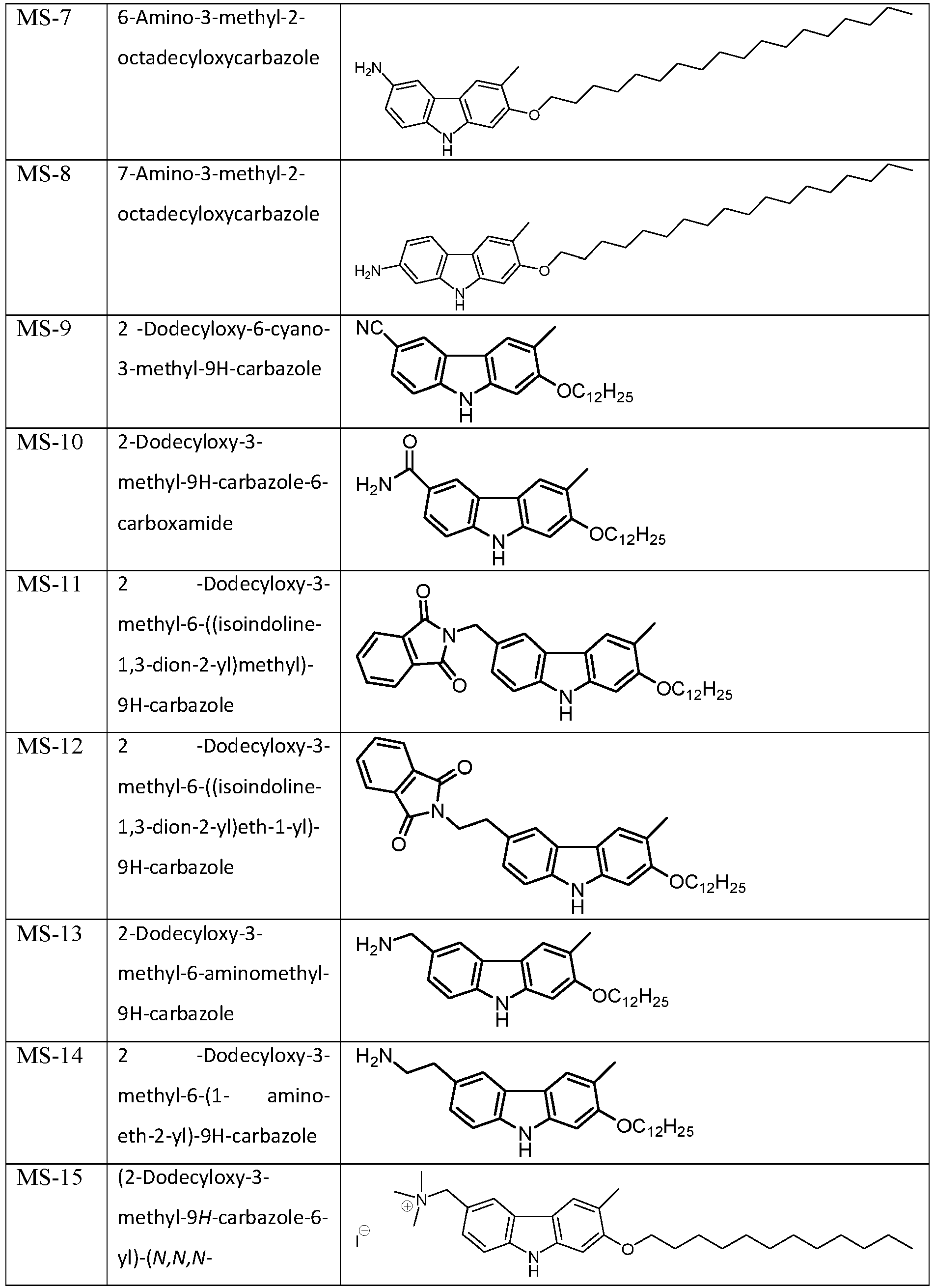

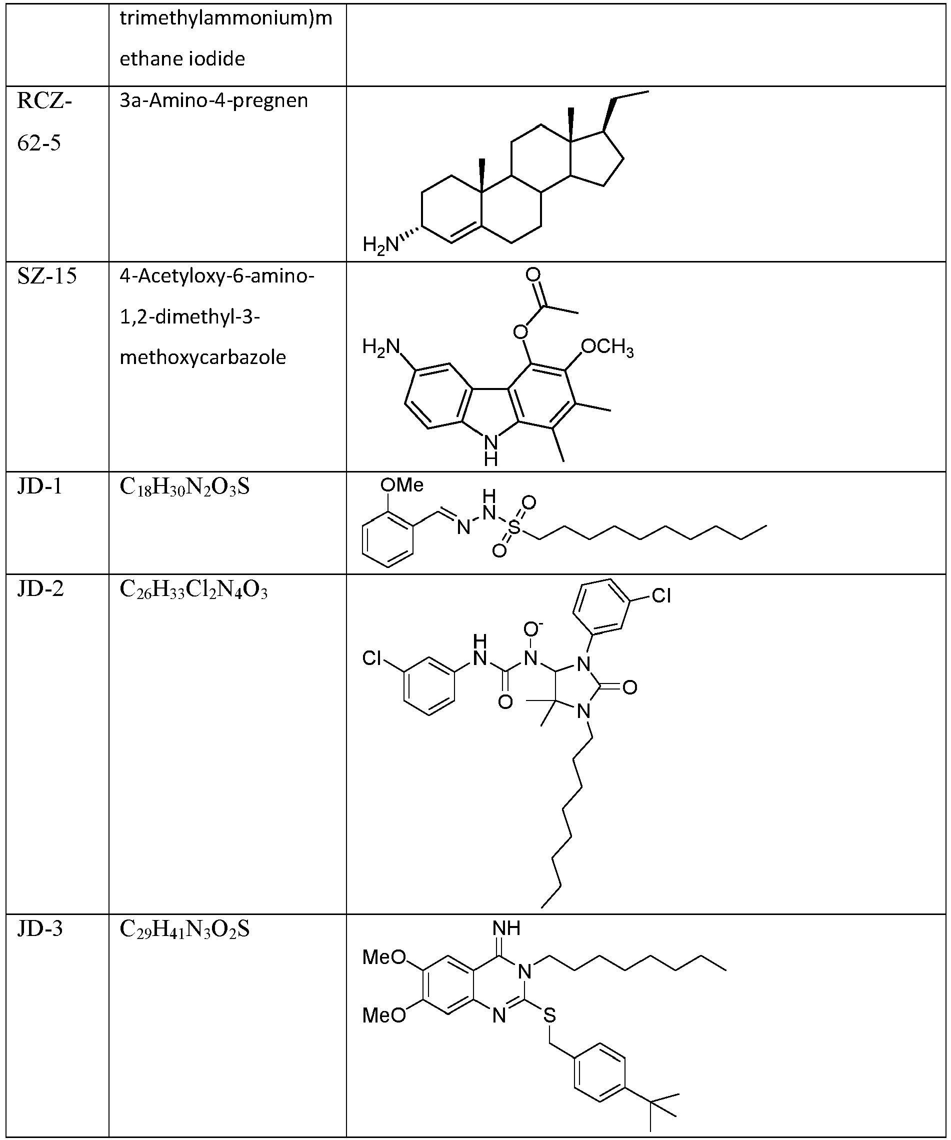

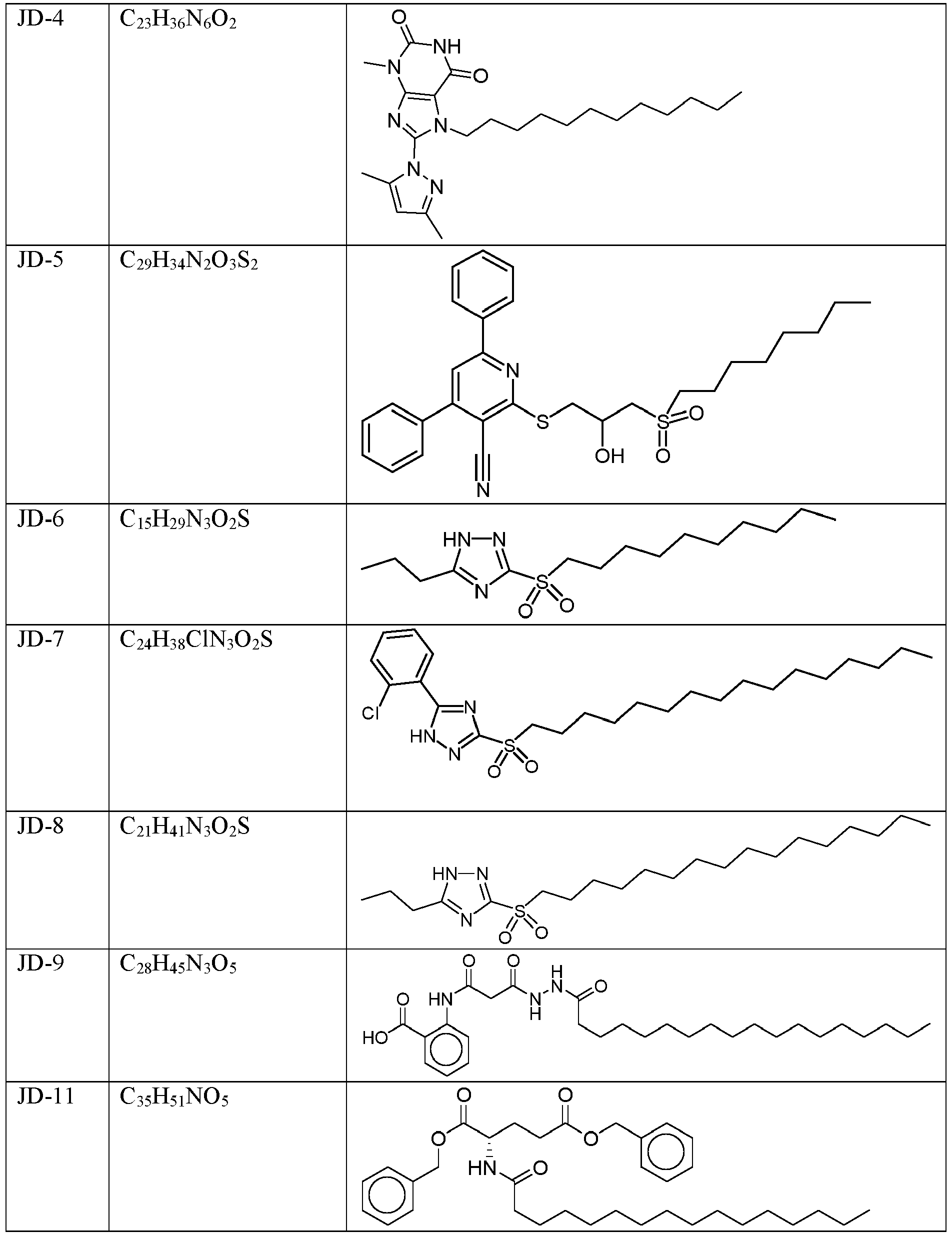

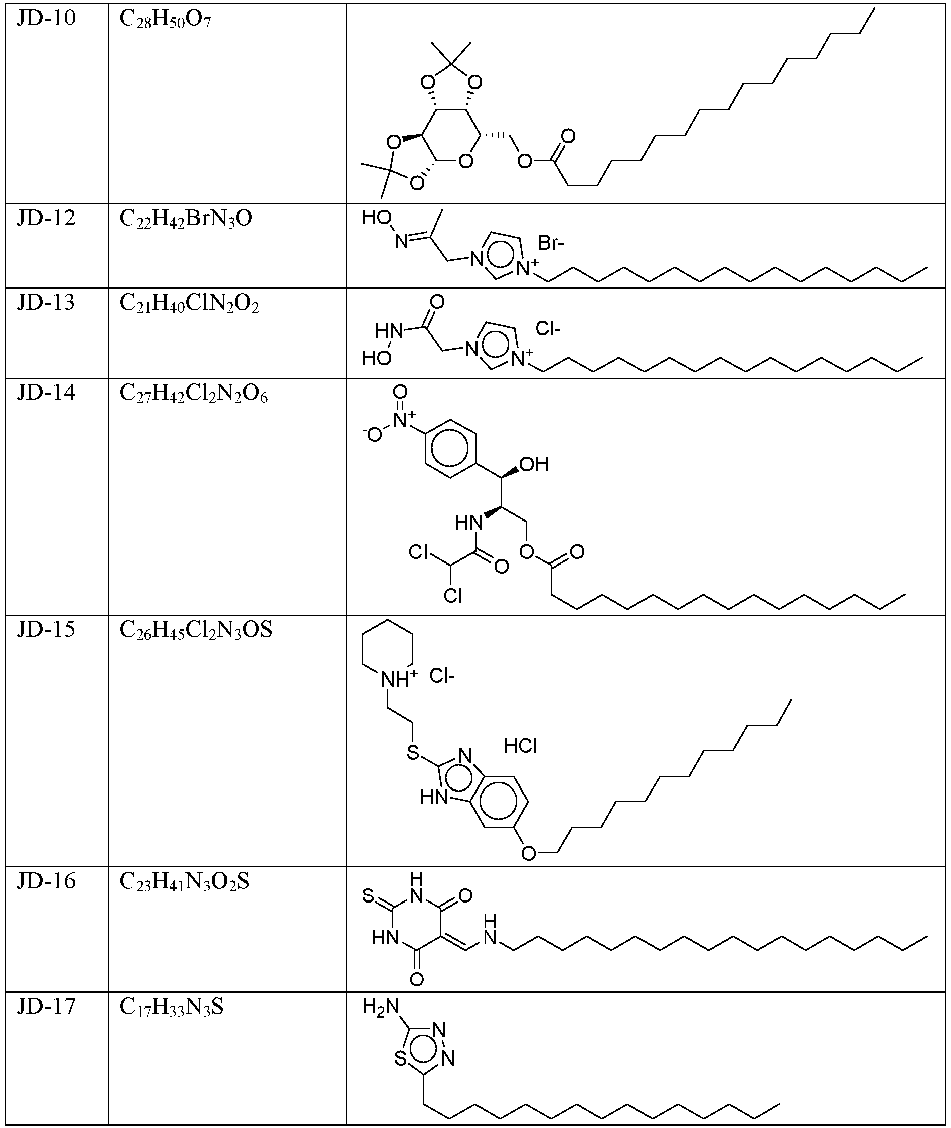

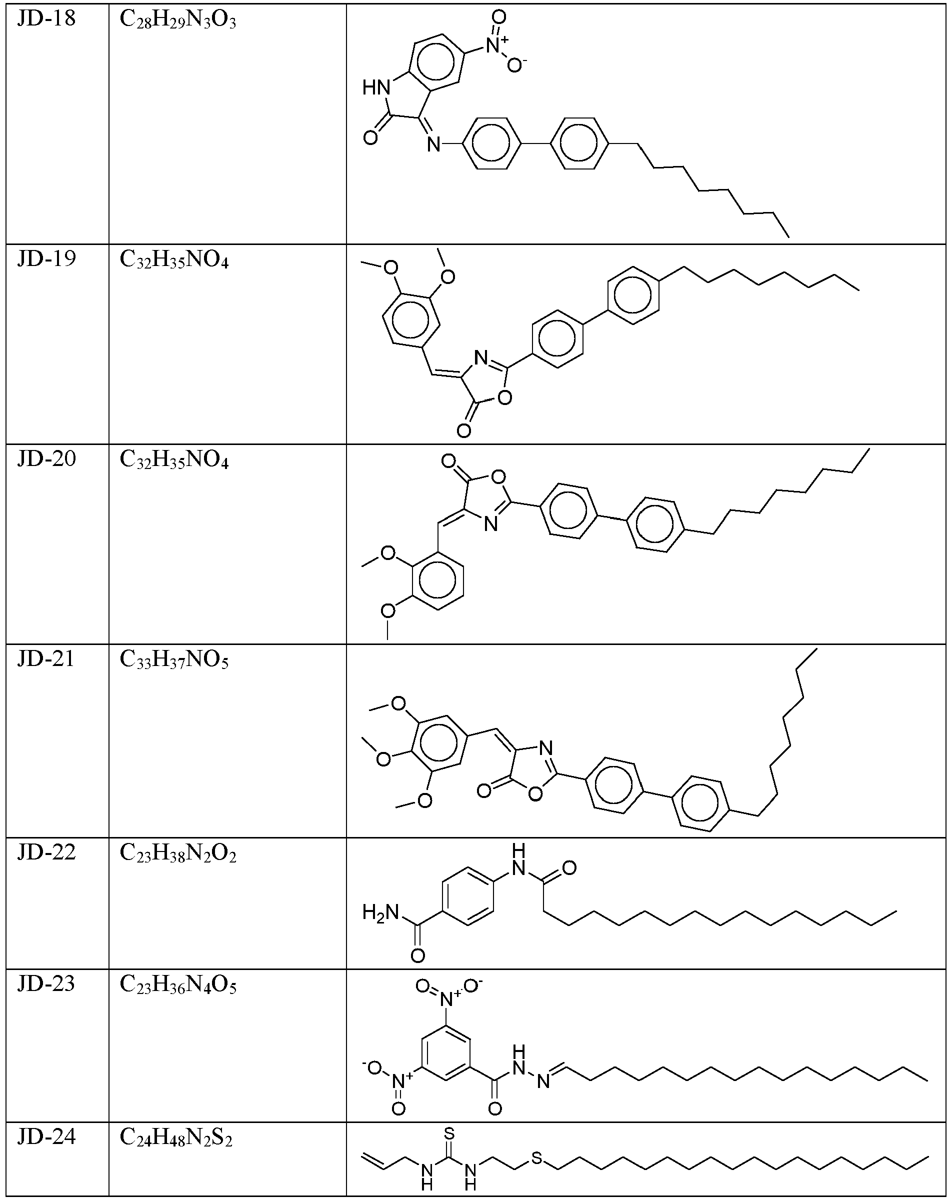

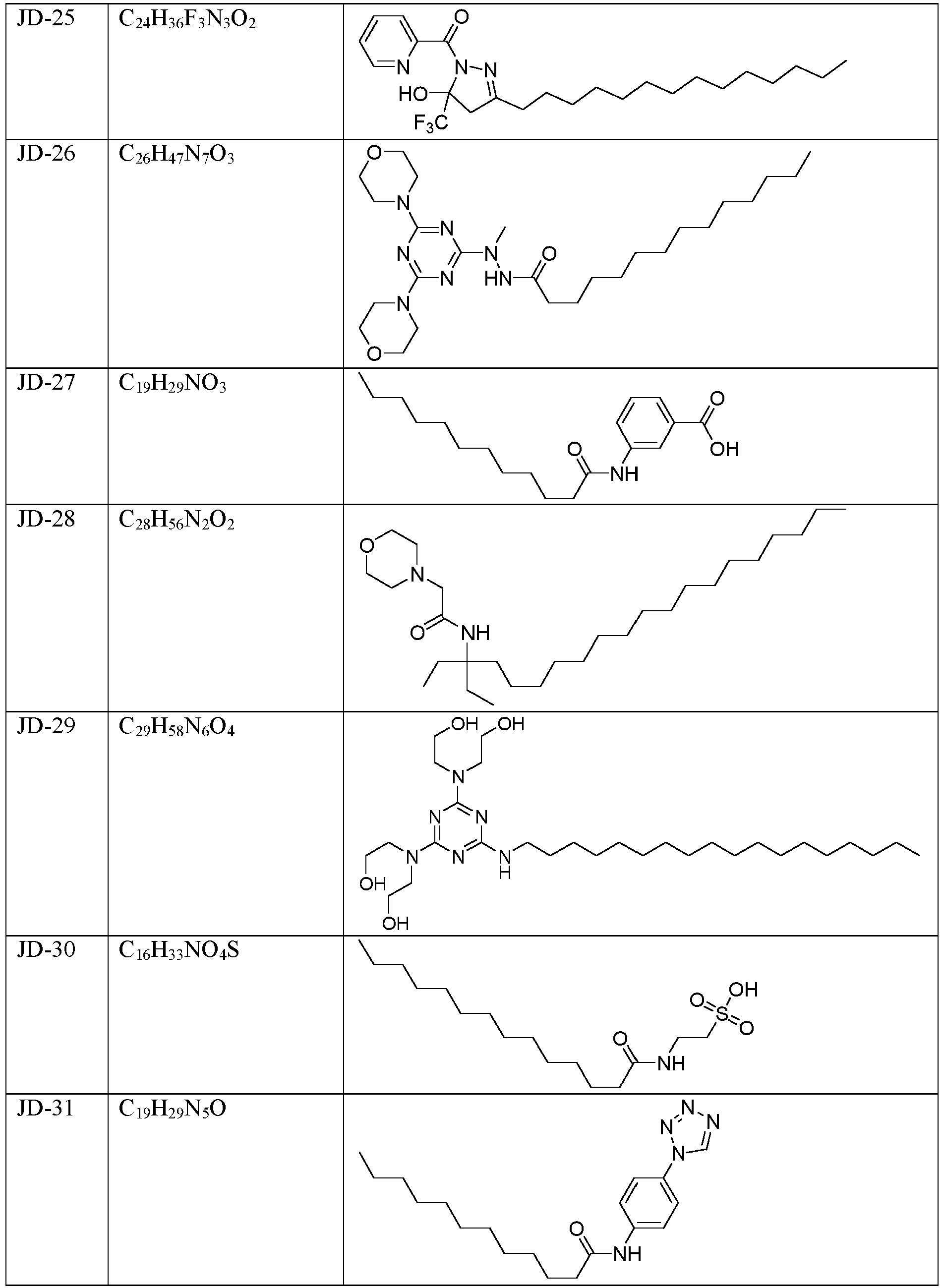

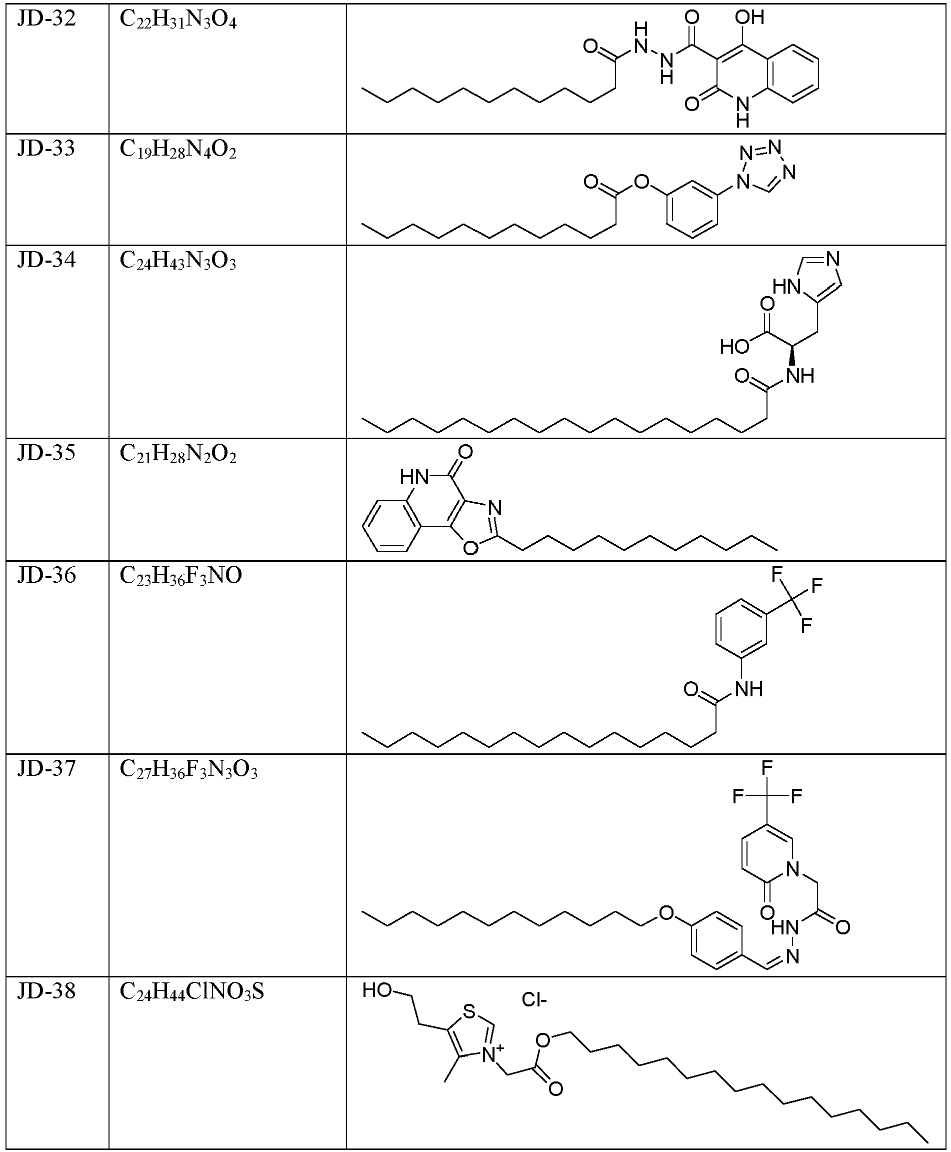

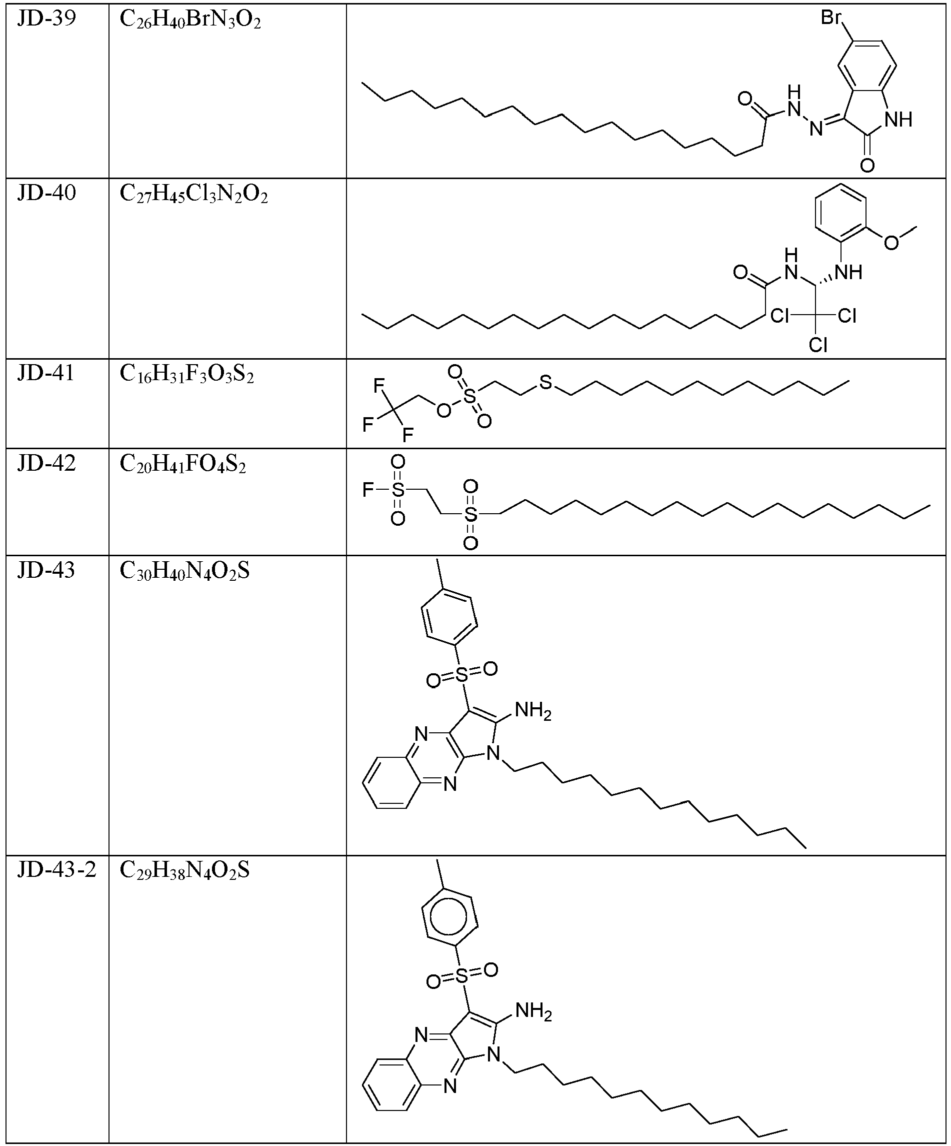

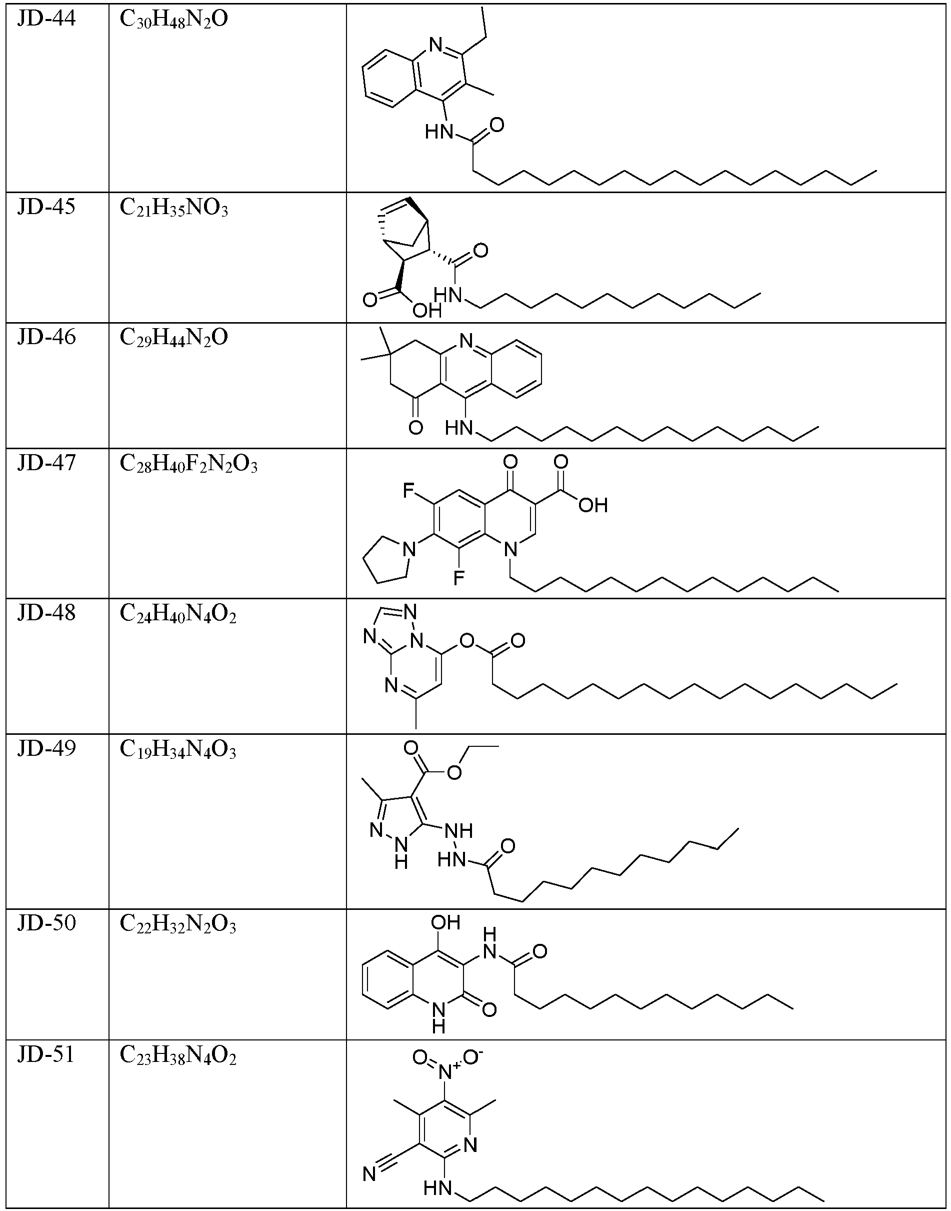

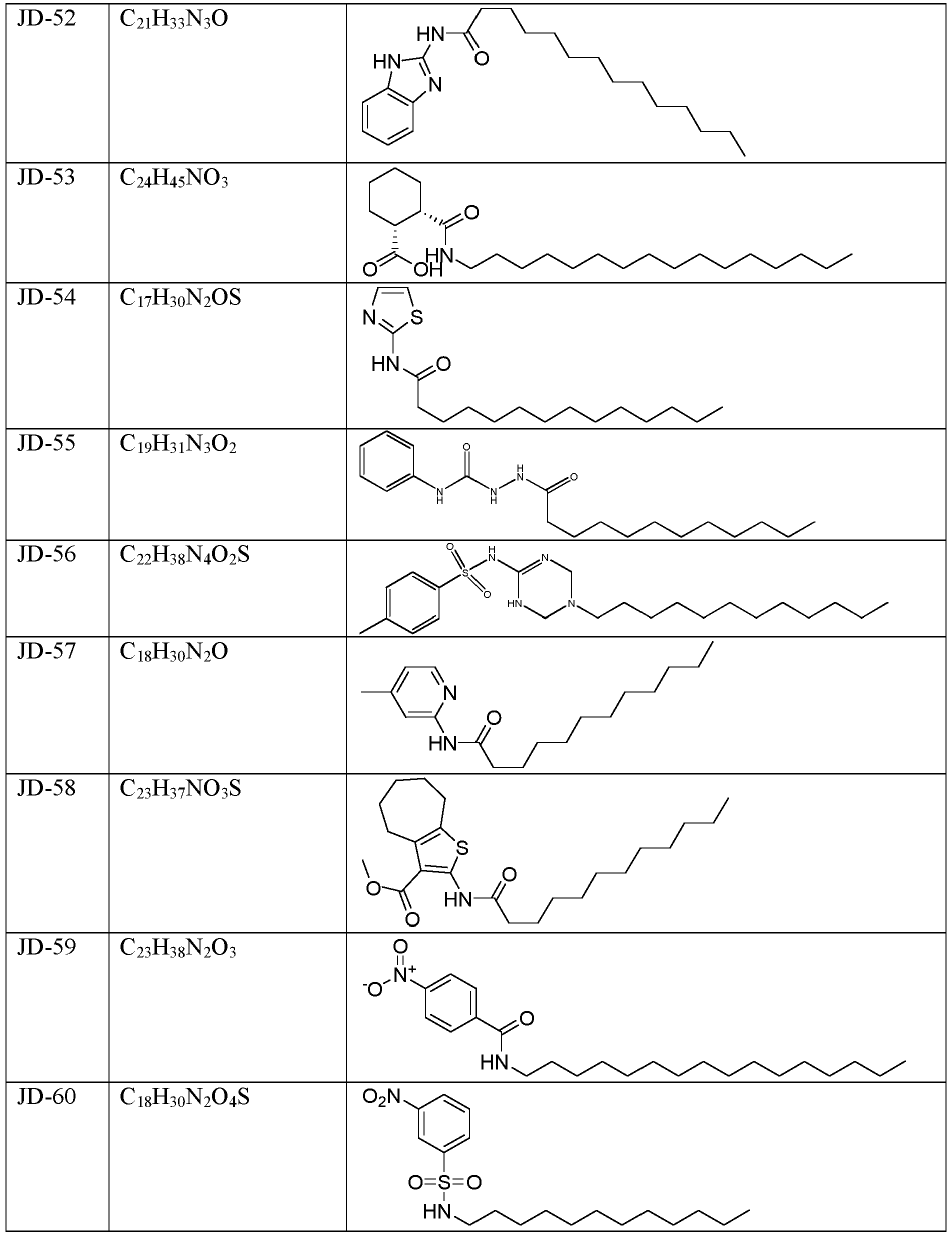

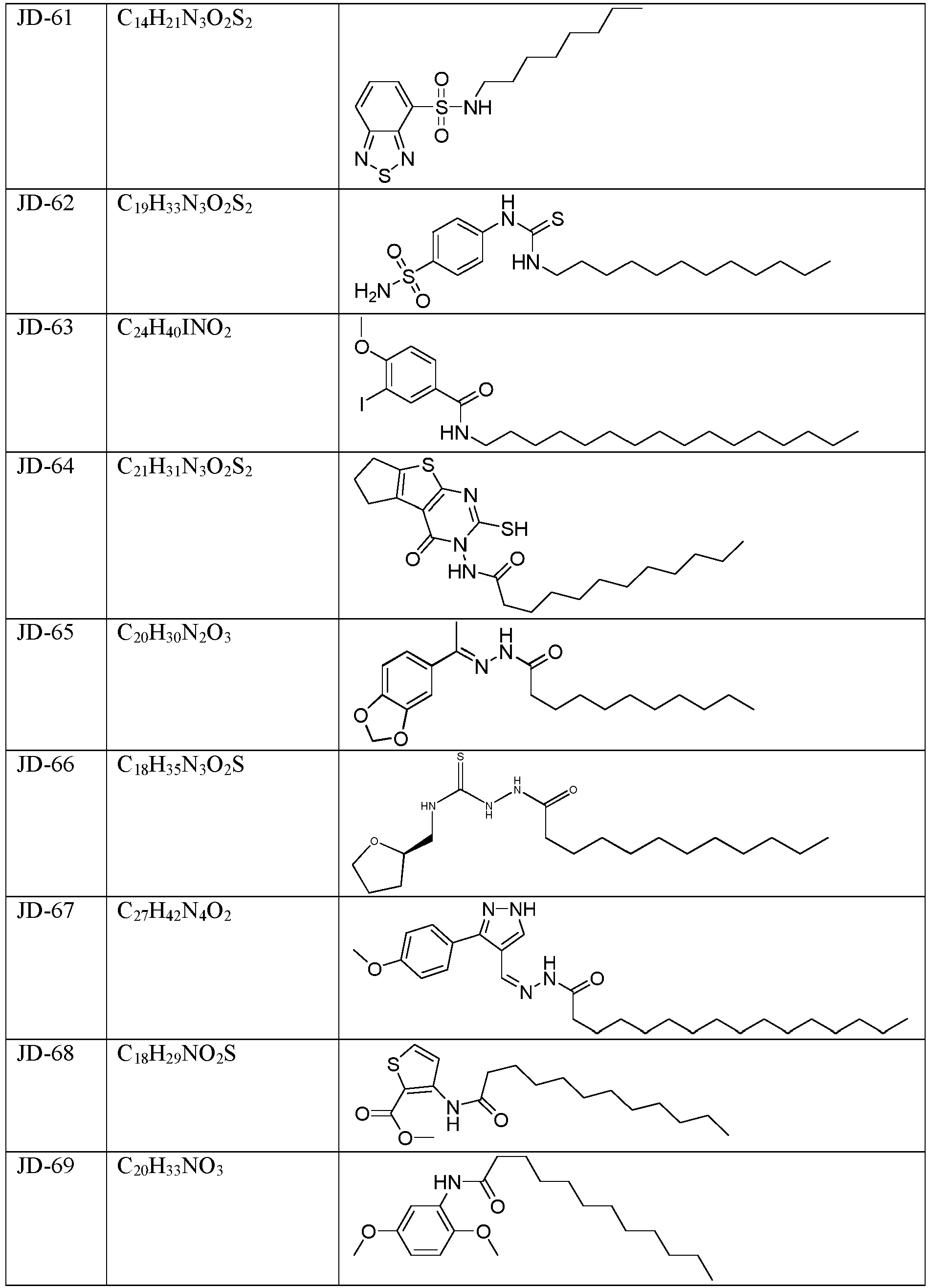

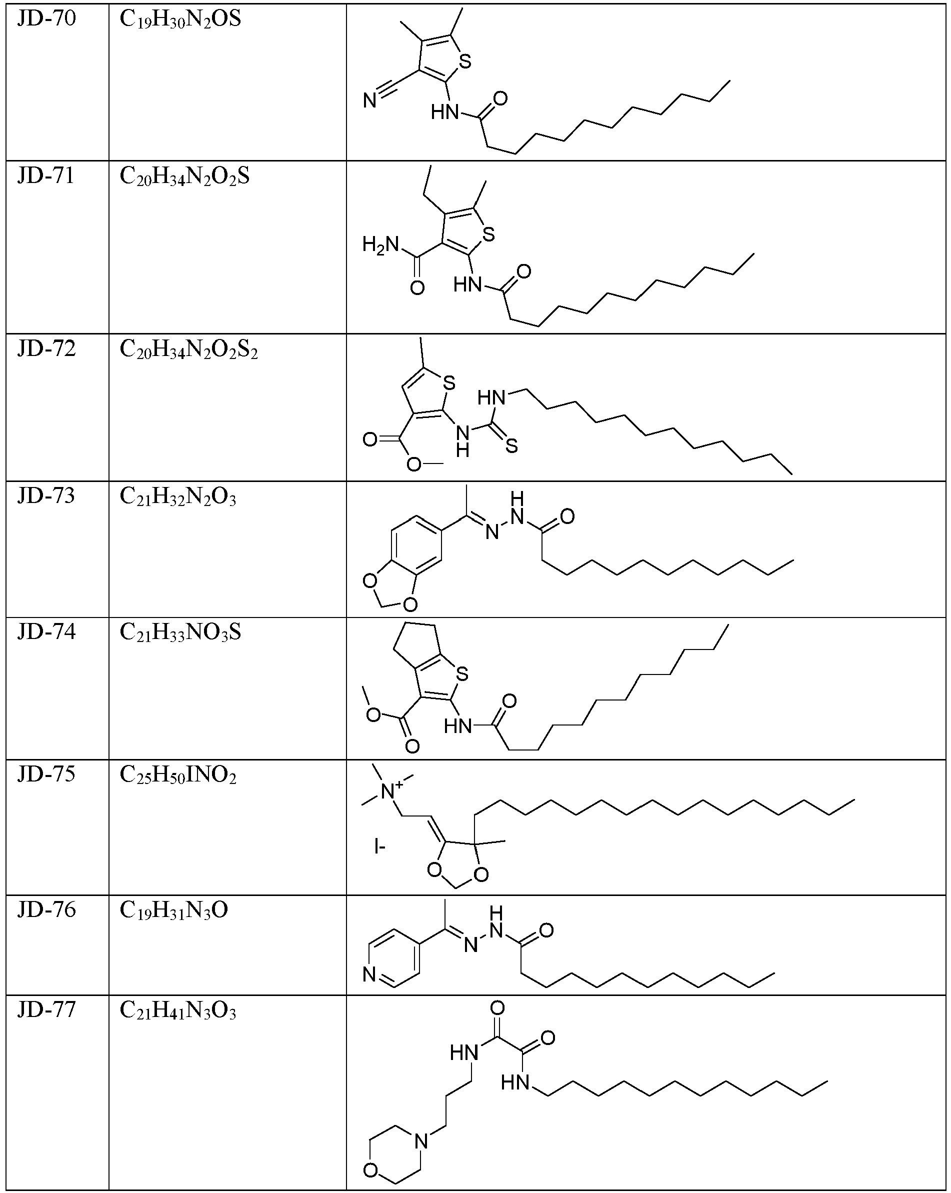

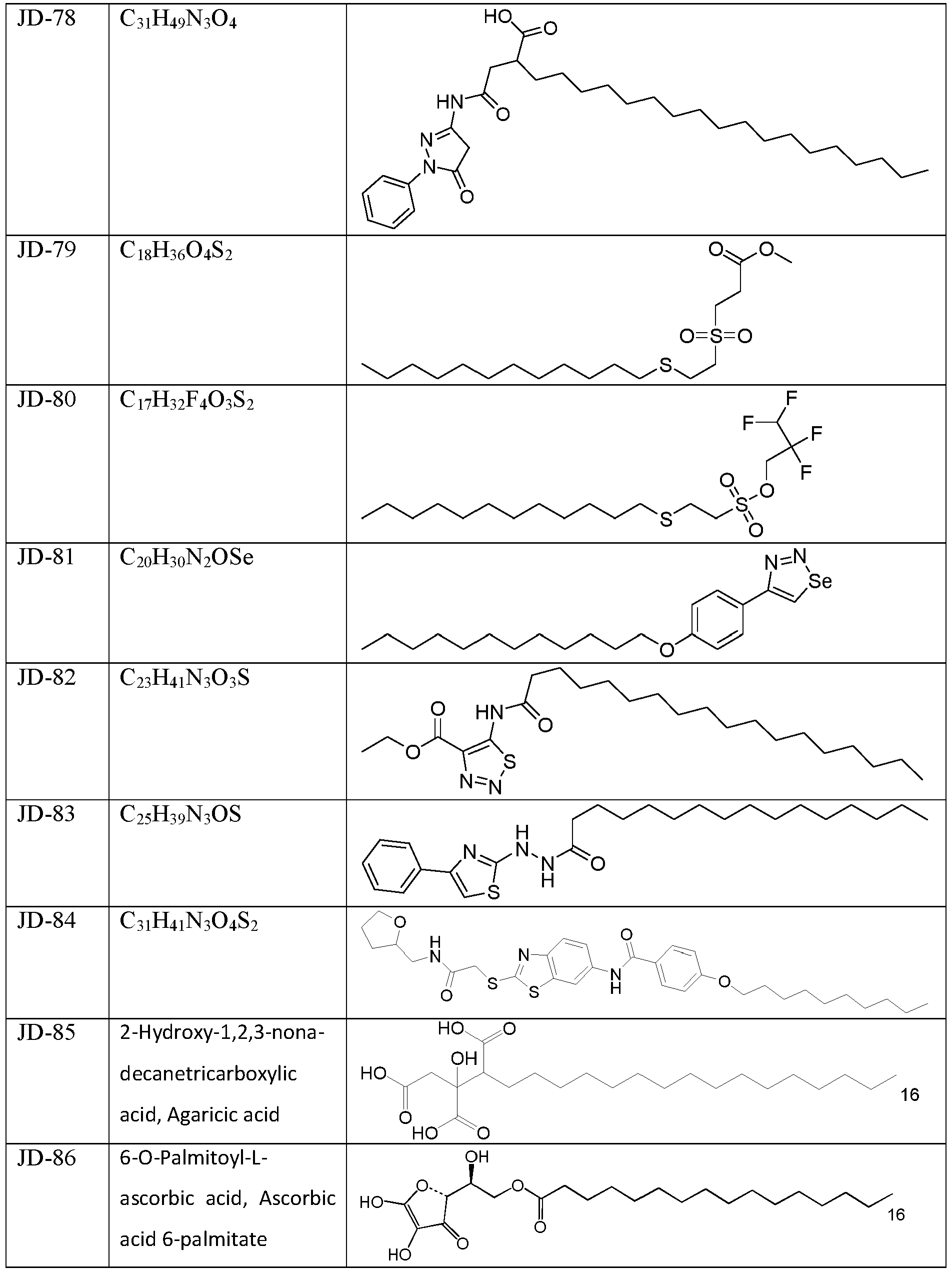

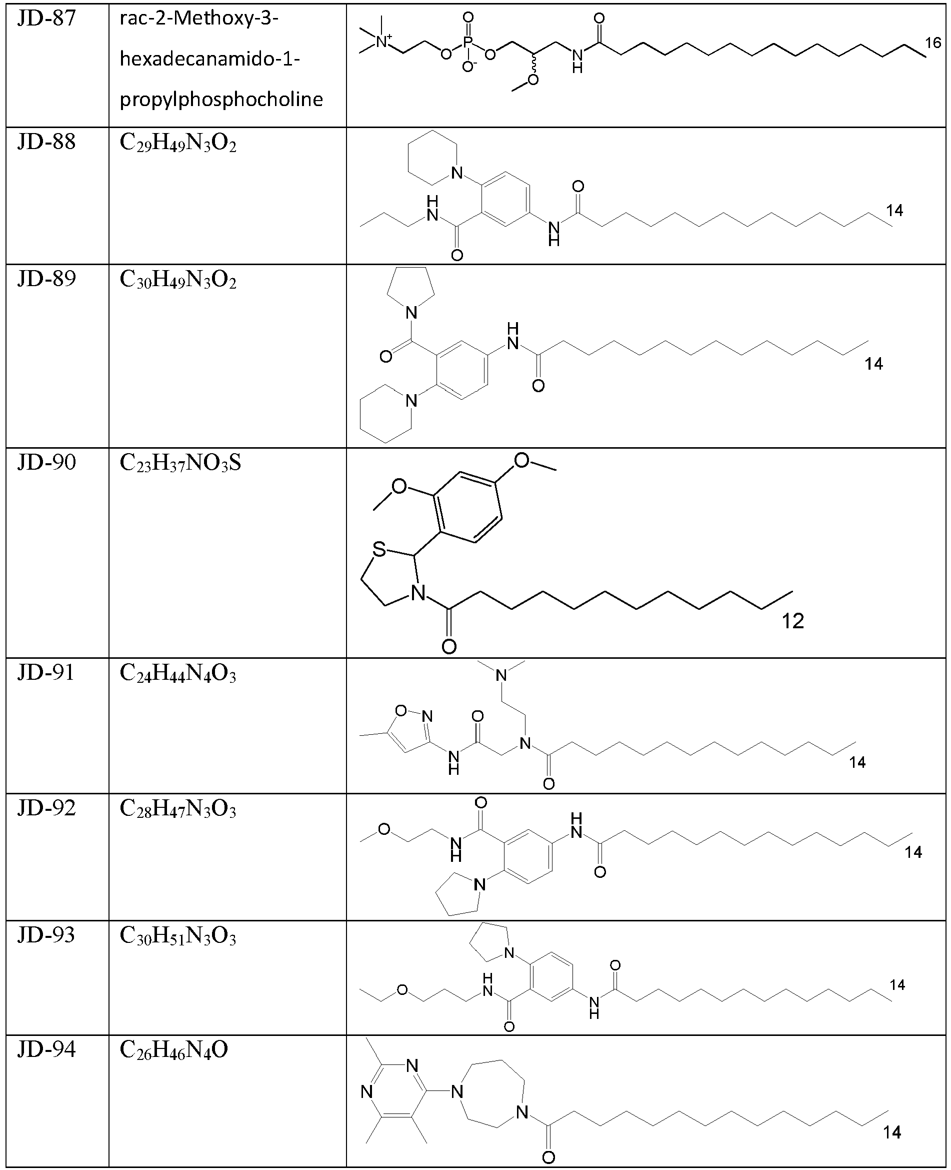

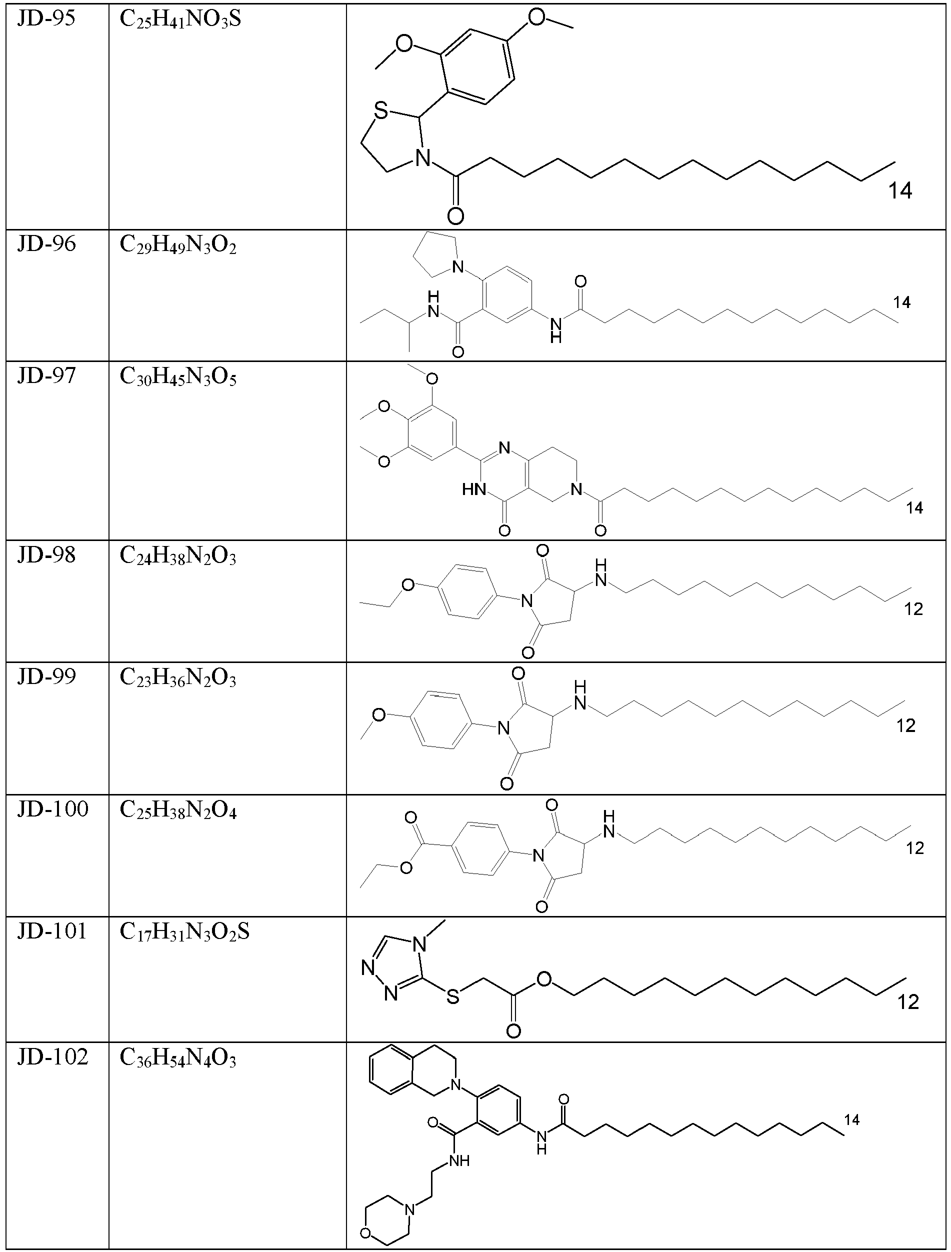

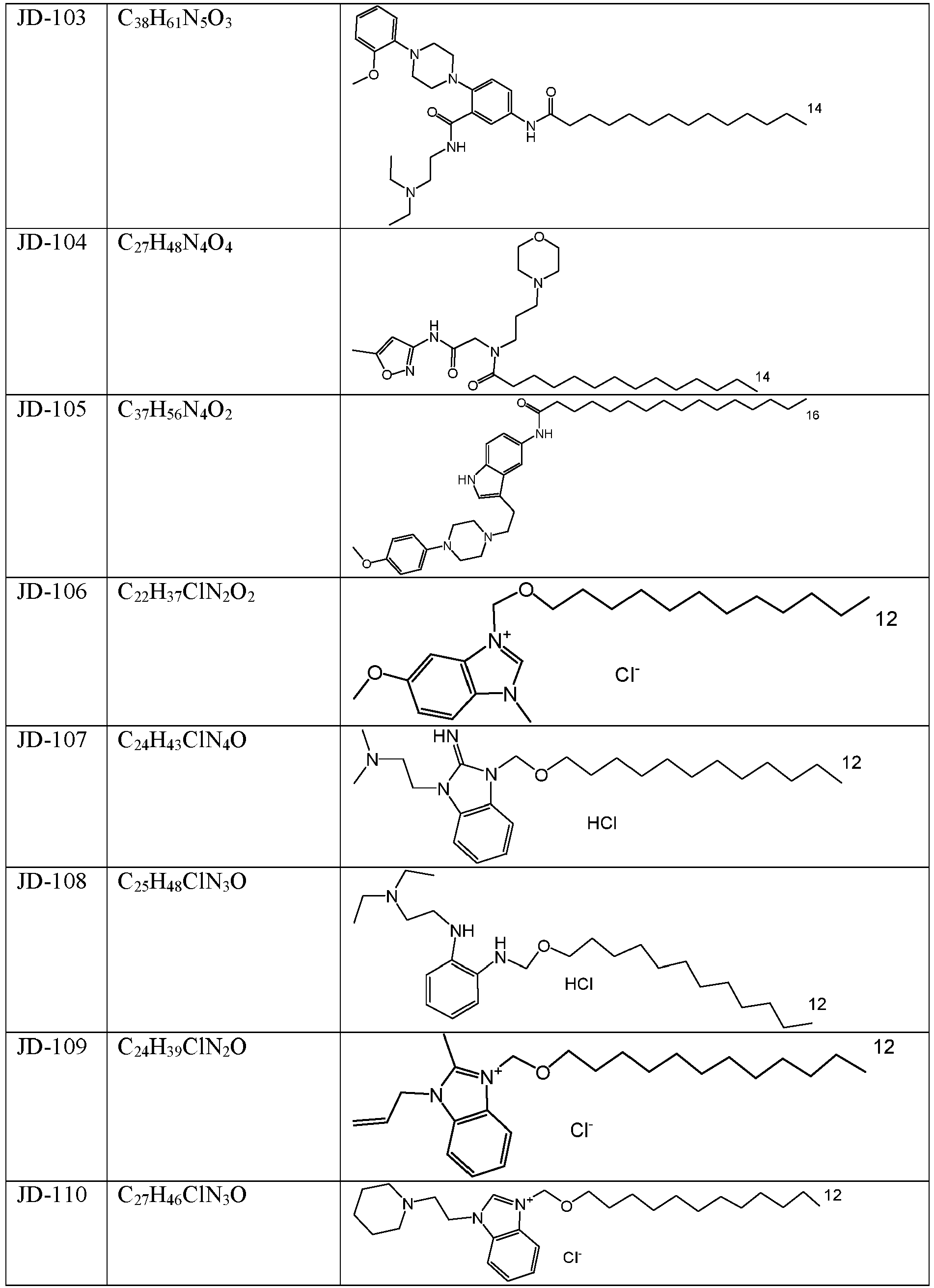

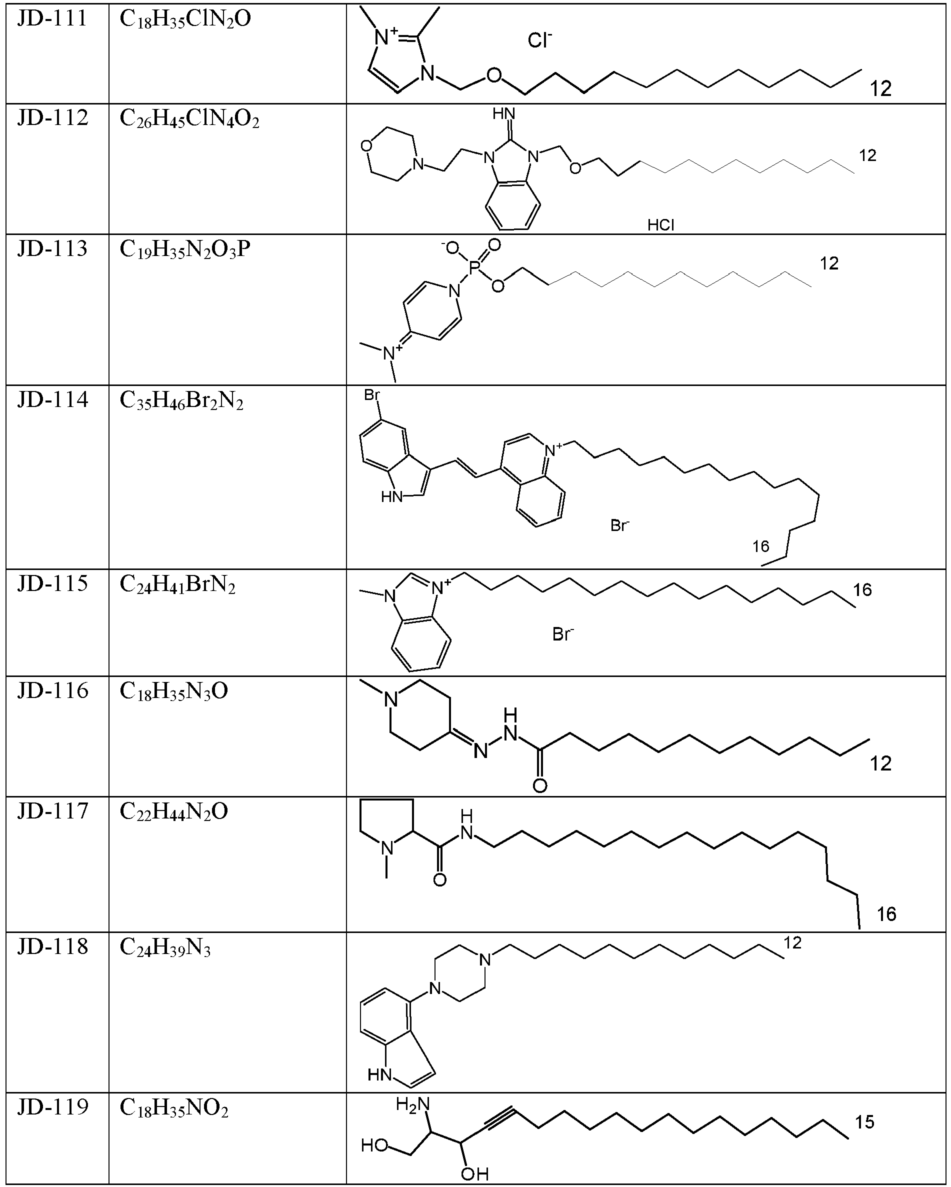

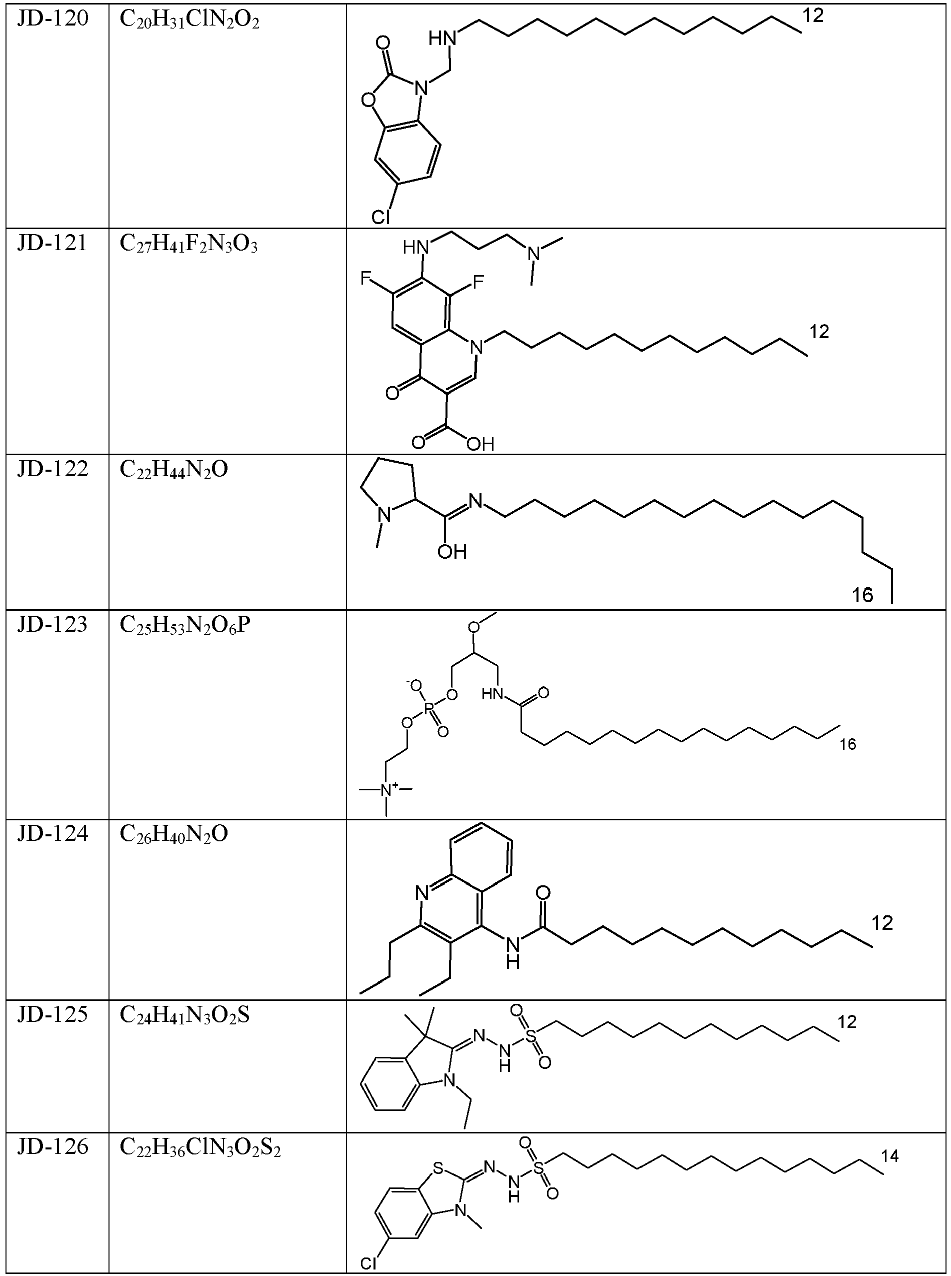

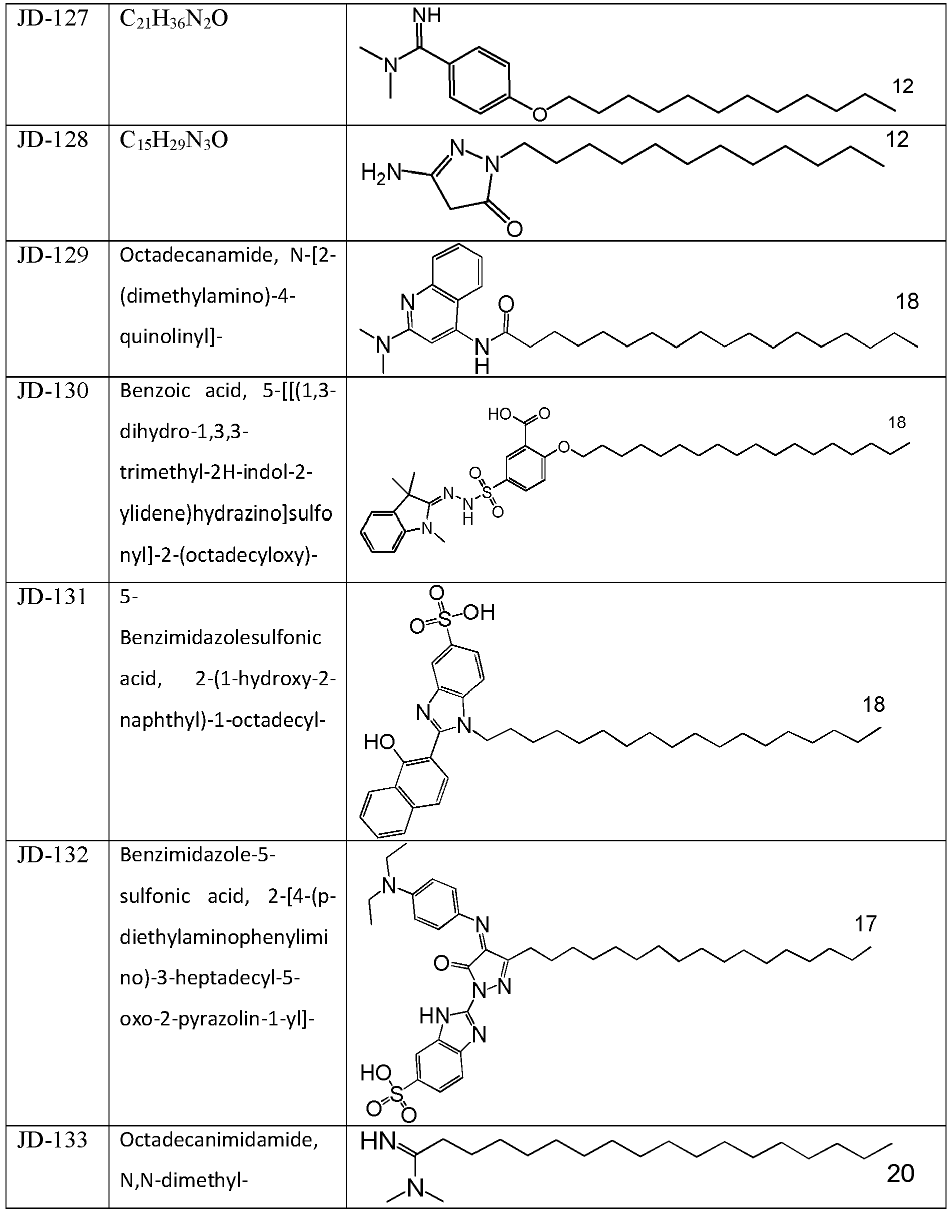

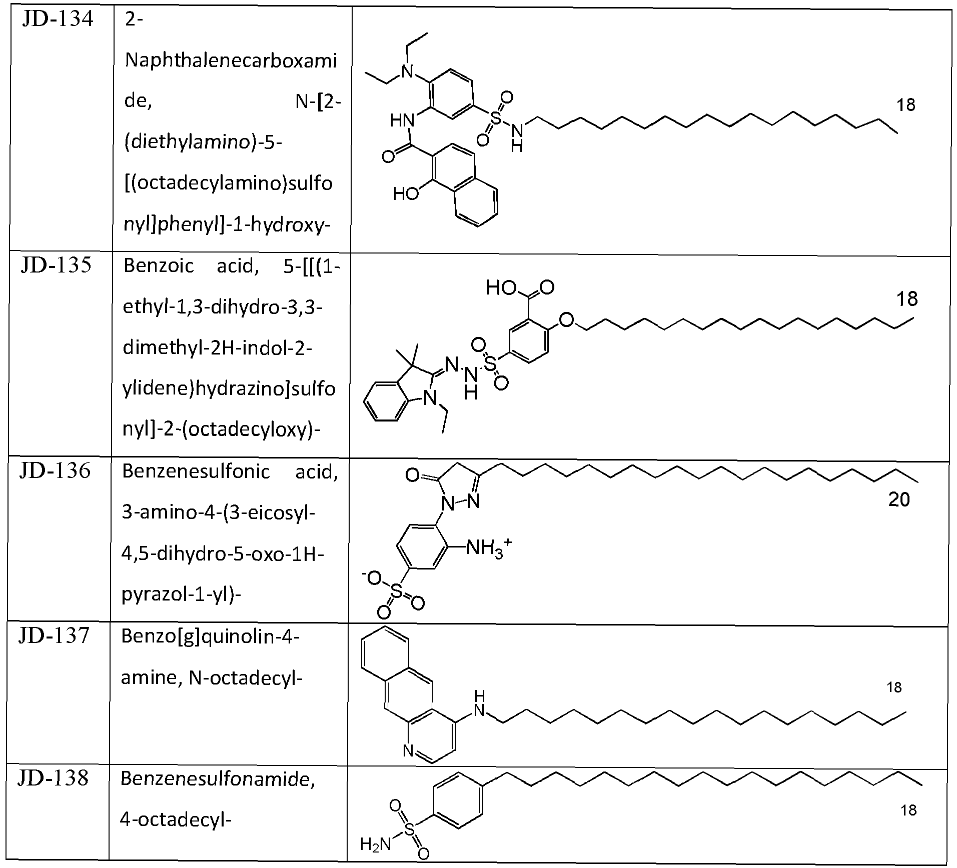

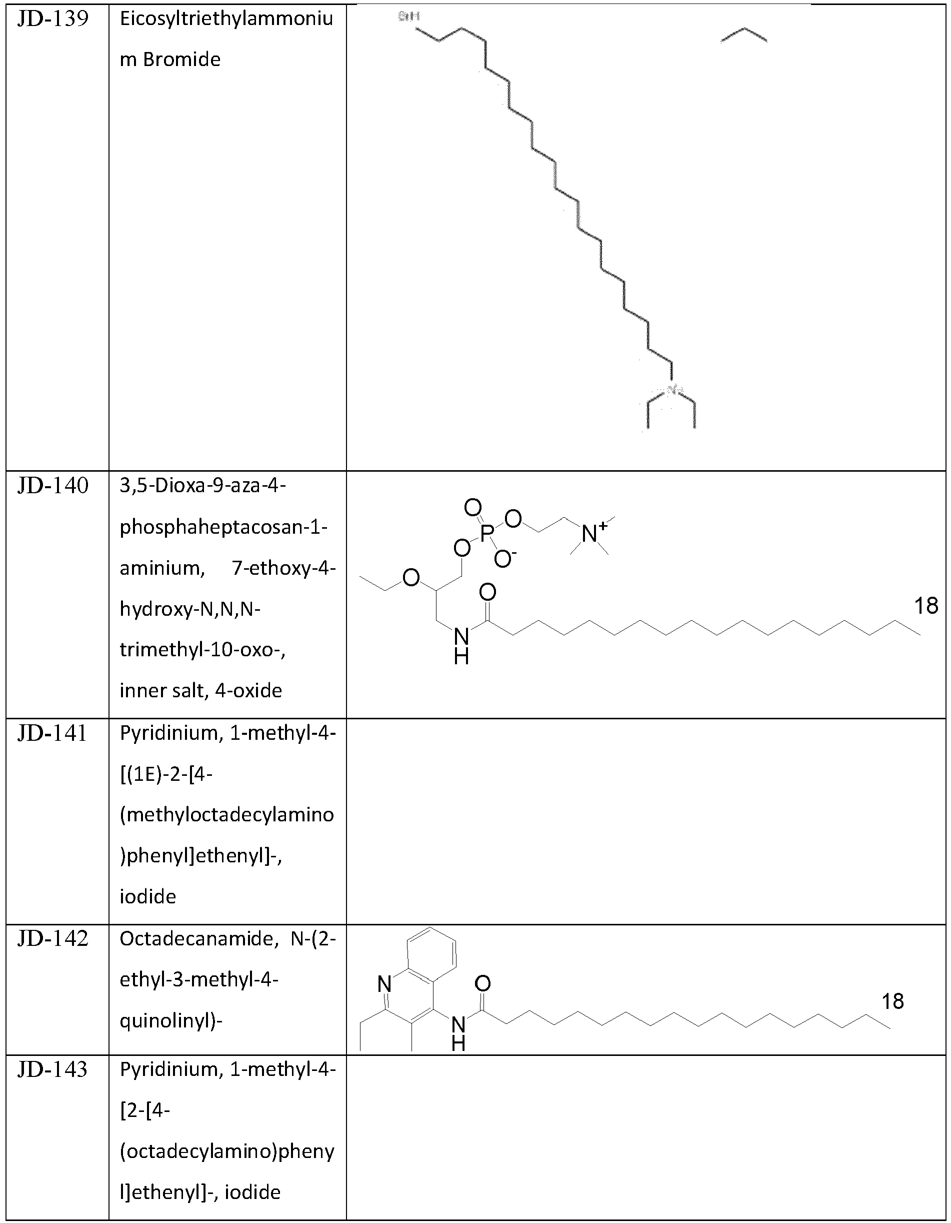

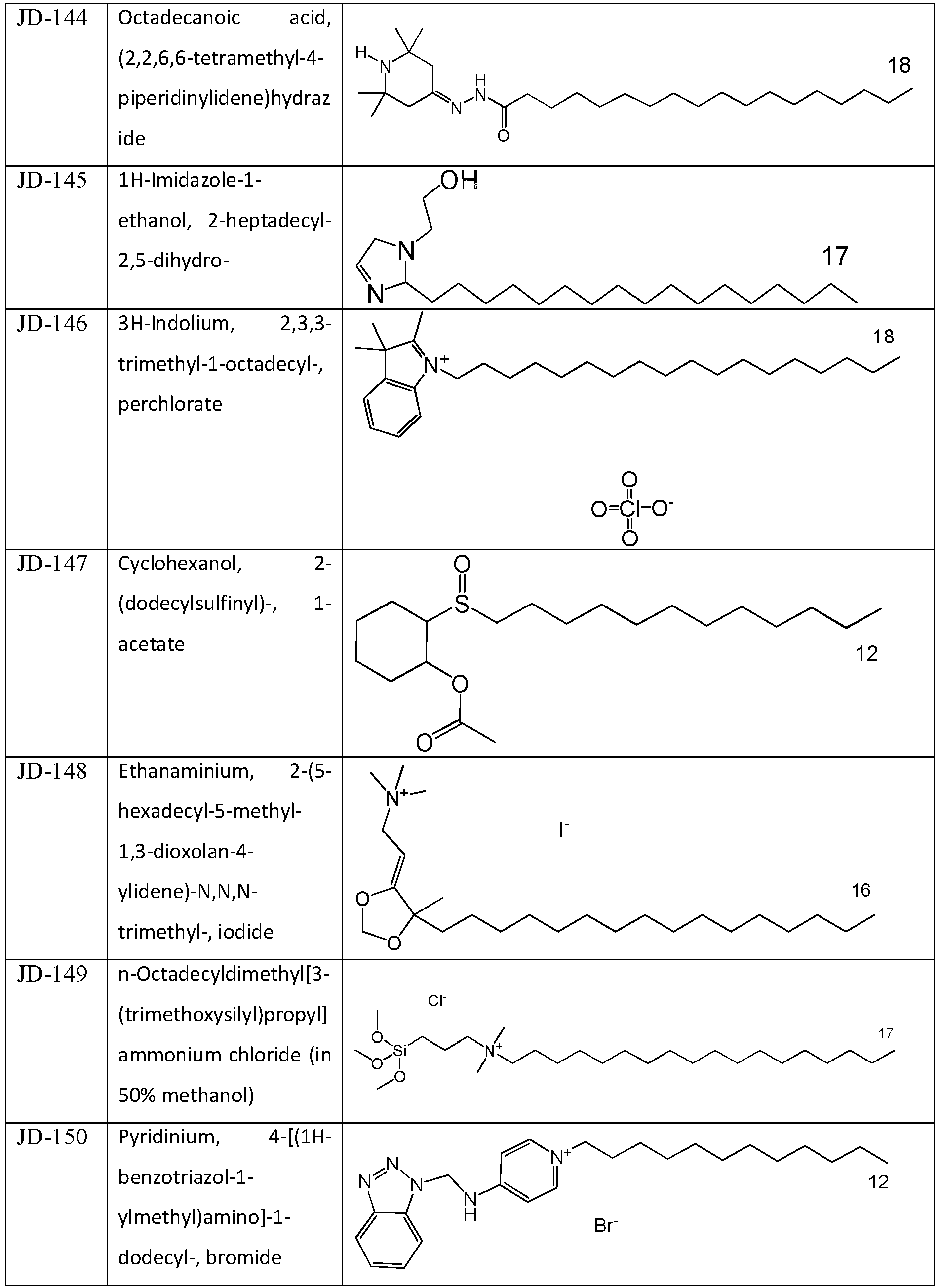

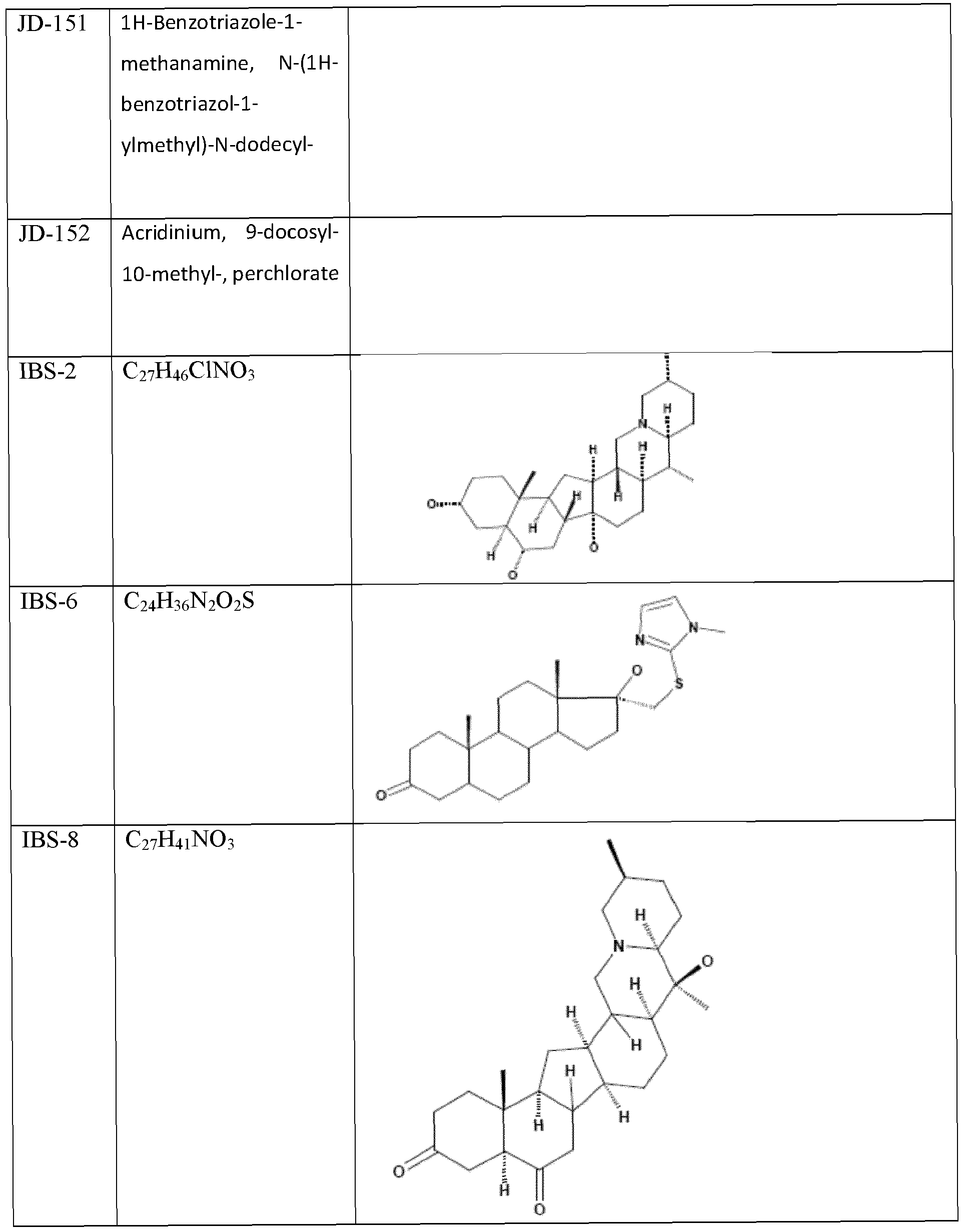

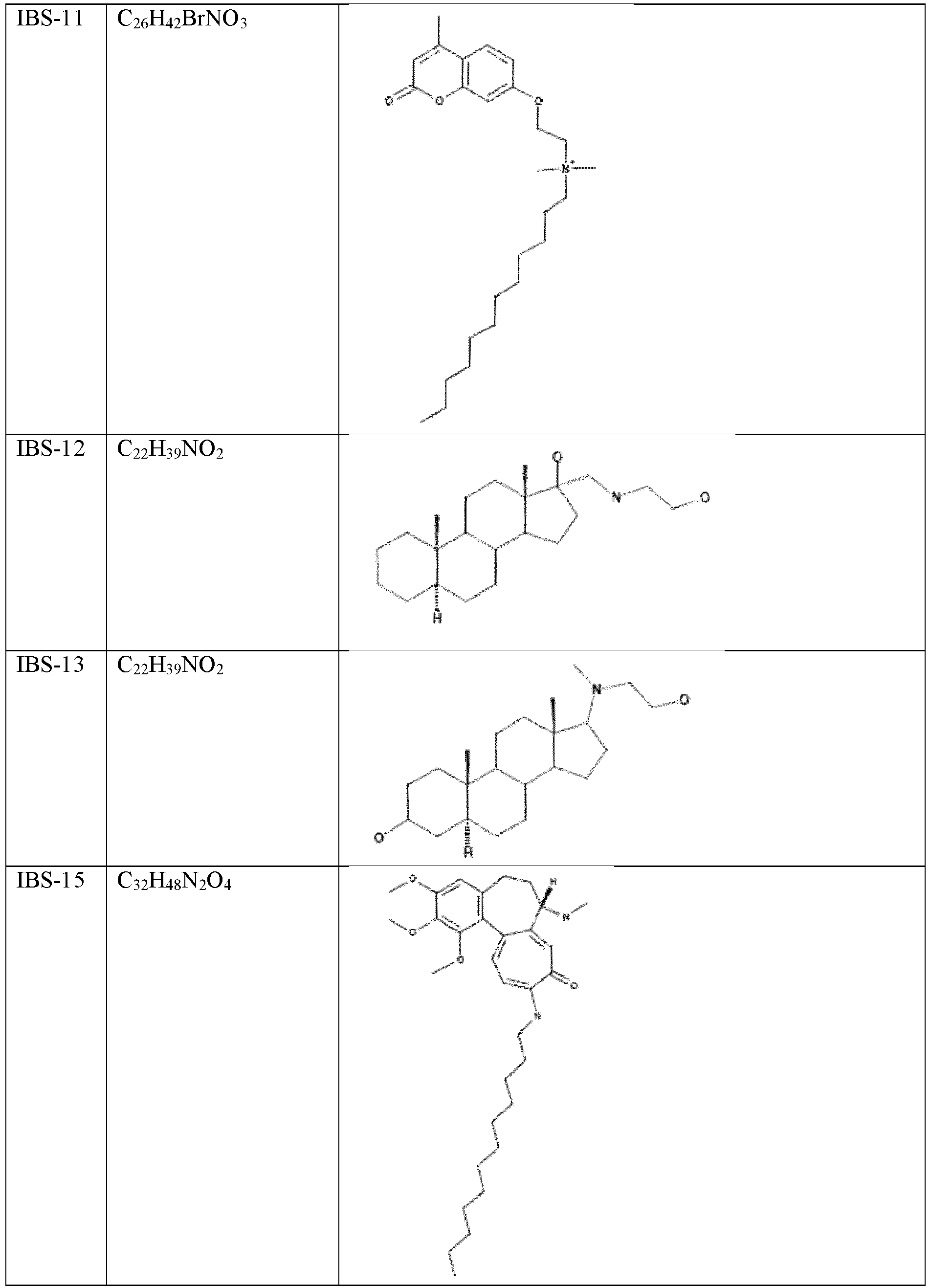

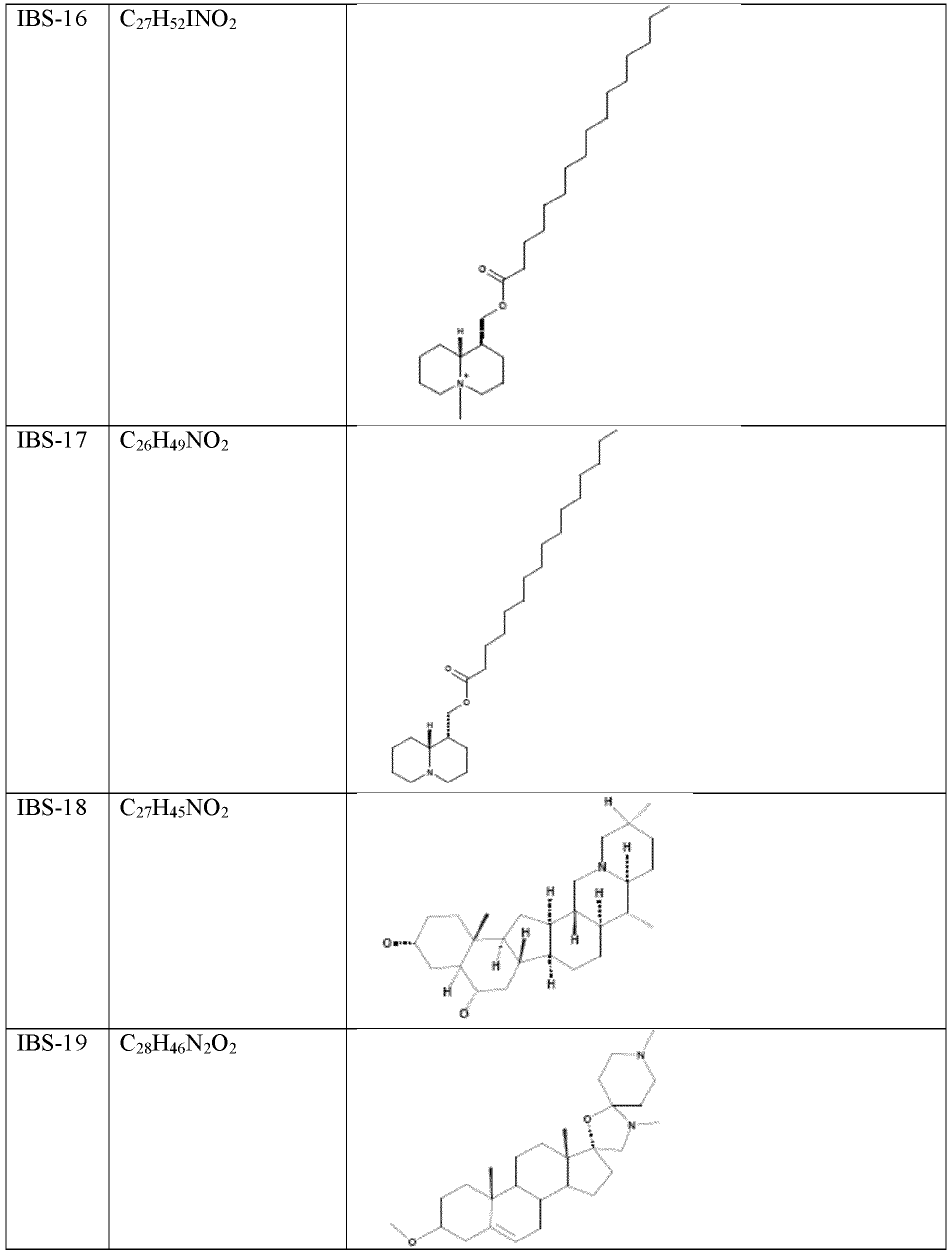

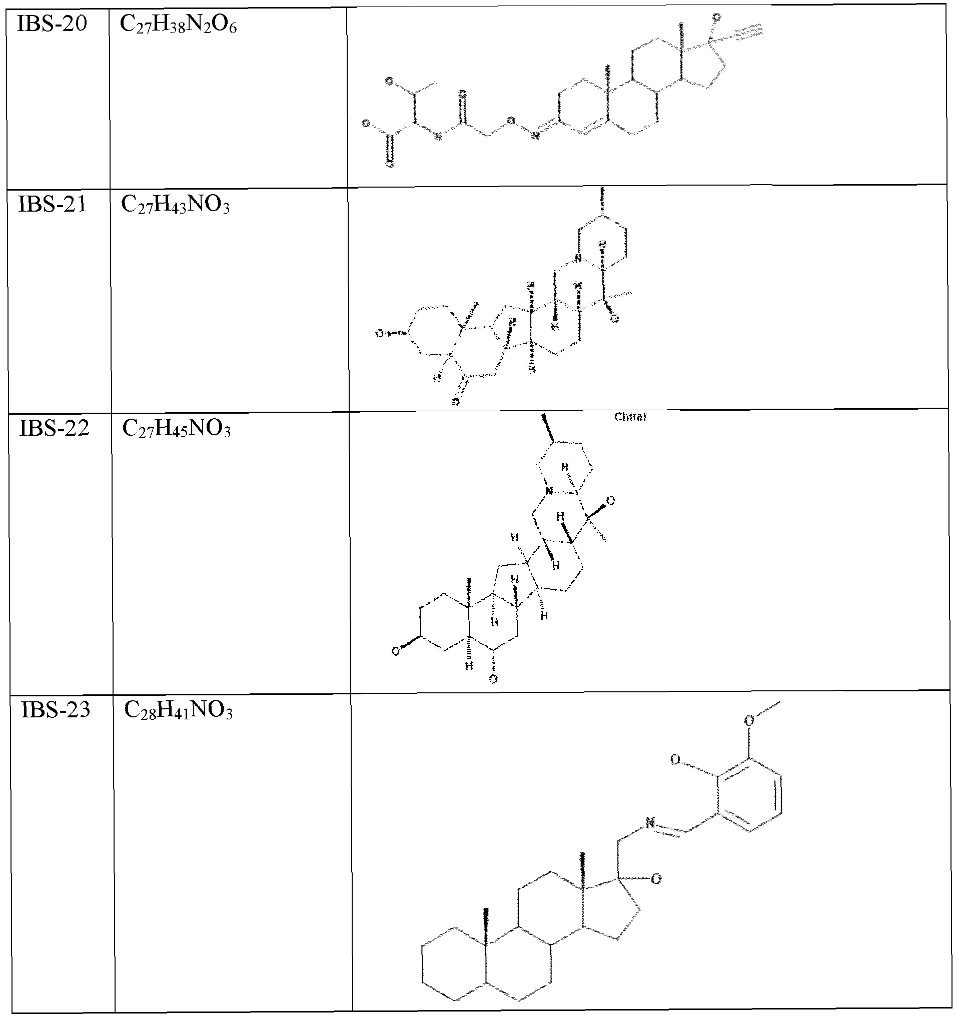

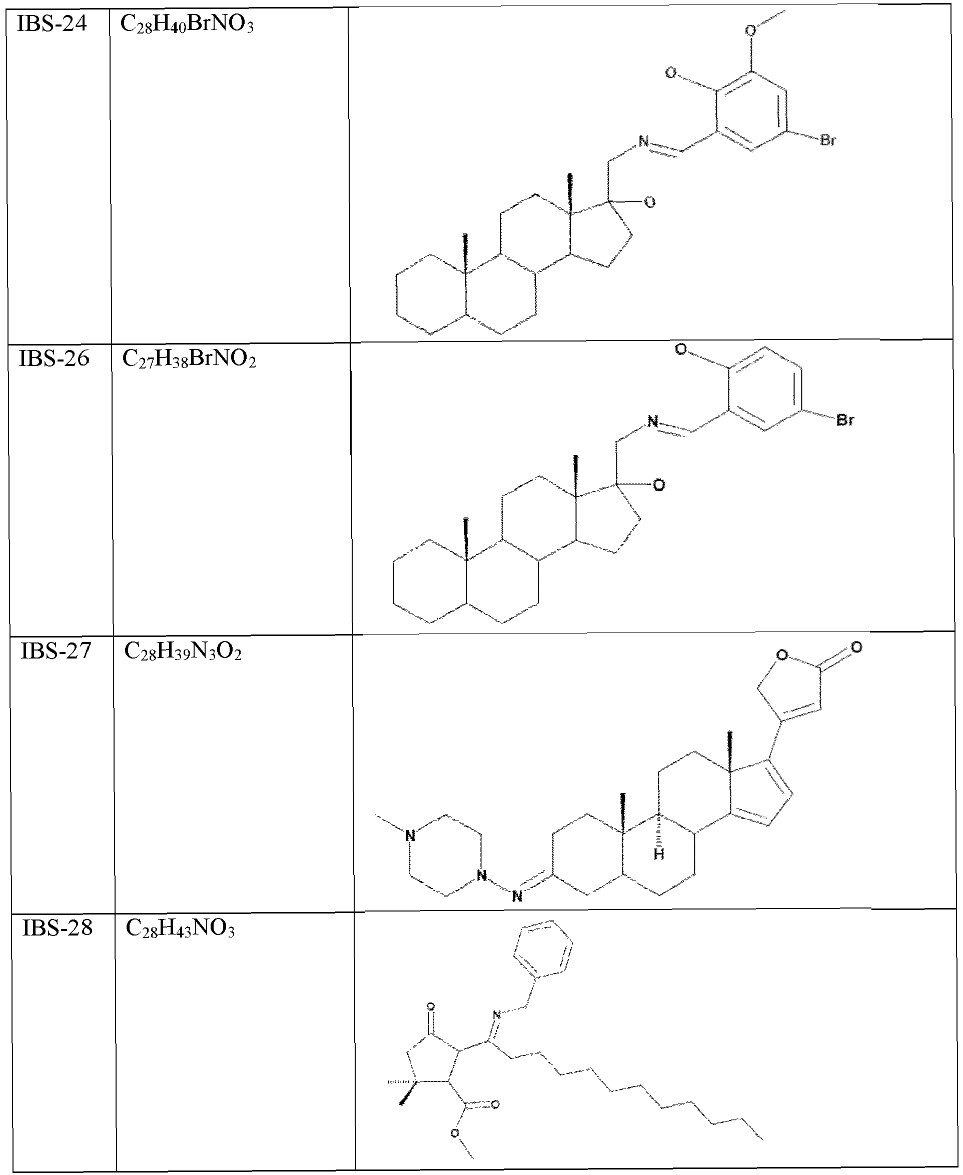

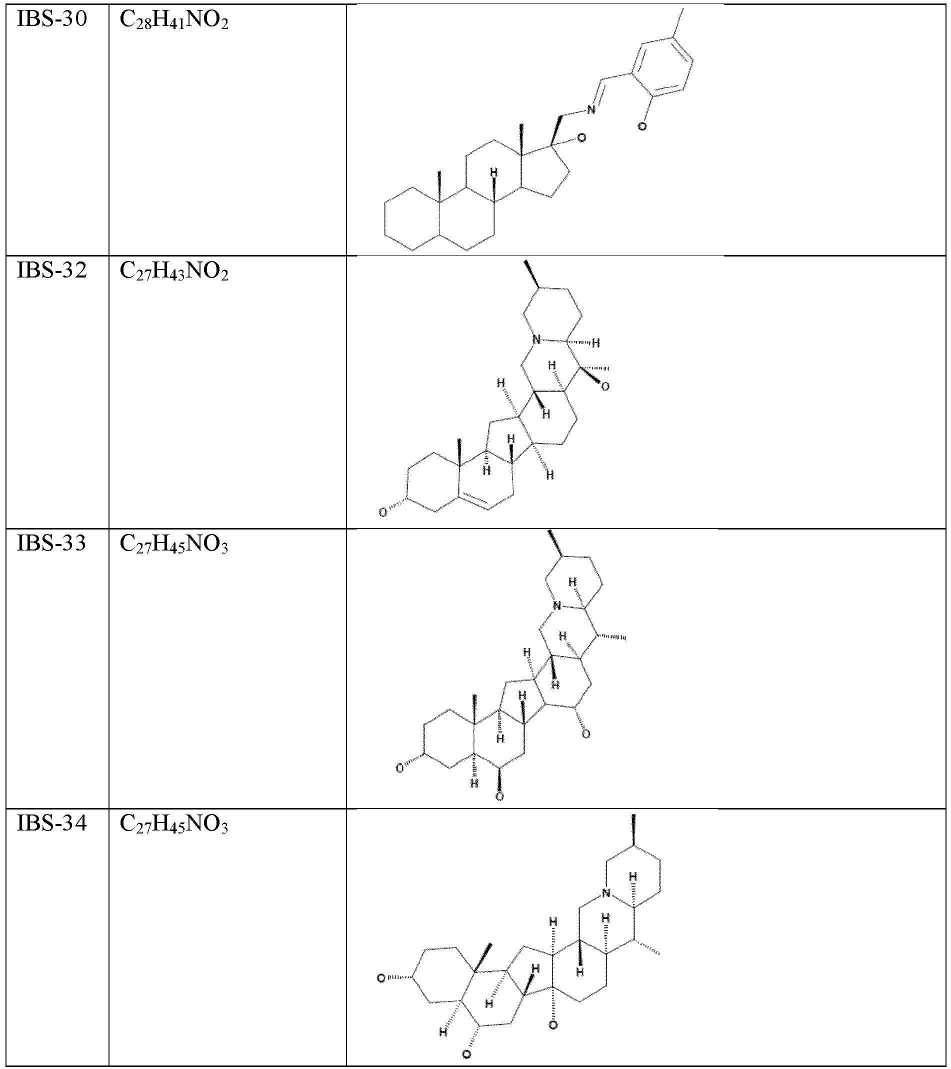

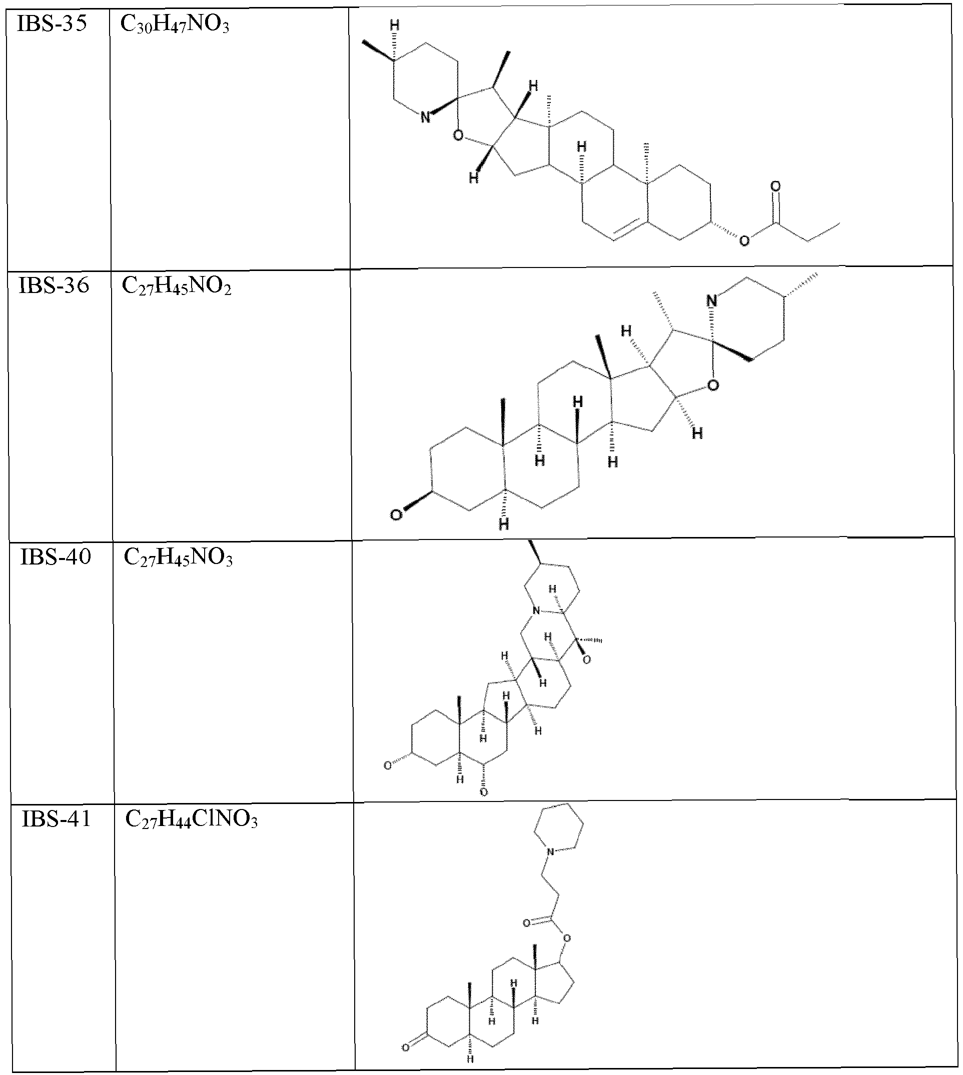

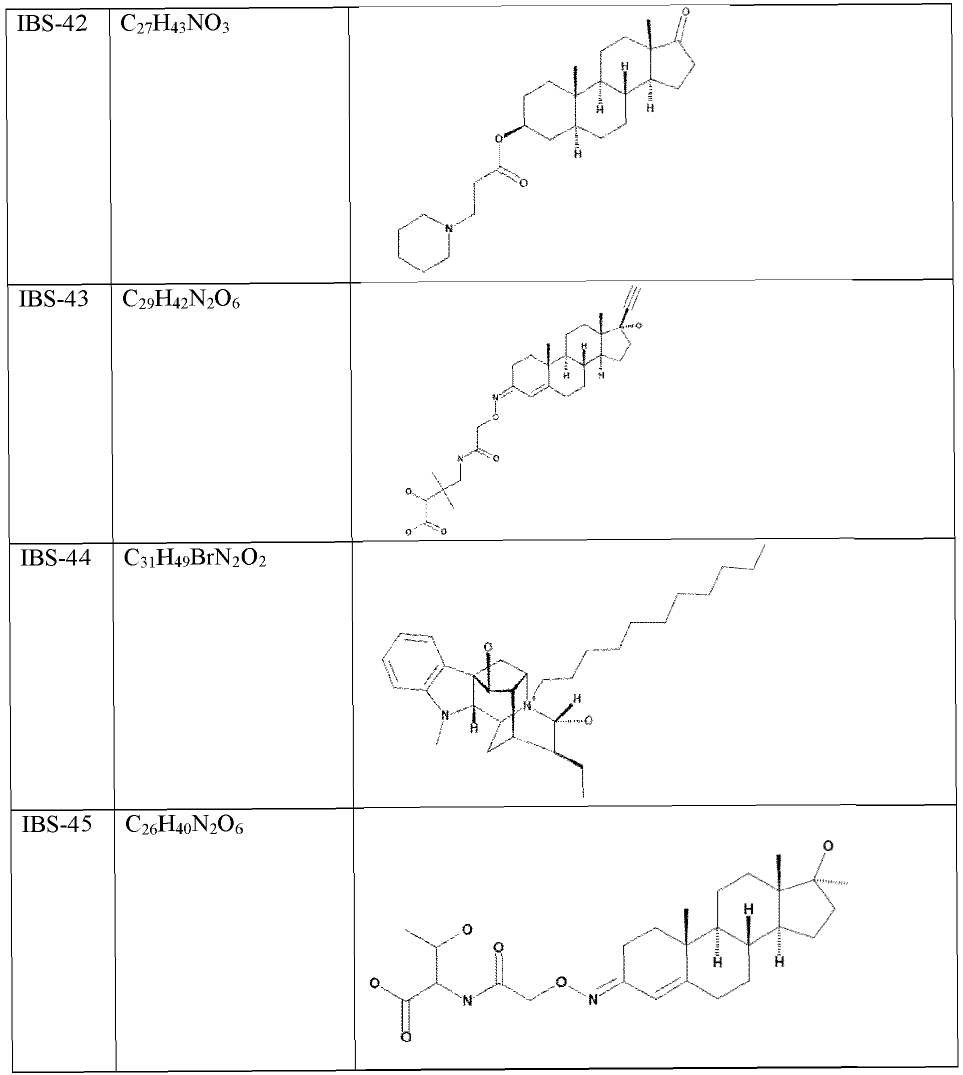

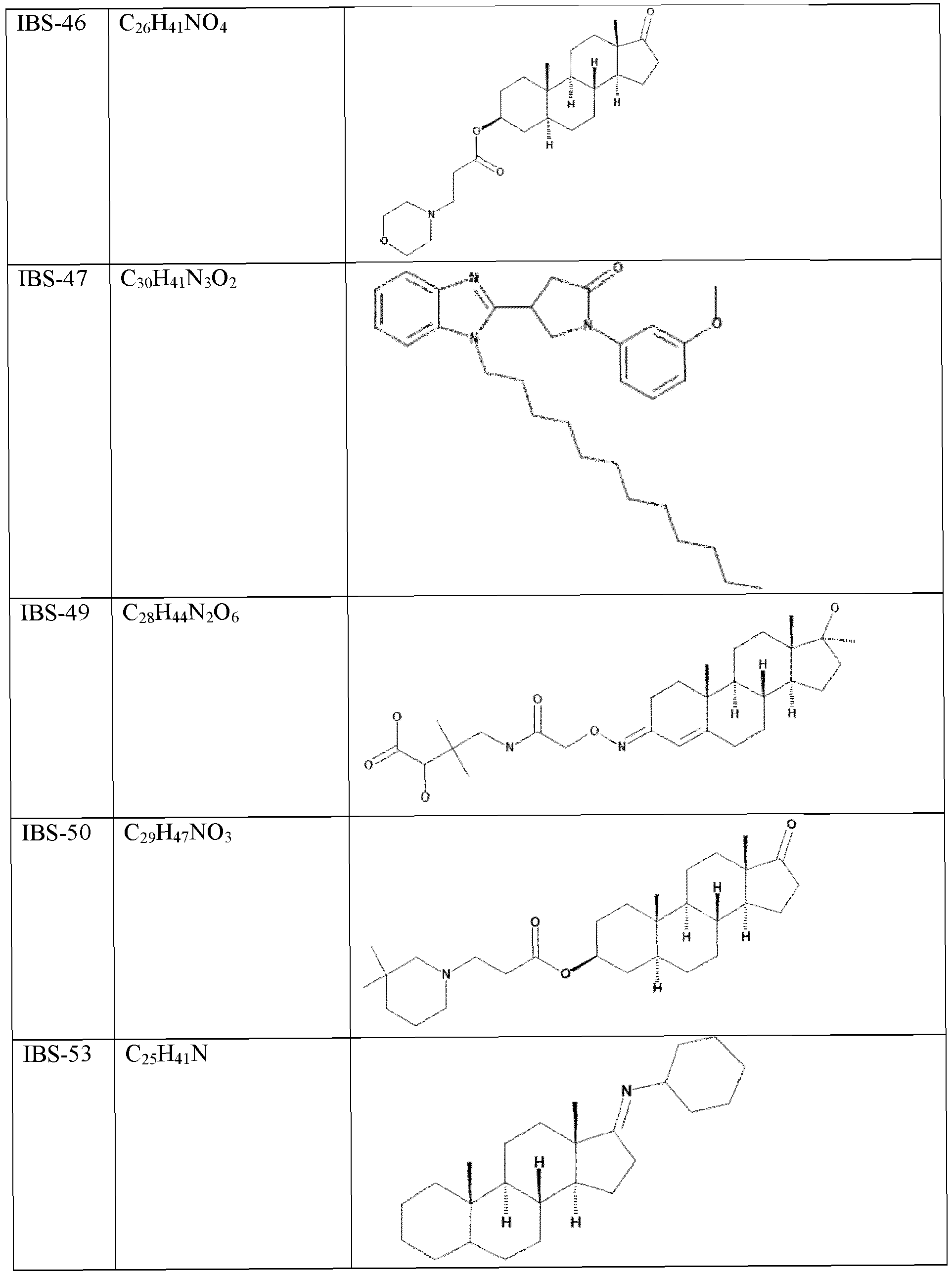

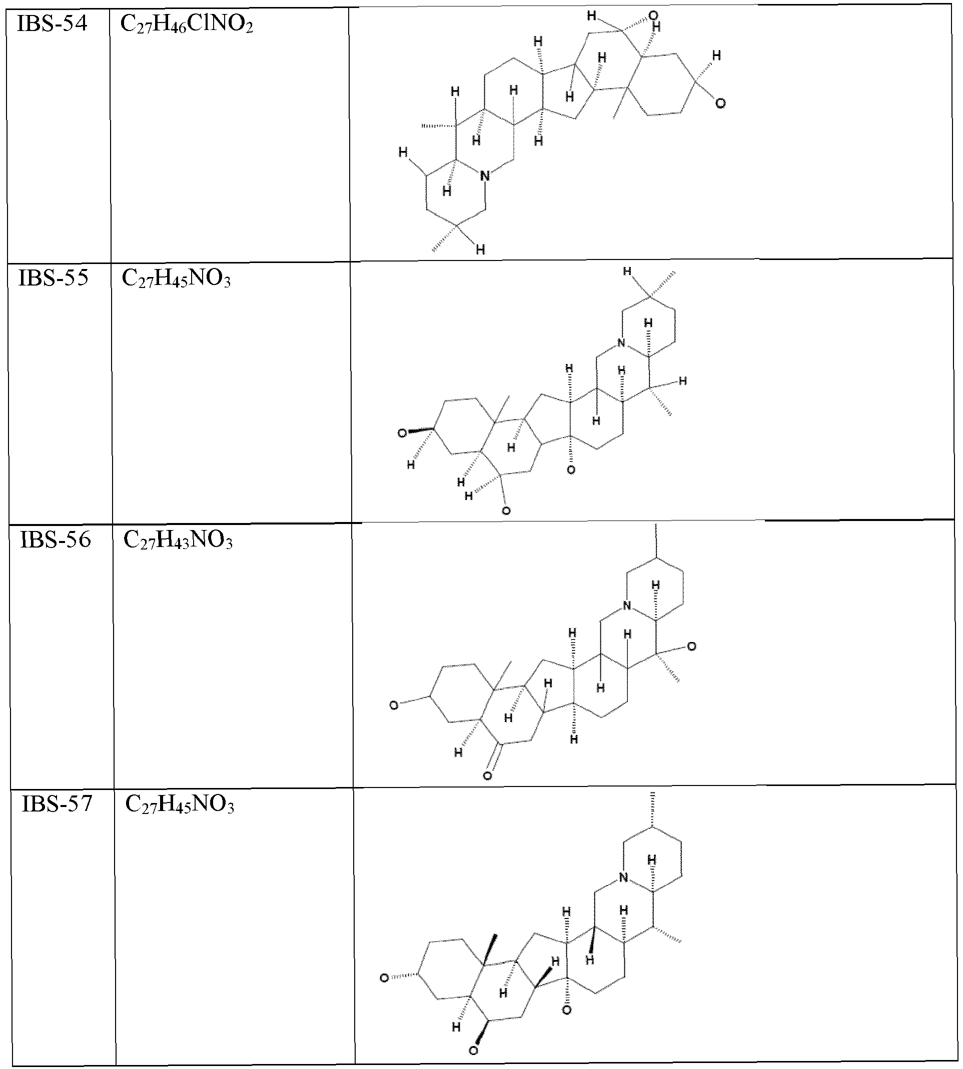

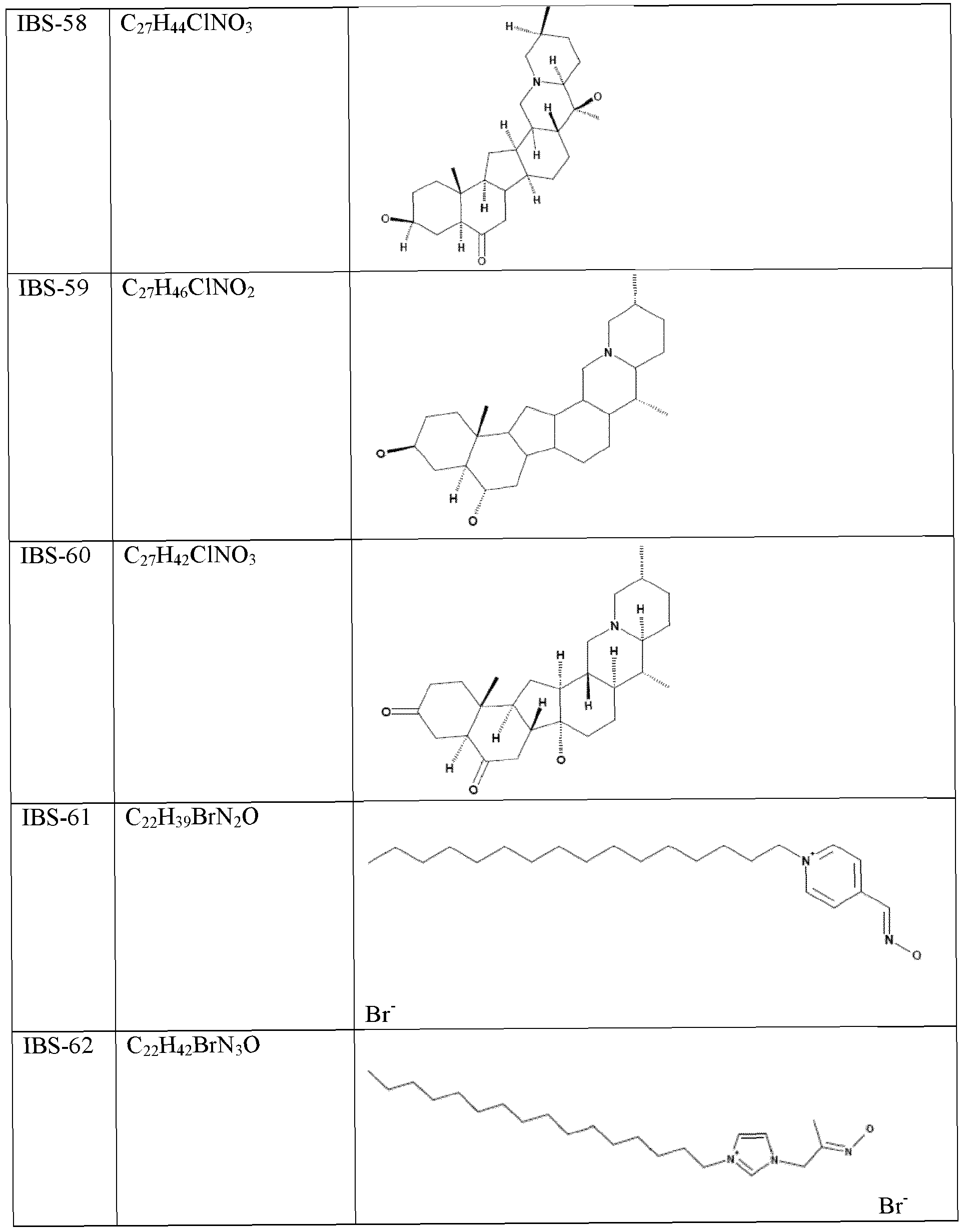

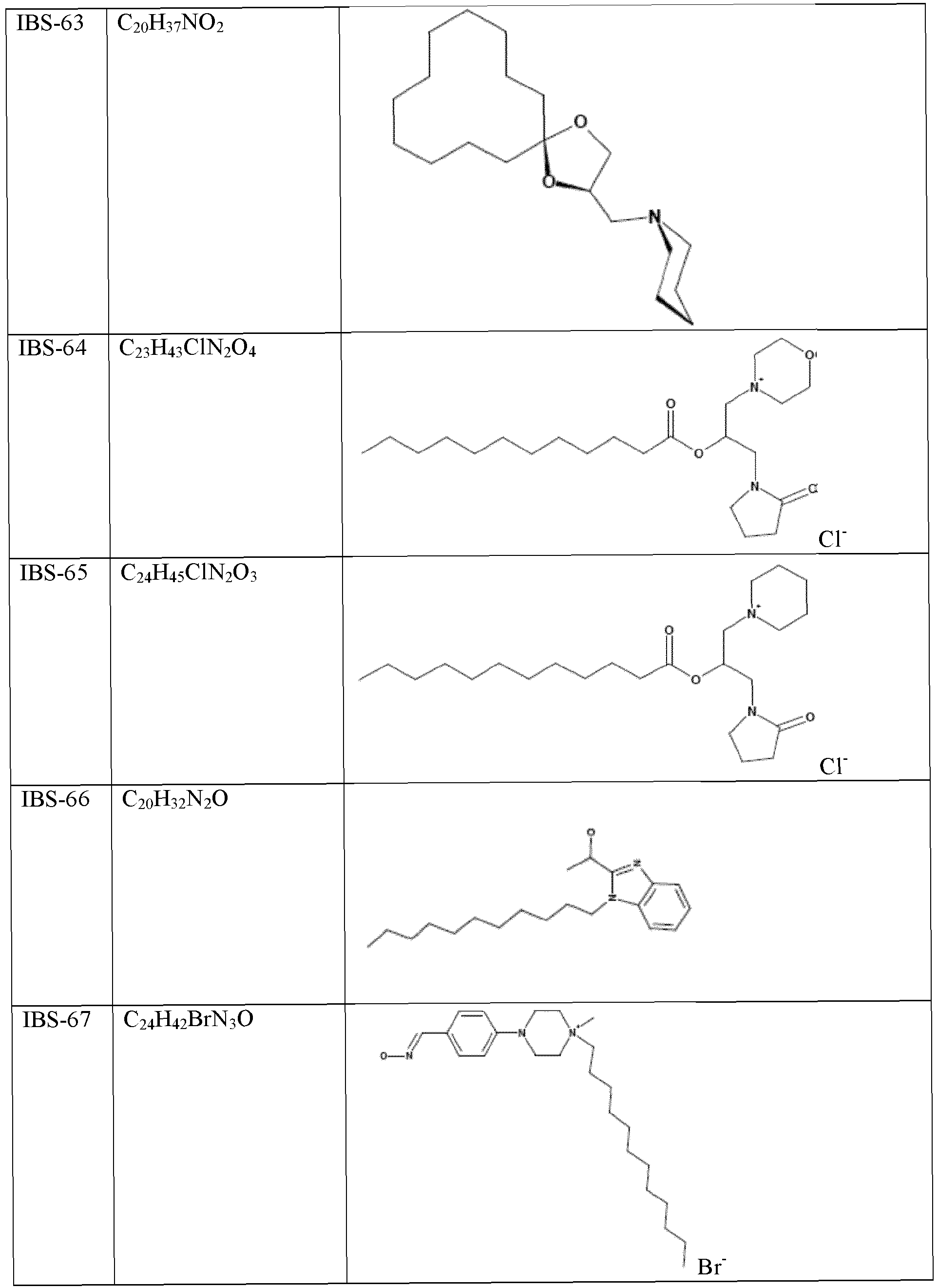

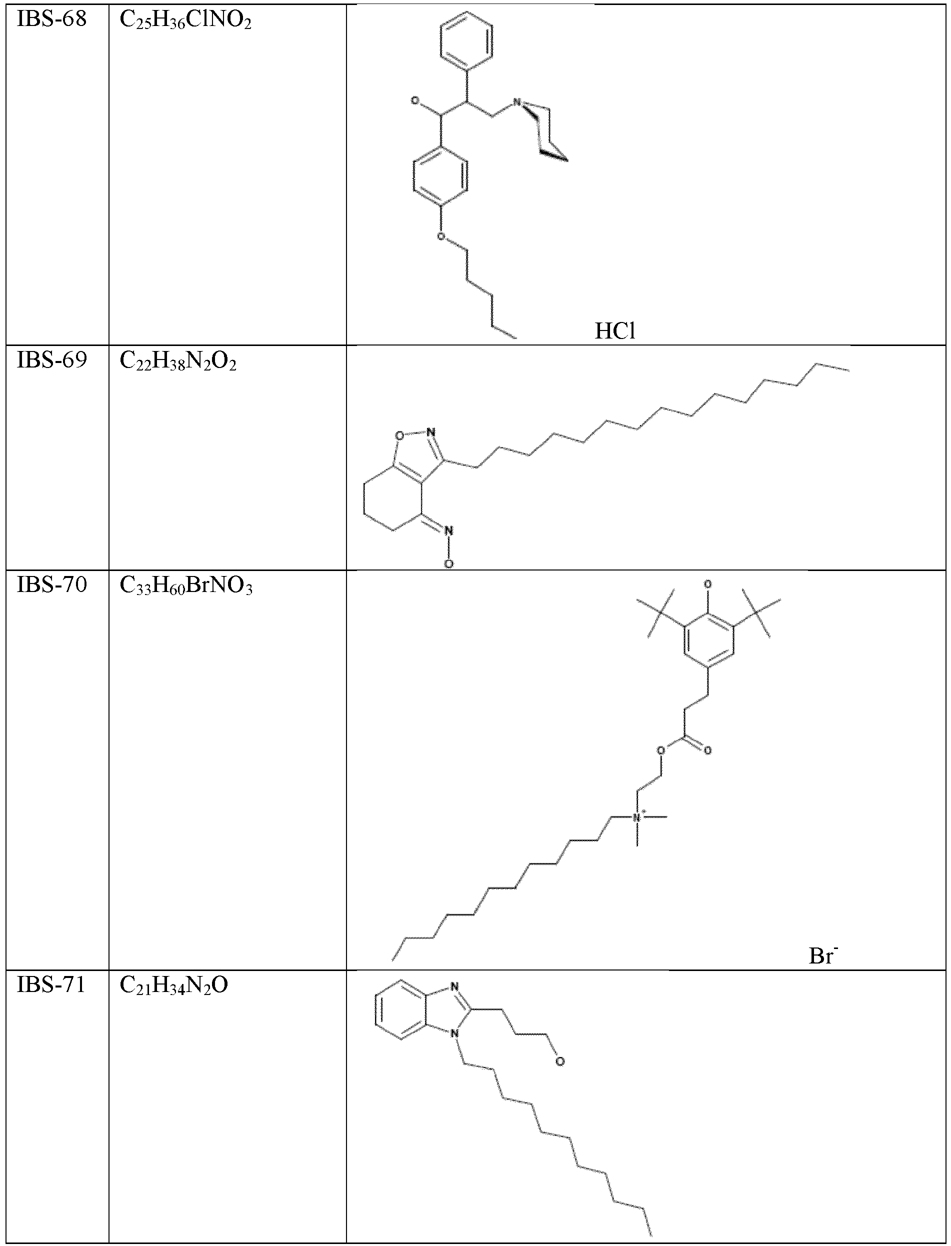

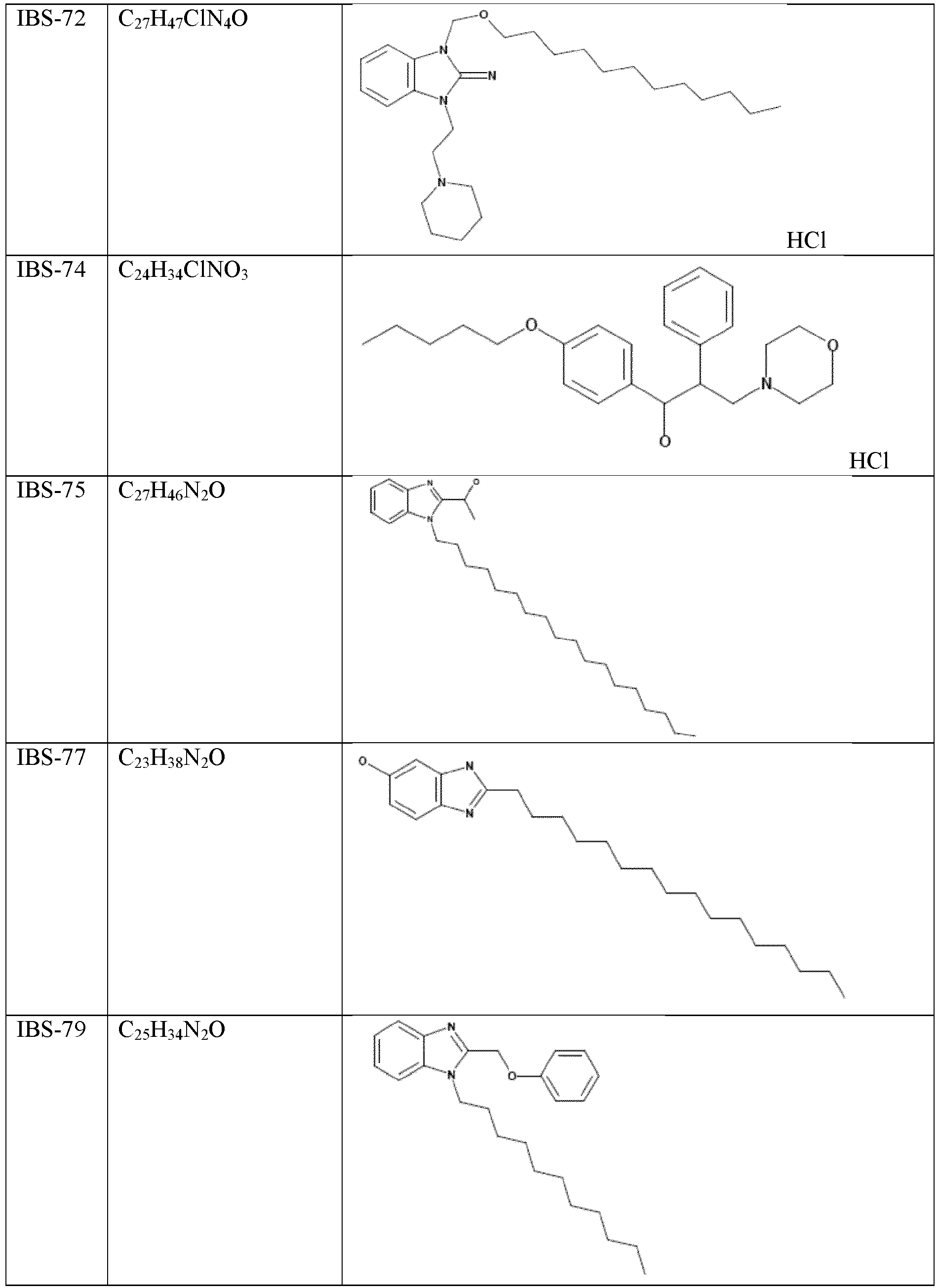

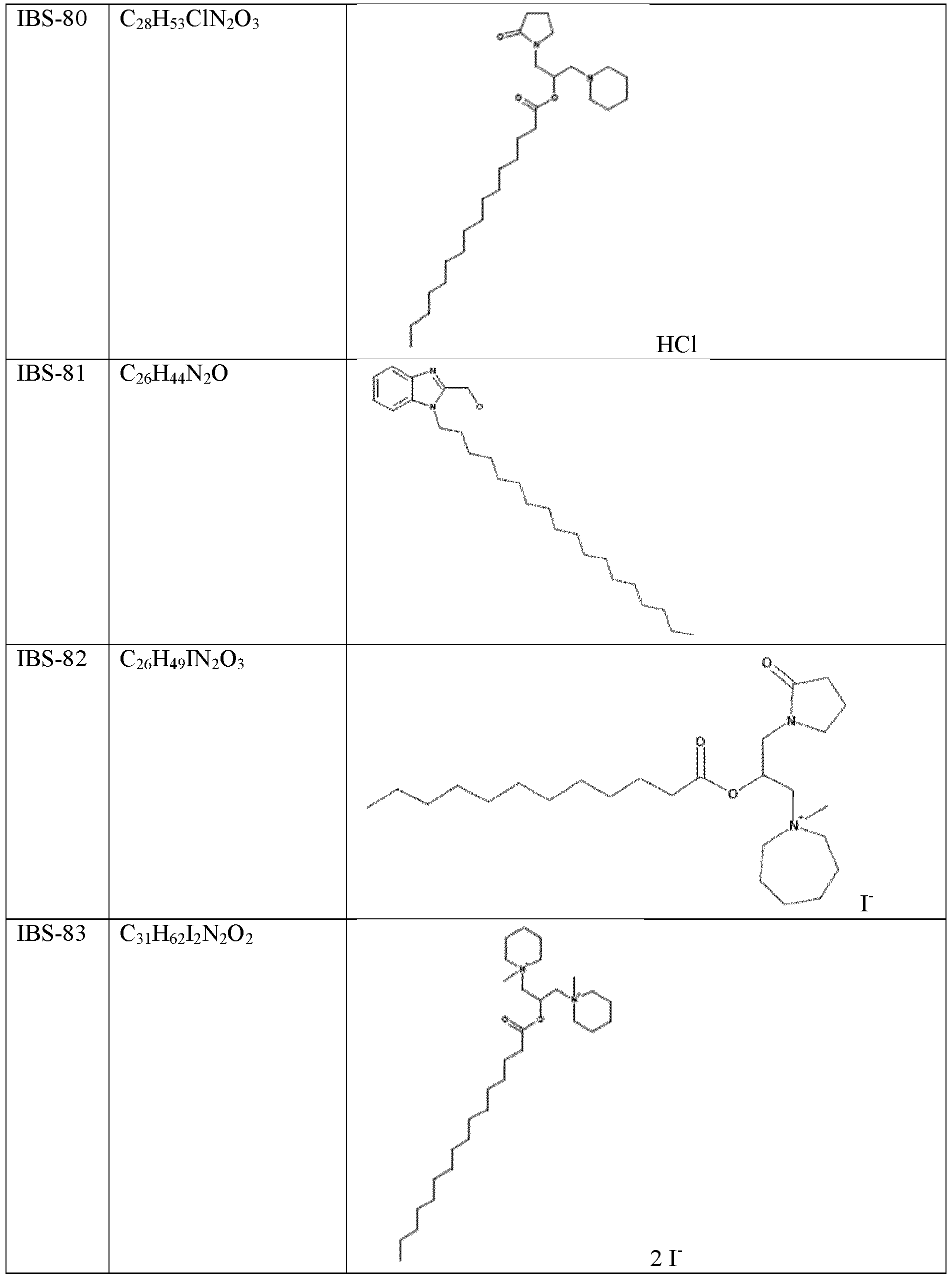

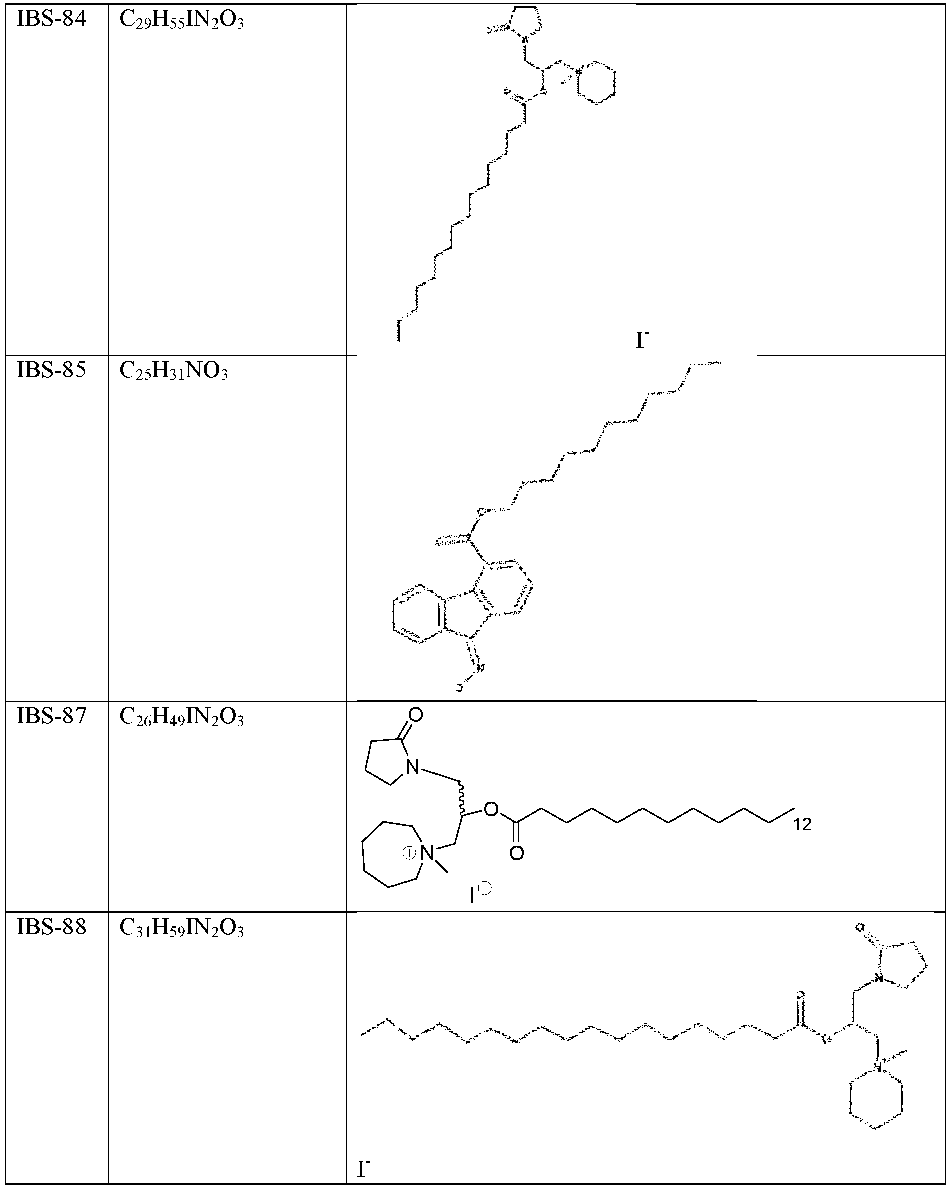

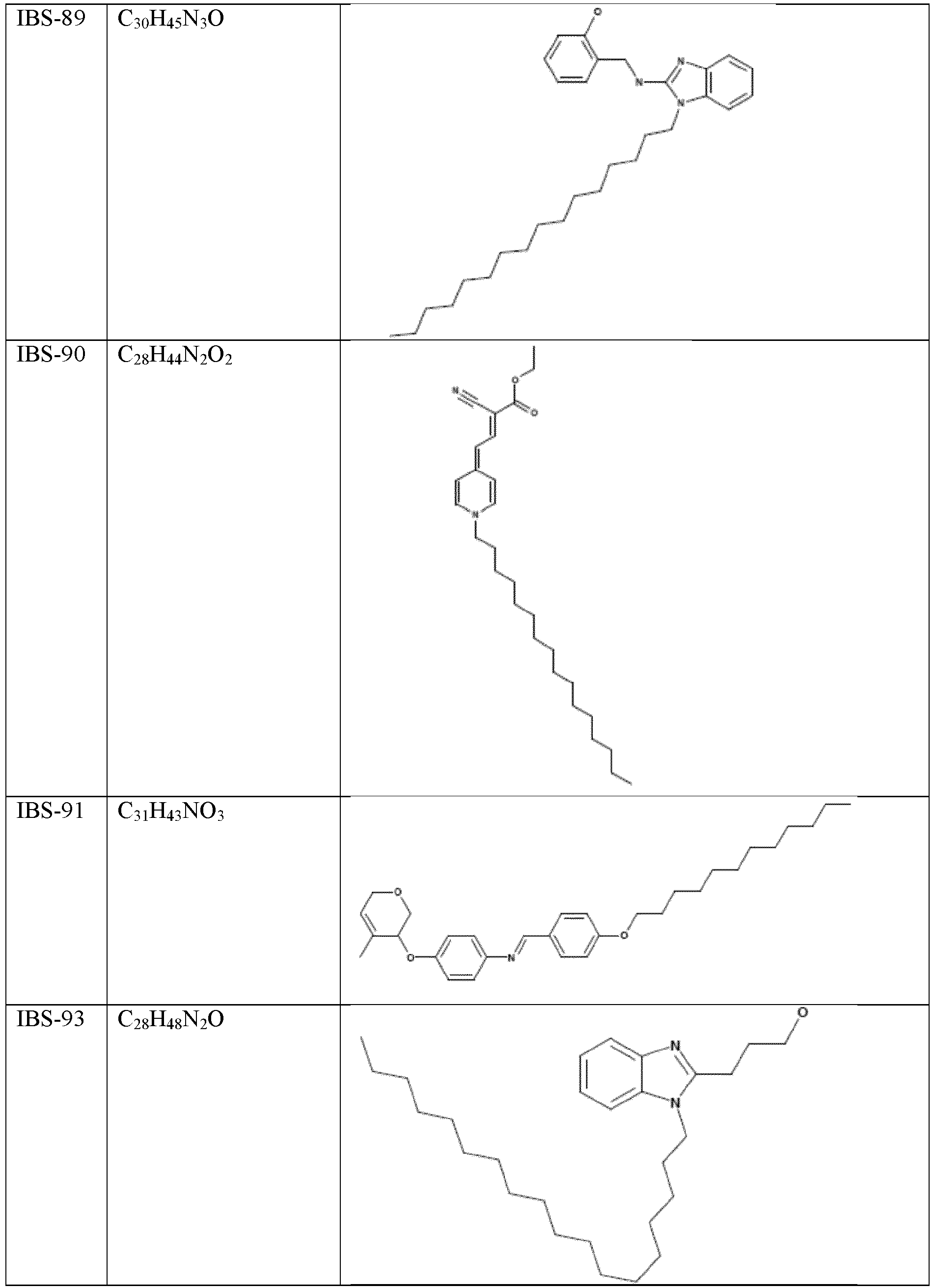

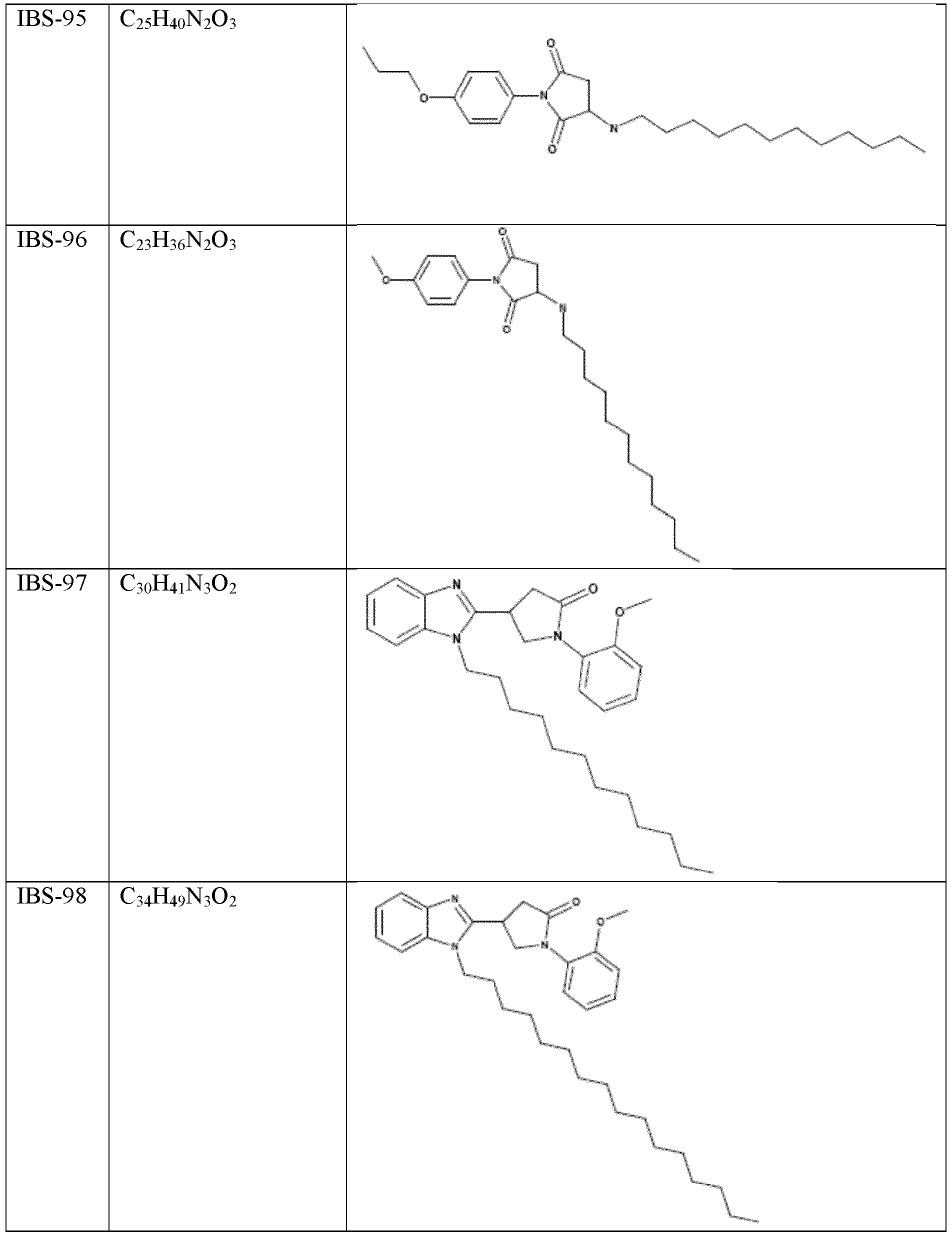

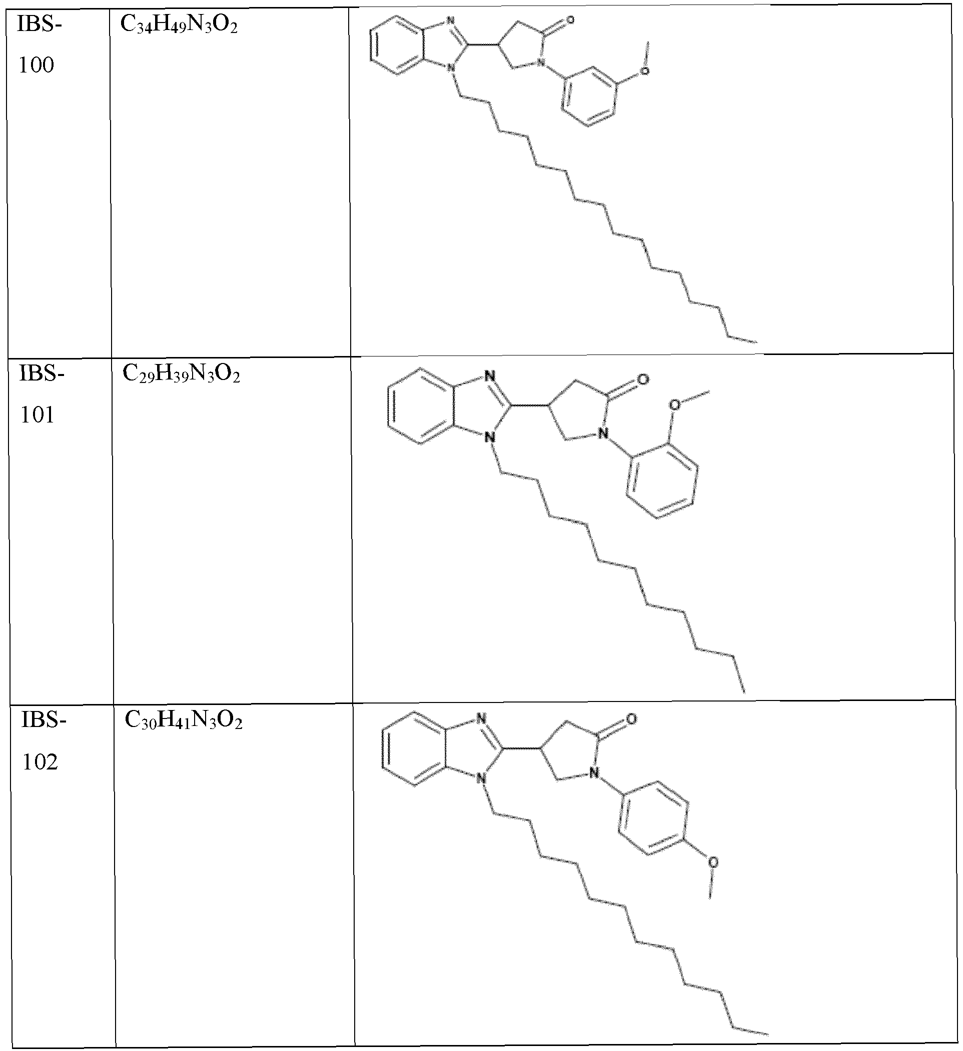

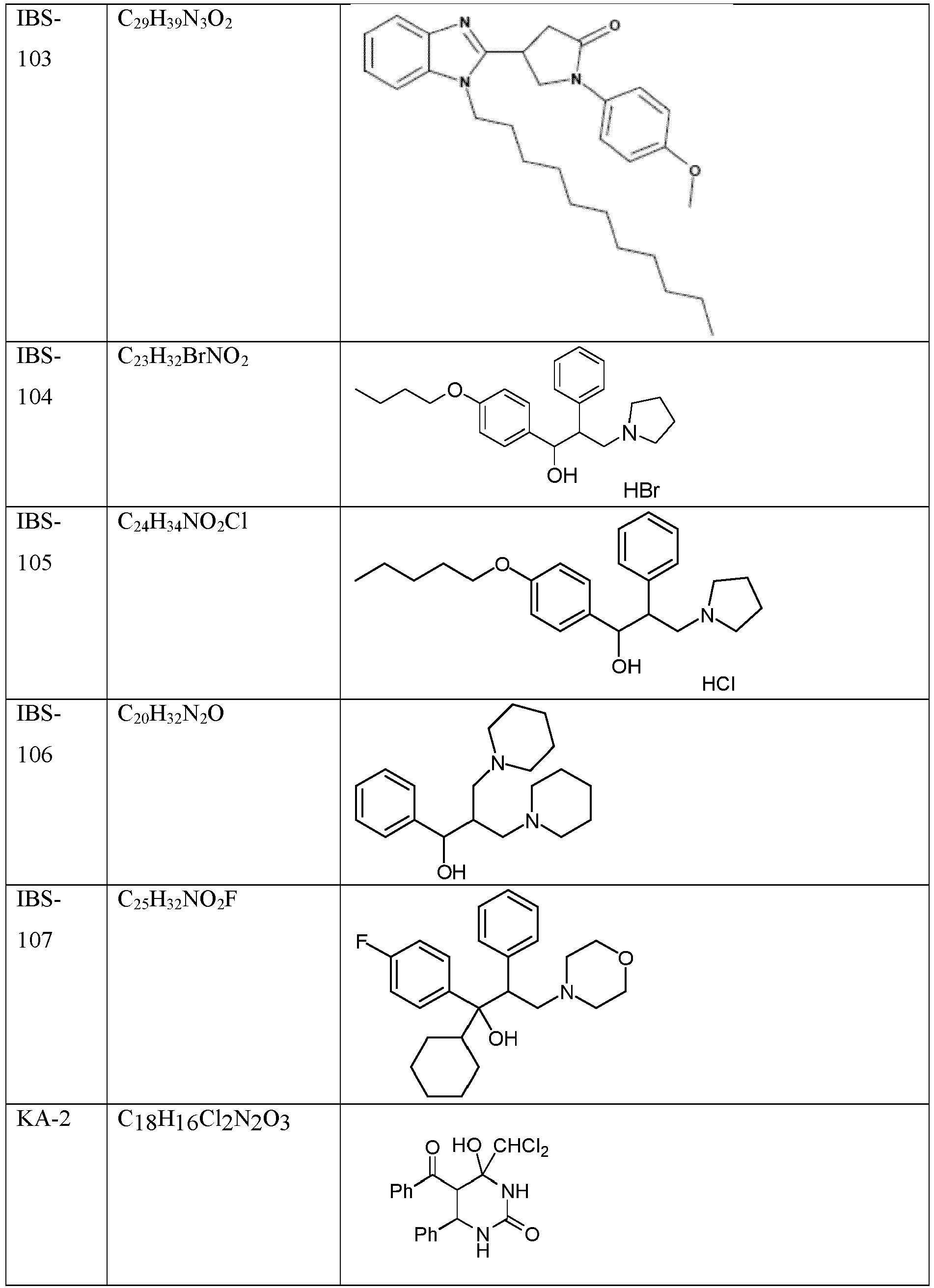

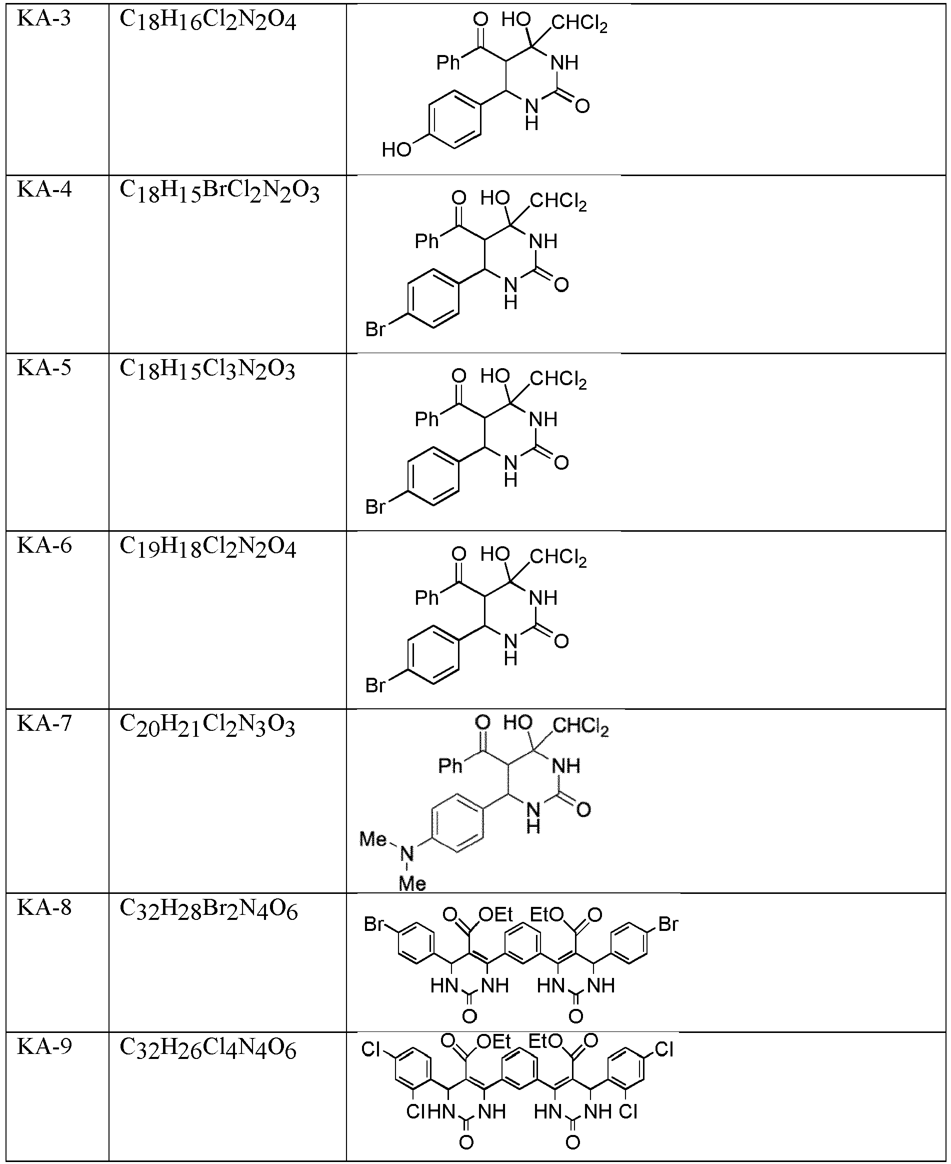

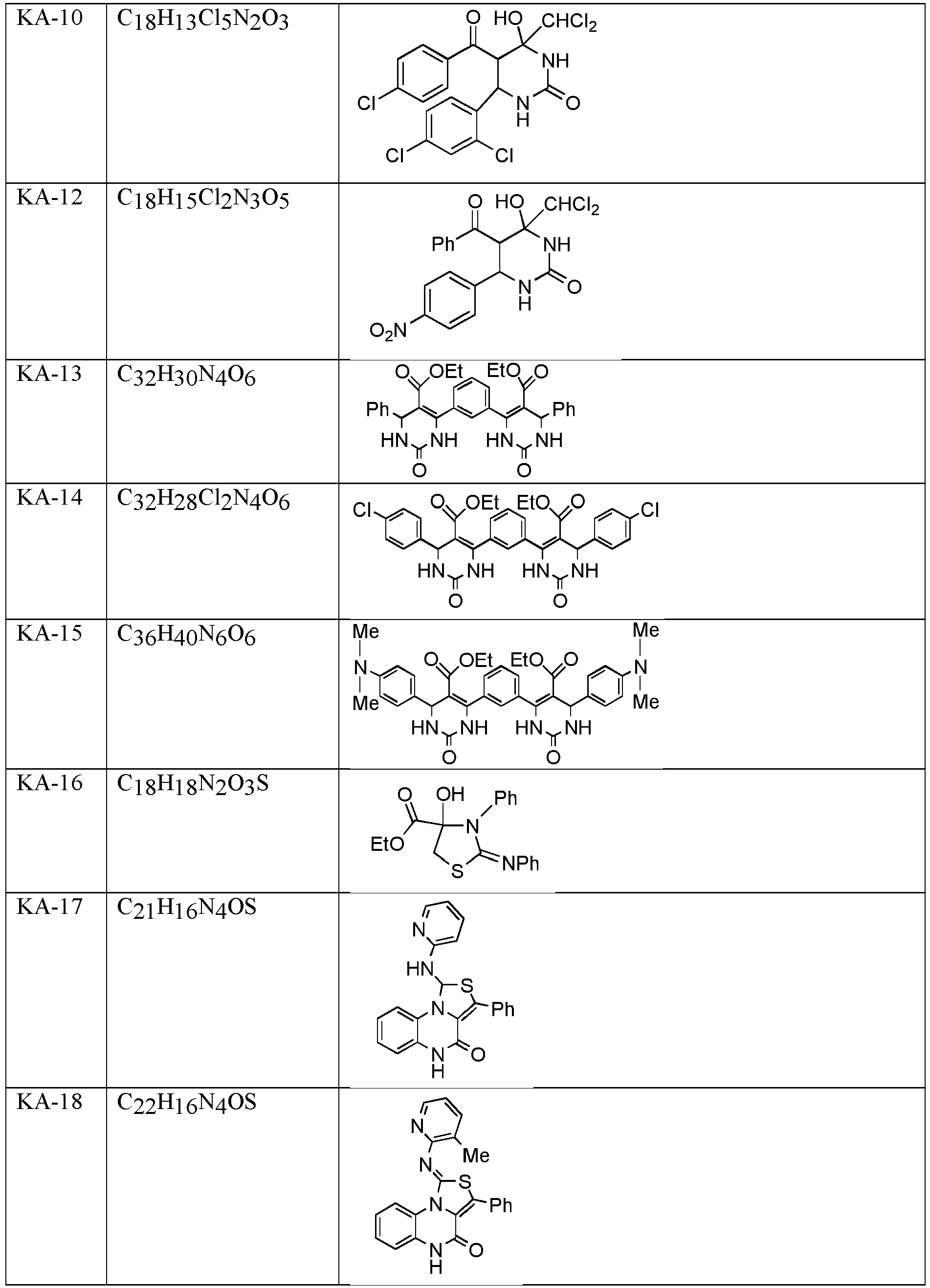

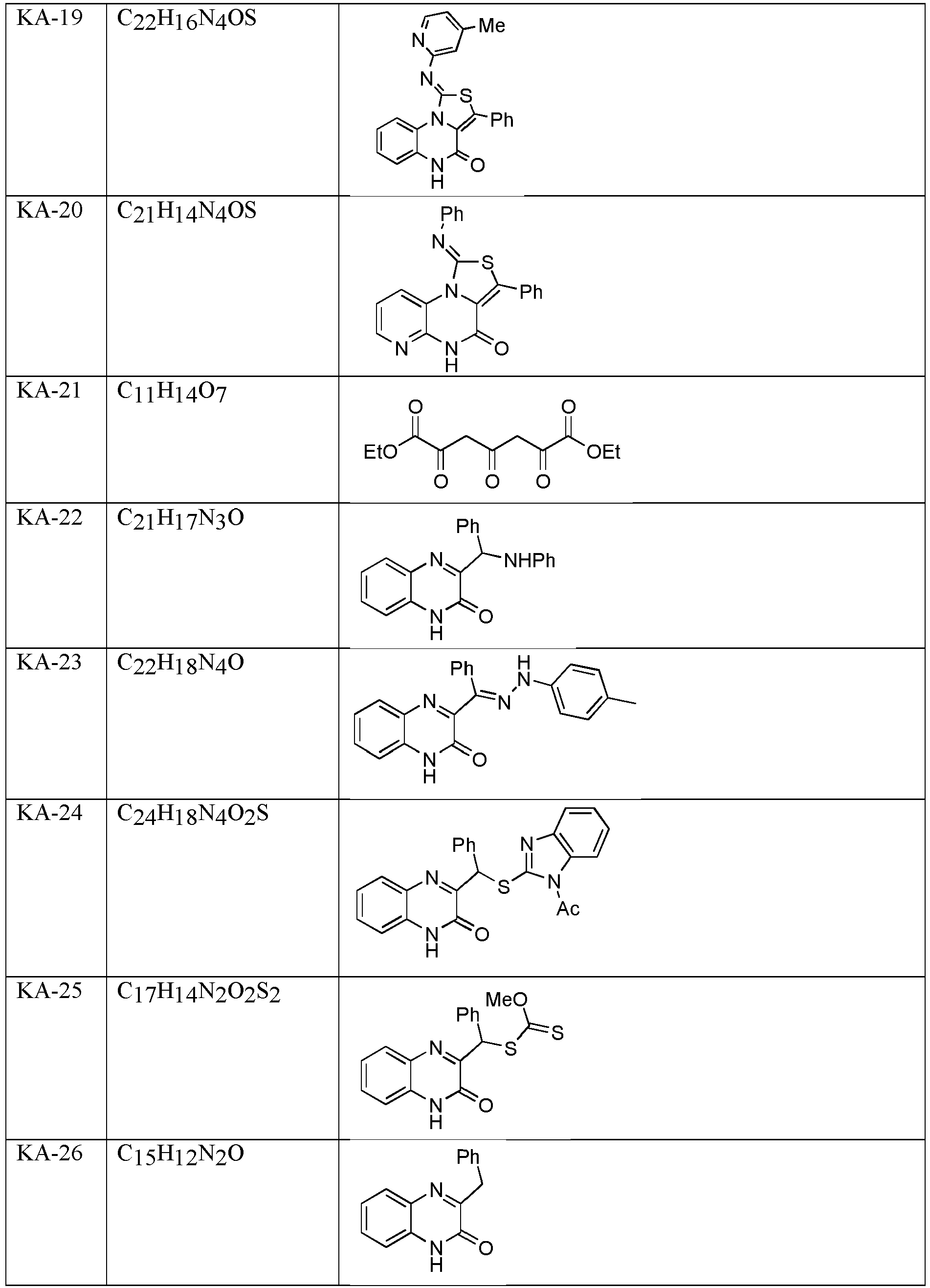

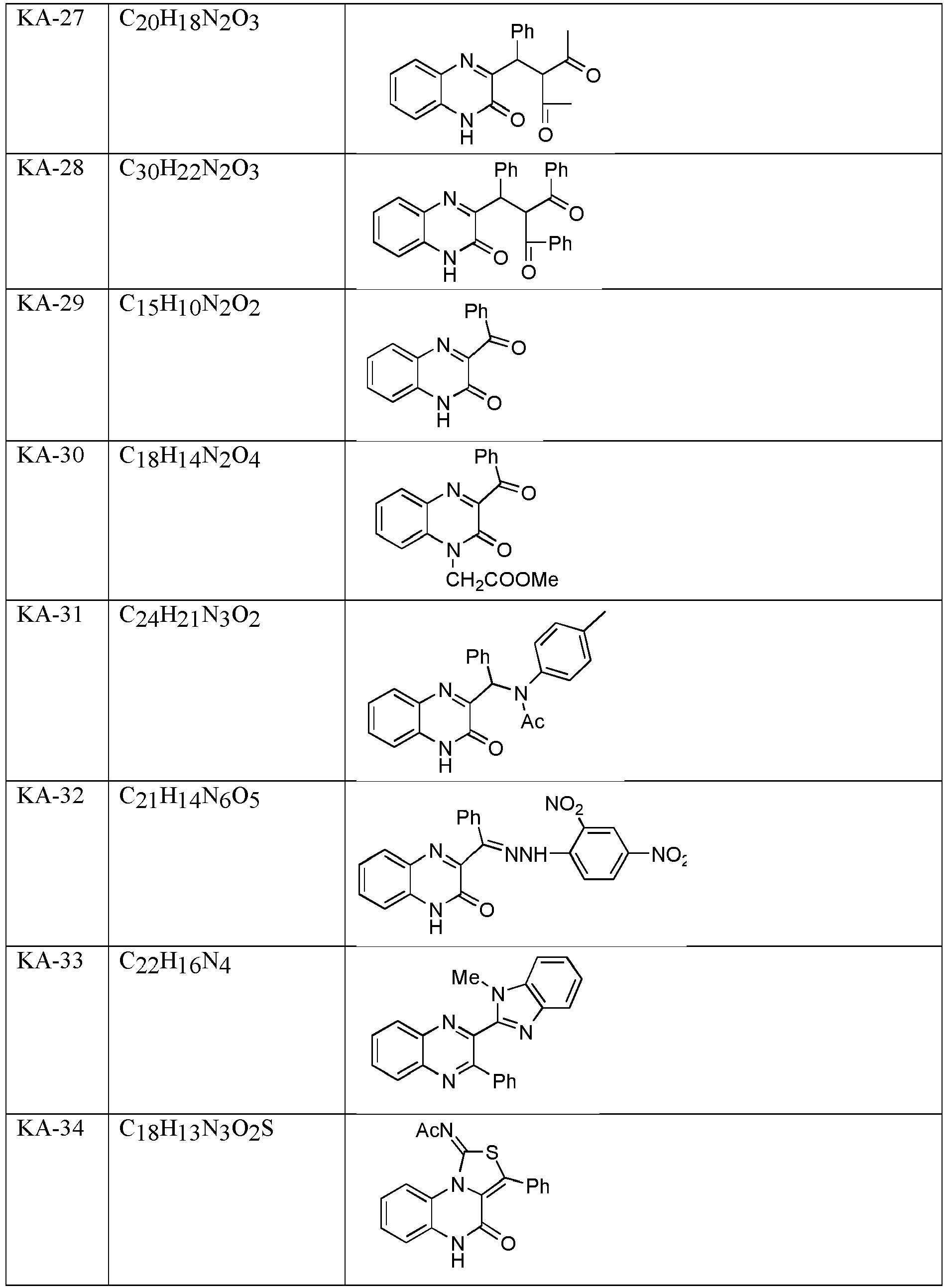

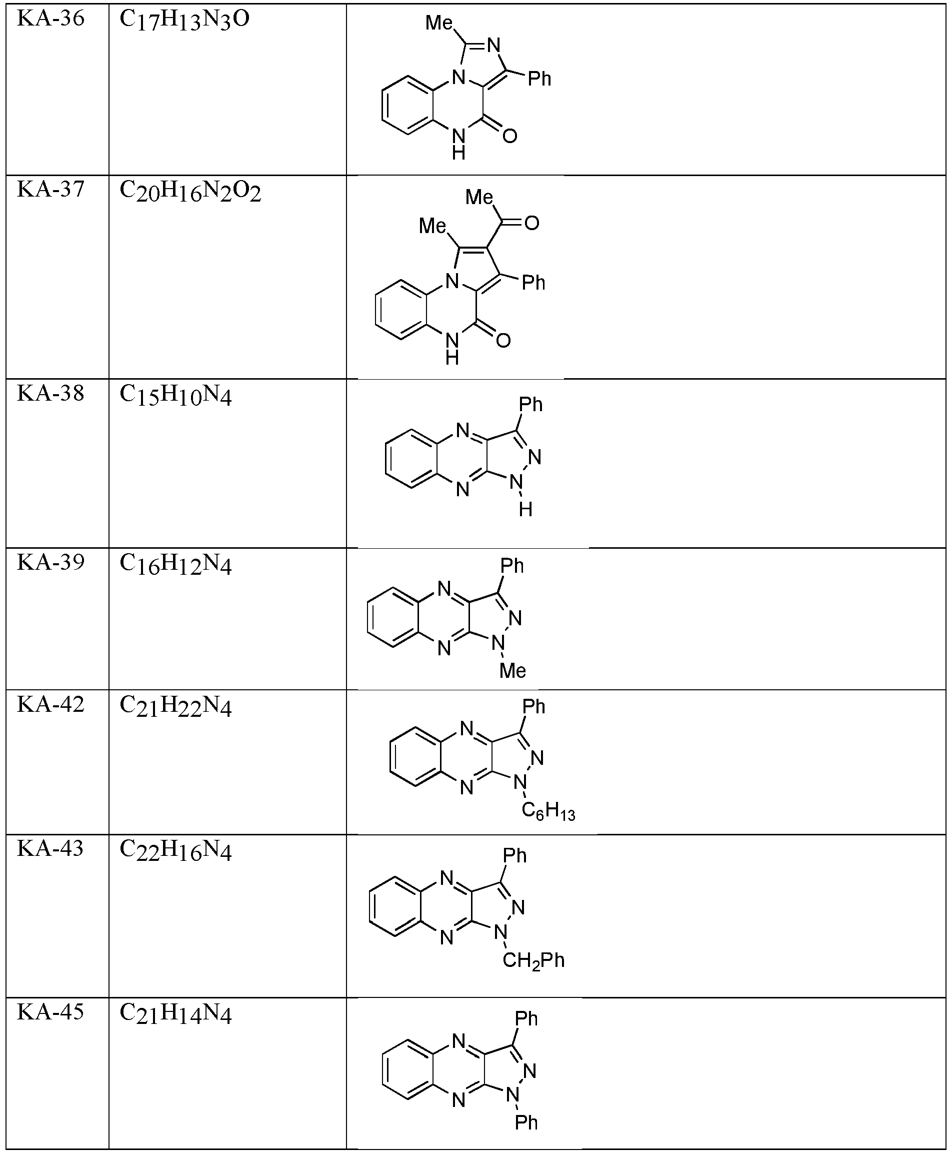

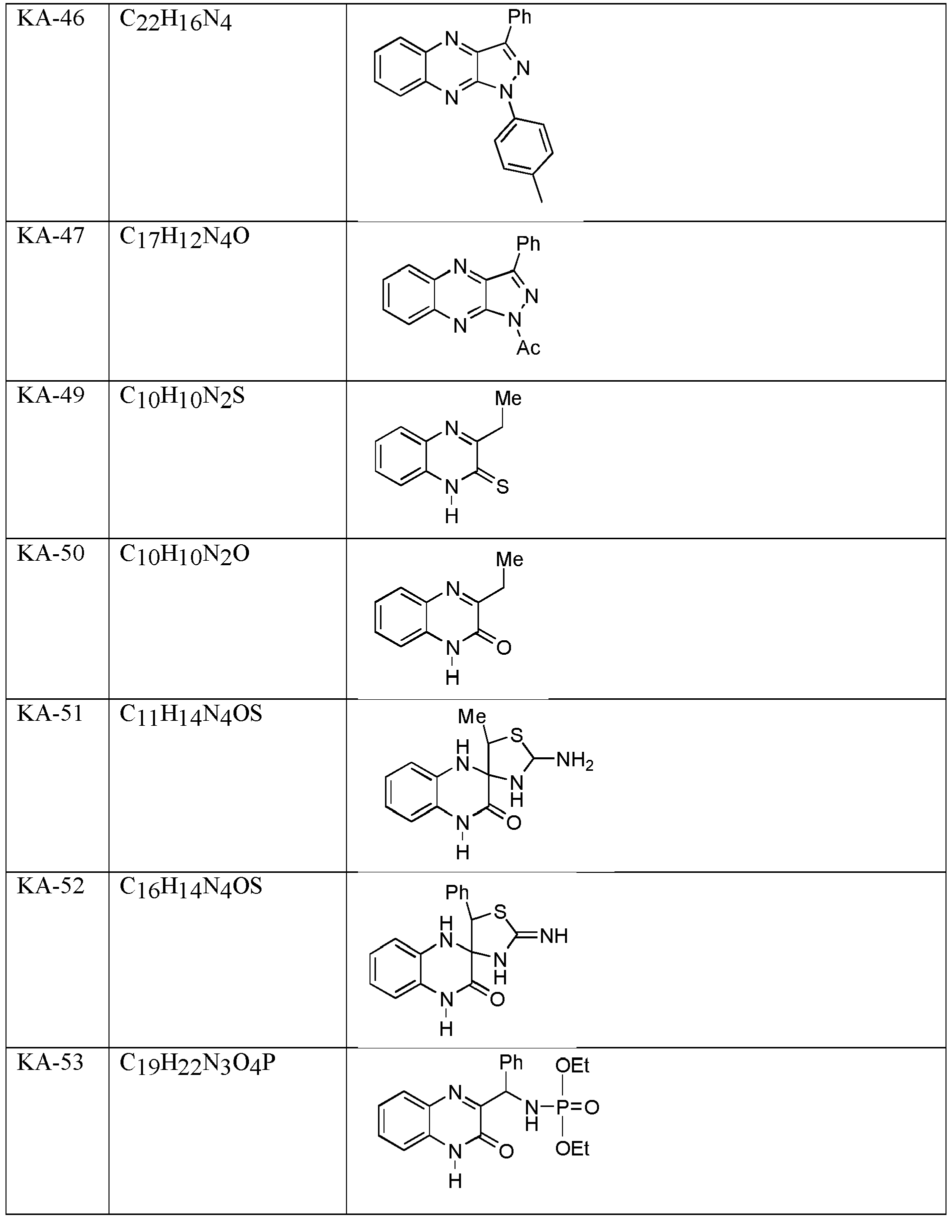

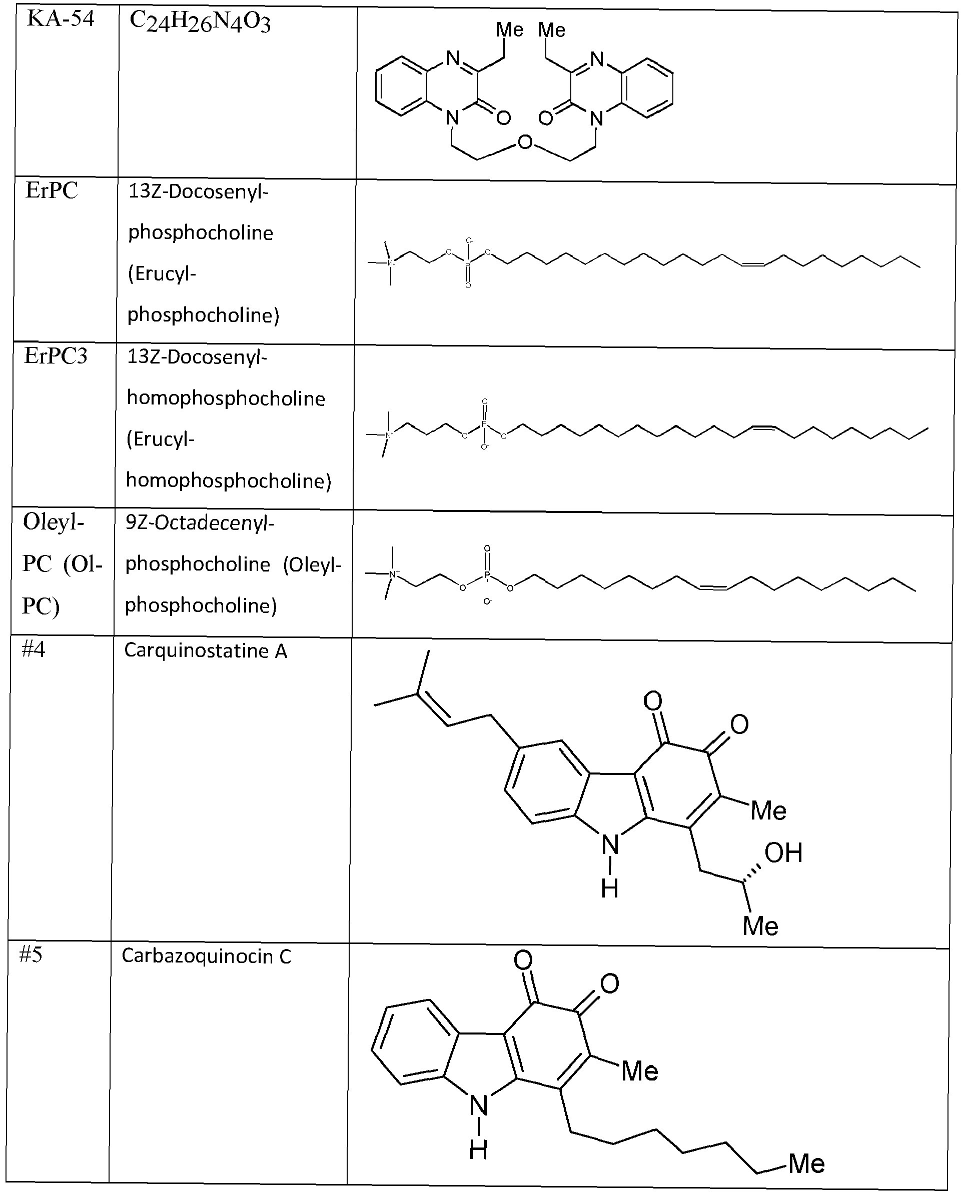

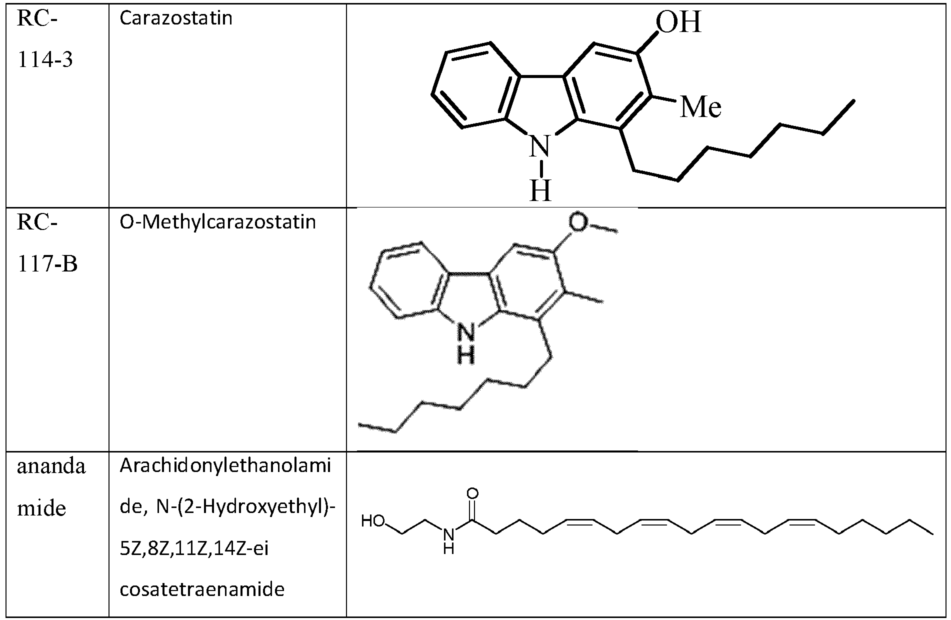

In some aspects of the disclosure, the miltefosine analog comprises, consists of, or consists essentially of any of the compounds shown in Table 2.1. In some embodiments, the miltefosine analog comprises, consists of, or consists essentially of a compound selected from Table 2.1. In some aspects of the disclosure, the miltefosine analog comprises, consists of, or consists essentially of any of the compounds shown in Table 2.1 or Table 2.2. In some aspects of the disclosure, the miltefosine analog comprises, consists of, or consists essentially of a compound selected from Table 2.1 or Table 2.2. In some aspects of the disclosure, the miltefosine analog comprises, consists of, or consists essentially of any of the compounds shown in Table 2.2. In some aspects of the disclosure, the miltefosine analog comprises, consists of, or consists essentially of a compound selected from Table 2.2. In some aspects of the disclosure, the miltefosine analog comprises, consists of, or consists essentially of a compound of formula V.

All modes of administration of miltefosine and miltefosine analogs (the latter forming part of the present disclosure) are contemplated, for example, orally, rectally, parenterally, topically, or by intravenous, intramuscular, intrasternal or subcutaneous injection or in a form suitable by inhalation. The formulations may, where appropriate, be conveniently presented in discrete dosage units and may be prepared by any of the methods well known in the art of pharmacy. The active ingredients will ordinarily be formulated with one or more pharmaceutically acceptable excipients in accordance with known and established practice. Thus, the pharmaceutical composition can be formulated as a liquid, powder, elixir, injectable solution, suspension, suppository, etc.

Miltefosine can be suitable for oral or topical administration. As such, in some embodiments, miltefosine or a miltefosine analog (the latter forming part of the present disclosure) is administered orally, the latter forming part of the present disclosure. In some embodiments, miltefosine or a miltefosine analog (the latter forming part of the present disclosure) is administered topically, the latter forming part of the present disclosure. In some embodiments, miltefosine or a miltefosine analog (the latter forming part of the present disclosure) is administered by intravenous, intramuscular, intrasternal or subcutaneous injection or in a form suitable by inhalation, the latter forming part of the present disclosure.

In accordance with some embodiments herein, the specific amount of miltefosine or a miltefosine analog (the latter forming part of the present disclosure) administered to a subject will vary depending upon the disease or condition being treated, as well as the age, weight and sex of the patient being treated. In some embodiments, miltefosine or a miltefosine analog (the latter forming part of the present disclosure) is administered in a dosage of about 0.1 - 10 mg/kg body weight. In some embodiments, miltefosine or a miltefosine analog (the latter forming part of the present disclosure) is administered in a dosage of about 0.1 mg/kg body weight, 0.2 mg/kg body weight, 0.3 mg/kg body weight, 0.5 mg/kg body weight, 1 mg/kg body weight, 2 mg/kg body weight, 3 mg/kg body weight, 4 mg/kg body weight, 5 mg/kg body weight, 6 mg/kg body weight, 7 mg/kg body weight, 8 mg/kg body weight, 9 mg/kg body weight, including ranges between any two of the listed values, for example about 0.2 - 0.5 mg/kg body weight, 0.2 - 1 mg/kg body weight, 0.2 - 5 mg/kg body weight, 0.2 - 10 mg/kg body weight, 0.5 - 1 mg/kg body weight, 0.5 - 3 mg/kg body weight, 0.5 - 5 mg/kg body weight, 0.5 - 10 mg/kg body weight, 1-3 mg/kg body weight, 1-5 mg/kg body weight, 1-10 mg/kg body weight, 2-3 mg/kg body weight, 2 -5 mg/kg body weight, 2-10 mg/kg body weight, or 5 - 10 mg/kg body weight. Preferably, this dosage is repeated as needed. Optionally, the dosage can be repeated five times daily, four times daily, three times daily, twice daily, once daily, or less frequently, for example once every 2, 3, 4, 5, 6, 7, 8, 9, 10, 11, 12, 13, 14, 15, 16, 17, 18, 19, or 20 days, including ranges between any two of the listed values, for example every 1-2 days, every 1-3 days, every 1-5 days, every 1-10 days, every 1-20 days, every 2-3 days, every 3-5 days, every 3-10 days, every 3-20 days, every 5-10 days, or every 5-20 days. In some embodiments, at least 2 dosages are administered, for example 2, 3, 4, 5, 6, 7, 8, 9, 10, 11, 12, 13, 14, 15, 16, 17, 18, 19, 20, 25, 30, 35, 40, 45, 50, 60, 70, 80, 90, or 100 doses, including ranges between any two of the listed values, for example, 2-10, 2-20, 2-50, 2-100, 5-10, 5-50, 5-100, 10-20, 10-50, 10-100, 20-50, or 20-100 doses. In some embodiments, the patient is a human.

In some embodiments, the subject to which the miltefosine or miltefosine analog (the latter forming part of the present disclosure) is administered has an inflammatory condition, for example, fatty liver disease, alcohol induced hepatitis, non-alcoholic steatosis hepatitis, cirrhosis, non-alcoholic fatty liver disease, fibrosis, primary sclerosing cholangitis, primary biliary sclerosis, fulminating cirrhosis, idiopathic hepatitis, viral-induced hepatitis (A, B, C and other), autoimmune hepatitis, inflammatory hepatitis associated with hepato-biliary carcinoma, multiple sclerosis, type 1 diabetes, ischemic reperfusion injury, solid organ transplantation, systemic lupus erythematosus, rheumatoid arthritis, amyotrophic lateral sclerosis, and inflammatory bowel disease (Crohn's and colitis).

### Pharmaceutical Compositions

The NKT-2 activators, such as miltefosine or miltefosine analogs or phospholipids, RAR agonist compounds, and sulfatide compounds described herein (the latter four examples forming part of the present disclosure) may be used as an active ingredient incorporated into a pharmaceutical composition. In some embodiments the pharmaceutical composition may comprise a single active ingredient. In some embodiments the pharmaceutical composition may comprise two, three, four, five or more active ingredients. Any of the following NKT-2 activator compositions can be for use in treating, ameliorating, or preventing an inflammatory condition, for example, fatty liver disease, alcohol induced hepatitis, non-alcoholic steatosis hepatitis, autoimmune hepatitis, cirrhosis, non-alcoholic fatty liver disease, fibrosis, primary sclerosing cholangitis, primary biliary sclerosis, fulminating cirrhosis, idiopathic hepatitis, viral-induced hepatitis (A, B, C and other), inflammatory hepatitis associated with hepato-biliary carcinoma, multiple sclerosis, type 1 diabetes, ischemic reperfusion injury, solid organ transplantation, systemic lupus erythematosus, rheumatoid arthritis, amyotrophic lateral sclerosis, and inflammatory bowel disease (Crohn's and colitis).

In some embodiments, the pharmaceutical composition comprises an amount of NKT-2 activator (for example miltefosine or a miltefosine analog or a phospholipid, the latter two examples forming part of the present disclosure) sufficient to activate type II NKT cells. Optionally, the pharmaceutical composition further comprises an amount or RAR agonist, such as a retinoid for example tazarotene sufficient to inhibit activation of type I NKT cells.

In some aspects of the disclosure, the pharmaceutical composition comprises an amount of NKT-2 activator (for example miltefosine or a miltefosine analog or a phospholipid) and an amount of sulfatide that together are sufficient to activate type II NK cells.

In some aspects of the disclosure, the pharmaceutical composition comprises an amount of NKT-2 activator (for example miltefosine or a miltefosine analog or a phospholipid) and an amount of sulfatide that together are sufficient to activate type II NK cells, and further comprises an amount of RAR agonist, such as a retinoid (for example tazarotene) sufficient to inhibit activation of type I NKT cells.

In some embodiments, the pharmaceutical composition comprises an amount of NKT-2 activator (for example miltefosine or a miltefosine analog or a phospholipid, the latter two examples forming part of the present disclosure) sufficient to activate type II NKT cells, and an amount of sulfatide sufficient to activate type II NKT cells.

In some embodiments, the pharmaceutical composition comprises an amount of NKT-2 activator (for example miltefosine or a miltefosine analog or a phospholipid, the latter two examples forming part of the present disclosure) sufficient to activate type II NKT cells, an amount of sulfatide sufficient to activate type II NKT cells, and an amount of RAR agonist, such as a retinoid (for example tazarotene) sufficient to inhibit activation of type I NKT cells.

In some embodiments, the pharmaceutical composition comprises an amount of miltefosine or a miltefosine analog (the latter forming part of the present disclosure)_sufficient to activate type II NKT cells and an amount of tazarotene sufficient to inhibit activation of type I NKT cells.

All modes of administration are contemplated, for example, orally, rectally, parenterally, topically, or by intravenous, intramuscular, intrasternal or subcutaneous injection or in a form suitable by inhalation. The formulations may, where appropriate, be conveniently presented in discrete dosage units and may be prepared by any of the methods well known in the art of pharmacy. The active ingredients will ordinarily be formulated with one or more pharmaceutically acceptable excipients in accordance with known and established practice. Thus, the pharmaceutical composition can be formulated as a liquid, powder, elixir, injectable solution, suspension, suppository, etc. Approaches for pharmaceutical formulations are described in detail in Remington's Pharmaceutical Sciences, 18th Edition, A.R. Gennaro, ed., Mack Publishing Company (1995).

Active ingredients according to some embodiments can be formulated for administration for oral administration as tablets, hard gelatin capsules, soft gelatin capsules, comprising the active in the form of a powder, a blend with excipients, a liquid or solution, a suspension, a solid solution. Active ingredients according to some embodiments can be formulated for administration for intra-oral administration (sublingual or buccal) as a solid dosage form rapidly dissolving or effervescent tablets, thin films, buccal wafers, or as a liquid or semi-solid form, such as a gel, solution, emulsion, suspension. Active ingredients according to some embodiments can be formulated for administration for injection as an aqueous or non-aqueous solution, suspension or emulsion. Oil-based solutions and suspensions comprise mixtures of natural and or synthetic oils such as soybean oil, cotton seed oil, mineral oil, sesame oil, castor oil, hydrogenated vegetable oils, beeswax. Active ingredients according to some embodiments can be formulated for administration for transdermal administration as a cream, a gel, an emulsion, an aqueous-based solution, an oil-based solution, a suspension, a film, a patch, a foam. Active ingredients according to some embodiments can be formulated for administration for intranasal administration as a powder, suspension, solution, emulsion. Active ingredients according to some embodiments can be formulated for administration for pulmonary delivery as a micronized powder. Oral administration is associated with first pass metabolism as well as induction of metabolizing enzymes. Thus dosage strength and dosing regimen of oral administered retinoic acids may be tailored for optimal effect. Alternative routes of delivery, e.g. sublingual, buccal, injection, pulmonary and transdermal, may be a preferred over oral administration. Alternative routes of administration, such as those as described, avoid first pass metabolism and GI absorption, demonstrate less enzyme induction and provide steady repeat dose pharmacokinetics.

Pharmaceutical formulations according to the present embodiments may be immediate release or modified release (e.g., sustained release, pulsatile release, controlled release, delayed release, slow release). Because it is immediately active, pharmacological amounts of orally administered Retinoid isomers may have side effects. These side effects have been a serious limitation to the use of oral retinoids in therapy. Although topically applied retinoids carry little teratogenic liability there are other toxicities associated with this route of administration that limit their use including skin irritation. A major reason for both oral and topical toxicity is that the retinoids are totally and immediately available upon administration. A process whereby a retinoid can be made available in vivo more slowly and more continuously would avoid peaks and valleys in the availability of the retinoid thereby providing an effective in vivo level of the compound over a more prolonged period of time and also avoiding or substantially reducing the toxicities that often result from the sudden availability of excessive amounts of the substance. An oil based injectable formulation of retinol, ester of retinol, and in particular a fatty ester of retinol, retinoic acid or a retinoic acid ester could be administered intra-muscularly on a weekly basis and provide a systemic slow-release delivery, according to such principles.

Some embodiments relate to formulation of an oral dosage form of one or more active ingredient of the present embodiments as described herein. 10g retinoic acid is dissolved in a mixture of beeswax, butylated hydroxyanisole, edetate disodium, hydrogenated soybean oil flakes, hydrogenated vegetable oils and soybean oil alcohol at slightly elevated temperature and with high-shear mixing. The mixture is sealed into soft-gelatin capsules at dosage strengths of 2mg, 5mg and 10mg for oral administration.

Some embodiments relate to formulation of a bioadhesive buccal tablet containing one or more active ingredient of the present embodiments. A blend of excipients is prepared containing 24% active ingredient, 21% HPMC, 18% Corn Starch, 24% Lactose, 1% Silica, 2.5% Polycarbophil (Noveon), 7.5% Carbomer 974P, 1.2% Talc and 0.7% Magnesium Stearate. The blend was pressed into tablets approximately 1cm in diameter.

Some embodiments relate to formulation of a sublingual film containing one or more active ingredient of the present embodiments. The following are mixed in 50g water: 3g Methocel E5, 5g Methocel E50, 1g Klucel, 1g Maltodextrin, 1g citric acid, 3g sucralose, 5g Orange flavor, 0.2g paraben, 0.1 edetate sodium and 5g Sorbitol. 1g retinoic acid is added and the mixture is degassed with stirring. The composition is thinly spread across a polyester film support and allowed to dry in the air in the absence of any direct light to avoid degradation. The film is then cut to size to generate doses of 2 to 10mg.

Some aspects of the disclosure relate to a kit, which may include one or more sulfatides, RAR agonists or any combination thereof, preferably as a pharmaceutical composition. In some aspects of the disclosure, cis-tetracosenoyl is provided in a pharmaceutically acceptable carrier. In some aspects of the disclosure, ATRA is provided in a pharmaceutically acceptable carrier. In some aspects of the disclosure, tazarotene is provided in a pharmaceutically acceptable carrier. In several aspects of the disclosure, hydrogen sulfide (H₂S) may optionally be provided. In several aspects of the disclosure, one or more antioxidants may optionally be provided. In several aspects of the disclosure, kits may further comprise suitable packaging and/or instructions for use. Kits may also comprise a means for the delivery of the one or more sulfatides, RAR agonists or any combination thereof, such as an inhaler, spray dispenser (e.g., nasal spray), syringe for injection, needle, IV bag or pressure pack for capsules, tables, suppositories. The one or more sulfatides, RAR agonists or any combination thereof can be in a dry or lyophilized form or in a solution, particularly a sterile solution. When in a dry form, the kit may comprise a pharmaceutically acceptable diluent for preparing a liquid formulation. The kit may contain a device for administration or for dispensing the compositions, including, but not limited to, syringe, pipette, transdermal patch, or inhalant. Some aspects of the disclosure relate to kits that contain sufficient dosages of the compounds or composition to provide effective treatment for an individual for an extended period, such as a week, 2 weeks, 3 weeks, 4 weeks, 6 weeks, or 8 weeks or more.

### CD8 Regulatory T cells

It is contemplated that a combination of administering CD8 T_{reg} cells and administering an NKT-2 activator in accordance with some aspects of the disclosure herein can be usefu for treating or preventing an inflammatory condition, for example an autoimmune disease. In some aspects of the disclosure, CD8 T_{reg} cells are administered to a subject who has, or is at risk of developing an inflammatory condition. An NKT-2 inhibitor can also be administered to the subject. Optionally, the NKT-2 inhibitor comprises miltefosine. Optionally, the inflammatory condition comprises at least one of: fatty liver disease, alcohol induced hepatitis, non-alcoholic steatosis hepatitis, autoimmune hepatitis, cirrhosis, non-alcoholic fatty liver disease, fibrosis, primary sclerosing cholangitis, primary biliary sclerosis, fulminating cirrhosis, idiopathic hepatitis, viral-induced hepatitis (A, B, C and other), inflammatory hepatitis associated with hepato-biliary carcinoma, multiple sclerosis, type 1 diabetes, ischemic reperfusion injury, solid organ transplantation, systemic lupus erythematosus, rheumatoid arthritis, amyotrophic lateral sclerosis, and inflammatory bowel disease (Crohn's and colitis), or a combination of two or more of these listed items.

The immune system maintains a state of equilibrium while responding to foreign antigens as well as self-antigens. Control mechanisms for maintaining homeostasis following an immune response to a foreign antigen or for preventing or aborting harmful responses to self-antigens include CTLA-4-mediated T cell inhibition, activation-induced cell death (AICD), IL-2-mediated regulation, and regulatory T cells (T_{reg})(Abbas, A.K., Lohr, J., Knoechel, B. & Nagabhushanam, V. T cell tolerance and autoimmunity. Autoimmun Rev 3, 471-5 (2004)). Induction of CD8 T_{reg} cells is also described in PCT Pub. No. WO 2007/136518 and US Pub. No. 2007/0286849.

Experiments using depleting antibodies or animals with a specific gene knockout have indicated an important regulatory role for CD8⁺ lymphocytes in autoimmune diseases (Koh, D.R. et al. Less mortality but more relapses in experimental allergic encephalomyelitis in CD8-/- mice. Science 256, 1210-3 (1992)), transplant tolerance (Seydel, K. et al. Anti-CD8 abrogates effect of anti-CD4-mediated islet allograft survival in rat model. Diabetes 40, 1430-4 (1991)), neonatal tolerance (Field, A.C., Caccavelli, L., Bloch, M.F. & Bellon, B. Regulatory CD8+ T cells control neonatal tolerance to a Th2-mediated autoimmunity. J Immunol 170, 2508-15 (2003)), and homeostasis of normal cellular and humoral immune responses (Nanda, N.K. & Sercarz, E. A truncated T cell receptor repertoire reveals underlying immunogenicity of an antigenic determinant. J Exp Med 184, 1037-43 (1996)).

In multiple sclerosis, for example, the immune system pathologically recognizes some self-antigens from myelin membranes as foreign and initiates an immune response against them. This results in demyelination, the destructive removal of myelin which is an insulating and protective fatty protein that sheaths nerve cells (neurons). The demyelination in multiple sclerosis is mediated by a T-cell guided immune response that is either initiated from antigen-presenting events in the CNS or induced following the peripheral activation by a systemic molecular mimicry response.

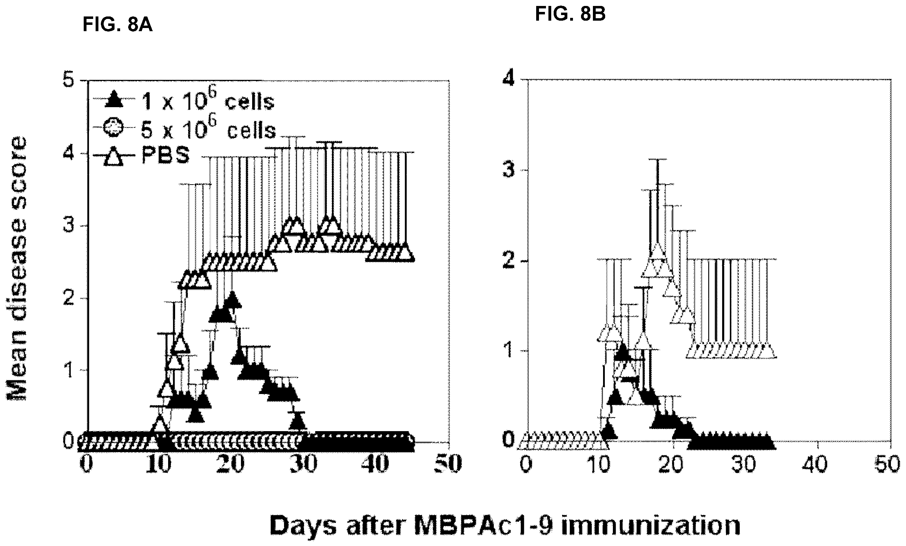

Without being limited by any theory, CD8αα⁺, TCRαβ⁺ T_{reg} cells can be useful to treat and/or prevent the indications of autoimmune and immune related diseases and disorders. An NKT-2 inhibitor such as miltefosine can induce CD8 T_{reg} cells in the liver of the subject (*see, e.g.* **Figures 8A-B**)**.** As such, in some embodiments, CD8 T_{reg} cells are administered to a subject having, or at risk of having an inflammatory condition as described herein. Optionally an NKT-2 inhibitor is further administered to the subject. In some embodiments, the NKT-2 inhbitor comprises miltefosine. Optionally, the administered CD8 T_{reg} cells are from the subject (i.e. "autologous" CD8 T_{reg} cells). Optionally, the administred CD8 T_{reg} cells are from another invidivual (i.e. "allogeneic" CD8 T_{reg} cells). Optionally, the administered CD8 T_{reg} cells are expanded before being adminstred to the subject, for example the cells can be expanded *in vitro.* It is contemplated that pathological self-reactive immune responses at the root of inflammatory conditions such as autoimmune diseases or disorders can be treated and reduced or eliminated.

Generally, to achieve an effective final concentration in, e.g., the intestines or blood, the amount of the expanded T_{reg} cell population in a single dosage composition in accordance with embodiments herein will generally be from about 10,000 to about 1 trillion cells, preferably from about 100,000 to about 100 million cells, more preferably from about 1 million to about 50 million cells, even more preferably from about 10 million to about 30 million cells, even more preferably from about 15 million to about 25 million cells, and even more preferably about 20 million cells. Likewise, the amount of a secondary therapeutic compound in a single oral dosage composition of the present embodiments will generally be in the range of about 0.01 milligrams to about 1000 milligrams, more preferably about 0.1 milligrams to about 100 milligrams. Obviously, the exact dosage will vary with the disease or disorder being treated, the preferred ranges being readily determinable.

In some embodiments, the expanded T_{reg} cell population can be combined with a pharmaceutically acceptable vehicle. Suitable pharmaceutically acceptable vehicles include, for example, phosphate buffered saline. In one embodiment, from about 1,000 to about 3,000,000 cells/kg body weight of T_{reg} cells are administered to the patient. Preferably, from about 100,000 to about 1 million cells/kg body weight of T_{reg} cells are administered. This dosage can be repeated as needed on an hourly, daily, weekly, monthly or sporadic basis. Exemplary dosages and dose schedules are discussed *infra.*

In some aspects of the disclosure, the T_{reg} cells administered comprise CD8αα⁺, TCRαβ⁺, CD200⁺ cells. In some aspects of the disclosure, the T_{reg} cells administered comprise CD8αα⁺, TCRαβ⁺, IL-2Rβ⁺ (CD122⁺). In some aspects of the disclosure, the T_{reg} cells administered comprise CD8αα⁺, TCRαβ⁺, CD200⁺, CD122⁺. Without being limited by any theory it is contemplated that each of CD8αα⁺, TCRαβ⁺, CD200⁺ T_{reg} cells, CD8αα⁺, TCRαβ⁺, CD122⁺ T_{reg} cells and CD8αα⁺, TCRαβ⁺, CD200⁺, CD122⁺ T_{reg} cells also control the population of activated Vβ8.2⁺ CD4 T cells *in vivo* and can be utilized in similar ways as the CD8αα⁺, TCRαβ⁺ T_{reg} cells described herein.

In some aspects of the disclosure, the regulatory T cells are administered to the same subject from which they were obtained. In other aspects of the disclosure, the regulatory T cells can be administered to a subject other than the subject from which the they were obtained. In still other aspects of the disclosure, the regulatory T cells can be obtained from a mammal that is not a subject. In some aspects of the disclosure, the administered regulatory T cells comprise a mixture of cells obtained from at least two of the subject to whom the regulatory T cells are administered, a subject other than the subject to whom the regulatory T cells are administered and a non-subject mammal.

In some aspects of the disclosure, anti-CD3 coated plates with growth factors such as IL-2, IL-7 and IL-15 are used to expand the T cell population. In some aspects of the disclosure, T_{reg} can be expanded *in vitro* using recombinant TCR proteins or peptides, for example p42-50 derived from the TCR Vβ 8.2 chain. Optionally, the the TCR Vβ or Vα chain gene utilized by disease-specific pathogenic T cells can be determined. Then the proteins corresponding to those TCR Vβ or Vα chain genes can be introduced into the body to activate the appropriate Treg cell population.

A number of different modes and methods of administration of the therapeutic T_{reg} cell population are suitable in accordance with embodiments described herein. In some aspects of the disclosure, delivery routes include, for example, intravenous, intraperitoneal, inhalation, intramuscular, subcutaneous, nasal and oral administration or any other delivery route available in the art. Depending on the particular administration route, the dosage form may be, for example, solid, semisolid, liquid, vapor or aerosol preparation. The dosage form may include, for example, those additives, lubricants, stabilizers, buffers, coatings, and excipients available in the art of pharmaceutical formulations.

A number of different pharmaceutical formulations are contemplated in accordance with embodiments described herein. In some aspects of the disclosure, a pharmaceutical formulation comprises CD8 T_{reg} cells and an NKT-2 activator as described herein. In some embodiments, a pharmaceutical formulation comprises CD8 T_{reg} cells and miltefosine. In some embodiments, a pharmaceutical formulation comprises at least one of the following types of CD8 T_{reg} cells: CD8αα⁺ TCRαβ⁺ T cells, CD8αα⁺ TCRαβ⁺ CD200⁺ T cells, CD8αα⁺ TCRαβ⁺ CD122⁺ T cells, and CD8αα⁺, TCRαβ⁺, CD200⁺ CD122⁺ T cells, and further comprises an NKT-2 activator as described herein. In some embodiments, a pharmaceutical formulation comprises at least one of the following types of CD8 T_{reg} cells: CD8αα⁺ TCRαβ⁺ T cells, CD8αα⁺ TCRαβ⁺ CD200⁺ T cells, CD8αα⁺ TCRαβ⁺ CD122⁺ T cells, and CD8αα⁺, TCRαβ⁺, CD200⁺ CD122⁺ T cells, and further comprises miltefosine. In some aspects of the disclosure, a first pharmaceutical formulation comprises CD8 T_{reg} cells and a second pharmaceutical formulation comprises an NKT-2 activator as described herein.

In some aspects of the disclosure, a first pharmaceutical formulation comprises CD8 T_{reg} cells and a second pharmaceutical formulation comprises miltefosine. In some aspects of the disclosure, a first pharmaceutical formulation comprises at least one of the following types of CD8 T_{reg} cells: CD8αα⁺ TCRαβ⁺ T cells, CD8αα⁺ TCRαβ⁺ CD200⁺ T cells, CD8αα⁺ TCRαβ⁺ CD122⁺ T cells, and CD8αα⁺, TCRαβ⁺ CD200⁺ CD122⁺ T cells and a second pharmaceutical formulation comprises an NKT-2 activator. In some aspects of the disclosure, a first pharmaceutical formulation comprises at least one of the following types of CD8 T_{reg} cells: CD8αα⁺ TCRαβ⁺ T cells, CD8αα⁺ TCRαβ⁺ CD200⁺ T cells, CD8αα⁺ TCRαβ⁺ CD122⁺ T cells, and CD8αα⁺, TCRαβ⁺, CD200⁺ CD122⁺ T cells and a second pharmaceutical formulation comprises miltefosine. In some aspects of the disclosure, the first and second pharmaceutical formulations are administered concurrently. In some aspects of the disclosure, the first and second pharmaceutical formulations are administered at different times. In some aspects of the disclosure, the first pharmaceutical formulation is administered prior to the second pharmaceutical formulation. In some aspects of the disclosure, the second pharmaceutical formulation is administered prior to the first pharmaceutical formulation. Optionally, any of the pharmaceutical formulations described herein can be prepared by conventional methods using the following pharmaceutically acceptable vehicles or the like: excipients such as solvents (e.g., water, physiological saline), bulking agents and filling agents (e.g., lactose, starch, crystalline cellulose, mannitol, maltose, calcium hydrogenphosphate, soft silicic acid anhydride and calcium carbonate); auxiliaries such as solubilizing agents (e.g., ethanol and polysolvates), binding agents (e.g., starch, polyvinyl pyrrolidine, hydroxypropyl cellulose, ethylcellulose, carboxymethyl cellulose and gum arabic), disintegrating agents (e.g., starch and carboxymethyl cellulose calcium), lubricating agents (e.g., magnesium stearate, talc and hydrogenated oil), stabilizing agents (e.g., lactose, mannitol, maltose, polysolvates, macrogol, and polyoxyethylene hydrogenated castor oil), isotonic agents, wetting agents, lubricating agents, dispersing agents, buffering agents and solubilizing agents; and additives such as antioxidants, preservatives, flavoring and aromatizing agents, analgesic agents, stabilizing agents, coloring agents and sweetening agents.

The following examples are provided for illustrative purposes only, and are in no way intended to limit the embodiments described herein.

### EXAMPLE 1

In a plate-bound CD1d assay miltefosine is able to stimulate type II NKT cell hybridoma, Hy19.3. The CDld protein coated 96-well plates were incubated with the indicated lipids for 6 hr, washed and a type II NKT cell Hy 19.3 was added (50,000 cells/well) and IL-2 measured 16 hr later in the supernatant. LSF, lysosulftatide; LPC, lysophosphatidylcholine.

These data show that miltefosine, an analog of lysophosphatidylcholine (LPC), can bind to CD 1d molecules and stimulate a type II NKT cell.

### EXAMPLE 2

Naive B6 mice were treated every day for 7 days with DMSO (Control) or Miltefosine 1 mg (dissolved in DMSO and diluted into PBS), using oral gavage. Splenocytes were cultured in vitro for 90 hrs in the presence or absence of alpha-GalCer. Stimulation (as stimulation Index) and expansion of type I NKT cells (as FACS staining with alpha-GalCer/CD1d⁻tetramer⁺ cells) at 10 ng/ml of alpha-GalCer concentration is shown in both **Figure 2A** and **Figure 2B****.**

The data in **Figure 2A** and **Figure 2B** show that in miltefosine treated animals there is a significant inhibition of type I NKT cells proliferation and expansion in the presence of their cognitive ligand alpha-GalCer.

These data are consistent with the observation that other lipids, such as sulfatide (a glycolipid) or lysophosphatidylcholine (LPC, a phospholipid) activate type II NKT cells, leading to inactivation or inhibition of type I NKT cells.

### EXAMPLE 3

Groups of BL/6 mice were either administered with DMSO/PBS or miltefosine 12 hr before ConA administration and liver injury was determined by H&E staining of liver sections and liver enzymes, such as ALT in serum examined 24 hr later **(****Figure 3A** and **Figure 3B****).** Data shown are representative of 2 independent experiments.

As shown in **Figure 3B** staining indicative of necrosis (red H&E staining) 1 was observed in liver sections treated with con A, but staining indicative of decreased necrosis (reduced red H&E staining) **2** was observed in liver sections treated with con A and miltefosine.

Accordingly, inhibition of ConA-induced hepatitis was observed following treatment with miltefosine. These data show that similar to the sulfatide or LPC, treatment of mice with miltefosine results in a significant inhibition of liver injury following Con A injection.

### EXAMPLE 4

Groups of C57BL/6 mice were fed liquid alcohol (EtOh) diet or isocaloric control (control) diet for 10 day either containing miltefosine (∼500 micrograms per day) or DMSO/PBS. On day 11, 9 hr after the binge (chronic plus binge feeding), accumulation and activation of type I NKT cells (alpha-GalCer/CD1d⁻tetramer⁺ cells) or neutrophils (Ly⁻6G⁺ CD11b⁺) were examined in liver. As shown in **Figure 4A** and **Figure 4B****,** in comparison to the control diet group, type I NKT cells accumulate into liver in mice fed liquid chronic plus binge EtOh diet. Also as shown in **Figure 4C** and **Figure 4D****,** type I NKT cells become activated (as examined by the expression of CD69 cell surface expression in alpha-GalCer/CD1d-tetramer⁺ cells) accompanied by accumulation of neutrophils (Ly⁻6G⁺CD11b⁺ cells) into liver. Accumulation of activated type I NKT cells and neutrophils leads to inflammatory cascade resulting in heaptocyte injury during ALD. Notably in mice tretaed with miltefosine both of these cells do not accumulate resulting in protection from liver injury.

These data show inhibition of the accumulation of activated type I NKT cells as well as neutrophils during alcoholic liver diseases (ALD) in mice treated with miltefosine. As such, it is contemplated that miltefosine can be used in the treatment of inflammatory liver diseases, including ALD, NASH, PBC, PSC, viral hepatitis etc.

### EXAMPLE 5

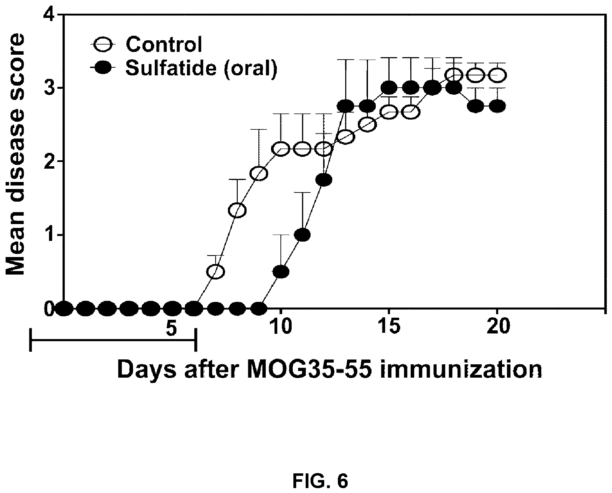

It has bene observed that treatment with sulfatide results in a significant inhibition of chronic and relapsing EAE in SJL/J mice. However as shown in **Figure 6** oral treatment with sulfatide though inhibits disease initially when given orally but not very efficient. Without being limited by any theory, it is contemplated that sulfatide is not very stable in the gut, which is consistent with the observed low efficiency. With reference to **Figure** 6, groups of B6 mice were orally treated with sulfatide (200 micrograms in 200 microliters in PBS) for 7 days, starting one day before the disease induction. Clinical paralysis was monitored daily and scored from 1 to 5. Although there is protection initially for 4-5 day but then mice in both groups contract similar severity of disease. Without being limited by any theory, the observed similarity of both groups in consistent with instability of sulfatide in the gut.

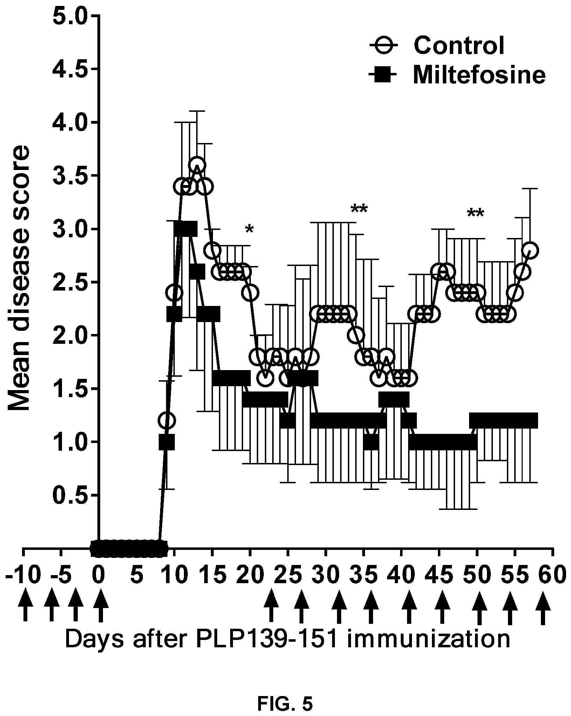

As shown in **Figure 5****,** it was examined whether oral treatment with miltefosine which is supposed to be relatively stable can inhibit chronic and relapsing EAE, a prototype for multiple sclerosis. Groups of SJL mice were orally gavaged with miltefosine (1mg in 200 microliters of 10% DMSO/PBS) or control (DMSO/PBS), twice a week as indicated by arrows. Clinical severity of disease was monitored daily as paralysis in both groups of mice from scale 1 to 5 and shown.

The data in **Figure 5** summarize results for treatment of chronic and relapsing experimental autoimmune encephalomyelitis (EAE), a prototype for multiple sclerosis with miltefosine. These experiments show that T cell mediated autoimmune diseases, including MS, T1DD, RA, Lupus and IBD etc. can be treated with miltefosine.

### EXAMPLE 6

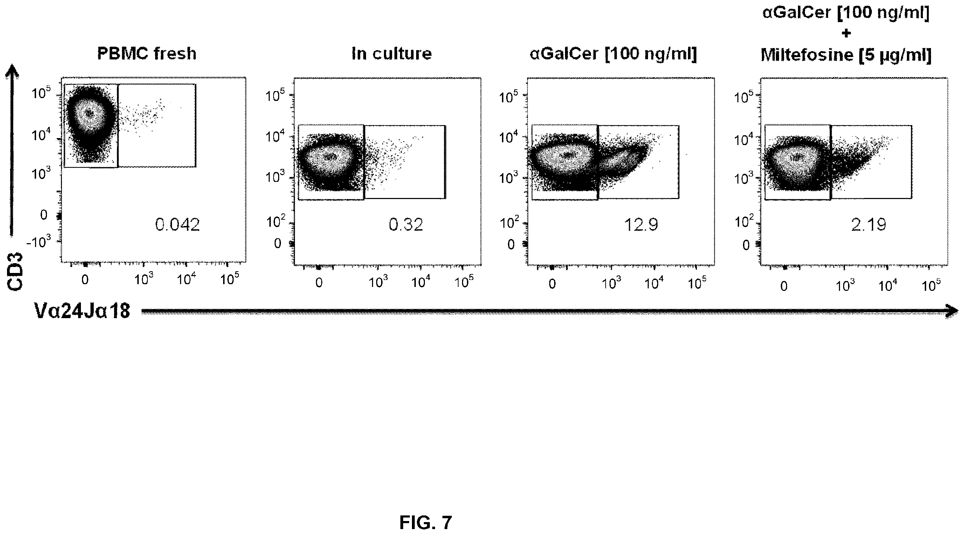

PBMC (1 x 10⁶/well) were stimulated in vitro with 100 ng/ml of αGalCer, 100 ng/ml of αGalCer + 5 µg/ml of Miltefosine or without stimulation. After 12 days, the cells were harvested and the percentage of type I NKT cells were assessed by flow cytometry. Stained cells were collected using the BD FACS Canto. Data were analyzed using FlowJo software. Human type I NKT cells were identified as CD3+/Vα24Jα18 TCR+ cells (CD1d-restricted type I NKT cells) and are shown as % NKT cells in the gated area in **Figure** 7. The percentages of type I NKT cells from fresh PBMC (without culture) and PBMC after in vitro expansion are shown within each plot. The data shown in **Figure 7** are representative of more than 5 independent experiments.

Accordingly, it is shown that in accordance with some embodiments herein, Miltefosine treatment can lead to expansion of human type I NKT cells.

In this application, the use of the singular can include the plural unless specifically stated otherwise or unless, as will be understood by one of skill in the art in light of the present disclosure, the singular is the only functional embodiment. Thus, for example, "a" can mean more than one, and "one embodiment" can mean that the description applies to multiple embodiments.

The foregoing description and Examples detail certain disclosures.

### EXAMPLE 7

Groups of female SJL/J mice were administered orally with 1 mg of miltefosine as shown in the figure 2, before and during the course of disease. Mice were sacrificied on day 59. Flow cytometric profiles of liver mononuclear cells stained with CD4, CD8α and CD8β mAb are shown in **Figures 8A-B**. Interestingly, while regulatory CD8αα⁺ T cells are significantly increased in liver, other CD4+ and CD8αβ+ T cells are not different in control vs. treated groups.

Accordingly, administration of NKT-2 inhbiitors such as miltefosine in accordance with some embodiments herein can increase induction of CD8αα⁺ T cells in the liver.

### EXAMPLE 8

To determine the in vivo regulatory potential of the CD8 Treg, 2D11 cells were adoptively transferred intravenously into syngeneic mice. After 24 hours, recipients were immunized with MBPAc1-9/CFA/PT for the induction of EAE, and clinical symptoms were monitored daily and scored for 35-45 days on a scale from 1-5: 1 being tail paralysis; 2 being hind limb paralysis; 3 being hind body paralysis; 4 being hind limb and partial body paralysis; and 5 being whole body paralysis or moribund. As shown in Figure 8A, mice injected with 1 million 2D11 cells recover more rapidly from a milder disease than those in the control group. To rule out the possibility that the ability to control disease is an artifact of the long-term culture of a particular clone, short-term p42-50-reactive T cell lines were generated and used in similar adoptive transfer experiments. As shown in Figure 8B, mice that receive five million cells of a p42-50-reactive T cell line are completely protected from MBP-induced EAE, and transfer of only one million cells enables a more rapid recovery from a milder paralysis. Adoptive transfer of short-term T cell lines raised against two irrelevant TCR peptides had no effect on EAE (data not shown). See Figures 8A-B.

Accordingly, adoptive transfer of CD8 Treg lines and clones in accordance with some embodiments herein results in quick recovery and protection from EAE. Accordingly, it is contemplated that adoptive transfer of CD8 Treg lines and clones in accordance with some embodiments herein can alleviate and/or prevent at least one inflammatory condition.

### EXAMPLE 9

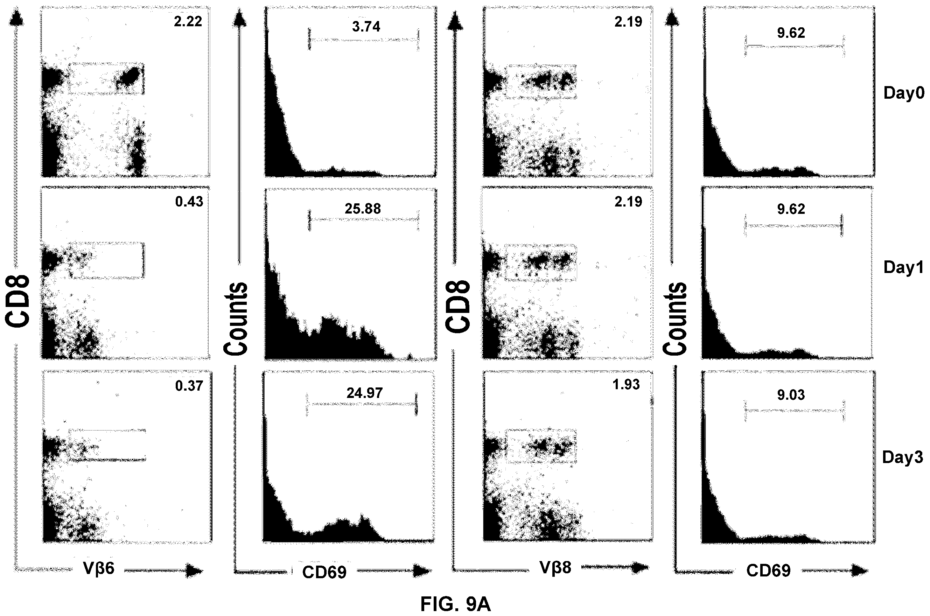

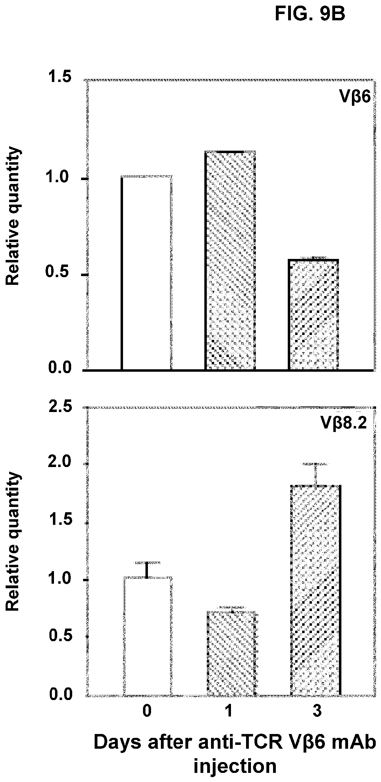

The predominant usage of the TCR Vβ6 chain by the CD8 T_{reg} is shown by an examination of whether treatment of mice with anti-Vβ6 mAb had any influence on the CD8 T_{reg} repertoire and the course of MBPAc1-9-induced EAE. Following a single intravenous injection of anti-TCR Vβ6 mAb (RR4-7, 300 µg/mouse), splenocytes were examined at different times for the presence of Vβ6⁺ cells *in vivo.* As expected, anti-TCR Vβ6 mAb injection led to early activation of TCR Vβ6⁺ T cells *in vivo* as determined by the expression of the early activation marker CD69 **(****Figure 9A****,** second column), followed by down-regulation of cell-surface Vβ6 expression **(****Figure 9A****,** first column). The effect of the anti-TCR Vβ6 mAb was TCR-specific because Vβ8⁺ T cells were not affected under identical experimental conditions **(****Figure 9A****,** third and fourth columns). Real-time RT-PCR analysis of TCR Vβ6 mRNA expression in splenocytes from anti-TCR Vβ6-treated mice revealed that most but not all of the TCR Vβ6⁺ T cells were depleted following continuous treatment **(****Figure 9B** and data not shown).

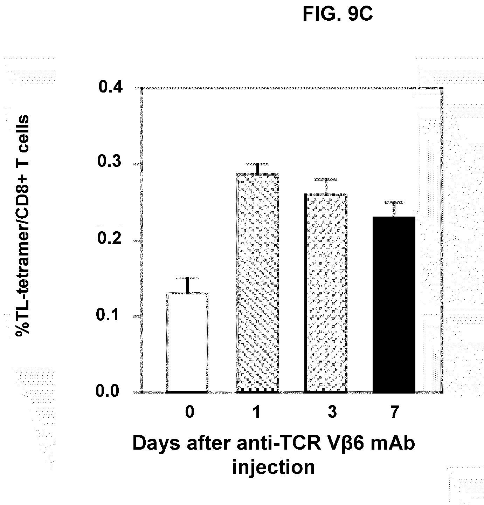

CD8αα T cells predominantly present in intraepithelial lymphocytes (IEL) in the intestine are relatively resistant to deletion by self-agonists and might differ from CD8αβ T cells in their response to activation through the T cell receptor. It was examined whether anti-TCR Vβ6 mAb administration led to deletion or activation of the CD8αα T cells in the periphery using staining with the TL-tetramer. The data in **Figure 9C** show that TL-tetramer⁺ CD8⁺ cells are not depleted following antibody administration. In fact, a slight increase in an *in vitro* recall response to p42-50 was found in antibody-treated mice (data not shown). To further test the regulatory function after activation of the CD8αα⁺ Vβ6⁺ T cells, groups of mice were immunized with MBPAc1-9/CFA/PT for the induction of EAE. One day and one week later a low dose of either the anti-TCR Vβ6 mAb (100 µg/mouse), an isotype control mAb (100 µg/mouse), or PBS was injected intravenously. Clinical symptoms were monitored daily after the second injection. The data in **Figure 9D** and in **Table 4** show that *in vivo* activation of Vβ6⁺ T cells with the anti-TCR Vβ6 mAb resulted in protection from EAE in B10.PL mice. In contrast, animals in the group injected with the isotype control mAb or an irrelevant mAb were not protected from disease (data not shown).

Activation (CD69 expression) followed by down-regulation/depletion of Vβ6⁺, but not Vβ8⁺ T cells after a single intravenous injection with anti-TCR Vβ6 mAbs (300 µg/mouse). At days 0, 1, and 3, splenocytes were stained with TCR Vβ6-FITC/CD8α-PE/CD69-PerCP (first and second columns) or TCR Vβ8-FITC/CD8α-PE/CD69-PerCP (third and fourth columns). The cells were gated on either TCR Vβ6/CD8α⁺ T cells (second column) or TCR Vβ8/CD8α⁺ T cells (fourth column) to analyze the expression of the early activation marker CD69. Data are representative of two independent experiments. (b) Significant depletion of Vβ6⁺ T cells following anti-Vβ6 injection as determined by Real-Time PCR. Expression of mRNA is presented as relative quantity after normalization against two internal control genes (L32 and Cyclophilin). Data are representative of two independent experiments. (c) TL-tetramer⁺ CD8 T cells are not deleted following anti-TCR Vβ6 injection. Splenocytes were stained with CD8α-FITC/TL-tetramer-PE on the indicated days following antibody injection. Data are representative of two independent experiments. (d) Mice injected with anti-TCR Vβ6 mAb are protected from EAE. One and seven days after the induction of EAE with MBPAc1-9/CFA/PT, groups of mice were injected with anti-TCR Vβ6 mAb (100 µg/mouse) or an IgG isotype control or PBS. Paralytic disease was monitored as described in the Methods. Data are combined from three independent experiments.

**Table 3: Response profile of the CD8αα⁺ T cell clone¹**

| | **H-2K** | **H-2D** | **H-2L** | **Qa-1** | **Qa-2** | **Response** |
|---|---|---|---|---|---|---|
| PL/J^{(u)2} | u | d | d | a | ? | + |
| B10.PL-H2u H2-T18a(73NS)SnJ^{(u)} | u | d | d | a | ? | + |
| NZB/BINJ^{(d)} | d | d | - | a | a(+) | + |
| B10.BR-H2k H2-T18a/SgSnJ^{(k)} | k | k | k | a | | + |
| SWR/J^{(q)} | q | q | q | a | a(+) | + |
| NOD.ALR-(D17Mit16-D17Mit10)NOD/Lt^{(g7)} | d | b | | a | ? | + |
| C57BL/6J^{(b)} | b | b | - | b | a(hi) | - |
| BALB/cJ ^{(d)} | d | d | d | b | a(lo) | - |
| SJL/J ^{(s)} | s | s | | b | a | - |

| | | | | | | |
|---|---|---|---|---|---|---|
| 1. The CD8αα T_{reg} clone 2D11 was examined for proliferation or cytokine secretion in response to p42-50 in the presence of antigen presenting cells (APCs) derived from different H-2 haplotyes. The H-2K, D, L, Qa-1, and Qa-2 haplotypes are shown. 2. H-2 haplotypes. 3. "+" and "-" in the last column refer to a positive response and no response to p42-50, respectively. Positive response is defined as stimulating indices (SI) greater than 3 (7.25+/-1.93 to 58.06+/-18.69). | | | | | | |

**Table 4: Activation of TCR Yβ6⁺ T cells leads to protection from EAE**

| **Antibodies¹** | **Incidence of EAE # of animals with disease/total # of animals (maximal individual disease scores)** | **Mean days of disease onset** |
|---|---|---|
| PBS | 3/4 (5,3,3,0) | 13.3+/-1.2 |
| IgG | 10/12 (5,5,4,3,3,3,3,2,1,1,0,0) | 14.8+/-3.2 |
| Anti-Vβ3 | 6/6 (5,5,4,4,2,1) | 13.1+/-2.2 |
| Anti-Vβ6 | 5/12 (5,4,2,1,1,0,0,0,0,0,0,0) | 26.7+/-5.5 |

| | | |
|---|---|---|
| 1. Antibodies (100 µg) were injected I.V. on Day1 and Day8 following MBPAc1-9 immunization (Day0). | | |

Accordingly, activation of TCR Vβ6+ T cells in vivo in accordance with some embodiments herein leads to protection from EAE

## Claims

1. A composition comprising miltefosine for use in alleviating or preventing at least one inflammatory condition in a subject, the method comprising administering the composition in an amount sufficient to activate type II NKT cells of the subject.

2. The composition for use according to claim 1 wherein the inflammatory condition is selected from the group consisting of: fatty liver disease, alcohol induced hepatitis, non-alcoholic steatosis hepatitis, autoimmune hepatitis, cirrhosis, non-alcoholic fatty liver disease, fibrosis, primary sclerosing cholangitis, primary biliary sclerosis, fulminating cirrhosis, idiopathic hepatitis, viral-induced hepatitis (A, B, C and other), inflammatory hepatitis associated with hepato-biliary carcinoma, multiple sclerosis, type 1 diabetes, ischemic reperfusion injury, systemic lupus erythematosus, rheumatoid arthritis, and amyotrophic lateral sclerosis.

3. The composition for use according to any one of claims 1-2, further comprising an amount of a RAR agonist sufficient to inhibit activation of type I NKT cells to the subject.

4. The composition for use according to any one of claims 1-3, wherein the NKT-2 activator is administered orally.

5. The composition for use according to any one of claims 3-4, wherein the RAR agonist comprises all-trans retinoic acid (ATRA) or isotretinoin.

6. The composition for use according to any one of claims 3-4, wherein the RAR agonist comprises tazarotene.

7. The composition for use according to any one of claims 3-4, wherein the RAR agonist comprises retinol or an ester of retinol.

8. The composition for use according to any one of claims 1-7, wherein activation of type II NKT cells comprises at least one of: production of IL-2 by the type II NKT cells, proliferation of type II NKT cells, or expression of CD69 on the surface of type II NKT cells.

9. The composition for use according to any one of claims 3-8, wherein inhibition of activation of type I NKT cells comprises inhibition of accumulation of alpha-GalCer/CD1d-tetramer⁺TCR-beta⁺ cells.

10. The composition for use according to any one of claims 1-9, wherein the amount of NKT-2 activator is sufficient to activate CD8αα⁺ T cells in the liver of the subject.

11. A composition for use according to claim 1, the composition further comprising:
an amount of a sulfatide sufficient to activate type II NKT cells in the subject.

12. The composition for use according to any one of claims 1-11, which is further **characterised by** being administered to the subject, to whom isolated CD8αα⁺, TCRαβ⁺ T cells are being administered.

## Patentansprüche

1. Zusammensetzung, umfassend Miltefosin zur Verwendung beim Lindern oder Verhindern wenigstens eines Entzündungszustands in einem Subjekt, wobei das Verfahren ein Verabreichen der Zusammensetzung in einer Menge umfasst, die ausreicht, um Typ-II-NKT-Zellen des Subjekts zu aktivieren.

2. Zusammensetzung zur Verwendung nach Anspruch 1, wobei der Entzündungszustand aus der Gruppe ausgewählt ist, die aus Folgendem besteht:
Fettlebererkrankung, alkoholinduzierter Hepatitis, nichtalkoholischer Steatohepatitis, Autoimmunhepatitis, Zirrhose, nichtalkoholischer Fettlebererkrankung, Fibrose, primär sklerosierender Cholangitis, primärer biliärer Sklerose, fulminanter Zirrhose, idiopathischer Hepatitis, virusinduzierter Hepatitis (A, B, C und anderen), entzündlicher Hepatitis im Zusammenhang mit hepatobiliärem Karzinom, multipler Sklerose, Typ-1-Diabetes, ischämischer Reperfusionsverletzung, systemischem Lupus erythematodes, rheumatoider Arthritis und amyotropher Lateralsklerose.

3. Zusammensetzung zur Verwendung nach einem der Ansprüche 1-2, ferner umfassend eine Menge eines RAR-Agonisten, die ausreicht, um die Aktivierung von Typ-I-NKT-Zellen des Subjekts zu hemmen.

4. Zusammensetzung zur Verwendung nach einem der Ansprüche 1-3, wobei der NKT-2-Aktivator oral verabreicht wird.

5. Zusammensetzung zur Verwendung nach einem der Ansprüche 3-4, wobei der RAR-Agonist all-trans-Retinsäure (ATRA) oder Isotretinoin umfasst.

6. Zusammensetzung zur Verwendung nach einem der Ansprüche 3-4, wobei der RAR-Agonist Tazaroten umfasst.

7. Zusammensetzung zur Verwendung nach einem der Ansprüche 3-4, wobei der RAR-Agonist Retinol oder einen Ester von Retinol umfasst.

8. Zusammensetzung zur Verwendung nach einem der Ansprüche 1-7, wobei die Aktivierung von Typ-II-NKT-Zellen Folgendes umfasst:
eine Herstellung von IL-2 durch die Typ-II-NKT-Zellen, eine Proliferation von Typ-II-NKT-Zellen und/oder eine Expression von CD69 auf der Oberfläche von Typ-II-NKT-Zellen.

9. Zusammensetzung zur Verwendung nach einem der Ansprüche 3-8, wobei die Hemmung der Aktivierung von Typ-I-NKT-Zellen die Hemmung einer Akkumulation von alpha-GalCer/CD1d-Tetramer⁺TCR-beta⁺-Zellen umfasst.

10. Zusammensetzung zur Verwendung nach einem der Ansprüche 1-9, wobei die Menge des NKT-2-Aktivators ausreicht, um CD8αα⁺-T-Zellen in der Leber des Subjekts zu aktivieren.

11. Zusammensetzung zur Verwendung nach Anspruch 1, wobei die Zusammensetzung ferner Folgendes umfasst:
eine Menge eines Sulfatids, die ausreicht, um Typ-II-NKT-Zellen in dem Subjekt zu aktivieren.

12. Zusammensetzung zur Verwendung nach einem der Ansprüche 1-11, die ferner **dadurch gekennzeichnet ist, dass** sie dem Subjekt verabreicht wird, dem isolierte CD8αα⁺-, TCRαβ⁺-T-Zellen verabreicht werden.

## Revendications

1. Composition comprenant de la miltéfosine destinée à être utilisée dans le soulagement ou la prévention d'au moins un état inflammatoire chez un sujet, le procédé comprenant l'administration de la composition en une quantité suffisante pour activer les cellules NKT de type II du sujet.

2. Composition destinée à être utilisée selon la revendication 1, dans laquelle l'état inflammatoire est choisi dans le groupe constitué par : la stéatose hépatique, l'hépatite alcoolique, la stéatohépatite non alcoolique, l'hépatite auto-immune, la cirrhose, la stéatose hépatique non alcoolique, la fibrose, la cholangite sclérosante primitive, la cholangite biliaire primitive, l'hépatite fulminante, l'hépatite idiopathique, l'hépatite virale (A, B, C et autres), l'hépatite inflammatoire associée à un carcinome hépatobiliaire, la sclérose en plaques, le diabète de type 1, la lésion d'ischémie-reperfusion, le lupus érythémateux disséminé, la polyarthrite rhumatoïde et la maladie de Charcot.

3. Composition destinée à être utilisée selon l'une quelconque des revendications 1 à 2, comprenant en outre une quantité d'un agoniste de RAR suffisante pour inhiber l'activation des cellules NKT de type I chez le sujet.

4. Composition destinée à être utilisée selon l'une quelconque des revendications 1 à 3, dans laquelle l'activateur NKT-2 est administré par voie orale.

5. Composition destinée à être utilisée selon l'une quelconque des revendications 3 à 4, dans laquelle l'agoniste de RAR comprend de l'acide tout-trans-rétinoïque (ATRA) ou de l'isotrétinoïne.

6. Composition destinée à être utilisée selon l'une quelconque des revendications 3 à 4, dans laquelle l'agoniste de RAR comprend du tazarotène.

7. Composition destinée à être utilisée selon l'une quelconque des revendications 3 à 4, dans laquelle l'agoniste de RAR comprend du rétinol ou un ester de rétinol.

8. Composition destinée à être utilisée selon l'une quelconque des revendications 1 à 7, dans laquelle l'activation de cellules NKT de type II comprend : la production d'IL-2 par les cellules NKT de type II, et/ou la prolifération de cellules NKT de type II et/ou l'expression de CD69 à la surface de cellules NKT de type II.

9. Composition destinée à être utilisée selon l'une quelconque des revendications 3 à 8, dans laquelle l'inhibition de l'activation de cellules NKT de type I comprend l'inhibition de l'accumulation de cellules alpha-GalCer/CD1d à tétramères⁺TCR-beta⁺.

10. Composition destinée à être utilisée selon l'une quelconque des revendications 1 à 9, dans laquelle la quantité d'activateur NKT-2 est suffisante pour activer les cellules T CD8αα⁺ dans le foie du sujet.

11. Composition destinée à être utilisée selon la revendication 1, la composition comprenant en outre :
une quantité d'un sulfatide suffisante pour activer des cellules NKT de type II chez le sujet.

12. Composition destinée à être utilisée selon l'une quelconque des revendications 1 à 11, qui est en outre **caractérisée par** le fait d'être administrée au sujet, auquel des cellules T isolées CD8αα⁺, TCRαβ⁺ sont administrées.
